# EUROPEAN PATENT APPLICATION

(11) **EP 4 574 830 A1**
(43) Date of publication of application: **25.06.2025**
(21) Application number: 23867656.3
(22) Date of filing: 25.09.2023
(51) Int. Cl.: C07D 498/04, C07D 471/04, A61K 31/437, A61P 9/00, A61P 13/12

(54) **ANNELATED PYRIDINE STEROID SYNTHETASE INHIBITOR, PREPARATION METHOD THEREFOR, AND USE THEREOF**

(30) Priority: 23.09.2022 CN 202211162092
(71) Applicant: Guangzhou Vinnocent Pharmaceutical Limited, Guangzhou, Guangdong 510660 (CN)
(72) Inventor: HU, Qingzhong, 66123 Saarland (DE); YIN, Lina, Guangzhou, Guangdong 510660 (CN); LI, Haiyan, Guangzhou, Guangdong 510660 (CN)
(74) Representative: Patentwerk B.V.
(86) International application number: PCT/CN2023/121186
(87) International publication number: WO 2024/061371

(57) **Abstract**

Provided is a small-molecule steroid synthetase inhibitor. Further provided is use of the steroid synthetase inhibitors.

## Description

This application claims priority to China Patent Application No. 202211162092.4 filed September 23rd 2022. The entirety of which is incorporated herein by reference.

### TECHNICAL FIELD

The present invention relates to the field of steroidogenesis inhibitors, particularly to a class of annulated pyridines, their preparation methods and applications. These compounds inhibit the biosynthesis of endogenous steroidal hormones, and thus treat diseases related to excessive steroidal hormones.

### BACKGROUND

Steroidal hormones are important endogenous regulators in the human body, widely involved in various physiological and pathological processes. Dysfunctions in their biosynthesis or signal perception and transduction lead to various serious diseases. The steroidogenesis enzymes are key factors in the biosynthesis of these steroidal hormones, and the inhibition of such an enzyme directly reduces concentrations of the corresponding steroidal hormone, hence treats related diseases.

Aldosterone, as a potent mineralocorticoid, modulates the function of renal tubules, controls the homeostasis of potassium ions, sodium ions, and water, and thus regulates blood volume and blood pressure. Recent studies reveal that aldosterone is a potent pro-inflammatory factor capable of inducing reactive oxygen species and upregulating the expression of multiple fibrotic factors, including plasminogen activator inhibitor (PAI). Excessively high levels of aldosterone are directly related to diseases such as congestive heart failure, resistant hypertension, chronic kidney disease, diabetic nephropathy, hyperaldosteronism, cardiofibrosis, renal fibrosis, cardio-renal syndrome, and metabolic syndrome. Aldosterone synthase (CYP11B2) is a key enzyme in the biosynthesis of aldosterone, and its inhibition effectively lowers aldosterone levels, thereby treating related diseases.

Cortisol is an important glucocorticoid in the human body, widely regulating immune responses, stress reactions, and metabolism of lipids and carbohydrates. Abnormal excessive secretion of cortisol due to tumors in the hypothalamic-pituitary-adrenal axis leads to Cushing's syndrome. High levels of cortisol are also directly related to metabolic syndrome, insulin resistance, obesity, and type 2 diabetes. 11β-hydroxylase (CYP11B1) is a key enzyme in the biosynthesis of cortisol, and its inhibition effectively reduces cortisol levels, thus treats related diseases.

Bile acids are endogenous steroids secreted by the liver. Besides forming chylomicrons in the small intestine to promote fat absorption, they are also signaling factors that broadly regulate the synthesis and metabolism of carbohydrates and lipids, as well as pathological processes such as inflammation and fibrosis. CYP7A1 and CYP8B1 are key enzymes in bile acid biosynthesis, and intervention of these enzymes modulates liver functions and systemic metabolism, which justifies them as potential targets for treating non-alcoholic fatty liver disease, fatty liver, cirrhosis, and liver fibrosis.

### SUMMARY

The invention is directed towards compounds including but not limited to any of the compounds and formulae delineated herein, methods of preparing these compounds, methods of modulating activity of steroidogenesis enzymes, and methods of treating diseases, disorders or symptoms thereof. The methods can comprise the compounds herein.

It is understood that the embodiments of the invention discussed below with respect to the preferred variable selections can be taken alone or in combination with one or more embodiments, or preferred variable selections, of the invention, as if each combination were explicitly listed herein.

In one aspect, provided are compounds of formula **I:**

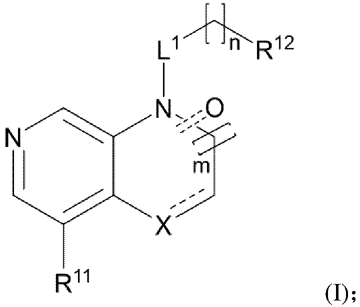

Wherein:
n is 0, 1, or 2;
m is 0 or 1;
X is C, N, O, or S;
L¹ is selected from the group consisting of hydrogen, -SO₂-, -CO-, and -CH₂-;
- - - is a single bond or a double bond;
::::::O means that one or more carbon atoms are replaced by carbonyl groups or not;
R¹¹ is selected from the group consisting of aryl, heteroaryl, spirocycloalkyl, azaspirocycloalkyl, oxaspirocycloalkyl, bicycloalkyl, azacycloalkyl, and azacyclic ketone; Said aryl, heteroaryl, spirocycloalkyl, bicycloalkyl, azacycloalkyl, and azacyclic ketone are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens; Alternatively, R¹¹ is of the following structure: 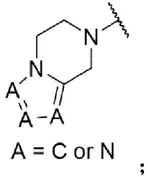
R¹² is selected from the group consisting of hydrogen, alkyl, azacycloalkyl, bicyclic hydrocarbyl, spirocycloalkyl, azaspirocycloalkyl, oxaspirocycloalkyl, aryl, heteroaryl, and absent; Said alkyl, aryl, and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens; Said azacycloalkyl, bicyclic hydrocarbyl, and spirocycloalkyl are optionally substituted with cyano, fluorophenyl, fluorophenylalkyl, or fluorophenylamino. Alternatively, R¹² is selected from the following structures:

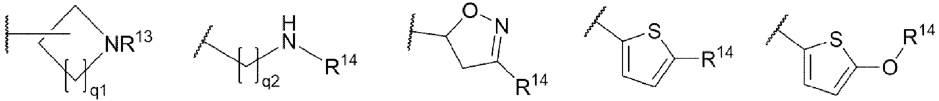

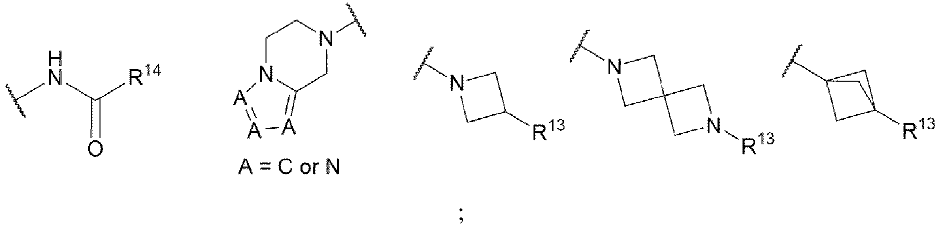

Wherein q¹ and q² are each independently selected from 1, 2, and 3; R¹³ is selected from the group consisting of hydrogen, cyano, cyclic ether, and -L²-R¹⁴, where L² is selected from -SO₂-, -CO-, hydrocarbyl, or absent; R¹⁴ is selected from aryl and heteroaryl, said aryl and heteroaryl are optionally substituted with one to three substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, and boronate ester.

In certain embodiments, the aryl is phenyl or naphthyl; The heteroaryl is selected from the group consisting of quinolinyl, thiophenyl, thiazolyl, indolinyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, benzoxazolyl, indolinonyl, quinolinonyl, triazolopiperazinyl, imidazopiperazinyl, and benzooxazinonyl.

In certain embodiments, R¹¹ is selected from the group consisting of aryl and heteroaryl; Said aryl and heteroaryl are optionally substituted with one or more substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, boronate ester, cyclic ether, cycloalkyl and alkyl optionally substituted with one to three halogens. Preferably, R¹¹ is selected from cyanophenyl, halogen-substituted benzofuranyl, and cyanobenzofuranyl. More preferably, R¹¹ is selected from 4-cyanophenyl, 5-chlorobenzofuran-2-yl, and 5-cyanobenzofuran-2-yl.

In certain embodiments, R¹² is selected from the group consisting of phenyl, naphthyl, quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, triazolopiperazine, imidazopiperazine, azetidinyl, piperidinyl, azabicyclo[3.3]heptanyl, oxabicyclo[3.3]heptanyl, and bicyclo[1.1.1]pentanyl. These groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens.

In certain embodiments, the alkyl group is selected from methyl, ethyl, propyl, isopropyl, n-butyl, isobutyl and tert-butyl.

In certain embodiments, wherein: n is 0, 1, or 2; m is 1; X is O; L¹ is selected from the group consisting of -SO₂-, -CO-, and -CH₂-; - - - is a single bond; :::::: O indicates that one or more carbon atoms are replaced by carbonyl groups or not. R¹¹ is selected from the group consisting of aryl, heteroaryl, spirocycloalkyl, azaspirocycloalkyl, oxaspirocycloalkyl, bicyclic hydrocarbyl, azacycloalkyl, or azacyclic ketone. These groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, R¹¹ is selected from cyanophenyl, cyano-substituted bicyclic hydrocarbyl, cyano-substituted azaspirocycloalkyl, or cyano-substituted azacycloalkyl; More preferably, R¹¹ is selected from 4-cyanophenyl, 1-cyano-bicyclo[1.1.1]pentyl, 1-cyanopiperazinyl, and 1-cyano-dihydropyridyl. R¹² is selected from the group consisting of alkyl, azacycloalkyl, bicyclic hydrocarbyl, spirocycloalkyl, azaspirocycloalkyl, oxaspirocycloalkyl, aryl, and heteroaryl; Said alkyl, azacycloalkyl, bicyclic hydrocarbyl, spirocycloalkyl, azaspirocycloalkyl, aryl, and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, cyclic ether, sulfuryl fluoride, boronate ester, and alkyl optionally substituted with one to three halogens.

In certain embodiments, the compounds are of formula **II:** 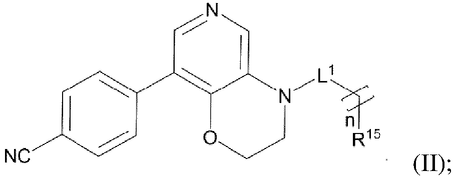
Wherein: n is 0, 1, or 2; L¹ is selected from the group consisting of -SO₂- and -CO-;
R¹⁵ is selected from the group consisting of alkyl, azacycloalkyl, bicyclic hydrocarbyl, spirocycloalkyl, azaspirocycloalkyl, oxaspirocycloalkyl, aryl, and heteroaryl; Said alkyl, azacycloalkyl, bicyclic hydrocarbyl, spirocycloalkyl, azaspirocycloalkyl, oxaspirocycloalkyl, aryl, and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, the alkyl is selected from methyl and ethyl; the aryl is selected from phenyl and naphthyl; the heteroaryl is selected from quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, and thienopyridinyl; the azacycloalkyl is selected from azetidinyl; the bicyclic hydrocarbyl is selected from bicyclo[1.1.1]pentyl and bicyclo[2.2.2]octyl; and the azaspirocycloalkyl is selected from 2,6-diazabicyclo[3.3]heptyl.

In certain embodiments, the compounds are of formula **III:**

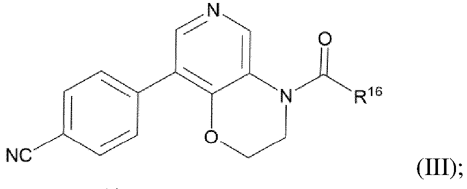

Wherein: R¹⁶ is selected from the following structures:

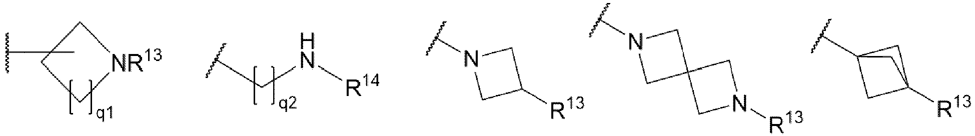

Wherein q¹, q², R¹³ and R¹⁴ are as defined above.

In certain embodiments, wherein: n is 0; m is 1; X is S; L¹ is selected from the group consisting of -SO₂-, -CO-, and -CH₂-; - - - is a single bond; :::::: O indicates that one or more carbon atoms are replaced by carbonyl groups or not; R¹¹ is selected from the group consisting of aryl, heteroaryl, spirocycloalkyl, azaspirocycloalkyl, oxaspirocycloalkyl, bicyclic hydrocarbyl, azacycloalkyl, and azacyclic ketone. Said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens. Preferably, R¹¹ is selected from cyanophenyl, halogen-substituted benzofuranyl, and cyano-substituted benzofuranyl. More preferably, R¹¹ is selected from 5-chlorobenzofuran-2-yl, 4-cyanophenyl, and 5-cyanobenzofuran-2-yl. R¹² is selected from the group consisting of aryl, heteroaryl, azacycloalkyl, bicyclic hydrocarbyl, spirocycloalkyl, azaspirocycloalkyl, and oxaspirocycloalkyl. Said aryl, heteroaryl, azacycloalkyl, bicyclic hydrocarbyl, spirocycloalkyl, azaspirocycloalkyl, and oxaspirocycloalkyl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

In certain embodiments, the compounds are of formula **IV:**

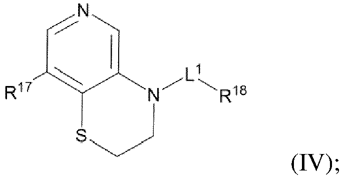

Wherein: L¹ is selected from the group consisting of -SO₂-, -CO- and -CH₂-; R¹⁷ is selected from the group consisting of aryl and heteroaryl. Said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, the aryl is phenyl; the heteroaryl is selected from the group consisting of quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, indolinonyl, and quinolinonyl. More preferably, R¹⁷ is selected from 4-cyanophenyl, 5-chlorobenzofuran-2-yl, and 5-cyanobenzofuran-2-yl. R¹⁸ is selected from the group consisting of aryl, heteroaryl, azacycloalkyl, bicyclic hydrocarbyl, spirocycloalkyl, azaspirocycloalkyl, and oxaspirocycloalkyl. Said aryl, heteroaryl, azacycloalkyl, bicyclic hydrocarbyl, spirocycloalkyl, azaspirocycloalkyl, and oxaspirocycloalkyl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, the aryl group is selected from phenyl and naphthyl; the heteroaryl group is selected from quinolinyl, thiophenyl, pyrazolyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, triazolopiperazinyl, and imidazopiperazinyl; the azacycloalkyl is selected from azetidinyl, the bicyclic hydrocarbyl is selected from bicyclo[1.1.1]pentyl and bicyclo[2.2.2]octyl, and the azaspirocycloalkyl is selected from 2-azabicyclo[3.3]heptyl.

In certain embodiments, the compounds are of formula **XI:**

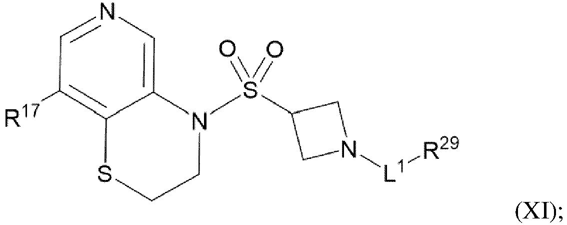

Wherein: L¹ is selected from the group consisting of -SO₂-, -CO-, -CH₂- or absent; R¹⁷ is as defined above, and R²⁹ is selected from the group consisting of cyano, cyclic ether, or phenyl optionally substituted with one to three substituents selected from halogen, cyano, and alkoxy.

In certain embodiments, the compounds are of formula **XII:**

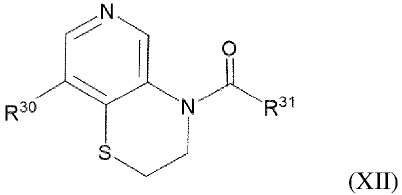

Wherein: R³⁰ is selected from aryl and heteroaryl. Said aryl and heteroaryl are optionally substituted with one or two substituents selected from halogen, cyano, and alkyl. Preferably, the aryl group is phenyl; and the heteroaryl group is selected from pyridyl, indazolyl, benzothiazolyl, indolyl, benzofuranyl, benzothienyl, benzimidazolyl, benzopyrazolyl, indolinonyl, imidazopyridyl, pyrazolopyridyl, quinolinonyl, and pyrrolopyridyl. R³¹ is selected from phenyl and pyridyl. The phenyl group is optionally substituted with one to three substituents selected from halogen, cyano, alkoxy, nitro, amino, hydroxyl, acetylamino, cycloalkyl and alkyl optionally substituted with one to three halogens.

In certain embodiments, the compounds are of formula **XIII:**

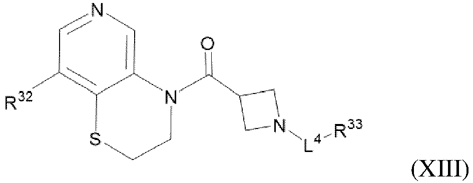

Wherein: R³² is selected from phenyl and quinolinone; Said phenyl and quinolinone are optionally substituted with one or two substituents selected from halogen, cyano, and alkyl. L⁴ is selected from -SO₂- and -CH₂-. R³³ is selected from aryl and heteroaryl. These groups are optionally substituted with one or more substituents selected from cyano, nitro, and hydroxyl. Preferably, the heteroaryl group is selected from thienyl and pyridyl.

In certain embodiments, the compounds are of formula **XIV:** 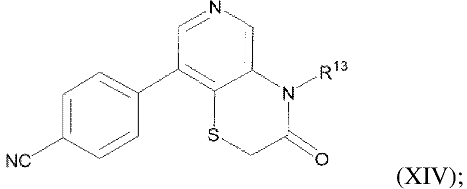 wherein: R¹³ is as defined above.

In certain embodiments, wherein: n is 0, 1, or 2; m is 0; X is C; L¹ is selected from the group consisting of -SO₂-, -CO-, and -CH₂-; - - - is a single bond; R¹¹ is selected from the group consisting of aryl, heteroaryl, bicyclic hydrocarbyl, azacycloalkyl, azaspirocycloalkyl, and azacyclic ketone. Said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, R¹¹ is selected from cyanophenyl, 1-cyanopiperidinyl, and 1(2H)-cyano-3,6-dihydropyridinyl. More preferably, R¹¹ is 4-cyanophenyl. R¹² is selected from the group consisting of alkyl, aryl, and heteroaryl. These groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

In certain embodiments, the compounds are of formula **V:**

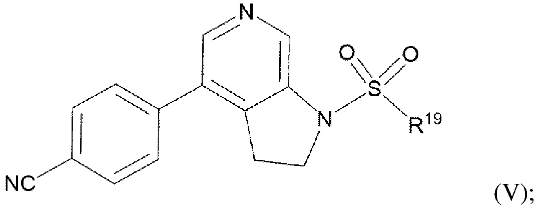

Wherein: R¹⁹ is selected from aryl and heteroaryl. These groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, the aryl is selected from phenyl and naphthyl, and the heteroaryl is selected from quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, and thienopyridinyl.

In certain embodiments, the compounds are of formula **VI:**

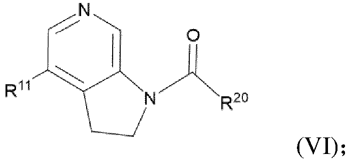

Wherein: R¹¹ is as defined above; R²⁰ is selected from alkyl, aryl, and heteroaryl. These groups are optionally substituted with one or more substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, boronate ester, cycloalkyl and alkyl optionally substituted with one to three halogens. Preferably, the aryl group is phenyl, and the heteroaryl group is selected from quinolinyl, thienyl, pyridyl, furanyl, indazolyl, imidazopyridyl, benzothiazolyl, indolyl, benzofuranyl, benzothienyl, pyrrolopyridinyl, pyrrolopyrimidinyl, pyrrolidinopyridinyl, imidazopyrimidinyl, imidazopiperidinyl, furopyridinyl, and thienopyridinyl.

In certain embodiments, the compounds are of formula **VII:** 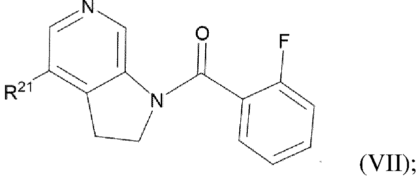 wherein: R²¹ is selected from aryl and heteroaryl; Said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

In certain embodiments, the compounds are of formula **VIII:** 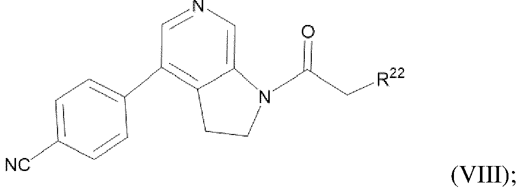 wherein: R²² is aryl; Said aryl is optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, the aryl is phenyl.

In certain embodiments, wherein: n is 0; m is 0; X is C; L¹ is selected from the group consisting of -SO₂- and -CO-; - - - is a double bond; R¹¹ is selected from the group consisting of aryl and heteroaryl. Said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens. Preferably, R¹¹ is selected from cyanophenyl. More preferably, R¹¹ is 4-cyanophenyl. R¹² is selected from the group consisting of aryl and heteroaryl. Said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

In certain embodiments, the compounds are of formula **IX:**

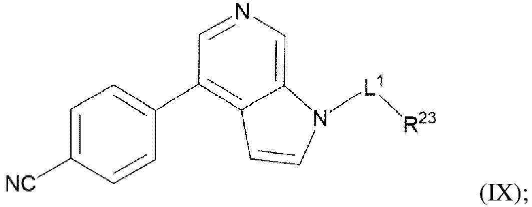

Wherein: L¹ is selected from the group consisting of -SO₂- and -CO-; R²³ is selected from aryl and heteroaryl. These groups are optionally substituted with one or more substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, boronate ester, cycloalkyl and alkyl optionally substituted with one to three halogens.

In certain embodiments, the compounds are of formula **X:**

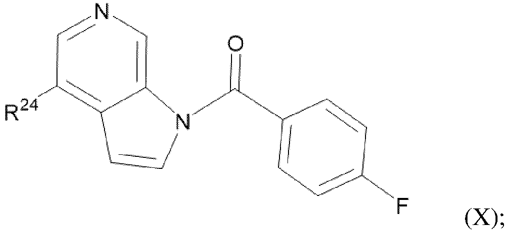

Wherein: R²⁴ is selected from aryl and heteroaryl; Said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

In certain embodiments, the compounds are of formula **XV:**

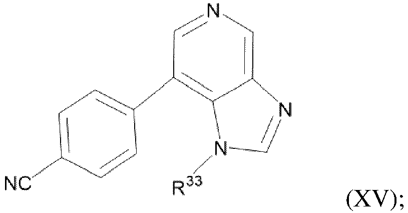

Wherein: R³³ is selected from hydrocarbyl and cyclohydrocarbyl.

In certain embodiments, the compounds are of formula **XVI:** 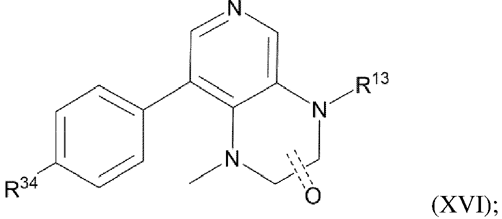
Wherein: R³⁴ is selected from cyano and halogen;
R¹³ is as defined above; :::::: O means that one or more carbon atoms are replaced by carbonyl groups or not.

In certain embodiments, the compounds are selected from the group consisting of:
4-(4-(phenylsulfonyl)-3,4-dihydro-2*H*-pyrido[4,3-b][1,4]oxazin-8-yl)benzonitrile (O1);
4-(4-((4-fluorophenyl)sulfonyl)-3,4-dihydro-2*H*-pyrido[4,3-b][1,4]oxazin-8-yl)benzonitrile (O2);
4-(4-((3-fluorophenyl)sulfonyl)-3,4-dihydro-2*H*-pyrido[4,3-b][1,4]oxazin-8-yl)benzonitrile (O3);
4-(4-tosyl-3,4-dihydro-2*H*-pyrido[4,3-b][1,4]oxazin-8-yl)benzonitrile (O4);
4-(4-(m-tolylsulfonyl)-3,4-dihydro-2*H*-pyrido[4,3-b][1,4]oxazin-8-yl)benzonitrile (O5);
4-(4-((4-chlorophenyl)sulfonyl)-3,4-dihydro-2*H*-pyrido[4,3-b][1,4]oxazin-8-yl)benzonitrile (O6);
4-(4-((3-chlorophenyl)sulfonyl)-3,4-dihydro-2*H*-pyrido[4,3-b][1,4]oxazin-8-yl)benzonitrile (O7);
4-(4-((4-methoxyphenyl)sulfonyl)-3,4-dihydro-2*H*-pyrido[4,3-b][1,4]oxazin-8-yl)benzonitrile (O8);
4-(4-((4-nitrophenyl)sulfonyl)-3,4-dihydro-2*H*-pyrido[4,3-b][1,4]oxazin-8-yl)benzonitrile (O9);
4-(4-((3-nitrophenyl)sulfonyl)-3,4-dihydro-2*H*-pyrido[4,3-b][1,4]oxazin-8-yl)benzonitrile (O10);
4-(4-((4-(trifluoromethyl)phenyl)sulfonyl)-3,4-dihydro-2*H*-pyrido[4,3-b][1,4]oxazin-8-yl)-benzonitrile (O11);
4-(4-((3-(trifluoromethyl)phenyl)sulfonyl)-3,4-dihydro-2*H*-pyrido[4,3-b][1,4]oxazin-8-yl)-benzonitrile (012);
4-(4-(pyridin-3-ylsulfonyl)-3,4-dihydro-2*H*-pyrido[4,3-b][1,4]oxazin-8-yl)benzonitrile (013);
4-(4-(methylsulfonyl)-3,4-dihydro-2*H*-pyrido[4,3-b][1,4]oxazin-8-yl)benzonitrile (014);
4-(4-(quinolin-8-ylsulfonyl)-3,4-dihydro-2*H*-pyrido[4,3-b][1,4]oxazin-8-yl)benzonitrile (O15);
4-(4-(thiophen-2-ylsulfonyl)-3,4-dihydro-2*H*-pyrido[4,3-b][1,4]oxazin-8-yl)benzonitrile(O16);
4-(4-(naphthalen-1-ylsulfonyl)-3,4-dihydro-2*H*-pyrido[4,3-b][1,4]oxazin-8-yl)benzonitrile (017);
4-(4-(naphthalen-2-ylsulfonyl)-3,4-dihydro-2*H*-pyrido[4,3-b][1,4]oxazin-8-yl)benzonitrile (018);
4-(4-(4-fluorobenzoyl)-3,4-dihydro-2*H*-pyrido[4,3-b][1,4]oxazin-8-yl)benzonitrile (019);
4-(4-(3-fluorobenzoyl)-3,4-dihydro-2*H*-pyrido[4,3-b][1,4]oxazin-8-yl)benzonitrile (O20);
4-(4-(2-fluorobenzoyl)-3,4-dihydro-2*H*-pyrido[4,3-b][1,4]oxazin-8-yl)benzonitrile (O21);
4-(4-(4-methoxybenzoyl)-3,4-dihydro-2*H-*pyrido[4,3-b][1,4]oxazin-8-yl)benzonitrile (O22);
4-(4-(3-methoxybenzoyl)-3,4-dihydro-2*H*-pyrido[4,3-b][1,4]oxazin-8-yl)benzonitrile (O23);
4-(4-(4-(trifluoromethyl)benzoyl)-3,4-dihydro-2*H*-pyrido[4,3-b][1,4]oxazin-8-yl)benzonitrile (O24);
4-(4-(3-(trifluoromethyl)benzoyl)-3,4-dihydro-2*H*-pyrido[4,3-b][1,4]oxazin-8-yl)benzonitrile (O25);
4-(4-(4-nitrobenzoyl)-3,4-dihydro-2*H*-pyrido[4,3-b][1,4]oxazin-8-yl)benzonitrile (O26);
4-(4-(3-nitrobenzoyl)-3,4-dihydro-2*H*-pyrido[4,3-b][1,4]oxazin-8-yl)benzonitrile (O27);
4-(4-(4-chlorobenzoyl)-3,4-dihydro-2*H*-pyrido[4,3-b][1,4]oxazin-8-yl)benzonitrile (O28);
4-(4-(3-chlorobenzoyl)-3,4-dihydro-2*H*-pyrido[4,3-b][1,4]oxazin-8-yl)benzonitrile (O29);
4-(4-nicotinoyl -3,4-dihydro-2*H*-pyrido[4,3-b][1,4]oxazin-8-yl)benzonitrile (O30);
4-(4-(furan-2-carbonyl)-3,4-dihydro-2*H*-pyrido[4,3-b][1,4]oxazin-8-yl)benzonitrile (O31);
4-(4-(2-cyanoacetyl)-3,4-dihydro-2*H*-pyrido[4,3-b][1,4]oxazin-8-yl)benzonitrile (O32);
4-(4-(2-chloroacetyl)-3,4-dihydro-2*H*-pyrido[4,3-b][1,4]oxazin-8-yl)benzonitrile (O33);
4-(4-(2-bromoacetyl)-3,4-dihydro-2*H*-pyrido[4,3-b][1,4]oxazin-8-yl)benzonitrile (O34);
4-(4-(2,2,2-trifluoroacetyl)-3,4-dihydro-2*H*-pyrido[4,3-b][1,4]oxazin-8-yl)benzonitrile (O35);
4-(4-(2-(4-fluorophenyl)acetyl)-3,4-dihydro-2*H*-pyrido[4,3-b][1,4]oxazin-8-yl)benzonitrile (O36);
4-(4-(2-(3-fluorophenyl)acetyl)-3,4-dihydro-2*H*-pyrido[4,3-b][1,4]oxazin-8-yl)benzonitrile (O37);
4-(4-(2-(4-chlorophenyl)acetyl)-3,4-dihydro-2*H*-pyrido[4,3-b][1,4]oxazin-8-yl)benzonitrile (O38);
4-(4-(2-(3-chlorophenyl)acetyl)-3,4-dihydro-2*H*-pyrido[4,3-b][1,4]oxazin-8-yl)benzonitrile (O39);
4-(4-(2-(4-methoxyphenyl)acetyl)-3,4-dihydro-2*H*-pyrido[4,3-b][1,4]oxazin-8-yl)benzonitrile (O40);
4-(4-(2-(3-methoxyphenyl)acetyl)-3,4-dihydro-2*H*-pyrido[4,3-b][1,4]oxazin-8-yl)benzonitrile (O41);
4-(4-(2-(p-tolyl)acetyl)-3,4-dihydro-2*H*-pyrido[4,3-b][1,4]oxazin-8-yl)benzonitrile (O42);
4-(4-(2-(m-tolyl)acetyl)-3,4-dihydro-2*H*-pyrido[4,3-b][1,4]oxazin-8-yl)benzonitrile (O43);
4-(4-(2-(o-tolyl)acetyl)-3,4-dihydro-2*H*-pyrido[4,3-b][1,4]oxazin-8-yl)benzonitrile (O44);
4-(4-(2-(4-(trifluoromethyl)phenyl)acetyl)-3,4-dihydro-2H-pyrido[4,3-b][1,4]oxazin-8-yl)-benzonitrile (O45);
4-(4-(2-(3-(trifluoromethyl)phenyl)acetyl)-3,4-dihydro-2H-pyrido[4,3-b][1,4]oxazin-8-yl)-benzonitrile (O46);
4-(4-(3-(4-fluorophenyl)propanoyl)-3,4-dihydro-2*H*-pyrido[4,3-b][1,4]oxazin-8-yl)benzonitrile (O47);
4-(4-(3-(3-fluorophenyl)propanoyl)-3,4-dihydro-2*H*-pyrido[4,3-b][1,4]oxazin-8-yl)benzonitrile (O48);
4-(4-(3-(4-chlorophenyl)propanoyl)-3,4-dihydro-2*H*-pyrido[4,3-b] [1,4]oxazin-8-yl)benzonitrile (O49);
4-(4-(3-(3-chlorophenyl)propanoyl)-3,4-dihydro-2*H*-pyrido[4,3-b][1,4]oxazin-8-yl)benzonitrile (O50);
4-(4-(3-(4-methoxyphenyl)propanoyl)-3,4-dihydro-2*H*-pyrido[4,3-b][1,4]oxazin-8-yl)benzonitrile (O51);
4-(4-(3-(3-methoxyphenyl)propanoyl)-3,4-dihydro-2*H*-pyrido[4,3-b][1,4]oxazin-8-yl)benzonitrile (O52);
4-(4-(3-(4-(trifluoromethyl)phenyl)propanoyl)-3,4-dihydro-2*H*-pyrido[4,3-b][1,4]oxazin-8-yl)-benzonitrile (O53);
4-(4-(3-(3-(trifluoromethyl)phenyl)-3,4-dihydro-2*H*-pyrido[4,3-b][1,4]oxazin-8-yl)benzonitrile (O54);
4-(4-(1-((3-fluorophenyl)sulfonyl)piperidine-4-carbonyl)-3,4-dihydro-2*H*-pyrido[4,3-b][1,4]oxazin-8-yl)benzonitrile (O55);
4-(4-(azetidine-3-carbonyl)-3,4-dihydro-2*H*-pyrido[4,3-b][1,4]oxazin-8-yl)benzonitrile (O56);
4-(4-(1-(4-fluorobenzoyl)azetidine-3-carbonyl)-3,4-dihydro-2*H*-pyrido[4,3-b][1,4]oxazin-8-yl)-benzonitrile (O57);
4-(4-(1-(3-fluorobenzoyl)azetidine-3-carbonyl)-3,4-dihydro-2*H*-pyrido[4,3-b][1,4]oxazin-8-yl)-benzonitrile(O58);
N-(2-(8-(4-cyanophenyl)-2,3-dihydro-4*H*-pyrido[4,3-b][1,4]oxazin-4-yl)-2-oxoethyl)-4-fluoro-benzamide (O59);
4-(4-(1-((4-fluorophenyl)sulfonyl)azetidine-3-carbonyl)-3,4-dihydro-2*H*-pyrido[4,3-*b*][1,4]oxazin-8 -yl)benzonitrile (O60);
4-(4-(1-((3-fluorophenyl)sulfonyl)azetidine-3-carbonyl)-3,4-dihydro-2*H*-pyrido[4,3-b][1,4]oxazin-8 -yl)benzonitrile (061);
4-(4-(1-((4-nitrophenyl)sulfonyl)azetidine-3-carbonyl)-3,4-dihydro-2*H*-pyrido[4,3-*b*][1,4]oxazin-8-yl)benzonitrile (O62);
4-(4-(1-((3-nitrophenyl)sulfonyl)azetidine-3-carbonyl)-3,4-dihydro-2*H*-pyrido[4,3-b][1,4]oxazin-8-yl)benzonitrile (O63);
4-(4-(1-(thiophen-2-ylsulfonyl)azetidine-3-carbonyl)-3,4-dihydro-2H-pyrido[4,3-b][1,4]oxazin-8-yl )benzonitrile (O64);
4-(4-(1-tosylazetidine-3-carbonyl)-3,4-dihydro-2*H*-pyrido[4,3-b][1,4]oxazin-8-yl)benzonitrile (O65);
4-(4-(1-((3-methoxyphenyl)sulfonyl)azetidine-3-carbonyl)-3,4-dihydro-2*H*-pyrido[4,3-b][1,4]-oxazin-8-yl)benzonitrile (O66);
4-(4-(1-((4-methoxyphenyl)sulfonyl)azetidine-3-carbonyl)-3,4-dihydro-2*H*-pyrido[4,3-b][1,4]-oxazin-8-yl)benzonitrile (O67);
3-((3-(8-(4-cyanophenyl)-3,4-dihydro-2*H*-pyrido[4,3-b][1,4]oxazine-4-carbonyl)azetidin-1-yl)-sulfonyl)benzonitrile (O68);
4-((3-(8-(4-cyanophenyl)-3,4-dihydro-2*H*-pyrido[4,3-b][1,4]oxazine-4-carbonyl)azetidin-1-yl)-sulfonyl)benzonitrile (O69);
4-(4-(1-(m-tolylsulfonyl)azetidine-3-carbonyl)-3,4-dihydro-2*H*-pyrido[4,3-b][1,4]oxazin-8-yl)-benzonitrile (O70);
4-(4-(3-((3-fluorophenyl)amino)bicyclo[1.1.1]pentane-1-carbonyl)-3,4-dihydro-2H-pyrido[4,3-b] [1, 4]oxazin-8-yl)benzonitrile (O71);
4-(4-(5-(4-fluorophenoxy)thiophene-2-carbonyl)-3,4-dihydro-2*H*-pyrido[4,3-b][1,4]oxazin-8-yl)-benzonitrile (O72);
4-(4-(6-(3-fluorobenzyl)-2,6-diazaspiro[3.3]heptane-2-carbonyl)-3,4-dihydro-2H-pyrido[4,3-b] [1,4] oxazin-8-yl)benzonitrile (O73);
3-((3-(8-(4-cyanophenyl)-3,4-dihydro-2*H*-pyrido[4,3-b][1,4]oxazine-4-carbonyl)azetidin-1-yl)-sulfonyl)benzonitrile (O74);
4-((3-(8-(4-cyanophenyl)-3,4-dihydro-2*H*-pyrido[4,3-b][1,4]oxazine-4-carbonyl)azetidin-1-yl)-sulfonyl)benzonitrile (O75);
4-(4-(1-(3-methoxybenzoyl)azetidine-3-carbonyl)-3,4-dihydro-2*H*-pyrido[4,3-b][1,4]oxazin-8-yl)-benzonitrile (O76);
4-(4-(1-(4-methoxybenzoyl)azetidine-3-carbonyl)-3,4-dihydro-2*H*-pyrido[4,3-b][1,4]oxazin-8-yl)-benzonitrile (O77);
4-(4-(1-(3-fluorobenzyl)azetidine-3-carbonyl)-3,4-dihydro-2*H*-pyrido[4,3-b][1,4]oxazin-8-yl)-benzonitrile (O78);
4-(4-(1-(4-fluorobenzyl)azetidine-3-carbonyl)-3,4-dihydro-2*H*-pyrido[4,3-b][1,4]oxazin-8-yl)-benzonitrile (O79);
3-((3-(8-(4-cyanophenyl)-3,4-dihydro-2*H*-pyrido[4,3-b][1,4]oxazine-4-carbonyl)azetidin-1-yl)-methyl)benzonitrile (O80);
4-(4-(1-(4-cyanobenzyl)azetidine-3-carbonyl)-3,4-dihydro-2*H*-pyrido[4,3-b][1,4]oxazin-8-yl)-benzonitrile (O81);
4-(4-(1-(3-methoxybenzyl)azetidine-3-carbonyl)-3,4-dihydro-2*H*-pyrido[4,3-b][1,4]oxazin-8-yl)-benzonitrile (O82);
4-(4-(1-(4-methoxybenzyl)azetidine-3-carbonyl)-3,4-dihydro-2*H*-pyrido[4,3-b][1,4]oxazin-8-yl)-benzonitrile (O83);
4-(4-(1-(3-fluorophenyl)azetidine-3-carbonyl)-3,4-dihydro-2*H*-pyrido[4,3-b][1,4]oxazin-8-yl)-benzonitrile (O84);
4-(4-(1-(4-fluorophenyl)azetidine-3-carbonyl)-3,4-dihydro-2*H*-pyrido[4,3-b][1,4]oxazin-8-yl)-benzonitrile (O85);
3-(3-(8-(4-cyanophenyl)-3,4-dihydro-2*H*-pyrido[4,3-b][1,4]oxazine-4-carbonyl)azetidin-1-yl)-benzonitrile (O86);
4-(3-(8-(4-cyanophenyl)-3,4-dihydro-2*H*-pyrido[4,3-b][1,4]oxazine-4-carbonyl)azetidin-1-yl)-benzonitrile (O87);
(8-(benzo[*d*]thiazol-6-yl)-2,3-dihydro-4*H*-pyrido[4,3-b][1,4]oxazin-4-yl)(1-(3-fluorobenzyl)-azetidi n-3-yl)methanone (O88);
5-(4-(1-(3-fluorobenzyl)azetidine-3-carbonyl)-3,4-dihydro-2*H*-pyrido[4,3-b][1,4]oxazin-8-yl)-indolin-2-one (O89);
(1-(3-fluorobenzyl)azetidin-3-yl)(8-(imidazo[1,2-*a*]pyridin-6-yl)-2,3-dihydro-4*H*-pyrido[4,3-b][1,4] oxazin-4-yl)methanone (O90);
(8-(6-chloro-1*H*-indol-2-yl)-2,3-dihydro-4*H*-pyrido[4,3-b][1,4]oxazin-4-yl)(1-(3-fluorobenzyl)-azetidin-3-yl)methanone (O91);
2-(4-(1-(3-fluorobenzyl)azetidine-3-carbonyl)-3,4-dihydro-2*H*-pyrido[4,3-b][1,4]oxazin-8-yl)-1*H-*indole-6-carbonitrile (O92);
(8-(6-fluoro-1*H*-indol-2-yl)-2,3-dihydro-4*H*-pyrido[4,3-b][1,4]oxazin-4-yl)(1-(3-fluorobenzyl)-azetidin-3-yl)methanone (O93);
2-(4-(1-(3-fluorobenzyl)azetidine-3-carbonyl)-3,4-dihydro-2*H*-pyrido[4,3-b][1,4]oxazin-8-yl)-1*H-*indole-5-carbonitrile (O94);
(8-(5-chlorobenzofuran-2-yl)-2,3-dihydro-4*H*-pyrido[4,3-b][1,4]oxazin-4-yl)(1-(3-fluorobenzyl)-azetidin-3-yl)methanone (O95);
2-(4-(1-(3-fluorobenzyl)azetidine-3-carbonyl)-3,4-dihydro-2*H*-pyrido[4,3-b][1,4]oxazin-8-yl)-benzofuran-5-carbonitrile (O96);
4-(4-(1-(3-fluorobenzyl)azetidine-3-carbonyl)-3,4-dihydro-2H-pyrido[4,3-b][1,4]oxazin-8-yl)-3,6-dihydropyridine-1(2H)-carbonitrile (O97);
4-(4-(1-(3-fluorobenzyl)azetidine-3-carbonyl)-3,4-dihydro-2H-pyrido[4,3-b][1,4]oxazin-8-yl)-piperazine-1-carbonitrile (O98);
4-(4-(1-(3-fluorobenzyl)azetidine-3-carbonyl)-3,4-dihydro-2H-pyrido[4,3-b][1,4]oxazin-8-yl)-3-oxopiperazine-1-carbonitrile (O99);
4-(4-((3-(3-methoxyphenyl)-4,5-dihydroisoxazol-5-yl)sulfonyl)-3,4-dihydro-2H-pyrido[4,3-b] [1,4] oxazin-8-yl)benzonitrile (O100);
3-(5-((8-(4-cyanophenyl)-2,3-dihydro-4*H*-pyrido[4,3-b][1,4]oxazin-4-yl)sulfonyl)-4,5-dihydro-isoxazol-3-yl)benzonitrile (O101);
4-(4-((3-phenyl-4,5-dihydroisoxazol-5-yl)sulfonyl)-3,4-dihydro-2*H*-pyrido[4,3-b][1,4]oxazin-8-yl)-benzonitrile (0102);
4-(4-((3-benzyl-4,5-dihydroisoxazol-5-yl)sulfonyl)-3,4-dihydro-2*H*-pyrido[4,3-b][1,4]oxazin-8-yl)-benzonitrile (0103);
(S)-4-(4-(1-(3-fluorobenzyl)pyrrolidine-3-carbonyl)-3,4-dihydro-2*H*-pyrido[4,3-b][1,4]oxazin-8-yl) benzonitrile (0104);
(R)-4-(4-(1-(3-fluorobenzyl)pyrrolidine-3-carbonyl)-3,4-dihydro-2*H*-pyrido[4,3-b][1,4]oxazin-8-yl) benzonitrile (0105);
2-(4-((S)-1-(3-fluorobenzyl)pyrrolidine-3-carbonyl)-3,4-dihydro-2*H*-pyrido[4,3-b][1,4]oxazin-8-yl) -2,3-dihydrobenzofuran-5-carbonitrile (0106);
2-(4-((R)-1-(3-fluorobenzyl)pyrrolidine-3-carbonyl)-3,4-dihydro-2*H*-pyrido[4,3-b][1,4]oxazin-8-yl) -2,3-dihydrobenzofuran-5-carbonitrile (0107);
8-(5-chlorobenzofuran-2-yl)-3,4-dihydro-2H-pyrido[4,3-b][1,4]oxazine (0108);
2-(3,4-dihydro-2H-pyrido[4,3-b][1,4]oxazin-8-yl)benzofuran-5-carbonitrile (0109);
4-(4-((1-(3-cyanobenzoyl)azetidin-3-yl)sulfonyl)-3,4-dihydro-2*H*-pyrido[4,3-b] [1,4]oxazin-8-yl)-benzonitrile (O110);
4-(4-((1-(4-cyanobenzoyl)azetidin-3-yl)sulfonyl)-3,4-dihydro-2*H*-pyrido[4,3-b] [1,4]oxazin-8-yl)-b enzonitrile (O111);
4-(4-((1-(3-methoxybenzoyl)azetidin-3-yl)sulfonyl)-3,4-dihydro-2*H*-pyrido[4,3-b][1,4]oxazin-8-yl) benzonitrile (0112);
4-(4-((1-(4-methoxybenzoyl)azetidin-3-yl)sulfonyl)-3,4-dihydro-2*H*-pyrido[4,3-b][1,4]oxazin-8-yl) benzonitrile (0113);
4-((3-((8-(4-cyanophenyl)-2,3-dihydro-4*H*-pyrido[4,3-b] [1,4]oxazin-4-yl)sulfonyl)azetidin-1-yl)-sulfonyl)benzonitrile (0114);
3-((3-((8-(4-cyanophenyl)-2,3-dihydro-4*H*-pyrido[4,3-b][1,4]oxazin-4-yl)sulfonyl)azetidin-1-yl)-sulfonyl)benzonitrile (0115);
4-(4-((1-((4-fluorophenyl)sulfonyl)azetidin-3-yl)sulfonyl)-3,4-dihydro-2*H*-pyrido[4,3-b][1,4]-oxazin-8-yl)benzonitrile (0116);
4-(4-((1-((3-fluorophenyl)sulfonyl)azetidin-3-yl)sulfonyl)-3,4-dihydro-2*H*-pyrido[4,3-b][1,4]-oxazin-8-yl)benzonitrile (0117);
4-(4-((1-((4-methoxyphenyl)sulfonyl)azetidin-3-yl)sulfonyl)-3,4-dihydro-2*H*-pyrido[4,3-b][1,4]-oxazin-8-yl)benzonitrile (0118);
4-(4-((1-((3-methoxyphenyl)sulfonyl)azetidin-3-yl)sulfonyl)-3,4-dihydro-2*H*-pyrido[4,3-b][1,4]-oxazin-8-yl)benzonitrile (0119);
3-((3-((8-(4-cyanophenyl)-2,3-dihydro-4*H*-pyrido[4,3-b][1,4]oxazin-4-yl)sulfonyl)azetidin-1-yl)-methyl)benzonitrile (0120);
4-(4-((1-(4-cyanobenzyl)azetidin-3-yl)sulfonyl)-3,4-dihydro-2*H*-pyrido 4,3-b][1,4]oxazin-8-yl)-benzonitrile (0121);
4-(4-((1-(3-fluorobenzyl)azetidin-3-yl)sulfonyl)-3,4-dihydro-2*H*-pyrido[4,3-b][1,4]oxazin-8-yl)-benzonitrile (0122);
4-(4-((1-(4-fluorobenzyl)azetidin-3-yl)sulfonyl)-3,4-dihydro-2*H*-pyrido[4,3-b][1,4]oxazin-8-yl)-benzonitrile (0123);
4-(4-((1-(3-methoxybenzyl)azetidin-3-yl)sulfonyl)-3,4-dihydro-2*H*-pyrido[4,3-b] [1,4]oxazin-8-yl)-benzonitrile (0124);
4-(4-((1-(4-methoxybenzyl)azetidin-3-yl)sulfonyl)-3,4-dihydro-2*H*-pyrido[4,3-b] [1,4]oxazin-8-yl)-benzonitrile (0125);
4-(4-((1-(3-fluorophenyl)azetidin-3-yl)sulfonyl)-3,4-dihydro-2*H*-pyrido[4,3-b][1,4]oxazin-8-yl)-benzonitrile (0126);
4-(4-((1-(4-fluorophenyl)azetidin-3-yl)sulfonyl)-3,4-dihydro-2*H*-pyrido[4,3-b][1,4]oxazin-8-yl)-benzonitrile (0127);
4-((8-(5-chlorobenzofuran-2-yl)-2,3-dihydro-4*H*-pyrido[4,3-b][1,4]oxazin-4-yl)sulfonyl)-benzonitrile (0128);
2-(4-((4-cyanophenyl)sulfonyl)-3,4-dihydro-2*H*-pyrido[4,3-b] [1,4]oxazin-8-yl)benzofuran-5-carbonitril (0129);
4-((8-(4-cyanophenyl)-2,3-dihydro-4*H*-pyrido[4,3-b][1,4]oxazin-4-yl)sulfonyl)benzonitrile (0130);
4-((8-(benzo[d]oxazol-6-yl)-2,3-dihydro-4*H*-pyrido[4,3-b][1,4]oxazin-4-yl)sulfonyl)benzonitrile (0131);
4-((8-(2-oxoindolin-6-yl)-2,3-dihydro-4*H*-pyrido[4,3-b][1,4]oxazin-4-yl)sulfonyl)benzonitrile (0132);
3-(4-(1-(3-Fluorobenzyl)azetidine-3-carbonyl)-3,4-dihydro-2H-pyrido[4,3-b][1,4]oxazin-8-yl)-bicyclo[1.1.1]pentane-1-carbonitrile (0133);
6-(4-(1-(3-Fluorobenzyl)azetidine-3-carbonyl)-3,4-dihydro-2H-pyrido[4,3-b][1,4]oxazin-8-yl)-2,6-diazaspiro[3.3]heptane-2-carbonitrile (0134);
(8-(5,6-Dihydro-[1,2,4]triazolo[4,3-a]pyrazin-7(8H)-yl)-2,3-dihydro-4H-pyrido[4,3-b][1,4]oxazin-4 -yl)(1-(3-fluorobenzyl)azetidin-3-yl)methanone (0135);
4-(4-(3-(3-fluorobenzyl)azetidine-1-carbonyl)-3,4-dihydro-2H-pyrido[4,3-b] [1,4]oxazin-8-yl)-benzonitrile (0136);
(8-(5,6-dihydroimidazo[1,2-a]pyrazin-7(8H)-yl)-2,3-dihydro-4H-pyrido[4,3-b][1,4]oxazin-4-yl)(1-( 3-fluorobenzyl)azetidin-3-yl)methanone (0137);
4-(4-(3-fluorobenzyl)-3,4-dihydro-2*H*-pyrido[4,3-b][1,4]thiazin-8-yl)benzonitrile (S1);
4-(4-(4-fluorobenzyl)-3,4-dihydro-2*H*-pyrido[4,3-b][1,4]thiazin-8-yl)benzonitrile (S2);
4-(4-(3-nitrobenzyl)-3,4-dihydro-2*H*-pyrido[4,3-b][1,4]thiazin-8-yl)benzonitrile (S3);
4-(4-(3-fluorobenzoyl)-3,4-dihydro-2*H*-pyrido[4,3-b][1,4]thiazin-8-yl)benzonitrile (S4);
4-(4-(4-fluorobenzoyl)-3,4-dihydro-2*H*-pyrido[4,3-b][1,4]thiazin-8-yl)benzonitrile (S5);
4-(4-(4-methoxybenzoyl)-3,4-dihydro-2*H*-pyrido[4,3-b][1,4]thiazin-8-yl)benzonitrile (S6);
4-(4-nicotinoyl-3,4-dihydro-2*H*-pyrido[4,3-b][1,4]thiazin-8-yl)benzonitrile (S7);
4-(4-(phenylsulfonyl)-3,4-dihydro-2*H*-pyrido[4,3-b][1,4]thiazin-8-yl)benzonitrile (S8);
4-(4-((3-fluorophenyl)sulfonyl)-3,4-dihydro-2*H*-pyrido[4,3-b][1,4]thiazin-8-yl)benzonitrile (S9);
4-(4-((4-fluorophenyl)sulfonyl)-3,4-dihydro-2*H*-pyrido[4,3-b][1,4]thiazin-8-yl)benzonitrile (S10);
4-(4-(m-tolylsulfonyl)-3,4-dihydro-2*H*-pyrido[4,3-b][1,4]thiazin-8-yl)benzonitrile (S11);
4-(4-tosyl-3,4-dihydro-2*H*-pyrido[4,3-b][1,4]thiazin-8-yl)benzonitrile (S12);
4-(4-((4-methoxyphenyl)sulfonyl)-3,4-dihydro-2*H*-pyrido[4,3-b][1,4]thiazin-8-yl)benzonitrile (S13);
4-(4-((3-chlorophenyl)sulfonyl)-3,4-dihydro-2*H*-pyrido[4,3-b][1,4]thiazin-8-yl)benzonitrile (S14);
4-(4-((4-chlorophenyl)sulfonyl)-3,4-dihydro-2*H*-pyrido[4,3-b][1,4]thiazin-8-yl)benzonitrile (S15);
4-(4-((3-(trifluoromethyl)phenyl)sulfonyl)-3,4-dihydro-2*H*-pyrido[4,3-b][1,4]thiazin-8-yl) benzonitrile (S16);
4-(4-((4-(trifluoromethyl)phenyl)sulfonyl)-3,4-dihydro-2*H*-pyrido[4,3-b] [1,4]thiazin-8-yl)-benzonitrile (S17);
4-(4-((3-nitrophenyl)sulfonyl)-3,4-dihydro-2*H*-pyrido[4,3-b][1,4]thiazin-8-yl)benzonitrile (S18);
4-(4-((4-nitrophenyl)sulfonyl)-3,4-dihydro-2*H*-pyrido[4,3-b][1,4]thiazin-8-yl)benzonitrile (S19);
3-((8-(4-cyanophenyl)-2,3-dihydro-4*H*-pyrido[4,3-b][1,4]thiazin-4-yl)sulfonyl)benzonitrile (S20);
4-((8-(4-cyanophenyl)-2,3-dihydro-4*H*-pyrido[4,3-b][1,4]thiazin-4-yl)sulfonyl)benzonitrile (S21);
4-(4-(naphthalen-1-ylsulfonyl)-3,4-dihydro-2*H*-pyrido[4,3-b][1,4]thiazin-8-yl)benzonitrile (S22);
4-(4-(naphthalen-2-ylsulfonyl)-3,4-dihydro-2*H*-pyrido[4,3-b][1,4]thiazin-8-yl)benzonitrile (S23);
4-(4-(thiophen-2-ylsulfonyl)-3,4-dihydro-2*H*-pyrido[4,3-b][1,4]thiazin-8-yl)benzonitrile (S24);
4-(4-(pyridin-3-ylsulfonyl)-3,4-dihydro-2*H*-pyrido[4,3-b][1,4]thiazin-8-yl)benzonitrile (S25);
4-(4-(quinolin-8-ylsulfonyl)-3,4-dihydro-2*H*-pyrido[4,3-b][1,4]thiazin-8-yl)benzonitrile (S26);
3-(4-((4-methoxyphenyl)sulfonyl)-3,4-dihydro-2*H*-pyrido[4,3-b][1,4]thiazin-8-yl)benzonitrile (S27);
8-(4-chlorophenyl)-4-((4-methoxyphenyl)sulfonyl)-3,4-dihydro-2*H*-pyrido[4,3-b][1,4]thiazine (S28);
8-(3-chlorophenyl)-4-((4-methoxyphenyl)sulfonyl)-3,4-dihydro-2*H*-pyrido[4,3-b][1,4]thiazine (S29);
8-(4-fluorophenyl)-4-((4-methoxyphenyl)sulfonyl)-3,4-dihydro-2*H*-pyrido[4,3-b][1,4]thiazine (S30)
8-(3-fluorophenyl)-4-((4-m;ethoxyphenyl)sulfonyl)-3,4-dihydro-2*H*-pyrido[4,3-b][1,4]thiazine (S31);
4-((4-methoxyphenyl) sulfonyl)-8-(4-(trifluoromethyl)phenyl)-3,4-dihydro-2*H*-pyrido[4,3-b][1,4]thiazine (S32);
8-(4-fluoro-3-methylphenyl)-4-((4-methoxyphenyl)sulfonyl)-3,4-dihydro-2*H*-pyrido[4,3-b][1,4]-thiazine (S33);
8-(3-chloro-4-fluorophenyl)-4-((4-methoxyphenyl)sulfonyl)-3,4-dihydro-2*H*-pyrido[4,3-b][1,4]-thiazine (S34);
2-fluoro-4-(4-((4-methoxyphenyl)sulfonyl)-3,4-dihydro-2*H*-pyrido[4,3-b][1,4]thiazin-8-yl)-benzonitrile (S35);
4-((4-methoxyphenyl) sulfonyl)-8-(thiophen-3-yl)-3,4-dihydro-2*H*-pyrido[4,3-b][1,4]thiazine (S36);
4-((4-methoxyphenyl) sulfonyl)-8-(thiophen-2-yl)-3,4-dihydro-2*H*-pyrido[4,3-b][1,4]thiazine (S37);
8-(furan-2-yl)-4-((4-methoxyphenyl)sulfonyl)-3,4-dihydro-2*H*-pyrido[4,3-b][1,4]thiazine (S38);
4-((4-methoxyphenyl)sulfonyl)-8-(1*H*-pyrazol-4-yl)-3,4-dihydro-2*H*-pyrido[4,3-b][1,4]thiazine (S39);
4-((8-(benzofuran-2-yl)-2,3-dihydro-4*H*-pyrido[4,3-b][1,4]thiazin-4-yl)sulfonyl)benzonitrile (S40);
4-((8-(5-chlorobenzofuran-2-yl)-2,3-dihydro-4*H*-pyrido[4,3-b][1,4]thiazin-4-yl)sulfonyl)-benzonitrile (S41);
2-(4-((4-cyanophenyl)sulfonyl)-3,4-dihydro-2*H*-pyrido[4,3-b] [1,4]thiazin-8-yl)benzofuran-5-carbonitrile (S42);
4-((8-(benzo[*b*]thiophen-2-yl)-2,3-dihydro-4*H*-pyrido[4,3-b][1,4]thiazin-4-yl)sulfonyl)benzonitrile (S43);
4-((8-(benzofuran-5-yl)-2,3-dihydro-4*H*-pyrido[4,3-b][1,4]thiazin-4-yl)sulfonyl)benzonitrile(S44);
4-((8-(1-methyl-1*H*-indazol-6-yl)-2,3-dihydro-4*H*-pyrido[4,3-b][1,4]thiazin-4-yl)sulfonyl)-benzonitrile (S45);
4-((8-(benzo[*d*]thiazol-6-yl)-2,3-dihydro-4*H*-pyrido[4,3-b][1,4]thiazin-4-yl)sulfonyl)benzonitrile (S46);
4-((8-(benzo[*d*]oxazol-6-yl)-2,3-dihydro-4*H*-pyrido[4,3-b][1,4]thiazin-4-yl)sulfonyl)benzonitrile (47);
4-((8-(1*H*-indazol-5-yl)-2,3-dihydro-4*H*-pyrido[4,3-b][1,4]thiazin-4-yl)sulfonyl)benzonitrile (S48);
4-((8-(1*H*-benzo[*d*]imidazol-6-yl)-2,3-dihydro-4*H*-pyrido[4,3-b][1,4]thiazin-4-yl)sulfonyl)-benzonitrile (S49);
4-((8-(imidazo[1,2-*a*]pyridin-7-yl)-2,3-dihydro-4*H*-pyrido[4,3-b][1,4]thiazin-4-yl)sulfonyl)-benzonitrile (S50);
4-((8-(2-oxoindolin-5-yl)-2,3-dihydro-4*H*-pyrido[4,3-b][1,4]thiazin-4-yl)sulfonyl)benzonitrile (S51);
4-((8-(8-fluoro-1-methyl-2-oxo-1,2,3,4-tetrahydroquinolin-6-yl)-2,3-dihydro-4*H*-pyrido[4,3-b] [1,4] thiazin-4-yl)sulfonyl)benzonitrile (S52);
8-(5-Chlorobenzofuran-2-yl)-4-((6-methoxypyridin-3-yl)sulfonyl)-3,4-dihydro-2*H*-pyrido[4,3-b][1, 4]thiazine (S53);
5-((8-(5-chlorobenzofuran-2-yl)-2,3-dihydro-4H-pyrido[4,3-b][1,4]thiazin-4-yl)sulfonyl)pyridin-2-ol (S54);
8-(5-Chlorobenzofuran-2-yl)-4-((1-methyl-1H-pyrazol-4-yl)sulfonyl)-3,4-dihydro-2H-pyrido[4,3-b] [1,4]thiazine (S55);
8-(5-chlorobenzofuran-2-yl)-4-((1-methyl-1H-pyrazol-3-yl)sulfonyl)-3,4-dihydro-2H-pyrido[4,3-b][ 1,4]thiazine (S56);
N-(5-((8-(5-Chlorobenzofuran-2-yl)-2,3-dihydro-4H-pyrido[4,3-b][1,4]thiazin-4-yl)sulfonyl)-4-methylthiazol-2-yl)acetamide (S57);
1-(5-((8-(5-Chlorobenzofuran-2-yl)-2,3-dihydro-4H-pyrido[4,3-b][1,4]thiazin-4-yl)sulfonyl)indolin -1-yl)ethan-1-one (S58);
6-((8-(5-Chlorobenzofuran-2-yl)-2,3-dihydro-4H-pyrido[4,3-b][1,4]thiazin-4-yl)sulfonyl)-2H-benzo[b][1,4]oxazin-3(4H)-one (S59);
8-(5-Chlorobenzofuran-2-yl)-4-((5-chlorothiophen-2-yl)sulfonyl)-3,4-dihydro-2H-pyrido[4,3-b][1,4 ]thiazine (S60);
8-(5-Chlorobenzofuran-2-yl)-4-(piperidin-4-ylsulfonyl)-3,4-dihydro-2H-pyrido[4,3-b][1,4]thiazine (S61);
4-(Azetidin-3-ylsulfonyl)-8-(5-chlorobenzofuran-2-yl)-3,4-dihydro-2H-pyrido[4,3-b][1,4]thiazine (S62);
4-((8-(5-Chlorobenzofuran-2-yl)-2,3-dihydro-4H-pyrido[4,3-b][1,4]thiazin-4-yl)sulfonyl)piperidine -1-carbonitrile (S63);
3-((8-(5-Chlorobenzofuran-2-yl)-2,3-dihydro-4H-pyrido[4,3-b][1,4]thiazin-4-yl)sulfonyl)azetidine-1-carbonitrile (S64);
3-((8-(5-chlorobenzofuran-2-yl)-2,3-dihydro-4H-pyrido[4,3-b][1,4]thiazin-4-yl)sulfonyl)bicyclo[1.1 .1]pentane-1-carbonitrile (S65);
4-((2-oxaspiro[3.3]heptan-6-yl)sulfonyl)-8-(5-chlorobenzofuran-2-yl)-3,4-dihydro-2H-pyrido[4,3-b][1,4]thiazine (S66);
6-((8-(5-chlorobenzofuran-2-yl)-2,3-dihydro-4H-pyrido[4,3-b][1,4]thiazin-4-yl)sulfonyl)-2-azaspiro [3.3]heptane-2-carbonitrile (S67);
4-(4-((1-(4-fluorobenzoyl)azetidin-3-yl)sulfonyl)-3,4-dihydro-2H-pyrido[4,3-b][1,4]thiazin-8-yl)-benzonitrile (S68);
4-(4-((1-(3-fluorobenzoyl)azetidin-3-yl)sulfonyl)-3,4-dihydro-2*H*-pyrido[4,3-b][1,4]thiazin-8-yl)-benzonitrile (S69);
4-(4-((1-(4-cyanobenzoyl)azetidin-3-yl)sulfonyl)-3,4-dihydro-2*H*-pyrido[4,3-b] [1,4]thiazin-8-yl)-benzonitrile(S70);
3-(3-((8-(4-cyanophenyl)-2,3-dihydro-4H-pyrido[4,3-b] [1,4]thiazin-4-yl)sulfonyl)azetidine-1-carbonyl)benzonitrile (S71);
4-(4-((1-(4-methoxybenzoyl)azetidin-3-yl)sulfonyl)-3,4-dihydro-2*H*-pyrido[4,3-b][1,4]thiazin-8-yl) benzonitrile (S72);
4-(4-((1-(3-methoxybenzoyl)azetidin-3-yl)sulfonyl)-3,4-dihydro-2*H*-pyrido[4,3-b][1,4]thiazin-8-yl) benzonitrile (S73);
4-(4-((1-((4-fluorophenyl)sulfonyl)azetidin-3-yl)sulfonyl)-3,4-dihydro-2*H*-pyrido[4,3-b][1,4]thiazin -8-yl)benzonitrile (S74);
4-(4-((1-((3-fluorophenyl)sulfonyl)azetidin-3-yl)sulfonyl)-3,4-dihydro-2*H*-pyrido[4,3-b][1,4]thiazin -8-yl)benzonitrile (S75);
4-((3-((8-(4-cyanophenyl)-2,3-dihydro-4*H*-pyrido[4,3-b][1,4]thiazin-4-yl)sulfonyl)azetidin-1-yl)-sulfonyl)benzonitrile (S76);
3-((3-((8-(4-cyanophenyl)-2,3-dihydro-4*H*-pyrido[4,3-b][1,4]thiazin-4-yl)sulfonyl)azetidin-1-yl)-sulfonyl)benzonitrile (S77);
4-(4-((1-((4-methoxyphenyl)sulfonyl)azetidin-3-yl)sulfonyl)-3,4-dihydro-2*H*-pyrido[4,3-b][1,4]-thiazin-8-yl)benzonitrile (S78);
4-(4-((1-((3-methoxyphenyl)sulfonyl)azetidin-3-yl)sulfonyl)-3,4-dihydro-2*H*-pyrido[4,3-b] [1,4]-thiazin-8-yl)benzonitrile (S79);
4-(4-((1-(4-cyanobenzyl)azetidin-3-yl)sulfonyl)-3,4-dihydro-2*H*-pyrido[4,3-b][1,4]thiazin-8-yl)-benzonitrile (S80);
3-((3-((8-(4-cyanophenyl)-2,3-dihydro-4*H*-pyrido[4,3-b][1,4]thiazin-4-yl)sulfonyl)azetidin-1-yl)-methyl)benzonitrile (S81);
4-(4-((1-(4-fluorobenzyl)azetidin-3-yl)sulfonyl)-3,4-dihydro-2*H*-pyrido[4,3-b][1,4]thiazin-8-yl)-benzonitrile (S82);
4-(4-((1-(3-fluorobenzyl)azetidin-3-yl)sulfonyl)-3,4-dihydro-2*H*-pyrido[4,3-b][1,4]thiazin-8-yl)-benzonitrile (S83);
4-(4-((1-(4-methoxybenzyl)azetidin-3-yl)sulfonyl)-3,4-dihydro-2*H*-pyrido[4,3-b] [1,4]thiazin-8-yl)-benzonitrile (S84);
4-(4-((1-(3-methoxybenzyl)azetidin-3-yl)sulfonyl)-3,4-dihydro-2*H*-pyrido[4,3-b] [1,4]thiazin-8-yl)-benzonitrile (S85);
*N*-(2-(8-(4-cyanophenyl)-3,4-dihydro-2*H*-pyrido[4,3-b][1,4]thiazine-4-carbonyl)phenyl)acetamide (S86);
4-(4-(3-(trifluoromethyl)benzoyl)-3,4-dihydro-2*H*-pyrido[4,3-b] [1,4]thiazin-8-yl)benzonitrile (S87);
4-(4-(4-(trifluoromethyl)benzoyl)-3,4-dihydro-2*H*-pyrido[4,3-b][1,4]thiazin-8-yl)benzonitrile (S88);
4-(4-(4-methylbenzoyl)-3,4-dihydro-2*H*-pyrido[4,3-b][1,4]thiazin-8-yl)benzonitrile (S89);
4-(4-(2-methylbenzoyl)-3,4-dihydro-2*H*-pyrido[4,3-b][1,4]thiazin-8-yl)benzonitrile (S90);
4-(4-(3-methylbenzoyl)-3,4-dihydro-2*H*-pyrido[4,3-b][1,4]thiazin-8-yl)benzonitrile (S91);
4-(4-(4-chlorobenzoyl)-3,4-dihydro-2*H*-pyrido[4,3-b][1,4]thiazin-8-yl)benzonitrile (S92);
4-(4-(3-chlorobenzoyl)-3,4-dihydro-2*H*-pyrido[4,3-b][1,4]thiazin-8-yl)benzonitrile (S93);
4-(4-(4-cyanobenzoyl)-3,4-dihydro-2*H*-pyrido[4,3-b][1,4]thiazin-8-yl)benzonitrile (S94);
3-(8-(4-cyanophenyl)-3,4-dihydro-2*H*-pyrido[4,3-b][1,4]thiazine-4-carbonyl)benzonitrile (S95);
4-(4-(3-methoxybenzoyl)-3,4-dihydro-2*H*-pyrido[4,3-b][1,4]thiazin-8-yl)benzonitrile (S96);
4-(4-(4-methoxybenzoyl)-3,4-dihydro-2*H*-pyrido[4,3-b][1,4]thiazin-8-yl)benzonitrile (S97);
2-(4-(4-methoxybenzoyl)-3,4-dihydro-2*H*-pyrido[4,3-b][1,4]thiazin-8-yl)benzofuran-5-carbonitrile (S98);
4-(3-oxo-3,4-dihydro-2H-pyrido[4,3-b][1,4]thiazin-8-yl)benzonitrile (S99);
4-(4-(4-chlorobenzoyl)-3-oxo-3,4-dihydro-2H-pyrido[4,3-b][1,4]thiazin-8-yl)benzonitrile (S100);
1-((8-(5-Chlorobenzofuran-2-yl)-2,3-dihydro-4H-pyrido[4,3-b][1,4]thiazin-4-yl)sulfonyl)azetidine-3-carbonitrile (S101);
8-(5-Chlorobenzofuran-2-yl)-4-((5,6-dihydro-[1,2,4]triazolo[4,3-a]pyrazin-7(8H)-yl)sulfonyl)-3,4-dihydro-2H-pyrido[4,3-b][1,4]thiazine (S102);
8-(5-Chlorobenzofuran-2-yl)-4-((1-(oxetan-3-yl)azetidin-3-yl)sulfonyl)-3,4-dihydro-2H-pyrido[4,3-b][1,4]thiazine (S103);
4-(1-tosyl-2,3-dihydro-1*H*-pyrrolo[2,3-c]pyridin-4-yl)benzonitrile (C1);
4-(1-((4-nitrophenyl) sulfonyl)-2,3-dihydro-1*H*-pyrrolo[2,3-*c*]pyridin-4-yl)benzonitrile (C2);
4-(1-((4-methoxyphenyl)sulfonyl)-2,3-dihydro-1*H*-pyrrolo[2,3-*c*]pyridin-4-yl)benzonitrile (C3);
4-(1-((4-(trifluoromethyl)phenyl)sulfonyl)-2,3-dihydro-1*H*-pyrrolo[2,3-*c*]pyridin-4-yl)benzonitrile (C4);
4-(1-((6-chloropyridin-3-yl)sulfonyl)-2,3-dihydro-1*H*-pyrrolo[2,3-*c*]pyridin-4-yl)benzonitrile (C5);
4-(1-((3-fluorophenyl)sulfonyl)-2,3-dihydro-1*H*-pyrrolo[2,3-*c*]pyridin-4-yl)benzonitrile (C6);
4-(1-((4-fluorophenyl)sulfonyl)-2,3-dihydro-1*H*-pyrrolo[2,3-*c*]pyridin-4-yl)benzonitrile (C7);
4-(1-(m-tolylsulfonyl)-2,3-dihydro-1*H*-pyrrolo[2,3-*c*]pyridin-4-yl)benzonitrile (C8);
4-(1-((3-nitrophenyl)sulfonyl)-2,3-dihydro-1*H*-pyrrolo[2,3-*c*]pyridin-4-yl)benzonitrile (C9);
4-(1-(pyridin-3-ylsulfonyl)-2,3-dihydro-1*H*-pyrrolo[2,3-*c*]pyridin-4-yl)benzonitrile (C10);
4-(1-(quinolin-8-ylsulfonyl)-2,3-dihydro-1*H*-pyrrolo[2,3-*c*]pyridin-4-yl)benzonitrile (C11);
4-(1-(naphthalen-2-ylsulfonyl)-2,3-dihydro-1*H*-pyrrolo[2,3-*c*]pyridin-4-yl)benzonitrile (C12);
4-(1-((4-chlorophenyl)sulfonyl)-2,3-dihydro-1*H*-pyrrolo[2,3-*c*]pyridin-4-yl)benzonitrile (C13);
4-(1-((3-chlorophenyl)sulfonyl)-2,3-dihydro-1*H*-pyrrolo[2,3-*c*]pyridin-4-yl)benzonitrile (C14);
4-((4-(4-cyanophenyl)-2,3-dihydro-1*H*-pyrrolo[2,3-*c*]pyridin-1-yl)sulfonyl)benzonitrile (C15);
3-((4-(4-cyanophenyl)-2,3-dihydro-1*H*-pyrrolo[2,3-*c*]pyridin-1-yl)sulfonyl)benzonitrile (C16);
4-(1-(thiophen-2-ylsulfonyl)-2,3-dihydro-1*H*-pyrrolo[2,3-*c*]pyridin-4-yl)benzonitrile (C17);
4-(1-(naphthalen-1-ylsulfonyl)-2,3-dihydro-1*H*-pyrrolo[2,3-*c*]pyridin-4-yl)benzonitrile (C18);
4-(1-((3-methoxyphenyl)sulfonyl)-2,3-dihydro-1*H*-pyrrolo[2,3-*c*]pyridin-4-yl)benzonitrile (C19);
4-(1-((3-[trifluoromethyl]phenyl)sulfonyl)-2,3-dihydro-1*H*-pyrrolo[2,3-*c*]pyridin-4-yl)benzonitrile (C20);
4-(1-(phenylsulfonyl)-2,3-dihydro-1*H*-pyrrolo[2,3-*c*]pyridin-4-yl)benzonitrile (C21);
4-(1-(4-(trifluoromethyl)benzoyl)-2,3-dihydro-1*H*-pyrrolo[2,3-*c*]pyridin-4-yl)benzonitrile (C22);
4-(1-(3-(trifluoromethyl)benzoyl)-2,3-dihydro-1*H*-pyrrolo[2,3-*c*]pyridin-4-yl)benzonitrile (C23);
4-(1-(4-methoxybenzoyl)-2,3-dihydro-1*H*-pyrrolo[2,3-*c*]pyridin-4-yl)benzonitrile (C24);
4-(1-(4-fluorobenzoyl)-2,3-dihydro-1*H*-pyrrolo[2,3-*c*]pyridin-4-yl)benzonitrile (C25);
4-(1-(3-methoxybenzoyl)-2,3-dihydro-1*H*-pyrrolo[2,3-*c*]pyridin-4-yl)benzonitrile (C26);
4-(1-(3-nitrobenzoyl)-2,3-dihydro-1*H*-pyrrolo[2,3-*c*]pyridin-4-yl)benzonitrile (C27);
4-(1-(3-fluorobenzoyl)-2,3-dihydro-1*H*-pyrrolo[2,3-*c*]pyridin-4-yl)benzonitrile (C28);
4-(1-nicotinoyl-2,3-dihydro-1*H*-pyrrolo[2,3-*c*]pyridin-4-yl)benzonitrile (C29);
4-(1-(4-nitrobenzoyl)-2,3-dihydro-1*H*-pyrrolo[2,3-*c*]pyridin-4-yl)benzonitrile (C30);
4-(1-(2-fluorobenzoyl)-2,3-dihydro-1*H*-pyrrolo[2,3-*c*]pyridin-4-yl)benzonitrile (C31);
4-(1-(2-(trifluoromethyl)benzoyl)-2,3-dihydro-1*H*-pyrrolo[2,3-*c*]pyridin-4-yl)benzonitrile (C32);
4-(1-(4-methylbenzoyl)-2,3-dihydro-1*H*-pyrrolo[2,3-*c*]pyridin-4-yl)benzonitrile (C33);
4-(1-(2-methoxybenzoyl)-2,3-dihydro-1*H*-pyrrolo[2,3-*c*]pyridin-4-yl)benzonitrile (C34);
4-(1-(2-nitrobenzoyl)-2,3-dihydro-1*H*-pyrrolo[2,3-*c*]pyridin-4-yl)benzonitrile (C35);
4-(1-(2-methylbenzoyl)-2,3-dihydro-1*H*-pyrrolo[2,3-*c*]pyridin-4-yl)benzonitrile (C36);
3-(4-(4-cyanophenyl)-2,3-dihydro-1*H*-pyrrolo[2,3-*c*]pyridine-1-carbonyl)benzonitrile (C37);
4-(1-(4-chlorobenzoyl)-2,3-dihydro-1*H*-pyrrolo[2,3-*c*]pyridin-4-yl)benzonitrile (C38);
4-(1-(3-chlorobenzoyl)-2,3-dihydro-1*H*-pyrrolo[2,3-*c*]pyridin-4-yl)benzonitrile (C39);
4-(1-(3-methylbenzoyl)-2,3-dihydro-1*H*-pyrrolo[2,3-*c*]pyridin-4-yl)benzonitrile (C40);
4-(1-benzoyl-2,3-dihydro-1*H*-pyrrolo[2,3-*c*]pyridin-4-yl)benzonitrile (C41);
4-(1-pivaloyl-2,3-dihydro-1*H*-pyrrolo[2,3-*c*]pyridin-4-yl)benzonitrile (C42);
4-(1-(2-chloroacetyl)-2,3-dihydro-1*H*-pyrrolo[2,3-*c*]pyridin-4-yl)benzonitrile (C43);
4-(1-(furan-2-carbonyl)-2,3-dihydro-1*H*-pyrrolo[2,3-*c*]pyridin-4-yl)benzonitrile (C44);
4-(1-(3-methylthiophene-2-carbonyl)-2,3-dihydro-1*H*-pyrrolo[2,3-*c*]pyridin-4-yl)benzonitrile (C45);
4-(1-(thiophene-2-carbonyl)-2,3-dihydro-1*H*-pyrrolo[2,3-*c*]pyridin-4-yl)benzonitrile (C46);
Methyl 4-(4-(4-cyanophenyl)-2,3-dihydro-1*H*-pyrrolo[2,3-*c*]pyridine-1-carbonyl)benzoate (C47);
4-(1-(4-cyanobenzoyl)-2,3-dihydro-1*H*-pyrrolo[2,3-*c*]pyridin-4-yl)benzonitrile (C48);
4-(1-(4-(bromomethyl)benzoyl)-2,3-dihydro-1*H*-pyrrolo[2,3-*c*]pyridin-4-yl)benzonitrile (C49);
(4-(1H-indazol-5-yl)-2,3-dihydro-1*H*-pyrrolo[2,3-*c*]pyridin-1-yl)(2-fluorophenyl)methanone (C50);
(2-fluorophenyl)(4-(1-methyl-1H-indazol-5-yl)-2,3-dihydro-1*H*-pyrrolo[2,3-*c*]pyridin-1-yl)-methanone (C51);
(2-fluorophenyl)(4-(3-fluorophenyl)-2,3-dihydro-1*H*-pyrrolo[2,3-*c*]pyridin-1-yl)methanone (C52);
(2-fluorophenyl)(4-(3-nitrophenyl)-2,3-dihydro-1*H*-pyrrolo[2,3-*c*]pyridin-1-yl)methanone (C53);
(4-(4-chlorophenyl)-2,3-dihydro-1*H*-pyrrolo[2,3-*c*]pyridin-1-yl)(2-fluorophenyl)methanone (C54);
3-(1-(2-fluorobenzoyl)-2,3-dihydro-1*H*-pyrrolo[2,3-*c*]pyridin-4-yl)benzonitrile (C55);
(2-fluorophenyl)(4-(furan-3-yl)-2,3-dihydro-1*H*-pyrrolo[2,3-*c*]pyridin-1-yl)methanone (C56);
(2-fluorophenyl)(4-(imidazo[1,2-*a*]pyridin-6-yl)-2,3-dihydro-1*H*-pyrrolo[2,3-c]pyridin-1-yl)-methanone (C57);
(4-(benzo[*d*]thiazol-6-yl)-2,3-dihydro-1*H*-pyrrolo[2,3-*c*]pyridin-1-yl)(2-fluorophenyl)methanone (C58);
(4-(1H-indol-2-yl)-2,3-dihydro-1*H*-pyrrolo[2,3-*c*]pyridin-1-yl)(2-fluorophenyl)methanone (C59);
(4-(benzofuran-2-yl)-2,3-dihydro-1*H*-pyrrolo[2,3-*c*]pyridin-1-yl)(2-fluorophenyl)methanone (C60);
(4-(benzo[*b*]thiophen-2-yl)-2,3-dihydro-1*H*-pyrrolo[2,3-*c*]pyridin-1-yl)(2-fluorophenyl)methanone (C61);
(4-(1H-indol-5-yl)-2,3-dihydro-1*H*-pyrrolo[2,3-*c*]pyridin-1-yl)(2-fluorophenyl)methanone (C62);
(4-(benzofuran-5-yl)-2,3-dihydro-1*H*-pyrrolo[2,3-*c*]pyridin-1-yl)(2-fluorophenyl)methanone (C63);
4-(1-(2-(o-tolyl)acetyl)-2,3-dihydro-1*H*-pyrrolo[2,3-*c*]pyridin-4-yl)benzonitrile (C64);
4-(1-(2-(2-bromophenyl)acetyl)-2,3-dihydro-1*H*-pyrrolo[2,3-*c*]pyridin-4-yl)benzonitrile (C65);
4-(1-(2-(m-tolyl)acetyl)-2,3-dihydro-1*H*-pyrrolo[2,3-*c*]pyridin-4-yl)benzonitrile (C66);
4-(1-(2-(2-fluorophenyl)acetyl)-2,3-dihydro-1*H*-pyrrolo[2,3-*c*]pyridin-4-yl)benzonitrile (C67);
4-(1-(2-(3-methoxyphenyl)acetyl)-2,3-dihydro-1*H*-pyrrolo[2,3-*c*]pyridin-4-yl)benzonitrile (C68);
4-(1-(2-(4-fluorophenyl)acetyl)-2,3-dihydro-1*H*-pyrrolo[2,3-*c*]pyridin-4-yl)benzonitrile (C69);
4-(1-(2-(2-nitrophenyl)acetyl)-2,3-dihydro-1*H*-pyrrolo[2,3-*c*]pyridin-4-yl)benzonitrile (C70);
4-(1-(2-(3-chlorophenyl)acetyl)-2,3-dihydro-1*H*-pyrrolo[2,3-*c*]pyridin-4-yl)benzonitrile (C71);
4-(1-(2-(p-tolyl)acetyl)-2,3-dihydro-1*H*-pyrrolo[2,3-*c*]pyridin-4-yl)benzonitrile (C72);
4-(1-(2-(4-bromophenyl)acetyl)-2,3-dihydro-1*H*-pyrrolo[2,3-*c*]pyridin-4-yl)benzonitrile (C73);
4-(1-(2-(3-(trifluoromethyl)phenyl)acetyl)-2,3-dihydro-1*H*-pyrrolo[2,3-*c*]pyridin-4-yl)benzonitrile (C74);
4-(1-(2-(4-(trifluoromethyl)phenyl)acetyl)-2,3-dihydro-1*H*-pyrrolo[2,3-*c*]pyridin-4-yl)benzonitrile (C75);
4-(1-(2-(4-methoxyphenyl)acetyl)-2,3-dihydro-1*H*-pyrrolo[2,3-*c*]pyridin-4-yl)benzonitrile (C76);
4-(1-(2-(4-chlorophenyl)acetyl)-2,3-dihydro-1*H*-pyrrolo[2,3-*c*]pyridin-4-yl)benzonitrile (C77);
4-((4-(5-chlorobenzofuran-2-yl)-2,3-dihydro-1*H*-pyrrolo[2,3-*c*]pyridin-1-yl)sulfonyl)benzonitrile (C78);
2-(1-(4-methoxybenzoyl)-2,3-dihydro-1*H*-pyrrolo[2,3-*c*]pyridin-4-yl)benzofuran-5-carbonitrile (C79);
(4-(5-chloro-1H-indol-2-yl)-2,3-dihydro-1*H*-pyrrolo[2,3-*c*]pyridin-1-yl)(1-(3-fluorobenzyl)azetidin-3-yl)methanone (C80);
2-(1-(1-(3-fluorobenzyl)azetidine-3-carbonyl)-2,3-dihydro-1*H*-pyrrolo[2,3-*c*]pyridin-4-yl)-1*H-*indole-5-carbonitrile (C81);
4-(1-(3-methoxybenzoyl)-2,3-dihydro-1H-pyrrolo[2,3-c]pyridin-4-yl)-3,6-dihydropyridine-1(2H)-carbonitrile (C82);
4-(1-(3-methoxybenzoyl)-2,3-dihydro-1H-pyrrolo[2,3-c]pyridin-4-yl)piperazine-1-carbonitrile (C83);
4-(1-((4-nitrophenyl)sulfonyl)-1*H*-pyrrolo[2,3-*c*]pyridin-4-yl)benzonitrile (E1);
4-(1-tosyl-1*H*-pyrrolo[2,3-*c*]pyridin-4-yl)benzonitrile (E2);
4-(1-(phenylsulfonyl)-1*H*-pyrrolo[2,3-*c*]pyridin-4-yl)benzonitrile (E3);
4-(1-(m-tolylsulfonyl)-1*H*-pyrrolo[2,3-*c*]pyridin-4-yl)benzonitrile (E4);
4-(1-((3-(trifluoromethyl)phenyl)sulfonyl)-1*H*-pyrrolo[2,3-*c*]pyridin-4-yl)benzonitrile (E5);
4-(1-((4-fluorophenyl)sulfonyl)-1*H*-pyrrolo[2,3-*c*]pyridin-4-yl)benzonitrile (E6);
4-(1-((4-(trifluoromethyl)phenyl)sulfonyl)-1*H*-pyrrolo[2,3-*c*]pyridin-4-yl)benzonitrile (E7);
4-(1-((4-chlorophenyl)sulfonyl)-1*H*-pyrrolo[2,3-*c*]pyridin-4-yl)benzonitrile (E8);
4-(1-((4-methoxyphenyl)sulfonyl)-1*H*-pyrrolo[2,3-*c*]pyridin-4-yl)benzonitrile (E9);
3-((4-(4-cyanophenyl)-1*H*-pyrrolo[2,3-*c*]pyridin-1-yl)sulfonyl)benzonitrile (E10);
4-(1-((3-chlorophenyl)sulfonyl)-1*H*-pyrrolo[2,3-*c*]pyridin-4-yl)benzonitrile (E11);
4-(1-((3-fluorophenyl)sulfonyl)-1*H*-pyrrolo[2,3-*c*]pyridin-4-yl)benzonitrile (E12);
4-(1-((3-nitrophenyl)sulfonyl)-1*H*-pyrrolo[2,3-*c*]pyridin-4-yl)benzonitrile (E13);
4-((4-(4-cyanophenyl)-1*H*-pyrrolo[2,3-*c*]pyridin-1-yl)sulfonyl)benzonitrile (E14);
4-(1-(naphthalen-1-ylsulfonyl)-1*H*-pyrrolo[2,3-*c*]pyridin-4-yl)benzonitrile (E15);
4-(1-(naphthalen-2-ylsulfonyl)-1*H*-pyrrolo[2,3-*c*]pyridin-4-yl)benzonitrile (E16);
4-(1-(thiophen-2-ylsulfonyl)-1*H*-pyrrolo[2,3-*c*]pyridin-4-yl)benzonitrile (E17);
4-(1-((6-chloropyridin-3-yl)sulfonyl)-1*H*-pyrrolo[2,3-*c*]pyridin-4-yl)benzonitrile (E18);
4-(1-(perfluorobenzoyl)-1*H*-pyrrolo[2,3-*c*]pyridin-4-yl)benzonitrile (E19);
4-(1-(4-(trifluoromethyl)benzoyl)-1*H*-pyrrolo[2,3-*c*]pyridin-4-yl)benzonitrile (E20);
4-(1-(2-iodobenzoyl)-1*H*-pyrrolo[2,3-*c*]pyridin-4-yl)benzonitrile (E21);
4-(1-(3-nitrobenzoyl)-1*H*-pyrrolo[2,3-*c*]pyridin-4-yl)benzonitrile (E22);
4-(1-(3-(trifluoromethyl)benzoyl)-1*H*-pyrrolo[2,3-*c*]pyridin-4-yl)benzonitrile (E23);
4-(1-(3-methoxybenzoyl)-1*H*-pyrrolo[2,3-*c*]pyridin-4-yl)benzonitrile (E24);
4-(1-(2-methoxybenzoyl)-1*H*-pyrrolo[2,3-*c*]pyridin-4-yl)benzonitrile (E25);
4-(1-(2-(trifluoromethyl)benzoyl)-1*H*-pyrrolo[2,3-*c*]pyridin-4-yl)benzonitrile (E26);
4-(1-(2-methylbenzoyl)-1*H*-pyrrolo[2,3-*c*]pyridin-4-yl)benzonitrile (E27);
4-(1-(2-fluorobenzoyl)-1*H*-pyrrolo[2,3-*c*]pyridin-4-yl)benzonitrile (E28);
4-(1-(2-nitrobenzoyl)-1*H*-pyrrolo[2,3-*c*]pyridin-4-yl)benzonitrile (E29);
4-(1-(4-fluorobenzoyl)-1*H*-pyrrolo[2,3-*c*]pyridin-4-yl)benzonitrile (E30);
(4-(benzo[*b*]thiophen-2-yl)-1*H*-pyrrolo[2,3-*c*]pyridin-1-yl)(4-fluorophenyl)methanone (E31);
(4-(benzofuran-2-yl)-1*H*-pyrrolo[2,3-*c*]pyridin-1-yl)(4-fluorophenyl)methanone (E32);
(4-(benzofuran-5-yl)-1*H*-pyrrolo[2,3-*c*]pyridin-1-yl)(4-fluorophenyl)methanone (E33);
(4-(1*H*-indol-2-yl)-1*H*-pyrrolo[2,3-*c*]pyridin-1-yl)(4-fluorophenyl)methanone (E34);
(4-fluorophenyl)(4-(1-methyl-1*H*-indazol-6-yl)-1*H*-pyrrolo[2,3-*c*]pyridin-1-yl)methanone (E35);
(4-(benzo[*d*]thiazol-6-yl)-1*H*-pyrrolo[2,3-*c*]pyridin-1-yl)(4-fluorophenyl)methanone (E36);
(4-fluorophenyl)(4-(imidazo[1,2-*a*]pyridin-6-yl)-1*H*-pyrrolo[2,3-*c*]pyridin-1-yl)methanone (E37);
(4-(benzo[*d*]oxazol-6-yl)-1*H*-pyrrolo[2,3-*c*]pyridin-1-yl)(4-fluorophenyl)methanone (E38);
(4-fluorophenyl)(4-(imidazo[1,2-*a*]pyridin-7-yl)-1*H*-pyrrolo[2,3-*c*]pyridin-1-yl)methanone (E39);
4-(1-Methyl-2,3-dioxo-1,2,3,4-tetrahydropyrido[3,4-b]pyrazin-8-yl)benzonitrile (N1);
8-(4-Fluorophenyl)-1-methyl-1,4-dihydropyrido[3,4-b]pyrazine-2,3-dione (N2);
4-(1-Methyl-1,2,3,4-tetrahydropyrido[3,4-b]pyrazin-8-yl)benzonitrile (N3);
4-((8-(4-Cyanophenyl)-1-methyl-2,3-dihydropyrido[3,4-b]pyrazin-4(1H)-yl)sulfonyl)benzonitrile (N4);
4-(4-(4-Fluorobenzoyl)-1-methyl-1,2,3,4-tetrahydropyrido[3,4-b]pyrazin-8-yl)benzonitrile (N5);
4-(1-Methyl-1H-imidazo[4,5-c]pyridin-7-yl)benzonitrile (N6);
4-(1-Cyclopropyl-1H-imidazo[4,5-c]pyridin-7-yl)benzonitrile (N7);

In another aspect, provided are pharmaceutical compositions comprising a compound aforementioned and pharmaceutically acceptable carriers.

In another aspect, provided is the use of a compound or a composition aforementioned in the preparation of a medicament for the treatment of diseases related to one or several endogenous steroids.

In another aspect, provided is the method for treating diseases related to one or several endogenous steroids in a subject, that is administering to the subject a therapeutically effective amount of a compound or a composition aforementioned.

In certain embodiments, the one or several steroids are selected from the group of aldosterone, cortisol, and bile acids.

In certain embodiments, the disease associated with aldosterone is selected from the group consisting of primary aldosteronism, secondary aldosteronism, heart failure, renal failure, hypertension, obesity, renal fibrosis, coronary artery disease, cardiac hypertrophy, cardiac fibrosis, arrhythmia, edema, hypokalemia and the resulting muscle weakness, atrial fibrillation, weakened myocardial contraction, congestive heart failure, chronic kidney disease, diabetic nephropathy, cardiorenal syndrome, metabolic syndrome, non-alcoholic fatty liver disease, non-alcoholic steatohepatitis, liver fibrosis, and cirrhosis.

In certain embodiments, the disease associated with cortisol is selected from the group consisting of Cushing's syndrome, metabolic syndrome, insulin resistance, obesity, type 2 diabetes, breast cancer, prostate cancer, tumor metastasis, non-alcoholic fatty liver disease, non-alcoholic steatohepatitis, liver fibrosis, cirrhosis, substance addiction, behavioral addiction, substance use disorders, mood disorders, anxiety disorders, bipolar disorder, sleep disorders, insomnia, post-traumatic stress disorder (PTSD), borderline personality disorder, disruptive behavior disorders, attention deficit hyperactivity disorder (ADHD), major depressive disorder, burnout, chronic fatigue syndrome, fibromyalgia, irritable bowel syndrome, eating disorders, depression, premenstrual syndrome (PMS), obsessive-compulsive disorder (OCD), social anxiety disorder, and generalized anxiety disorder.

In certain embodiments, the disease associated with bile acids is selected from the group consisting of non-alcoholic fatty liver disease (NAFLD), non-alcoholic steatohepatitis (NASH), fatty liver, cirrhosis, liver fibrosis, hepatocellular carcinoma, hyperglycemia, hyperlipidemia, myocardial infarction, heart failure, chronic kidney disease, renal failure, chronic colitis, and ulcerative colitis.

In another aspect, provided is a compound selected from the following:

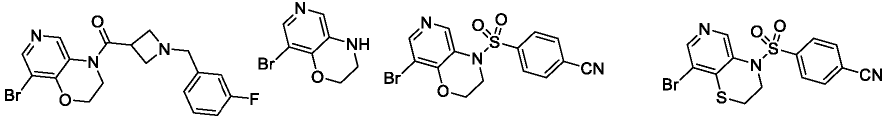

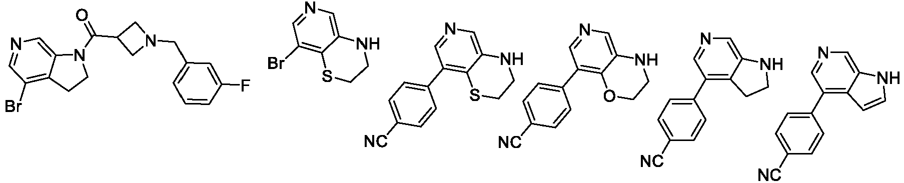

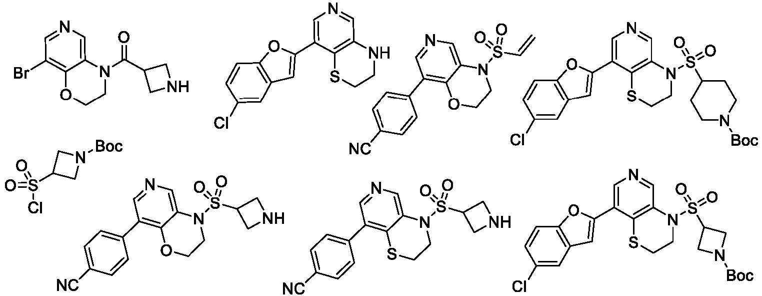

In another aspect, provided are methods for preparing any one of compounds aforementioned.

### DETAILED DESCRIPTION

To make the purpose, technical solutions, and advantages of the present invention clearer and more readily understood, in the following sections it is provide a detailed explanation of the invention with reference to specific embodiments. The embodiments described herein are provided for the purpose of explaining the invention and are not intended to limit the scope of the invention.

Unless stated otherwise, all technical and scientific terms used in this invention possess the meanings commonly understood by people skilled in the art.

The term "optionally substituted with one or more..." refers to that the described group is substituted by one, two, three, four, five, six, or more identical or different substituents from the list provided, or is not substituted by any substituent at all. Preferably, it means that the described group is substituted by one, two, or three identical or different substituents from the list provided, or is not substituted by any substituent. More preferably, it means that the described group is substituted by one, two, or three identical substituents from the list provided, or is not substituted by any substituent.

The term "alkyl" refers to a straight-chain or branched saturated hydrocarbon group containing 1 to 12 carbon atoms (C1-12 alkyl). In some embodiments, the alkyl group has 1 to 7 carbon atoms (C1-7 alkyl). In preferred embodiments, the alkyl group has 1 to 4 carbon atoms (C1-4 alkyl). Examples of alkyl groups include methyl, ethyl, propyl, isopropyl, n-butyl, isobutyl, sec-butyl, and tert-butyl. Particularly preferred alkyl groups are methyl and ethyl.

The term "alkoxy" refers to the group 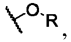 where R is an alkyl group, such as a C1-12 alkyl, preferably a C1-7 alkyl, and more preferably a C1-4 alkyl. Examples of alkoxy groups include methoxy, ethoxy, n-propoxy, isopropoxy, n-butoxy, isobutoxy, and tert-butoxy. Preferred alkoxy groups include methoxy.

The term "aryl" refers to a monovalent aromatic carbocyclic mono- or bi-cyclic system comprising 6 to 10 carbon ring atoms. Examples of aryl groups include phenyl and naphthyl. Particularly preferred aryl groups are phenyl.

The term "cyano" refers to the group

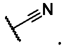

The terms "halogen" and "halo" are used interchangeably and refer to fluorine, chlorine, bromine, or iodine. Preferred halogens are chlorine and fluorine. Particularly preferred is fluorine.

The term "heteroaryl" refers to an aromatic heterocyclic mono- or bi-cyclic system containing 5 to 12 ring atoms, including 1, 2, 3, 4, 5 or 6 heteroatoms selected from N, O, and S, with the remaining ring atoms being carbon. Examples of heteroaryl groups include, but are not limited to, pyrrolyl, furanyl, thienyl, imidazolyl, pyrazolyl, oxazolyl, thiazolyl, isoxazolyl, isothiazolyl, pyridyl, pyrazinyl, pyridazinyl, pyrimidinyl, benzofuranyl, benzothienyl, benzothiazolyl, benzisothiazolyl, benzoxazolyl, benzisoxazolyl, benzimidazolyl, indolyl, isoindolyl, indazolyl, benzoxadiazolyl, benzothiadiazolyl, benzotriazolyl, imidazopyridyl, purinyl, pyrrolopyridyl, pyrrolopyrimidinyl, pyrrolidinyl, imidazopyrimidinyl, imidazolidinyl, furopyridyl, thienopyridyl, quinolinyl, isoquinolinyl, quinazolinyl, and quinoxalinyl. Preferred heteroaryl groups include pyridyl, thienyl, quinolinyl, benzofuranyl, and pyrazolyl.

The term "alkoxycarbonyl" refers to the group 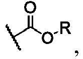 where R is an alkyl group, such as a C1-12 alkyl, preferably a C1-7 alkyl, and more preferably a C1-4 alkyl.

The term "boronate ester" refers to the group 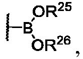 where R²⁵ and R²⁶ are independently alkyl or hydrogen (R²⁵ and R²⁶ are not both hydrogen), such as C1-12 alkyl, preferably C1-7 alkyl, and more preferably C1-4 alkyl.

The term "sulfoximino" refers to the group 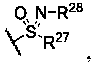 where R²⁷ and R²⁸ are independently hydrogen, alkyl, aryl or heteroaryl.

"The term 'azacycloalkyl' refers to a monocyclic or bicyclic heterocyclic group containing one or two nitrogen atoms, which may be a five- or six-membered saturated hydrocarbon group (i.e., azacycloalkyl) or an unsaturated hydrocarbon group. Examples of azacycloalkyl include but are not limited to piperidinyl, azetidinyl, dihydropyridinyl, piperazinyl."

The term "azacyclic ketone" refers to an oxo-substituted azacycloalkyl, such as 3-oxopiperazinyl."

The term "bicycloalkyl" refers to a bridged bicyclic alkyl group, which can be further substituted, for example, by a cyano group. Examples of bicycloalkyl include but are not limited to bicyclo[1.1.1]pentanyl, bicyclo[2.2.1]heptanyl, bicyclo[2.2.2]octanyl, bicyclo[3.2.1]octanyl, and bicyclo[3.2.2]nonanyl, and bicyclo[3.3.2]decanyl. Preferably, the bicycloalkyl is bicyclo[1.1.1]pentanyl:

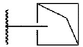

The term "spirocycloalkyl" refers to a monospiro cycloalkyl, especially a saturated monospiro cycloalkyl. The term "azaspirocycloalkyl" refers to a spirocycloalkyl containing at least one N atom in the ring. The term "oxaspirocycloalkyl" refers to a spirocycloalkyl containing at least one O atom in the ring. Examples of spirocycloalkyl include, but are not limited to, the following structures: 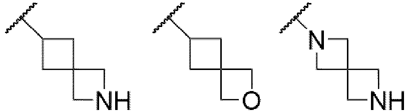 where the ring carbon atoms and/or N atoms may be further substituted."

The aforementioned alkyl, aryl, heteroaryl, spirocycloalkyl, azaspirocycloalkyl, oxaspirocycloalkyl, bicycloalkyl, azacycloalkyl, and azacyclic ketone can be substituted by one or more substituents. In some embodiments, the alkyl can be substituted by one or more substituents selected from halogens and cyano groups. Preferred substituted alkyls include cyanomethyl, cyanoethyl, chloromethyl, chloroethyl, bromoethyl, bromomethyl, bromoethyl, trifluoromethyl, etc.

In some embodiments, the aryl group is optionally substituted by one or more substituents selected from halogen, cyano, nitro, alkoxy, alkoxycarbonyl, and alkyl. Preferred substituted aryl groups include halophenyl, cyanophenyl, alkylphenyl, alkoxyphenyl, and nitrophenyl. The substitution position on the phenyl ring can be ortho, meta, para, or a combination thereof. The number of substituents on the phenyl ring can be 1, 2, 3, 4, or 5, preferably 1 or 2. Examples of substituted phenyl groups include but are not limited to 2-fluorophenyl, 3-fluorophenyl, 4-fluorophenyl, 2-methylphenyl, 3-methylphenyl, 4-methylphenyl, 3-chlorophenyl, 4-chlorophenyl, 2-bromophenyl, 3-bromophenyl, 4-bromophenyl, 2-iodophenyl, 2-methoxyphenyl, 3-methoxyphenyl, 4-methoxyphenyl, 2-nitrophenyl, 3-nitrophenyl, 4-nitrophenyl, 2-trifluoromethylphenyl, 3-trifluoromethylphenyl, 4-trifluoromethylphenyl, 3-cyanophenyl, 4-cyanophenyl, perfluorophenyl, 4-fluoro-3-methylphenyl, 3-chloro-4-fluorophenyl, and 3-fluoro-4-cyanophenyl, where the alkyl substituent can be further substituted with halogen or cyano. When the aryl group is naphthyl, it can be 1-naphthyl or 2-naphthyl.

In some embodiments, the heteroaryl group is optionally substituted by one or more substituents selected from halogen, cyano, nitro, alkoxy, and alkyl. The substitution position on the heteroaryl ring can be at different ring carbon atoms, and the number of substituents can be 1, 2, 3, 4, or 5, preferably 1 or 2. Examples of substituted heteroaryl groups include but are not limited to halogen-substituted heteroaryls, such as chloropyridinyl; alkyl-substituted heteroaryls, such as methylthienyl and methylindazolyl.

When aryl and heteroaryl groups are used as substituents, they can be attached to the substituted group at different ring carbon atoms, such as:

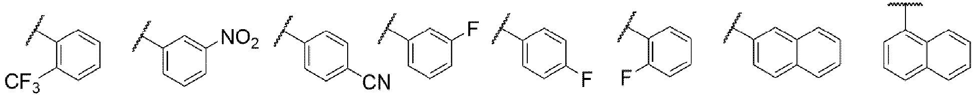

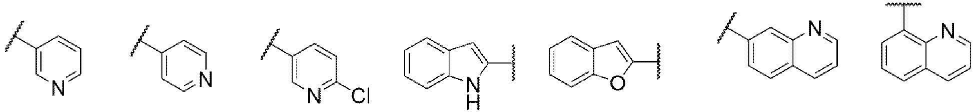

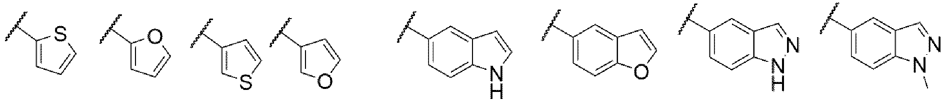

Wherein the curved solid line 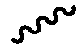 indicates the point of attachment to the substituted group.

In some embodiments, compounds of formula I are provided,

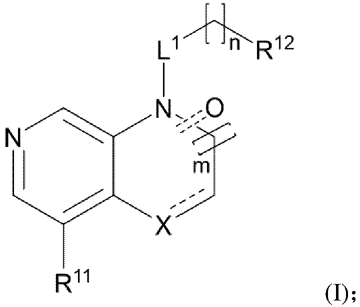

Wherein:
n is 0, 1, or 2;
m is 0 or 1;
X is C, N, O, or S;
L¹ is selected from the group consisting of hydrogen, -SO₂-, -CO-, and -CH₂-;
- - - is a single bond or a double bond;
:::::: O means that one or more carbon atoms are replaced by carbonyl groups or not;
R¹¹ is selected from the group consisting of aryl, heteroaryl, spirocycloalkyl, azaspirocycloalkyl, oxaspirocycloalkyl, bicycloalkyl, azacycloalkyl, and azacyclic ketone; Said aryl, heteroaryl, spirocycloalkyl, bicycloalkyl, azacycloalkyl, and azacyclic ketone are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens;
R¹² is selected from the group consisting of hydrogen, alkyl, azacycloalkyl, bicyclic hydrocarbyl, spirocycloalkyl, azaspirocycloalkyl, oxaspirocycloalkyl, aryl, heteroaryl, and absent; Said alkyl, aryl, and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens; Said azacycloalkyl, bicyclic hydrocarbyl, and spirocycloalkyl are optionally substituted with cyano, fluorophenyl, fluorophenylalkyl, or fluorophenylamino. Alternatively, R¹² is selected from the following structures:

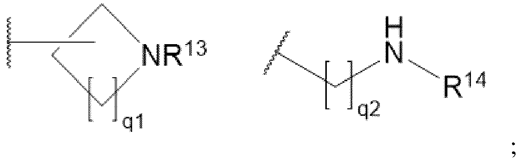

Wherein q¹ and q² are each independently selected from 1, 2, and 3; R¹³ is selected from the group consisting of hydrogen, cyano, cyclic ether, and -L²-R¹⁴, where L² is selected from -SO₂-, -CO-, hydrocarbyl, or absent; R¹⁴ is selected from aryl and heteroaryl, said aryl and heteroaryl are optionally substituted with one to three substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, and boronate ester.

The terms alkyl, aryl, heteroaryl, spirocycloalkyl, azaspirocycloalkyl, oxaspirocycloalkyl, bicycloalkyl, azacycloalkyl, and azacyclic ketone are as defined above. Therefore, the compounds of formula I include the specific compounds explicitly listed herein, as well as any other specific compounds obtained by selecting various specific substituents, even if not explicitly listed.

In other specific embodiments, compounds of formula III are provided,

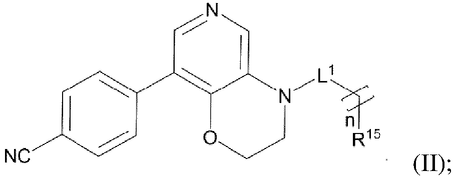

Wherein: n is 0, 1, or 2; L¹ is selected from the group consisting of -SO₂- and -CO-; R¹⁵is as defined above.

In other specific embodiments, compounds of formula III are provided,

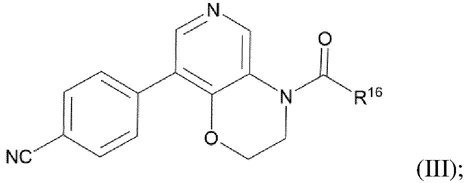

R¹⁶ is selected from the following structures:

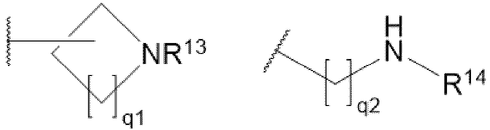

Wherein q¹, q², R¹³ and R¹⁴ are as defined above.

In other specific embodiments, compounds of formula **IV** are provided,

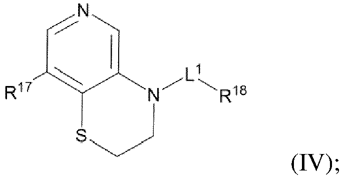

Wherein: L¹ is selected from the group consisting of -SO₂-, -CO- and -CH₂-; R¹⁷ and R¹⁸ are as defined above.

In other specific embodiments, compounds of formula **V** are provided,

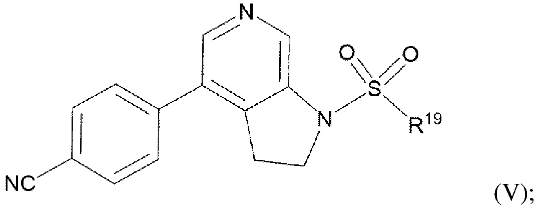

Wherein: R¹⁹ is as defined above.

In other specific embodiments, compounds of formula **VI** are provided,

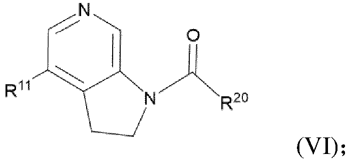

Wherein: R¹¹ and R²⁰ are as defined above.

In other specific embodiments, compounds of formula **VII** are provided, 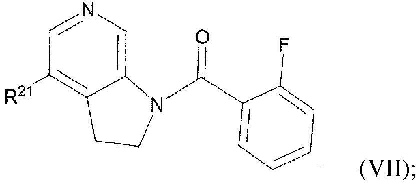 wherein: R²¹ is as defined above.

In other specific embodiments, compounds of formula **VIII** are provided, 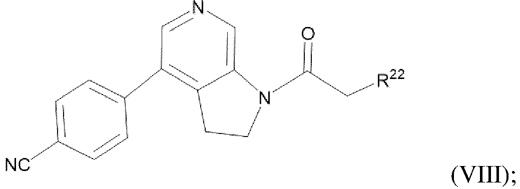 wherein: R²² is as defined above.

In other specific embodiments, compounds of formula **IX** are provided,

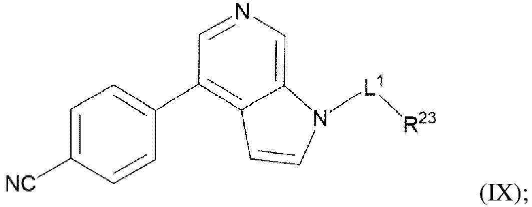

Wherein: L¹ and R²³ are as defined above.

In other specific embodiments, compounds of formula **X** are provided,

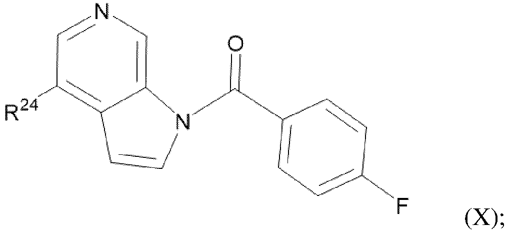

Wherein: R²⁴ is as defined above.

The compounds of formula (I)-(X) encompass all their conformational isomers, such as cis-trans isomers. The compounds of formula (I)-(X) also encompass all their optical isomers, such as enantiomers and diastereomers, as well as racemates. The compounds of formula (I)-(X) further encompass their tautomers and stereoisomers, as well as any mixtures thereof.

Methods are also provided for preparing the compounds of formula (I)-(X), as well as intermediates involved in the preparation of these compounds. These intermediates include, but are not limited to, the following compounds:

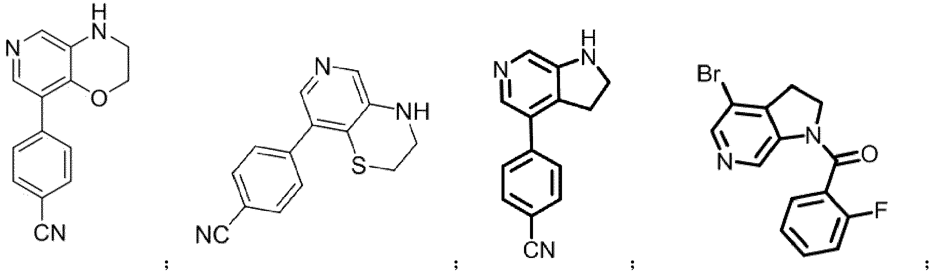

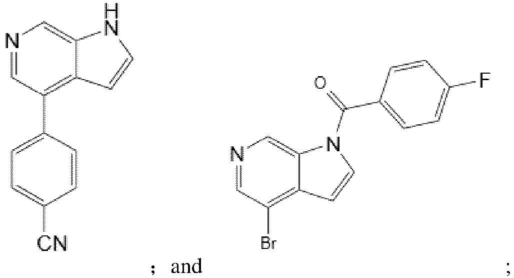

In other embodiments, provided are pharmaceutical compositions comprising at least one compound of formulae (I)-(X) and one or more pharmaceutically acceptable carriers. Here, "pharmaceutically acceptable carriers" refers to solid or liquid diluents, fillers, antioxidants, stabilizers, and other substances that can be safely administered. These substances are suitable for administration to humans and/or animals without significant adverse side effects and are appropriate for maintaining the efficacy of the drug or active pharmaceutical ingredient contained within. Standard pharmaceutical carriers can be found in Remington's Pharmaceutical Sciences, Mack Publishing Co., Easton, PA, 19th ed., 1995. Depending on the route of administration, various different carriers well-known in the art can be used, including but not limited to sugars, starches, cellulose and its derivatives, maltose, gelatin, talc, calcium sulfate, vegetable oils, synthetic oils, polyols, alginates, phosphate buffers, emulsifiers, isotonic saline, and/or pyrogen-free water. The pharmaceutical compositions provided can be administered to subjects via any suitable routes, such as orally, intravenously, intramuscularly, subcutaneously, intraperitoneally, rectally, sublingually, or through inhalation, transdermal delivery, etc.

When intended for oral administration to subjects, the carrier enables the compounds of formulae (I)-(X) to be formulated into tablets, pills, lozenges, capsules, liquids, gels, syrups, slurries, suspensions, etc., for oral ingestion by the subject being treated. Oral pharmaceutical preparations can be obtained by adding the compound of formulae (I)-(X) disclosed herein to solid excipients, optionally grinding the resulting mixture, and if necessary, processing the granular mixture after adding suitable auxiliaries to obtain tablets or lozenge cores. Suitable excipients include fillers and cellulose formulations. If needed, disintegrants may also be added.

When used for parenteral administration to a subject, compounds of formulae (I)-(X) can be formulated for administration by injection, such as by bolus injection or continuous infusion. Injectable preparations may be in unit dosage form, for example, in ampoules or in multi-dose containers, with added preservatives. The pharmaceutical compositions can take the form of suspensions, solutions, or emulsions in oily or aqueous vehicles, and may contain formulating agents such as suspending agents, stabilizers, and/or dispersants. For parenteral administration, pharmaceutical compositions include aqueous solutions of water-soluble forms of compounds of formulae (I)-(X). Additionally, suspensions of compounds of formulae (I)-(X) can be prepared as suitable oil-based injectable suspensions. Suitable lipophilic solvents or carriers include fatty oils or synthetic fatty acid esters. Aqueous injection suspensions may contain substances that increase the viscosity of the suspension. Optionally, suspensions may also contain suitable stabilizers or agents that increase the solubility of the compound and allow the preparation of highly concentrated solutions. Alternatively, the pharmaceutical compositions provided herein can be in powder form, to be reconstituted with a suitable carrier such as sterile pyrogen-free water or a lipophilic solvent before use.

Compounds of formulae (I)-(X) can also be formulated into rectal compositions, such as suppositories or retention enemas, for example, containing conventional suppository bases. In addition to the previously described formulations, the compounds of this disclosure can also be formulated as depot preparations. Such long-acting formulations can be administered by implantation (for example, subcutaneously or intramuscularly) or by intramuscular injection. Thus, for example, the compounds of this disclosure can be formulated with suitable polymers or hydrophobic materials (for example, as an emulsion in an acceptable oil) or ion exchange resins.

Specifically, compounds of formulae (I)-(X) can be administered orally, buccally, or sublingually in tablet form containing excipients such as starch or lactose, or in capsule form either alone or mixed with excipients, or as elixirs or suspensions containing flavoring or coloring agents. Such liquid formulations can be prepared using pharmaceutically acceptable additives such as suspending agents.

The exact formulation, route of administration, and dosage of the pharmaceutical compositions can be determined by the individual physician based on the diagnosed condition or disease. The dose and dosing interval can be adjusted individually to provide an amount sufficient to maintain the desired therapeutic effect.

In other embodiments, the present disclosure provides the use of compounds of formulae (I)-(X) in the preparation of drugs for treating diseases related to the synthesis and/or metabolism of steroidal compounds in subjects.

The terms "steroids" and "steroidal compounds" in the present disclosure refers to a broad class of natural compounds widely found in nature, including mineralocorticoids, glucocorticoids, phytosterols, bile acids, C21 steroids, insect molting hormones, cardiotonic glycosides, steroidal saponins, steroidal alkaloids, sapogenins, and sex hormones. Steroid compounds share a common structural feature, namely the perhydro-cyclopenta[a]phenanthrene skeleton. Additionally, they typically have two angular methyl groups (C-10, C-13) on the perhydro-cyclopenta[a]phenanthrene cores and a side chain or oxygen-containing group such as hydroxyl or carbonyl groups (C-17) with varying numbers of carbon atoms. Examples of steroid compounds include aldosterone, cortisol, cortisone, corticosterone, and bile acids. Preferably, the aforementioned diseases are associated with elevated levels of aldosterone, cortisol, corticosterone, and/or bile acids in the body.

In other embodiments, the present disclosure provides a method for treating diseases associated with abnormal levels of steroid compounds in a subject, which includes administering to a subject in need thereof a therapeutically effective amount of a compound of formulae (I)-(X) or the aforementioned pharmaceutical compositions. Compounds of formulae (I)-(X) can act in vivo as inhibitors of aldosterone synthase CYP11B2, or cortisol biosynthesis enzyme CYP11B1 (11β-hydroxylase), or bile acid biosynthesis enzymes CYP7A1 or CYP8B1, thereby inhibiting the synthesis of aldosterone, cortisol, corticosterone, and/or bile acids.

Diseases associated with abnormal (e.g., elevated) aldosterone levels include, but are not limited to, primary aldosteronism, secondary aldosteronism, heart failure, chronic renal failure, hypertension, restenosis, obesity, nephropathy, post-myocardial infarction, renal fibrosis, coronary artery disease, sodium retention, water retention, hypokalemia, hypomagnesemia, hypertension, left ventricular hypertrophy and cardiac fibrosis, cardiovascular damage, plasma renin inhibition, renal fibrosis, arrhythmia, nephropathy, edema, and muscle weakness due to hypokalemia, cardiac fibrillation and weakened myocardial contraction, congestive heart failure, resistant hypertension, chronic kidney disease, diabetic nephropathy, hyperaldosteronism, cardio-renal fibrosis, cardio-renal syndrome, and metabolic syndrome. Preferably, diseases associated with abnormal (e.g., elevated) aldosterone levels include primary aldosteronism, congestive heart failure, resistant hypertension, chronic kidney disease, diabetic nephropathy.

Diseases associated with abnormal (e.g., elevated) cortisol levels primarily involve diseases related to abnormal activity of the HPA axis. The HPA axis refers to the hypothalamic-pituitary-adrenal axis, which includes positive and negative feedback interactions between three endocrine glands: the hypothalamus, pituitary gland, and adrenal gland, forming part of the neuroendocrine system. Hormones released by these glands affect the body's response to stress, regulate bodily processes such as digestion, immune system function, emotions, sexual desire, and energy storage and expenditure. Corticotropin-releasing hormone (CRH or CRF) is secreted by the paraventricular nucleus (PVN) of the hypothalamus in response to stress. Other factors influencing CRH release include physical activity, illness, blood cortisol levels, and circadian rhythms. Under the influence of neurotransmitters like dopamine, serotonin, and norepinephrine, stress activates the HPA axis. Chronic stress activates the HPA axis in different ways depending on various factors, including whether the stressor is controllable, threats to physical integrity, trauma, individual physiology, social quality, etc. For example, oxytocin secreted under positive social influences inhibits the HPA axis and counteracts stress. In healthy individuals, cortisol levels exhibit a specific diurnal variation, peaking shortly after waking, gradually declining through late morning and midday, rising again in the late afternoon, and dropping again at night, reaching a trough in the middle of the night. Abnormalities in this cycle lead to pathological conditions. For example, flattened cortisol cycles result in chronic fatigue syndrome, insomnia, and burnout, while increased cortisol production mediates vigilance responses to stress, systemic adaptation syndrome, immune suppression, etc. Examples include Cushing's syndrome, Cushing's disease, pseudo-Cushing's syndrome, or features characterized by elevated plasma cortisol levels due to pituitary or ectopic tumors. Preferably, diseases associated with abnormal (e.g., elevated) cortisol levels include substance addiction, behavioral addiction, substance use disorders, mood disorders, anxiety disorders, bipolar disorder, sleep disorders, insomnia, post-traumatic stress syndrome, borderline personality disorder, disruptive behavior disorders, ADHD, major depression, burnout, chronic fatigue syndrome, fibromyalgia, irritable bowel syndrome, eating disorders, obesity, depression, amenorrhea, premenstrual syndrome (PMS), obsessive-compulsive disorder (OCD), social anxiety disorder, generalized anxiety disorder, dysthymia, schizophrenia, Cushing's syndrome, metabolic syndrome, insulin resistance, obesity, and type II diabetes.

Bile acids act as physiological detergents that play an important role in intestinal absorption and transport of lipids, nutrients, and vitamins. They are also signaling molecules that activate nuclear receptors and regulate cellular signaling pathways involved in lipid, glucose, and energy metabolism. Therefore, diseases requiring bile acid level inhibition include, for example, cardiovascular diseases, fatty acid metabolism and glucose utilization disorders, gastrointestinal diseases, and liver diseases. Cardiovascular diseases, fatty acid metabolism, and glucose utilization disorders include but are not limited to hypercholesterolemia; fatty acid metabolism disorders; type I and type II diabetes; complications of diabetes, including cataracts, microvascular and macrovascular diseases, retinopathy, neuropathy, nephropathy, delayed wound healing, tissue ischemia, diabetic foot, arteriosclerosis, myocardial infarction, acute coronary syndrome, unstable angina, stable angina, stroke, peripheral arterial occlusive disease, cardiomyopathy, heart failure, arrhythmias, and vascular restenosis; diabetes-related diseases such as insulin resistance (impaired glucose homeostasis), hyperglycemia, hyperinsulinemia, elevated blood levels of fatty acids or glycerol, obesity, dyslipidemia, including hypertriglyceridemia, hyperlipidemia, metabolic syndrome (syndrome X), atherosclerosis, and hypertension; and for increasing high-density lipoprotein content. Gastrointestinal diseases and disorders include constipation (including chronic constipation, functional constipation, chronic idiopathic constipation (CIC), intermittent/occasional constipation, constipation secondary to diabetes, constipation secondary to stroke, constipation secondary to chronic kidney disease, constipation secondary to multiple sclerosis, constipation secondary to Parkinson's disease, constipation secondary to systemic sclerosis, drug-induced constipation, constipation-predominant irritable bowel syndrome (IBS-C), mixed-type IBS (IBS-M), pediatric functional constipation, and opioid-induced constipation); Crohn's disease; primary bile acid malabsorption; irritable bowel syndrome (IBS); inflammatory bowel disease (IBD); ileitis; and reflux diseases and their complications, such as Barrett's esophagus, bile reflux esophagitis, and bile reflux gastritis. Liver diseases refer to any diseases of the liver and connected organs such as the pancreas, portal vein, hepatic parenchyma, intrahepatic bile duct tree, extrahepatic bile duct tree, and gallbladder. In some embodiments, liver diseases are bile acid-dependent liver diseases, including but not limited to inherited liver metabolic disorders; congenital errors in bile acid synthesis; congenital biliary abnormalities; biliary atresia; post-Kasai biliary atresia; post-liver transplant biliary atresia; neonatal hepatitis; neonatal cholestasis; genetic forms of cholestasis; cerebrotendinous xanthomatosis; secondary BA synthesis defects; Zellweger's syndrome; cystic fibrosis-related liver disease; alpha-1 antitrypsin deficiency; Alagille syndrome (ALGS); Byler syndrome; primary bile acid (BA) synthesis defects; progressive familial intrahepatic cholestasis (PFIC), including PFIC 1, PFIC 2, PFIC 3, and non-specific PFIC, post-biliary diversion PFIC, and post-liver transplant PFIC; benign recurrent intrahepatic cholestasis (BRIC), including BRIC1, BRIC2, and non-specific BRIC, post-biliary diversion BRIC, and post-liver transplant BRIC; autoimmune hepatitis; primary biliary cholangitis (PBC); hepatic fibrosis; non-alcoholic fatty liver disease (NAFLD); non-alcoholic steatohepatitis (NASH); portal hypertension; cholestasis; Down syndrome cholestasis; drug-induced cholestasis; intrahepatic cholestasis of pregnancy (jaundice of pregnancy); intrahepatic cholestasis; extrahepatic cholestasis; parenteral nutrition-associated cholestasis (PNAC); low phospholipid-associated cholestasis; lymphedema-cholestasis syndrome 1 (LSC1); primary sclerosing cholangitis (PSC); IgG4-related cholangitis; primary biliary cholangitis; cholelithiasis (gallstones); biliary stones; common bile duct stones; gallstone pancreatitis; Caroli disease; biliary tract malignancies; malignant neoplasms causing obstruction of the biliary tree; biliary strictures; AIDS cholangiopathy; ischemic cholangiopathy; pruritus caused by cholestasis or jaundice; pancreatitis; chronic autoimmune liver diseases leading to progressive cholestasis; hepatic steatosis; alcoholic hepatitis; acute fatty liver; pregnancy fatty liver; drug-induced hepatitis; iron overload; congenital bile acid synthesis defect type 1 (BAS type 1); drug-induced liver injury (DILI); hepatic fibrosis; congenital hepatic fibrosis; cirrhosis; Langerhans cell histiocytosis (LCH); neonatal ichthyosis-sclerosing cholangitis (NISCH); erythropoietic protoporphyria (EPP); idiopathic adulthood ductopenia (IAD); idiopathic neonatal hepatitis (INH); non-syndromic paucity of interlobular bile ducts (NS PILBD); North American Indian childhood cirrhosis (NAIC); hepatic sarcoidosis; amyloidosis; necrotizing enterocolitis; serum bile acid-induced toxicity, including arrhythmias (e.g., atrial fibrillation) due to abnormal patterns of serum bile acids, cardiomyopathy associated with cirrhosis ("cholecardia"), and skeletal muscle atrophy associated with cholestatic liver disease; polycystic liver disease; viral hepatitis (including hepatitis A, B, C, D, and E); hepatocellular carcinoma (liver cancer); cholangiocarcinoma; bile acid-related gastrointestinal cancers; and cholestasis caused by liver, biliary, and pancreatic tumors and neoplasms.

The therapeutically effective amount of the compounds of formulae (I)-(X) varies depending on the nature of the condition being treated, the desired duration of activity, and the age and condition of the subject, ultimately determined by the attending physician. Doses and intervals can be adjusted individually to provide plasma levels of the compounds of formulae (I)-(X) sufficient to maintain the desired therapeutic effect. The required dose can be administered as a single dose or as multiple doses at appropriate intervals.

The dosage of the compounds of formulae (I)-(X) used in the treatment methods provided in the following examples can be about 0.005 mg to about 500 mg/dose, about 0.05 mg to about 250 mg/dose, or about 0.5 mg to about 100 mg/dose. For example, the compounds of formulae (I)-(X) can be administered in amounts of about 0.005 mg, about 0.05 mg, about 0.5 mg, about 5 mg, about 10 mg, about 20 mg, about 30 mg, about 40 mg, about 50 mg, about 100 mg, about 150 mg, about 200 mg, about 250 mg, about 300 mg, about 350 mg, about 400 mg, about 450 mg, or about 500 mg per dose, including all doses between 0.005 mg and 500 mg.

The dosage of pharmaceutical compositions containing the compounds of formulae (I)-(X) can be about 1 ng/kg to about 200 mg/kg, about 1 µg/kg to about 100 mg/kg, or about 1 mg/kg to about 50 mg/kg. The dosage of the pharmaceutical compositions can be any dose, including but not limited to about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg, or more. The above doses are examples of general cases, but there may be individual cases where higher or lower doses are needed, and these are also within the scope of protection of this application. In practice, the physician determines the actual administration regimen that is most suitable for the subject, which may vary according to the age, weight, and response of the specific individual.

In the following embodiments, the compounds of formulae (I)-(X) can also be co-administered with other drugs to enhance the therapeutic effects of the compounds of formulae (I)-(X) or to enhance the therapeutic effects of the other drugs. It is expected that the compounds of formulae (I)-(X) may have a synergistic effect when used in combination with some drugs, for example, where the dose of one drug is lower than its therapeutically effective amount when used alone, or preferably, where the doses of both drugs are lower than their respective therapeutically effective amounts when used alone. Synergistic effects can also be manifested as producing new therapeutic effects, reducing treatment side effects, extending dosing intervals, shortening treatment duration, etc.

In the following embodiments, the term "subject" refers to animals, such as mammals, including (but not limited to) humans, rodents, primates, felines, canines, equines, bovines, swine, sheep, goats, mammalian laboratory animals, mammalian livestock, mammalian sports animals, and mammalian pets. The subject can be male or female and can be of any suitable age, including infants, juveniles, young adults, adults, and elderly subjects. In some instances, the subject refers to an individual who needs treatment for a disease or condition. In some instances, the treated subject may be a patient suffering from a condition associated with the treatment or at risk of developing the condition. In other instances, the subject is a healthy individual or an individual suffering from a disease not of concern. In specific instances, the subject is a human, such as a human patient. This term can generally be used interchangeably with "patient," "test subject," "treatment subject," etc.

The synthesis and bioactivities of the provided compounds and the procedures of bioactivity assays are detailed in the following examples.

### Example 1:

Synthetic procedures and structural characterizations of provided compounds of formulae **(I)-(IV)** and intermediates are described:
**General method OA:** To a solution of the corresponding amine (1 mmol), DMAP (0.3 mmol), and triethylamine (3 mmol) in anhydrous dichloromethane (5 mL) was added dropwise the corresponding acyl chloride (1.5 mmol) in an ice bath. Afterward, the reaction mixture was warmed to room temperature and stirred under N₂ for 12 hours. When the reaction was complete as monitored by TLC, the reaction mixture was diluted with water (6 mL) and extracted with DCM (3×6 mL). The combined organic layers were washed with brine and dried over anhydrous Na₂SO₄. The solvent was removed under reduced pressure to give the crude product, which was subsequently purified by silica gel column chromatography (PE/EA) to yield the target compound.
**General method OB:** Dissolved the corresponding amine (1 mmol) and the corresponding sulfonyl chloride (2 mmol) in pyridine (4 mL). The reaction system was heated to 120 °C under N₂, and stirred for 12 hours. After the reaction was complete as monitored by TLC, the reaction mixture was cooled to room temperature, diluted with water (6 mL), and extracted with ethyl acetate (3×12 mL). The combined organic phase was washed with saturated CuSO₄ solution (5 mL) and brine, and dried over anhydrous Na₂SO₄. The solvent was removed under reduced pressure to give the crude product, which was subsequently purified by silica gel column chromatography (PE/EA) to yield the target compound.
**General method OC:** The corresponding bromide (1 mmol), potassium carbonate (4 mmol), and the corresponding boronic acid (1.5 mmol) were added to a mixture of 1,4-dioxane (3 mL) and water (1 mL). To this mixture were added PdCl₂(PPh₃)₂ (0.1 mmol) and tricyclohexylphosphine (0.2 mmol) under nitrogen atmosphere. The reaction mixture was subsequently heated to 90 °C and stirred for 24 hours. After the reaction was complete as monitored by TLC, it was cooled to room temperature, diluted with water (5 mL), and extracted with ethyl acetate (3×5 mL). The combined organic layers were washed with brine (5 mL) and dried over anhydrous Na₂SO₄. The solvent was removed under reduced pressure to give the crude product, which was subsequently purified by silica gel column chromatography (PE/EA) to yield the target compound.
**General method OD:** To a solution of the corresponding amine (1 mmol) and triethylamine (1.1 mmol) in anhydrous dichloromethane (5 mL), was added slowly the corresponding anhydride (1.1 mmol) were at 0°C. The resulting mixture was warmed to room temperature and stirred overnight. After the reaction was complete as monitored by TLC, the mixture was diluted with water (6 mL) and extracted with dichloromethane (2×6 mL). The combined organic layers were washed with brine and dried over anhydrous Na₂SO₄. The solvent was removed under reduced pressure to give the crude product, which was subsequently purified by silica gel column chromatography (PE/EA) to yield the target compound.
**General method OE:** To a solution of the corresponding amine (1 mmol), carboxylic acid (1.1 mmol), and N,N-diisopropylethylamine (3 mmol) in anhydrous dichloromethane (5 mL), was added HATU (1.05 mmol) at 0 °C. The reaction mixture was warmed to room temperature and stirred overnight. After the reaction was complete as monitored by TLC, the mixture was diluted with water (6 mL) and extracted with dichloromethane (2×6 mL). The combined organic layers were washed with brine and dried over anhydrous Na₂SO₄. The solvent was removed under reduced pressure to give the crude product, which was subsequently purified by silica gel column chromatography (PE/EA) to yield the target compound.
**General method OF:** To a suspension of the corresponding amine (0.30 mmol) and potassium carbonate (0.59 mmol) in dry DMF, was added benzyl chloride (0.45 mmol) or benzyl bromide (0.45 mmol). The resulting mixture was stirred at room temperature for 2 h. After the reaction was complete as monitored by TLC, the reaction mixture was diluted with water (10 mL) and extracted with ethyl acetate (3×10 mL). The combined organic layers were washed with brine and dried over anhydrous Na₂SO₄. The solvent was removed under reduced pressure to give the crude product, which was subsequently purified by silica gel column chromatography (dichloromethane/methanol) to yield the target compound.
**General method OG:** To a suspension of the corresponding amine (0.30 mmol) and cesium carbonate (0.89 mmol) in toluene (5 mL) was added the corresponding bromide (0.59 mmol). The mixture was purged with N₂ for three times, and stirred for 5 min before Xantphos (0.02 mmol) and Pd₂(dba)₃ (0.02 mmol) were added. The resulting mixture was heated to 90 °C and stirred for 3 h. After the reaction was complete as monitored by TLC, the reaction mixture was diluted with water (10 mL) and extracted with ethyl acetate (3×10 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography (PE/EA) to yield the target compound.
**General method OH:** To a solution of vinyl sulfonamide (0.15 mmol) and the corresponding oxime (0.08 mmol) in anhydrous dichloromethane (10 mL) was added dropwise sodium hypochlorite solution (0.15 mmol) at 0°C, and then the mixture was warmed to room temperature and stirred for 1 hour. After the reaction was complete as monitored by TLC, the reaction mixture was diluted with water (10 mL) and extracted with dichloromethane (3×10 mL). The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.
**General method OI:** To a solution of the corresponding amine (0.2 mmol), DMAP (7.2 mg, 0.06 mmol), and triethylamine (0.6 mmol) in anhydrous dichloromethane (5 mL) was added dropwise the corresponding sulfonyl chloride (0.3 mmol) at 0°C. The mixture was then warmed to room temperature and stirred for 8 hours. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.

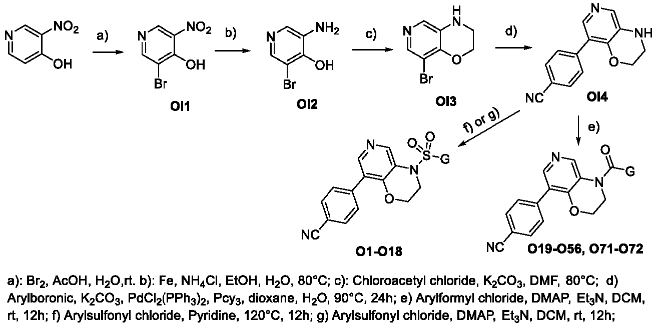

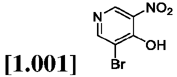

### 3-Bromo-5-nitropyridin-4-ol (OI1)

4-Hydroxy-5-nitropyridine (50 mg, 0.36 mmol) was dissolved in a 50% AcOH aqueous solution (10 mL). Liquid bromine (0.02 mL, 0.36 mmol) was slowly added at 0 °C. The mixture was then warmed to room temperature and stirred for 1 hour. After the reaction was complete as monitored by TLC, the reaction mixture was filtered. The filter cake was washed with water and dried at 60 °C to yield a white solid compound of **OI1** (62 mg, 80%). ¹H-NMR (400 MHz, DMSO*-d₆*): *δ* 12.75 (s, 1H), 8.82 (s, 1H), 8.37 (s, 1H).

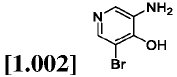

### 3-Amino-5-bromopyridin-4-ol (OI2)

To a solution of **OI1** (200 mg, 0.93 mmol) in a mixture of ethanol (60 mL) and water (20 mL) were added iron powder (308 mg, 5.52 mmol) and ammonium chloride (98 mg, 1.84 mmol). The mixture was refluxed at 80 °C under N₂ for 1 hour. After the reaction was complete as monitored by TLC, the reaction mixture was cooled to room temperature and filtered through diatomite. The filter cake was washed with cold ethanol, and the filtrate was concentrated under reduced pressure to yield **OI2** (104 mg, 60%) as a gray solid. ¹H-NMR (400 MHz, DMSO-d6): δ 11.51 (s, 1H), 7.81 (s, 1H), 7.14 (s, 1H), 4.80 (s, 2H).

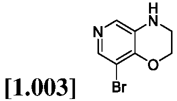

### 8-Bromo-3,4-dihydro-2H-pyrido[4,3-b][1,4]oxazine (OI3)

To a solution of **OI2** (200 mg, 1.06 mmol) and potassium carbonate (441 mg, 3.20 mmol) in DMF (40 mL) was added slowly 1,2-dibromoethane (199 mg, 1.06 mmol) under N₂ at room temperature. The reaction mixture was heated to 80°C and stirred for 24 hours. After the reaction was complete as monitored by TLC, the mixture was cooled to room temperature, diluted with water (20 mL) and extracted with EA (3×20 mL). The combined organic layers were washed with water (3×5 mL) and brine (20 mL). Subsequently. The organic phase was dried over anhydrous Na₂SO₄ and the solvent was removed under reduced pressure. The resulting crude product was purified by silica gel column chromatography (PE/EA = 1/1) to give **OI3** (68 mg, 30% yield) as a yellow solid. ¹H-NMR (400 MHz, CDCl₃): *δ* 7.94 (s, 1H), 7.75 (s, 1H), 4.43 (s, 1H), 3.69-3.56 (m, 2H), 3.22-3.07 (m, 2H).

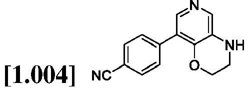

### 4-(3,4-Dihydro-2H-pyrido[4,3-b][1,4]oxazin-8-yl)benzonitrile (OI4)

Following the general method OC, synthesized with **OI3** (500 mg, 2.34 mmol), potassium carbonate (1.29 g, 9.346 mmol), 4-cyanophenylboronic acid (514 mg, 3.5 mmol), PdCl₂(PPh₃)₂ (164 mg, 0.23 mmol) and tricyclohexylphosphine (131 mg, 0.47 mmol) in a mixed solvent of 1,4-dioxane (15 mL) and water (5 mL). The crude product was purified by silica gel column chromatography (PE/EA = 1/2) to give **OI4** (500 mg, 90% yield) as a light yellow solid. ¹H-NMR (400 MHz, DMSO-d6): δ 7.94-7.82 (m, 3H), 7.78-7.66 (m, 3H), 6.17 (s, 1H), 4.32-4.12 (m, 2H), 3.35-3.29 (m, 2H).

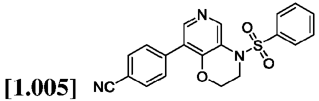

### 4-(4-(Phenylsulfonyl)-3,4-dihydro-2H-pyrido[4,3-b][1,4]oxazin-8-yl)benzonitrile (O1)

Following the general method OB, synthesized with **OI4** (50 mg, 0.21 mmol) and benzenesulfonyl chloride (74 mg, 0.42 mmol) to give **O1** (58 mg, 73% yield) as a white solid after chromatographic purification (PE/EA = 1/2), mp: 181.0-182.0 °C. ¹H-NMR (400 MHz, CDCl₃): *δ* 9.00 (s, 1H), 8.27 (s, 1H), 7.74 (d, *J =* 7.6 Hz, 2H), 7.69 (d, *J* = 8.3 Hz, 2H), 7.64 (s, 1H), 7.56-7.49 (m, 4H), 3.91 (s, 2H), 3.89 (s, 2H); ¹³C-NMR (101 MHz, CDCl₃): *δ* 149.88, 146.73, 146.07, 138.44, 138.08, 133.93, 132.06 (2C), 130.07 (2C), 129.67 (2C), 127.37 (2C), 124.21, 121.94, 118.56, 111.77, 64.06, 43.31. HRMS (ESI): m/z calcd. for C₂₀H₁₆N₃O₃S⁺ [M+H]⁺: 378.0907, found 378.0908.

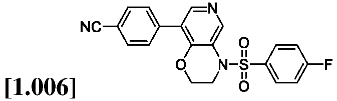

### 4-(4-((4-Fluorophenyl)sulfonyl)-3,4-dihydro-2H-pyrido[4,3-b][1,4]oxazin-8-yl)benzonitrile (O2)

Following the general method OB, synthesized with **OI4** (50 mg, 0.21 mmol) and 4-fluorobenzenesulfonyl chloride (81 mg, 0.42 mmol) to give **O2** (60 mg, 72% yield) as a light yellow solid after chromatographic purification (PE/EA = 2/1), mp: 184.0-185.0 °C. ¹H-NMR (400 MHz, CDC13): δ 9.01 (s, 1H), 8.31 (s, 1H), 7.78 (s, 2H), 7.69 (s, 2H), 7.55 (s, 2H), 7.21 (s, 2H), 3.93 (s, 4H); ¹³C-NMR (101 MHz, CDC13): δ 167.02, 164.46 (¹*J*_{CF} = 257.5), 149.84, 146.98, 146.04, 138.35, 134.18, 134.15, 132.07 (2C), 130.23, 130.14 (³*J*_{CF} = 9.5), 130.07 (2C), 124.29, 121.74, 118.54, 117.16, 116.93 (²*J*_{CF} = 22.6), 111.82, 64.13, 43.37. HRMS (ESI): m/z calcd. for C₂₀H₁₅N₃O₃S⁺ [M+H]⁺: 396.0813, found 396.0809.

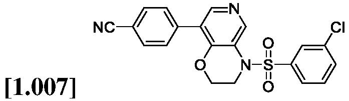

### 4-(4-((3-Fluorophenyl)sulfonyl)-3,4-dihydro-2H-pyrido[4,3-b][1,4]oxazin-8-yl)benzonitrile (O3)

Following the general method OB, synthesized with **OI4** (50 mg, 0.21 mmol) and 3-toluenesulfonyl chloride (81.50 mg, 0.42 mmol) to give **O3** (55 mg, 66% yield) as a white solid after chromatographic purification (PE/EA = 2/1), mp: 165.0-166.0 °C. ¹H-NMR (400 MHz, CDCl₃): *δ* 8.97 (s, 1H), 8.29 (s, 1H), 7.69 (d, *J* = 8.4 Hz, 2H), 7.53 (s, 4H), 7.44 (s, 1H), 7.34 (s, 1H), 3.94 (s, 4H); ¹³C-NMR (101 MHz, CDCl₃): *δ* 163.83, 161.31 (¹*J*_{CF} = 253.7), 149.81, 147.08, 145.92, 140.10, 140.04 (³*J*_{CF} = 7.8), 138.33, 132.07 (2C), 131.58, 131.50 (³*J*_{CF} = 7.8), 130.08 (2C), 124.26, 123.20, 123.16 (⁴*J*_{CF} = 3.5), 121.63, 121.33, 121.12 (²*J*_{CF} = 21.2), 118.54, 114.87, 114.62 (²*J*_{CF} = 21.2), 111.82, 64.19, 43.44. HRMS (ESI): m/z calcd. for C₂₀H₁₅FN₃O₃S⁺ [M+H]⁺: 396.0813, found 396.0709.

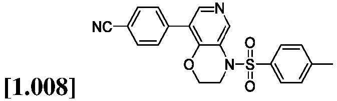

### 4-(4-Tosyl-3,4-dihydro-2H-pyrido[4,3-b][1,4]oxazin-8-yl)benzonitrile (O4)

Following the general method OB, synthesized with **OI4** (50 mg, 0.21 mmol) and 4-toluenesulfonyl chloride (80 mg, 0.42 mmol) to give **O4** (62 mg, yield 80%) as a white solid after chromatographic purification (PE/EA = 2/1), mp: 141.0-142.0 °C. ¹H-NMR (400 MHz, CDCl₃): *δ* 9.00 (s, 1H), 8.27 (s, 1H), 7.69 (d, *J* = 8.2 Hz, 2H), 7.61 (d, *J* = 8.2 Hz, 2H), 7.54 (d, *J* = 8.2 Hz, 2H), 7.30 (d, *J* = 8.2 Hz, 2H), 3.89 (s, 4H), 2.42 (s, 3H); ¹³C-NMR (101 MHz, CDCl₃); *δ* 149.86, 146.61, 146.13, 145.06, 138.50, 135.06, 132.04 (2C), 130.26 (2C), 130.07 (2C), 127.41 (2C), 124.12, 122.04, 118.56, 111.73, 64.04, 43.27, 21.62. HRMS (ESI): m/z calcd. for C₂₁H₁₈N₃O₃S⁺ [M+H]⁺: 392.1063, found 392.1058.

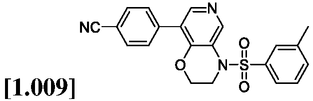

### 4-(4-(m-Tolylsulfonyl)-3,4-dihydro-2H-pyrido[4,3-b][1,4]oxazin-8-yl)benzonitrile (O5)

Following the general method OB, synthesized with **OI4** (50 mg, 0.21 mmol) and 3-methylbenzenesulfonyl chloride (80 mg, 0.42 mmol) to give **O5** (40 mg, 49% yield) as a pale yellow solid after chromatographic purification (PE/EA = 2/1), mp: 153.0-154.0 °C. ¹H-NMR (400 MHz, CDCl₃): *δ* 8.98 (s, 1H), 8.27 (s, 1H), 7.69 (d, *J* = 6.9 Hz, 2H), 7.53 (d, *J* = 13.7 Hz, 4H), 7.41 (d, *J =* 27.6 Hz, 2H), 3.91 (s, 4H), 2.39 (s, 3H); ¹³C-NMR (101 MHz, CDCl₃): *δ* 148.89, 145.67, 145.07, 139.07, 137.51, 136.97, 133.73, 131.07 (2C), 129.07 (2C), 128.50, 126.63, 123.54, 123.16, 121.01, 117.58, 110.75, 63.13, 42.30, 20.35. HRMS (ESI): m/z calcd. for C₂₁H₁₈N₃O₃S⁺ [M+H]⁺: 392.1063, found 392.1042.

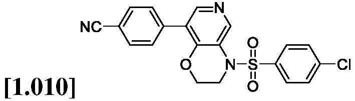

### 4-(4-((4-Chlorophenyl)sulfonyl)-3,4-dihydro-2H-pyrido[4,3-b][1,4]oxazin-8-yl)benzonitrile (O6)

Following the general method OB, synthesized with **OI4** (50 mg, 0.21 mmol) and 4-chlorobenzenesulfonyl chloride (88 mg, 0.42 mmol) to give **O6** (51 mg, 59% yield) as a white solid after chromatographic purification (PE/EA = 2/1), mp: 168.0-169.0 °C. ¹H-NMR (400 MHz, CDCl₃): *δ* 8.98 (s, 1H), 8.28 (s, 1H), 7.69 (d, *J* = 14.3 Hz, 4H), 7.55 (d, *J* = 8.4 Hz, 2H), 7.49 (d, *J* = 8.6 Hz, 2H), 3.94 (d, *J* = 3.3 Hz, 4H); ¹³C-NMR (101 MHz, CDCl₃): *δ* 149.82, 147.00, 145.93, 140.69, 138.31, 136.55, 132.07 (2C), 130.08 (2C), 130.00 (2C), 128.76 (2C), 124.26, 121.69, 118.54, 111.82, 64.16, 43.40. HRMS (ESI): m/z calcd. for C₂₀H₁₅ClN₃O₃S⁺ [M+H]⁺: 412.0517, found 412.0511.

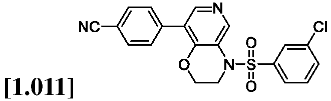

### 4-(4-((3-Chlorophenyl)sulfonyl)-3,4-dihydro-2H-pyrido[4,3-b][1,4]oxazin-8-yl)benzonitrile (O7)

Following the general method OB, synthesized with **OI4** (50 mg, 0.21 mmol) and 3-chlorobenzenesulfonyl chloride (88 mg, 0.42 mmol) to give **O7** (66 mg, 80% yield) as a white solid after chromatographic purification (PE/EA = 2/1), mp: 130.0-131.0 °C. ¹H-NMR (400 MHz, CDCl₃): *δ* 8.97 (s, 1H), 8.30 (s, 1H), 7.74 (s, 1H), 7.70 (d, *J* = 8.1 Hz, 2H), 7.62 (s, 2H), 7.55 (d, *J* = 8.3 Hz, 2H), 7.47 (s, 1H), 3.98 (s, 2H), 3.93 (s, 2H); ¹³C-NMR (101 MHz, CDCl₃): *δ* 149.03, 145.83, 144.63, 138.84, 137.22, 135.03, 133.09, 131.11 (2C), 129.96, 129.10 (2C), 126.32, 124.47, 120.68, 120.50, 117.53, 110.92, 63.33, 42.42. HRMS (ESI): m/z calcd. for C₂₀H₁₅ClN₃O₃S⁺ [M+H]⁺: 412.0517, found 412.0517.

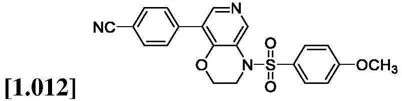

### 4-(4-((4-Methoxyphenyl)sulfonyl)-3,4-dihydro-2H-pyrido[4,3-b][1,4]oxazin-8-yl)benzonitrile (O8)

Following the general method OB, synthesized with **OI4** (50 mg, 0.21 mmol) and 4-methoxylbenzenesulfonyl chloride (88 mg, 0.42 mmol) to give **O8** (43 mg, 50% yield) as a white solid after chromatographic purification (PE/EA = 2/1), mp: 148.0-149.0 °C.¹H-NMR (400 MHz, CDCl₃): *δ* 8.99 (s, 1H), 8.27 (s, 1H), 7.68 (dd, *J* = 12.8, 8.5 Hz, 4H), 7.55 (d, *J* = 8.3 Hz, 2H), 6.96 (d, *J* = 8.8 Hz, 2H), 3.90 (s, 4H), 3.86 (s, 3H); ¹³C-NMR (101 MHz, CDCl₃): *δ* 163.85, 152.91, 150.05, 146.38, 146.02, 138.44, 132.07 (2C), 130.09 (2C), 129.64 (2C), 129.39, 124.69, 118.57, 114.81 (2C), 111.80, 64.11, 55.73, 43.24. HRMS (ESI): m/z calcd. for C₂₁H₁₈N₃O₄S⁺ [M+H]⁺: 408.1013, found 408.1005.

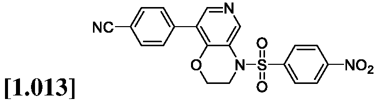

### 4-(4-((4-Nitrophenyl)sulfonyl)-3,4-dihydro-2H-pyrido[4,3-b][1,4]oxazin-8-yl)benzonitrile (O9)

Following the general method OB, synthesized with **OI4** (50 mg, 0.21 mmol) and 4-nitrobenzenesulfonyl chloride (93 mg, 0.42 mmol) to give **O8** (33 mg, 37% yield) as a yellow solid after chromatographic purification (PE/EA = 2/1), mp: 222.0-227.0 °C. ¹H-NMR (400 MHz, CDCl₃): *δ* 8.99 (s, 1H), 8.37 (d, *J* = 8.8 Hz, 2H), 8.31 (s, 1H), 7.97 (d, *J* = 8.8 Hz, 2H), 7.70 (d, *J* = 8.3 Hz, 2H), 7.54 (d, *J =* 8.3 Hz, 2H), 4.00 (s, 4H); ¹³C-NMR (101 MHz, CDCl₃): *δ* 150.72, 149.88, 149.31, 147.25, 145.36, 143.80, 137.99, 135.29, 132.13 (2C), 130.08 (2C), 128.67 (2C), 124.88 (2C), 118.46, 112.03, 64.46, 43.62. HRMS (ESI): m/z calcd. for C₂₀H₁₅N₄O₅S⁺ [M+H]⁺: 423.0758, found 423.0747.

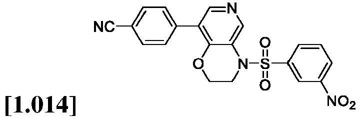

### 4-(4-((3-Nitrophenyl)sulfonyl)-3,4-dihydro-2H-pyrido[4,3-b][1,4]oxazin-8-yl)benzonitrile (O10)

Following the general method OB, synthesized with **OI4** (50 mg, 0.21 mmol) and 3-nitrobenzenesulfonyl chloride (93 mg, 0.42 mmol) to give **O10** (63 mg, 70% yield) as a yellow solid after chromatographic purification (PE/EA = 2/1), mp: 209.0-212.0 °C. ¹H-NMR (400 MHz, CDCl₃): *δ* 8.96 (s, 1H), 8.61 (s, 1H), 8.48 (s, 1H), 8.31 (s, 1H), 8.10 (s, 1H), 7.77 (s, 1H), 7.70 (d, *J* = 8.2 Hz, 2H), 7.54 (d, *J* = 8.3 Hz, 2H), 4.05 (s, 2H), 4.01 (s, 2H); ¹³C-NMR (101 MHz, CDCl₃): *δ* 149.91, 148.56, 147.15, 145.22, 142.53, 140.46, 138.06, 137.98, 132.79, 132.12 (2C), 131.18, 130.08 (2C), 128.27, 122.41, 118.47, 111.99, 64.62, 43.63. HRMS (ESI): m/z calcd. for C₂₀H₁₅N₄O₅S⁺ [M+H]⁺: 423.0758, found 423.0755.

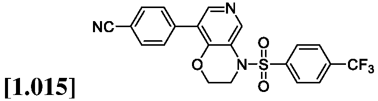

### 4-(4-((4-(Trifluoromethyl)phenyl)sulfonyl)-3,4-dihydro-2H-pyrido[4,3-b][1,4]oxazin-8-yl)benz onitrile (O11)

Following the general method OB, synthesized with **OI4** (50 mg, 0.21 mmol) and 4-trifluoromethylbenzenesulfonyl chloride (103 mg, 0.42 mmol) to give **O11** (41 mg, 42% yield) as a white solid after chromatographic purification (PE/EA = 2/1), mp: 192.0-193.0 °C. ¹H-NMR (400 MHz, CDCl₃): *δ* 8.98 (s, 1H), 8.30 (s, 1H), 7.90 (d, *J* = 8.2 Hz, 2H), 7.80 (d, *J* = 8.3 Hz, 2H), 7.70 (d, *J* = 8.3 Hz, 2H), 7.54 (d, *J* = 8.4 Hz, 2H), 3.97 (s, 4H); ¹³C-NMR (101 MHz, CDCl₃): *δ* 149.80, 147.12, 145.60, 141.72, 138.19, 135.70, 135.36 (²*J*_{CF} = 33.3), 132.10 (2C), 130.08 (2C), 127.93 (2C), 126.89, 126.85 (³*J*_{CF} = 3.5), 126.82, 126.78 (³*J*_{CF} = 3.6), 124.38, 124.22, 121.64, 121.50 (¹*J*_{CF} = 273.3), 118.50, 111.90, 64.38, 43.51. HRMS (ESI): m/z calcd. for C₂₁H₁₅F₃N₃O₃S⁺ [M+H]⁺: 446.0781, found 446.0766.

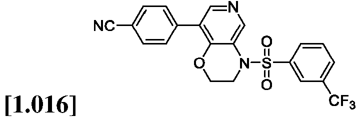

### 4-(4-((3-(Trifluoromethyl)phenyl)sulfonyl)-3,4-dihydro-2H-pyrido[4,3-b][1,4]oxazin-8-yl)benz onitrile (012)

Following the general method OB, synthesized with **OI4** (50 mg, 0.21 mmol) and 3-trifluoromethylbenzenesulfonyl chloride (103 mg, 0.42 mmol) to give **012** (52 mg, 55% yield) as a white solid after chromatographic purification (PE/EA = 2/1), mp: 64.0-66.0 °C. ¹H-NMR (400 MHz, CDCl₃): *δ* 8.98 (s, 1H), 8.31 (s, 1H), 7.94 (d, *J* = 33.6 Hz, 3H), 7.71 (d, *J* = 7.7 Hz, 3H), 7.53 (d, *J* = 8.1 Hz, 2H), 3.97 (s, 4H); ¹³C-NMR (101 MHz, CDCl₃): *δ* 150.00, 147.15, 145.80, 139.50, 138.20, 137.10, 132.60, 132.26 (²*J*_{CF} = 34.0), 132.10 (2C), 130.56 (2C), 130.51, 130.48 (³*J*_{CF} = 3.6), 130.06 (2C), 124.34, 124.30 (³*J*_{CF} = 3.7), 124.16, 121.45 (¹*J*_{CF} = 273.2), 118.50, 113.32, 111.92, 64.25, 43.47. HRMS (ESI): m/z calcd. for C₂₁H₁₅F₃N₃O₃S⁺ [M+H]⁺: 446.0781, found 446.0772.

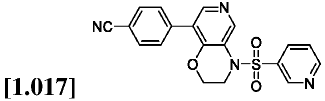

### 4-(4-(Pyridin-3-ylsulfonyl)-3,4-dihydro-2H-pyrido[4,3-b][1,4]oxazin-8-yl)benzonitrile (013)

Following the general method OB, synthesized with **OI4** (50 mg, 0.21 mmol) and 3-pyridinylsulfonyl chloride (93 mg, 0.42 mmol) to give **O13** (45 mg, 57% yield) as a white solid after chromatographic purification (PE/EA = 2/1), mp: 181.0-183.0 °C. ¹H-NMR (400 MHz, CDCl₃): *δ* 9.00 (s, 2H), 8.86 (s, 1H), 8.30 (s, 1H), 8.05 (s, 1H), 7.70 (d, *J* = 8.3 Hz, 2H), 7.54 (d, *J* = 8.3 Hz, 2H), 7.49 (s, 1H), 4.01 (s, 2H), 3.98 (s, 2H); ¹³C-NMR (101 MHz, CDCl₃): *δ* 154.41, 152.79, 150.00, 148.07, 147.07, 145.52, 143.07, 138.15, 135.05, 132.14 (2C), 130.13 (2C), 124.20, 121.48, 118.54, 111.98, 64.47, 43.48. HRMS (ESI): m/z calcd. for C₁₉H₁₅N₄O₃S⁺ [M+H]⁺: 379.0859, found 379.0847.

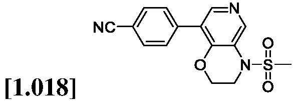

### 4-(4-(Methylsulfonyl)-3,4-dihydro-2H-pyrido[4,3-b][1,4]oxazin-8-yl)benzonitrile (014)

Following the general method OB, synthesized with **OI4** (50 mg, 0.21 mmol) and methanesulfonic anhydride (40 mg, 0.23 mmol), triethylamine (23 mg, 0.23 mmol) to give **O14** (20 mg, 30% yield) as a white solid after chromatographic purification (PE/EA = 2/1), mp: 175.0-177.0 °C. ¹H-NMR (400 MHz, CDCl₃): *δ* 8.86 (s, 1H), 8.28 (s, 1H), 7.74 (d, *J* = 8.1 Hz, 2H), 7.62 (d, *J* = 8.2 Hz, 2H), 4.41 (s, 2H), 3.94 (s, 2H), 3.12 (s, 3H); ¹³C-NMR (101 MHz, CDCl₃): *δ* 149.55, 146.21, 144.00, 138.34, 133.73, 132.14 (2C), 131.21, 130.14 (2C), 118.56, 111.93, 65.62, 43.29, 40.03. HRMS (ESI): m/z calcd. for C₁₅H₁₄N₃O₃S⁺ [M+H]⁺: 316.0750, found 316.0748.

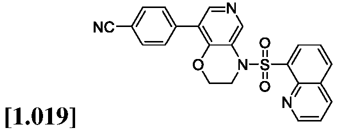

### 4-(4-(Quinolin-8-ylsulfonyl)-3,4-dihydro-2H-pyrido[4,3-b][1,4]oxazin-8-yl)benzonitrile (O15)

Following the general method OB, synthesized with **OI4** (100 mg, 0.42 mmol) and 8-quinolinylsulfonyl chloride (192 mg, 0.84 mmol) to give **O15** (78 mg, 43% yield) as a yellow solid after chromatographic purification (PE/EA = 2/1), mp: 215.0-217.0 °C. ¹H-NMR (400 MHz, CDCl₃): *δ* 8.94 (s, 1H), 8.81 (s, 1H), 8.72 (s, 1H), 8.27 (s, 1H), 8.11 (s, 2H), 7.73 (d, *J =* 7.7 Hz, 3H), 7.53 (d, *J =* 14.0 Hz, 3H), 4.37 (d, *J =* 9.8 Hz, 4H); ¹³C-NMR (101 MHz, CDCl₃): *δ* 151.29, 148.90, 144.84, 143.76, 142.29, 138.80, 136.76, 136.41, 134.68, 133.82, 132.00 (2C), 130.05 (2C), 129.10, 125.78, 124.15, 123.41, 122.46, 118.63, 111.58, 66.12, 44.32. HRMS (ESI): m/z calcd. for C₂₃H₁₇N₄O₃S⁺ [M+H]⁺: 429.1016, found 429.1001.

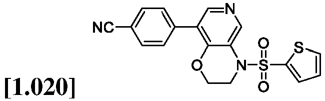

### 4-(4-(Naphthalen-1-ylsulfonyl)-3,4-dihydro-2H-pyrido[4,3-b][1,4]oxazin-8-yl)benzonitrile (017)

Following the general method OB, synthesized with **OI4** (50 mg, 0.21 mmol) and 1-naphthalenylsulfonyl chloride (95 mg, 0.42 mmol) to give **O17** (30 mg, 33% yield) as a white solid after chromatographic purification (PE/EA = 2/1), mp: 189.0-190.0 °C. ¹H-NMR (400 MHz, CDCl₃): *δ* 8.82 (s, 1H), 8.34 (s, 2H), 8.15 (s, 1H), 7.95 (s, 1H), 7.67 (d, *J* = 7.5 Hz, 2H), 7.60 (d, *J* = 7.6 Hz, 2H), 7.52 (s, 1H), 7.42 (d, *J* = 7.8 Hz, 2H), 7.26 (s, 1H), 3.92 (s, 2H), 3.85 (s, 2H); ¹³C-NMR (101 MHz, DMSO*-d₆*): *δ* 150.02, 146.73, 146.03, 145.45, 138.34, 137.69, 135.61, 134.04, 132.69, 132.16 (2C), 131.10, 130.05 (2C), 129.32, 128.24, 127.44, 127.29, 124.95, 123.81, 118.62, 110.53, 64.31, 42.34. HRMS (ESI): m/z calcd. for C₂₄H₁₈N₃O₃S⁺ [M+H]⁺: 428.1063, found 428.1053.

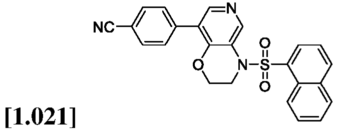

### 4-(4-(Naphthalen-2-ylsulfonyl)-3,4-dihydro-2H-pyrido[4,3-b][1,4]oxazin-8-yl)benzonitrile (018)

Following the general method OB, synthesized with **OI4** (50 mg, 0.21 mmol) and 2-naphthalenylsulfonyl chloride (95 mg, 0.42 mmol) to give **018** (15 mg, 17% yield) as a light yellow solid after chromatographic purification (PE/EA = 2/1), mp: 177.0-178.0 °C. ¹H-NMR (400 MHz, CDCl₃): *δ* 9.07 (s, 1H), 8.37 (s, 1H), 8.27 (s, 1H), 7.93 (d, *J* = 22.5 Hz, 3H), 7.66 (d, *J* = 34.9 Hz, 5H), 7.50 (d, *J* = 8.3 Hz, 2H), 3.97 (s, 2H), 3.89 (s, 2H); ¹³C-NMR (101 MHz, CDCl₃): *δ* 149.89, 146.68, 146.02, 138.39, 135.14, 134.85, 132.05, 132.00 (2C), 130.07, 130.05 (2C), 129.57, 129.32, 129.18, 128.10, 128.01, 124.21, 122.03, 121.94, 118.55, 111.69, 64.19, 43.36. HRMS (ESI): m/z calcd. for C₂₄H₁₈N₃O₃S⁺ [M+H]⁺: 428.1063, found 428.1055.

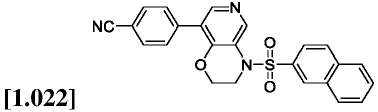

### 4-(4-(Thiophen-2-ylsulfonyl)-3,4-dihydro-2H-pyrido[4,3-b][1,4]oxazin-8-yl)benzonitrile(O16)

Following the general method OB, synthesized with **OI4** (50 mg, 0.21 mmol) and 2-thiophenylsulfonyl chloride (77 mg, 0.42 mmol) to give **O16** (27 mg, 33% yield) as a yellow solid after chromatographic purification (PE/EA = 2/1), mp: 147.0-148.0 °C. ¹H-NMR (400 MHz, CDCl₃): *δ* 9.02 (s, 1H), 8.29 (s, 1H), 7.70 (d, *J* = 10.3 Hz, 2H), 7.64 (d, *J* = 5.0 Hz, 1H), 7.55 (d, *J* = 8.1 Hz, 3H), 7.12 (s, 1H), 3.94 (s, 4H); ¹³C-NMR (101 MHz, CDCl₃): *δ* 149.99, 147.03, 146.36, 138.41, 137.83, 133.72, 133.38, 132.06 (2C), 130.08 (2C), 128.00, 124.20, 121.52, 118.55, 111.77, 63.77, 43.53. HRMS (ESI): m/z calcd. for C₁₈H₁₄N₃O₃S₂⁺ [M+H]⁺: 384.0471, found 384.0468.

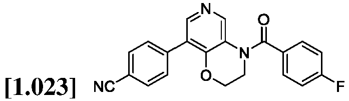

### 4-(4-(4-Fluorobenzoyl)-3,4-dihydro-2H-pyrido[4,3-b][1,4]oxazin-8-yl)benzonitrile (019)

Following the general method OA, synthesized with **OI4** (50 mg, 0.21 mmol), 4-fluorobenzoyl chloride (50 mg, 0.315 mmol), DMAP (8 mg, 0.063 mmol) and triethylamine (64 mg, 0.63 mmol) to give **O19** (53 mg, 70% yield) as a white solid after chromatographic purification (PE/EA = 2/1), mp: 191.0-193.0 °C. ¹H-NMR (400 MHz, CDCl₃): *δ* 8.23 (d, *J* = 31.0 Hz, 2H), 7.74 (d, *J* = 8.3 Hz, 2H), 7.65 (d, *J* = 8.3 Hz, 2H), 7.58 (d, *J* = 13.9 Hz, 2H), 7.13 (d, *J* = 17.1 Hz, 2H), 4.47 (s, 2H), 4.07 (s, 2H); ¹³C-NMR (101 MHz, CDCl₃): *δ* 167.87, 165.64, 163.13 (¹*J*_{CF} = 253.30), 149.09, 146.00, 145.60, 138.59, 132.10 (2C), 130.99, 130.90 (³*J*_{CF} = 8.84), 130.10 (2C), 129.95, 129.92 (⁴*J*_{CF} = 3.42), 124.12, 118.60, 116.20, 115.98 (²*J*_{CF} = 22.07), 115.41, 111.75, 67.32, 42.13. HRMS (ESI): m/z calcd. for C₂₁H₁₅FN₃O₂⁺ [M+H]⁺: 360.1143, found 360.1142.

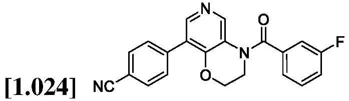

### 4-(4-(3-Fluorobenzoyl)-3,4-dihydro-2H-pyrido[4,3-b][1,4]oxazin-8-yl)benzonitrile (O20)

Following the general method OA, synthesized with **OI4** (50 mg, 0.21 mmol), 3-fluorobenzoyl chloride (50 mg, 0.315 mmol), DMAP (8 mg, 0.063 mmol) and triethylamine (64 mg, 0.63 mmol) to give **O20** (55 mg, 72% yield) as a white solid after chromatographic purification (PE/EA = 2/1), mp: 167.0-169.0 °C. ¹H-NMR (400 MHz, CDCl₃): *δ* 8.35 (s, 1H), 8.21 (s, 1H), 7.74 (d, *J* = 8.4 Hz, 2H), 7.65 (d, *J* = 8.4 Hz, 2H), 7.42 (s, 1H), 7.31 (d, *J* = 18.7 Hz, 2H), 7.21 (s, 1H), 4.47 (s, 2H), 4.07 (s, 2H); ¹³C-NMR (101 MHz, CDCl₃): *δ* 167.52, 163.84, 161.36 (¹J_{CF} = 249.4), 149.19, 146.21, 145.55, 138.55, 136.08, 136.01, 132.13 (2C), 130.76, 130.68 (³*J*_{CF} = 8.1), 130.13 (2C), 124.03, 124.00 (⁴*J*_{CF} = 3.2), 118.65, 118.61, 118.44 (²*J*_{CF} = 21.1), 115.72, 115.49 (²*J*_{CF} = 22.9), 111.81, 67.27, 42.28. HRMS (ESI): m/z calcd. for C₂₁H₁₅FN₃O₂⁺ [M+H]⁺: 360.1143, found 360.1135.

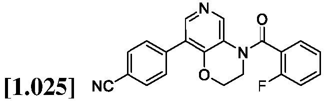

### 4-(4-(2-Fluorobenzoyl)-3,4-dihydro-2H-pyrido[4,3-b][1,4]oxazin-8-yl)benzonitrile (O21)

Following the general method OA, synthesized with **OI4** (50 mg, 0.21 mmol), 2-fluorobenzoyl chloride (50 mg, 0.315 mmol), DMAP (8 mg, 0.063 mmol) and triethylamine (64 mg, 0.63 mmol) to give **O21** (30 mg, 42% yield) as a white solid after chromatographic purification (PE/EA = 2/1), mp: 85.0-87.0 °C. ¹H-NMR (400 MHz, DMSO*-d₆*): *δ* 9.20 (s, 1H), 8.23 (s, 1H), 7.93 (d, *J* = 8.2 Hz, 2H), 7.78 (d, *J =* 8.3 Hz, 2H), 7.69-7.51 (m, 2H), 7.33 (dt, *J =* 24.2, 7.6 Hz, 2H), 4.43 (s, 2H), 3.98 (dd, *J* = 35.6, 28.5 Hz, 2H); ¹³C-NMR (101 MHz, DMSO*-d₆*): *δ* 169.76, 159.01, 156.55 (¹*J*_{CF} = 247.7), 149.43, 146.04, 144.41, 138.71, 134.70, 132.79, 132.71 (³*J*_{CF} = 7.8), 132.22 (2C), 130.25 (2C), 125.20, 125.16 (⁴*J*_{CF} = 3.1), 123.39, 123.21 (²*J*_{CF} = 20.4), 123.05, 118.74, 116.16, 115.95 (²*J*_{CF} = 21.1), 115.10, 110.50, 66.96, 47.85. HRMS (ESI): m/z calcd. for C₂₁H₁₅FN₃O₂⁺ [M+H]⁺: 360.1143, found 360.1139.

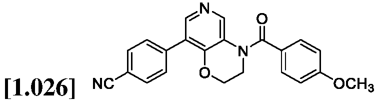

### 4-(4-(4-Methoxybenzoyl)-3,4-dihydro-2H-pyrido[4,3-b][1,4]oxazin-8-yl)benzonitrile (O22)

Following the general method OA, synthesized with **OI4** (50 mg, 0.21 mmol), 4-methoxybenzoyl chloride (54 mg, 0.315 mmol), DMAP (8 mg, 0.063 mmol) and triethylamine (64 mg, 0.63 mmol) to give **O22** (50 mg, 64% yield) as a white solid after chromatographic purification (PE/EA = 2/1), mp: 176.0-178.0 °C. ¹H-NMR (400 MHz, CDCl₃): *δ* 8.28 (s, 1H), 8.17 (s, 1H), 7.73 (d, *J* = 8.3 Hz, 2H), 7.65 (d, *J* = 8.2 Hz, 2H), 7.54 (d, *J* = 8.7 Hz, 2H), 6.92 (d, *J* = 8.7 Hz, 2H), 4.45 (s, 2H), 4.07 (s, 2H), 3.85 (s, 3H); ¹³C-NMR (101 MHz, CDCl₃): *δ* 168.72, 162.17, 148.97, 145.66, 145.54, 138.76, 132.07 (2C), 130.71 (2C), 130.10 (2C), 125.73, 124.37, 124.01, 118.65, 114.09 (2C), 111.66, 67.36, 55.42, 42.19. HRMS (ESI): m/z calcd. for C₂₂H₁₈N₃O₃⁺ [M+H]⁺: 372.1343, found 372.1340.

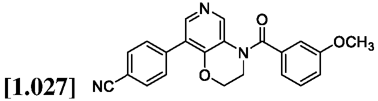

### 4-(4-(3-Methoxybenzoyl)-3,4-dihydro-2H-pyrido[4,3-b][1,4]oxazin-8-yl)benzonitrile (O23)

Following the general method OA, synthesized with **OI4** (50 mg, 0.21 mmol), 3-methoxybenzoyl chloride (54 mg, 0.315 mmol), DMAP (8 mg, 0.063 mmol) and triethylamine (64 mg, 0.63 mmol) to give **O23** (55 mg, 70% yield) as a white solid after chromatographic purification (PE/EA = 2/1), mp: 169.0-171.0 °C. ¹H-NMR (400 MHz, CDCl₃): *δ* 8.45 (s, 1H), 8.20 (s, 1H), 7.74 (d, *J* = 8.2 Hz, 2H), 7.65 (d, *J* = 8.3 Hz, 2H), 7.34 (s, 1H), 7.06 (d, *J* = 32.3 Hz, 3H), 4.45 (s, 2H), 4.06 (s, 2H), 3.83 (s, 3H); ¹³C-NMR (101 MHz, CDCl₃): *δ* 167.88, 158.92, 154.09, 148.19, 144.55, 137.65, 134.22, 131.13 (2C), 129.15 (2C), 128.96, 126.22, 124.09, 119.32, 117.64, 116.06, 112.85, 110.78, 66.43, 54.48, 28.70. HRMS (ESI): m/z calcd. for C₂₂H₁₈N₃O₃⁺ [M+H]⁺: 372.1343, found 372.1325.

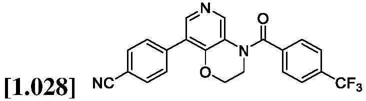

### 4-(4-(4-(Trifluoromethyl)benzoyl)-3,4-dihydro-2H-pyrido[4,3-b][1,4]oxazin-8-yl)benzonitrile (O24)

Following the general method OA, synthesized with **OI4** (50 mg, 0.21 mmol), 4-trifluoromethylbenzoyl chloride (65.52 mg, 0.315 mmol), DMAP (8 mg, 0.063 mmol) and triethylamine (64 mg, 0.63 mmol) to give **O24** (47 mg, 54% yield) as a white solid after chromatographic purification (PE/EA = 2/1), mp: 187.0-189.0 °C. ¹H-NMR (400 MHz, CDCl₃): *δ* 8.33 (s, 1H), 8.22 (s, 1H), 7.76 (s, 4H), 7.64 (s, 4H), 4.49 (s, 2H), 4.08 (s, 2H); ¹³C-NMR (101 MHz, CDCl₃): *δ* 167.50, 149.29, 146.39, 145.47, 143.18, 138.43, 137.50, 133.32, 132.99 (²*J*_{CF} = 32.9), 132.1 (2C), 130.12 (2C), 128.77, 125.98, 125.94 (³*J*_{CF} = 3.7), 124.76, 124.23, 123.49, 122.05 (¹*J*_{CF} = 272.5), 118.58, 111.90, 67.31, 47.83. HRMS (ESI): m/z calcd. for C₂₂H₁₅F₃N₃O₂⁺ [M+H]⁺: 410.1111, found 410.1104.

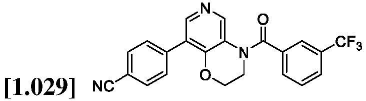

### 4-(4-(3-(Trifluoromethyl)benzoyl)-3,4-dihydro-2H-pyrido[4,3-b][1,4]oxazin-8-yl)benzonitrile (O25)

Following the general method OA, synthesized with **OI4** (50 mg, 0.21 mmol), 3-trifluoromethylbenzoyl chloride (65.52 mg, 0.315 mmol), DMAP (8 mg, 0.063 mmol) and triethylamine (64 mg, 0.63 mmol) to give **O25** (70 mg, 81% yield) as a white solid after chromatographic purification (PE/EA = 2/1), mp: 156.0-158.0 °C. ¹H-NMR (400 MHz, CDCl₃): *δ* 8.33 (t, *J* = 21.4 Hz, 1H), 8.23 (s, 1H), 7.85 (s, 1H), 7.81-7.75 (m, 2H), 7.73 (d, *J* = 11.0 Hz, 2H), 7.65 (d, *J* = 8.4 Hz, 2H), 7.59 (t, *J* = 7.7 Hz, 1H), 4.52-4.45 (m, 2H), 4.12-4.03 (m, 2H); ¹³C-NMR (101 MHz, CDCl₃): *δ* 167.38, 149.31, 146.27, 145.45, 138.44, 134.85, 132.15 (2C), 131.76, 131.52, 131.43 (²*J*_{CF} = 32.9), 130.13 (2C), 129.46, 128.10, 128.06 (³*J*_{CF} = 3.6), 125.49, 125.46 (³*J*_{CF} = 3.6), 124.73, 124.24, 123.46, 122.02 (¹*J*_{CF} = 272.3), 118.59, 111.88, 67.24, 42.28. HRMS (ESI): m/z calcd. for C₂₂H₁₅F₃N₃O₂⁺ [M+H]⁺: 410.1111, found 410.1096.

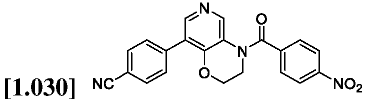

### 4-(4-(4-Nitrobenzoyl)-3,4-dihydro-2H-pyrido[4,3-b][1,4]oxazin-8-yl)benzonitrile (O26)

Following the general method OA, synthesized with **OI4** (50 mg, 0.21 mmol), 4-nitrobenzoyl chloride (58 mg, 0.315 mmol), DMAP (8 mg, 0.063 mmol) and triethylamine (64 mg, 0.63 mmol) to give **O26** (48 mg, 59% yield) as a light yellow solid after chromatographic purification (PE/EA = 2/1), mp: 229.0-232.0 °C. ¹H-NMR (400 MHz, CDCl₃): *δ* 8.30 (s, 2H), 8.23 (s, 2H), 7.74 (s, 4H), 7.66 (s, 2H), 4.51 (s, 2H), 4.09 (s, 2H); ¹³C-NMR (101 MHz, CDCl₃): *δ* 166.51, 149.53, 149.22, 146.39, 145.02, 144.31, 139.89, 138.15, 132.19 (2C), 130.12 (2C), 129.43, 124.41, 124.16 (2C), 123.45, 118.52, 112.05, 67.36, 41.83. HRMS (ESI): m/z calcd. for C₂₁H₁₅N₄O₄⁺ [M+H]⁺: 387.1088, found 387.1089.

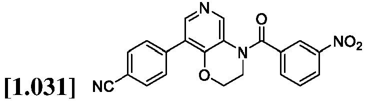

### 4-(4-(3-Nitrobenzoyl)-3,4-dihydro-2H-pyrido[4,3-b][1,4]oxazin-8-yl)benzonitrile (O27)

Following the general method OA, synthesized with **OI4** (50 mg, 0.21 mmol), 3-nitrobenzoyl chloride (58 mg, 0.315 mmol), DMAP (8 mg, 0.063 mmol) and triethylamine (64 mg, 0.63 mmol) to give **O27** (60 mg, 74% yield) as a light yellow solid after chromatographic purification (PE/EA = 2/1), mp: 247.0-249.0 °C. ¹H-NMR (400 MHz, CDCl₃): *δ* 8.38 (s, 2H), 8.23 (s, 2H), 7.90 (s, 1H), 7.76 (s, 2H), 7.67 (s, 3H), 4.53 (s, 2H), 4.12 (s, 2H); ¹³C-NMR (101 MHz, CDCl₃): *δ* 166.21, 149.38, 148.19, 146.63, 145.30, 138.29, 136.89, 135.72, 134.26, 132.17 (2C), 130.25, 130.18, 126.02, 123.50, 121.81, 118.56, 113.98, 111.96, 67.33, 42.73. HRMS (ESI): m/z calcd. for C₂₁H₁₅N₄O₄⁺ [M+H]⁺: 387.1088, found 387.1086.

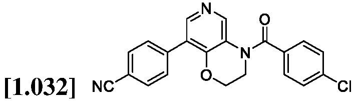

### 4-(4-(4-Chlorobenzoyl)-3,4-dihydro-2H-pyrido[4,3-b][1,4]oxazin-8-yl)benzonitrile (O28)

Following the general method OA, synthesized with **OI4** (50 mg, 0.21 mmol), 4-chlorobenzoyl chloride (55 mg, 0.315 mmol), DMAP (8 mg, 0.063 mmol) and triethylamine (64 mg, 0.63 mmol) to give **O28** (33 mg, 42% yield) as a light yellow solid after chromatographic purification (PE/EA = 2/1), mp: 200.0-202.0 °C. ¹H-NMR (400 MHz, CDCl₃): *δ* 8.28 (d, *J* = 55.9 Hz, 2H), 7.75 (d, *J* = 8.2 Hz, 2H), 7.65 (d, *J* = 8.3 Hz, 2H), 7.51 (d, *J* = 8.4 Hz, 2H), 7.43 (d, *J* = 8.4 Hz, 2H), 4.48 (s, 2H), 4.07 (s, 2H); ¹³C-NMR (101 MHz, CDCl₃): *δ* 167.88, 149.54, 145.50, 144.99, 138.29, 137.89, 136.10, 132.17 (2C), 132.14, 130.14 (2C), 129.93 (2C), 129.25 (2C), 124.24, 118.56, 111.96, 67.37, 42.17. HRMS (ESI): m/z calcd. for C₂₁H₁₅ClN₄O₂⁺ [M+H]⁺: 376.0847, found 376.0831.

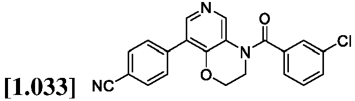

### 4-(4-(3-Chlorobenzoyl)-3,4-dihydro-2H-pyrido[4,3-b][1,4]oxazin-8-yl)benzonitrile (O29)

Following the general method OA, synthesized with **OI4** (50 mg, 0.21 mmol), 3-chlorobenzoyl chloride (55 mg, 0.315 mmol), DMAP (8 mg, 0.063 mmol) and triethylamine (64 mg, 0.63 mmol) to give **O29** (40 mg, 51% yield) as a white solid after chromatographic purification (PE/EA = 2/1), mp: 188.0-190.0 °C. ¹H-NMR (400 MHz, CDCl₃): *δ* 8.37 (s, 1H), 8.21 (s, 1H), 7.74 (d, *J* = 8.4 Hz, 2H), 7.65 (d, *J* = 8.4 Hz, 2H), 7.56 (s, 1H), 7.47 (s, 1H), 7.38 (d, *J* = 7.5 Hz, 2H), 4.46 (s, 2H), 4.05 (s, 2H); ¹³C-NMR (101 MHz, CDCl₃): *δ* 167.47, 149.20, 146.19, 145.53, 138.53, 135.74, 135.08, 132.11 (2C), 131.51, 130.16, 130.11 (2C), 129.19, 128.45, 126.31, 124.17, 118.60, 111.79, 67.21, 42.34. HRMS (ESI): m/z calcd. for C₂₁H₁₅ClN₄O₂⁺ [M+H]⁺: 376.0847, found 376.0835.

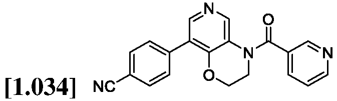

### 4-(4-Nicotinoyl -3,4-dihydro-2H-pyrido[4,3-b][1,4]oxazin-8-yl)benzonitrile (O30)

Following the general method OA, synthesized with **OI4** (50 mg, 0.21 mmol), 3-nicotinoyl chloride (56 mg, 0.315 mmol), DMAP (8 mg, 0.063 mmol) and triethylamine (64 mg, 0.63 mmol) to give **O30** (30 mg, 42% yield) as a white solid after chromatographic purification (PE/EA = 2/1), mp: 210.0-212.0 °C. ¹H-NMR (400 MHz, CDCl₃): *δ* 8.73 (d, *J* = 19.2 Hz, 2H), 8.29 (s, 1H), 8.18 (s, 1H), 7.88 (s, 1H), 7.71 (d, *J =* 8.3 Hz, 2H), 7.62 (d, *J =* 8.3 Hz, 2H), 7.39 (s, 1H), 4.47 (s, 2H), 4.07 (s, 2H); ¹³C-NMR (101 MHz, CDCl₃): *δ* 166.42, 151.99, 149.27, 149.08, 146.35, 145.40, 138.37, 136.13, 132.06 (2C), 130.28, 130.05 (2C), 124.19, 123.63, 120.87, 118.53, 111.74, 67.27, 42.04. HRMS (ESI): m/z calcd. for C₂₀H₁₅N₄O₂⁺ [M+H]⁺: 343.1190, found 343.1181.

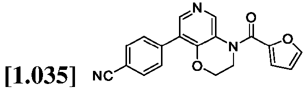

### 4-(4-(Furan-2-carbonyl)-3,4-dihydro-2H-pyrido[4,3-b][1,4]oxazin-8-yl)benzonitrile (O31)

Following the general method OA, synthesized with **OI4** (50 mg, 0.21 mmol), 2-furancarbonyl chloride (41 mg, 0.315 mmol), DMAP (8 mg, 0.063 mmol) and triethylamine (64 mg, 0.63 mmol) to give **O31** (30 mg, 43.16% yield) as a white solid after chromatographic purification (PE/EA = 2/1), mp: 193.0-194.0 °C. ¹H-NMR (400 MHz, CDCl₃): *δ* 8.47 (s, 1H), 8.22 (s, 1H), 7.74 (d, *J* = 8.0 Hz, 2H), 7.66 (d, *J* = 8.2 Hz, 2H), 7.51 (s, 1H), 7.19 (s, 1H), 6.56 (s, 1H), 4.47 (s, 2H), 4.18 (s, 2H); ¹³C-NMR (101 MHz, CDCl₃): *δ* 157.93, 149.18, 146.58, 146.06, 145.19, 145.10, 138.77, 132.07 (2C), 130.12 (2C), 124.05, 123.60, 118.87, 118.66, 112.10, 111.66, 67.24, 41.81. HRMS (ESI): m/z calcd. for C₁₉H₁₄N₃O₃⁺ [M+H]⁺: 332.1030, found 332.1017.

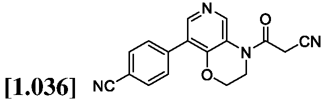

### 4-(4-(2-Cyanoacetyl)-3,4-dihydro-2H-pyrido[4,3-b][1,4]oxazin-8-yl)benzonitrile (O32)

Following the general method OE, **OI4** (50 mg, 0.21 mmol), cyanoacetic acid (20 mg, 0.32 mmol), N, N-diisopropylethylamine (81 mg, 0.063 mmol) and HATU (83.00 mg, 0.22 mmol) to give **O32** (20 mg, 31% yield) as a white solid after chromatographic purification (PE/EA = 1/2), mp: 212.0-214.0 °C. ¹H-NMR (400 MHz, DMSO*-d₆*): *δ* 9.07 (s, 1H), 8.26 (s, 1H), 7.93 (d, *J* = 8.1 Hz, 2H), 7.76 (d, *J =* 8.1 Hz, 2H), 4.44 (d, *J =* 8.4 Hz, 2H), 4.38 (s, 2H), 3.88 (s, 2H); ¹³C-NMR (101 MHz, DMSO*-d₆*): *δ* 162.48, 149.43, 145.72, 145.00, 138.78, 132.21 (2C), 130.25 (2C), 123.44, 118.74, 115.63, 114.40, 110.48, 66.53, 42.86, 26.42. HRMS (ESI): m/z calcd. for C₁₇H₁₃N₄O₂⁺ [M+H]⁺: 305.1033, found 305.1023.

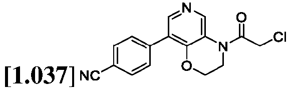

### 4-(4-(2-Chloroacetyl)-3,4-dihydro-2H-pyrido[4,3-b][1,4]oxazin-8-yl)benzonitrile (O33)

Following the general method OA, synthesized with **OI4** (50 mg, 0.21 mmol), chloroacetyl chloride (35 mg, 0.315 mmol), DMAP (8 mg, 0.063 mmol) and triethylamine (64 mg, 0.63 mmol) to give **O33** (18 mg, 27% yield) as a white solid after chromatographic purification (PE/EA = 2/1), mp: decomposed. ¹H-NMR (400 MHz, CDCl₃): *δ* 8.42 (s, 1H), 8.27 (s, 1H), 7.74 (d, *J* = 8.3 Hz, 2H), 7.64 (d, *J* = 8.3 Hz, 2H), 4.73 (s, 2H), 4.52 (s, 2H), 4.23 (s, 2H); ¹³C-NMR (101 MHz, CDCl₃): *δ* 190.42, 170.14, 149.53, 146.88, 144.97, 138.17, 132.14, 130.09, 124.28, 121.17, 118.53, 111.91, 90.47, 73.49, 66.57, 42.97.

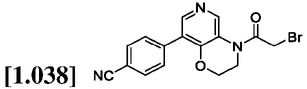

### 4-(4-(2-Bromoacetyl)-3,4-dihydro-2H-pyrido[4,3-b][1,4]oxazin-8-yl)benzonitrile (O34)

Following the general method OA, synthesized with **OI4** (50 mg, 0.21 mmol), bromoacetyl chloride 50mg, 0.315 mmol), DMAP (8 mg, 0.063 mmol) and triethylamine (64 mg, 0.63 mmol) to give **O34** (20 mg, 27% yield) as a white solid after chromatographic purification (PE/EA = 2/1), mp: decomposed. ¹H-NMR (400 MHz, DMSO*-d₆*): *δ* 8.45 (s, 1H), 8.25 (s, 1H), 7.93 (d, *J* = 8.3 Hz, 2H), 7.79 (d, *J* = 8.3 Hz, 2H), 4.84 (s, 2H), 4.51 (s, 2H), 4.16 (s, 2H); ¹³C-NMR (101 MHz, DMSO*-d₆*): *δ* 190.77, 170.81, 149.85, 146.45, 145.33, 138.95, 132.72, 130.75, 123.95, 121.79, 119.21, 111.06, 89.53, 74.20, 67.16, 43.36.

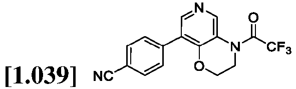

### 4-(4-(2,2,2-Trifluoroacetyl)-3,4-dihydro-2H-pyrido[4,3-b][1,4]oxazin-8-yl)benzonitrile (O35)

Following the general method OD, synthesized with **OI4** (100 mg, 0.42 mmol), trifluoroacetic anhydride (97 mg, 0.46 mmol), DMAP (8 mg, 0.063 mmol) and triethylamine (47 mg, 0.46 mmol) to give **O35** (60 mg, 43% yield) as a white solid after chromatographic purification (PE/EA = 2/1), mp: 156.0-158.0 °C. ¹H-NMR (400 MHz, CDCl₃): *δ* 8.97 (s, 1H), 8.12 (s, 1H), 7.54 (s, 2H), 7.42 (s, 2H), 4.29 (s, 2H), 3.87 (s, 2H); ¹³C-NMR (101 MHz, CDCl₃): *δ* 148.99, 146.84, 144.71, 137.23, 131.17 (2C), 129.15 (2C), 123.36, 120.92, 117.56, 116.47, 113.61, 110.95, 65.60, 41.67. HRMS (ESI): m/z calcd. for C₁₆H₁₁F₃N₃O₂⁺ [M+H]⁺: 334.0798, found 334.0790.

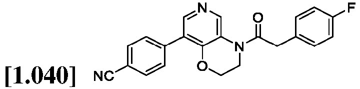

### 4-(4-(2-(4-Fluorophenyl)acetyl)-3,4-dihydro-2H-pyrido[4,3-b][1,4]oxazin-8-yl)benzonitrile (O36)

Following the general method OE, synthesized with **OI4** (50 mg, 0.21 mmol), 4-fluorophenylacetic acid (49 mg, 0.32 mmol), N, N-diisopropylethylamine (81 mg, 0.63 mmol) and HATU (83.00 mg, 0.22 mmol) to give **O36** (35 mg, 44.87% yield) as a white solid after chromatographic purification (PE/EA = 1/1), mp: 168.0-169.0 °C. ¹H-NMR (400 MHz, CDCl₃): *δ* 8.50 (s, 1H), 8.28 (s, 1H), 7.72 (d, *J =* 8.3 Hz, 2H), 7.58 (d, *J =* 8.4 Hz, 2H), 7.19 (s, 2H), 7.01 (t, *J* = 8.4 Hz, 2H), 4.28 (s, 2H), 3.97 (s, 4H); ¹³C-NMR (101 MHz, DMSO*-d₆*): *δ* 169.61, 162.26, 159.86 (¹*J*_{CF} = 242.1), 151.93, 149.44, 145.39, 138.94, 132.18 (2C), 131.42, 131.34 (³*J*_{CF} = 8.1), 131.24, 131.21 (⁴*J*_{CF} = 2.8), 130.23 (2C), 124.14, 123.33, 118.75, 115.07, 114.86 (²*J*_{CF} = 21.2), 110.40, 67.13, 26.09, 20.74. HRMS (ESI): m/z calcd. for C₂₂H₁₇FN₃O₂⁺ [M+H]⁺: 374.1299, found 374.1290.

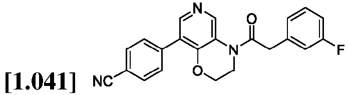

### 4-(4-(2-(3-Fluorophenyl)acetyl)-3,4-dihydro-2H-pyrido[4,3-b][1,4]oxazin-8-yl)benzonitrile (O37)

Following the general method OE, synthesized with **OI4** (100 mg, 0.42 mmol), 3-fluorophenylacetic acid (97 mg, 0.63 mmol), N, N-diisopropylethylamine (163 mg, 1.26 mmol) and HATU (168.00 mg, 0.44 mmol) to give **O37** (120 mg, 77% yield) as a white solid after chromatographic purification (PE/EA = 1/1), mp: 95.0-96.0 °C. ¹H-NMR (400 MHz, CDCl₃): *δ* 8.53 (s, 1H), 8.25 (s, 1H), 7.69 (d, *J* = 8.0 Hz, 2H), 7.57 (d, *J* = 8.0 Hz, 2H), 7.26 (s, 1H), 6.93 (s, 3H), 4.27 (s, 2H), 3.97 (s, 4H); ¹³C-NMR (101 MHz, CDCl₃): *δ* 168.87, 163.99, 161.53 (¹*J*_{CF} = 246.9), 149.92, 147.06, 145.45, 145.42, 138.45, 135.99, 131.96 (2C), 130.28, 130.20 (³*J*_{CF} = 7.9), 129.95 (2C), 124.39, 123.98, 118.51, 115.82, 115.61 (²*J*_{CF} = 21.9), 114.27, 114.06 (²*J*_{CF} = 20.9), 111.55, 77.20, 67.41, 40.58. HRMS (ESI): m/z calcd. for C₂₂H₁₇FN₃O₂⁺ [M+H]⁺: 374.1299, found 374.1300.

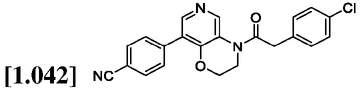

### 4-(4-(2-(4-Chlorophenyl)acetyl)-3,4-dihydro-2H-pyrido[4,3-b][1,4]oxazin-8-yl)benzonitrile (O38)

Following the general method OE, synthesized with **OI4** (50 mg, 0.21 mmol), 4-chlorophenylacetic acid (34 mg, 0.32 mmol), N, N-diisopropylethylamine (81 mg, 0.63 mmol) and HATU (83.00 mg, 0.22 mmol) to give **O38** (56 mg, 68% yield) as a light yellow solid after chromatographic purification (PE/EA = 1/1), mp: 169.0-170.0 °C. ¹H-NMR (400 MHz, CDCl₃): *δ* 8.63 (s, 1H), 8.28 (s, 1H), 7.73 (d, *J* = 8.4 Hz, 2H), 7.58 (d, *J* = 8.5 Hz, 2H), 7.29 (d, *J* = 8.1 Hz, 2H), 7.16 (d, *J* = 7.5 Hz, 2H), 4.28 (s, 2H), 3.97 (s, 4H); ¹³C-NMR (101 MHz, CDCl₃): *δ* 169.27, 159.31, 150.02, 147.26, 145.43, 138.43, 133.29, 132.12 (2C), 130.74, 130.14, 130.05 (2C), 129.00, 128.62, 126.57, 124.18, 118.58, 111.82, 67.94, 40.69, 26.79. HRMS (ESI): m/z calcd. for C₂₂H₁₇ClN₃O₂⁺ [M+H]⁺: 390.1004, found 390.1009.

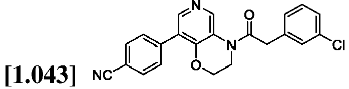

### 4-(4-(2-(3-Chlorophenyl)acetyl)-3,4-dihydro-2H-pyrido[4,3-b][1,4]oxazin-8-yl)benzonitrile (O39)

Following the general method OE, synthesized with **OI4** (100 mg, 0.42 mmol), 3-chlorophenylacetic acid (107 mg, 0.63 mmol), N, N-diisopropylethylamine (163 mg, 1.26 mmol) and HATU (168 mg, 0.44 mmol) to give **O39** (127 mg, 77% yield) as a white solid after chromatographic purification (PE/EA = 1/1), mp: 67.0-68.0 °C. ¹H-NMR (400 MHz, CDCl₃): *δ* 8.55 (s, 1H), 8.29 (s, 1H), 7.72 (d, *J* = 8.3 Hz, 2H), 7.59 (d, *J* = 8.4 Hz, 2H), 7.18 (d, *J* = 52.5 Hz, 4H), 4.29 (s, 2H), 3.98 (s, 4H); ¹³C-NMR (101 MHz, CDCl₃): *δ* 169.03, 150.02, 147.18, 145.41, 138.44, 135.63, 134.67, 132.11 (2C), 130.06 (2C), 128.90, 127.56, 127.00, 124.19, 121.37, 118.58, 116.43, 111.82, 77.20, 60.44, 40.67. HRMS (ESI): m/z calcd. for C₂₂H₁₇ClN₃O₂⁺ [M+H]⁺: 390.1004, found 390.1010.

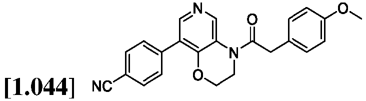

### 4-(4-(2-(4-Methoxyphenyl)acetyl)-3,4-dihydro-2H-pyrido[4,3-b][1,4]oxazin-8-yl)benzonitrile (O40)

Following the general method OE, synthesized with **OI4** (100 mg, 0.42 mmol), 4-methoxyphenylacetic acid (105 mg, 0.63 mmol), N, N-diisopropylethylamine (163 mg, 1.26 mmol) and HATU (168 mg, 0.44 mmol) to give **O40** (121 mg, 74% yield) as a white solid after chromatographic purification (PE/EA = 1/1), mp: 151.0-152.0 0°C. ¹H-NMR (400 MHz, CDCl₃): *δ* 8.65 (s, 1H), 8.26 (s, 1H), 7.71 (d, *J* = 8.3 Hz, 2H), 7.57 (d, *J* = 8.3 Hz, 2H), 7.12 (d, *J* = 7.2 Hz, 2H), 6.84 (d, *J* = 8.1 Hz, 2H), 4.22 (s, 2H), 3.94 (d, *J* = 8.6 Hz, 4H), 3.77 (s, 3H); ¹³C-NMR (101 MHz, CDCl₃): *δ* 170.03, 158.76, 149.87, 146.90, 145.58, 138.52, 132.08 (2C), 130.35, 130.05 (2C), 129.71, 125.40, 124.09, 118.60, 116.64, 114.31, 113.95, 111.74, 77.20, 67.54, 55.25, 40.45. HRMS (ESI): m/z calcd. for C₂₃H₂₀N₃O₃⁺ [M+H]⁺: 386.1499, found 386.1499.

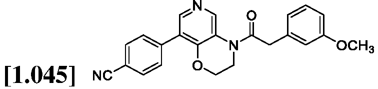

### 4-(4-(2-(3-Methoxyphenyl)acetyl)-3,4-dihydro-2H-pyrido[4,3-b][1,4]oxazin-8-yl)benzonitrile (041)

Following the general method OE, synthesized with **OI4** (100 mg, 0.42 mmol), 3-methoxyphenylacetic acid (105 mg, 0.63 mmol), N, N-diisopropylethylamine (163 mg, 1.26 mmol) and HATU (168 mg, 0.44 mmol) to give **O41** (136 mg, 84% yield) as a white solid after chromatographic purification (PE/EA = 1/1), mp: 60.0-61.0 °C. ¹H-NMR (400 MHz, CDCl₃): *δ* 8.68 (s, 1H), 8.24 (s, 1H), 7.69 (d, *J =* 8.3 Hz, 2H), 7.56 (d, *J =* 8.4 Hz, 2H), 7.21 (s, 1H), 6.77 (d, *J* = 8.1 Hz, 3H), 4.22 (s, 2H), 3.95 (s, 4H), 3.75 (s, 3H); ¹³C-NMR (101 MHz, CDCl₃): *δ* 169.44, 159.94, 150.02, 146.63, 145.51, 138.59, 134.94, 131.97 (2C), 129.97 (2C), 129.83, 123.99, 120.76, 118.54, 114.89, 114.24, 112.59, 111.57, 77.20, 67.33, 55.10, 41.51. HRMS (ESI): m/z calcd. for C₂₃H₂₀N₃O₃⁺ [M+H]⁺: 386.1499, found 386.1489.

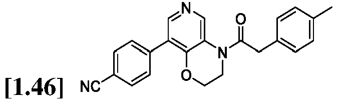

### 4-(4-(2-(p-Tolyl)acetyl)-3,4-dihydro-2H-pyrido[4,3-b][1,4]oxazin-8-yl)benzonitrile (O42)

Following the general method OE, synthesized with **OI4** (100 mg, 0.42 mmol), 4-methylphenylacetic acid (95 mg, 0.63 mmol), N, N-diisopropylethylamine (162.85 mg, 1.26 mmol) and HATU (168 mg, 0.44 mmol) to give **O42** (109 mg, 70% yield) as a white solid after chromatographic purification (PE/EA = 1/1), mp: 89.0-91.0 °C. ¹H-NMR (400 MHz, CDCl₃): *δ* 8.72 (s, 1H), 8.25 (s, 1H), 7.70 (d, *J =* 8.3 Hz, 2H), 7.56 (d, *J =* 8.3 Hz, 2H), 7.11 (s, 4H), 4.22 (s, 2H), 3.95 (s, 4H), 2.30 (s, 3H); ¹³C-NMR (101 MHz, CDCl₃): *δ* 169.94, 149.83, 146.90, 145.77, 145.71, 138.65, 136.88, 132.00 (2C), 130.43, 130.00 (2C), 129.53, 128.44, 124.30, 123.92, 120.80, 118.58, 111.59, 67.31, 41.06, 40.45, 20.95. HRMS (ESI): m/z calcd. for C₂₃H₂₀N₃O₂⁺ [M+H]⁺: 370.1550, found 370.1546.

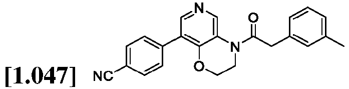

### 4-(4-(2-(m-Tolyl)acetyl)-3,4-dihydro-2H-pyrido[4,3-b][1,4]oxazin-8-yl)benzonitrile (O43)

Following the general method OE, synthesized with **OI4** (100 mg, 0.42 mmol), 3-methylphenylacetic acid (97 mg, 0.63 mmol), N, N-diisopropylethylamine (163 mg, 1.26 mmol) and HATU (168 mg, 0.44 mmol) to give **O43** (126 mg, 81% yield) as a white solid after chromatographic purification (PE/EA = 1/1), mp: 91.0-92.0 °C. ¹H-NMR (400 MHz, CDCl₃): *δ* 8.66 (s, 1H), 8.25 (s, 1H), 7.69 (d, *J =* 8.2 Hz, 2H), 7.56 (d, *J =* 8.3 Hz, 2H), 7.19 (s, 1H), 7.04 (d, *J* = 20.8 Hz, 3H), 4.21 (s, 2H), 3.95 (s, 4H), 2.29 (s, 3H); ¹³C-NMR (101 MHz, CDCl₃): *δ* 169.60, 149.80, 146.73, 145.61, 138.54, 133.35, 131.95 (2C), 129.94 (2C), 129.20, 128.67, 127.91, 125.51, 124.16, 123.90, 118.52, 116.47, 111.52, 77.20, 67.28, 41.21, 21.23. HRMS (ESI): m/z calcd. for C₂₃H₂₀N₃O₂⁺ [M+H]⁺: 370.1550, found 370.1557.

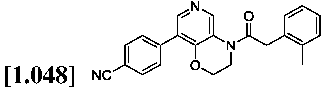

### 4-(4-(2-(o-Tolyl)acetyl)-3,4-dihydro-2H-pyrido[4,3-b][1,4]oxazin-8-yl)benzonitrile (O44)

Following the general method OE, synthesized with **OI4** (100 mg, 0.42 mmol), 2-methylphenylacetic acid (95 mg, 0.63 mmol), N, N-diisopropylethylamine (163 mg, 1.26 mmol) and HATU (168 mg, 0.44 mmol) to give **O44** (114 mg, 73% yield) as a white solid after chromatographic purification (PE/EA = 1/1), mp: 174.0-175.0 °C. ¹H-NMR (400 MHz, CDCl₃): *δ* 8.84 (s, 1H), 8.26 (s, 1H), 7.71 (d, *J =* 8.3 Hz, 2H), 7.61 (d, *J =* 8.3 Hz, 2H), 7.16 (d, *J =* 11.5 Hz, 4H), 4.33 (s, 2H), 3.95 (s, 4H), 2.25 (s, 3H); ¹³C-NMR (101 MHz, CDCl₃): *δ* 169.49, 149.81, 146.75, 145.37, 138.56, 136.19, 134.06, 132.01 (2C), 130.55, 130.03 (2C), 128.77, 127.43, 126.28, 124.01, 118.56, 116.52, 111.61, 77.20, 67.54, 39.07, 19.61. HRMS (ESI): m/z calcd. for C₂₃H₂₀N₃O₂⁺ [M+H]⁺: 370.1550, found 370.1553.

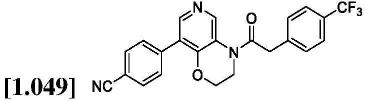

### 4-(4-(2-(4-(Trifluoromethyl)phenyl)acetyl)-3,4-dihydro-2H-pyrido[4,3-b][1,4]oxazin-8-yl)benz onitrile (O45)

Following the general method OE, synthesized with **OI4** (100 mg, 0.42 mmol), 4-trifluoromethylphenylacetic acid (129 mg, 0.63 mmol), N, N-diisopropylethylamine (163 mg, 1.26 mmol) and HATU (168 mg, 0.44 mmol) to give **O45** (128 mg, 72% yield) as a white solid after chromatographic purification (PE/EA = 1/1), mp: 124.0-125.0 °C. ¹H-NMR (400 MHz, CDCl₃): *δ* 8.52 (s, 1H), 8.29 (s, 1H), 7.72 (d, *J =* 8.3 Hz, 2H), 7.58 (d, *J =* 13.0 Hz, 4H), 7.36 (d, *J =* 7.4 Hz, 2H), 4.32 (s, 2H), 4.06 (s, 2H), 4.01 (s, 2H); ¹³C-NMR (101 MHz, CDCl₃): *δ* 168.88, 147.25, 145.24, 138.28, 137.68, 132.13 (2C), 130.04 (2C), 129.85, 129.53 (²*J*_{CF} = 32.9), 129.76, 129.30, 125.75, 125.73, 125.43, 125.39 (³*J*_{CF} = 3.7), 125.29, 122.58 (¹*J*_{CF} = 272.2), 124.30, 118.55, 116.43, 111.90, 77.20, 67.25, 40.77. HRMS (ESI): m/z calcd. for C₂₃H₁₇F₃N₃O₂⁺ [M+H]⁺: 424.1267, found 424.1262.

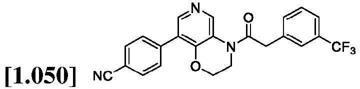

### 4-(4-(2-(3-(Trifluoromethyl)phenyl)acetyl)-3,4-dihydro-2H-pyrido[4,3-b][1,4]oxazin-8-yl)benz onitrile (O46)

Following the general method OE, synthesized with **OI4** (100 mg, 0.42 mmol), 3-trifluoromethylphenylacetic acid (129 mg, 0.63 mmol), N, N-diisopropylethylamine (163 mg, 1.26 mmol) and HATU (168 mg, 0.44 mmol) to give **O46** (138 mg, 77% yield) as a white solid after chromatographic purification (PE/EA = 1/1), mp: 58.0-60.0 °C. ¹H-NMR (400 MHz, CDCl₃): *δ* 8.54 (s, 1H), 8.30 (s, 1H), 7.73 (d, *J =* 8.4 Hz, 2H), 7.57 (d, *J =* 1.6 Hz, 2H), 7.53 (s, 1H), 7.45 (s, 3H), 4.31 (s, 2H), 4.07 (s, 2H), 4.01 (s, 2H); ¹³C-NMR (101 MHz, CDCl₃): *δ* 168.94, 153.66, 147.07, 145.40, 138.34, 136.65, 134.69, 132.42, 132.14 (2C), 131.32, 131.02 (²*J*_{CF} = 32.2), 130.05 (2C), 129.30, 125.68, 125.64 (³*J*_{CF} = 3.8), 125.19, 122.48 (¹*J*_{CF} = 272.3), 124.28, 124.23, 118.57, 111.90, 77.20, 67.83, 40.73. HRMS (ESI): m/z calcd. for C₂₃H₁₇F₃N₃O₂⁺ [M+H]⁺: 424.1267, found 424.1274.

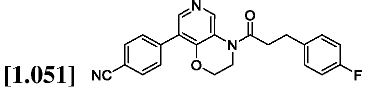

### 4-(4-(3-(4-Fluorophenyl)propanoyl)-3,4-dihydro-2H-pyrido[4,3-b][1,4]oxazin-8-yl)benzonitrile (O47)

Following the general method OE, synthesized with **OI4** (50 mg, 0.21 mmol), 4-fluorophenylpropanoic acid (53 mg, 0.32 mmol), N, N-diisopropylethylamine (81 mg, 0.63 mmol) and HATU (83 mg, 0.22 mmol) to give **O47** (40 mg, 49.38% yield) as a white solid after chromatographic purification (PE/EA = 1/2), mp: 148.0-150.0 °C. ¹H-NMR (400 MHz, CDCl₃): *δ* 10.11-8.29 (m, 1H), 8.25 (s, 1H), 7.72 (d, *J =* 8.4 Hz, 2H), 7.60 (d, *J =* 8.4 Hz, 2H), 7.12 (s, 2H), 6.93 (t, *J =* 8.5 Hz, 2H), 4.26-4.19 (m, 2H), 3.92 (d, *J =* 16.6 Hz, 2H), 3.03 (t, *J =* 6.7 Hz, 2H), 2.94 (s, 2H); ¹³C-NMR (101 MHz,CDCl₃): *δ* 170.63, 162.69, 160.26 (¹*J*_{CF} = 244.8), 149.85, 147.00, 145.54, 138.51, 135.85, 132.08 (2C), 130.03 (2C), 129.88, 129.80 (³*J*_{CF} = 7.9), 124.07, 123.84, 118.59, 115.40, 115.19 (²*J*_{CF} = 21.2), 111.73, 77.20, 67.99, 35.48, 30.76. HRMS (ESI): m/z calcd. for C₂₃H₁₉FN₃O₂⁺ [M+H]⁺: 388.1456, found 388.1441.

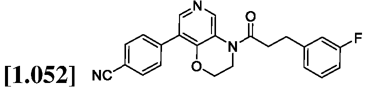

### 4-(4-(3-(3-Fluorophenyl)propanoyl)-3,4-dihydro-2H-pyrido[4,3-b][1,4]oxazin-8-yl)benzonitrile (O48)

Following the general method OE, synthesized with **OI4** (100 mg, 0.42 mmol), 3-fluorophenylpropanoic acid (106 mg, 0.63 mmol), N, N-diisopropylethylamine (163 mg, 1.26 mmol) and HATU (168 mg, 0.44 mmol) to give **O48** (76 mg, 46.77% yield) as a colorless oil after chromatographic purification (PE/EA = 1/1). ¹H-NMR (400 MHz, CDCl₃): *δ* 8.66 (s, 1H), 8.25 (s, 1H), 7.71 (d, *J =* 8.3 Hz, 2H), 7.60 (d, *J =* 8.3 Hz, 2H), 7.20 (s, 1H), 6.93 (s, 1H), 6.86 (s, 2H), 4.22 (s, 2H), 3.94 (s, 2H), 3.04 (s, 2H), 2.96 (s, 2H); ¹³C-NMR (101 MHz, CDCl₃): *δ* 170.44, 163.98, 161.54 (¹*J*_{CF} = 246.2), 149.89, 147.03, 145.46, 142.74, 142.67 (³*J*_{CF} = 7.1), 138.51, 132.03 (2C), 130.01 (2C), 129.92, 124.07, 124.05, 123.91, 118.57, 115.27, 115.06 (²*J*_{CF} = 20.9), 113.38, 113.18 (²*J*_{CF} = 21.1), 111.66, 77.20, 68.04, 35.16, 31.18. HRMS (ESI): m/z calcd. for C₂₃H₁₉FN₃O₂⁺ [M+H]⁺: 388.1456, found 388.1456.

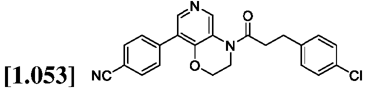

### 4-(4-(3-(4-Chlorophenyl)propanoyl)-3,4-dihydro-2H-pyrido[4,3-b][1,4]oxazin-8-yl)benzonitril e (O49)

Following the general method OE, synthesized with **OI4** (100 mg, 0.42 mmol), 4-chlorophenylpropanoic acid (116 mg, 0.63 mmol), N, N-diisopropylethylamine (163 mg, 1.26 mmol) and HATU (168 mg, 0.44 mmol) to give **O49** (45 mg, 26.67% yield) as a colorless oil after chromatographic purification (PE/EA = 1/1). ¹H-NMR (400 MHz, CDCl₃): *δ* 8.52 (s, 1H), 8.26 (s, 1H), 7.72 (d, *J =* 8.4 Hz, 2H), 7.60 (d, *J =* 8.4 Hz, 2H), 7.22 (d, *J =* 8.1 Hz, 2H), 7.09 (d, *J =* 7.2 Hz, 2H), 4.22 (s, 2H), 3.94 (s, 2H), 3.01 (s, 2H), 2.94 (s, 2H); ¹³C-NMR (101 MHz, CDCl₃): *δ* 170.51, 149.93, 147.02, 145.52, 138.64, 138.47, 132.20, 132.09 (2C), 130.05 (2C), 129.79 (2C), 129.66, 128.60, 128.49, 124.10, 118.58, 111.74, 77.20, 68.03, 35.21, 30.86. HRMS (ESI): m/z calcd. for C₂₃H₁₉ClN₃O₂⁺ [M+H]⁺: 404.1160, found 404.1167.

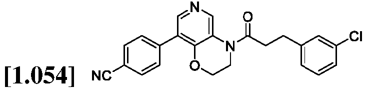

### 4-(4-(3-(3-Chlorophenyl)propanoyl)-3,4-dihydro-2H-pyrido[4,3-b][1,4]oxazin-8-yl) benzonitrile (O50)

Following the general method OE, synthesized with **OI4** (100 mg, 0.42 mmol), 3-chlorophenylpropanoic acid (116 mg, 0.63 mmol), N, N-diisopropylethylamine (163 mg, 1.26 mmol) and HATU (168 mg, 0.44 mmol) to give **O50** (82 mg, 48% yield) as a colorless oil after chromatographic purification (PE/EA = 1/1). ¹H-NMR (400 MHz, CDCl₃): *δ* 8.72 (s, 1H), 8.25 (s, 1H), 7.71 (d, *J =* 8.3 Hz, 2H), 7.60 (d, *J =* 8.3 Hz, 2H), 7.15 (s, 3H), 7.05 (s, 1H), 4.22 (s, 2H), 3.94 (s, 2H), 3.02 (s, 2H), 2.96 (s, 2H); ¹³C-NMR (101 MHz, CDCl₃): *δ* 170.41, 149.73, 146.96, 145.43, 142.20, 138.49, 134.15, 132.00 (2C), 130.00 (2C), 129.73, 128.34, 126.63, 126.53, 124.01, 123.91, 118.55, 111.62, 77.20, 67.89, 35.00, 31.09. HRMS (ESI): m/z calcd. for C₂₃H₁₉ClN₃O₂⁺ [M+H]⁺: 404.1160, found 404.1165.

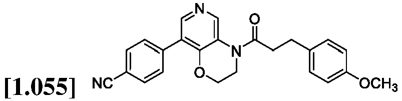

### 4-(4-(3-(4-Methoxyphenyl)propanoyl)-3,4-dihydro-2H-pyrido[4,3-b][1,4]oxazin-8-yl)benzonitr ile (O51)

Following the general method OE, synthesized with **OI4** (100 mg, 0.42 mmol), 4-methoxyphenylpropanoic acid (113 mg, 0.63 mmol), N, N-diisopropylethylamine (163 mg, 1.26 mmol) and HATU (168 mg, 0.44 mmol) to give **O51** (54 mg, 32% yield) as a colorless oil after chromatographic purification (PE/EA = 1/1). ¹H-NMR (400 MHz, CDCl₃): *δ* 8.75 (s, 1H), 8.25 (s, 1H), 7.71 (d, *J =* 8.3 Hz, 2H), 7.60 (d, *J =* 8.3 Hz, 2H), 7.07 (s, 2H), 6.77 (d, *J =* 8.1 Hz, 2H), 4.18 (s, 2H), 3.92 (s, 2H), 3.73 (s, 3H), 2.99 (s, 2H), 2.94 (s, 2H); ¹³C-NMR (101 MHz, CDCl₃): *δ* 171.07, 158.13, 149.85, 146.84, 145.67, 138.64, 132.08, 132.02 (2C), 130.03 (2C), 129.31 (2C), 129.16, 123.96, 118.59, 113.88, 113.80, 111.64, 77.20, 67.76, 55.17, 35.62, 30.83. HRMS (ESI): m/z calcd. for C₂₄H₂₂N₃O₃⁺ [M+H]⁺: 400.1656, found 400.1657.

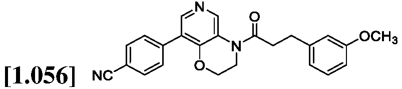

### 4-(4-(3-(3-Methoxyphenyl)propanoyl)-3,4-dihydro-2H-pyrido[4,3-b][1,4]oxazin-8-yl)benz onitrile (O52)

Following the general method OE, synthesized with **OI4** (100 mg, 0.42 mmol), 3-methoxyphenylpropanoic acid (114 mg, 0.63 mmol), N, N-diisopropylethylamine (163 mg, 1.26 mmol) and HATU (168 mg, 0.44 mmol) to give **O52** (96 mg, 57% yield) as a colorless oil after chromatographic purification (PE/EA = 1/1). ¹H-NMR (400 MHz, CDCl₃): *δ* 8.64 (s, 1H), 8.25 (s, 1H), 7.72 (d, *J =* 8.4 Hz, 2H), 7.60 (d, *J =* 8.4 Hz, 2H), 7.16 (s, 1H), 6.70 (d, *J =* 21.3 Hz, 3H), 4.18 (s, 2H), 3.92 (s, 2H), 3.72 (s, 3H), 2.98 (d, *J* = 28.0 Hz, 4H); ¹³C-NMR (101 MHz, CDCl₃): *δ* 170.87, 159.66, 150.01, 146.69, 145.40, 141.67, 138.52, 132.04 (2C), 130.03 (2C), 129.50, 129.38, 124.08, 120.69, 118.58, 114.03, 111.70, 111.68, 77.20, 67.88, 55.06, 38.94, 31.76. HRMS (ESI): m/z calcd. for C₂₄H₂₂N₃O₃⁺ [M+H]⁺: 400.1656, found 400.1641.

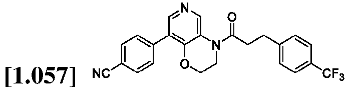

### 4-(4-(3-(4-(Trifluoromethyl)phenyl)propanoyl)-3,4-dihydro-2H-pyrido[4,3-b][1,4]oxazin-8-yl) benzonitrile (O53)

Following the general method OE, synthesized with **OI4** (100 mg, 0.42 mmol), 4-trimethylphenylpropanoic acid (137 mg, 0.63 mmol), N, N-diisopropylethylamine (163 mg, 1.26 mmol) and HATU (168 mg, 0.44 mmol) to give **O53** (78.41 mg, 43.7% yield) as a white solid after chromatographic purification (PE/EA = 1/1), mp: 52.0-53.0 °C. ¹H-NMR (400 MHz, CDCl₃): *δ* 8.43 (s, 1H), 8.27 (s, 1H), 7.73 (d, *J =* 8.2 Hz, 2H), 7.60 (d, *J =* 8.2 Hz, 2H), 7.53 (d, *J =* 7.6 Hz, 2H), 7.30 (d, *J =* 7.2 Hz, 2H), 4.24 (s, 2H), 3.96 (s, 2H), 3.13 (s, 2H), 2.98 (s, 2H); ¹³C-NMR (101 MHz, CDCl₃): *δ* 170.30, 150.12, 145.26, 144.39, 138.37, 134.15, 132.14 (2C), 130.05 (2C), 129.04, 128.72 (²*J*_{CF} = 32.4), 128.82 (2C), 128.64, 125.50, 125.46 (³*J*_{CF} = 3.5), 124.28, 121.81, 119.00 (¹*J*_{CF} = 263.9), 118.57, 116.38, 111.89, 77.20, 44.23, 34.99, 30.63. HRMS (ESI): m/z calcd. for C₂₄H₁₉F₃N₃O₂⁺ [M+H]⁺: 438.1424, found 438.1413.

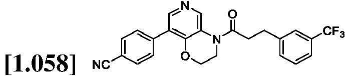

### 4-(4-(3-(3-(Trifluoromethyl)phenyl)-3,4-dihydro-2H-pyrido[4,3-b][1,4]oxazin-8-yl)benzon itrile (O54)

Following the general method OE, synthesized with **OI4** (100 mg, 0.42 mmol), 3-trimethylphenylpropanoic acid (137 mg, 0.63 mmol), N, N-diisopropylethylamine (163 mg, 1.26 mmol) and HATU (168 mg, 0.44 mmol) to give **O54** (40 mg, 22% yield) as a colorless oil after chromatographic purification (PE/EA = 1/1). ¹H-NMR (400 MHz, CDCl₃): *δ* 8.56 (s, 1H), 8.27 (s, 1H), 7.72 (d, *J =* 8.4 Hz, 2H), 7.60 (d, *J =* 8.4 Hz, 2H), 7.46 (s, 1H), 7.38 (s, 3H), 4.23 (s, 2H), 3.96 (s, 2H), 3.13 (s, 2H), 2.99 (s, 2H); ¹³C-NMR (101 MHz, CDCl₃): δ 170.22, 149.89, 147.02, 145.39, 141.22, 138.47, 132.09 (2C), 131.98, 130.99, 130.67 (²*J*_{CF} = 32.0), 130.05 (2C), 129.00, 128.85, 125.36, 122.65 (¹*J*_{CF} = 272.3), 125.00, 124.96 (³*J*_{CF} = 3.7), 124.16, 123.34, 123.30 (³*J*_{CF} = 3.8), 118.59, 111.77, 77.20, 67.81, 35.18, 31.19. HRMS (ESI): m/z calcd. for C₂₄H₁₉F₃N₃O₂⁺ [M+H]⁺: 438.1424, found 438.1420.

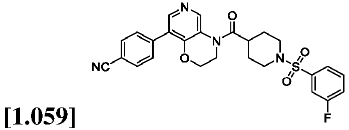

### 4-(4-(1-((3-Fluorophenyl)sulfonyl)piperidine-4-carbonyl)-3,4-dihydro-2H-pyrido[4,3-b][1,4]ox azin-8-yl)benzonitrile (O55)

Following the general method OA, synthesized with **OI4** (50 mg, 0.21 mmol), 1-((3-fluorophenyl)sulfonyl)piperidine-4-carbonyl chloride (96 mg, 0.315 mmol), DMAP (8 mg, 0.063 mmol) and triethylamine (64 mg, 0.63 mmol) to give **O55** (42 mg, 39.62% yield) as a white solid after chromatographic purification (PE/EA = 2/1), mp: 245.0-246.0 °C. ¹H-NMR (400 MHz, DMSO-*d₆*): δ 8.91 (s, 1H), 8.20 (s, 1H), 7.91 (d, *J =* 8.3 Hz, 2H), 7.75 (d, *J =* 8.3 Hz, 3H), 7.61 (d, *J* = 14.2 Hz, 3H), 4.36 (s, 2H), 3.95 (s, 2H), 3.69 (d, *J =* 11.6 Hz, 2H), 2.94 (s, 1H), 2.41 (s, 2H), 1.85 (d, *J* = 11.9 Hz, 2H), 1.64 (d, *J =* 32.9 Hz, 2H); ¹³C-NMR (101 MHz, DMSO-*d₆*): δ 173.11, 163.61, 161.14 (¹*J*_{CF} = 248.9), 149.97, 146.06, 139.44, 137.97, 137.91, 132.65(2C), 132.34, 132.26 (³*J*_{CF} *=* 7.9), 130.72 (2C), 124.29, 123.84, 120.93, 120.72 (²*J*_{CF} = 21.3), 119.24, 115.11, 114.87 (²*J*_{CF} = 24.1), 110.87, 100.07, 68.02, 60.25, 45.70, 42.61, 37.30, 34.87, 27.97.60.25, 45.70, 42.61, 37.30, 34.87, 27.97. HRMS (ESI): m/z calcd. for C₂₆H₂₃FN₄O₄S ⁺ [M+H]⁺: 507.1497, found 507.1501.

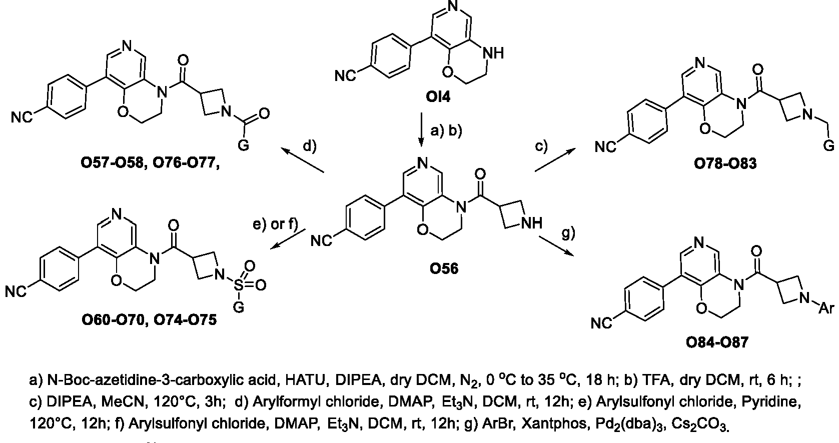

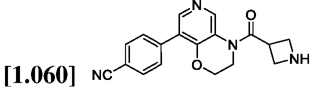

### 4-(4-(Azetidine-3-carbonyl)-3,4-dihydro-2H-pyrido[4,3-b][1,4]oxazin-8-yl)benzonitrile (O56)

To a solution of **OI4** (50 mg, 0.21 mmol) in anhydrous dichloromethane (5 mL) were added 1-Boc-azetidine-3-carboxylic acid (53 mg, 0.21 mmol), N, N-diisopropylethylamine (81 mg, 0.63 mmol) and HATU (83 mg, 0.22 mmol) at 0 °C. The reaction mixture was warmed to room temperature and stirred under N₂ for 12 hours. After the reaction was complete as monitored by TLC, the reaction mixture was diluted with water (6 mL) and extracted with dichloromethane (2×6 mL). The combined organic layers were washed with brine, dried over anhydrous sodium sulfate, and concentrated under reduced pressure. The crude product was dissolved in methanol (3 mL), and then 1 mL of hydrochloric acid was added. The mixture was stirred at room temperature for 30 minutes. After the reaction was complete as monitored by TLC, the reaction mixture was diluted with water (6 mL) and extracted with dichloromethane (2×3 mL). The aqueous phase was basified to pH 12 with NaOH and then extracted with dichloromethane (2×3 mL). The combined organic layers were washed with saturated brine, dried over anhydrous sodium sulfate, and concentrated under reduced pressure. The crude product was purified by silica gel column chromatography (PE/EA = 1/1) to afford the desired compound **O56** (35 mg, 52% yield) as a white solid. 169.0-170.0 °C. ¹H-NMR (400 MHz, DMSO-*d₆*): δ 7.89 (s, 1H), 7.87 (d, *J =* 1.9 Hz, 2H), 7.73 (d, *J* = 14.1 Hz, 3H), 6.18 (s, 1H), 4.23 (s, 2H), 3.34 (s, 2H), 1.73 (d, *J =* 58.2 Hz, 2H), 1.25 (d, *J =* 34.0 Hz, 2H); ¹³C-NMR (101 MHz, DMSO-*d₆*): δ 146.21, 140.29, 138.91, 136.84, 132.70, 132.56(2C), 130.52(2C), 123.03, 119.35, 110.41, 66.01, 34.66, 26.84, 26.73, 26.33, 26.26.

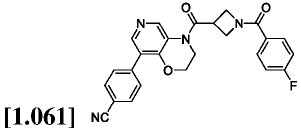

### 4-(4-(1-(4-Fluorobenzoyl)azetidine-3-carbonyl)-3,4-dihydro-2H-pyrido[4,3-b][1,4]oxazin-8-yl) benzonitrile (O57)

Following the general method OA, synthesized with **O56** (100 mg, 0.31 mmol), 4-fluorobenzoyl chloride (74 mg, 0.47 mmol), DMAP (11 mg, 0.094 mmol) and triethylamine (95 mg, 0.94 mmol) to give **O57** (50 mg, 36.50% yield) as a white solid after chromatographic purification (PE/EA = 1/1), mp: 234.0-235.0 °C. ¹H-NMR (400 MHz, DMSO-*d₆*): δ 9.19 (s, 1H), 8.22 (s, 1H), 7.92 (d, *J =* 8.3 Hz, 2H), 7.75 (d, *J =* 18.8 Hz, 4H), 7.29 (s, 2H), 4.56 (s, 1H), 4.42 (s, 3H), 4.18 (d, *J* = 92.2 Hz, 3H), 3.80 (s, 2H); ¹³C-NMR (101 MHz, DMSO-*d₆*): δ 170.35, 168.41, 165.30, 162.83 (¹*J*_{CF} = 248.7), 149.71, 145.63, 139.38, 137.35, 132.69 (2C), 130.94, 130.85 (³*J*_{CF} *=* 8.9), 130.73(2C), 129.72, 129.69 (⁴*J*_{CF} = 3.1), 124.10, 123.83, 119.24, 116.07, 115.85 (²*J*_{CF} = 21.7), 110.92, 66.99, 55.00, 50.91, 42.33, 32.47. HRMS (ESI): m/z calcd. for C₂₅H₁₉FN₄O₃ ⁺ [M+H]⁺: 443.1514, found 443.1506.

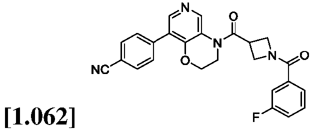

### 4-(4-(1-(3-Fluorobenzoyl)azetidine-3-carbonyl)-3,4-dihydro-2H-pyrido[4,3-b][1,4]oxazin-8-yl) benzonitrile(O58)

Following the general method OA, synthesized with **O56** (100 mg, 0.31 mmol), 3-fluorobenzoyl chloride (74 mg, 0.47 mmol), DMAP (11 mg, 0.094 mmol) and triethylamine (95 mg, 0.94 mmol) to give **O58** (89 mg, 65% yield) as a white solid after chromatographic purification (PE/EA = 1/1), mp: 78.0-80.0 °C. ¹H-NMR (400 MHz, CDCl₃): δ 8.31 (s, 1H), 8.14 (s, 1H), 7.73 (d, *J* = 8.2 Hz, 2H), 7.62 (d, *J =* 8.0 Hz, 2H), 7.41 (s, 2H), 7.33 (s, 1H), 7.18 (s, 1H), 4.66 (s, 1H), 4.43 (s, 4H), 4.32 (s, 2H), 4.18-3.85 (m, 2H); ¹³C-NMR (101 MHz, DMSO-*d₆*): δ 169.79, 168.95, 163.72 (¹*J*_{CF} = 247.9), 161.26, 150.20, 148.06, 143.67, 138.07, 134.59, 134.52 (³*J*_{CF} = 6.5), 132.14 (2C), 130.25, 130.18 (³*J*_{CF} = 7.9), 130.07 (2C), 124.38, 123.49, 123.46 (⁴*J*_{CF} = 3.0), 121.75, 118.52, 118.28 (²*J*_{CF} = 23.8), 116.03, 115.13, 114.90 (²*J*_{CF} = 22.8), 111.94, 68.20, 55.14, 51.67, 38.97, 31.81. HRMS (ESI): m/z calcd. for C₂₅H₁₉FN₄O₃ ⁺ [M+H]⁺: 443.1514, found 443.1505.

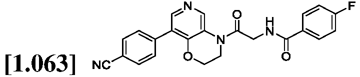

### N-(2-(8-(4-Cyanophenyl)-2,3-dihydro-4H-pyrido[4,3-b][1,4]oxazin-4-yl)-2-oxoethyl)-4-fluorob enzamide (O59)

Following the general method OE, synthesized with **OI4** (50 mg, 0.21 mmol), 4-fluorobenzoylglycine (63 mg, 0.32 mmol), N, N-diisopropylethylamine (81 mg, 0.63 mmol) and HATU (83 mg, 0.22 mmol) to give **O59** (50 mg, 56.81% yield) as a white solid after chromatographic purification (PE/EA = 2/1), mp: 220.0-221.0 °C. ¹H-NMR (400 MHz, DMSO-*d₆*): δ 9.03 (s, 1H), 8.90 (s, 1H), 8.24 (s, 1H), 7.95 (d, *J =* 23.0 Hz, 4H), 7.77 (d, *J =* 8.3 Hz, 2H), 7.32 (s, 2H), 4.44 (s, 2H), 4.40 (d, *J =* 5.5 Hz, 2H), 4.02 (s, 2H); ¹³C-NMR (101 MHz, DMSO-*d₆*): δ 167.93, 165.57, 165.23, 162.75 (¹*J*_{CF} = 248.9), 149.34, 145.79, 145.01, 138.89, 132.19 (2C), 130.27 (2C), 130.02, 129.93 (³*J*_{CF} = 9.2), 123.86, 123.43, 120.51, 118.76, 115.40, 115.19 (²*J*_{CF} = 21.9), 110.44, 71.51, 67.00, 42.04. HRMS (ESI): m/z calcd. for C₂₃H₁₈FN₃O₂⁺ [M+H]⁺: 417.1358, found 417.1363.

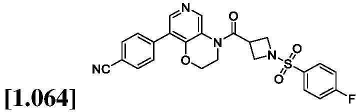

### 4-(4-(1-((4-Fluorophenyl)sulfonyl)azetidine-3-carbonyl)-3,4-dihydro-2H-pyrido[4,3-b][1,4]oxa zin-8-yl)benzonitrile (O60)

Following the general method OB, synthesized with **O56** (100 mg, 0.31 mmol) and 4-fluorobenzenesulfonyl chloride (74 mg, 0.47 mmol) to give **O60** (30 mg, 33% yield) as a white solid after chromatographic purification (PE/EA = 2/1), mp: 172.0-173.0 °C. ¹H-NMR (400 MHz, CDCl₃): δ 8.26 (s, 1H), 7.99 (s, 1H), 7.84 (d, *J =* 13.8 Hz, 2H), 7.69 (d, *J =* 8.1 Hz, 2H), 7.57 (d, *J =* 7.7 Hz, 2H), 7.22 (s, 2H), 4.32 (s, 2H), 4.02 (s, 3H), 3.94 (s, 2H), 3.66 (s, 2H); ¹³C-NMR (101 MHz, CDCl₃): δ 168.68, 166.86, 164.31 (¹*J*_{CF} = 256.1), 150.12, 148.11, 143.45, 138.01, 132.09 (2C), 131.04, 130.94 (²*J*_{CF} = 22.6), 130.51, 130.01 (2C), 124.25, 123.31, 118.49, 116.68, 116.46 (³*J*_{CF} *=* 9.4), 111.84, 68.09, 52.98, 38.90, 30.55, 20.88. HRMS (ESI): m/z calcd. for C₂₄H₁₉FN₄O₄S⁺ [M+H]⁺: 479.1184, found 479.1193.

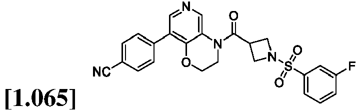

### 4-(4-(1-((3-Fluorophenyl)sulfonyl)azetidine-3-carbonyl)-3,4-dihydro-2H-pyrido[4,3-b][1,4]oxa zin-8-yl)benzonitrile (O61)

Following the general method OB, synthesized with **O56** (100 mg, 0.31 mmol) and 3-fluorobenzenesulfonyl chloride (120 mg, 0.62 mmol) to give **O61** (30 mg, 33% yield) as a white solid after chromatographic purification (PE/EA = 2/1), mp: 189.0-190.0 °C. ¹H-NMR (400 MHz, DMSO-*d₆*): δ 8.95 (s, 1H), 8.19 (s, 1H), 7.92 (d, *J =* 8.3 Hz, 2H), 7.72 (d, *J =* 52.3 Hz, 6H), 4.34 (s, 2H), 4.02 (d, *J* = 27.9 Hz, 3H), 3.87 (s, 2H), 3.70 (s, 2H); ¹³C-NMR (101 MHz, DMSO-*d₆*): δ 169.34, 163.73, 161.25 (¹*J*_{CF} = 249.1), 149.71, 145.80, 145.32, 139.36, 136.14, 136.06 (³*J*_{CF} = 6.8), 132.67 (2C), 132.42, 132.34 (³*J*_{CF} = 7.8), 130.72 (2C), 125.12, 125.09 (⁴*J*_{CF} = 2.7), 123.85, 123.84, 121.45, 121.24 (²*J*_{CF}=21.1), 119.23, 115.88, 115.64 (²*J*_{CF} = 24.2), 110.92, 66.87, 52.71, 42.16, 31.05, 21.02. HRMS (ESI): m/z calcd. for C₂₄H₁₉FN₄O₄S⁺ [M+H]⁺: 479.1184, found 479.1194.

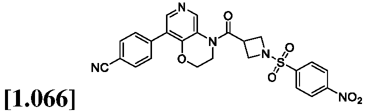

### 4-(4-(1-((4-Nitrophenyl)sulfonyl)azetidine-3-carbonyl)-3,4-dihydro-2H-pyrido[4,3-b][1,4]oxazi n-8-yl)benzonitrile (O62)

Following the general method OB, synthesized with **O56** (100 mg, 0.31 mmol) and 4-nitrobenzenesulfonyl chloride (137 mg, 0.62 mmol) to give **O62** (50 mg, 32% yield) as a yellow solid after chromatographic purification (PE/EA = 2/1), mp: 230.0-231.0 °C. ¹H-NMR (400 MHz, DMSO-*d₆*): δ 8.92 (s, 1H), 8.50 (d, *J =* 8.8 Hz, 2H), 8.18 (s, 1H), 8.13 (d, *J =* 8.7 Hz, 2H), 7.91 (d, *J* = 8.3 Hz, 2H), 7.73 (d, *J =* 7.5 Hz, 2H), 4.34 (s, 2H), 4.06 (d, *J =* 28.7 Hz, 4H), 3.78 (d, *J =* 73.2 Hz, 3H); ¹³C-NMR (101 MHz, DMSO-*d₆*): δ 168.74, 150.28, 149.26, 145.35, 144.87, 139.34, 138.85, 132.20 (2C), 130.23 (2C), 129.91, 124.71 (2C), 123.32, 119.44, 118.75, 114.55, 110.45, 66.43, 52.52, 48.59, 41.69, 30.94. HRMS (ESI): m/z calcd. for C₂₄H₁₉N₅O₆S⁺ [M+H]⁺: 506.1129, found 506.1030.

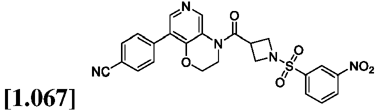

### 4-(4-(1-((3-Nitrophenyl)sulfonyl)azetidine-3-carbonyl)-3,4-dihydro-2H-pyrido[4,3-b][1,4]oxazi n-8-yl)benzonitrile (O63)

Following the general method OB, synthesized with **O56** (100 mg, 0.31 mmol) and 3-nitrobenzenesulfonyl chloride (137 mg, 0.62 mmol) to give **O63** (58 mg, 37% yield) as a white solid after chromatographic purification (PE/EA = 2/1), mp: 216.0-217.0 °C. ¹H-NMR (400 MHz, DMSO-*d₆*): δ 8.95 (s, 1H), 8.62 (s, 1H), 8.47 (s, 1H), 8.32 (s, 1H), 8.18 (s, 1H), 8.01 (s, 1H), 7.91 (d, *J* = 8.2 Hz, 2H), 7.72 (d, *J* = 7.6 Hz, 2H), 4.33 (s, 2H), 4.03 (s, 3H), 3.86 (s, 2H), 3.68 (s, 2H); ¹³C-NMR (101 MHz, DMSO-*d₆*): δ 168.81, 149.22, 148.14, 145.33, 144.93, 138.89, 135.43, 134.19, 132.20 (2C), 131.71, 130.23 (2C), 128.20, 123.36, 122.84, 119.52, 118.75, 110.44, 66.32, 54.88, 52.52, 41.56, 30.90. HRMS (ESI): m/z calcd. for C₂₄H₁₉N₅O₆S⁺ [M+H]⁺: 506.1129, found 506.1116.

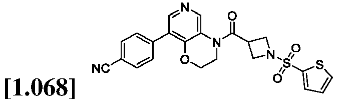

### 4-(4-(1-(Thiophen-2-ylsulfonyl)azetidine-3-carbonyl)-3,4-dihydro-2H-pyrido[4,3-b][1,4]oxazin -8-yl)benzonitrile (O64)

Following the general method OB, synthesized with **O56** (100 mg, 0.31 mmol) and 2-thiophenesulfonyl chloride (112.79 mg, 0.62 mmol) to give **O64** (58 mg, 37% yield) as a white solid after chromatographic purification (PE/EA = 2/1), mp: 176.0-177.0 °C. ¹H-NMR (400 MHz, DMSO-*d₆*): δ 8.97 (s, 1H), 8.19 (s, 1H), 8.17 (s, 1H), 7.92 (d, *J =* 8.1 Hz, 2H), 7.76 (d, *J =* 25.8 Hz, 3H), 7.37 (s, 1H), 4.34 (s, 2H), 4.07 (s, 1H), 3.99 (s, 2H), 3.88 (s, 2H), 3.72 (s, 2H); ¹³C-NMR (101 MHz, DMSO-*d₆*): δ 168.82, 160.32, 150.07, 144.89, 142.49, 134.74, 134.33, 132.74, 132.19 (2C), 130.24 (2C), 128.59, 123.37, 118.74, 110.43, 109.16, 66.43, 52.63, 41.57, 30.65, 14.13. HRMS (ESI): m/z calcd. for C₂₂H₁₈N₄O₄S₂⁺ [M+H]⁺: 467.0842, found 467.0846.

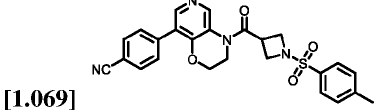

### 4-(4-(1-Tosylazetidine-3-carbonyl)-3,4-dihydro-2H-pyrido[4,3-b][1,4]oxazin-8-yl)benzonitrile (O65)

Following the general method OI, **O56** (100 mg, 0.30 mmol), 4-toluenesulfonyl chloride (85.0 mg, 0.45 mmol), DMAP (11.0 mg, 0.09 mmol) and triethylamine (91.1 mg, 0.90 mmol) to give **O65** (55 mg, 70% yield) as a white solid after chromatographic purification (PE/EA = 2/1), mp: 205.0-206.0 °C, *R*_{f} = 0.83 (CH₂Cl₂: MeOH (V/V) = 40/1) . ¹H-NMR (400 MHz, CDCl₃): *δ* 8.29 (s, 1H), 8.02 (s, 1H), 7.78-7.73 (m, 2H), 7.73-7.70 (m, 2H), 7.60 (d, *J* = 8.2 Hz, 2H), 7.39 (d, *J* = 8.2 Hz, 2H), 4.34 (t, *J =* 4.4 Hz, 2H), 4.08-3.98 (m, 3H), 3.98-3.83 (m, 2H), 3.83-3.52 (m, 2H), 2.47 (s, 3H); ¹³C-NMR (101 MHz, DMSO-*d*₆): *δ* 168.94, 149.28, 145.21, 145.06, 144.15, 138.87, 132.19 (2C), 130.51, 130.25 (2C), 129.99 (2C), 128.36 (2C), 123.39 (2C), 118.76, 110.46, 66.48, 52.13, 41.64 (2C), 30.90, 21.08; HRMS (ESI): m/z calcd. for C₂₅H₂₂N₄O₄S [M+H]⁺: 475.1435, found: 475.1426.

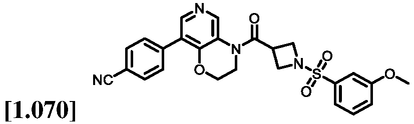

### 4-(4-(1-((3-Methoxyphenyl)sulfonyl)azetidine-3-carbonyl)-3,4-dihydro-2H-pyrido[4,3-b][1,4]o xazin-8-yl)benzonitrile (O66)

Following the general method OI, synthesized with **O56** (85.0 mg, 0.25 mmol), 3-methoxybenzenesulphonyl chloride (78.5 mg, 0.38 mmol), DMAP (9.8 mg, 0.08 mmol) and triethylamine (75.9 mg, 0.75 mmol) to give **O66** (60.7 mg, 48.9% yield) as a white solid after chromatographic purification (PE/EA = 2/1), mp: 206.0-207.0 °C, *R*_{f} = 0.30 (PE/EA = 1/20). ¹H-NMR (400 MHz, CDCl₃): *δ* 8.29 (s, 1H), 8.03 (s, 1H), 7.74 (d, *J =* 8.3 Hz, 2H), 7.61 (d, *J =* 8.2 Hz, 2H), 7.51 (t, *J =* 8.0 Hz, 1H), 7.42 (d, *J =* 7.8 Hz, 1H), 7.34 (s, 1H), 7.20 (dd, *J =* 7.8, 3.1 Hz, 1H), 4.35 (t, *J =* 4.7 Hz, 2H), 4.17-4.01 (m, 3H), 4.00-3.92 (m, 2H), 3.88 (s, 3H), 3.87-3.48 (m, 2H); ¹³C-NMR (101 MHz, DMSO-*d*₆): *δ* 168.93, 159.68, 149.31, 144.93 (2C), 138.90, 134.68, 132.20 (2C), 130.77, 130.25 (2C), 123.37 (2C), 120.40, 119.55, 118.76, 112.99, 110.45, 66.46, 55.72, 52.27, 41.66 (2C), 30.85; HRMS (ESI): m/z calcd. for C₂₅H₂₂N₄O₅S [M+H]⁺: 491.1384, found: 491.1382.

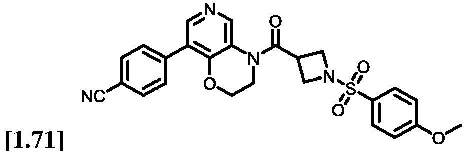

### 4-(4-(1-((4-Methoxyphenyl)sulfonyl)azetidine-3-carbonyl)-3,4-dihydro-2H-pyrido[4,3-b][1,4]o xazin-8-yl)benzonitrile (O67)

Following the general method OI, synthesized with **O56** (100 mg, 0.30 mmol), 4-methoxybenzenesulphonyl chloride (92.2 mg, 0.45 mmol), DMAP (11.0 mg, 0.09 mmol) and triethylamine (91.1 mg, 0.90 mmol) to give **O67** (56.5 mg, 38.8% yield) as a white solid after chromatographic purification (PE/EA = 2/1), mp: 204.0-205.0 °C, *R*_{f} *=* 0.63 (CH₂Cl₂: MeOH (V/V) = 40:1) . ¹H-NMR (400 MHz, CDCl₃): *δ* 8.28 (s, 1H), 8.03 (s, 1H), 7.78 (d, *J =* 8.8 Hz, 2H), 7.73 (d, *J* = 8.3 Hz, 2H), 7.60 (d, *J* = 8.1 Hz, 2H), 7.05 (d, *J* = 8.8 Hz, 2H), 4.34 (t, *J* = 4.8 Hz, 2H), 4.06-3.97 (m, 3H), 3.97-3.91 (m, 2H), 3.90 (s, 3H), 3.88-3.65 (m, 2H); ¹³C-NMR (101 MHz, DMSO-*d*₆): *δ* 168.98, 163.12, 149.30, 144.85 (2C), 138.87, 132.20 (2C), 130.58, 130.24 (2C), 124.93 (2C), 123.39 (2C), 118.75, 114.67 (2C), 110.45, 66.56, 55.77, 52.02, 41.64 (2C), 30.85; HRMS (ESI): m/z calcd. for C₂₅H₂₂N₄O₅S [M+H]⁺: 491.1384, found: 491.1375.

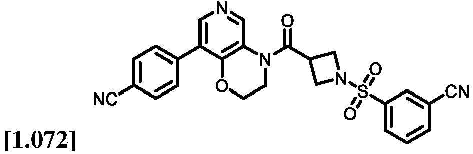

### 3-((3-(8-(4-Cyanophenyl)-3,4-dihydro-2H-pyrido[4,3-b][1,4]oxazine-4-carbonyl)azetidin-1-yl)s ulfonyl)benzonitrile (O68)

Following the general method OI, synthesized with **O56** (100 mg, 0.30 mmol), 3-cyanobenzenesulfonyl chloride (90.0 mg, 0.45 mmol), DMAP (11.0 mg, 0.09 mmol) and triethylamine (91.1 mg, 0.90 mmol) to give **O68** (117.1 mg, 81.3% yield) as a white solid after chromatographic purification (PE/EA =1/1), mp: 182.0-183.0 °C, *R*_{f} = 0.37 (PE/EA (V/V) = 1/2) . ¹H-NMR (400 MHz, CDCl₃): *δ* 8.32 (s, 1H), 8.14 (s, 1H), 8.09 (d, *J* = 8.1 Hz, 1H), 8.01 (s, 1H), 7.95 (d, *J =* 7.8 Hz, 1H), 7.77 (d, *J* = 7.9 Hz, 1H), 7.73 (d, *J =* 8.8 Hz, 2H), 7.61 (d, *J =* 8.3 Hz, 2H), 4.37 (t, *J =* 4.8 Hz, 2H), 4.16-4.02 (m, 4H), 4.00-3.95 (m, 1H), 3.94-3.69 (m, 2H); ¹³C-NMR (101 MHz, CDCl₃): *δ* 168.63, 150.38, 148.32, 143.57, 138.11, 136.91, 136.62, 132.25, 132.17 (2C), 131.77, 130.50, 130.16 (2C), 124.47, 123.38, 118.65, 117.20, 113.98, 112.02, 68.24, 53.50, 39.10 (2C), 30.48; HRMS (ESI): m/z calcd. for C₂₅H₁₉N₅O₄S [M+H]⁺: 486.1231, found: 486.1234.

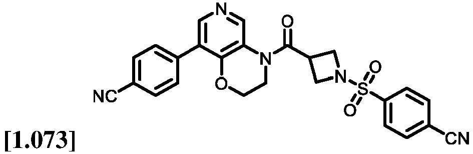

### 4-((3-(8-(4-Cyanophenyl)-3,4-dihydro-2H-pyrido[4,3-b][1,4]oxazine-4-carbonyl)azetidin-1-yl)s ulfonyl)benzonitrile (O69)

Following the general method OI, synthesized with **O56** (100 mg, 0.30 mmol), 4-cyanobenzenesulfonyl chloride (90.0 mg, 0.45 mmol), DMAP (11.0 mg, 0.09 mmol) and triethylamine (91.1 mg, 0.90 mmol) to give **O69** (119.2 mg, 82.7% yield) as a white solid after chromatographic purification (PE/EA =1/1), mp: 226.0-227.0 °C, *R*_{f} = 0.21 (PE/EA (V/V) = 1/2.5). ¹H-NMR (400 MHz, CDCl₃): *δ* 8.32 (s, 1H), 8.18-7.92 (m, 3H), 7.90 (d, *J =* 8.3 Hz, 2H), 7.73 (d, *J* = 8.3 Hz, 2H), 7.60 (d, *J =* 8.2 Hz, 2H), 4.37 (t, *J =* 4.8 Hz, 2H), 4.18-4.02 (m, 4H), 4.00-3.95 (m, 1H), 3.95-3.62 (m, 2H); ¹³C-NMR (101 MHz, DMSO-*d*₆): *δ* 168.78, 149.27, 145.35, 144.59, 138.90, 137.88, 133.64 (2C), 132.21 (2C), 130.24 (2C), 129.02 (2C), 123.38 (2C), 118.77, 117.62, 116.01, 110.47, 66.44, 52.48, 41.71 (2C), 30.85; HRMS (ESI): m/z calcd. for C₂₅H₁₉N₅O₄S [M+H]⁺: 486.1231, found: 486.1230.

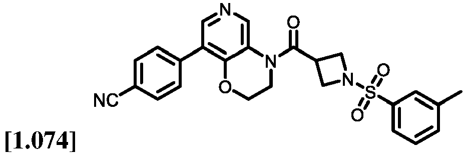

### 4-(4-(1-(m-Tolylsulfonyl)azetidine-3-carbonyl)-3,4-dihydro-2H-pyrido[4,3-b][1,4]oxazin-8-yl)b enzonitrile (O70)

Following the general method OI, synthesized with **O56** (85 mg, 0.25 mmol), 3-toluenesulfonyl chloride (72.4 mg, 0.38 mmol), DMAP (9.8 mg, 0.08 mmol) and triethylamine (75.9 mg, 0.75 mmol) to give **O70** (39.1 mg, 32.6 % yield) as a white solid after chromatographic purification (PE/EA = 2/1), mp: 227.0-228.0 °C, *R*_{f} = 0.38 (PE/EA (V/V) = 1/2). ¹H-NMR (400 MHz, CDCl₃): *δ* 8.29 (s, 1H), 8.03 (s, 1H), 7.72 (d, *J =* 8.2 Hz, 2H), 7.65 (d, *J =* 4.9 Hz, 2H), 7.61 (t, *J* = 7.6 Hz, 2H), 7.47 (d, *J =* 5.0 Hz, 2H), 4.34 (t, *J =* 4.8 Hz, 2H), 4.16-4.00 (m, 3H), 3.98-3.88 (m, 2H), 3.88-3.57 (m, 2H), 2.46 (s, 3H); ¹³C-NMR (101 MHz, DMSO-*d*₆): *δ* 168.93, 149.23, 145.31, 144.90, 139.36, 138.89, 134.30, 133.46, 132.19 (2C), 130.23 (2C), 129.37, 128.38 (2C), 125.45, 123.34, 118.75, 110.44, 66.48, 52.16, 41.65 (2C), 30.85, 20.84; HRMS (ESI): m/z calcd. for C₂₅H₂₂N₄O₄S [M+H]⁺: 475.1435, found: 475.1437.

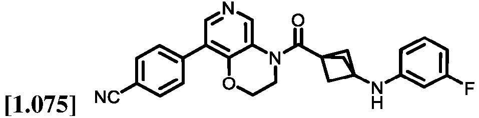

### 4-(4-(3-((3-Fluorophenyl)amino)bicyclo[1.1.1]pentane-1-carbonyl)-3,4-dihydro-2H-pyrido[4,3-b][1,4]oxazin-8-yl)benzonitrile (O71)

Following the general method OE, synthesized with **OI4** (100 mg, 0.42 mmol), 3-((3-fluorophenyl)amino)bicyclo[1.1.1]pentane-1-carboxylic acid (139 mg, 0.63 mmol), N,N-diisopropylethylamine (163 mg, 1.26 mmol) and HATU (168 mg, 0.44 mmol) to give **O71** (59 mg, 32% yield) as a light yellow oil after chromatographic purification (PE/EA = 1/1). ¹H-NMR (400 MHz, CDCl₃): *δ* 8.56(s, 1H), 8.03 (s, 2H), 7.75 (d, *J =* 8.3 Hz, 2H), 7.60 (d, *J =* 8.1 Hz, 2H), 7.33-7.27 (m, 1H), 7.14-6.98 (m, 2H), 6.96-6.88 (m, 1H), 4.42 (t, *J* =4.8 Hz, 2H), 3.85 (t, *J =*4.8 Hz, 2H), 2.17-2.13 (m, 3H), 1.99-2.07 (m, 3H). HRMS (ESI): m/z calcd. for C₂₆H₂₂FN₄O₂ [M+H]⁺: 441.1721, found: 441.1712.

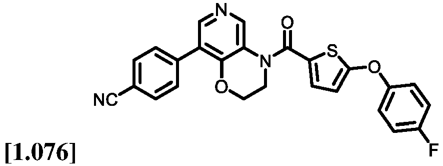

### 4-(4-(5-(4-Fluorophenoxy)thiophene-2-carbonyl)-3,4-dihydro-2H-pyrido[4,3-b][1,4]oxazin-8-y l)benzonitrile (O72)

Following the general method OA, synthesized with **OI4** (132.8 mg, 0.56 mmol), (4-fluorophenoxy)thiophene-2-carbonyl chloride (215.6 mg, 0.84 mmol), DMAP (20.8 mg, 0.17 mmol) and triethylamine (170.0 mg, 1.68 mmol) to give **O72** (152.5 mg, 59.5% yield) as a white solid after chromatographic purification (PE/EA = 1/1), mp: 79.0-80.0 °C, *R*_{f} = 0.50 (PE: EA (V/V) = 1:2). ¹H-NMR (400 MHz, CDCl₃): *δ* 8.55 (s, 1H), 8.20 (s, 1H), 7.73 (d, *J =* 8.4 Hz, 2H), 7.64 (d, *J* = 8.4 Hz, 2H), 7.19-7.11 (m, 3H), 7.10-7.02 (m, 2H), 6.35 (d, *J* = 4.2 Hz, 2H), 4.45 (t, *J =* 4.7 Hz, 2H), 4.17-4.11 (m, 3H). ¹³C-NMR (101 MHz, CDCl₃): *δ* 168.22, 162.19, 161.15 (¹*J*_{CF} = 245.3 Hz), 153.41, 149.30, 146.28, 146.04, 138.83, 132.25 (2C), 131.58, 130.25 (2C), 125.08, 124.22, 124.09, 120.49 (³*J*_{CF} = 8.6 Hz) (2C), 118.79, 116.95 (²*J*_{CF} = 23.8 Hz) (2C), 111.87, 111.03, 67.52, 42.61; ¹⁹F NMR (376 MHz, CDCl₃): *δ* -116.97; HRMS (ESI): m/z calcd. for C₂₅H₁₆FN₃O₃S [M+H]⁺: 458.0969, found: 458.0956.

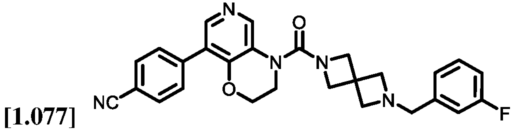

### 4-(4-(6-(3-Fluorobenzyl)-2,6-diazaspiro[3.3]heptane-2-carbonyl)-3,4-dihydro-2H-pyrido[4,3-b] [1,4]oxazin-8-yl)benzonitrile (O73)

To a solution of **OI4** (100 mg, 0.42 mmol) and pyridine (79 mg, 0.63 mmol) in chloroform (10 mL) in an ice bath, a chloroform solution (3 mL) of triphosgene (623 mg, 2.1 mmol) was slowly added dropwise. The reaction mixture was stirred under N₂ at 60 °C for 12 hours. After the reaction was complete as monitored by TLC, 1 N HCl aqueous solution (10 mL) was added dropwise. The mixture was extracted with chloroform, and the organic phase was washed successively with saturated Na₂CO₃ aqueous solution and brine, dried over anhydrous sodium sulfate, and concentrated under reduced pressure to obtain an oily intermediate. This intermediate was dissolved in anhydrous 1,4-dioxane (10 mL). Subsequently, triethylamine (0.63 mmol) and 2-(3-fluorobenzyl)-2,6-diazabicyclo[3.3.0]octane (475 mg, 0.63 mmol) were added. The reaction was carried out under N₂ at room temperature for 12 hours. After the reaction was complete as monitored by TLC, the mixture was concentrated under vacuum. The resulting oil was extracted with chloroform/water, the organic phases were combined, washed successively with saturated Na₂CO₃ aqueous solution and saturated brine, dried over anhydrous sodium sulfate, and concentrated under reduced pressure to obtain the crude product. The crude product was purified by silica gel column chromatography (PE/EA) to give **O73.**¹H-NMR (400 MHz, CDCl₃): *δ* 8.72 (s, 1H), 8.19 (s, 1H), 7.76 (d, *J =* 8.4 Hz, 2H), 7.65 (d, *J =* 8.4 Hz, 2H), 7.36-7.25 (m, 1H), 7.13-6.98 (m, 2H), 6.95-6.83 (m, 1H), 4.42 (t, *J* = 4.8 Hz, 2H), 3.80-3.72 (m, 4H), 3.61-3.58 (m, 3H), 3.25-3.17 (m, 4H). HRMS (ESI): m/z calcd. for C₂₇H₂₅FN₅O₂ [M+H]⁺: 470.5279, found: 470.5283.

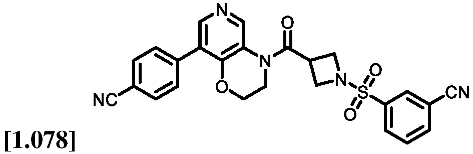

### 3-((3-(8-(4-Cyanophenyl)-3,4-dihydro-2H-pyrido[4,3-b][1,4]oxazine-4-carbonyl)azetidin-1-yl)s ulfonyl)benzonitrile (O74)

Following the general method OB, synthesized with **O56** (100 mg, 0.30 mmol), 3-cyanobenzene-1-sulfonyl chloride (90 mg, 0.45 mmol), DMAP (11.0 mg, 0.09 mmol) and triethylamine (91.1 mg, 0.90 mmol) to give **O74** (117.1 mg, 81.3% yield) as a white solid after chromatographic purification (PE/EA = 1/2), mp: 182.0-183.0 °C, *R*_{f} = 0.37 (PE/EA (V/V) = 1/2). ¹H-NMR (400 MHz, CDCl₃): *δ* 8.32 (s, 1H), 8.14 (s, 1H), 8.09 (d, *J* = 8.1 Hz, 1H), 8.01 (s, 1H), 7.95 (d, *J =* 7.8 Hz, 1H), 7.77 (d, *J* = 7.9 Hz, 1H), 7.73 (d, *J =* 8.8 Hz, 2H), 7.61 (d, *J =* 8.3 Hz, 2H), 4.37 (t, *J =* 4.8 Hz, 2H), 4.16-4.02 (m, 4H), 4.00-3.95 (m, 1H), 3.94-3.69 (m, 2H); ¹³C-NMR (101 MHz, CDCl₃): *δ* 168.63, 150.38, 148.32, 143.57, 138.11, 136.91, 136.62, 132.25, 132.17 (2C), 131.77, 130.50, 130.16 (2C), 124.47, 123.38, 118.65, 117.20, 113.98, 112.02, 68.24, 53.50, 39.10 (2C), 30.48; HRMS (ESI): m/z calcd. for C₂₅H₁₉N₅O₄S [M+H]⁺: 486.1231, found: 486.1234.

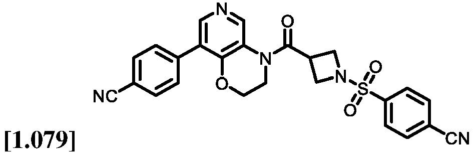

### 4-((3-(8-(4-Cyanophenyl)-3,4-dihydro-2H-pyrido[4,3-b][1,4]oxazine-4-carbonyl)azetidin-1-yl)sulfonyl)benzonitrile (O75)

Following the general method OB, synthesized with **O56** (100 mg, 0.30 mmol), 4-cyanobenzene-1-sulfonyl chloride (90 mg, 0.45 mmol), DMAP (11.0 mg, 0.09 mmol) and triethylamine (91.1 mg, 0.90 mmol) to give **O75** (119.2 mg, 82.7% yield) as a white solid after chromatographic purification (PE/EA = 1/1), mp: 226.0-227.0 °C, *R*_{f} = 0.21 (PE/EA (V/V) = 1/2.5). ¹H-NMR (400 MHz, CDCl₃): *δ* 8.32 (s, 1H), 8.18-7.92 (m, 3H), 7.90 (d, *J =* 8.3 Hz, 2H), 7.73 (d, *J* = 8.3 Hz, 2H), 7.60 (d, *J* = 8.2 Hz, 2H), 4.37 (t, *J* = 4.8 Hz, 2H), 4.18-4.02 (m, 4H), 4.00-3.95 (m, 1H), 3.95-3.62 (m, 2H); ¹³C-NMR (101 MHz, DMSO-*d*₆): *δ* 168.78, 149.27, 145.35, 144.59, 138.90, 137.88, 133.64 (2C), 132.21 (2C), 130.24 (2C), 129.02 (2C), 123.38 (2C), 118.77, 117.62, 116.01, 110.47, 66.44, 52.48, 41.71 (2C), 30.85; HRMS (ESI): m/z calcd. for C₂₅H₁₉N₅O₄S [M+H]⁺: 486.1231, found: 486.1230.

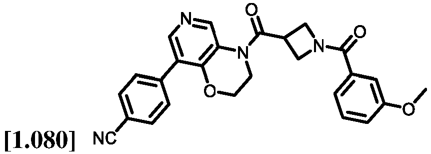

### 4-(4-(1-(3-Methoxybenzoyl)azetidine-3-carbonyl)-3,4-dihydro-2H-pyrido[4,3-b][1,4]oxazin-8-y l)benzonitrile (O76)

Following the general method OA, synthesized with **O56** (100 mg, 0.30 mmol), 3-methoxybenzoyl chloride (90 mg, 0.45 mmol), DMAP (11.0 mg, 0.09 mmol) and triethylamine (91.1 mg, 0.90 mmol) to give **O76** (125.2 mg, 92.8% yield) as a white solid after chromatographic purification (CH₂Cl₂/MeOH = 50/1), mp: 104.0-105.0 °C, *R*_{f} *=* 0.87 (CH₂Cl₂/MeOH (V/V) = 10/1). ¹H-NMR (400 MHz, CDCl₃): *δ* 8.31 (s, 1H), 8.14 (s, 1H), 7.74 (d, *J =* 8.2 Hz, 2H), 7.62 (d, *J =* 8.2 Hz, 2H), 7.32 (t, *J =* 7.9 Hz, 1H), 7.17 (d, *J =* 10.6 Hz, 2H), 7.01 (d, *J =* 8.2 Hz, 1H), 4.74-4.59 (m, 1H), 4.56-4.38 (m, 4H), 4.36-4.27 (m, 2H), 4.26-3.97 (m, 2H), 3.83 (s, 3H); ¹³C-NMR (101 MHz, CDCl₃): *δ* 170.44, 167.28, 159.80, 150.21, 148.27, 143.97 (2C), 138.34, 133.91, 132.31 (2C), 130.23 (2C), 129.62, 124.50, 123.64, 120.06, 118.69, 117.64, 113.09, 112.08, 68.29, 55.55, 51.55, 39.35, 32.12; HRMS (ESI): m/z calcd. for C₂₆H₂₂N₄O₄ [M+H]⁺: 455.1714, found: 455.1711.

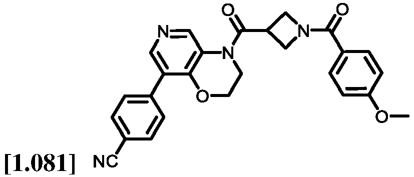

### 4-(4-(1-(4-Methoxybenzoyl)azetidine-3-carbonyl)-3,4-dihydro-2H-pyrido[4,3-b][1,4]oxazin-8-y l)benzonitrile (O77)

Following the general method OA, synthesized with **O56** (100 mg, 0.30 mmol), 4-methoxybenzoyl chloride (76.1 mg, 0.45 mmol), DMAP (11.0 mg, 0.09 mmol) and triethylamine (91.1 mg, 0.90 mmol) to give **O77** (119.9 mg, 88.9% yield) as a white solid after chromatographic purification (CH₂Cl₂/MeOH = 20/1), mp: 84.0-85.0 °C. *R*_{f} = 0.83 (CH₂Cl₂/MeOH (V/V) = 10/1). ¹H-NMR (400 MHz, CDCl₃): *δ* 8.31 (s, 1H), 8.15 (s, 1H), 7.74 (d, *J =* 8.4 Hz, 2H), 7.65-7.63 (m, 2H), 7.63-7.59 (m, 2H), 6.91 (d, *J =* 8.8 Hz, 2H), 4.93-4.56 (m, 1H), 4.55-4.37 (m, 4H), 4.36-4.15 (m, 2H), 4.14-3.85 (m, 2H), 3.84 (s, 3H); ¹³C-NMR (101 MHz, CDCl₃): *δ* 170.31, 167.42, 162.13, 150.20, 148.12, 143.91, 138.35, 132.28 (2C), 130.23 (2C), 129.97 (2C), 124.83, 124.46, 123.88, 118.69, 113.85 (2C), 112.02, 68.38, 55.51 (2C), 51.89, 39.17, 32.07; HRMS (ESI): m/z calcd. for C₂₆H₂₂N₄O₄ [M+H]⁺: 455.1714, found: 455.1712.

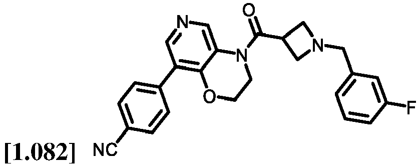

### 4-(4-(1-(3-Fluorobenzyl)azetidine-3-carbonyl)-3,4-dihydro-2H-pyrido[4,3-b][1,4]oxazin-8-yl)b enzonitrile (O78)

Following the general method OF, synthesized with **O56** (95.0 mg, 0.28 mmol), 3-fluorobenzyl chloride (61.3 mg, 0.42 mmol), K₂CO₃ (77.4 mg, 0.56 mmol) to give **O78** (35.0 mg, 28.9% yield) as a white solid after chromatographic purification (CH₂Cl₂/MeOH = 50/1), mp: 79.0-80.0 °C. *R*_{f} = 0.21 (ethyl acetate). ¹H-NMR (400 MHz, CDCl₃): *δ* 8.51 (s, 1H), 8.02 (s, 1H), 7.72 (d, *J =* 8.3 Hz, 2H), 7.62 (d, *J =* 8.1 Hz, 2H), 7.31-7.22 (m, 1H), 7.09-6.98 (m, 2H), 6.97-6.89 (m, 1H), 4.38 (t, *J* =4.8 Hz, 2H), 4.17-3.72 (m, 3H), 3.69-3.53 (m, 4H), 3.51-3.36 (m, 2H); ¹³C-NMR (101 MHz, CDCl₃): *δ* 170.60, 164.20 (¹*J*_{CF} = 246.7 Hz), 149.85, 147.57, 144.34, 140.15, 138.64, 132.18 (2C), 130.19 (2C), 130.01 (³*J*_{CF} = 8.1 Hz), 124.13, 124.07, 123.78, 118.71, 115.39, 114.32 (²*J*_{CF} = 21.4 Hz), 111.81, 68.29, 62.60, 57.02 (2C), 39.03, 33.64; ¹⁹F NMR (376 MHz, CDCl₃): *δ* -113.34; HRMS (ESI): m/z calcd. for C₂₅H₂₁FN₄O₂ [M+H]⁺: 429.1721, found: 429.1728.

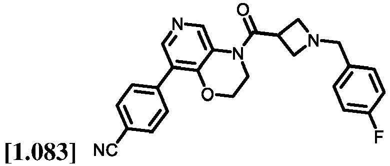

### 4-(4-(1-(4-Fluorobenzyl)azetidine-3-carbonyl)-3,4-dihydro-2H-pyrido[4,3-b][1,4]oxazin-8-yl)b enzonitrile (O79)

Following the general method OF, synthesized with **O56** (100.0 mg, 0.30 mmol), 4-fluorobenzyl chloride (64.5 mg, 0.45 mmol), K₂CO₃ (82.9 mg, 0.60 mmol) to give **O79** (47.0 mg, 36.9% yield) as a white solid after chromatographic purification (CH₂Cl₂/MeOH = 25/1), mp: 155.0-156.0 °C. *R*_{f} = 0.16 (ethyl acetate). ¹H-NMR (400 MHz, CDCl₃): *δ* 8.27 (s, 1H), 8.18 (s, 1H), 7.73 (d, *J* = 8.3 Hz, 2H), 7.62 (d, *J =* 8.3 Hz, 2H), 7.26-7.21 (m, 2H), 6.99 (t, *J =* 8.7 Hz, 2H), 4.38 (t, *J* = 5.1 Hz, 2H), 4.14-3.65 (m, 3H), 3.61 (s, 2H), 3.60-3.52 (m, 2H), 3.40 (t, *J =* 7.6 Hz, 2H); ¹³C-NMR (101 MHz, CDCl₃): *δ* 170.60, 163.42 (¹*J*_{CF} = 245.9 Hz), 150.84, 147.68, 143.90, 138.76, 133.31, 132.24 (2C), 130.23 (2C), 130.18 (³*J*_{CF} = 8.1 Hz, 2C), 124.18, 123.60, 118.74, 115.48 (²*J*_{CF} = 21.4 Hz, 2C), 111.91, 68.04, 62.54, 57.03 (2C), 39.04, 33.52; ¹⁹F NMR (376 MHz, CDCl₃): *δ* -115.49; HRMS (ESI): m/z calcd. for C₂₅H₂₁FN₄O₂ [M+H]⁺: 429.1721, found: 429.1724.

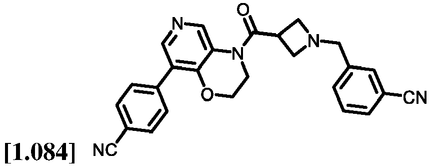

### 3-((3-(8-(4-Cyanophenyl)-3,4-dihydro-2H-pyrido[4,3-b][1,4]oxazine-4-carbonyl)azetidin-1-yl) methyl)benzonitrile (O80)

Following the general method OF, synthesized with **O56** (100.0 mg, 0.30 mmol), 3-cyanobenzyl bromide (87.4 mg, 0.45 mmol), K₂CO₃ (82.9 mg, 0.60 mmol) to give **O80** (51.4 mg, 39.7% yield) as a white solid after chromatographic purification (CH₂Cl₂/MeOH = 20/1), mp: 83.0-84.0 °C. *R*_{f} = 0.21 (ethyl acetate). ¹H-NMR (400 MHz, CDCl₃): *δ* 8.28 (s, 1H), 8.17 (s, 1H), 7.72 (d, *J =* 8.3 Hz, 2H), 7.67-7.57 (m, 3H), 7.53 (t, *J =* 7.2 Hz, 2H), 7.43 (d, *J =* 7.7 Hz, 1H), 4.39 (t, *J* = 4.8 Hz, 2H), 4.15-3.72 (m, 3H), 3.68 (s, 2H), 3.65-3.52 (m, 2H), 3.44 (t, *J* = 7.4 Hz, 2H); ¹³C-NMR (101 MHz, CDCl₃): *δ* 170.62, 149.98, 147.60, 144.10, 139.27, 138.59, 132.94, 132.24 (2C), 131.96, 131.12, 130.21 (2C), 129.38, 124.22, 123.81, 118.88, 118.72, 112.65, 111.91, 68.52, 62.29, 57.15 (2C), 39.09, 33.83; HRMS (ESI): m/z calcd. for C₂₆H₂₁N₅O₂ [M+H]⁺: 436.1768, found: 436.1757.

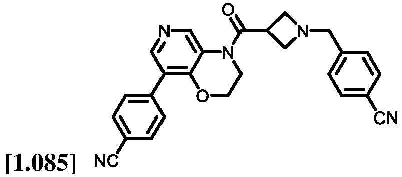

### 4-(4-(1-(4-Cyanobenzyl)azetidine-3-carbonyl)-3,4-dihydro-2H-pyrido[4,3-b][1,4]oxazin-8-yl)b enzonitrile (O81)

Following the general method OF, synthesized with **O56** (110.0 mg, 0.33 mmol) and 4-cyanobenzyl bromide (96.1 mg, 0.49 mmol), K₂CO₃ (82.9 mg, 0.60 mmol) to give **O81** (61.8 mg, 43.4% yield) as a white solid after chromatographic purification (CH₂Cl₂/MeOH = 20/1), mp: 168.0-169.0 °C. *R*_{f} = 0.17 (ethyl acetate). ¹H-NMR (400 MHz, CDCl₃): *δ* 8.28 (s, 1H), 8.11 (s, 1H), 7.72 (d, *J* = 8.4 Hz, 2H), 7.67-7.56 (m, 4H), 7.40 (d, *J* = 8.2 Hz, 2H), 4.39 (t, *J* = 4.9 Hz, 2H), 4.12-3.74 (m, 3H), 3.71 (s, 2H), 3.64-3.53 (m, 2H), 3.44 (t, *J =* 7.4 Hz, 2H); ¹³C-NMR (101 MHz, CDCl₃): *δ* 170.65, 150.09, 147.76, 144.26, 143.34, 138.54, 132.38 (2C), 132.25 (2C), 130.21 (2C), 129.05 (2C), 124.23, 123.85, 118.94, 118.72, 111.94, 111.17, 68.37, 62.74, 57.25 (2C), 39.02, 34.00; HRMS (ESI): m/z calcd. for C₂₆H₂₁N₅O₂ [M+H]⁺: 436.1768, found: 436.1758.

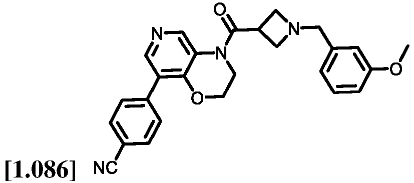

### 4-(4-(1-(3-Methoxybenzyl)azetidine-3-carbonyl)-3,4-dihydro-2H-pyrido[4,3-b][1,4]oxazin-8-yl )benzonitrile (O82)

Following the general method OF, synthesized with **O56** (100.0 mg, 0.30 mmol), 3-methoxybenzyl bromide (70.0 mg, 0.45 mmol), K₂CO₃ (82.9 mg, 0.60 mmol) to give **O82** (45.0 mg, 34.3% yield) as a white solid after chromatographic purification (CH₂Cl₂/MeOH = 25/1), mp: 135.0-136.0 °C. *R*_{f} = 0.16 (ethyl acetate). ¹H-NMR (400 MHz, CDCl₃): *δ* 8.41-7.98 (m, 2H), 7.73 (d, *J =* 8.4 Hz, 2H), 7.62 (d, *J =* 8.3 Hz, 2H), 7.22 (t, *J =* 7.8 Hz, 1H), 6.89-6.83 (m, 2H), 6.80 (dd, *J =* 7.8, 3.0 Hz, 1H), 4.46-4.30 (m, 2H), 4.13-3.84 (m, 2H), 3.81-3.67 (m, 4H), 3.65-3.55 (m, 4H), 3.42 (t, *J* = 7.5 Hz, 2H); ¹³C-NMR (101 MHz, CDCl₃): *δ* 170.87, 159.85, 149.95, 147.73, 144.41, 139.08, 138.68, 132.23 (2C), 130.22 (2C), 129.53, 124.17, 123.53, 120.91, 118.75, 113.94, 113.00, 111.87, 68.24, 63.29, 57.03 (2C), 55.33, 39.08, 33.78; HRMS (ESI): m/z calcd. for C₂₆H₂₄N₄O₃ [M+H]⁺: 441.1921, found: 441.1925.

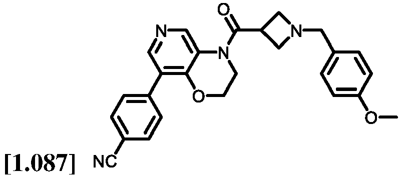

### 4-(4-(1-(4-Methoxybenzyl)azetidine-3-carbonyl)-3,4-dihydro-2H-pyrido[4,3-b][1,4]oxazin-8-yl )benzonitrile (O83)

Following the general method OF, synthesized with **O56** (100.0 mg, 0.30 mmol), 1-(chloromethyl)-4-methoxybenzene (69.8 mg, 0.45 mmol), K₂CO₃ (82.9 mg, 0.60 mmol) to give **O83** (47.3 mg, 35.8% yield) as a white solid after chromatographic purification (CH₂Cl₂/MeOH = 20/1), mp: 134.0-135.0 °C. *R*_{f} = 0.09 (ethyl acetate).¹H-NMR (400 MHz, CDCl₃): *δ* 8.37-7.93 (m, 2H), 7.73 (d, *J =* 8.6 Hz, 2H), 7.62 (d, *J =* 8.4 Hz, 2H), 7.20 (d, *J =* 8.7 Hz, 2H), 6.85 (d, *J =* 8.7 Hz, 2H), 4.43-4.32 (m, 2H), 4.07-3.82 (m, 2H), 3.82-3.68 (m, 4H), 3.62-3.53 (m, 4H), 3.39 (t, *J =* 7.3 Hz, 2H); ¹³C-NMR (101 MHz, CDCl₃): *δ* 170.69, 158.97, 149.97, 147.74, 144.30, 138.66, 132.22 (2C), 130.22 (2C), 129.82 (2C), 129.56, 124.13, 123.42, 118.74, 113.93 (2C), 111.86, 68.18, 62.72, 56.88 (2C), 55.35, 39.06, 33.83; HRMS (ESI): m/z calcd. for C₂₆H₂₄N₄O₃ [M+H]⁺: 441.1921, found: 441.1926.

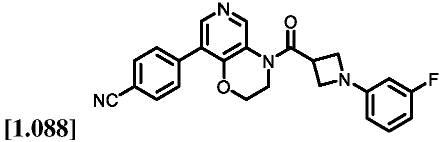

### 4-(4-(1-(3-Fluorophenyl)azetidine-3-carbonyl)-3,4-dihydro-2H-pyrido[4,3-b][1,4]oxazin-8-yl)b enzonitrile (O84)

Following the general method OG, synthesized with **O56** (100.0 mg, 0.30 mmol), 3-bromofluorobenzene (104.0 mg, 0.59 mmol), Cs₂CO₃ (293.2 mg, 0.90 mmol) and Pd₂(dba)₃ (18.3 mg, 0.02 mmol) to give **O84** (82.6 mg, 67.0% yield) as a white solid after chromatographic purification (PE/EA = 3/1), mp: 176.0-177.0 °C. *R*_{f} = 0.38 (PE/EA (V/V) = 1/2). ¹H-NMR (400 MHz, CDCl₃): *δ* 8.32 (s, 1H), 8.23 (s, 1H), 7.75 (d, *J* = 8.3 Hz, 2H), 7.64 (d, *J* = 8.3 Hz, 2H), 7.20-7.10 (m, 1H), 6.45 (t, *J* = 9.7 Hz, 1H), 6.22 (d, *J* = 8.1 Hz, 1H), 6.15 (d, *J =* 11.0 Hz, 1H), 4.43 (t, *J* = 4.8 Hz, 2H), 4.20-3.78 (m, 7H); ¹³C-NMR (101 MHz, CDCl₃): *δ* 170.38, 165.14 (¹*J*_{CF} *=* 244.5 Hz), 152.81 (³*J*_{CF} = 10.3 Hz), 150.13, 148.04, 144.22, 138.51, 132.28 (2C), 130.40, 130.23 (2C), 124.35 (2C), 118.72, 112.00, 107.32, 105.00, 98.93 (²*J*_{CF} = 25.0 Hz), 68.42, 54.74 (2C), 39.07, 32.95; ¹⁹F NMR (376 MHz, CDCl₃): *δ* -112.61; HRMS (ESI): m/z calcd. for C₂₄H₁₉FN₄O₂ [M+H]⁺: 415.1565, found: 415.1562.

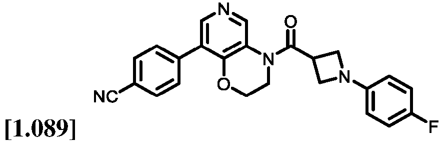

### 4-(4-(1-(4-Fluorophenyl)azetidine-3-carbonyl)-3,4-dihydro-2H-pyrido[4,3-b][1,4]oxazin-8-yl)b enzonitrile (O85)

Following the general method OG, synthesized with **O56** (70.0 mg, 0.21 mmol), 4-bromofluorobenzene (72.8 mg, 0.42 mmol), Cs₂CO₃ (203.3 mg, 0.62 mmol), Xantphos (5.8 mg, 0.01 mmol) and Pd₂(dba)₃ (9.2 mg, 0.01 mmol) to give **O85** (53.0 mg, 61.3% yield) as a white solid after chromatographic purification (PE/EA = 3/1), mp: 188.0-189.0 °C. *R*_{f} = 0.27 (PE/EA (V/V) = 1/2). ¹H-NMR (400 MHz, CDCl₃): *δ* 8.31 (s, 1H), 8.25 (s, 1H), 7.74 (d, *J =* 8.4 Hz, 2H), 7.64 (d, *J =* 8.3 Hz, 2H), 6.93 (t, *J* = 8.7 Hz, 2H), 6.45-6.37 (m, 2H), 4.43 (t, *J* = 4.8 Hz, 2H), 4.18-3.63 (m, 7H); ¹³C-NMR (101 MHz, CDCl₃): *δ* 170.44, 157.69 (¹*J*_{CF} = 237.1 Hz), 150.07, 147.88 (2C), 144.09, 138.63, 132.28 (2C), 130.23 (2C), 124.35 (2C), 118.72, 115.76 (²*J*_{CF} = 22.8 Hz, 2C), 112.76 (³*J*_{CF} = 7.4 Hz, 2C), 112.00, 68.40, 55.16 (2C), 39.11, 33.00; ¹⁹F NMR (376 MHz, CDCl₃): *δ* -126.89; HRMS (ESI): m/z calcd. for C₂₄H₁₉FN₄O₂ [M+H]⁺: 415.1565, found: 415.1573.

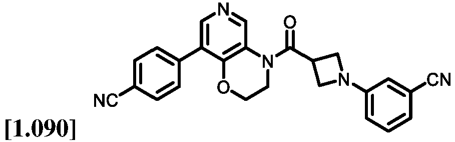

### 3-(3-(8-(4-Cyanophenyl)-3,4-dihydro-2H-pyrido[4,3-b][1,4]oxazine-4-carbonyl)azetidin-1-yl)b enzonitrile (O86)

Following the general method OG, synthesized with **O56** (100.0 mg, 0.30 mmol), 3-bromobenzonitrile (59.5 mg, 0.33 mmol), Cs₂CO₃ (293.2 mg, 0.90 mmol), Xantphos (11.6 mg, 0.02 mmol) and Pd₂(dba)₃ (18.3 mg, 0.02 mmol) to give **O86** (57.4 mg, 45.8% yield) as a white solid after chromatographic purification (PE/EA = 2/1), mp: 210.0-211.0 °C. *R*_{f} = 0.33 (PE/EA (V/V) = 1/2).¹H-NMR (400 MHz, CDCl₃): *δ* 8.33 (s, 1H), 8.21 (s, 1H), 7.75 (d, *J* = 8.4 Hz, 2H), 7.64 (d, *J* = 8.2 Hz, 2H), 7.29 (d, *J =* 7.6 Hz, 1H), 7.03 (d, *J =* 7.6 Hz, 1H), 6.70-6.62 (m, 2H), 4.45 (t, *J* = 4.8 Hz, 2H), 4.23-3.81 (m, 7H); ¹³C-NMR (101 MHz, CDCl₃): *δ* 170.25, 150.95, 150.37, 148.16, 144.16, 138.48, 132.28 (2C), 130.22 (2C), 129.86, 124.43 (2C), 121.52, 119.32, 118.69, 115.76, 114.30, 112.83, 112.04, 68.40, 54.59 (2C), 39.19, 32.72; HRMS (ESI): m/z calcd. for C₂₅H₁₉N₅O₂ [M+H]⁺: 422.1612, found: 422.1615.

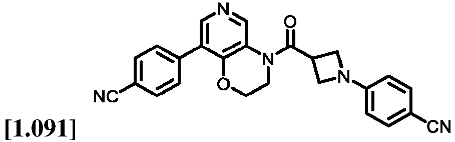

### 4-(3-(8-(4-Cyanophenyl)-3,4-dihydro-2H-pyrido[4,3-b][1,4]oxazine-4-carbonyl)azetidin-1-yl)b enzonitrile (O87)

Following the general method OG, synthesized with **O56** (50.0 mg, 0.15 mmol), 4-bromobenzonitrile (29.9 mg, 0.16 mmol), Cs₂CO₃ (146.6 mg, 0.45 mmol), Xantphos (4.6 mg, 0.01 mmol) and Pd₂(dba)₃ (7.3 mg, 0.01 mmol) to give **O87** (29.3 mg, 46.7% yield) as a white solid after chromatographic purification (PE/EA = 1/1), mp: 257.0-258.0 °C. *R*_{f} = 0.24 (PE/EA (V/V) = 1/2). ¹H-NMR (400 MHz, CDCl₃): *δ* 8.33 (s, 1H), 8.19 (s, 1H), 7.75 (d, *J =* 8.3 Hz, 2H), 7.64 (d, *J =* 8.3 Hz, 2H), 7.46 (d, *J =* 8.7 Hz, 2H), 6.40 (d, *J =* 8.7 Hz, 2H), 4.46 (t, *J =* 4.8 Hz, 2H), 4.33-4.11 (m, 5H), 4.10-3.81 (m, 2H); ¹³C-NMR (101 MHz, CDCl₃): *δ* 169.92, 152.79, 150.17, 148.39, 143.90, 138.46, 133.51 (2C), 132.31 (2C), 130.22 (2C), 124.45 (2C), 120.36, 118.68, 112.09, 110.93 (2C), 99.49, 68.74, 54.17 (2C), 39.24, 32.68; HRMS (ESI): m/z calcd. for C₂₅H₁₉N₅O₂ [M+H]⁺: 422.1612, found: 422.1611.

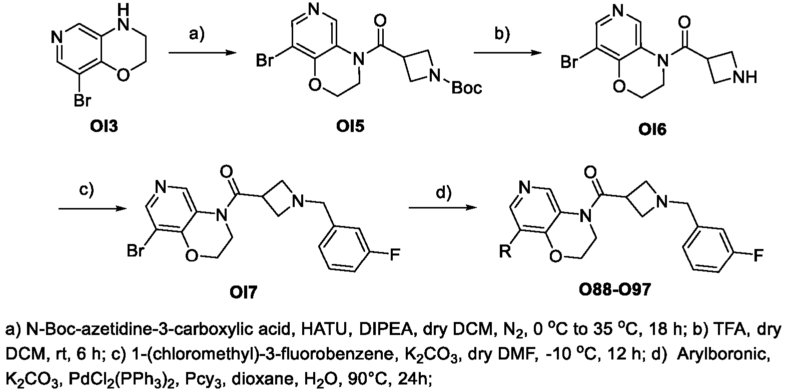

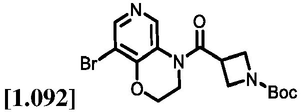

### tert-Butyl 3-(8-bromo-3,4-dihydro-2H-pyrido[4,3-b][1,4]oxazine-4-carbonyl)azetidine-1-carboxylate (OI5)

Following the general method OE, synthesized with **O56** (5.000 g, 23.25 mmol), 1-Boc-azetidine-3-carboxylic acid (7.018 g, 34.88 mmol), HATU (18.565 g, 48.83 mmol) and DIPEA (9.015 g, 69.75 mmol) to give an intermediate of **O25** (9.223 g, 99.9% yield) as a yellow solid with *R*_{f} = 0.56 (PE/EA (V/V) = 1/1) after chromatographic purification (PE/EA = 1/1). The resulting intermediate was not further characterized and was directly used for the next step.

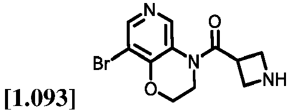

### Azetidin-3-yl(8-bromo-2,3-dihydro-4H-pyrido[4,3-b][1,4]oxazin-4-yl)methanone (OI6)

Stirred a solution of **OI5** (8.888 g, 22.39 mmol) in trifluoroacetic acid (25.529 g, 223.90 mmol) in an ice bath for 4 h. Neutralization with saturated Na₂CO₃ aqueous solution gave **OI6** (486.2 mg, 67.0% yield) as a yellow solid. *R*_{f} = 0.14 (CH₂Cl₂/MeOH (V/V) = 10/1). The resulting product was not further characterized and was directly used for the next step.

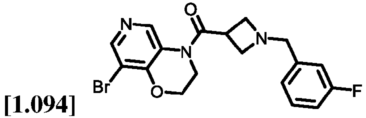

### (8-bromo-2,3-dihydro-4H-pyrido[4,3-b][1,4]oxazin-4-yl)(1-(3-fluorobenzyl)azetidin-3-yl)meth anone (OI7)

Following the general method OF, synthesized with **OI6** (4.700 g, 15.82 mmol), 3-fluorobenzyl chloride (3.432 g, 23.74 mmol), K₂CO₃ (4.373 g, 31.64 mmol) to give **OI7** (2.523 g, 39.4% yield) as a white solid with *R*_{f} = 0.46 (petroleum ether/acetone (V/V) = 1/1) after chromatographic purification (CH₂Cl₂/MeOH = 100/1). ¹H-NMR (400 MHz, CDCl₃): *δ* 8.49-7.73 (m, 2H), 7.29-7.22 (m, 1H), 7.06-6.88 (m, 3H), 4.45 (t, *J =* 4.8 Hz, 2H), 4.12-3.67 (m, 3H), 3.62 (s, 2H), 3.59-3.47 (m, 2H), 3.37 (t, *J =* 7.0 Hz, 2H).

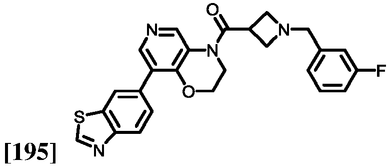

### (8-(Benzo[d]thiazol-6-yl)-2,3-dihydro-4H-pyrido[4,3-b][1,4]oxazin-4-yl)(1-(3-fluorobenzyl)azet idin-3-yl)methanone (O88)

Following the general method OC, synthesized with **OI7** (61.9 mg, 0.15 mmol), 1,3-benzothiazol-6-ylboronic acid (41.2 mg, 0.23 mmol), K₂CO₃ (82.9 mg, 0.60 mmol), PdCl₂(PPh₃)₂ (14.0 mg, 0.02 mmol) and Pcy₃ (8.4 mg, 0.03 mmol) to give **O88** (58.9 mg, 83.7% yield) as a white solid after chromatographic purification (CH₂Cl₂/MeOH = 40/1), mp: 83.0-84.0 °C. *R*_{f} = 0.18 (petroleum ether/acetone (V/V) = 1/1). ¹H-NMR (400 MHz, CDCl₃): *δ* 9.03 (s, 1H), 8.34 (s, 1H), 8.18 (d, *J =* 8.6 Hz, 2H), 8.09 (s, 1H), 7.64 (d, *J =* 6.7 Hz, 1H), 7.29-7.22 (m, 1H), 7.07-6.98 (m, 2H), 6.93 (td, *J* = 8.6, 3.2 Hz, 1H), 4.46-4.33 (m, 2H), 4.14-3.70 (m, 3H), 3.66-3.56 (m, 4H), 3.42 (t, *J =* 7.6 Hz, 2H); ¹³C-NMR (101 MHz, CDCl₃): *δ* 170.90, 164.23 (¹*J*_{CF} = 246.7 Hz), 154.81, 152.95, 149.97, 148.20, 143.61, 140.33, 134.14, 131.41, 130.00 (³*J*_{CF} = 8.8 Hz), 125.29, 124.05, 123.75, 123.44, 122.77 (2C), 115.39 (²*J*_{CF} = 21.4 Hz), 114.07, 68.22, 62.70 (2C), 57.12, 39.02, 33.66; ¹⁹F NMR (376 MHz, CDCl₃): *δ* -113.32; HRMS (ESI): m/z calcd. for C₂₅H₂₁FN₄O₂S [M+H]⁺: 461.1442, found: 461.1447.

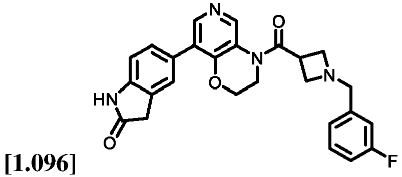

### 5-(4-(1-(3-Fluorobenzyl)azetidine-3-carbonyl)-3,4-dihydro-2H-pyrido[4,3-b][1,4]oxazin-8-yl)in dolin-2-one (O89)

Following the general method OC, synthesized with **OI7** (111.0 mg, 0.27 mmol), 5-(4,4,5,5-Tetramethyl-1,3,2-dioxaborolan-2-yl)indolin-2-one (106.5 mg, 0.41 mmol), K₂CO₃ (149.3 mg, 1.08 mmol), PdCl₂(PPh₃)₂ (21.1 mg, 0.03 mmol) and Pcy₃ (14.0 mg, 0.05 mmol) to give **O89** (66.1 mg, 52.7% yield) as a white solid after chromatographic purification (CH₂Cl₂/MeOH = 20/1), mp: 135.0-136.0 °C. *R*_{f} = 0.59 (CH₂Cl₂/MeOH (V/V) = 10/1). ¹H-NMR (400 MHz, CDCl₃): *δ* 9.25 (s, 1H), 8.25 (s, 1H), 8.12 (s, 1H), 7.40-7.31 (m, 2H), 7.29-7.22 (m, 1H), 7.07-6.97 (m, 2H), 6.97-6.90 (m, 2H), 4.41-4.33 (m, 2H), 4.10-3.74 (s, 3H), 3.64 (s, 2H), 3.62-3.52 (m, 4H), 3.46-3.38 (m, 2H); ¹³C-NMR (101 MHz, CDCl₃): *δ* 177.91, 170.81, 164.17 (¹*J*_{CF} = 246.7 Hz), 149.91, 147.90, 142.97, 140.88, 140.19, 131.09, 129.98 (³*J*_{CF} = 8.1 Hz), 129.23, 127.69, 125.77, 125.65, 124.03, 123.61, 115.36 (²*J*_{CF} = 21.6 Hz), 114.25, 109.78, 68.10, 62.63 (2C), 57.09, 39.25, 36.42, 33.67; ¹⁹F NMR (376 MHz, CDCl₃): *δ* -113.30; HRMS (ESI): m/z calcd. for C₂₆H₂₃FN₄O₃ [M+H]⁺: 459.1827, found: 459.1847.

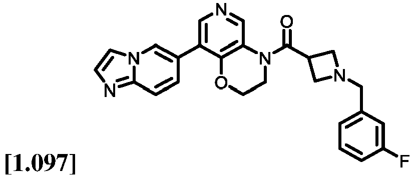

### (1-(3-Fluorobenzyl)azetidin-3-yl)(8-(imidazo[1,2-a]pyridin-6-yl)-2,3-dihydro-4H-pyrido[4,3-b] [1,4]oxazin-4-yl)methanone (O90)

Following the general method OC, synthesized with **OI7** (111.0 mg, 0.27 mmol), imidazo[1,2-a]pyridine-6-boronic acid (34.5 mg, 0.21 mmol), K₂CO₃ (77.4 mg, 0.56 mmol), PdCl₂(PPh₃)₂ (7.0 mg, 0.01 mmol) and Pcy₃ (8.4 mg, 0.03 mmol) to give **O90** (32.6 mg, 51.8% yield) as a yellow solid after chromatographic purification (CH₂Cl₂/MeOH = 20/1), mp: 89.0-90.0 °C. *R*_{f} = 0.36 (CH₂Cl₂/MeOH (V/V) = 10/1). ¹H-NMR (400 MHz, CDCl₃): *δ* 8.31 (d, *J =* 11.6 Hz, 2H), 8.17 (s, 1H), 7.69-7.64 (m, 2H), 7.63 (s, 1H), 7.33-7.28 (m, 1H), 7.28-7.23 (m, 1H), 7.07-6.99 (m, 2H), 6.94 (td, *J =* 7.7, 2.8 Hz, 1H), 4.47-4.35 (m, 2H), 4.14-3.72 (m, 3H), 3.67-3.56 (m, 4H), 3.43 (t, *J =* 7.5 Hz, 2H); ¹³C-NMR (101 MHz, CDCl₃): *δ* 170.56, 164.21 (¹*J*_{CF} = 246.6 Hz), 150.59, 147.46, 144.68, 143.91, 140.32, 134.31, 129.98 (³*J*_{CF} = 8.2 Hz), 126.38, 125.52, 124.03 (2C), 122.04, 119.18, 117.40, 115.36 (²*J*_{CF} = 21.6 Hz), 114.26, 112.91, 68.80, 62.70 (2C), 57.13, 38.99, 33.79; ¹⁹F NMR (376 MHz, CDCl₃): *δ* -113.34; HRMS (ESI): m/z calcd. for C₂₅H₂₂FN₅O₂ [M+H]⁺: 444.1830, found: 444.1834.

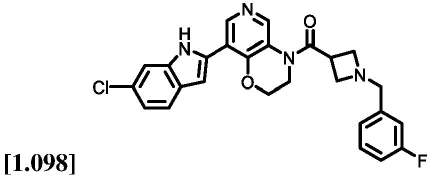

### (8-(6-Chloro-1H-indol-2-yl)-2,3-dihydro-4H-pyrido[4,3-b][1,4]oxazin-4-yl)(1-(3-fluorobenzyl)a zetidin-3-yl)methanone (091)

Following the general method OC, synthesized with **OI7** (200.0 mg, 0.49 mmol), (1-(*tert*-butoxycarbonyl)-6-chloro-1H-indol-2-yl)boronic acid (219.0 mg, 0.74 mmol), K₂CO₃ (273.1 mg, 1.98 mmol), PdCl₂(PPh₃)₂ (34.4 mg, 0.05 mmol) and Pcy₃ (27.8 mg, 0.01 mmol) to give **091** (76.0 mg, 32.3% yield) as a white solid after chromatographic purification (CH₂Cl₂/MeOH = 30/1), mp: 162.0-163.0 °C. *R*_{f} =0.16 (CH₂Cl₂/MeOH (V/V) = 20/1). ¹H-NMR (400 MHz, CDCl₃): *δ* 9.30 (s, 1H), 8.76 (s, 1H), 8.08 (s, 1H), 7.54 (d, *J* = 8.4 Hz, 1H), 7.40 (s, 1H), 7.29-7.26 (m, 1H), 7.09 (dd, *J =* 8.5, 1.9 Hz, 1H), 7.07-6.97 (m, 2H), 6.97-6.90 (m, 2H), 4.67-4.50 (m, 2H), 4.20-3.72 (m, 3H), 3.65 (s, 2H), 3.62-3.51 (m, 2H), 3.43 (d, *J* = 7.0 Hz, 2H); ¹³C-NMR (101 MHz, DMSO-*d*₆): *δ* 170.62, 163.43 (¹*J*_{CF} = 243.7 Hz), 158.43, 148.74, 143.62, 141.27, 136.98 (2C), 132.03, 130.17 (³*J*_{CF} = 8.4 Hz), 126.73, 126.38, 124.21, 123.81, 121.48, 119.73, 116.24, 114.83 (²*J*_{CF} = 21.4 Hz), 113.76, 110.93, 102.73, 67.05, 61.37 (2C), 55.95, 33.50; ¹⁹F NMR (376 MHz, DMSO-*d*₆): *δ* -113.58; HRMS (ESI): m/z calcd. for C₂₆H₂₂ClFN₄O₂ [M+H]⁺: 477.1488, found: 477.1473.

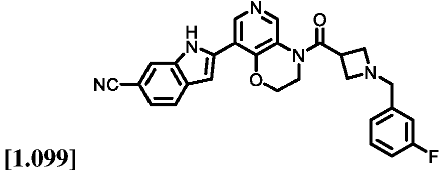

### 2-(4-(1-(3-Fluorobenzyl)azetidine-3-carbonyl)-3,4-dihydro-2H-pyrido[4,3-b][1,4]oxazin-8-yl)-1 H-indole-6-carbonitrile (O92)

Following the general method OC, synthesized with **OI7** (200.0 mg, 0.49 mmol), 1-Boc-6-cyanoindole-2-boronic acid (212.0 mg, 0.74 mmol), K₂CO₃ (273.1 mg, 1.98 mmol), PdCl₂(PPh₃)₂ (34.4 mg, 0.05 mmol) and Pcy₃ (27.8 mg, 0.01 mmol) to give **O92** (49.2 mg, 21.3% yield) as a white solid after chromatographic purification (CH₂Cl₂/MeOH = 40/1), mp: 128.0-129.0 °C. *R*_{f} =0.07 (petroleum ether/acetone (V/V) = 1/1). ¹H-NMR (400 MHz, CDCl₃): *δ* 9.82 (s, 1H), 8.78 (s, 1H), 8.14 (s, 1H), 7.74 (s, 1H), 7.68 (d, *J =* 8.2 Hz, 1H), 7.33 (dd, *J =* 8.2, 1.5 Hz, 1H), 7.26-7.21 (m, 1H), 7.07-6.96 (m, 3H), 6.93 (td, *J =* 8.5, 3.1 Hz, 1H), 4.68-4.54 (m, 2H), 4.21-3.71 (m, 3H), 3.62 (s, 2H), 3.61-3.50 (m, 2H), 3.41 (t, *J =* 7.5 Hz, 2H); ¹³C-NMR (101 MHz, CDCl₃): *δ* 170.69, 158.77, 164.21 (¹*J*_{CF} = 246.7 Hz), 149.07, 146.41, 143.36, 140.11, 135.33 (2C), 131.17, 130.04 (³*J*_{CF} = 8.1 Hz), 124.07, 123.05, 121.48, 120.86, 116.13, 116.05, 115.38 (²*J*_{CF} = 21.4 Hz), 114.32, 104.55, 102.30, 68.79, 62.67 (2C), 56.96, 39.28, 33.90; ¹⁹F NMR (376 MHz, CDCl₃): *δ* -113.28; HRMS (ESI): m/z calcd. for C₂₇H₂₂FN₅O₂ [M+H]⁺: 468.1830, found: 468.1833.

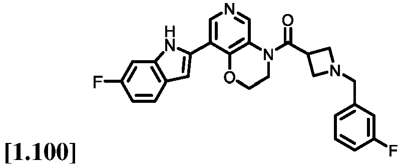

### (8-(6-Fluoro-1H-indol-2-yl)-2,3-dihydro-4H-pyrido[4,3-b][1,4]oxazin-4-yl)(1-(3-fluorobenzyl)a zetidin-3-yl)methanone (O93)

Following the general method OC, synthesized with **OI7** (200.0 mg, 0.49 mmol), 1-Boc-6-fluoro-1*H*-indole-2-boronic acid (206.8 mg, 0.74 mmol), Cs₂CO₃ (643.8 mg, 1.98 mmol), Pd(OAc)₂ (5.6 mg, 0.03 mmol), dppf (27.2 mg, 0.05 mmol) and CuCl (48.9 mg, 0.49 mmol) to give **O93** (18.7 mg, 19.7% yield) as a white solid after chromatographic purification (CH₂Cl₂/MeOH = *30*/*1), R*_{f} = 0.15 (petroleum ether/acetone (V/V) = 1/1). ¹H-NMR (400 MHz, CDCl₃): *δ* 9.26 (s, 1H), 8.76 (s, 1H), 8.07 (s, 1H), 7.56 (dd, *J* = 8.7, 5.4 Hz, 1H), 7.30-7.26 (m, 1H), 7.09 (dd, *J* = 9.6, 2.6 Hz, 1H), 7.07-6.98 (m, 2H), 6.96 (s, 1H), 6.95-6.87 (m, 2H), 4.66-4.52 (m, 2H), 4.20-3.75 (m, 3H), 3.65 (s, 2H), 3.63-3.50 (m, 2H), 3.46-3.36 (m, 1H).

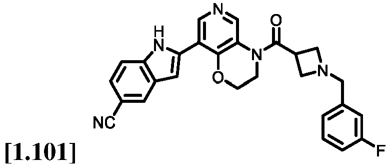

### 2-(4-(1-(3-Fluorobenzyl)azetidine-3-carbonyl)-3,4-dihydro-2H-pyrido[4,3-b][1,4]oxazin-8-yl)-1 H-indole-5-carbonitrile (O94)

Following the general method OC, synthesized with **OI7** (237.0 mg, 0.59 mmol), 1-Boc-5-cyano-1*H*-indole-2-boronic acid (236.8 mg, 0.88 mmol), Cs₂CO₃ (762.4 mg, 2.34 mmol), Pd(OAc)₂ (6.5 mg, 0.03 mmol), dppf (32.7 mg, 0.06 mmol) and CuCl (57.9 mg, 0.59 mmol) to give **O94** (66.3 mg, 24.3% yield) as a white solid after chromatographic purification (CH₂Cl₂/MeOH = *40*/*1), R*_{f} = 0.22 (petroleum ether/acetone (V/V) = 1/1). ¹H-NMR (400 MHz, CDCl₃): *δ* 9.70 (s, 1H), 8.78 (s, 1H), 8.14 (s, 1H), 7.99 (s, 1H), 7.46 (d, *J =* 8.4 Hz, 1H), 7.42 (dd, *J =* 8.5, 1.5 Hz, 1H), 7.24 (d, *J* = 7.9 Hz, 1H), 7.07-6.97 (m, 3H), 6.93 (td, *J =* 8.4, 2.9 Hz, 1H), 4.66-4.52 (m, 2H), 4.21-3.72 (m, 3H), 3.64 (s, 2H), 3.62-3.51 (m, 2H), 3.41 (t, *J =* 7.5 Hz, 2H).

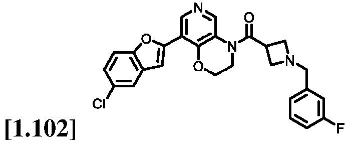

### (8-(5-Chlorobenzofuran-2-yl)-2,3-dihydro-4H-pyrido[4,3-b][1,4]oxazin-4-yl)(1-(3-fluorobenzyl )azetidin-3-yl)methanone (O95)

Following the general method OC, synthesized with **OI7** (80.7 mg, 0.20 mmol), 5-chlorobenzofuran-2-boronic acid (58.7 mg, 0.30 mmol), K₂CO₃ (110.0 mg, 0.80 mmol), PdCl₂(PPh₃)₂ (14.0 mg, 0.02 mmol) and Pcy₃ (11.2 mg, 0.04 mmol) to give **O95** (18.7 mg, 19.7% yield) as a white solid after chromatographic purification (CH₂Cl₂/MeOH = 30/1), *R*_{f} = 0.31 (CH₂Cl₂/MeOH (V/V) = 30/1). ¹H-NMR (400 MHz, CDCl₃): *δ* 8.96 (s, 1H), 8.13 (s, 1H), 7.55 (d, *J* = 2.2 Hz, 1H), 7.45 (d, *J =* 8.8 Hz, 1H), 7.32-7.26 (m, 3H), 7.04 (dd, *J =* 17.7, 8.6 Hz, 2H), 6.94 (t, *J =* 8.4 Hz, 1H), 4.65-4.50 (m, 2H), 4.22-3.75 (m, 3H), 3.66 (s, 2H), 3.64-3.53 (s, 2H), 3.48-3.37 (m, 2H).

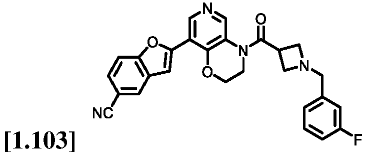

### 2-(4-(1-(3-Fluorobenzyl)azetidine-3-carbonyl)-3,4-dihydro-2H-pyrido[4,3-b][1,4]oxazin-8-yl)b enzofuran-5-carbonitrile (O96)

Following the general method OC, synthesized with **OI7** (110.0 mg, 0.27 mmol), (5-cyanobenzofuran-2-yl)boronic acid (76.3 mg, 0.41 mmol), K₂CO₃ (150.6 mg, 1.09 mmol), PdCl₂(PPh₃)₂ (21.1 mg, 0.03 mmol) and Pcy₃ (14.0 mg, 0.05 mmol) to give **O96** (63.5 mg, 49.9% yield) as a white solid after chromatographic purification (CH₂Cl₂/MeOH = 30/1), mp: 157.0-158.0 °C. *R*_{f} = 0.40(petroleum ether/acetone (V/V) = 1/1). ¹H-NMR (400 MHz, CDCl₃): *δ* 8.98 (s, 1H), 8.17 (s, 1H), 7.92 (s, 1H), 7.66-7.56 (m, 2H), 7.36 (s, 1H), 7.30-7.23 (m, 1H), 7.03 (dd, *J* = 17.7, 8.9 Hz, 2H), 6.97-6.90 (m, 1H), 4.70-4.52 (m, 2H), 4.17-3.73 (m, 3H), 3.70-3.53 (m, 4H), 3.50-3.37 (m, 2H); ¹³C-NMR (101 MHz, CDCl₃): *δ* 170.73, 164.22 (¹*J*_{CF} = 246.7 Hz), 155.71, 151.34, 149.59, 145.34, 144.04, 140.31, 129.99 (³*J*_{CF} = 8.1 Hz), 129.66, 128.62, 126.17, 124.03, 123.58, 119.41, 115.36 (²*J*_{CF} = 21.4 Hz), 114.51, 114.27, 112.39, 107.62, 107.11, 68.67, 62.70 (2C), 57.06, 38.81, 33.59; ¹⁹F NMR (376 MHz, CDCl₃): *δ* -113.34; HRMS (ESI): m/z calcd. for C₂₇H₂₁FN₄O₃ [M+H]⁺: 469.1598, found: 469.1649.

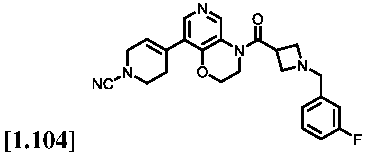

### 4-(4-(1-(3-Fluorobenzyl)azetidine-3-carbonyl)-3,4-dihydro-2H-pyrido[4,3-b][1,4]oxazin-8-yl)-3 ,6-dihydropyridine-1(2H)-carbonitrile (O97)

According to general method OC, **OI7** (200.0 mg, 0.49 mmol), 1-cyano-1,2,3,6-tetrahydroquinoline-4-boronic acid (111 mg, 0.75 mmol), K₂CO₃ (273.1 mg, 1.98 mmol), PdCl₂(PPh₃)₂ (34.4 mg, 0.05 mmol) and Pcy₃ (27.8 mg, 0.01 mmol) were reacted in a mixture of 1,4-dioxane (3 mL) and water (1 mL) to produce **O96** (110 mg, 52% yield) as a white solid after chromatographic purification. ¹H-NMR (400 MHz, DMSO-*d*₆): δ 8.32-7.85 (m, 2H), 7.31-7.22 (m, 1H), 7.13-6.92 (m, 3H), 6.25 (s, 1H), 4.18 (t, *J =* 8.4 Hz, 2H), 3.83 (d, *J =* 4.8 Hz, 3H), 3.31-3.26 (m, 2H), 3.27-3.21 (m, 4H), 2.15-2.11 (m, 2H); HRMS (ESI): m/z calcd. for C₂₄H₂₄FN₅O₂ [M+H]⁺: 433.1914, found: 433.1917.

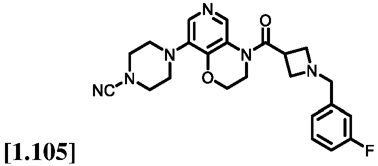

### 4-(4-(1-(3-Fluorobenzyl)azetidine-3-carbonyl)-3,4-dihydro-2H-pyrido[4,3-b][1,4]oxazin-8-yl)pi perazine-1-carbonitrile (O98)

**OI7** (125 mg, 0.30 mmol), Cs₂CO₃ (343.3 mg, 1.05 mmol), Xantphos (52.1 mg, 0.09 mmol), Pd(OAc)₂ (7.2 mg, 0.03 mmol) and 4-cyanopiperazine (33.3 mg, 0.30 mmol) were suspended in 1,4-dioxane (9 mL). The reaction mixture was stirred at 100°C under N₂ for 18 hours. After the reaction was complete as monitored by TLC, it was cooled to room temperature, quenched with water (5 mL), and extracted with ethyl acetate (5×5 mL). The combined organic layers were washed with brine (5 mL) and dried over anhydrous sodium sulfate. It was then concentrated under reduced pressure to give the crude product, which was subsequently purified by column chromatography with a gradient elution of CH₂Cl₂: MeOH (V/V) = 100:1 to 10:1, yielding the compound **O98** (46 mg, 35% yield). ¹H-NMR (400 MHz, CDCl₃): *δ* 8.16 (s, 1H), 7.96 (s, 1H), 7.33-7.25 (m, 1H), 7.09-6.95 (m, 3H), 4.21 (t, *J =* 4.8 Hz, 2H), 3.82-3.67 (m, 6H), 3.53-3.41 (m, 2H), 3.33-3.28 (m, 4H), 3.23-3.17 (m, 5H). HRMS (ESI): m/z calcd. for C₂₃H₂₆FN₆O₂ [M+H]⁺: 437.2096, found: 437.2110.

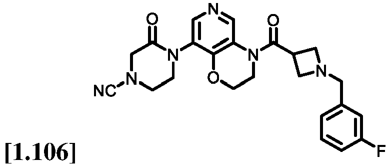

### 4-(4-(1-(3-Fluorobenzyl)azetidine-3-carbonyl)-3,4-dihydro-2H-pyrido[4,3-b][1,4]oxazin-8-yl)-3 -oxopiperazine-1-carbonitrile (O99)

**OI7** (125 mg, 0.30 mmol), Cs₂CO₃ (343.3 mg, 1.05 mmol), Xantphos (52.1 mg, 0.09 mmol), Pd(OAc)₂ (7.2 mg, 0.03 mmol) and 4-cyano-3-oxo-piperidine (37.5 mg, 0.30 mmol) were suspended in 1,4-dioxane (9 mL). The reaction mixture was stirred at 100°C under N₂ for 18 hours. After the reaction was complete as monitored by TLC, the reaction mixture was cooled to room temperature, quenched with water (5 mL), and extracted with ethyl acetate (5×5 mL). The combined organic layers were washed with brine (5 mL) and dried over anhydrous sodium sulfate. It was then concentrated under reduced pressure to give the crude product, which was subsequently purified by column chromatography with a gradient elution of CH₂Cl₂: MeOH (V/V) = 100:1 to 10:1, yielding the compound **O98** (16 mg, 12% yield). ¹H-NMR (400 MHz, CDCl₃): *δ* 8.15 (s, 1H), 8.02 (s, 1H), 7.31-7.25 (m, 1H), 7.09-6.99 (m, 3H), 4.15 (t, *J* = 4.8 Hz, 2H), 3.79-3.65 (m, 8H), 3.53-3.41 (m, 2H), 3.36 (t, *J* = 4.0 Hz, 2H), 3.17-3.13 (m, 3H). HRMS (ESI): m/z calcd. for C₂₃H₂₄FN₆O₃ [M+H]⁺: 451.1888, found: 451.1879.

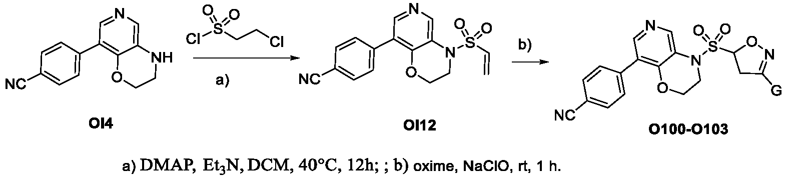

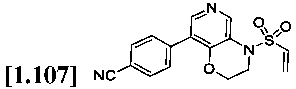

### 4-(4-(Vinylsulfonyl)-3,4-dihydro-2H-pyrido[4,3-b][1,4]thiazin-8-yl)benzonitrile (OI12)

Following the general method OI, synthesized with **OI4** (100.0 mg, 0.42 mmol), 2-chloroethanesulfonyl chloride (82.5 mg, 0.51 mmol), DMAP (15.5 mg, 0.13 mmol) and triethylamine (64.1 mg, 0.51 mmol) to give **OI12** (20 mg, 15% yield) as a white solid after chromatographic purification (PE/EA = 1/1). ¹H-NMR (400 MHz, DMSO-_{*d*6}): δ 8.65 (s, 1H), 8.29 (s, 1H), 7.93 (d, *J* = 8.3 Hz, 2H), 7.77 (d, *J* = 8.3 Hz, 2H), 7.07 (dd, *J* = 16.3, 9.9 Hz, 1H), 6.32-6.24 (m, 2H), 4.37 (s, 2H), 3.88 (s, 2H).

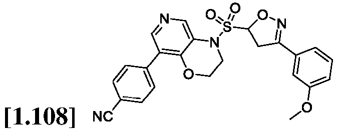

### 4-(4-((3-(3-Methoxyphenyl)-4,5-dihydroisoxazol-5-yl)sulfonyl)-3,4-dihydro-2H-pyrido[4,3-b] [1,4]oxazin-8-yl)benzonitrile (O100)

Following the general method OH, synthesized with **OI12** (30.0 mg, 0.09 mmol), 3-methoxybenzaldehyde oxime (6.9 mg, 0.05 mmol) and NaClO (6.9 mg, 0.09 mmol) to give **O100** (25.0 mg, 59.5% yield) as a white solid after chromatographic purification (PE/EA = 1/1). ¹H-NMR(400 MHz, Chloroform-d): δ 8.94 (s, 1H), 8.20 (s, 1H), 7.72 (s, 2H), 7.57 (s, 2H), 7.33 (t, *J* = 8.0 Hz, 1H), 7.20 (s, 1H), 7.13 (s, 1H), 7.03 (s, 1H), 5.74 (s, 1H), 4.63 (ddd, *J =* 11.5, 7.3, 2.9 Hz, 1H), 4.54 (ddd, *J* = 11.5, 5.2, 3.0 Hz, 1H), 4.19-4.13 (m, 1H), 4.03 (dd, *J* = 18.4, 4.3 Hz, 1H), 3.95-3.84 (m, 2H), 3.81 (s, 3H).

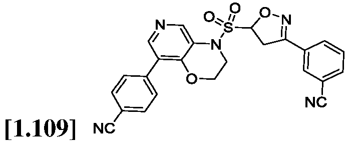

### 3-(5-((8-(4-Cyanophenyl)-2,3-dihydro-4H-pyrido[4,3-b][1,4]oxazin-4-yl)sulfonyl)-4,5-dihydroi soxazol-3-yl)benzonitrile (O101)

Following the general method OH, synthesized with **OI12** (30.0 mg, 0.09 mmol), 3-cyanobenzaldehyde oxime (6.7 mg, 0.05 mmol) and NaClO (6.9 mg, 0.09 mmol) to give **O101** (15.0 mg, 35.7% yield) as a white solid after chromatographic purification (PE/EA = 1/1). ¹H-NMR (400 MHz, Chloroform-d): δ 8.92 (s, 1H), 8.25 (s, 1H), 7.96 (s, 1H), 7.87 (dt, *J =* 7.9, 1.4 Hz, 1H), 7.73 (s, 3H), 7.61 (s, 3H), 5.82 (s, 1H), 4.64 (s, 2H), 4.21 (ddd, *J =* 14.5, 4.9, 2.9 Hz, 1H), 4.02 (d, *J* = 4.3 Hz, 1H), 3.91 (s, 2H).

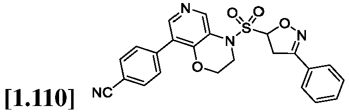

### 4-(4-((3-Phenyl-4,5-dihydroisoxazol-5-yl)sulfonyl)-3,4-dihydro-2H-pyrido[4,3-b][1,4]oxazin-8-yl)benzonitrile (O102 )

Following the general method OH, synthesized with **OI12** (50.0 mg, 0.15 mmol), benzaldehyde oxime (9.3 mg, 0.08 mmol) and NaClO (11.3 mg, 0.15 mmol) to give **0102** (20.0 mg, 30.2% yield) as a white solid after chromatographic purification (PE/EA = 1/1). ¹H-NMR (400 MHz, DMSO-d6): δ 8.70 (s, 1H), 8.23 (s, 1H), 7.91 (s, 2H), 7.75 (s, 2H), 7.48 (s, 5H), 6.44 (s, 1H), 4.47 (s, 2H), 4.15-4.05 (m, 2H), 3.95 (dt, *J =* 15.6, 4.5 Hz, 2H).

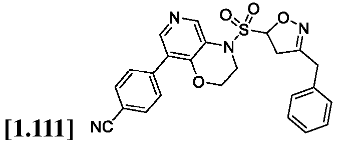

### 4-(4-((3-benzyl-4,5-dihydroisoxazol-5-yl)sulfonyl)-3,4-dihydro-2H-pyrido[4,3-b][1,4]oxazin-8-yl)benzonitrile (0103)

Following the general method OH, synthesized with **OI12** (30.0 mg, 0.09 mmol), benzeneacetaldehyde oxime (24.9 mg, 0.18 mmol) and NaClO (13.7 mg, 0.18 mmol) to give **0103** (20.0 mg, 50.0% yield) as a white solid after chromatographic purification (PE/EA = 1/1). ¹H-NMR (400 MHz, Chloroform-d): δ 8.84 (s, 1H), 8.27 (d, *J =* 6.1 Hz, 1H), 7.75-7.72 (m, 2H), 7.64-7.61 (m, 2H), 7.34-7.30 (m, 3H), 7.24-7.20 (m, 2H), 5.53 (dd, *J =* 10.5, 4.3 Hz, 1H), 4.63-4.58 (m, 1H), 4.46-4.40 (m, 1H), 4.20-4.12 (m, 1H), 3.82-3.76 (m, 1H), 3.73 (d, *J =* 2.0 Hz, 2H), 3.53-3.47 (m, 1H), 3.36 (dd, *J =* 18.8, 10.5 Hz, 1H).

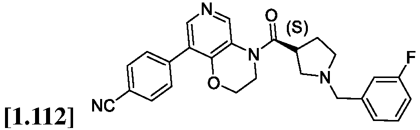

### (S)-4-(4-(1-(3-Fluorobenzyl)pyrrolidine-3-carbonyl)-3,4-dihydro-2H-pyrido[4,3-b][1,4]oxazin-8-yl)benzonitrile (0104)

Following the general method OC, synthesized with (*S*)-(8-Bromo-2,3-dihydro-4*H*-pyrido-[4,3-b][1,4]oxazine-4-yl)(1-(3-fluorobenzyl)pyrrolidin-3-yl)methanone (90.0 mg, 0.21 mmol), 4-cyanophenylboronic acid (47.2 mg, 0.32 mmol), Cs₂CO₃ (278.9 mg, 0.86 mmol), Pd(OAc)₂ (2.4 mg, 0.011 mmol), dppf (11.86 mg, 0.02 mmol) and CuCl (21.2 mg, 0.214 mmol), to give **0104** (56 mg, 59.6% yield) as a light yellow solid after chromatographic purification (ethyl acetate). ¹H-NMR(400 MHz, DMSO-d6): δ 8.21 (s, 1H), 7.91 (d, *J =* 8.2 Hz, 2H), 7.75 (d, *J =* 8.1 Hz, 2H), 7.35 (td, *J =* 7.9, 6.0 Hz, 1H), 7.15 (t, *J =* 8.8 Hz, 2H), 7.06 (td, *J =* 8.6, 2.7 Hz, 1H), 4.38 (t, *J =* 4.6 Hz, 2H), 3.97 (dd, *J* = 6.2, 3.6 Hz, 2H), 3.66-3.60 (m, 2H), 3.55 (dd, *J =* 14.6, 6.5 Hz, 1H), 2.82 (t, *J =* 8.7 Hz, 1H), 2.70 (t, *J =* 8.0 Hz, 1H), 2.66-2.58 (m, 1H), 2.47 (d, *J =* 7.9 Hz, 1H), 2.10-1.96 (m, 2H); ¹³C-NMR (101 MHz, DMSO-d6): δ 172.82, 161.04 (¹*J*_{CF} = 241.5 Hz), 149.47, 145.54, 142.28, 142.21, 139.02, 132.21, 130.28, 130.04 (³*J*_{CF} = 8.3 Hz), 124.42, 124.08, 123.38, 118.80, 115.05, 114.83, 113.50 (²*J*_{CF} = 20.8 Hz), 110.42, 59.79, 58.48, 53.48, 20.78; ¹⁹F NMR (377 MHz, DMSO-d6): δ -113.78; HRMS (ESI): m/z calcd. for C₂₆H₂₃FN₄O₂ [M+H]⁺: 443.1878, found: 443.1868.

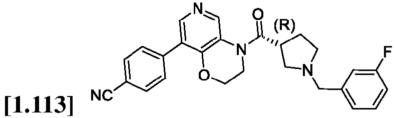

### (R)-4-(4-(1-(3-Fluorobenzyl)pyrrolidine-3-carbonyl)-3,4-dihydro-2H-pyrido[4,3-b][1,4]oxazin-8-yl)benzonitrile (0105)

Following the general method OC, synthesized with (*R*)-(8-bromo-2,3-dihydro-4*H*-pyrido-[4,3-b][1,4]oxazine-4-yl)(1-(3-fluorobenzyl)pyrrolidin-3-yl)methanone (90.0 mg, 0.21 mmol), 4-cyanophenylboronic acid (47.2 mg, 0.32 mmol), Cs₂CO₃ (278.9 mg, 0.86 mmol), Pd(OAc)₂ (2.4 mg, 0.011 mmol), dppf (11.86 mg, 0.02 mmol) and CuCl (21.2 mg, 0.214 mmol), to give **0105** (57 mg, 59.7% yield) as a light yellow solid after chromatographic purification (ethyl acetate). ¹H-NMR(400 MHz, DMSO-d6): δ 8.95 (s, 1H), 8.21 (s, 1H), 7.96-7.88 (m, 2H), 7.79-7.72 (m, 2H), 7.36 (td, *J =* 8.0, 6.1 Hz, 1H), 7.19-7.11 (m, 2H), 7.06 (ddd, *J =* 10.3, 8.0, 2.7 Hz, 1H), 4.38 (t, *J =* 4.6 Hz, 2H), 3.97 (p, *J =* 4.5, 3.8 Hz, 2H), 3.68-3.60 (m, 2H), 3.56 (dd, *J =* 14.5, 6.4 Hz, 1H), 2.82 (t, *J* = 8.7 Hz, 1H), 2.71 (dd, *J =* 14.1, 6.1 Hz, 1H), 2.64 (dt, *J =* 8.8, 6.4 Hz, 1H), 2.11-1.97 (m, 2H); ¹³C-NMR (101 MHz, DMSO-d6): δ 173.26, 161.49 (¹*J*_{CF} = 241.6 Hz), 149.91, 145.99, 142.72, 142.65, 139.47, 132.66, 130.72, 130.48 (³*J*_{CF} = 8.2 Hz), 124.86, 124.84, 124.53, 123.82, 119.24, 115.49, 115.28, 113.95 (²*J*_{CF} = 20.8 Hz), 110.87, 58.92, 56.75, 53.93, 21.53; ¹⁹F NMR (377 MHz, DMSO-*d*6): δ -113.78; HRMS (ESI): m/z calcd. for C₂₆H₂₃FN₄O₂ [M+H]⁺: 443.1878, found: 443.1882.

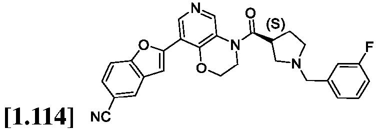

### 2-(4-((S)-1-(3-Fluorobenzyl)pyrrolidine-3-carbonyl)-3,4-dihydro-2H-pyrido[4,3-b][1,4]oxazin-8-yl)-2,3-dihydrobenzofuran-5-carbonitrile (0106)

Following the general method OC, synthesized with (*S*)-(8-Bromo-2,3-dihydro-4*H*-pyrido-[4,3-b][1,4]oxazine-4-yl)(1-(3-fluorobenzyl)pyrrolidin-3-yl)methanone (120.0 mg, 0.284 mmol), 5-cyano-1-benzofuran-2-boronic acid (80.0 mg, 0.426 mmol), Cs₂CO₃ (368.8 mg, 1.14 mmol), Pd(OAc)₂ (3.2 mg, 0.014 mmol), dppf (16.0 mg, 0.028 mmol) and CuCl (28.0 mg, 0.284 mmol) to give **0106** (50 mg, 36.8% yield) as a light yellow solid after chromatographic purification (PE/EA = 1/2). ¹H-NMR (400 MHz, DMSO-d6): δ 8.78 (s, 1H), 8.24 (d, *J =* 1.6 Hz, 1H), 7.86 (d, *J =* 8.5 Hz, 1H), 7.78 (dd, *J* = 8.6, 1.7 Hz, 1H), 7.52 (s, 1H), 7.35 (td, *J =* 8.0, 6.0 Hz, 1H), 7.20-7.11 (m, 2H), 7.06 (td, *J =* 8.6, 8.2, 2.7 Hz, 1H), 4.61 (t, *J =* 4.5 Hz, 2H), 4.03 (dt, *J =* 7.2, 3.3 Hz, 2H), 3.62 (d, *J* = 4.7 Hz, 2H), 3.60-3.52 (m, 1H), 2.83 (t, *J =* 8.7 Hz, 1H), 2.75-2.68 (m, 1H), 2.64 (dt, *J =* 8.6, 6.5 Hz, 1H), 2.02 (d, *J* = 24.9 Hz, 2H); ¹³C-NMR (101 MHz, DMSO-d6): δ 172.82, 161.02 (¹*J*_{CF} = 241.6 Hz), 155.19, 151.22, 142.27, 142.20, 130.08 (³*J*_{CF} = 8.3 Hz), 129.35, 128.76, 126.54, 124.38, 124.35, 119.26, 115.01, 114.80, 113.46 (²*J*_{CF} = 20.8 Hz), 112.60, 107.25, 106.19, 58.45, 53.44, 20.75; ¹⁹F NMR (377 MHz, DMSO-d6): δ -113.79; HRMS (ESI): m/z calcd. for C₂₈H₂₅FN₄O₃ [M+H]⁺: 483.1827, found: 483.1821.

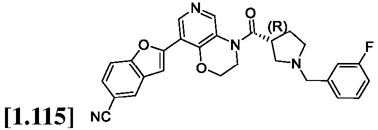

### 2-(4-((R)-1-(3-Fluorobenzyl)pyrrolidine-3-carbonyl)-3,4-dihydro-2H-pyrido[4,3-b][1,4]oxazin-8-yl)-2,3-dihydrobenzofuran-5-carbonitrile (0107)

Following the general method OC, synthesized with (*R*)-(8-bromo-2,3-dihydro-4*H*-pyrido-[4,3-b][1,4]oxazine-4-yl)(1-(3-fluorobenzyl)pyrrolidin-3-yl)-methanone (120.0 mg, 0.284 mmol) and 5-cyano-1-benzofuran-2-boronic acid (80.0 mg, 0.426 mmol), Cs₂CO₃ (368.8 mg, 1.14 mmol), Pd(OAc)₂ (3.2 mg, 0.014 mmol), dppf (16.0 mg, 0.028 mmol) and CuCl (28.0 mg, 0.284 mmol), to give **O107** (52 mg, 38.0 % yield) as a light yellow solid after chromatographic purification (PE/EA = 1/2). ¹H-NMR(400 MHz, DMSO-d6): δ 8.96 (s, 1H), 8.76 (s, 1H), 8.23 (d, *J =* 1.7 Hz, 1H), 7.85 (d, *J* = 8.6 Hz, 1H), 7.77 (dd, *J* = 8.5, 1.7 Hz, 1H), 7.50 (s, 1H), 7.35 (td, *J* = 7.9, 6.0 Hz, 1H), 7.19-7.11 (m, 2H), 7.06 (ddd, *J =* 10.6, 8.2, 2.7 Hz, 1H), 4.60 (t, *J =* 4.6 Hz, 2H), 4.03 (q, *J =* 3.3, 2.1 Hz, 2H), 3.62 (d, *J =* 4.9 Hz, 2H), 3.59-3.51 (m, 1H), 2.83 (t, *J =* 8.7 Hz, 1H), 2.71 (t, *J =* 8.0 Hz, 1H), 2.63 (dt, *J* = 8.9, 6.4 Hz, 1H), 2.03 (dd, J = 13.3, 6.7 Hz, 2H); ¹³C-NMR (101 MHz, DMSO-d6): δ 172.81, 161.03 (¹*J*_{CF} = 241.6 Hz), 155.18, 151.21, 149.30, 145.55, 142.25, 142.18, 130.01 (³*J*_{CF} = 8.1 Hz), 129.35, 128.75, 126.53, 124.37, 119.27, 115.03, 114.82, 113.48 (²*J*_{CF} *=* 20.7 Hz), 112.59, 107.24, 106.18, 67.80, 58.46, 56.29, 53.45, 29.03; ¹⁹F NMR (377 MHz, DMSO-d6): δ -113.78; HRMS (ESI): m/z calcd. for C₂₈H₂₅FN₄O₃ [M+H]⁺: 483.1827, found: 483.1828.

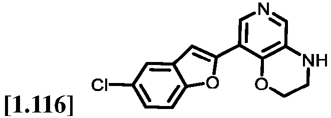

### 8-(5-Chlorobenzofuran-2-yl)-3,4-dihydro-2H-pyrido[4,3-b][1,4]oxazine (0108)

Following the general method OC, 8-bromo-3,4-dihydro-2*H*-pyrido[4,3-b][1,4]oxazine (200.0 mg, 0.93 mmol), 5-chlorobenzofuran-2-boronic acid (548 mg, 2.79 mmol), Cs₂CO₃ (1515 mg, 4.65 mmol) and Pd(PPh₃)₄ (3.2mg, 0.014mmol) were reacted in a mixed solvent of 1,4-dioxane (3 mL) and water (1 mL). The crude product was purified by silica gel column chromatography (CH₂Cl₂/MeOH = 400/1) to give **O108** (20.1 mg, 7.5% yield) as a light yellow solid. ¹H-NMR (400 MHz, Chloroform-d): δ 8.58 (s, 1H), 7.90 (s, 1H), 7.53 (d, *J =* 2.1 Hz, 1H), 7.43 (d, *J =* 8.7 Hz, 1H), 7.23 (d, *J* = 6.2 Hz, 2H), 4.51 (t, *J* = 4.4 Hz, 2H), 3.53 (t, *J* = 4.4 Hz, 2H).¹³C-NMR (101 MHz, Chloroform-d): δ 152.42, 151.50, 146.92, 138.79, 136.84, 130.58, 128.37, 124.72, 120.50, 112.04, 106.81, 66.34, 39.92. HRMS (ESI): m/z calcd. for C₁₅H₁₁ClN₂O₂⁺ [M+H]⁺:287.0582, found 287.0574.

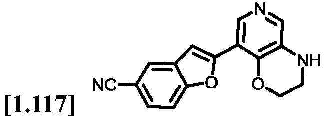

### 2-(3,4-Dihydro-2H-pyrido[4,3-b][1,4]oxazin-8-yl)benzofuran-5-carbonitrile (0109)

Following the general method OC, 8-bromo-3,4-dihydro-2H-pyrido[4,3-b][1,4]oxazine (200.0 mg, 0.93 mmol), 5-cyanobenzofuran-2-boronic acid (527.2 mg, 2.79 mmol), Cs₂CO₃ (1515 mg, 4.65 mmol) and Pd(PPh₃)₄ (3.2mg, 0.014mmol) were reacted in a mixed solvent of 1,4-dioxane (3 mL) and water (1 mL). The crude product was purified by silica gel column chromatography (CH₂Cl₂/MeOH = 400/1) to give **0109** (21.3 mg, 8.26% yield) as a yellow solid. ¹H-NMR (400 MHz, Chloroform-d): δ 8.59 (s, 1H), 7.94 (s, 1H), 7.90 (dd, *J =* 1.6, 0.7 Hz, 1H), 7.60 (dt, *J =* 8.5, 0.9 Hz, 1H), 7.56 (dd, *J =* 8.5, 1.6 Hz, 1H), 7.34 (d, *J =* 0.9 Hz, 1H), 4.56-4.49 (m, 2H), 3.59-3.50 (m, 2H). ¹³C-NMR (101 MHz, Chloroform-d): δ 155.65, 152.53, 147.10, 138.73, 137.25, 129.91, 128.15, 125.89, 119.56, 113.79, 112.22, 106.77, 106.67, 66.43, 39.87. HRMS (ESI): m/z calcd. for C₁₆H₁₁N₃O₂⁺ [M+H]⁺:278.0924, found 278.0918.

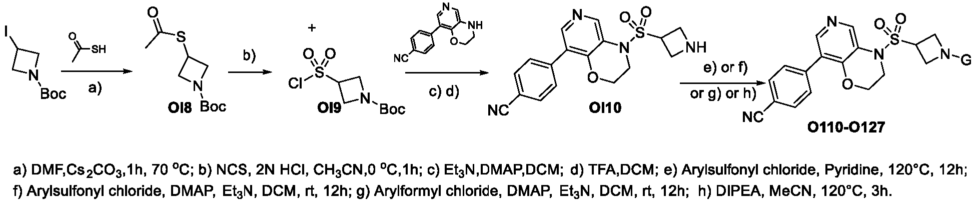

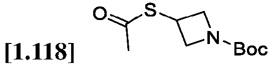

### tert-Butyl 3-(acetylthio)azetidine-1-carboxylate (OI8)

1-Boc-3-iodoazetidine (100.0 mg, 0.35 mmol) and thioacetic acid (53.74 mg, 0.71 mmol) were dissolved in DMF (1.0 mL). Subsequently, CsCO₃ (230.03 mg, 0.71 mmol) was added. The resulting mixture was stirred at 70 °C for 1 hour. The reaction was complete TLC detection is performed with iodine staining. After the reaction was complete as monitored by TLC (I₂ fuming), the reaction mixture was quenched with water and extracted with ethyl acetate. The organic phases were combined and washed with saturated brine, dried over anhydrous sodium sulfate, filtered. The solvent is removed under reduced pressure. The residue was purified by silica gel column chromatography (PE/EA = 100/1) to give **OI8** (51 mg, 62% yield) as a yellow oil. ¹H-NMR (400 MHz, Chloroform-d): δ 4.31 (s, 2H), 4.10 (tt, *J =* 8.2, 5.5 Hz, 1H), 3.74 (s, 2H), 2.29 (s, 3H), 1.38 (s, 9H).

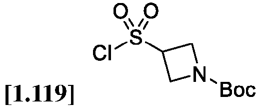

### tert-Butyl 3-(chlorosulfonyl)azetidine-1-carboxylate (OI9)

To a solution of **OI8** (50.0 mg, 0.22 mmol) in CH₃CN (1.25 mL) was added 2N HCl (0.80 mL) dropwise slowly at 0°C. Subsequently, NCS (115.37 mg, 0.86 mmol) was added, and the resulting mixture was stirred for 1 hour. After the reaction was complete as monitored by TLC (stained by KMnO₄), the reaction mixture was quenched with water and extracted with ethyl acetate. The organic phases were combined and washed with saturated brine, dried over anhydrous sodium sulfate and filtered. The solvent is removed under reduced pressure. The residue was purified by silica gel column chromatography (PE/EA = 8/1) to give **OI9** (20.0 mg, 37% yield) as a yellow solid. ¹H-NMR (400 MHz, Chloroform-d): δ 4.50 (td, *J* = 9.5, 8.7, 4.1 Hz, 1H), 4.36 (td, *J =* 10.2, 6.5 Hz, 4H), 1.44 (s, 9H).

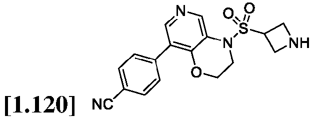

### 4-(4-(Azetidin-3-ylsulfonyl)-3,4-dihydro-2H-pyrido[4,3-b][1,4]oxazin-8-yl)benzonitrile (OI10)

To a solution of **OI4** (30.0 mg, 0.13 mmol) and DMAP (4.6 mg, 0.04 mmol) in anhydrous CH₂Cl₂ (1.0 mL) was added triethylamine (38.25 mg, 0.38 mmol) dropwise slowly at 0°C, followed by the addition of compound **OI3** (38.66 mg, 0.15 mmol). The resulting mixture was warmed to room temperature and stirred for 1 hour. After the reaction was complete as monitored by TLC, the reaction mixture was quenched with water and extracted with CH₂Cl₂. The organic phases were combined and washed with saturated brine, dried over anhydrous sodium sulfate and filtered. The solvent was removed under reduced pressure. The residue was purified by silica gel column chromatography (PE/EA = 1/1) to give a light yellow solid (20.0 mg, 37% yield). ¹H-NMR (400 MHz, Chloroform-d): δ 8.71 (s, 1H), 8.27 (s, 1H), 7.76-7.71 (m, 2H), 7.63-7.58 (m, 2H), 4.41-4.37 (m, 2H), 4.31 (dd, *J* = 9.3, 5.7 Hz, 2H), 4.23 (q, *J =* 3.4, 1.8 Hz, 3H), 3.92 (t, *J =* 4.5 Hz, 2H), 1.45 (s, 9H).

The resulting solid (50 mg, 0.11 mmol) was dissolved in anhydrous CH₂Cl₂ (1.0 mL) at 0°C. Trifluoroacetic acid (0.08 mL, 1.05 mmol) was added dropwise. The reaction mixture was warmed to room temperature and stirred for 2 hours. After the reaction was complete as monitored by TLC, the reaction mixture was quenched with water, the pH was adjusted to 10, and extracted with CH₂Cl₂. The organic phases were combined and washed with saturated brine, dried over anhydrous sodium sulfate and filtered. The solvent was removed under reduced pressure. The residue was purified by silica gel column chromatography (CH₂Cl₂/MeOH = 12/1) to give **OI10** (33 mg, 90% yield) as a white solid. ¹H-NMR (400 MHz, DMSO-d6): δ 8.64 (s, 1H), 8.25 (s, 1H), 7.95-7.91 (m, 2H), 7.80-7.76 (m, 2H), 4.79 (p, *J =* 7.5 Hz, 1H), 4.34 (dd, *J =* 5.2, 3.8 Hz, 2H), 3.85 (t, *J =* 4.5 Hz, 3H), 3.80 (s, 2H), 3.68 (d, *J =* 8.5 Hz, 2H).

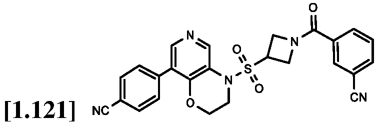

### 4-(4-((1-(3-Cyanobenzoyl)azetidin-3-yl)sulfonyl)-3,4-dihydro-2H-pyrido[4,3-b][1,4]oxazin-8-yl )benzonitrile (OI10)

Following the general method OA, synthesized with **OI10** (80 mg, 0.22 mmol), 3-cyanobenzoyl chloride (55.8 mg, 0.34 mmol), DMAP (8.21 mg, 0.07 mmol) and triethylamine (68 mg, 0.67 mmol), to give **OI10** (100 mg, 93.8% yield) as a white solid after chromatographic purification (PE/EA = 1/2), mp: 125.0-126.0 °C. ¹H-NMR (400 MHz, Chloroform-d): δ 8.69 (s, 1H), 8.28 (s, 1H), 7.94-7.85 (m, 2H), 7.81 (dt, *J =* 7.8, 1.4 Hz, 1H), 7.76-7.70 (m, 2H), 7.64-7.57 (m, 3H), 4.59 (s, 4H), 4.40 (q, *J =* 3.7, 2.8 Hz, 3H), 3.94 (d, *J =* 6.0 Hz, 2H); ¹³C-NMR (101 MHz, Chloroform-d): δ 168.32, 150.22, 146.60, 143.96, 138.06, 135.19, 133.24, 132.37 (2C), 132.19, 131.61, 130.30 (2C), 129.97, 121.79, 118.64, 117.86, 113.41, 112.32, 66.51, 50.12 (2C), 44.07; HRMS (ESI): m/z calcd. for C₂₅H₁₉N₅O₄S [M+H]⁺: 486.1231, found: 486.1197.

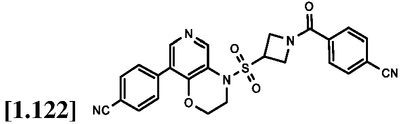

### 4-(4-((1-(4-Cyanobenzoyl)azetidin-3-yl)sulfonyl)-3,4-dihydro-2H-pyrido[4,3-b][1,4]oxazin-8-yl )benzonitrile (O111)

Following the general method OA, synthesized with **OI10** (80 mg, 0.22 mmol), 4-cyanobenzoyl chloride (55.8 mg, 0.34 mmol), DMAP (8.21 mg, 0.07 mmol) and triethylamine (68 mg, 0.67 mmol), to give **O111** (95 mg, 89.1% yield) as a white solid after chromatographic purification (PE/EA = 1/2), mp: 253.0-254.0 °C. ¹H-NMR (400 MHz, Chloroform-d): δ 8.33 (s, 1H), 7.93 (s, 1H), 7.39 (q, *J =* 6.1, 3.7 Hz, 6H), 7.25 (d, *J =* 7.9 Hz, 2H), 4.25 (d, *J =* 40.7 Hz, 4H), 4.04 (q, *J =* 8.6, 6.6 Hz, 3H), 3.58 (s, 2H); ¹³C-NMR (101 MHz, Chloroform-d): δ 167.56, 149.82, 146.22, 144.44, 138.56, 136.03, 132.70 (2C), 132.22, 130.35, 128.66 (2C), 123.83, 121.72, 118.73, 118.18, 113.89, 110.62, 66.07, 53.76 (2C), 50.10, 48.77, 43.05; HRMS (ESI): m/z calcd. for C₂₅H₁₉N₅O₄S [M+H]⁺: 486.1231, found: 486.1202.

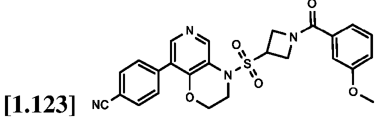

### 4-(4-((1-(3-Methoxybenzoyl)azetidin-3-yl)sulfonyl)-3,4-dihydro-2H-pyrido[4,3-b][1,4]oxazin-8 -yl)benzonitrile (0112)

Following the general method OA, synthesized with **OI10** (60 mg, 0.17 mmol), 3-methoxybenzoyl chloride (42.89 mg, 0.25 mmol), DMAP (6.11 mg, 0.05 mmol) and triethylamine (51 mg, 0.50 mmol), to give **O112** (75 mg, 90.1% yield) as a white solid after chromatographic purification (PE/EA = 1/2), mp: 176.0-177.0 °C. ¹H-NMR (400 MHz, Chloroform-d): δ 8.71 (s, 1H), 8.27 (s, 1H), 7.73 (d, *J =* 8.2 Hz, 2H), 7.60 (d, *J =* 8.3 Hz, 2H), 7.33 (t, *J* = 7.9 Hz, 1H), 7.19-7.11 (m, 2H), 7.04 (dd, *J =* 8.2, 2.6 Hz, 1H), 4.60 (d, *J =* 50.5 Hz, 4H), 4.42-4.31 (m, 3H), 3.93 (s, 2H), 3.83 (s, 3H); ¹³C-NMR (101 MHz, Chloroform-d): δ 170.65, 161.23, 151.52, 147.68, 144.17, 138.22, 133.14, 132.32 (2C), 130.30 (2C), 129.86, 124.87, 121.85, 119.99, 118.67, 118.02, 113.25, 112.20, 64.56, 55.59 (2C), 49.50, 44.04; HRMS (ESI): m/z calcd. for C₂₅H₂₂N₄O₅S [M+H]⁺: 491.1384, found: 491.1350.

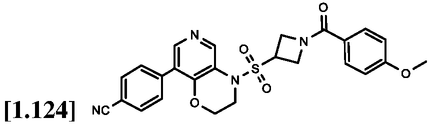

### 4-(4-((1-(4-Methoxybenzoyl)azetidin-3-yl)sulfonyl)-3,4-dihydro-2H-pyrido[4,3-b][1,4]oxazin-8 -yl)benzonitrile (0113)

Following the general method OA, synthesized with **OI10** (60 mg, 0.17 mmol), 4-methoxybenzoyl chloride (42.89 mg, 0.25 mmol), DMAP (6.11 mg, 0.05 mmol) and triethylamine (51 mg, 0.50 mmol), to give **0113** (72 mg, 86.5% yield) as a white solid after chromatographic purification (PE/EA = 1/2), mp: 200.0-201.0 °C. ¹H-NMR (400 MHz, Chloroform-d): δ 8.71 (s, 1H), 8.27 (s, 1H), 7.78-7.70 (m, 2H), 7.65-7.54 (m, 4H), 6.98-6.86 (m, 2H), 4.53 (s, 4H), 4.41-4.31 (m, 3H), 3.93 (t, *J =* 4.6 Hz, 2H), 3.84 (s, 3H); ¹³C-NMR (101 MHz, Chloroform-d): δ 170.88, 162.53, 150.41, 146.84, 144.36, 138.30, 132.86 (2C), 130.29 (2C), 130.05 (2C), 124.78, 124.03, 122.28, 119.14, 114.08 (2C), 112.13, 66.41, 56.19 (2C), 51.98, 44.98; HRMS (ESI): m/z calcd. for C₂₅H₂₂N₄O₅S [M+H]⁺: 491.1384, found: 491.1378.

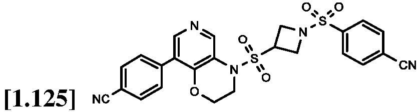

### 4-((3-((8-(4-Cyanophenyl)-2,3-dihydro-4H-pyrido[4,3-b][1,4]oxazin-4-yl)sulfonyl)azetidin-1-yl )sulfonyl)benzonitrile (0114)

Following the general method OI, synthesized with **OI10** (60 mg, 0.17 mmol), 4-cyanobenzenesulfonyl chloride (50.81 mg, 0.25 mmol), DMAP (6.11 mg, 0.05 mmol) and triethylamine (51 mg, 0.50 mmol), to give **O114** (70 mg, 79.1% yield) as a white solid after chromatographic purification (EA/MeOH = 50/1), mp: 260.0-261.0 °C. ¹H-NMR (400 MHz, DMSO-d6): δ 8.48 (s, 1H), 8.26 (s, 1H), 8.22-8.17 (m, 2H), 8.07-8.01 (m, 2H), 7.95-7.89 (m, 2H), 7.80-7.73 (m, 2H), 4.68 (tt, *J* = 8.4, 6.0 Hz, 1H), 4.30-4.25 (m, 2H), 4.21 (t, *J* = 9.0 Hz, 2H), 4.03-3.96 (m, 2H), 3.80 (t, *J =* 4.5 Hz, 2H); ¹³C-NMR (101 MHz, DMSO-d6): δ 149.66, 146.35, 144.66, 138.59, 137.40, 134.39 (2C), 132.23 (2C), 130.85 (2C), 129.14 (2C), 123.82, 121.91, 119.16, 118.15, 116.24, 110.62, 65.94, 52.86 (2C), 47.89, 43.75; HRMS (ESI): m/z calcd. for C₂₄H₁₉N₅O₅S₂ [M+H]⁺: 522.0900, found: 522.0893.

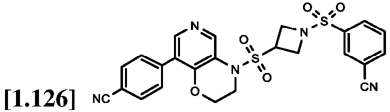

### 3-((3-((8-(4-Cyanophenyl)-2,3-dihydro-4H-pyrido[4,3-b][1,4]oxazin-4-yl)sulfonyl)azetidin-1-yl )sulfonyl)benzonitrile (0115)

Following the general method OI, synthesized with **OI10** (60 mg, 0.17 mmol), 3-cyanobenzenesulfonyl chloride (50.81 mg, 0.25 mmol), DMAP (6.11 mg, 0.05 mmol) and triethylamine (51 mg, 0.50 mmol), to give **O115** (75 mg, 84.7% yield) as a white solid after chromatographic purification (PE/EA = 1/2), mp: 211.0-212.0 °C. ¹H-NMR (400 MHz, DMSO-d6): δ 8.53 (s, 1H), 8.28 (s, 1H), 8.15 (d, *J =* 1.8 Hz, 1H), 8.08 (d, *J =* 8.0 Hz, 1H), 7.98 (d, *J =* 7.8 Hz, 1H), 7.80 (t, *J* = 7.9 Hz, 1H), 7.74 (d, *J =* 8.2 Hz, 2H), 7.59 (d, *J =* 8.1 Hz, 2H), 4.37-4.32 (m, 2H), 4.26 (dd, *J =* 8.8, 6.2 Hz, 2H), 4.23-4.17 (m, 2H), 4.15 (d, *J =* 5.5 Hz, 1H), 3.82 (t, *J =* 4.5 Hz, 2H); ¹³C-NMR (101 MHz, DMSO-d6): δ 149.66, 146.33, 144.28, 138.59, 137.47, 134.67, 132.80, 132.23 (2C), 132.05, 130.99, 130.36 (2C), 123.82, 121.52, 118.77, 117.54, 113.13, 110.62, 65.53, 52.69 (2C), 47.14, 43.95; HRMS (ESI): m/z calcd. for C₂₄H₁₉N₅O₅S₂ [M+H]⁺: 522.900, found: 522.0897.

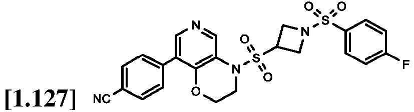

### 4-(4-((1-((4-Fluorophenyl)sulfonyl)azetidin-3-yl)sulfonyl)-3,4-dihydro-2H-pyrido[4,3-b][1,4]ox azin-8-yl)benzonitrile (0116)

Following the general method OI, synthesized with **OI10** (80 mg, 0.22 mmol), 4-fluorobenzenesulfonyl chloride (65.58 mg, 0.34 mmol), DMAP (8.21 mg, 0.07 mmol) and triethylamine (68 mg, 0.67 mmol) to give **0116** (102 mg, 90.3% yield) as a white solid after chromatographic purification (PE/EA = 1/1), mp: 204.0-205.0 °C. ¹H-NMR (400 MHz, Chloroform-d): δ 8.56 (s, 1H), 8.27 (s, 1H), 7.87 (dd, *J* = 8.5, 4.9 Hz, 2H), 7.73 (d, *J =* 7.8 Hz, 2H), 7.59 (d, *J =* 8.0 Hz, 2H), 7.31 (t, *J =* 8.4 Hz, 2H), 4.40-4.30 (m, 2H), 4.23-4.05 (m, 5H), 3.82 (t, *J =* 4.5 Hz, 2H); ¹³C-NMR (101 MHz, Chloroform-d): δ 167.49 (¹*J*_{CF} = 247.1 Hz), 149.81, 147.20, 144.54, 138.25, 132.33 (2C), 131.34 (³*J*_{CF} = 9.5 Hz), 130.29 (2C), 124.86, 121.46, 118.68, 116.94 (²*J*_{CF} = 22.5 Hz), 112.20, 66.68, 53.05 (2C), 47.78, 44.62; ¹⁹F NMR (377 MHz, Chloroform-d): δ -102.60; HRMS (ESI): m/z calcd. for C₂₃H₁₉FN₄O₅S₂ [M+H]⁺: 515.0854, found: 515.0849.

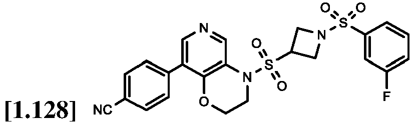

### 4-(4-((1-((3-Fluorophenyl)sulfonyl)azetidin-3-yl)sulfonyl)-3,4-dihydro-2H-pyrido[4,3-b][1,4]ox azin-8-yl)benzonitrile (0117)

Following the general method OI, synthesized with **OI10** (80 mg, 0.22 mmol), 3-fluorobenzenesulfonyl chloride (65.58 mg, 0.34 mmol), DMAP (8.21 mg, 0.07 mmol) and triethylamine (68 mg, 0.67 mmol) to give **0117** (100 mg, 88.5% yield) as a white solid after chromatographic purification (PE/EA= 1/1), mp: 199.0-200.0 °C. ¹H-NMR (400 MHz, DMSO-d6): δ 8.51 (s, 1H), 8.26 (s, 1H),7.92 (d, *J =* 8.1 Hz, 2H), 7.82-7.65 (m, 6H), 4.69 (s, 1H), 4.28 (s, 2H), 4.19 (s, 2H), 4.00 (s, 2H), 3.80 (s, 2H); ¹³C-NMR (101 MHz, DMSO-d6): δ 161.34 (¹*J*_{CF} = 205.2 Hz), 149.61, 146.28, 144.25, 138.58, 135.08, 132.19 (2C), 131.94 (³*J*_{CF} = 8.1 Hz), 130.33 (2C), 124.68, 123.77, 121.52, 121.29 (²*J*_{CF} = 21.1 Hz), 118.73, 115.54, 115.30, 110.59, 65.89, 52.45, 47.86, 43.72; ¹⁹F NMR (377 MHz, DMSO-d6): δ -109.83; HRMS (ESI): m/z calcd. for C₂₃H₁₉FN₄O₅S₂ [M+H]⁺: 515.0854, found: 515.0849.

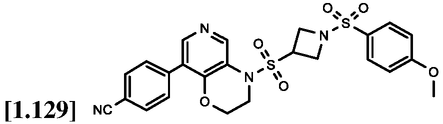

### 4-(4-((1-((4-Methoxyphenyl)sulfonyl)azetidin-3-yl)sulfonyl)-3,4-dihydro-2H-pyrido[4,3-b][1,4] oxazin-8-yl)benzonitrile (0118)

Following the general method OI, synthesized with **OI10** (80 mg, 0.22 mmol), 4-methoxybenzenesulfonyl chloride (69.61 mg, 0.34 mmol), DMAP (8.21 mg, 0.07 mmol) and triethylamine (68 mg, 0.67 mmol) to give **O118** (105 mg, 90.8% yield) as a white solid after chromatographic purification (PE/EA = 1/1), mp: 148.0-149.0 °C. ¹H-NMR (400 MHz, Chloroform-d): δ 8.55 (s, 1H), 8.25 (s, 1H), 7.81-7.75 (m, 2H), 7.75-7.70 (m, 2H), 7.62-7.56 (m, 2H), 7.10-7.05 (m, 2H), 4.34 (dd, *J =* 5.2, 3.8 Hz, 2H), 4.18-4.05 (m, 5H), 3.90 (s, 3H), 3.84-3.78 (m, 2H); ¹³C-NMR (101 MHz, Chloroform-d): δ 164.61, 149.76, 147.07, 144.58, 137.83, 132.29 (2C), 130.77 (2C), 130.29 (2C), 125.16, 124.74, 121.54, 118.70, 114.86 (2C), 112.11, 66.21, 57.06, 52.18 (2C), 49.36, 43.91. HRMS (ESI): m/z calcd. for C₂₄H₂₂N₄O₆S₂ [M+H]⁺: 527.1054, found: 527.1054.

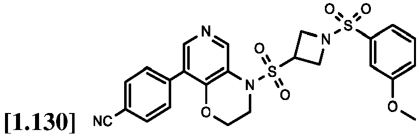

### 4-(4-((1-((3-Methoxyphenyl)sulfonyl)azetidin-3-yl)sulfonyl)-3,4-dihydro-2H-pyrido[4,3-b][1,4] oxazin-8-yl)benzonitrile (0119)

Following the general method OI, synthesized with **OI10** (80 mg, 0.22 mmol), 3-methoxybenzenesulfonyl chloride (69.61 mg, 0.34 mmol), DMAP (8.21 mg, 0.07 mmol) and triethylamine (68 mg, 0.67 mmol) to give **O119** (109 mg, 94.3% yield) as a white solid after chromatographic purification (PE/EA = 1/1), mp: 200.0-201.0 °C. ¹H-NMR (400 MHz, Chloroform-d): δ 8.54 (s, 1H), 8.25 (s, 1H), 7.72 (d, *J =* 8.1 Hz, 2H), 7.59 (d, *J =* 8.0 Hz, 2H), 7.53 (t, *J=* 8.0 Hz, 1H), 7.41 (d, *J =* 7.7 Hz, 1H), 7.32 (t, *J =* 2.1 Hz, 1H), 7.22 (dd, *J =* 8.3, 2.6 Hz, 1H), 4.37-4.29 (m, 2H), 4.14 (s, 5H), 3.89 (s, 3H), 3.80 (t, *J* = 4.5 Hz, 2H); ¹³C-NMR (101 MHz, Chloroform-d): δ 159.96, 149.77, 147.10, 144.52, 138.31, 135.44, 132.67 (2C), 130.72, 130.29 (2C), 124.76, 121.52, 120.60, 120.31, 118.70, 113.29, 112.14, 66.67, 57.26, 53.04 (2C), 48.83, 42.88; HRMS (ESI): m/z calcd. for C₂₄H₂₂N₄O₆S₂ [M+H]⁺: 527.1054, found: 527.1051.

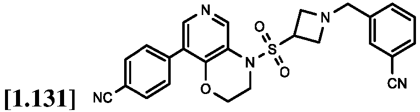

### 3-((3-((8-(4-Cyanophenyl)-2,3-dihydro-4H-pyrido[4,3-b][1,4]oxazin-4-yl)sulfonyl)azetidin-1-yl )methyl)benzonitrile (0120)

Following the general method OF, synthesized with **OI10** (60 mg, 0.17 mmol), 3-cyanobenzyl bromide (49.41mg, 0.25mmol) and anhydrous K₂CO₃ (46.44mg, 0.34mmol) to give **O120** (30 mg, 37.5% yield) as a white solid after chromatographic purification (PE/EA = 1/2), mp: 158.0-159.0 °C. ¹H-NMR (400 MHz, Chloroform-d): δ 8.72 (s, 1H), 8.25 (s, 1H), 7.74-7.70 (m, 2H), 7.62-7.58 (m, 2H), 7.59-7.53 (m, 2H), 7.50 (dt, *J =* 7.9, 1.6 Hz, 1H), 7.42 (t, *J =* 7.7 Hz, 1H), 4.37 (dd, *J =* 5.2, 3.9 Hz, 2H), 4.22 (p, *J =* 7.5 Hz, 1H), 3.87 (dd, *J =* 5.2, 3.9 Hz, 2H), 3.72 (s, 2H), 3.68 (td, *J* = 7.6, 1.3 Hz, 2H), 3.61-3.54 (m, 2H); ¹³C-NMR (101 MHz, Chloroform-d): δ 149.70, 146.87, 144.85, 138.52, 133.28, 132.26 (2C), 131.87, 131.39, 130.27 (2C), 129.52, 124.58, 121.94, 118.71, 112.84, 112.00, 66.85, 62.51, 56.17, 51.00, 43.01; HRMS (ESI): m/z calcd. for C₂₅H₂₁N₅O₃S [M+H]⁺: 472.1438, found: 472.1434.

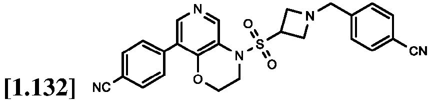

### 4-(4-((1-(4-Cyanobenzyl)azetidin-3-yl)sulfonyl)-3,4-dihydro-2H-pyrido[4,3-b][1,4]oxazin-8-yl) benzonitrile (0121)

Following the general method OF **OI10** (80 mg, 0.22 mmol), 4-cyanobenzyl bromide (65.85 mg, 0.34 mmol) and anhydrous K₂CO₃ (61.92 mg, 0.44 mmol) to give **O121** (40 mg, 38.6% yield) as a white solid after chromatographic purification (PE/EA = 1/1), mp: 181.0-182.0 °C. ¹H-NMR (400 MHz, Chloroform-d): δ 8.72 (s, 1H), 8.25 (s, 1H), 7.77-7.69 (m, 2H), 7.60 (dd, *J =* 8.2, 3.6 Hz, 4H), 7.39 (d, *J =* 8.0 Hz, 2H), 4.37 (t, *J =* 4.4 Hz, 2H), 4.22 (p, *J =* 7.5 Hz, 1H), 3.87 (t, *J =* 4.5 Hz, 2H), 3.76 (s, 2H), 3.69 (t, *J* = 7.9 Hz, 2H), 3.59 (t, *J* = 7.7 Hz, 2H); ¹³C-NMR (101 MHz, Chloroform-d): δ 149.72, 146.90, 144.88, 142.31, 138.48, 132.53 (2C), 132.28 (2C), 130.27 (2C), 129.07 (2C), 124.29, 121.90, 118.78, 118.70, 112.06, 111.63, 68.25, 63.28, 55.82 (2C), 52.28, 43.32; HRMS (ESI): m/z calcd. for C₂₅H₂₁N₅O₃S [M+H]⁺: 471.5350, found: 472.1438.

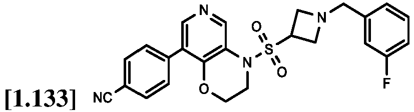

### 4-(4-((1-(3-Fluorobenzyl)azetidin-3-yl)sulfonyl)-3,4-dihydro-2H-pyrido[4,3-b][1,4]oxazin-8-yl) benzonitrile (0122)

Following the general method OF, synthesized with **OI10** (80 mg, 0.22 mmol), 3-fluorobenzyl chloride (48.58 mg, 0.34 mmol), anhydrous K₂CO₃ (61.92 mg, 0.44 mmol) to give **O122** (50 mg, 49.0% yield) as a white solid after chromatographic purification (PE/EA = 1/1), mp: 164.0-165.0 °C. ¹H-NMR (400 MHz, Chloroform-d): δ 8.73 (s, 1H), 8.25 (s, 1H), 7.72 (d, *J =* 8.0 Hz, 2H), 7.60 (d, *J =* 8.0 Hz, 2H), 7.30-7.24 (m, 1H), 7.07-6.91 (m, 3H), 4.42-4.32 (m, 2H), 4.22 (p, *J =* 7.6 Hz, 1H), 3.87 (t, *J =* 4.5 Hz, 2H), 3.69 (d, *J =* 9.1 Hz, 4H), 3.58 (t, *J =* 7.8 Hz, 2H): ¹³C-NMR (101 MHz, Chloroform-d): δ 162.33 (¹*J*_{CF} = 244.9 Hz), 149.69, 146.90, 144.90, 139.08, 138.55, 132.27 (2C), 130.28 (³*J*_{CF} = 8.3 Hz), 124.55, 124.14, 124.11, 121.94, 118.74, 116.56, 115.47, 114.62 (²*J*_{CF} = 21.1 Hz), 112.00, 66.18, 62.20, 55.17, 51.12, 43.78; ¹⁹F NMR (377 MHz, Chloroform-d): δ-112.80; HRMS (ESI): m/z calcd. for C₂₄H₂₁FN₄O₃S [M+H]⁺: 465.1391, found: 465.1393.

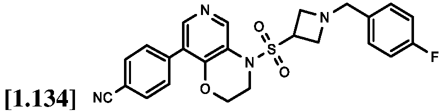

### 4-(4-((1-(4-Fluorobenzyl)azetidin-3-yl)sulfonyl)-3,4-dihydro-2H-pyrido[4,3-b][1,4]oxazin-8-yl) benzonitrile (0123)

Following the general method OF, synthesized with **OI10** (80 mg, 0.22 mmol), 4-fluorobenzyl chloride (48.58 mg, 0.34 mmol) and anhydrous K₂CO₃ (61.92 mg, 0.44 mmol) to give **O123** (45 mg, 44.1% yield) as a white solid after chromatographic purification (PE/EA = 1/1), mp: 120.0-121.0 °C. ¹H-NMR (400 MHz, Chloroform-d): δ 8.73 (s, 1H), 8.25 (s, 1H), 7.76-7.69 (m, 2H), 7.63-7.56 (m, 2H), 7.22 (dd, *J =* 8.4, 5.6 Hz, 2H), 6.99 (t, *J =* 8.6 Hz, 2H), 4.40-4.33 (m, 2H), 4.19 (p, *J =* 7.5 Hz, 1H), 3.86 (t, *J =* 4.5 Hz, 2H), 3.70-3.61 (m, 4H), 3.58-3.51 (m, 2H); ¹³C-NMR (101 MHz, Chloroform-d): δ 161.19 (¹*J*_{CF} = 244.7 Hz), 149.68, 146.88, 144.93, 138.56, 133.25, 132.71 (2C), 130.27 (³*J*_{CF} = 8.7 Hz), 124.54, 121.96, 118.71, 115.48 (²*J*_{CF} = 21.2 Hz), 112.01, 66.17, 62.06, 55.04, 51.14, 43.76; ¹⁹F NMR (377 MHz, Chloroform-d): δ -114.67; HRMS (ESI): m/z calcd. for C₂₄H₂₁FN₄O₃S [M+H]⁺: 465.1391, found: 465.1386.

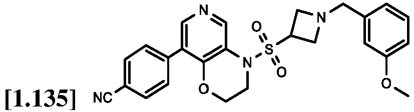

### 4-(4-((1-(3-Methoxybenzyl)azetidin-3-yl)sulfonyl)-3,4-dihydro-2H-pyrido[4,3-b][1,4]oxazin-8-yl)benzonitrile (0124)

Following the general method OF, synthesized with **OI10** (60 mg, 0.17 mmol), 3-methoxybenzyl chloride (40 mg, 0.25 mmol) and anhydrous K₂CO₃ (46.44 mg, 0.37 mmol) to give **0124** (45 mg, 56% yield) as a white solid after chromatographic purification (PE/EA = 1/1), mp: 123.0-124.0 °C. ¹H-NMR (400 MHz, Chloroform-d): δ 8.73 (s, 1H), 8.24 (s, 1H), 7.72 (d, *J =* 8.1 Hz, 2H), 7.60 (d, *J =* 8.0 Hz, 2H), 7.22 (td, *J =* 7.4, 1.6 Hz, 1H), 6.86-6.77 (m, 3H), 4.36 (t, *J =* 4.5 Hz, 2H), 4.21 (p, *J =* 7.6 Hz, 1H), 3.86 (t, *J =* 4.5 Hz, 2H), 3.79 (s, 3H), 3.68 (d, *J =* 2.6 Hz, 4H), 3.57 (t, *J* = 7.8 Hz, 2H); ¹³C-NMR (101 MHz, Chloroform-*d*): δ 159.95, 149.65, 146.87, 144.94, 138.58, 137.96, 132.25 (2C), 130.28 (2C), 129.73, 124.52, 121.97, 120.89, 118.74, 114.07, 113.27, 111.97, 66.16, 62.79, 55.35, 55.11, 51.17, 43.75; HRMS (ESI): m/z calcd. for C₂₅H₂₄N₄O₄S [M+H]⁺: 477.1591, found: 477.1583.

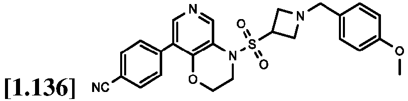

### 4-(4-((1-(4-Methoxybenzyl)azetidin-3-yl)sulfonyl)-3,4-dihydro-2H-pyrido[4,3-b][1,4]oxazin-8-yl)benzonitrile (0125)

Following the general method OF, synthesized with **OI10** (60 mg, 0.17 mmol), 4-methoxybenzyl chloride (40 mg, 0.25 mmol) and anhydrous K₂CO₃ (46.44 mg, 0.37 mmol) to give **O125** (40 mg, 49.5% yield) as a white solid after chromatographic purification (PE/EA = 1/1), mp: 130.0-131.0 °C. ¹H-NMR (400 MHz, Chloroform-d): δ 8.73 (s, 1H), 8.24 (s, 1H), 7.75-7.70 (m, 2H), 7.62-7.57 (m, 2H), 7.19-7.13 (m, 2H), 6.86-6.80 (m, 2H), 4.35 (dd, *J =* 5.2, 3.8 Hz, 2H), 4.18 (p, *J =* 7.6 Hz, 1H), 3.89-3.83 (m, 2H), 3.78 (s, 3H), 3.66-3.58 (m, 4H), 3.52 (dd, *J =* 8.5, 7.2 Hz, 2H); ¹³C-NMR (101 MHz, Chloroform-*d*): δ 159.22, 149.63, 146.81, 144.94, 138.58, 132.23 (2C), 130.27 (2C), 129.86, 128.49, 124.48, 121.98, 118.73, 114.06, 111.93, 66.14, 62.26, 55.36, 54.89, 51.19, 43.72; HRMS (ESI): m/z calcd. for C₂₅H₂₄N₄O₄S [M+H]⁺: 477.1591, found: 477.1582.

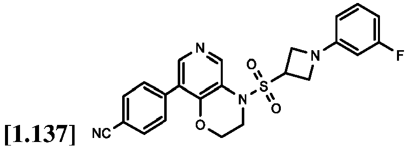

### 4-(4-((1-(3-Fluorophenyl)azetidin-3-yl)sulfonyl)-3,4-dihydro-2H-pyrido[4,3-b][1,4]oxazin-8-yl) benzonitrile (0126)

Following the general method OF, synthesized with **OI10** (80 mg, 0.22 mmol), 3-fluorobromobenzene (78.4 mg, 0.45 mmol), Cs₂CO₃ (218.95 mg, 0.67 mmol), Xantphos (6.48 mg, 0.01 mmol) and Pd₂(dba)₃ (10.26 mg, 0.01 mmol) to give **O126** (40 mg, 40% yield) as a white solid after chromatographic purification (PE/EA = 1/1), mp: 236.0-237.0 °C. ¹H-NMR (400 MHz, Chloroform-d): δ 8.76 (s, 1H), 8.29 (s, 1H), 7.74 (d, *J =* 7.9 Hz, 2H), 7.61 (d, *J =* 7.9 Hz, 2H), 7.19 (q, *J* = 7.8 Hz, 1H), 6.58-6.46 (m, 1H), 6.22 (d, *J* = 8.2 Hz, 1H), 6.15 (d, *J* = 10.9 Hz, 1H), 4.51-4.37 (m, 3H), 4.23 (p, *J* = 7.9 Hz, 4H), 3.95 (t, *J* = 4.6 Hz, 2H); ¹³C-NMR (101 MHz, DMSO-d6): δ 164.37 (¹*J*_{CF} = 239.9 Hz), 152.28, 152.18, 149.84, 146.15, 144.56, 138.61, 132.22 (2C), 130.44 (³*J*_{CF} = 9.9 Hz, 2C), 130.36, 128.84, 123.85, 121.83, 118.75, 110.61, 107.62, 104.17 (²*J*_{CF} = 21.3 Hz), 98.70, 98.45, 66.06, 53.14 (2C), 49.27, 3.02; ¹⁹F NMR (377 MHz, Chloroform-d): δ -121.89; HRMS (ESI): m/z calcd. for C₂₃H₁₉FN₄O₃S [M+H]⁺: 451.1235, found: 451.1236.

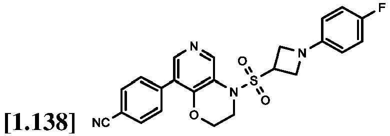

### 4-(4-((1-(4-Fluorophenyl)azetidin-3-yl)sulfonyl)-3,4-dihydro-2H-pyrido[4,3-b][1,4]oxazin-8-yl) benzonitrile (0127)

Following the general method OF, synthesized with **OI10** (80 mg, 0.22 mmol), 4-fluorobromobenzene (78.4 mg, 0.45 mmol), Cs₂CO₃ (218.95 mg, 0.67 mmol), Xantphos (6.48 mg, 0.01 mmol) and Pd₂(dba)₃ (10.26 mg, 0.01 mmol) to give **0126** (42 mg, 42.5% yield) as a white solid after chromatographic purification (PE/EA = 1/1), mp: 224.0-225.0 °C. ¹H-NMR (400 MHz, Chloroform-*d*): δ 8.75 (s, 1H), 8.28 (s, 1H), 7.74 (d, *J =* 8.0 Hz, 2H), 7.61 (d, *J =* 8.0 Hz, 2H), 6.95 (t, *J =* 8.4 Hz, 2H), 6.40 (dd, *J =* 8.8, 4.3 Hz, 2H), 4.42 (q, *J =* 5.4, 4.3 Hz, 3H), 4.20 (h, *J =* 7.9, 7.4 Hz, 4H), 3.94 (t, *J =* 4.5 Hz, 2H); ¹³C-NMR (101 MHz, Chloroform-d): δ 155.84 (¹*J*_{CF} = 235.9 Hz), 149.74, 146.98, 146.80, 144.79, 138.52, 132.31 (2C), 130.31 (2C), 129.31, 124.67, 121.97, 118.73, 116.05 (²*J*_{CF} = 22.6 Hz), 112.92 (³*J*_{CF} = 7.5 Hz), 112.09, 111.86, 66.37, 53.87, 53.50, 51.03, 44.00; ¹⁹F NMR (377 MHz, Chloroform-d): δ -125.52; HRMS (ESI): m/z calcd. for C₂₃H₁₉FN₄O₃S [M+H]⁺: 451.1235, found: 451.1232.

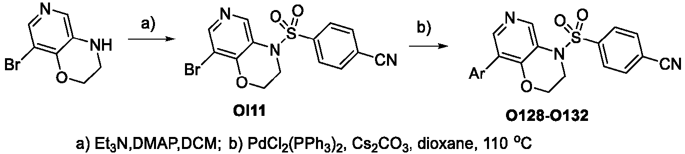

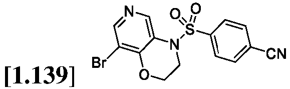

### 4-((8-Bromo-2,3-dihydro-4H-pyrido[4,3-b][1,4]oxazin-4-yl)sulfonyl)benzonitrile (OI11)

Following the general method OB, synthesized with **OI3** (100 mg, 0.47 mmol) and 4-cyanobenzenesulfonyl chloride (187.5 mg, 0.93 mmol) in pyridine (20 mL) to give **OI11** (157.3 mg, 88.0% yield) as a white solid after chromatographic purification (PE/EA = 6/2). ¹H-NMR (400 MHz, Chloroform-d): δ 8.85 (s, 1H), 8.42 (s, 1H), 7.94-7.72 (m, 4H), 4.01 (t, *J =* 4.2 Hz, 2H), 3.94 (t, *J* = 4.2 Hz, 2H).

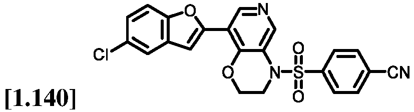

### 4-((8-(5-Chlorobenzofuran-2-yl)-2,3-dihydro-4H-pyrido[4,3-b][1,4]oxazin-4-yl)sulfonyl)benzo nitrile (0128)

Following the general method OC, synthesized with **OI11** (100 mg, 0.26 mmol) and 5-chlorobenzofuran-2-boronic acid (76 mg, 0.39 mmol), Cs₂CO₃ (339 mg, 1.04 mmol), Pd(OAc)₂ (3 mg, 0.013mmol), dppf (14 mg, 0.026 mmol) and CuCl (26mg, 0.026 mmol) to give 0128 (32 mg, 26.8% yield) as a white solid after chromatographic purification (PE/EA = 4/1). ¹H-NMR (400 MHz, Chloroform-d): δ 8.99 (s, 1H), 8.93 (s, 1H), 7.86 (d, *J =* 8.5 Hz, 2H), 7.80 (d, *J =* 8.5 Hz, 2H), 7.51 (d, *J* = 2.1 Hz, 1H), 7.45 (d, *J =* 8.7 Hz, 1H), 7.28 (d, *J =* 2.1 Hz, 1H), 7.15 (s, 1H), 4.17-4.10 (m, 2H), 4.05-3.99 (m, 2H). ¹³C-NMR (101 MHz, Chloroform-d): δ 151.47, 148.75, 148.23, 144.06, 141.26, 132.42, 129.10, 127.69, 126.95, 124.43, 119.69, 116.68, 115.73, 111.15, 106.89, 76.27, 76.01, 75.70, 63.50, 42.50, 28.68.

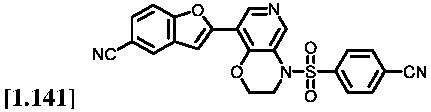

### 2-(4-((4-Cyanophenyl)sulfonyl)-3,4-dihydro-2H-pyrido[4,3-b][1,4]oxazin-8-yl)benzofuran-5-ca rbonitril (0129)

Following the general method OC, synthesized with **OI11** (100 mg, 0.26 mmol), 5-cyanobenzofuran-2-boronic acid (73 mg, 0.39 mmol), Cs₂CO₃ (339 mg, 1.04 mmol), Pd(OAc)₂ (3 mg, 0.013mmol), dppf (14 mg, 0.026 mmol) and CuCl (26mg, 0.026 mmol) to give **0129** (41 mg, 35.3% yield) as a white solid after chromatographic purification (PE/EA = 3/1). ¹H-NMR (400 MHz, DMSO-*d₆*): δ 8.84 (s, 1H), 8.79 (s, 1H), 8.19 (s, 1H), 8.10 (d, *J =* 7.9 Hz, 2H), 7.98 (d, *J =* 7.9 Hz, 2H), 7.84 (d, *J* = 8.2 Hz, 1H), 7.77 (d, *J =* 8.2 Hz, 1H), 7.45 (s, 1H), 4.21-3.95 (m, 4H).

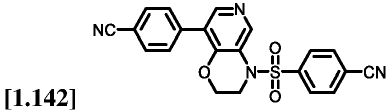

### 4-((8-(4-Cyanophenyl)-2,3-dihydro-4H-pyrido[4,3-b][1,4]oxazin-4-yl)sulfonyl)benzonitrile (O130)

Following the general method OC, synthesized with **OI11** (100 mg, 0.26 mmol), 4-cyanophenylboronic acid (80mg, 0.29 mmol), K₃PO₄ (165mg, 0.78 mmol) and PdCl₂(dppf) (19 mg, 0.026mmol) to give **O130** (41 mg, 35.3% yield) as a white solid after chromatographic purification (PE/EA = 4/1). ¹H-NMR (400 MHz, Chloroform-d): δ 8.91 (s, 1H), 8.27 (s, 1H), 7.87 (d, *J* = 8.3 Hz, 2H), 7.81 (d, *J* = 8.3 Hz, 2H), 7.38 (d, *J* = 8.5 Hz, 2H), 7.34 (d, *J* = 8.5 Hz, 2H), 4.04-3.84 (m, 4H).

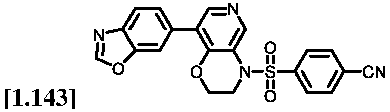

### 4-((8-(Benzo[d]oxazol-6-yl)-2,3-dihydro-4H-pyrido[4,3-b][1,4]oxazin-4-yl)sulfonyl)benzonitril e (0131)

Following the general method OC, synthesized with **OI11** (100 mg, 0.26 mmol), 1,3-benzoxazole-6-boronic acid pinacol ester (96mg, 0.29 mmol), K₃PO₄ (165mg, 0.78 mmol) and PdCl₂(dppf) (19 mg, 0.026mmol) to give **O131** (36 mg, 32.7% yield) as a light yellow solid after chromatographic purification (PE/EA = 3/1). ¹H-NMR (400 MHz, Chloroform-d): δ 8.94 (s, 1H), 8.35 (s, 1H), 8.13 (s, 1H), 7.88 (d, *J =* 8.4 Hz, 2H), 7.83-7.81 (m, 3H), 7.66 (s, 1H), 7.41 (dd, *J =* 8.3, 1.2 Hz, 1H), 4.05-3.87 (m, 4H).

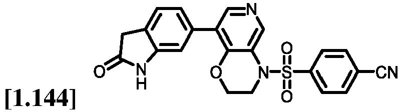

### 4-((8-(2-Oxoindolin-6-yl)-2,3-dihydro-4H-pyrido[4,3-b][1,4]oxazin-4-yl)sulfonyl)benzonitrile (O132)

Following the general method OC, synthesized with **OI11** (100 mg, 0.26 mmol), 5-(4,4,5,5-tetramethyl-1,3,2-dioxaborolan-2-yl)indolin-2-one (75 mg, 0.29 mmol), K₂CO₃ (144 mg, 1.04 mmol), PdCl₂(PPh₃)₂ (18 mg, 0.026mmol) and Pcy₃ (14 mg, 0.0052 mmol) to give **O132** (45 mg, 39.5% yield) as a yellow solid after chromatographic purification (PE/EA = 1/1). ¹H-NMR (400 MHz, DMSO-*d₆*): δ 10.46 (s, 1H), 8.69 (s, 1H), 8.19 (s, 1H), 8.12 (d, *J =* 8.4 Hz, 2H), 7.96 (d, *J =* 8.4 Hz, 2H), 7.29 (s, 1H), 7.23 (s, 1H), 6.85 (d, *J =* 8.0 Hz, 1H), 4.02-3.96 (m, 2H), 3.90-3.85 (m, 2H), 3.48 (s, 2H).

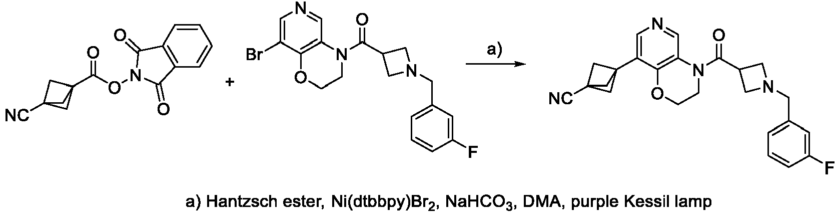

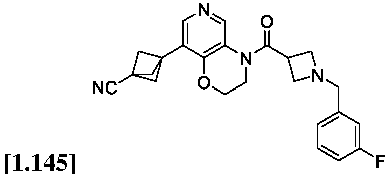

### 3-(4-(1-(3-Fluorobenzyl)azetidine-3-carbonyl)-3,4-dihydro-2H-pyrido[4,3-b][1,4]oxazin-8-yl)bi cyclo[1.1.1]pentane-1-carbonitrile (0133)

3-Cyanobicyclo[1.1.1]pentane-1-carboxylic acid-1,3-dioxoisoindolin-2-yl ester (0.75 mmol, 1.5 equiv), **OI7** (203 mg, 0.50 mmol), Hantzsch ester (253 mg, 1.0 mmol), NiBr₂(dtbbpy) (48.7 mg, 0.01 mmol) and NaHCO₃ (168 mg, 2.0 mmol) were suspended in anhydrous DMA. The reaction mixture was reacted under N₂ with UV irradiation at 390 nm (Kessil^{®} PR160 lamp) for 12 hours. After dilution with 20 mL of diethyl ether, the resulting mixture was extracted with EA and 2.0 N HCl (aq). The organic phases were combined, washed with saturated Na₂CO₃ (aq) and saturated brine, dried over anhydrous sodium sulfate and concentrated under reduced pressure. The crude product was purified by silica gel column chromatography to give **0133** (40 mg, 19 % yield). ¹H-NMR (400 MHz, CDCl₃): *δ* 8.36 (s, 1H), 8.02 (s, 1H), 7.32-7.27 (m, 1H), 7.10-6.99 (m, 3H), 4.46 (t, *J =* 4.8 Hz, 2H), 4.10-3.66 (m, 3H), 3.58 (s, 2H), 3.52-3.45 (m, 2H), 3.37 (t, *J =* 7.0 Hz, 2H), 2.29-2.25 (m, 3H), 2.05-1.98 (m, 3H). HRMS (ESI): m/z calcd. for C₂₄H₂₄FN₄O₂ [M+H]⁺: 419.1878, found: 419.1893.

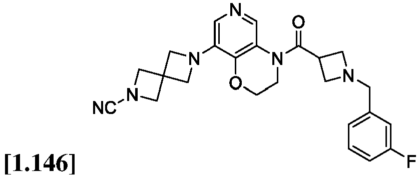

### 6-(4-(1-(3-Fluorobenzyl)azetidine-3-carbonyl)-3,4-dihydro-2H-pyrido[4,3-b][1,4]oxazin-8-yl)-2 ,6-diazaspiro[3.3]heptane-2-carbonitrile (0134)

**OI7** (125 mg, 0.30 mmol), Cs₂CO₃ (343.3 mg, 1.05 mmol), Xantphos (52.1 mg, 0.09 mmol), palladium acetate (7.2 mg, 0.03 mmol), and 2-cyano-2,6-diazabicyclo[3.3]heptane (37 mg, 0.30 mmol) were suspended in 1,4-dioxane (9 mL) under N₂ and stirred at 100°C for 18 hours. The reaction was monitored by TLC until completion. The mixture cooled to room temperature, quenched with water (5 mL) and extracted with ethyl acetate (5 mL × 5). The organic phases were combined, washed with saturated brine (5 mL), dried over anhydrous Na₂SO₄ for 30 minutes, and concentrated under reduced pressure. The crude product was purified by column chromatography with a gradient elution of CH₂Cl₂:MeOH (V/V) = 100:1-10:1 to give **O134** (16 mg, 12% yield). ¹H-NMR (400 MHz, CDCl₃): *δ* 8.17 (s, 1H), 7.92 (s, 1H), 7.29-7.22 (m, 1H), 7.06-6.88 (m, 3H), 4.48 (t, *J* = 4.8 Hz, 2H), 4.08-3.66 (m, 3H), 3.61 (s, 2H), 3.59-3.48 (m, 4H), 3.41 (s, 2H), 3.37 (t, *J* = 7.0 Hz, 2H). HRMS (ESI): m/z calcd. for C₂₄H₂₆FN₆O₂ [M+H]⁺: 449.2096, found: 449.2103.

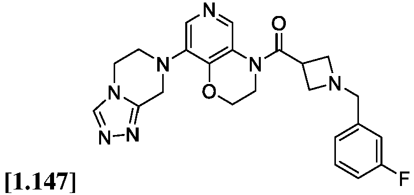

### (8-(5,6-Dihydro-[1,2,4]triazolo[4,3-a]pyrazin-7(8H)-yl)-2,3-dihydro-4H-pyrido[4,3-b][1,4]oxazi n-4-yl)(1-(3-fluorobenzyl)azetidin-3-yl)methanone (0135)

**OI7** (125 mg, 0.30 mmol), Cs₂CO₃ (343.3 mg, 1.05 mmol), Xantphos (52.1 mg, 0.09 mmol), palladium acetate (7.2 mg, 0.03 mmol), and 5,6,7,8-tetrahydro-[1,2,4]triazolo[4,3-a]pyrazine (37 mg, 0.30 mmol) were suspended in 1,4-dioxane (9 mL) under N₂ protection and stirred at 100°C for 18 hours. The reaction was monitored by TLC until completion. The mixture cooled to room temperature, quenched with water (5 mL) and extracted with ethyl acetate (5 mL × 5). The organic phases were combined, washed with saturated brine (5 mL), dried over anhydrous Na₂SO₄ for 30 minutes, and concentrated under reduced pressure. The crude product was purified by column chromatography with a gradient elution of CH₂Cl₂:MeOH (V/V) = 100:1-10:1 to give **0135** (12 mg, 9 % yield). ¹H-NMR (400 MHz, CDCl₃): *δ* 8.36 (s, 1H), 8.15 (s, 1H), 8.01 (s, 1H), 7.26-7.21 (m, 1H), 7.06-6.85 (m, 3H), 4.52 (t, *J =* 4.8 Hz, 2H), 4.32 (s, 2H), 4.25 (t, *J =* 4.6 Hz, 2H), 4.09-3.69 (m, 5H), 3.61 (s, 2H), 3.50-3.38 (m, 2H), 3.32 (t, *J* = 7.0 Hz, 2H). HRMS (ESI): m/z calcd. for C₂₃H₂₅FN₇O₂ [M+H]⁺: 450.2048, found: 450.2039.

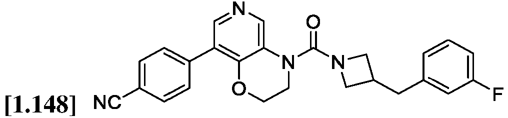

### 4-(4-(3-(3-Fluorobenzyl)azetidine-1-carbonyl)-3,4-dihydro-2H-pyrido[4,3-b][1,4]oxazin-8-yl)b enzonitrile (0136)

To a solution of **OI4** (100 mg, 0.42 mmol) and pyridine (79 mg, 0.63 mmol) in chloroform (10 mL) was slowly added a mixture of triphosgene (623 mg, 2.1 mmol) dissolved in chloroform (3 mL) in an ice bath. The reaction mixture was heated and stirred Under N₂ at 60°C for 12 hours. After the reaction was complete as monitored by TLC, 1 N HCl aq (10 mL) was added dropwise. The resulting mixture was extracted with chloroform, washed successively with saturated Na₂CO₃ aq and saturated brine, dried over anhydrous sodium sulfate and concentrated under reduced pressure to give an oil. The oily intermediate was dissolved in anhydrous 1,4-dioxane (10 mL). Subsequently, triethylamine (0.63 mmol) and 3-(3-fluorobenzyl)azetidine (105 mg, 0.63 mmol) were added. The mixture was stirred at room temperature under N₂ for 12 hours. After the reaction was complete as monitored by TLC, the mixture was concentrated under vacuum. The resulting oil was extracted with chloroform and water. The organic phases were combined, washed successively with saturated Na₂CO₃ aq and brine, dried over anhydrous sodium sulfate, and concentrated under reduced pressure. The crude product was purified by silica gel column chromatography (PE/EA) to obtain compound **0136** (41 mg, 23% yield). ¹H-NMR (400 MHz, CDCl₃): *δ* 8.67 (s, 1H), 8.18 (s, 1H), 7.78 (d, *J =* 8.3 Hz, 2H), 7.59 (d, *J =* 8.1 Hz, 2H), 7.28-7.22 (m, 1H), 7.12-6.99 (m, 2H), 6.95-6.88 (m, 1H), 4.29 (t, *J* =4.8 Hz, 2H), 3.98-3.93 (m, 2H), 3.65-3.59 (m, 4H), 2.59-2.52 (m, 3H). HRMS (ESI): m/z calcd. for C₂₅H₂₂FN₄O₂ [M+H]⁺: 429.1721, found: 429.1729.

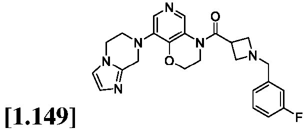

### (8-(5,6-Dihydroimidazo[1,2-a]pyrazin-7(8H)-yl)-2,3-dihydro-4H-pyrido[4,3-b][1,4]oxazin-4-yl) (1-(3-fluorobenzyl)azetidin-3-yl)methanone (0137)

**OI7** (125 mg, 0.30 mmol), Cs₂CO₃ (343.3 mg, 1.05 mmol), Xantphos (52.1 mg, 0.09 mmol), palladium acetate (7.2 mg, 0.03 mmol), and 5,6,7,8-tetrahydro-[1,2,4]imidazo[4,3-a]pyrazine (36 mg, 0.30 mmol) were suspended in 1,4-dioxane (9 mL) under N₂ and stirred at 100°C for 18 hours. The reaction was monitored by TLC until completion. After cooled to room temperature, the mixture was quenched with water (5 mL), and extracted with ethyl acetate (5 mL × 5). The organic phases were combined, washed with saturated brine (5 mL), dried over anhydrous sodium sulfate for 30 minutes and concentrated under reduced pressure. The crude product was purified by column chromatography with a gradient elution of CH₂Cl₂:MeOH (V/V) = 100:1-10:1 to obtain **0137** (12 mg, 9% yield). ¹H-NMR (400 MHz, CDCl₃): *δ* 8.13 (s, 1H), 8.04 (s, 1H), 7.25-7.21 (m, 1H), 7.06-6.92 (m, 3H), 6.97 (d, *J* = 4.2 Hz, 2H), 6.75 (d, *J =* 4.2 Hz, 2H), 4.55 (t, *J =* 4.8 Hz, 2H), 4.32 (s, 2H), 4.28 (t, *J =* 4.6 Hz, 2H), 4.07-3.89 (m, 5H), 3.61 (s, 2H), 3.52-3.39 (m, 2H), 3.30 (t, *J* = 7.0 Hz, 2H). HRMS (ESI): m/z calcd. for C₂₄H₂₆FN₆O₂ [M+H]⁺: 449.2096, found: 449.2110.

### Example 2:

Synthetic procedures and structural characterizations of provided compounds of formulae (**V**)-(**IX**) and intermediates are described:
**General method SA:** To a suspension of the corresponding amine (0.30 mmol) and potassium carbonate (0.59 mmol) in dry DMF, was added benzyl chloride (0.45 mmol) or benzyl bromide (0.45 mmol). The resulting mixture was stirred at room temperature for 2 h. After the reaction was complete as monitored by TLC, the reaction mixture was diluted with water (10 mL) and extracted with ethyl acetate (3×10 mL). The combined organic layers were washed with brine and dried over anhydrous Na₂SO₄. The solvent was removed under reduced pressure to give the crude product, which was subsequently purified by silica gel column chromatography (dichloromethane/methanol) to yield the target compound.
**General method SB:** To a solution of the corresponding amine (1 mmol), DMAP (0.3 mmol), and triethylamine (3 mmol) in anhydrous dichloromethane (5 mL) was added dropwise the corresponding acyl chloride (1.5 mmol) in an ice bath. Afterward, the reaction mixture was warmed to room temperature and stirred under N₂ for 12 hours. When the reaction was complete as monitored by TLC, the reaction mixture was diluted with water (6 mL) and extracted with DCM (3×6 mL). The combined organic layers were washed with brine and dried over anhydrous Na₂SO₄. The solvent was removed under reduced pressure to give the crude product, which was subsequently purified by silica gel column chromatography (PE/EA) to yield the target compound.
**General method SC:** Dissolved the corresponding amine (1 mmol) and the corresponding sulfonyl chloride (2 mmol) in pyridine (4 mL). The reaction system was heated to 120 °C under N₂, and stirred for 12 hours. After the reaction was complete as monitored by TLC, the reaction mixture was cooled to room temperature, diluted with water (6 mL), and extracted with ethyl acetate (3×12 mL). The combined organic phase was washed with saturated CuSO₄ solution (5 mL) and brine, and dried over anhydrous Na₂SO₄. The solvent was removed under reduced pressure to give the crude product, which was subsequently purified by silica gel column chromatography (PE/EA) to yield the target compound.
**General method SD:** The corresponding bromide (1 mmol), potassium carbonate (4 mmol), and the corresponding boronic acid (1.5 mmol) were added to a mixture of 1,4-dioxane (3 mL) and water (1 mL). To this mixture were added PdCl₂(PPh₃)₂ (0.1 mmol) and tricyclohexylphosphine (0.2 mmol) under nitrogen atmosphere. The reaction mixture was subsequently heated to 90 °C and stirred for 24 hours. After the reaction was complete as monitored by TLC, it was cooled to room temperature, diluted with water (5 mL), and extracted with ethyl acetate (3×5 mL). The combined organic layers were washed with brine (5 mL) and dried over anhydrous Na₂SO₄. The solvent was removed under reduced pressure to give the crude product, which was subsequently purified by silica gel column chromatography (PE/EA) to yield the target compound.
**General method SE:** To a solution of the corresponding amine (0.2 mmol), DMAP (7.2 mg, 0.06 mmol), and triethylamine (0.6 mmol) in anhydrous dichloromethane (5 mL) was added dropwise the corresponding sulfonyl chloride (0.3 mmol) at 0°C. The mixture was then warmed to room temperature and stirred for 8 hours. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.
**General method SF:** To a solution of the corresponding amine (1 mmol) and triethylamine (1.1 mmol) in anhydrous dichloromethane (5 mL), was added slowly the corresponding anhydride (1.1 mmol) were at 0°C. The resulting mixture was warmed to room temperature and stirred overnight. After the reaction was complete as monitored by TLC, the mixture was diluted with water (6 mL) and extracted with dichloromethane (2×6 mL). The combined organic layers were washed with brine and dried over anhydrous Na₂SO₄. The solvent was removed under reduced pressure to give the crude product, which was subsequently purified by silica gel column chromatography (PE/EA) to yield the target compound.
**General method SG:** To a solution of the corresponding amine (1 mmol), carboxylic acid (1.1 mmol), and N,N-diisopropylethylamine (3 mmol) in anhydrous dichloromethane (5 mL), was added HATU (1.05 mmol) at 0 °C. The reaction mixture was warmed to room temperature and stirred overnight. After the reaction was complete as monitored by TLC, the mixture was diluted with water (6 mL) and extracted with dichloromethane (2×6 mL). The combined organic layers were washed with brine and dried over anhydrous Na₂SO₄. The solvent was removed under reduced pressure to give the crude product, which was subsequently purified by silica gel column chromatography (PE/EA) to yield the target compound.
**General method SH:** To a suspension of the corresponding amine (0.30 mmol) and cesium carbonate (0.89 mmol) in toluene (5 mL) was added the corresponding bromide (0.59 mmol). The mixture was purged with N₂ for three times, and stirred for 5 min before Xantphos (0.02 mmol) and Pd₂(dba)₃ (0.02 mmol) were added. The resulting mixture was heated to 90 °C and stirred for 3 h. After the reaction was complete as monitored by TLC, the reaction mixture was diluted with water (10 mL) and extracted with ethyl acetate (3×10 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography (PE/EA) to yield the target compound.
**General method SI:** To a suspension of the corresponding bromide (0.30 mmol), Pd(OAc)₂ (0.015 mmol), dppf (0.03 mmol) and cesium carbonate (0.45 mmol) in anhydrous DMF (5 mL) was added the corresponding boronic acid (0.36 mmol). The reaction mixture was stirred at 100 °C for 4 h under N₂. After the reaction was complete as monitored by TLC, the reaction mixture was diluted with water (10 mL) and extracted with ethyl acetate (3×10 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography (PE/EA) to yield the target compound.
**General method SJ:** To a solution of the corresponding amine (0.20 mmol) and NaOH (0.40 mmol) in anhydrous dichloromethane (5 mL) was added tetrabutylammonium hydrogen sulfate (TBAHS, 0.01 mmol). The resulting mixture was stirred at 0 °C for 5 min before the corresponding sulfonyl chloride (0.30 mmol) was added dropwise at 0 °C. The mixture was subsequently heated to 40 °C and stirred for further 12 hours. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.

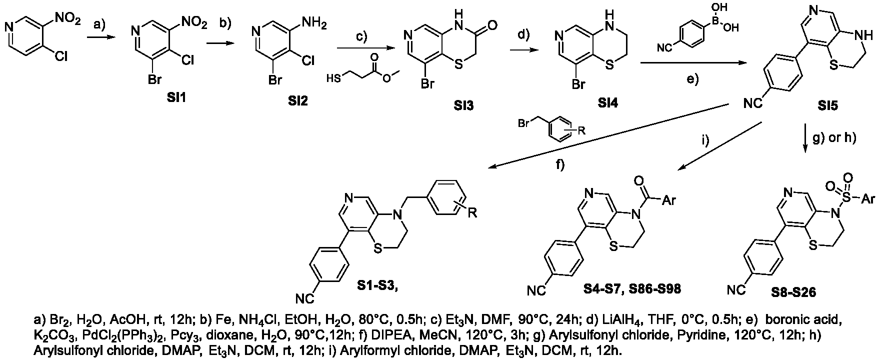

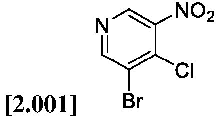

### 3-Bromo-4-chloro-5-nitropyridine (SI1)

To a solution of 4-chloro-3-nitropyridine (100 mg, 0.63 mmol) in a mixture of AcOH and water (V/V 1:1, 8.0 mL) was added Br₂ dropwise at 0 °C. After being stirred for 0.5 h at 0 °C, the reaction mixture was warmed to room temperature and continued stirring for 12 h. The resulting participate was collected, washed with water and dried at 60 °C to give **SI1** as a white solid (97.2 mg, yield 65.0%), which was used directly in the next step without further purification and characterization.

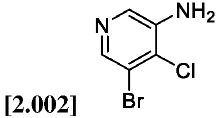

### 5-Bromo-4-chloropyridin-3-amine (SI2)

To a solution of **SI1** (2.002 g, 8.4 mmol) in a mixture of ethanol (24.0 mL) and water (8.0 mL) was added Fe powder (2.815 g, 50.4 mmol) and NH₄Cl (898.5 mg, 16.8 mmol). The resulting mixture was stirred at 80 °C under N₂ for 05 h. After the reaction was complete as monitored by TLC, the mixture was cooled to room temperature and filtered through Celite. The filtrate was diluted with water (50 mL) and extracted with dichloromethane (50 mL) for three times. The combined organic layers were washed with brine (50 mL) and dried over anhydrous Na₂SO₄. The solvent was removed under reduced pressure to give the crude product, which was subsequently purified by silica gel column chromatography (PE/EA) to yield compound **SI2** as alight yellow solid (1.640 g, 94.0% yield). The crude product was used directly in the next step without further purification and characterization.

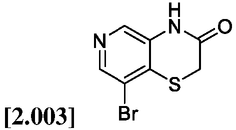

### 8-Bromo-2H-pyrido[4,3-b][1,4]thiazin-3(4H)-one (SI3)

To a solution of SI2 (1.003 g, 4.82 mmol) and Et₃N (0.67 mL, 4.82 mmol) in DMF (15.0 mL) was added methyl 2-mercaptoacetate (0.43 mL, 4.821 mmol) dropwise under N₂. The resulting mixture was subsequently heated to 90 °C and stirred for 24 h. After the reaction was complete as monitored by TLC, the reaction mixture was diluted with water (15 mL) and extracted with ethyl acetate (3×10 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography with an elution of CH₂Cl₂/MeOH (V/V, 30:1) to give **SI3** (682.4 mg, 57.8% yield) as a yellow solid. ¹H-NMR (400 MHz, DMSO-*d6*) δ 10.99 (s, 1H), 8.30 (s, 1H), 8.10 (s, 1H), 3.68 (s, 2H).

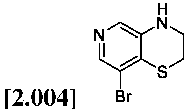

### 8-Bromo-3,4-dihydro-2H-pyrido[4,3-b][1,4]thiazine (SI4)

To a solution of **SI3** (100.0 mg, 0.4 mmol) in anhydrous THF (12.0 mL) was added LiAlH₄ (52.6 mg, 1.4 mmol) in batch at 0 °C. The resulting mixture was stirred at 0 °C for 0.5 h before being warmed to room temperature and stirred overnight. After the reaction was complete as monitored by TLC, it was cooled to 0 °C, quenched with water (10 mL), and filtered through Celite. The filtrate was extracted with ethyl acetate (3×10 mL). The combined organic layers were washed with brine (5 mL) and dried over anhydrous sodium sulfate. It was then concentrated under reduced pressure to give the crude product, which was subsequently purified by column chromatography with an elution of CH₂Cl₂/MeOH (V/V, 30:1) to give **SI4** (56.0mg, yield 59.4%) as a white solid. ¹H-NMR (400 MHz, CDCl₃) δ 7.94 (s, 1H), 7.69 (s, 1H), 4.26 (s, 1H), 3.65-3.57 (m, 2H), 3.19-3.09 (m, 2H).

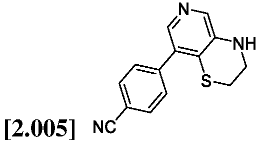

### 4-(3,4-Dihydro-2H-pyrido[4,3-b][1,4]thiazin-8-yl)benzonitrile (SI5)

Following the general method SD, synthesized with **SI4** (50.1 mg, 0.2 mmol), (4-cyanophenyl)boronic acid (47.6 mg, 0.32 mmol), PdCl₂(PPh₃)₂ (15.2 mg, 0.02 mmol), Pcy₃ (12.1 mg, 0.04 mmol) and K₂CO₃ (119.4 mg, 0.86 mmol) in a mixture of dioxane (3.0 mL) and water (1.0 mL) to give **SI5** (102 mg, 90.3% yield) as a light yellow solid after chromatographic purification (PE/EA = 1/2). ¹H-NMR (400 MHz, CDCl₃) δ 7.89 (s, 1H), 7.73 (d, *J* = 8.4 Hz, 2H), 7.68 (s, 1H), 7.53 (d, *J =* 8.4 Hz, 2H), 4.61 (s, 1H), 3.72-3.65 (m, 2H), 3.06-3.00 (m, 2H).

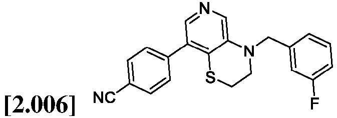

### 4-(4-(3-Fluorobenzyl)-3,4-dihydro-2H-pyrido[4,3-b][1,4]thiazin-8-yl)benzonitrile (S1)

Following the general method SA, synthesized with **SI5** (30.3 mg, 0.12 mmol), 1-(bromomethyl)-3-fluorobenzene (45.4 mg, 0.24 mmol) and N,N-diisopropylethylamine (0.06 mL, 0.36 mmol) in acetonitrile (3.0 mL) to give **S1** (77.2 mg, 90.1% yield) as a yellow solid after chromatographic purification (DCM/MeOH = 20/1). mp: 83.0-84.0 °C. ¹H-NMR (400 MHz, DMSO-*d₆*) δ 8.32 (d, *J =* 1.2 Hz, 1H), 8.11-8.05 (m, 2H), 8.02 (s, 1H), 7.76-7.70 (m, 2H), 7.66 (s, 1H), 7.51 (td, *J =* 8.0, 6.1 Hz, 1H), 7.44 (d, *J =* 9.6 Hz, 1H), 7.38 (d, *J =* 7.7 Hz, 1H), 7.28 (td, *J =* 8.3, 2.2 Hz, 1H), 5.60 (s, 2H), 3.62-3.54 (m, 2H), 3.16-3.09 (m, 2H). ¹³C-NMR (100 MHz, DMSO-*d₆*) δ 162.19 (¹*J*_{CF} = 245.1 Hz), 142.03, 138.12, 136.97 (³*J*_{CF} = 7.8 Hz), 135.44, 135.41, 132.83 (2C), 131.28 (³*J*_{CF} = 8.4 Hz), 130.27 (2C), 129.73, 124.93 (⁴*J*_{CF} = 2.8 Hz), 124.80, 118.31, 116.14 (²*J*_{CF} = 20.8 Hz), 115.76 (²*J*_{CF} = 22.1 Hz), 112.36, 61.41, 54.92, 24.62. HRMS (ESI): m/z calcd. for C₂₁H₁₆FN₃S [M+H]⁺: 362.1122, found: 362.1122.

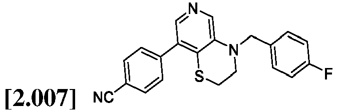

### 4-(4-(4-Fluorobenzyl)-3,4-dihydro-2H-pyrido[4,3-b][1,4]thiazin-8-yl)benzonitrile (S2)

Following the general method SA, synthesized with **SI5** (30.3 mg, 0.12 mmol), 1-(bromomethyl)-4-fluorobenzene (45.4 mg, 0.24 mmol) and N,N-diisopropylethylamine (0.06 mL, 0.36 mmol) in acetonitrile (3.0 mL) to give S2 (32.5 mg, 74.9% yield) as a yellow solid after chromatographic purification (DCM/MeOH = 20/1). mp: 203.0-204.0 °C. ¹H-NMR (400 MHz, DMSO-*d₆*) δ 8.32 (d, *J =* 1.3 Hz, 1H), 8.06 (d, *J =* 8.5 Hz, 2H), 8.00 (d, *J* = 1.4 Hz, 1H), 7.71 (d, *J =* 8.5 Hz, 2H), 7.67 (s, 1H), 7.62 (dd, *J =* 8.7, 5.4 Hz, 2H), 7.30 (t, *J =* 8.9 Hz, 2H), 5.58 (s, 2H), 3.58 (d, *J =* 2.8 Hz, 2H), 3.16-3.06 (m, 2H). ¹³C-NMR (100 MHz, DMSO-*d₆*) δ 160.08 (¹*J*_{CF} = 246.0 Hz), 142.01, 138.12, 135.41, 135.26, 132.84 (2C), 131.29 (³*J*_{CF} = 8.8 Hz, 2C), 130.78 (⁴*J*_{CF} = 3.0 Hz), 130.25 (2C), 129.55, 124.69, 118.31, 116.06 (²*J*_{CF} = 21.8 Hz, 2C), 112.38, 61.35, 33,78, 24.61. HRMS (ESI): m/z calcd. for C₂₁H₁₆FN₃S [M+H]⁺: 362.1122, found: 362.1119.

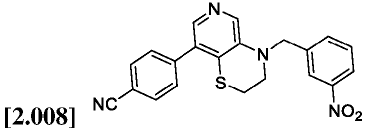

### 4-(4-(3-Nitrobenzyl)-3,4-dihydro-2H-pyrido[4,3-b][1,4]thiazin-8-yl)benzonitrile (S3)

Following the general method SA, synthesized with **SI5** (200.0 mg, 0.8 mmol), 1-(bromomethyl)-3-nitrobenzene (345.7 mg, 1.6 mmol) and N,N-diisopropylethylamine (0.4 mL, 2.4 mmol) in acetonitrile (21.0 mL) to give S3 (291.1 mg, 94.8% yield) as a yellow solid after chromatographic purification (DCM/MeOH = 20/1). mp: 239.0-240.0 °C. ¹H-NMR (400 MHz, CDCl₃) δ 9.67 (s, 1H), 8.30 (d, *J =* 6.6 Hz, 2H), 8.16 (d, *J =* 7.7 Hz, 1H), 7.81 (d, *J =* 8.2 Hz, 2H), 7.72 (t, *J* = 7.6 Hz, 1H), 7.53 (d, *J* = 8.3 Hz, 2H), 7.46 (s, 1H), 5.67 (s, 2H), 3.85-3.75 (m, 2H), 3.09-3.03 (m, 2H). ¹³C-NMR (100 MHz, DMSO-*d₆*) δ 147.99, 142.05, 138.08, 136.28, 135.61, 135.52, 135.43, 132.83 (2C), 130.78, 130.25 (2C), 129.74, 124.79, 124.15, 124.09, 118.30, 112.36, 60.91, 45.68, 24.63. HRMS (ESI): m/z calcd. for C₂₁H₁₆N₄O₂S [M+H]⁺: 389.1067, found: 389.1054.

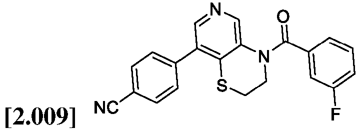

### 4-(4-(3-Fluorobenzoyl)-3,4-dihydro-2H-pyrido[4,3-b][1,4]thiazin-8-yl)benzonitrile (S4)

Following the general method SB, synthesized with **SI5** (100.0 mg, 0.4 mmol), 3-fluorobenzoyl chloride (0.07 mL, 0.60 mmol), DMAP (14.5 mg, 0.12 mmol), Et₃N (0.16 mL, 1.2 mmol) in anhydrous dichloromethane (6 mL) to give **S4** (101.1 mg, 68.2% yield) as a white solid after chromatographic purification (PE/EA = 20/1). mp: 178.0-179.0 °C. ¹H-NMR (400 MHz, DMSO-*d₆*) δ 8.04 (s, 1H), 7.99 (d, *J =* 8.3 Hz, 3H), 7.70 (d, *J =* 8.3 Hz, 2H), 7.44 (dd, *J =* 15.0, 7.3 Hz, 1H), 7.31 (dd, *J* = 8.7, 5.6 Hz, 2H), 7.18 (d, *J* = 7.9 Hz, 1H), 4.06 (s, 2H), 3.33 (s, 2H). ¹³C-NMR (100 MHz, DMSO-*d₆*) δ 167.43, 161.66 (¹*J*_{CF} = 245.1 Hz), 145.80, 144.10, 140.35, 137.41, 137.34, 132.57 (2C), 130.70, 130.61, 130.40 (2C), 124.17, 124.15, 118.53, 117.40 (²*J*_{CF} *=* 21.0 Hz), 115.20 (²*J*_{CF} = 23.2 Hz), 111.40, 41.35, 28.69. HRMS (ESI): m/z calcd. for C₂₁H₁₄FN₃OS [M+H]⁺: 376.0914, found: 376.0920.

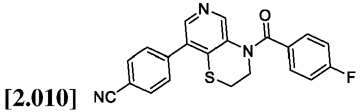

### 4-(4-(4-Fluorobenzoyl)-3,4-dihydro-2H-pyrido[4,3-b][1,4]thiazin-8-yl)benzonitrile (S5)

Following the general method SB, synthesized with **SI5** (100.0 mg, 0.4 mmol), 4-fluorobenzoyl chloride (0.07 mL, 0.60 mmol), DMAP (14.5 mg, 0.12 mmol), Et₃N (0.16 mL, 1.2 mmol) in anhydrous dichloromethane (6 mL) to give S5 (119.1 mg, 80.3% yield) as a white solid after chromatographic purification (PE/EA = 1/1). mp: 174.0-175.0 °C. ¹H-NMR (400 MHz, CDCl₃) δ 8.04 (s, 1H), 7.79 (d, *J* = 8.4 Hz, 3H), 7.59 (d, *J* = 8.3 Hz, 2H), 7.40 (dd, J = 8.7, 5.2 Hz, 2H), 7.04 (t, *J* = 8.6 Hz, 2H), 4.23 (s, 2H), 3.30 (t, *J* = 5.5 Hz, 2H). ¹³C-NMR (100 MHz, DMSO-*d₆*) δ 168.24, 163.45 (¹*J*_{CF} = 248.5 Hz), 146.73, 144.86, 140.97, 137.21, 134.54, 133.20, 133.02 (2C), 131.99 (⁴*J*_{CF} = 3.3 Hz), 131.50 (³*J*_{CF} = 9.0 Hz, 2C), 130.88 (2C), 119.02, 115.92 (²*J*_{CF} = 21.9 Hz, 2C), 111.81, 41.76, 29.33. HRMS (ESI): m/z calcd. for C₂₁H₁₄FN₃OS [M+H]⁺: 376.0914, found: 376.0911.

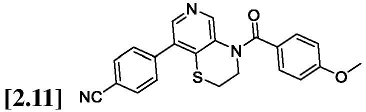

### 4-(4-(4-Methoxybenzoyl)-3,4-dihydro-2H-pyrido[4,3-b][1,4]thiazin-8-yl)benzonitrile (S6)

Following the general method SB, synthesized with **SI5** (100.0 mg, 0.4 mmol), 4-methoxybenzoyl chloride (0.08 mL, 0.60 mmol), DMAP (14.5 mg, 0.12 mmol), Et₃N (0.16 mL, 1.2 mmol) in anhydrous dichloromethane (6 mL) to give **S6** (115.3 mg, 74.4% yield) as a white solid after chromatographic purification (PE/EA = 1/1). mp: 187.0-188.0 °C. ¹H-NMR (400 MHz, CDCl₃) δ 8.01 (s, 1H), 7.82 (s, 1H), 7.79 (d, *J =* 8.2 Hz, 2H), 7.59 (d, *J =* 8.2 Hz, 2H), 7.35 (d, *J =* 8.7 Hz, 2H), 6.83 (d, *J =* 8.7 Hz, 2H), 4.23 (s, 2H), 3.81 (s, 3H), 3.29 (t, *J =* 5.4 Hz, 2H). ¹³C-NMR (100 MHz, DMSO-*d6*) δ 168.36, 160.92, 146.15, 144.04, 140.57, 136.31, 134.66, 132.71 (2C), 132.53, 131.33, 130.55 (2C), 130.40 (2C), 126.86, 118.55, 113.80, 113.66 (2C), 111.30, 55.27, 41.17, 29.01. HRMS (ESI): m/z calcd. for C₂₂H₁₇N₃O₂S [M+H]⁺: 378.1114, found: 388.1115.

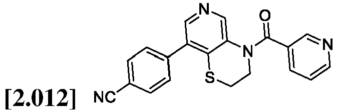

### 4-(4-Nicotinoyl-3,4-dihydro-2H-pyrido[4,3-b][1,4]thiazin-8-yl)benzonitrile (S7)

Following the general method SB, synthesized with **SI5** (100.0 mg, 0.4 mmol), nicotinoyl chloride (105.5 mg, 0.60 mmol), DMAP (14.5 mg, 0.12 mmol), Et₃N (0.16 mL, 1.2 mmol) in anhydrous dichloromethane (6 mL) to give **S7** (98.4 mg, 69.5% yield) as a white solid after chromatographic purification (DCM/MeOH = 20/1). mp: 218.0-219.0 °C. ¹H-NMR (400 MHz, CDCl₃) δ 8.68-8.61 (m, 1H), 8.52 (s, 1H), 8.05 (s, 1H), 7.85-7.76 (m, 4H), 7.59 (d, *J =* 8.3 Hz, 2H), 7.35 (dd, *J* = 7.8, 4.9 Hz, 1H), 4.32-4.17 (m, 2H), 3.33 (t, *J* = 5.6 Hz, 2H). ¹³C-NMR (100 MHz, DMSO-*d₆*) δ 166.71, 151.05, 148.85, 146.40, 144.69, 140.45, 137.19, 136.18, 133.62, 132.79, 132.58 (2C), 131.10, 130.44 (2C), 123.45, 118.58, 111.38, 59.78, 28.79. HRMS (ESI): m/z calcd. for C₂₀H₁₄N₄OS [M+H]⁺: 359.0961, found: 359.1057.

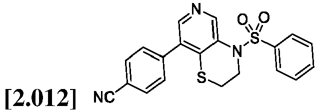

### 4-(4-(Phenylsulfonyl)-3,4-dihydro-2H-pyrido[4,3-b][1,4]thiazin-8-yl)benzonitrile (S8)

Following the general method SC synthesized with **SI5** (150.0 mg, 0.6 mmol), benzenesulfonyl chloride (0.15 mL, 0.12 mmol) in pyridine (6 mL) to give **S8** (132.4 mg, 57.1% yield) as a white solid after chromatographic purification (DCM/MeOH = 20/1). mp: 134.0-135.0 °C. ¹H-NMR (400 MHz, DMSO-*d₆*) δ 8.58 (s, 1H), 8.16 (s, 1H), 7.99-7.92 (m, 2H), 7.81-7.73 (m, 1H), 7.69 (t, *J =* 4.3 Hz, 2H), 7.66-7.60 (m, 2H), 7.58-7.51 (m, 2H), 3.92 (dd, *J =* 7.2, 3.9 Hz, 2H), 2.94-2.77 (m, 2H). ¹³C-NMR (100 MHz, DMSO-*d₆*) δ 146.83, 145.37, 140.20, 138.84, 137.73, 134.02, 132.73, 132.56 (2C), 130.44, 130.31 (2C), 129.88 (2C), 127.07 (2C), 118.48, 111.39, 42.86, 25.30. HRMS (ESI): m/z calcd. for C₂₀H₁₅N₃O₂S₂ [M+H]⁺: 394.0678, found: 394.0673.

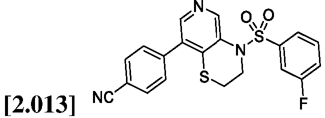

### 4-(4-((3-Fluorophenyl)sulfonyl)-3,4-dihydro-2H-pyrido[4,3-b][1,4]thiazin-8-yl)benzonitrile (S9)

Following the general method SC synthesized with **SI5** (100.0 mg, 0.4 mmol), 3-fluorobenzenesulfonyl chloride (153.7 mg, 0.8 mmol) in pyridine (4 mL) to give **S9** (132.4 mg, 57.1% yield) as a white solid after chromatographic purification (PE/EA = 1/1). mp: 138.0-139.0 °C. ¹H-NMR (400 MHz, CDCl₃) δ 8.72 (s, 1H), 8.16 (s, 1H), 7.74 (d, *J =* 8.2 Hz, 2H), 7.52 (d, *J* = 1.5 Hz, 2H), 7.44 (d, *J =* 8.2 Hz, 2H), 7.39 (d, *J =* 7.6 Hz, 1H), 7.35 (s, 1H), 4.02-3.95 (m, 2H), 2.96-2.89 (m, 2H). ¹³C-NMR (100 MHz, CDCl₃) δ 162.65 (¹*J*_{CF} = 253.0 Hz), 147.17, 145.68, 141.68 (*J* = 6.7 Hz), 140.18, 132.61 (2C), 131.52 (*J* = 7.8 Hz), 130.29 (2C), 123.20 (*J* = 3.4 Hz), 121.22, 121.01, 118.45, 114.93, 114.69, 112.91, 43.52, 26.32. HRMS (ESI): m/z calcd. for C₂₀H₁₅N₃O₂S₂ [M+H]⁺: 412.0584, found: 412.0577.

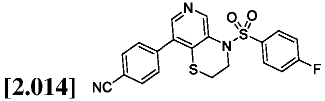

### 4-(4-((4-Fluorophenyl)sulfonyl)-3,4-dihydro-2H-pyrido[4,3-b][1,4]thiazin-8-yl)benzonitrile (S10)

Following the general method SC synthesized with **SI5** (100.0 mg, 0.4 mmol), 4-fluorobenzenesulfonyl chloride (153.7 mg, 0.8 mmol) in pyridine (4 mL) to give **S10** (133.0 mg, 57.2% yield) as a white solid after chromatographic purification (PE/EA = 2/1). mp: 69.0-70.0 °C. ¹H-NMR (400 MHz, CDCl₃) δ 8.71 (s, 1H), 8.14 (s, 1H), 7.75-7.71 (m, 4H), 7.44 (d, *J* = 8.2 Hz, 2H), 7.19 (t, *J* = 8.5 Hz, 2H), 4.02-3.91 (m, 2H), 2.97-2.86 (m, 2H). ¹³C-NMR (100 MHz, DMSO-*d₆*) δ 165.02 (¹*J*_{CF} = 253.5 Hz), 146.91, 145.45, 140.15, 138.03, 135.20 (⁴*J*_{CF} = 3.0 Hz), 132.82, 132.58 (2C), 130.35 (³*J*_{CF} = 9.4 Hz, 2C), 130.33 (3C), 118.48, 117.12 (²*J*_{CF} = 22.85 Hz, 2C), 111.42, 42.92, 25.49. HRMS (ESI): m/z calcd. for C₂₀H₁₅N₃O₂S₂ [M+H]⁺: 412.0584, found: 412.0572.

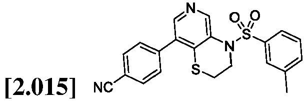

### 4-(4-(m-Tolylsulfonyl)-3,4-dihydro-2H-pyrido[4,3-b][1,4]thiazin-8-yl)benzonitrile (S11)

Following the general method SC synthesized with **SI5** (100.0 mg, 0.4 mmol), 3-methylbenzenesulfonyl chloride (150.6 mg, 0.8 mmol) in pyridine (4 mL) to give **S11** (93.1 mg, 57.1% yield) as a white solid after chromatographic purification (PE/EA = 2/1). mp: 174.0-175.0 °C. ¹H-NMR (400 MHz, CDCl₃) δ 8.72 (s, 1H), 8.14 (s, 1H), 7.73 (d, *J =* 8.2 Hz, 2H), 7.52 (d, *J* = 8.8 Hz, 2H), 7.45 (d, *J* = 8.1 Hz, 3H), 7.40 (t, *J* = 7.5 Hz, 1H), 4.00-3.90 (m, 2H), 2.95-2.84 (m, 2H), 2.40 (s, 3H). ¹³C-NMR (100 MHz, DMSO-*d₆*) δ 146.87, 145.36, 140.28, 139.75, 138.84, 137.56, 135.69, 134.58, 132.59 (2C), 130.28 (2C), 129.70, 127.13, 124.22, 118.48, 111.39, 42.81, 25.26, 20.73. HRMS (ESI): m/z calcd. for C₂₁H₁₇N₃O₂S₂ [M+H]⁺: 408.0835, found: 408.0826.

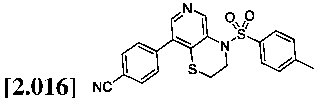

### 4-(4-Tosyl-3,4-dihydro-2H-pyrido[4,3-b][1,4]thiazin-8-yl)benzonitrile (S12)

Following the general method SC synthesized with **SI5** (100.0 mg, 0.4 mmol), 4-methylbenzenesulfonyl chloride (150.6 mg, 0.8 mmol) in pyridine (4 mL) to give **S12** (91.1 mg, 55.8% yield) as a white solid after chromatographic purification (PE/EA = 2/1). mp: 141.0-142.0 °C. ¹H-NMR (400 MHz, DMSO-*d₆*) δ 8.56 (s, 1H), 8.14 (s, 1H), 7.95 (d, *J* = 8.1 Hz, 2H), 7.57 (dd, *J* = 12.3, 8.2 Hz, 4H), 7.43 (d, *J* = 8.2 Hz, 2H), 3.94-3.87 (m, 2H), 2.87-2.81 (m, 2H), 2.41 (s, 3H). ¹³C-NMR (100 MHz, DMSO-*d₆*) δ 146.82, 145.27, 144.59, 140.25, 137.47, 136.05, 132.68, 132.57 (2C), 130.50, 130.32 (2C), 130.28 (2C), 127.15 (2C), 118.49, 111.38, 42.73, 25.25, 21.08. HRMS (ESI): m/z calcd. for C₂₁H₁₇N₃O₂S₂ [M+H]⁺: 408.0835, found: 408.0827.

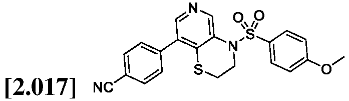

### 4-(4-((4-Methoxyphenyl)sulfonyl)-3,4-dihydro-2H-pyrido[4,3-b][1,4]thiazin-8-yl)benzonitril e (S13)

Following the general method SC synthesized with **SI5** (100.0 mg, 0.4 mmol), 4-methoxybenzenesulfonyl chloride (163.3 mg, 0.8 mmol) in pyridine (4 mL) to give **S13** (101.9 mg, 60.1% yield) as a white solid after chromatographic purification (PE/EA = 2/1). mp: 60.0-61.0 °C. ¹H-NMR (400 MHz, CDCl₃) δ 8.71 (s, 1H), 8.12 (s, 1H), 7.74 (d, *J* = 7.9 Hz, 2H), 7.65 (d, *J =* 8.7 Hz, 2H), 7.46 (d, *J =* 8.0 Hz, 2H), 6.97 (d, *J =* 8.7 Hz, 2H), 3.98-3.91 (m, 2H), 3.89 (s, 3H), 2.98-2.87 (m, 2H). ¹³C-NMR (100 MHz, DMSO-*d₆*) δ 163.32, 146.92, 145.17, 140.25, 132.70, 132.57 (2C), 130.54, 130.34 (2C), 130.19, 129.43 (2C), 118.49, 115.11, 114.99 (2C), 111.38, 55.88, 42.65, 25.12. HRMS (ESI): m/z calcd. for C₂₁H₁₇N₃O₂S₂ [M+H]⁺: 427.0784, found: 427.0782.

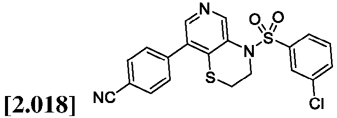

### 4-(4-((3-Chlorophenyl)sulfonyl)-3,4-dihydro-2H-pyrido[4,3-b][1,4]thiazin-8-yl)benzonitrile (S14)

Following the general method SC synthesized with **SI5** (100.0 mg, 0.4 mmol), 3-chlorobenzenesulfonyl chloride (166.7 mg, 0.8 mmol) in pyridine (4 mL) to give **S14** (98.3 mg, 57.4% yield) as a white solid after chromatographic purification (PE/EA = 2/1). mp: 126.0-127.0 °C. ¹H-NMR (400 MHz, CDCl₃) δ 8.72 (s, 1H), 8.18 (s, 1H), 7.74 (d, *J =* 8.2 Hz, 2H), 7.67-7.57 (m, 3H), 7.52-7.40 (m, 3H), 4.04-3.90 (m, 2H), 2.98-2.86 (m, 2H). ¹³C-NMR (100 MHz, DMSO-*d₆*) δ 147.01, 145.72, 140.63, 140.10, 138.18, 134.35, 133.97, 132.83, 132.61 (2C), 131.97, 130.67, 130.26 (2C), 126.57, 125.79, 118.46, 111.45, 43.13, 25.66. HRMS (ESI): m/z calcd. for C₂₀H₁₄ClN₃O₂S₂ [M+H]⁺: 428.0289, found: 428.0284.

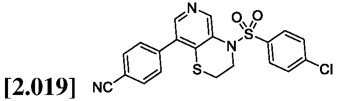

### 4-(4-((4-Chlorophenyl)sulfonyl)-3,4-dihydro-2H-pyrido[4,3-b][1,4]thiazin-8-yl)benzonitrile (S15)

Following the general method SC synthesized with **SI5** (100.0 mg, 0.4 mmol), 4-chlorobenzenesulfonyl chloride (166.7 mg, 0.8 mmol) in pyridine (4 mL) to give **S15** (94.5 mg, 55.9% yield) as a light yellow solid after chromatographic purification (PE/EA = 2/1). mp: 171.0-172.0 °C. ¹H-NMR (400 MHz, CDCl₃) δ 8.71 (s, 1H), 8.15 (s, 1H), 7.75 (d, *J =* 8.2 Hz, 2H), 7.66 (d, *J* = 8.6 Hz, 2H), 7.50 (d, *J* = 8.6 Hz, 2H), 7.45 (d, *J* = 8.2 Hz, 2H), 4.01-3.94 (m, 2H), 2.97-2.91 (m, 2H). ¹³C-NMR (100 MHz, DMSO-*d₆*) δ 146.90, 145.57, 140.17, 138.98, 137.91 137.78, 132.58 (2C), 132.09, 130.33 (2C), 130.01 (2C), 129.08 (2C), 118.48, 111.42 (s), 42.97, 25.59. HRMS (ESI): m/z calcd. for C₂₀H₁₄ClN₃O₂S₂ [M+H]⁺: 428.0289, found: 428.0282.

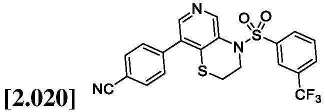

### 4-(4-((3-(Trifluoromethyl)phenyl)sulfonyl)-3,4-dihydro-2H-pyrido[4,3-b][1,4]thiazin-8-yl)be nzonitrile (S16)

Following the general method SC synthesized with **SI5** (100.0 mg, 0.4 mmol), 3-(trifluoromethyl)benzenesulfonyl chloride (193.3 mg, 0.8 mmol) in pyridine (4 mL) to give **S16** (101.6 mg, 55.0% yield) as a white solid after chromatographic purification (PE/EA = 2/1). mp: 100.0-101.0 °C. ¹H-NMR (400 MHz, CDCl₃) δ 8.72 (s, 1H), 8.16 (s, 1H), 7.94 (d, *J* = 7.9 Hz, 1H), 7.89 (d, *J =* 7.9 Hz, 1H), 7.81 (s, 1H), 7.71 (d, *J =* 8.4 Hz, 2H), 7.67 (d, *J* = 7.9 Hz, 1H), 7.40 (d, *J =* 8.4 Hz, 2H), 4.04-3.94 (m, 2H), 2.95-2.83 (m, 2H). ¹³C-NMR (100 MHz, CDCl₃) δ 147.67, 146.23, 140.91, 140.10, 138.16, 132.52 (2C), 132.23 (²*J*_{CF} = 33.5 Hz), 131.91 (¹*J*_{CF} = 266.9 Hz), 130.44, 130.41, 130.26 (³*J*_{CF} = 7.0 Hz), 130.17 (2C), 124.40 (³*J*_{CF} = 9.9 Hz), 124.34, 121.64, 118.40, 112.76, 43.62, 26.21. HRMS (ESI): m/z calcd. for C₂₁H₁₄F₃N₃O₂S₂ [M+H]⁺: 462.0552, found: 462.0537.

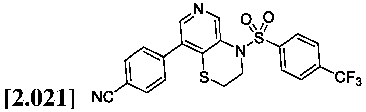

### 4-(4-((4-(Trifluoromethyl)phenyl)sulfonyl)-3,4-dihydro-2H-pyrido[4,3-b][1,4]thiazin-8-yl)be nzonitrile (S17)

Following the general method SC synthesized with **SI5** (100.0 mg, 0.4 mmol), 4-(trifluoromethyl)benzenesulfonyl chloride (193.3 mg, 0.8 mmol) in pyridine (4 mL) to give **S17** (70.8 mg, 38.4% yield) as a light yellow solid after chromatographic purification (PE/EA = 2/1). mp: 149.0-150.0 °C. ¹H-NMR (400 MHz, CDCl₃) δ 8.70 (s, 1H), 8.16 (s, 1H), 7.87 (d, *J* = 8.3 Hz, 2H), 7.79 (d, *J* = 8.4 Hz, 2H), 7.74 (d, *J* = 8.3 Hz, 2H), 7.42 (d, *J* = 8.3 Hz, 2H), 4.09-3.89 (m, 2H), 3.05-2.85 (m, 2H). ¹³C-NMR (100 MHz, DMSO-*d₆*) δ 146.79, 145.71, 142.79, 140.11, 138.14, 133.41 (²*J*_{CF} = 32.3 Hz), 132.88, 132.56 (2C), 130.29 (2C), 128.23 (2C), 127.02 (³*J*_{CF} = 10.7 Hz, 2C), 123.29 (¹*J*_{CF} = 272.9 Hz), 118.46, 111.46, 43.23, 25.96. HRMS (ESI): m/z calcd. for C₂₁H₁₄F₃N₃O₂S₂ [M+H]⁺: 462.0552, found: 462.0548.

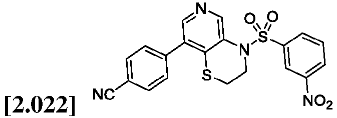

### 4-(4-((3-Nitrophenyl)sulfonyl)-3,4-dihydro-2H-pyrido[4,3-b][1,4]thiazin-8-yl)benzonitrile (S18)

Following the general method SE synthesized with **SI5** (50.0 mg, 0.2 mmol), DMAP (7.2 mg, 0.06 mmol), Et₃N (0.02 mL, 0.6mmol), 3-nitrobenzenesulfonyl chloride (65.5 mg, 0.3 mmol) in anhydrous dichloromethane (5 mL) to give **S18** (36.7 mg, 41.8% yield) as a light yellow solid after chromatographic purification (PE/EA = 1/1). mp: 88.0-89.0 °C. ¹H-NMR (400 MHz, CDCl₃) δ 8.73 (s, 1H), 8.49 (d, *J =* 6.4 Hz, 2H), 8.21 (s, 1H), 8.08 (d, *J* = 7.8 Hz, 1H), 7.76 (dd, *J =* 12.0, 8.4 Hz, 3H), 7.43 (d, *J* = 8.1 Hz, 2H), 4.09-3.97 (m, 2H), 3.01-2.91 (m, 2H). ¹³C-NMR (100 MHz, DMSO-*d₆*) δ 148.09,147.11, 145.98, 140.36, 140.04, 138.46, 132.94, 132.59 (2C), 132.10, 130.25 (2C), 130.14, 128.50, 121.86, 118.46, 111.71, 111.47, 43.32, 26.01. HRMS (ESI): m/z calcd. for C₂₀H₁₄N₄O₄S₂ [M+H]⁺: 439.0529, found: 439.0527.

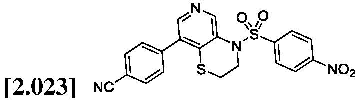

### 4-(4-((4-Nitrophenyl)sulfonyl)-3,4-dihydro-2H-pyrido[4,3-b][1,4]thiazin-8-yl)benzonitrile (S19)

Following the general method SC synthesized with **SI5** (100.0 mg, 0.4 mmol), 4-nitrobenzenesulfonyl chloride (133.3 mg, 0.6 mmol) in pyridine (4 mL) to give **S19** (70.8 mg, 38.4% yield) as a light yellow solid after chromatographic purification (PE/EA = 2/1). mp: 196.0-197.0 °C. ¹H-NMR (400 MHz, CDCl₃) δ 8.72 (s, 1H), 8.36 (d, *J* = 8.8 Hz, 2H), 8.19 (s, 1H), 7.92 (d, *J* = 8.8 Hz, 2H), 7.74 (d, *J* = 8.2 Hz, 2H), 7.43 (d, *J* = 8.3 Hz, 2H), 4.11-3.97 (m, 2H), 3.06-2.92 (m, 2H). ¹³C-NMR (100 MHz, DMSO-*d₆*) δ 150.41, 146.79, 145.82, 144.36, 140.09, 138.1, 132.93, 132.56 (2C), 130.37 (2C), 130.15 (2C), 128.84 (2C), 125.09, 118.48, 111.44, 43.27, 26.06. HRMS (ESI): m/z calcd. for C₂₀H₁₄N₄O₄S₂ [M+H]⁺: 439.0529, found: 439.0523.

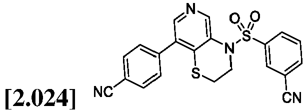

### 3-((8-(4-Cyanophenyl)-2,3-dihydro-4H-pyrido[4,3-b][1,4]thiazin-4-yl)sulfonyl)benzonitrile (S20)

Following the general method SC synthesized with **SI5** (100.0 mg, 0.4 mmol), 3-cynobenzenesulfonyl chloride (159.3 mg, 0.8 mmol) in pyridine (4 mL) to give **S20** (98.3 mg, 58.7% yield) as a light yellow solid after chromatographic purification (PE/EA = 1/1). mp: 167.0-168.0 °C. ¹H-NMR (400 MHz, CDCl₃) δ 8.72 (s, 1H), 8.22 (s, 1H), 7.94 (dd, *J =* 10.5, 7.0 Hz, 3H), 7.75 (d, *J =* 8.1 Hz, 2H), 7.68 (t, *J =* 8.1 Hz, 1H), 7.45 (d, *J =* 8.2 Hz, 2H), 4.04-3.96 (m, 2H), 2.99-2.90 (m, 2H). ¹³C-NMR (100 MHz, CDCl₃) δ 141.53, 139.92, 138.31, 136.79, 133.31, 132.68 (2C), 131.21, 130.90, 130.74, 130.27 (2C), 128.66, 128.14, 127.47, 118.39, 116.81, 114.41, 113.06, 43.79, 26.68. HRMS (ESI): m/z calcd. for C₂₁H₁₄N₄O₂S₂ [M+H]⁺: 419.0631, found: 419.0625.

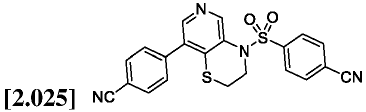

### 4-((8-(4-Cyanophenyl)-2,3-dihydro-4H-pyrido[4,3-b][1,4]thiazin-4-yl)sulfonyl)benzonitrile (S21)

Following the general method SC synthesized with **SI5** (100.0 mg, 0.4 mmol), 4-cynobenzenesulfonyl chloride (159.3 mg, 0.8 mmol) in pyridine (4 mL) to give **S21** (102.6 mg, 61.3% yield) as a light yellow solid after chromatographic purification (PE/EA = 1/1). mp: 203.0-204.0 °C. ¹H-NMR (400 MHz, CDCl₃) δ 8.70 (s, 1H), 8.20 (s, 1H), 7.83 (q, *J =* 8.1 Hz, 4H), 7.75 (d, *J =* 7.8 Hz, 2H), 7.43 (d, *J =* 8.0 Hz, 2H), 4.14-3.86 (m, 2H), 3.04-2.82 (m, 2H). ¹³C-NMR (100 MHz, DMSO-*d₆*) δ 146.77, 145.75, 142.96, 140.10, 138.10, 133.96 (2C), 132.89, 132.58 (2C), 130.35 (2C), 130.16, 127.90 (2C), 118.48, 117.48, 116.26, 111.45, 43.21, 25.99. HRMS (ESI): m/z calcd. for C₂₁H₁₄N₄O₂S₂ [M+H]⁺: 419.0631, found: 419.0634.

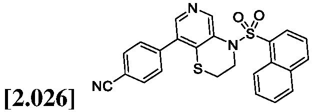

### 4-(4-(Naphthalen-1-ylsulfonyl)-3,4-dihydro-2H-pyrido[4,3-b][1,4]thiazin-8-yl)benzonitrile (S22)

Following the general method SC synthesized with **SI5** (100.0 mg, 0.4 mmol), naphthalene-1-sulfonyl chloride (179.1 mg, 0.8 mmol) in pyridine (4 mL) to give **S22** (113.1 mg, 63.7% yield) as a light yellow solid after chromatographic purification (PE/EA = 1/1). mp: 222.0-223.0 °C. ¹H-NMR (400 MHz, CDCl₃) δ 8.56 (s, 1H), 8.31 (d, *J =* 7.3 Hz, 1H), 8.16 (d, *J =* 8.3 Hz, 2H), 8.12 (s, 1H), 7.96 (d, *J =* 8.2 Hz, 1H), 7.68 (d, *J =* 8.0 Hz, 2H), 7.59 (dd, *J =* 11.4, 5.3 Hz, 2H), 7.43 (t, *J =* 7.7 Hz, 1H), 7.24 (s, 2H), 4.04-3.95 (m, 2H), 2.71-2.61 (m, 2H). ¹³C-NMR (100 MHz, CDCl₃) δ 147.05, 144.98, 140.11, 138.40, 135.67, 134.80, 134.69, 132.53 (3C), 130.57, 130.18 (2C), 129.19, 128.22, 127.33, 124.65, 124.54, 118.43, 112.84, 110.53, 43.07, 25.93. HRMS (ESI): m/z calcd. for C₂₄H₁₇N₃O₂S₂ [M+H]⁺: 444.0833, found: 444.0836.

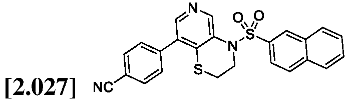

### 4-(4-(Naphthalen-2-ylsulfonyl)-3,4-dihydro-2H-pyrido[4,3-b][1,4]thiazin-8-yl)benzonitrile (S23)

Following the general method SC synthesized with **SI5** (100.0 mg, 0.4 mmol), naphthalene-2-sulfonyl chloride (179.1 mg, 0.8 mmol) in pyridine (4 mL) to give **S23** (107.4 mg, 60.5% yield) as a light yellow solid after chromatographic purification (PE/EA = 2/1). mp: 195.0-196.0 °C. ¹H-NMR (400 MHz, DMSO-*d₆*) δ 8.63 (s, 1H), 8.49 (d, *J* = 1.3 Hz, 1H), 8.17 (dd, *J* = 13.0, 8.5 Hz, 3H), 8.09 (d, *J* = 8.1 Hz, 1H), 7.91 (d, *J* = 8.3 Hz, 2H), 7.81-7.74 (m, 1H), 7.73-7.67 (m, 1H), 7.63 (dd, *J* = 8.7, 1.9 Hz, 1H), 7.50 (d, *J* = 8.3 Hz, 2H), 4.02-3.89 (m, 2H), 2.91-2.80 (m, 2H). ¹³C-NMR (100 MHz, DMSO-*d₆*) δ 146.87, 145.36, 140.18, 137.70, 135.87, 134.62, 132.73, 132.50 (2C), 131.76, 130.51, 130.24 (2C), 130.00, 129.55, 129.45, 128.77, 127.96, 127.95, 121.93, 118.44, 111.36, 42.88, 25.50. HRMS (ESI): m/z calcd. for C₂₄H₁₇N₃O₂S₂ [M+H]⁺: 444.0833, found: 444.0833.

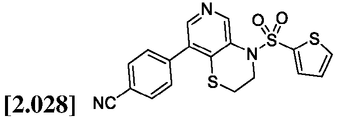

### 4-(4-(Thiophen-2-ylsulfonyl)-3,4-dihydro-2H-pyrido[4,3-b][1,4]thiazin-8-yl)benzonitrile (S24)

Following the general method SC synthesized with **SI5** (100.0 mg, 0.4 mmol), thiophene-2-sulfonyl chloride (144.3 mg, 0.8 mmol) in pyridine (4 mL) to give **S24** (45.2 mg, 28.5% yield) as a light yellow solid after chromatographic purification (PE/EA = 2/1). mp: 190.0-191.0 °C. ¹H-NMR (400 MHz, DMSO-*d₆*) δ 8.59 (s, 1H), 8.18 (s, 1H), 8.11 (dd, *J =* 5.0, 1.2 Hz, 1H), 7.96 (d, *J* = 8.1 Hz, 2H), 7.69 (dd, J = 3.8, 1.3 Hz, 1H), 7.56 (d, *J* = 8.1 Hz, 2H), 7.30-7.20 (m, 1H), 4.06-3.90 (m, 2H), 2.97-2.81 (m, 2H). ¹³C-NMR (100 MHz, DMSO-*d₆*) δ 147.31, 145.60, 140.14, 138.34, 138.18, 135.90, 133.79, 132.78, 132.58 (2C), 130.31 (2C), 130.01, 128.34, 118.47, 111.42, 43.20, 24.72. HRMS (ESI): m/z calcd. for C₁₈H₁₃N₃O₂S₃ [M+H]⁺: 400.0243, found: 400.0240.

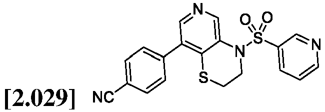

### 4-(4-(Pyridin-3-ylsulfonyl)-3,4-dihydro-2H-pyrido[4,3-b][1,4]thiazin-8-yl)benzonitrile (S25)

Following the general method SC synthesized with **SI5** (150.0 mg, 0.6 mmol), pyridine-3-sulfonyl chloride (0.14 mL, 1.2 mmol) in pyridine (6 mL) to give S25 (45.2 mg, 28.5% yield) as a light yellow solid after chromatographic purification (DCM/MeOH = 20/1). mp: 180.0-181.0 °C. ¹H-NMR (400 MHz, DMSO-*d₆*) δ 8.92 (d, *J* = 3.8 Hz, 1H), 8.86 (d, *J* = 2.2 Hz, 1H), 8.59 (s, 1H), 8.20 (s, 1H), 8.12 (d, *J =* 8.2 Hz, 1H), 7.96 (d, *J =* 8.3 Hz, 2H), 7.68 (dd, *J =* 8.1, 4.9 Hz, 1H), 7.54 (d, *J =* 8.2 Hz, 2H), 4.06-3.87 (m, 2H), 3.07-2.82 (m, 2H). ¹³C-NMR (100 MHz, DMSO-*d₆*) δ 154.42, 147.33, 147.02, 145.77, 140.05, 138.36, 135.41, 135.18, 132.89, 132.59 (2C), 130.27 (2C), 130.12, 124.81, 118.47, 111.44, 43.24, 26.02. HRMS (ESI): m/z calcd. for C₁₉H₁₄N₄O₂S₂ [M+H]⁺: 395.0631, found: 395.0633.

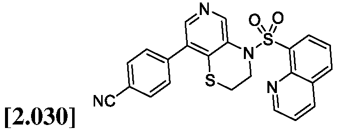

### 4-(4-(Quinolin-8-ylsulfonyl)-3,4-dihydro-2H-pyrido[4,3-b][1,4]thiazin-8-yl)benzonitrile (S26)

Following the general method SC synthesized with **SI5** (100.0 mg, 0.4 mmol), quinoline-8-sulfonyl chloride (179.9 mg, 0.8 mmol) in pyridine (6 mL) to give **S26** (100.5 mg, 57.2% yield) as a light yellow solid after chromatographic purification (PE/EA = 1/1). mp: 198.0-199.0 °C. ¹H-NMR (400 MHz, CDCl₃) δ 8.95 (dd, *J =* 4.2, 1.7 Hz, 1H), 8.65 (dd, *J =* 7.4, 1.3 Hz, 1H), 8.47 (s, 1H), 8.29 (dd, *J =* 8.4, 1.6 Hz, 1H), 8.14 (dd, *J =* 8.2, 1.2 Hz, 1H), 7.98 (s, 1H), 7.77-7.70 (m, 3H), 7.56 (dd, *J* = 8.3, 4.2 Hz, 1H), 7.46 (d, *J* = 8.3 Hz, 3H), 4.42-4.29 (m, 2H), 3.26-3.15 (m, 2H). ¹³C-NMR (100 MHz, DMSO-*d₆*) δ 151.25, 145.00, 144.18, 142.84, 140.54, 136.98, 136.55, 136.08, 135.16, 132.67, 132.51 (2C), 131.52, 131.42, 130.27 (2C), 128.80, 128.68, 122.74, 118.50, 111.27, 43.34, 26.43. HRMS (ESI): m/z calcd. for C₂₃H₁₆N₄O₂S₂ [M+H]⁺: 445.0787, found: 445.0782.

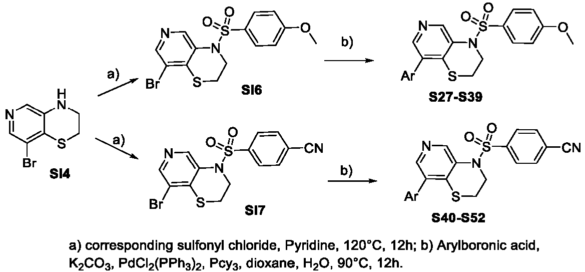

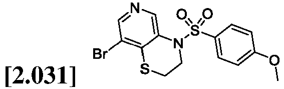

### 8-Bromo-4-((4-methoxyphenyl)sulfonyl)-3,4-dihydro-2H-pyrido[4,3-b][1,4]thiazine (SI6)

Following the general method SC synthesized with **SI4** (500.0 mg, 2.16 mmol), 4-methoxybenzenesulfonyl chloride (892.7 mg, 4.32 mmol) in pyridine (10 mL) to give SI6 (635.1 mg, 73.3% yield) as a white solid after chromatographic purification (PE/EA = 3/1). ¹H-NMR (400 MHz, CDCl₃) δ 8.67 (s, 1H), 8.40 (s, 1H), 7.58 (d, *J* = 8.9 Hz, 2H), 6.94 (d, *J* = 8.9 Hz, 2H), 3.95-3.89 (m, 2H), 3.87 (s, 3H), 2.96-2.88 (m, 2H).

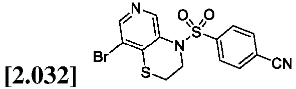

### 4-((8-Bromo-2,3-dihydro-4H-pyrido[4,3-b][1,4]thiazin-4-yl)sulfonyl)benzonitrile (SI7)

Following the general method SC synthesized with **SI4** (1.30 g, 5.65 mmol), 4-cynobenzenesulfonyl chloride (2.85g, 14.7 mmol) in pyridine (10 mL) to give SI7 (1.20 g, 54.3% yield) as a light yellow solid after chromatographic purification (PE/EA = 4/1). This intermediate was used directly in the next step without further characterization.

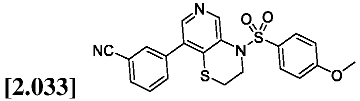

### 3-(4-((4-Methoxyphenyl)sulfonyl)-3,4-dihydro-2H-pyrido[4,3-b][1,4]thiazin-8-yl)benzonitril e (S27)

Following the general method SD, synthesized with **SI6** (100.0 mg, 0.25 mmol), (3-cyanophenyl)boronic acid (55.1 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S27** (89.1 mg, 84.2% yield) as a light yellow solid after chromatographic purification (PE/EA = 2/1). mp: 129.0-130.0 °C. ¹H-NMR (400 MHz, CDCl₃) δ 8.72 (s, 1H), 8.11 (s, 1H), 7.70 (s, 1H), 7.63 (d, *J* = 7.2 Hz, 3H), 7.56 (d, *J* = 4.9 Hz, 2H), 6.97 (d, *J* = 8.9 Hz, 2H), 3.98-3.91 (m, 2H), 3.89 (s, 3H), 2.96-2.86 (m, 2H). ¹³C-NMR (100 MHz, DMSO-*d₆*) δ 163.32, 146.92, 145.45, 137.77, 136.60, 134.22, 132.82, 132.37, 132.23, 130.51, 130.18, 129.96, 129.43 (2C), 118.36, 114.99 (2C), 111.78, 55.83, 42.68, 25.15. HRMS (ESI): m/z calcd. for C₂₁H₁₇N₃O₃S₂ [M+H]⁺: 424.0784, found: 424.0776.

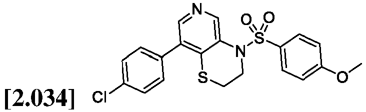

### 8-(4-Chlorophenyl)-4-((4-methoxyphenyl)sulfonyl)-3,4-dihydro-2H-pyrido[4,3-b][1,4]thiazin e (S28)

Following the general method SD, synthesized with **SI6** (100.0 mg, 0.25 mmol), (4-chlorophenyl)boronic acid (58.6 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S28** (89.1 mg, 84.2% yield) as a light yellow solid after chromatographic purification (PE/EA = 3/1). mp: 83.0-84.0 °C. ¹H-NMR (400 MHz, CDCl₃) δ 8.68 (s, 1H), 8.11 (s, 1H), 7.62 (d, *J* = 8.9 Hz, 2H), 7.41 (d, *J* = 8.4 Hz, 2H), 7.25 (d, *J* = 4.8 Hz, 2H), 6.95 (d, *J* = 8.9 Hz, 2H), 3.97-3.91 (m, 2H), 3.88 (s, 3H), 2.92-2.85 (m, 2H). ¹³C-NMR (100 MHz, DMSO-*d₆*) δ 163.29, 146.61, 145.34, 137.70, 134.24, 133.43, 133.01, 131.08 (2C), 130.44, 130.24, 129.40 (2C), 128.98, 128.68 (2C), 114.96 (2C), 55.86, 42.71, 25.09. HRMS (ESI): m/z calcd. for C₂₀H₁₇ClN₂O₃S₂ [M+H]⁺: 433.0442, found: 433.0430.

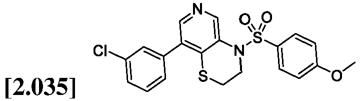

### 8-(3-Chlorophenyl)-4-((4-methoxyphenyl)sulfonyl)-3,4-dihydro-2H-pyrido[4,3-b][1,4]thiazin e (S29)

Following the general method SD, synthesized with **SI6** (100.0 mg, 0.25 mmol), (3-chlorophenyl)boronic acid (58.6 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S29** (68.0 mg, 62.8% yield) as a light yellow solid after chromatographic purification (PE/EA = 3/1). mp: 176.0-177.0 °C. ¹H-NMR (400 MHz, CDCl₃) δ 8.69 (s, 1H), 8.12 (s, 1H), 7.61 (d, *J* = 7.0 Hz, 2H), 7.41-7.34 (m, 2H), 7.30 (s, 1H), 7.19 (d, *J* = 6.5 Hz, 1H), 6.96 (d, *J* = 8.9 Hz, 2H), 3.97-3.91 (m, 2H), 3.88 (s, 3H), 2.92-2.85 (m, 2H). ¹³C-NMR (100 MHz, DMSO-*d₆*) δ 163.29, 146.73, 145.35, 137.85, 137.43, 133.15, 132.75, 130.57, 130.43, 130.15, 129.41 (2C), 128.91, 128.52, 127.99, 114.94 (2C), 55.75, 42.67, 25.15. HRMS (ESI): m/z calcd. for C₂₀H₁₇ClN₂O₃S₂ [M+H]⁺: 433.0442, found: 433.0425.

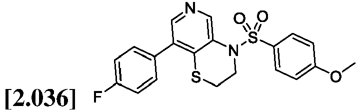

### 8-(4-Fluorophenyl)-4-((4-methoxyphenyl)sulfonyl)-3,4-dihydro-2H-pyrido[4,3-b][1,4]thiazin e (S30)

Following the general method SD, synthesized with **SI6** (100.0 mg, 0.25 mmol), (4-flourophenyl)boronic acid (52.1 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S30** (96.3 mg, 92.5% yield) as a light yellow solid after chromatographic purification (PE/EA = 3/1). mp: 178.0-179.0 °C. ¹H-NMR (400 MHz, CDCl₃) δ 8.67 (s, 1H), 8.11 (s, 1H), 7.63 (d, *J* = 8.9 Hz, 2H), 7.28 (dd, *J* = 8.7, 5.4 Hz, 2H), 7.12 (t, *J* = 8.6 Hz, 2H), 6.95 (d, *J =* 8.9 Hz, 2H), 3.98-3.90 (m, 2H), 3.88 (s, 3H), 2.93-2.83 (m, 2H). ¹³C-NMR (100 MHz, DMSO-*d₆*) δ 163.28, 162.15 (¹*J*_{CF} = 245.7 Hz), 146.48, 145.44, 137.84, 133.23, 131.75 (⁴*J*_{CF} = 3.2 Hz), 131.41 (³*J*_{CF} = 8.4 Hz, 2C), 130.41, 130.27, 129.39 (2C), 115.58 (²*J*_{CF} = 21.7 Hz, 2C), 114.95 (2C), 55.86, 42.73, 25.06. HRMS (ESI): m/z calcd. for C₂₀H₁₇FN₂O₃S₂ [M+H]⁺: 417.0737, found: 417.0723.

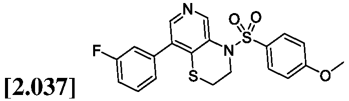

### 8-(3-Fluorophenyl)-4-((4-methoxyphenyl)sulfonyl)-3,4-dihydro-2H-pyrido[4,3-b][1,4]thiazin e (S31)

Following the general method SD, synthesized with **SI6** (100.0 mg, 0.25 mmol), (3-flourophenyl)boronic acid (52.1 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S31** (103.5 mg, 98.9% yield) as a white solid after chromatographic purification (PE/EA = 3/1). mp: 164.0-165.0 °C. ¹H-NMR (400 MHz, CDCl₃) δ 8.69 (s, 1H), 8.13 (s, 1H), 7.62 (d, *J* = 8.8 Hz, 2H), 7.41 (dd, *J* = 14.7, 7.0 Hz, 1H), 7.11 (dd, *J* = 11.5, 9.1 Hz, 2H), 7.04 (d, *J* = 9.3 Hz, 1H), 6.96 (d, *J* = 8.8 Hz, 2H), 3.99-3.92 (m, 2H), 3.88 (s, 3H), 2.94-2.85 (m, 2H). ¹³C-NMR (100 MHz, DMSO-*d₆*) δ 163.30, 161.94 (¹*J*_{CF} = 244.5 Hz), 146.70, 145.37, 137.80, 137.64 (³*J*_{CF} = 137.64 Hz), 132.91 (⁴*J*_{CF} = 1.4 Hz), 130.73 (³*J*_{CF} = 8.6 Hz), 130.45, 130.21, 129.41 (2C), 125.47 (⁴*J*_{CF} = 2.5 Hz), 116.10 (²*J*_{CF} = 22.0 Hz), 115.44 (²*J*_{CF} = 20.7 Hz), 114.96 (2C), 55.85, 42.75, 25.06. HRMS (ESI): m/z calcd. for C₂₀H₁₇FN₂O₃S₂ [M+H]⁺: 417.0737, found: 417.0726.

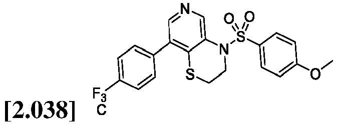

### 4-((4-methoxyphenyl) sulfonyl)-8-(4-(trifluoromethyl)phenyl)-3,4-dihydro-2H-pyrido[4,3-b][1,4]thiazine (S32)

Following the general method SD, synthesized with **SI6** (100.0 mg, 0.25 mmol), (3-triflouromethylphenyl)boronic acid (71.2 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S32** (80.1 mg, 68.7% yield) as a white solid after chromatographic purification (PE/EA = 2/1). mp: 112.0-113.0 °C. ¹H-NMR (400 MHz, CDCl₃) δ 8.70 (s, 1H), 8.12 (s, 1H), 7.68 (d, *J* = 8.0 Hz, 2H), 7.62 (d, *J* = 8.8 Hz, 2H), 7.44 (d, *J* = 8.0 Hz, 2H), 6.95 (d, *J* = 8.8 Hz, 2H), 3.98-3.90 (m, 2H), 3.87 (s, 3H), 2.93-2.84 (m, 2H). ¹³C-NMR (100 MHz, DMSO-*d₆*) δ 163.32, 146.83, 145.23, 139.62, 137.74, 131.75 (¹*J*_{CF} = 235.3 Hz), 130.22, 130.20 (2C), 129.42 (2C), 129.01 (²*J*_{CF} = 31.7 Hz), 125.53 (³*J*_{CF} = 10.8 Hz, 2C), 125.44, 122.73, 114.97 (2C), 55.86, 42.68, 25.09. HRMS (ESI): m/z calcd. for C₂₁H₁₇F₃N₂O₃S₂ [M+H]⁺: 467.0705, found: 467.0693.

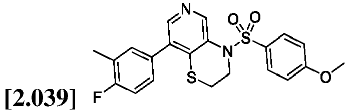

### 8-(4-Fluoro-3-methylphenyl)-4-((4-methoxyphenyl)sulfonyl)-3,4-dihydro-2H-pyrido[4,3-b][1 ,4]thiazine (S33)

Following the general method SD, synthesized with **SI6** (100.0 mg, 0.25 mmol), (4-flouro-3-methylphenyl)boronic acid (57.7 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S33** (95.9 mg, 89.1% yield) as a white solid after chromatographic purification (PE/EA = 2/1). mp: 152.0-153.0 °C. ¹H-NMR (400 MHz, DMSO-*d₆*) δ 8.54 (s, 1H), 8.14 (s, 1H), 7.63-7.50 (m, 4H), 7.40-7.33 (m, 1H), 7.13 (d, *J* = 10.1 Hz, 2H), 3.92-3.79 (m, 5H), 3.32 (s, 3H), 2.91-2.81 (m, 2H). ¹³C-NMR (100 MHz, DMSO-*d₆*) δ 163.28, 160.68 (¹*J*_{CF} = 244.9 Hz), 146.37, 145.39, 137.90, 133.38, 132.48 (³*J*_{CF} = 5.5 Hz), 131.47 (⁴*J*_{CF} = 3.6 Hz), 130.37, 130.23, 129.40 (2C), 128.63 (³*J*_{CF} = 8.4 Hz), 124.64 (²*J*_{CF} = 17.7 Hz), 115.15 (²*J*_{CF} = 22.5 Hz), 114.95 (2C), 55.86, 42.79, 25.08, 14.12 (³*J*_{CF} = 3.2 Hz). HRMS (ESI): m/z calcd. for C₂₁H₁₉FN₂O₃S₂ [M+H]⁺: 431.0894, found: 431.0884.

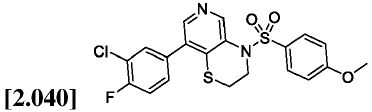

### 8-(3-chloro-4-fluorophenyl)-4-((4-methoxyphenyl)sulfonyl)-3,4-dihydro-2H-pyrido[4,3-b] [1, 4]thiazine (S34)

Following the general method SD, synthesized with **SI6** (100.0 mg, 0.25 mmol), (3-chloro-4-flouro-phenyl)boronic acid (65.4 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S34** (99.7 mg, 88.4% yield) as a white solid after chromatographic purification (PE/EA = 2/1). mp: 155.0-156.0 °C. ¹H-NMR (400 MHz, CDCl₃) δ 8.68 (s, 1H), 8.11 (s, 1H), 7.63 (d, *J* = 8.7 Hz, 2H), 7.37 (d, *J* = 6.5 Hz, 1H), 7.24-7.16 (m, 2H), 6.97 (d, *J* = 8.8 Hz, 2H), 3.98-3.91 (m, 2H), 3.89 (s, 3H), 2.96-2.86 (m, 2H). ¹³C-NMR (100 MHz, DMSO-*d₆*) δ 163.31, 157.25 (¹*J*_{CF} = 248.6 Hz), 146.78, 145.46, 137.91, 133.09 (J = 3.8 Hz), 131.99, 131.38, 130.45, 130.28, 130.21, 130.18, 129.43 (2C), 119.70 (²*J*_{CF} = 17.9 Hz), 117.22 (²*J*_{CF} = 21.1 Hz), 114.97 (2C), 55.86, 42.70, 25.11. HRMS (ESI): m/z calcd. for C₂₀H₁₆ClFN₂O₃S₂ [M+H]⁺: 451.0348, found: 451.0328.

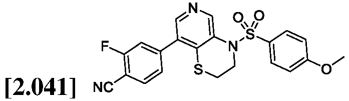

### 2-Fluoro-4-(4-((4-methoxyphenyl)sulfonyl)-3,4-dihydro-2H-pyrido[4,3-b][1,4]thiazin-8-yl)be nzonitrile (S35)

Following the general method SD, synthesized with **SI6** (100.0 mg, 0.25 mmol), (4-cyno-3-flouro-phenyl)boronic acid (61.9 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S35** (103.0 mg, 93.3% yield) as a white solid after chromatographic purification (PE/EA = 2/1). mp: 147.0-148.0 °C. ¹H-NMR (400 MHz, CDCl₃) δ 8.71 (s, 1H), 8.11 (s, 1H), 7.70 (dd, *J* = 8.1, 6.6 Hz, 1H), 7.64 (d, *J* = 8.9 Hz, 2H), 7.26-7.21 (m, 2H), 6.97 (d, *J* = 8.9 Hz, 2H), 3.97-3.91 (m, 2H), 3.89 (s, 3H), 2.97-2.89 (m, 2H). ¹³C-NMR (100 MHz, CDCl₃) δ 163.89, 163.02 (¹*J*_{CF} = 259.2 Hz), 147.80, 145.19, 143.07 (³*J*_{CF} = 8.1 Hz), 137.66, 133.87, 131.97 (³*J_{CF} =* 2.3 Hz), 131.31 (⁴*J*_{CF} = 1.1 Hz), 130.94, 129.69 (2C), 126.11 (⁴*J*_{CF} = 3.5 Hz), 117.77 (²*J*_{CF} = 20.2 Hz), 114.82 (2C), 113.65, 101.79 (²*J*_{CF} = 15.3 Hz), 55.91, 43.04, 26.05. HRMS (ESI): m/z calcd. for C₂₁H₁₆FN₃O₃S₂ [M+H]⁺: 442.0690, found: 442.0684.

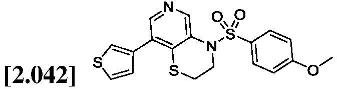

### 4-((4-Methoxyphenyl) sulfonyl)-8-(thiophen-3-yl)-3,4-dihydro-2H-pyrido[4,3-b][1,4]thiazine (S36)

Following the general method SD, synthesized with **SI6** (100.0 mg, 0.25 mmol), thiophen-3-ylboronic acid (48.0 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S36** (95.0 mg, 93.7% yield) as a white solid after chromatographic purification (PE/EA = 2/1). mp: 125.0-126.0 °C. ¹H-NMR (400 MHz, CDCl₃) δ 8.64 (s, 1H), 8.22 (s, 1H), 7.62 (d, *J* = 8.9 Hz, 2H), 7.41 (dd, *J* = 4.9, 3.0 Hz, 1H), 7.37 (dd, *J* = 2.9, 1.3 Hz, 1H), 7.16 (d, *J* = 3.6 Hz, 1H), 6.94 (d, *J* = 8.9 Hz, 2H), 3.98-3.91 (m, 2H), 3.87 (s, 3H), 2.94-2.85 (m, 2H). ¹³C-NMR (100 MHz, DMSO-*d₆*) δ 163.27, 146.15, 145.53, 137.69, 135.44, 130.47, 130.30, 129.39 (2C), 129.37, 128.23, 126.70, 125.54, 114.95 (2C), 55.85, 42.67, 25.11. HRMS (ESI): m/z calcd. for C₁₈H₁₆N₂O₃S₃ [M+H]⁺: 405.0396, found: 405.0391.

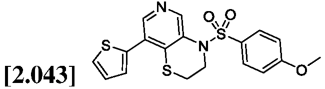

### 4-((4-Methoxyphenyl) sulfonyl)-8-(thiophen-2-yl)-3,4-dihydro-2H-pyrido[4,3-b][1,4]thiazine (S37)

Following the general method SD, synthesized with **SI6** (100.0 mg, 0.25 mmol), thiophen-2-ylboronic acid (48.0 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S37** (53.0 mg, 52.3% yield) as a white solid after chromatographic purification (PE/EA = 2/1). mp: 146.0-147.0 °C. ¹H-NMR (400 MHz, CDCl₃) δ 8.67 (s, 1H), 8.31 (s, 1H), 7.64-7.55 (m, 2H), 7.42 (dd, *J =* 5.1, 1.0 Hz, 1H), 7.18 (dd, *J* = 3.6, 1.1 Hz, 1H), 7.11 (dd, *J* = 5.1, 3.6 Hz, 1H), 6.94 (d, *J* = 8.9 Hz, 2H), 3.98-3.90 (m, 2H), 3.87 (s, 3H), 2.95-2.85 (m, 2H). ¹³C-NMR (100 MHz, DMSO-*d₆*) δ 163.30, 146.50, 145.91, 138.44, 135.49, 131.13, 130.15, 129.41 (2C), 128.76, 127.98, 127.67, 127.30, 114.96 (2C), 55.86, 42.56, 25.37. HRMS (ESI): m/z calcd. for C₁₈H₁₆N₂O₃S₃ [M+H]⁺: 405.0396, found: 405.0379.

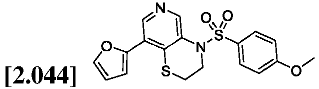

### 8-(Furan-2-yl)-4-((4-methoxyphenyl)sulfonyl)-3,4-dihydro-2H-pyrido[4,3-b][1,4]thiazine (S38)

Following the general method SD, synthesized with **SI6** (100.0 mg, 0.25 mmol), furan-2-ylboronic acid (42.0 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S38** (45.1 mg, 50.5% yield) as a white solid after chromatographic purification (PE/EA = 2/1). mp: 146.0-147.0 °C. ¹H-NMR (400 MHz, CDCl₃) δ 8.64 (s, 2H), 7.60 (d, *J* = 8.9 Hz, 2H), 7.54 (d, *J* = 1.4 Hz, 1H), 6.92 (d, *J* = 8.9 Hz, 2H), 6.82 (d, *J* = 3.4 Hz, 1H), 6.52 (dd, *J* = 3.4, 1.8 Hz, 1H), 4.01-3.94 (m, 2H), 3.85 (s, 3H), 3.02-2.93 (m, 2H). ¹³C-NMR (100 MHz, DMSO-*d₆*) δ 163.27, 147.78, 145.96, 144.12, 143.77, 135.79, 132.23, 130.64, 130.29, 129.37 (2C), 114.99 (2C), 111.88, 111.57, 55.83, 42.44, 25.19. HRMS (ESI): m/z calcd. for C₁₈H₁₆N₂O₄S₂ [M+H]⁺: 389.0624, found: 389.0625.

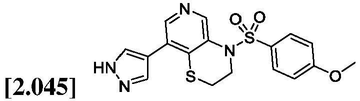

### 4-((4-Methoxyphenyl)sulfonyl)-8-(1H-pyrazol-4-yl)-3,4-dihydro-2H-pyrido[4,3-b][1,4]thiazi ne (S39)

Following the general method SD, synthesized with **SI6** (100.0 mg, 0.25 mmol), (1H-pyrazol-4-yl)boronic acid (72.8 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S39** (52.3 mg, 53.9% yield) as a white solid after chromatographic purification (DCM/MeOH = 20/1). mp: 114.0-115.0 °C. ¹H-NMR (400 MHz, DMSO-*d₆*) δ 13.14 (s, 1H), 8.40 (s, 1H), 8.25 (s, 1H), 7.99 (d, *J* = 29.2 Hz, 1H), 7.74 (s, 1H), 7.61 (d, *J* = 8.9 Hz, 2H), 7.12 (d, *J* = 9.0 Hz, 2H), 3.94-3.86 (m, 2H), 3.84 (s, 3H), 2.96-2.90 (m, 2H). ¹³C-NMR (100 MHz, DMSO-*d₆*) δ 163.23, 145.30, 145.17, 138.25, 136.82, 130.48, 130.41. 129.37 (2C), 128.11, 126.11, 114.95 (2C), 114.71, 55.83, 42.61, 25.19. HRMS (ESI): m/z calcd. for C₁₇H₁₆N₄O₃S₂ [M+H]⁺: 389.0737, found: 389.0745.

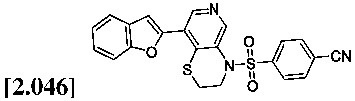

### 4-((8-(Benzofuran-2-yl)-2,3-dihydro-4H-pyrido[4,3-b][1,4]thiazin-4-yl)sulfonyl)benzonitrile (S40)

Following the general method SD, synthesized with **SI7** (100.0 mg, 0.25 mmol), benzofuran-2-ylboronic acid (45.3 mg, 0.375 mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S40** (49.6 mg, 45.8% yield) as a white solid after chromatographic purification (DCM/MeOH = 300/1). mp: 185.0-186.0 °C. ¹H-NMR (400 MHz, Chloroform-d) δ 8.87 (s, 1H), 8.67 (s, 1H), 7.79 (s, 2H), 7.78 (s, 2H), 7.62 (d, *J* = 7.2 Hz, 1H), 7.55 (d, *J* = 8.3 Hz, 1H), 7.36 (s, 1H), 7.28 (d, *J* = 6.9 Hz, 1H), 7.17 (s, 1H), 4.06 (s, 2H), 3.04 (s, 2H).¹³C-NMR (101 MHz, Chloroform-d) *δ* 154.55, 149.80, 146.95, 146.48, 143.91, 137.56, 133.33 (2C), 128.38, 127.98 (2C), 125.73, 123.50, 121.61, 117.43, 117.10, 111.47, 108.56, 43.75, 26.79. HRMS (ESI): m/z calcd. for C₂₂H₁₆N₃O₃S₂⁺ [M+H]⁺:434.0628, found: 434.0621.

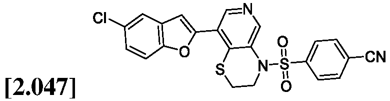

### 4-((8-(5-Chlorobenzofuran-2-yl)-2,3-dihydro-4H-pyrido[4,3-b][1,4]thiazin-4-yl)sulfonyl)ben zonitrile (S41)

Following the general method SD, synthesized with **SI7** (100.0 mg, 0.25 mmol), (5-chlorobenzofuran-2-yl)boronic acid (55.0 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S41** (52.3 mg, 44.7% yield) as a white solid after chromatographic purification (DCM/MeOH = 300/1). mp: 200.0-201.0 °C. ¹H-NMR (400 MHz, Chloroform-d) *δ* 8.85 (s, 1H), 8.69 (s, 1H), 7.80 (s, 2H), 7.78 (d, *J* = 8.0 Hz, 2H), 7.58 (d, *J* = 2.1 Hz, 1H), 7.47 (d, *J* = 8.7 Hz, 1H), 7.31 (dd, *J* = 8.7, 2.1 Hz, 1H), 7.13 (s, 1H), 4.06 (s, 2H), 3.06 (s, 2H). ¹³C-NMR (101 MHz, Chloroform-d) *δ* 152.93, 147.22, 146.52, 143.90, 133.38 (2C), 129.72, 129.12, 128.00 (2C), 125.96, 121.12, 117.51, 117.08, 112.50, 107.98, 43.69, 26.84. HRMS (ESI): m/z calcd. for C₂₂H₁₅ClN₃O₃S₂⁺ [M+H]⁺:462.0238, found: 462.0238.

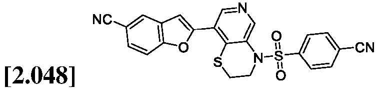

### 2-(4-((4-Cyanophenyl)sulfonyl)-3,4-dihydro-2H-pyrido[4,3-b][1,4]thiazin-8-yl)benzofuran-5-carbonitrile (S42)

Following the general method SD, synthesized with **SI7** (100.0 mg, 0.25 mmol), (5-cyanobenzofuran-2-yl)boronic acid (52.3 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S42** (58.1 mg, 50.7% yield) as a white solid after chromatographic purification (DCM/MeOH = 300/1). mp: 207.0-208.0 °C. ¹H-NMR (400 MHz, Chloroform-d) *δ* 8.85 (s, 1H), 8.70 (s, 1H), 7.96 (s, 1H), 7.81 (d, *J* = 8.7 Hz, 2H), 7.78 (d, *J* = 8.7 Hz, 2H), 7.63 (s, 2H), 7.24 (s, 1H), 4.07 (s, 2H), 3.07 (s, 2H). ¹³C-NMR (101 MHz, Chloroform-d) δ 156.01, 152.35, 147.74, 146.70, 143.85, 137.83, 133.39 (2C), 129.12, 129.08, 127.97 (2C), 126.58, 119.23, 117.54, 117.05, 112.71, 107.91, 107.54, 43.60, 26.83. HRMS (ESI): m/z calcd. for C₂₃H₁₅N₄O₃S₂⁺ [M+H]⁺:459.0580, found: 459.0584.

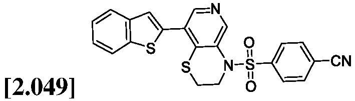

### 4-((8-(Benzo[b]thiophen-2-yl)-2,3-dihydro-4H-pyrido[4,3-b][1,4]thiazin-4-yl)sulfonyl)benzon itrile (S43)

Following the general method SD, synthesized with **SI7** (100.0 mg, 0.25 mmol), benzo[b]thiophen-2-ylboronic acid (49.8 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S43** (47.7 mg, 42.4% yield) as a white solid after chromatographic purification (DCM/MeOH = 300/1). mp: 167.0-168.0 °C. ¹H-NMR (400 MHz, Chloroform-*d*) *δ* 8.79 (s, 1H), 8.44 (s, 1H), 7.89-7.83 (m, 1H), 7.82 (d, *J* = 2.5 Hz, 1H), 7.81 (s, 4H), 7.43-7.40 (m, 1H), 7.39 (s, 1H), 7.36 (s, 1H), 4.03 (s, 2H), 2.95 (s, 2H). ¹³C-NMR (101 MHz, Chloroform-*d*) δ 143.91, 140.46, 139.63, 136.02, 133.32 (2C), 128.03 (2C), 125.69, 125.32, 124.94, 124.20, 122.33, 117.47, 117.13, 43.85, 26.77. HRMS (ESI): m/z calcd. for C₂₂H₁₆N₃O₂S₃⁺ [M+H]⁺:450.0399, found: 450.0392.

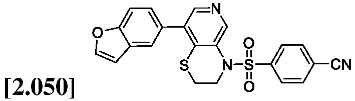

### 4-((8-(Benzofuran-5-yl)-2,3-dihydro-4H-pyrido[4,3-b][1,4]thiazin-4-yl)sulfonyl)benzonitrile( S44)

Following the general method SD, synthesized with **SI7** (100.0 mg, 0.25 mmol), benzofuran-5-ylboronic acid (45.3 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S44** (54.2 mg, 50.0% yield) as a white solid after chromatographic purification (DCM/MeOH = 300/1). mp: 179.0-180.0 °C. ¹H-NMR (400 MHz, Chloroform-d) *δ* 8.70 (s, 1H), 8.24 (s, 1H), 7.81 (s, 4H), 7.69 (d, *J* = 2.2 Hz, 1H), 7.57 (d, *J* = 8.5 Hz, 1H), 7.48 (s, 1H), 7.17 (d, *J* = 10.3 Hz, 1H), 6.82 (s, 1H), 4.02 (s, 2H), 2.89 (s, 2H). ¹³C-NMR (101 MHz, Chloroform-d) *δ* 155.04, 147.01, 146.54, 146.18, 144.03, 138.86, 133.24 (2C), 130.01, 128.05 (2C), 125.56, 122.18, 117.36, 117.17, 111.83, 106.84, 44.07, 26.67. HRMS (ESI): m/z calcd. for C₂₂H₁₆N₃O₃S₂⁺ [M+H]⁺:434.0628, found: 434.0632.

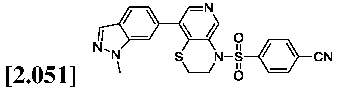

### 4-((8-(1-Methyl-1H-indazol-6-yl)-2,3-dihydro-4H-pyrido[4,3-b][1,4]thiazin-4-yl)sulfonyl)ben zonitrile (S45)

Following the general method SD, synthesized with **SI7** (100.0 mg, 0.25 mmol), (1-methyl-1H-indazol-6-yl)boronic acid (49.0 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S45** (57.2 mg, 51.1% yield) as a white solid after chromatographic purification (DCM/MeOH = 300/1). mp: 190.0-191.0 °C. ¹H-NMR (400 MHz, Chloroform-*d*) δ 8.70 (s, 1H), 8.28 (s, 1H), 8.03 (s, 1H), 7.86 (d, *J* = 8.2 Hz, 2H), 7.82 (d, *J* = 8.3 Hz, 2H), 7.79 (d, *J* = 8.2 Hz, 1H), 7.31 (s, 1H), 7.03 (d, *J* = 8.2 Hz, 1H), 4.10 (s, 3H), 4.02 (s, 2H), 2.93 (s, 2H). ¹³C-NMR (101 MHz, Chloroform-d) *δ* 143.94, 139.90, 133.32 (2C), 133.01, 128.11 (2C), 124.15, 121.86, 121.63, 117.46, 117.13, 109.82, 43.98, 35.85, 26.74. HRMS (ESI): m/z calcd. for C₂₂H₁₈N_{S}O₂S₂⁺ [M+H]⁺: 448.0896, found: 448.0897.

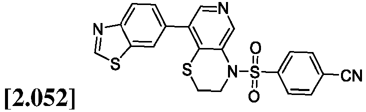

### 4-((8-(Benzo[d]thiazol-6-yl)-2,3-dihydro-4H-pyrido[4,3-b][1,4]thiazin-4-yl)sulfonyl)benzonit rile (S46)

Following the general method SD, synthesized with **SI7** (100.0 mg, 0.25 mmol), benzo[d]thiazol-6-ylboronic acid (49.9 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S46** (49.7 mg, 44.1% yield) as a white solid after chromatographic purification (DCM/MeOH = 150/1). mp: 253.0-254.0 °C. ¹H-NMR (400 MHz, Chloroform-*d*) *δ* 9.07 (s, 1H), 8.74 (s, 1H), 8.20 (d, *J* = 8.4 Hz, 1H), 7.89 (s, 1H), 7.85 (d, *J* = 8.5 Hz, 2H), 7.82 (d, *J* = 8.4 Hz, 2H), 7.43 (d, *J* = 10.1 Hz, 1H), 4.03 (s, 2H), 2.92 (s, 2H).¹³C-NMR (101 MHz, Chloroform-d) *δ* 155.36, 153.49, 133.32 (2C), 128.05 (2C), 127.70, 123.96, 122.84, 117.48, 117.13, 43.95, 26.70. HRMS (ESI): m/z calcd. for C₂₁H₁₅N₄O₂S₃⁺ [M+H]⁺:451.0352, found: 451.0359.

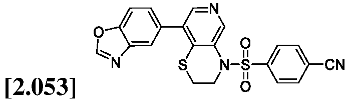

### 4-((8-(Benzo[d]oxazol-6-yl)-2,3-dihydro-4H-pyrido[4,3-b][1,4]thiazin-4-yl)sulfonyl)benzonitr ile (47)

Following the general method SD, synthesized with **SI7** (100.0 mg, 0.25 mmol), benzo[d]oxazol-6-ylboronic acid (48.5 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S47** (51.3 mg, 51.1% yield) as a white solid after chromatographic purification (PE/EA = 2/1). mp: 221.0-222.0 °C. ¹H-NMR (400 MHz, Chloroform-d) δ 8.69 (s, 1H), 8.23 (s, 1H), 8.16 (s, 1H), 7.89-7.76 (m, 5H), 7.53 (d, *J* = 1.6 Hz, 1H), 7.28 (dd, *J* = 8.2, 1.6 Hz, 1H), 4.01 (t, *J* = 5.6 Hz, 2H). HRMS (ESI): m/z calcd. for C₂₁H₁₅N₄O₃S₂⁺[M+H] ⁺: 435.0580, found: 435.0581.

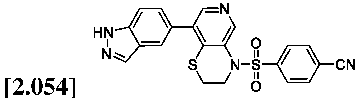

### 4-((8-(1H-Indazol-5-yl)-2,3-dihydro-4H-pyrido[4,3-b][1,4]thiazin-4-yl)sulfonyl)benzonitrile (S48)

Following the general method SD, synthesized with **SI7** (100.0 mg, 0.25 mmol), (1*H*-indazol-5-yl)boronic acid (45.1 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S48** (50.4 mg, 46.5% yield) as a white solid after chromatographic purification (DCM/MeOH = 150/1). mp: 190.0-191.0 °C. ¹H-NMR (400 MHz, DMSO-*d₆*) *δ* 8.53 (s, 1H), 8.47 (s, 1H), 8.24 (s, 1H), 8.05 (d, *J* = 8.1 Hz, 2H), 7.80 (d, *J* = 8.0 Hz, 2H), 7.69 (s, 1H), 3.95 (s, 2H), 2.96 (s, 2H). ¹³C-NMR (101 MHz, DMSO-*d₆*) δ 166.30, 147.03, 145.24, 140.88, 139.56, 139.04, 128.96 (2C), 127.19 (2C), 42.63, 26.24. HRMS (ESI): m/z calcd. for C₂₁H₁₆N₅O₂S₂⁺ [M+H]⁺:434.0740, found: 434.0635.

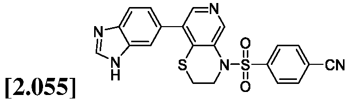

### 4-((8-(1H-Benzo[d]imidazol-6-yl)-2,3-dihydro-4H-pyrido[4,3-b][1,4]thiazin-4-yl)sulfonyl)ben zonitrile (S49)

Following the general method SD, synthesized with **SI7** (100.0 mg, 0.25 mmol), (1H-benzo[d]imidazol-5-yl)boronic acid (68.1 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S49** (52.0 mg, 48.0% yield) as a white solid after chromatographic purification (DCM/MeOH = 30/1). mp: 195.0-196.0 °C. ¹H-NMR (400 MHz, DMSO-*d₆*) *δ* 8.54 (s, 1H), 8.47 (s, 1H), 8.23 (s, 1H), 8.05 (d, *J* = 8.1 Hz, 2H), 7.80 (d, *J* = 8.2 Hz, 2H), 7.68 (s, 1H), 3.95 (s, 2H), 2.96 (s, 2H). ¹³C-NMR (101 MHz, DMSO-*d₆*) δ 166.30, 147.03, 145.25, 140.88, 139.56, 139.03, 139.00, 131.13, 128.95 (2C), 127.20 (2C), 117.81, 42.62, 26.23. HRMS (ESI): m/z calcd. for C₂₁H₁₆N₅O₂S₂⁺ [M+H]⁺:434.0740, found: 434.0638.

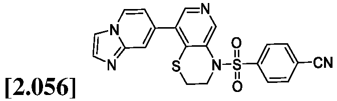

### 4-((8-(Imidazo[1,2-a]pyridin-7-yl)-2,3-dihydro-4H-pyrido[4,3-b][1,4]thiazin-4-yl)sulfonyl)be nzonitrile (S50)

Following the general method SD, synthesized with **SI7** (100.0 mg, 0.25 mmol), imidazo[1,2-a]pyridin-7-ylboronic acid (67.9 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S50** (49.6 mg, 45.8% yield) as a white solid after chromatographic purification (DCM/MeOH = 30/1). mp: 228.0-229.0 °C. ¹H-NMR (400 MHz, Chloroform-d) *δ* 8.73 (s, 1H), 8.25 (s, 1H), 8.18 (d, *J* = 6.9 Hz, 1H), 7.83 (d, *J* = 8.5 Hz, 2H), 7.80 (d, *J* = 8.8 Hz, 2H), 7.70 (s, 1H), 7.65 (s, 1H), 7.59 (s, 1H), 6.73 (d, *J* = 5.2 Hz, 1H), 4.01 (t, *J* = 5.5 Hz, 2H), 2.93 (t, *J* = 5.7 Hz, 2H).¹³C-NMR (101 MHz, Chloroform-*d*) *δ* 147.19, 146.57, 145.00, 143.99, 138.20, 134.89, 133.31 (2C), 133.04, 131.56, 130.49, 127.99 (2C), 125.84, 118.49, 117.48, 117.04, 113.61, 112.90, 43.83, 26.62. HRMS (ESI): m/z calcd. for C₂₁H₁₆N₅O₂S₂⁺ [M+H]⁺:434.0740, found: 434.0750.

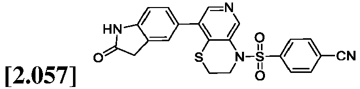

### 4-((8-(2-Oxoindolin-5-yl)-2,3-dihydro-4H-pyrido[4,3-b][1,4]thiazin-4-yl)sulfonyl)benzonitril e (S51)

Following the general method SD, synthesized with **SI7** (100.0 mg, 0.25 mmol), (2-oxoindolin-5-yl)boronic acid (72.68 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S51** (37.5 mg, 33.4% yield) as a white solid after chromatographic purification (DCM/MeOH = 50/1). mp: 197.0-198.0 °C. ¹H-NMR (400 MHz, DMSO-*d₆*) *δ* 10.52 (s, 1H), 8.48 (s, 1H), 8.13 (s, 1H), 8.10 (s, 2H), 7.88 (d, *J* = 8.5 Hz, 2H), 7.15 (s, 1H), 7.11 (d, *J* = 9.8 Hz, 1H), 6.89 (d, *J* = 7.9 Hz, 1H), 3.96 (s, 2H), 3.51 (s, 2H), 2.89 (s, 2H). ¹³C-NMR (101 MHz, DMSO-*d₆*) *δ* 176.30, 145.94, 145.80, 144.13, 143.02, 138.42, 134.62, 133.92 (2C), 130.00, 128.68, 128.11, 127.88 (2C), 126.19, 125.15, 117.50, 116.20, 109.09, 43.44, 35.73, 24.96. HRMS (ESI): m/z calcd. for C₂₂H₁₇N₄O₃S₂⁺ [M+H]⁺:449.0737, found: 449.0739.

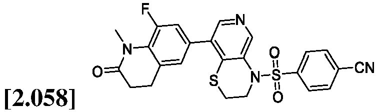

### 4-((8-(8-Fluoro-1-methyl-2-oxo-1,2,3,4-tetrahydroquinolin-6-yl)-2,3-dihydro-4H-pyrido[4,3-b][1,4]thiazin-4-yl)sulfonyl)benzonitrile (S52)

Following the general method SD, synthesized with **SI7** (100.0 mg, 0.25 mmol), (8-fluoro-1-methyl-2-oxo-1,2,3,4-tetrahydroquinolin-6-yl)boronic acid (85.13 mg, 0.28mmol), K₂CO₃ (138.2 mg, 1.00 mmol), PdCl₂(PPh₃)₂ (17.5 mg, 0.025 mmol), and Pcy₃ (14.0 mg, 0.05 mmol) in a mixture of dioxane (3 mL) and water (1 mL) to give **S52** (42.7 mg, 34.5% yield) as a white solid after chromatographic purification (DCM/MeOH = 50/1). mp: 226.0-227.0 °C. ¹H-NMR (400 MHz, Chloroform-*d*) *δ* 8.62 (s, 1H), 8.14 (s, 1H), 7.81 (s, 2H), 7.80 (s, 2H), 6.95 (d, *J* = 13.1 Hz, 1H), 6.87 (s, 1H), 3.99 (d, *J* = 5.7 Hz, 2H), 3.43 (s, 3H), 2.92 (s, 2H), 2.90 (s, 2H), 2.64 (s, 2H).¹³C-NMR (101 MHz, Chloroform-d) *δ* 170.96, 152.85 (¹*J*_{CF} = 250.4 Hz), 146.87, 146.54, 143.91, 138.14, 133.27 (2C), 133.18, 131.54, 131.26 (³*J*_{CF} = 8.0 Hz), 130.39, 129.53, 127.98 (2C), 123.94 (⁴*J*_{CF} = 2.0 Hz), 117.37, 117.06, 116.92 ( ²*J*_{CF} = 23.2 Hz), 43.83, 33.42, 31.87, 26.61, 26.14. HRMS (ESI): m/z calcd. for C₂₄H₂₀FN₄O₃S₂⁺ [M+H]⁺: 495.0955, found: 495.0951

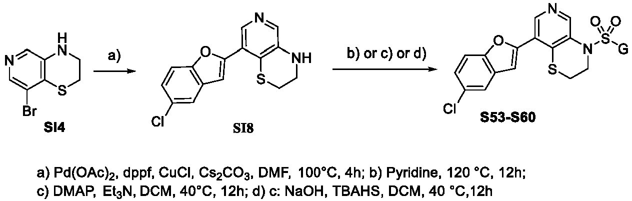

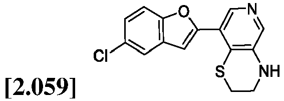

### 8-(5-Chlorobenzofuran-2-yl)-3,4-dihydro-2H-pyrido[4,3-b][1,4]thiazine (SI8)

Following the general method SI, synthesized with **SI4** (500 mg, 4.35 mmol), (5-chlorobenzofuran-2-yl)boronic acid (687.5 mg, 6.52 mmol), Pd(OAc)₂ (22.5 mg, 0.22 mmol), dppf (111 mg, 0.44 mmol), Cs₂CO₃ (2.606 g, 17.4 mmol) and CuCl (200 mg, 4.35 mmol) in anhydrous DMF (30 mL) to give **SI8** (300.0 mg, 46.0%yield) as a green solid after chromatographic purification (DCM/MeOH = 50/1). ¹H-NMR (400 MHz, Chloroform-d) δ 8.35 (s, 1H), 7.80 (s, 1H), 7.58 (d, *J* = 2.1 Hz, 1H), 7.45 (d, *J* = 8.7 Hz, 1H), 7.28 (d, *J* = 2.1 Hz, 1H), 7.13 (s, 1H), 4.28 (s, 1H), 3.71 (s, 2H), 3.16-3.10 (m, 2H).

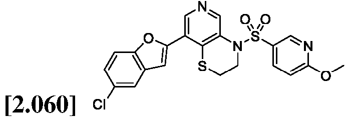

### 8-(5-Chlorobenzofuran-2-yl)-4-((6-methoxypyridin-3-yl)sulfonyl)-3,4-dihydro-2H-pyrido[4,3 -b][1,4]thiazine (S53)

Following the general method SE, synthesized with **SI8** (100.0 mg, 0.33 mmol), 6-methoxypyridine-3-sulfonyl chloride (297.4 mg, 1.32 mmol), DMAP (12.10 mg, 0.10 mmol) and Et₃N (101.19 mg, 1.00 mmol) in anhydrous dichloromethane (5 mL) to give **S53** (20.4 mg, 13.0% yield) as a light red solid after chromatographic purification (PE/EA = 1/1). ¹H-NMR (400 MHz, Chloroform-d) δ 8.79 (s, 1H), 8.58 (d, *J* = 2.5 Hz, 1H), 8.53 (s, 1H), 7.92 (dd, *J* = 8.7, 2.6 Hz, 1H), 7.64 (d, *J* = 2.1 Hz, 1H), 7.51 (d, *J* = 8.7 Hz, 1H), 7.36-7.30 (m, 1H), 6.96 (d, *J* = 8.7 Hz, 1H), 4.07 (s, 3H), 4.02 (s, 1H), 3.46-3.35 (m, 1H), 3.27 (t, *J* = 13.3 Hz, 1H), 3.06 (d, *J* = 14.4 Hz, 1H). HRMS (ESI): m/z calcd. for C₂₁H₁₆ClN₃O₄S₂⁺ [M+H]⁺: 474.0271, found 474.0333.

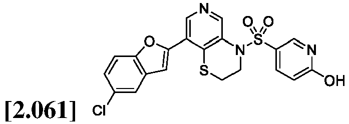

### 5-((8-(5-Chlorobenzofuran-2-yl)-2,3-dihydro-4H-pyrido[4,3-b][1,4]thiazin-4-yl)sulfonyl)pyri din-2-ol (S54)

To a solution of **S53** (50 mg, 0.10 mmol) in anhydrous dichloromethane (5 mL) was added BBr₃ (1M in DCM, 0.94 mL, 0.94 mmol) dropwise at -20 °C under N₂. The resulting mixture was warmed to room temperature and stirred for 12 h. After the reaction was complete as monitored by TLC, the mixture was neutralized with saturated Na₂CO₃ aq. to pH of around 7, and extracted with dichloromethane (3×5 mL) for three times. The combined organic layers were washed with brine (10 mL) and dried over anhydrous Na₂SO₄. The solvent was removed under reduced pressure to give the crude product, which was subsequently purified by silica gel column chromatography (DCM/MeOH = 20/1) to give **S54** (15.0 mg, 30.1% yield) as a white solid. ¹H-NMR (400 MHz, Chloroform-*d*) δ 8.82 (s, 1H), 8.61 (s, 1H), 7.89 (d, *J* = 2.6 Hz, 1H), 7.59 (d, *J* = 2.1 Hz, 1H), 7.47 (d, *J* = 2.7 Hz, 1H), 7.45 (d, *J* = 3.4 Hz, 1H), 7.31 (dd, *J* = 8.8, 2.2 Hz, 1H), 7.17 (s, 1H), 6.56 (d, *J* = 9.8 Hz, 1H), 4.01 (t, *J* = 5.7 Hz, 2H), 3.23 (t, *J* = 5.6 Hz, 2H). HRMS (ESI): m/z calcd. for C₂₀H₁₄ClN₃O₄S₂⁺ [M+H]⁺: 460.0187, found 460.0185.

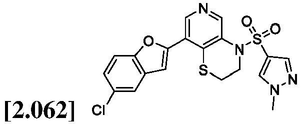

### 8-(5-Chlorobenzofuran-2-yl)-4-((1-methyl-1H-pyrazol-4-yl)sulfonyl)-3,4-dihydro-2H-pyrido[ 4,3-b][1,4]thiazine (S55)

Following the general method SC, synthesized with **SI8** (100.0 mg, 0.33 mmol) and 1-methyl-1*H*-pyrazole-4-sulfonyl chloride (216.73 mg, 1.32 mmol) in anhydrous pyridine (2 mL) to give **S55** (45.30 mg, 30.6% yield) as a white solid after chromatographic purification (PE/EA = 1/1). ¹H-NMR (400 MHz, Chloroform-d) δ 8.89 (s, 1H), 8.79 (s, 1H), 7.73 (d, *J* = 5.4 Hz, 2H), 7.67 (d, *J* = 2.1 Hz, 1H), 7.55 (d, *J* = 8.7 Hz, 1H), 7.39 (dd, *J* = 8.7, 2.2 Hz, 1H), 4.15-4.10 (m, 2H), 4.00 (s, 3H), 3.30-3.26 (m, 2H). ¹³C-NMR (101 MHz, Chloroform-d) δ 152.77, 151.64, 147.47, 146.05, 138.44, 137.14, 132.09, 130.83, 129.76, 128.90, 125.67, 123.34, 121.78, 120.97, 112.36, 107.68, 43.09, 39.80, 26.09. HRMS (ESI): m/z calcd. for C₁₉H₁₅ClN₄O₃S₂⁺ [M+H]⁺: 447.0347, found 447.0335.

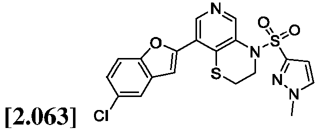

### 8-(5-Chlorobenzofuran-2-yl)-4-((1-methyl-1H-pyrazol-3-yl)sulfonyl)-3,4-dihydro-2H-pyrido[ 4,3-b][1,4]thiazine (S56)

Following the general method SC, synthesized with **SI8** (100.0 mg, 0.33 mmol) and 1-methyl-1*H*-pyrazole-3-sulfonyl chloride (216.73 mg, 1.32 mmol) in anhydrous pyridine (2 mL) to give **S56** (45.30 mg, 30.6% yield) as a white solid after chromatographic purification (PE/EA = 1/1). ¹H-NMR (400 MHz, Chloroform-*d*) δ 8.84 (d, *J* = 4.3 Hz, 2H), 7.67 (d, *J* = 2.1 Hz, 1H), 7.55 (d, *J* = 8.7 Hz, 1H), 7.48 (d, *J* = 2.3 Hz, 1H), 7.39 (dd, *J* = 8.8, 2.2 Hz, 1H), 6.60 (s, 1H), 4.20-4.15 (m, 2H), 4.06 (s, 3H), 3.47-3.43 (m, 2H). ¹³C-NMR (101 MHz, Chloroform-d) δ 152.74, 151.89, 149.34, 146.96, 145.82, 137.11, 132.23, 132.21, 129.84, 128.86, 125.58, 120.93, 112.36, 108.48, 108.47, 107.52, 43.22, 40.10, 26.69. HRMS (ESI): m/z calcd. for C₁₉H₁₅ClN₄O₃S₂⁺ [M+H]⁺: 447.0347, found 447.0339.

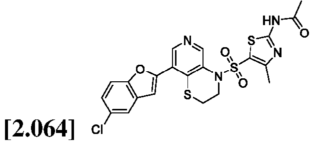

### N-(5-((8-(5-Chlorobenzofuran-2-yl)-2,3-dihydro-4H-pyrido[4,3-b][1,4]thiazin-4-yl)sulfonyl)-4-methylthiazol-2-yl)acetamide (S57)

Following the general method SC, synthesized with **SI8** (100.0 mg, 0.33 mmol) and 2-acetamido-4-methylthiazole-5-sulfonyl chloride (336.5 mg, 1.32 mmol) in anhydrous pyridine (2 mL) to give **S57** (25.70 mg, 14.5% yield) as a light yellow solid after chromatographic purification (PE/EA = 1/1). ¹H-NMR (400 MHz, DMSO-*d₆*) δ 12.73 (s, 1H), 8.76 (s, 1H), 8.54 (s, 1H), 7.81 (d, *J* = 2.3 Hz, 1H), 7.69 (d, *J* = 8.8 Hz, 1H), 7.41 (dd, *J* = 8.7, 2.3 Hz, 1H), 7.38 (s, 1H), 4.04 (t, *J* = 5.7 Hz, 2H), 3.17-3.13 (m, 2H), 2.17 (s, 3H), 2.14 (s, 3H). ¹³C-NMR (101 MHz, DMSO-*d₆*) δ 169.83, 160.31, 153.70, 152.40, 151.52, 147.92, 145.79, 138.47, 130.42, 129.66, 127.84,125.49, 122.58, 121.08, 120.46, 112.89, 107.67, 42.86, 25.56, 22.40, 15.96. HRMS (ESI): m/z calcd. for C₂₁H₁₇ClN₄O₄S₃⁺ [M+H]⁺: 521.0173, found 521.0169.

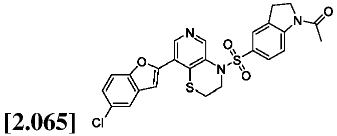

### 1-(5-((8-(5-Chlorobenzofuran-2-yl)-2,3-dihydro-4H-pyrido[4,3-b][1,4]thiazin-4-yl)sulfonyl)i ndolin-1-yl)ethan-1-one (S58)

Following the general method SJ, synthesized with **SI8** (100.0 mg, 0.33 mmol), 1-acetylindoline-5-sulfonyl chloride (340.15 mg, 1.32 mmol), NaOH (26.4 mg, 0.66 mmol) and TBAHS (6.80 mg, 0.02 mmol) in anhydrous dichloromethane (5 mL) to give **S58** (25.70 mg, 14.5% yield) as a white solid after chromatographic purification (PE/EA = 1/2). ¹H-NMR (400 MHz, DMSO-*d₆*) δ 8.71 (s, 1H), 8.54 (s, 1H), 8.14 (d, *J =* 8.3 Hz, 1H), 7.81 (d, *J* = 2.2 Hz, 1H), 7.69 (d, *J* = 8.8 Hz, 1H), 7.57-7.51 (m, 2H), 7.41 (dd, *J* = 8.8, 2.2 Hz, 1H), 7.37 (s, 1H), 4.15 (t, *J* = 8.4 Hz, 2H), 3.93 (t, *J* = 5.3 Hz, 2H), 3.15 (t, *J* = 8.9 Hz, 2H), 3.02 (t, *J* = 5.6 Hz, 2H), 2.19 (s, 3H). ¹³C-NMR (101 MHz, DMSO-*d₆*) δ 169.85, 152.38, 151.69, 147.26, 145.31, 137.52, 132.26, 129.68, 127.80, 127.70, 125.41, 121.06, 112.87, 107.54, 48.70, 42.31, 25.40, 24.11. HRMS (ESI): m/z calcd. for C₂₅H₂₀ClN₃O₄S₂⁺ [M+H]⁺: 526.0653, found 526.0628.

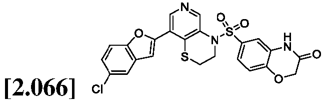

### 6-((8-(5-Chlorobenzofuran-2-yl)-2,3-dihydro-4H-pyrido[4,3-b][1,4]thiazin-4-yl)sulfonyl)-2H -benzo[b][1,4]oxazin-3(4H)-one (S59)

Following the general method SC, synthesized with **SI8** (100.0 mg, 0.33 mmol) and 3-oxo-3,4-dihydro-2H-benzo[b][1,4]oxazine-6-sulfonyl chloride (204.32 mg, 0.83 mmol) in anhydrous pyridine (2 mL) to give **S59** (17.6 mg, 10.4% yield) as a light yellow solid after chromatographic purification (PE/EA = 1/1). ¹H-NMR (400 MHz, DMSO-*d₆*) δ 10.89 (s, 1H), 8.73 (s, 1H), 8.54 (s, 1H), 7.81 (s, 1H), 7.68 (d, *J* = 8.8 Hz, 1H), 7.42 (s, 1H), 7.37 (s, 1H), 7.29 (d, *J* = 10.7 Hz, 1H), 7.19 (s, 1H), 7.12 (d, *J* = 8.5 Hz, 1H), 4.72 (s, 2H), 3.94 (s, 2H), 3.03 (s, 2H). ¹³C-NMR (101 MHz, DMSO-*d₆*) δ 163.90, 152.35, 151.65, 147.33, 147.14, 145.43, 137.63, 132.06, 129.68, 128.09, 127.82, 125.44, 122.68, 121.07, 117.11, 114.31, 112.84, 107.62, 66.69, 42.49, 25.49. HRMS (ESI): m/z calcd. for C₂₃H₁₆ClN₃O₅S₂⁺ [M+H]⁺: 514.0293, found 514.0278.

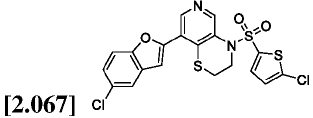

### 8-(5-Chlorobenzofuran-2-yl)-4-((5-chlorothiophen-2-yl)sulfonyl)-3,4-dihydro-2H-pyrido[4,3-b][1,4]thiazine (S60)

Following the general method SC, synthesized with **SI8** (100.0 mg, 0.33 mmol) and 5-chlorothiophene-2-sulfonyl chloride (286.56 mg, 1.32 mmol) in anhydrous pyridine (2 mL) to give **S60** (15.0 mg, 16.4% yield) as a light yellow solid after chromatographic purification (PE/EA = 5/1). ¹H-NMR (400 MHz, Chloroform-d) δ 8.93 (s, 1H), 8.81 (s, 1H), 7.67 (d, *J* = 2.1 Hz, 1H), 7.56 (d, *J =* 8.7 Hz, 1H), 7.41 (d, *J* = 2.1 Hz, 1H), 7.38 (d, *J* = 3.1 Hz, 1H), 7.37 (d, *J* = 3.2 Hz, 1H), 7.01 (d, *J* = 4.0 Hz, 1H), 4.13 (s, 2H), 3.25 (s, 2H). ¹³C-NMR (101 MHz, Chloroform-d) δ 147.49, 146.30, 137.49, 132.56, 127.23, 125.70, 120.96, 112.36, 107.77, 43.44, 26.10. HRMS (ESI): m/z calcd. for C₁₉H₁₂Cl₂N₂O₃S₃⁺ [M+H]⁺: 482.9460, found 482.9455.

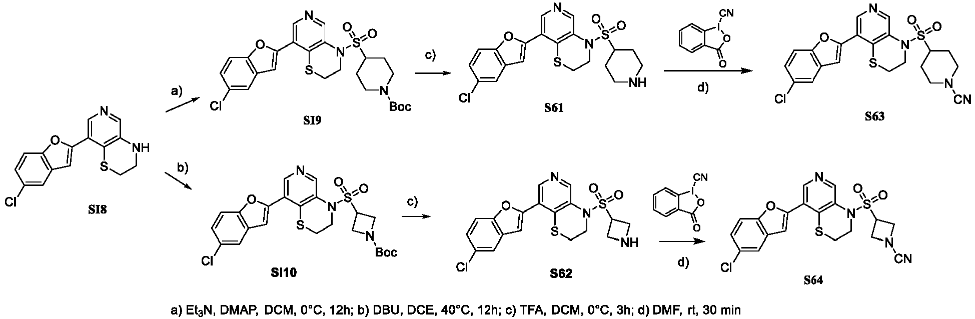

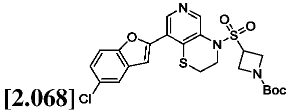

### tert-Butyl 3-((8-(5-chlorobenzofuran-2-yl)-2,3-dihydro-4H-pyrido[4,3-b][1,4]thiazin-4-yl)sulfonyl)azetidi ne-1-carboxylate (SI10)

Following the general method SE, synthesized with **SI8** (100.0 mg, 0.33 mmol), tert-butyl 3-(chlorosulfonyl)azetidine-1-carboxylate (337.5 mg, 1.32 mmol), DMAP (12.10 mg, 0.10 mmol) and Et₃N (101.19 mg, 1.00 mmol) in anhydrous dichloromethane (5 mL) to give **SI10** (19.8 mg, 12.1% yield) as a light yellow solid after chromatographic purification (PE/EA = 1/1). ¹H-NMR (400 MHz, Chloroform-d) δ 8.83 (s, 1H), 8.56 (s, 1H), 7.61 (d, *J* = 2.1 Hz, 1H), 7.48 (d, *J* = 8.7 Hz, 1H), 7.32 (dd, *J* = 8.7, 2.1 Hz, 1H), 7.22 (d, *J* = 0.9 Hz, 1H), 4.24 (dd, *J* = 9.1, 5.7 Hz, 2H), 4.15 (t, *J* = 8.6 Hz, 2H), 4.12-4.08 (m, 1H), 3.98 (t, *J* = 5.8 Hz, 2H), 3.35 (t, *J* = 5.8 Hz, 2H), 1.43 (s, 9H).

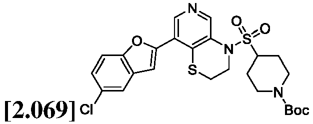

### tert-Butyl 4-((8-(5-chlorobenzofuran-2-yl)-2,3-dihydro-4H-pyrido[4,3-b][1,4]thiazin-4-yl)sulfonyl)piperi dine-1-carboxylate (SI9)

A solution of **SI8** ((100 mg, 0.33 mmol) and DBU (80 mg, 0.53 mmol) in anhydrous 1,2-dichloroethane (5 mL) was stirred at 0 °C for 5 min under N₂ before adding tert-butyl 4-(chlorosulfonyl)piperidine-1-carboxylate (235 mg, 0.83 mmol) dropwise at 0 °C. The resulting mixture was warmed to room temperature and stirred for 12 h. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA = 2:1) to give **SI9** (38.9 mg, 24.2%) as a light yellow solid. ¹H-NMR (400 MHz, Chloroform-*d*) δ 8.76 (s, 1H), 8.50 (s, 1H), 7.61 (d, *J* = 2.1 Hz, 1H), 7.50-7.46 (m, 1H), 7.32 (dd, *J* = 8.7, 2.1 Hz, 1H), 7.23 (d, *J* = 0.9 Hz, 1H), 4.30 (s, 2H), 3.95-3.90 (m, 2H), 3.41-3.37 (m, 2H), 3.31 (ddt, *J* = 11.8, 7.2, 3.7 Hz, 1H), 2.76 (s, 2H), 2.15 (d, *J* = 13.0 Hz, 2H), 1.86 (qd, *J* = 12.4, 4.6 Hz, 2H), 1.47 (s, 9H).

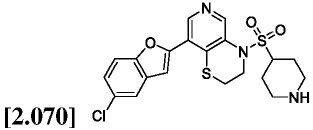

### 8-(5-Chlorobenzofuran-2-yl)-4-(piperidin-4-ylsulfonyl)-3,4-dihydro-2H-pyrido[4,3-b][1,4]thi azine (S61)

To a solution of **SI9** (50 mg, 0.09 mmol) in anhydrous dichloromethane (2 mL) was added trifluoroacetic acid (102.6 mg, 0.9 mmol) dropwise at 0 °C before further stirring of 3 h at the sam temperature. After the reaction was complete as monitored by TLC, it was quenched with water (5 mL) at 0 °C, adjusted pH to around 10 with saturated Na₂CO₃ aq. and extracted with dichloromethane (3×5 mL). The combined organic layers were washed with brine (5 mL) and dried over anhydrous sodium sulfate. It was then concentrated under reduced pressure to give the crude product, which was subsequently purified by column chromatography with an elution of CH₂Cl₂: MeOH (V/V) = 20:1, yielding the compound **S61** (15.0 mg, 37.0% yield) as a light yellow solid. ¹H-NMR (400 MHz, Chloroform-d) δ 8.74 (s, 1H), 8.50 (s, 1H), 7.61 (d, *J* = 2.2 Hz, 1H), 7.47 (d, *J* = 8.8 Hz, 1H), 7.31 (dd, *J* = 8.8, 2.2 Hz, 1H), 7.23 (s, 1H), 3.90 (s, 2H), 3.38 (s, 3H), 3.31 (d, *J =* 12.8 Hz, 1H), 3.25 (d, *J* = 3.7 Hz, 2H), 2.65 (s, 3H), 2.16 (d, *J =* 12.3 Hz, 3H), 1.90-1.78 (m, 3H). HRMS (ESI): m/z calcd. for C₂₀H₂₀ClN₃O₃S₂⁺ [M+H]⁺: 450.0707, found 450.0706.

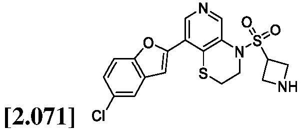

### 4-(Azetidin-3-ylsulfonyl)-8-(5-chlorobenzofuran-2-yl)-3,4-dihydro-2H-pyrido[4,3-b][1,4]thia zine (S62)

To a solution of **SI10** (50 mg, 0.09 mmol) in anhydrous dichloromethane (2 mL) was added trifluoroacetic acid (102.6 mg, 0.9 mmol) dropwise at 0 °C before further stirring of 3 h at the same temperature. After the reaction was complete as monitored by TLC, it was quenched with water (5 mL) at 0 °C, adjusted pH to around 10 with saturated Na₂CO₃ aq. and extracted with dichloromethane (3×5 mL). The combined organic layers were washed with brine (5 mL) and dried over anhydrous sodium sulfate. It was then concentrated under reduced pressure to give the crude product, which was subsequently purified by column chromatography with an elution of CH₂Cl₂: MeOH (V/V) = 20:1, yielding the compound **S62** (14.0 mg, 35.0% yield) as a light yellow solid. ¹H-NMR (400 MHz, Chloroform-*d*) δ 8.80 (s, 1H), 8.56 (s, 1H), 7.61 (d, *J* = 2.1 Hz, 1H), 7.48 (d, *J* = 8.7 Hz, 1H), 7.32 (dd, *J* = 8.7, 2.2 Hz, 1H), 7.21 (s, 1H), 4.30 (d, *J =* 7.7 Hz, 1H), 4.07 (s, 2H), 3.97-3.93 (m, 2H), 3.73 (s, 2H), 3.36-3.32 (m, 2H). HRMS (ESI): m/z calcd. for C₁₈H₁₆ClN₃O₃S₂⁺ [M+H]⁺: 422.0394, found 422.0392.

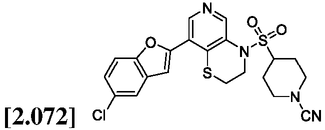

### 4-((8-(5-Chlorobenzofuran-2-yl)-2,3-dihydro-4H-pyrido[4,3-b][1,4]thiazin-4-yl)sulfonyl)pipe ridine-1-carbonitrile (S63)

To a solution of **S61** (50 mg, 0.11 mmol) in anhydrous DMF (2 mL) was added 3-oxo-1λ³-benzo[d][1,2]iodaoxole-1(3*H*)-carbonitrile (27.3 mg, 0.11 mmol). The reaction mixture was stirred at room temperature for 30 min. After the reaction was complete as monitored by TLC, the reaction mixture was diluted with water (4 mL) and extracted with ethyl acetate (3×5 mL). The combined organic layers were washed with water (2×5 mL) and brine (2×5 mL) and dried over anhydrous Na₂SO₄. The solvent was removed under reduced pressure to give the crude product, which was subsequently purified by silica gel column chromatography (PE/EA) to yield the target compound **S63** (26.8 mg, 51.5%) as a light yellow solid. ¹H-NMR (400 MHz, Chloroform-*d*) δ 8.78 (s, 1H), 8.49 (s, 1H), 7.61 (d, *J* = 2.1 Hz, 1H), 7.48 (d, *J* = 8.8 Hz, 1H), 7.32 (dd, *J* = 8.7, 2.1 Hz, 1H), 7.24 (s, 1H), 3.96-3.91 (m, 2H), 3.63 (d, *J* = 13.0 Hz, 2H), 3.42-3.37 (m, 2H), 3.27 (t, *J* = 11.5 Hz, 1H), 3.15-3.05 (m, 2H), 2.23 (d, *J* = 12.5 Hz, 2H), 2.14-2.04 (m, 2H). HRMS (ESI): m/z calcd. for C₂₁H₂₀ClN₄O₃S₂ [M+H]⁺: 475.0660, found: 475.0653.

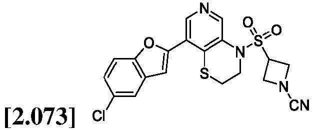

### 3-((8-(5-Chlorobenzofuran-2-yl)-2,3-dihydro-4H-pyrido[4,3-b][1,4]thiazin-4-yl)sulfonyl)azet idine-1-carbonitrile (S64)

To a solution of **S62** (50 mg, 0.11 mmol) in anhydrous DMF (2 mL) was added 3-oxo-1λ³-benzo[d][1,2]iodaoxole-1(3*H*)-carbonitrile (27.3 mg, 0.11 mmol). The reaction mixture was stirred at room temperature for 30 min. After the reaction was complete as monitored by TLC, the reaction mixture was diluted with water (4 mL) and extracted with ethyl acetate (3×5 mL). The combined organic layers were washed with water (2×5 mL) and brine (2×5 mL) and dried over anhydrous Na₂SO₄. The solvent was removed under reduced pressure to give the crude product, which was subsequently purified by silica gel column chromatography (PE/EA) to yield the target compound **S64** (28.6mg, 55.0%) as a light yellow solid. ¹H-NMR (400 MHz, Chloroform-d) δ 8.86 (s, 1H), 8.55 (s, 1H), 7.62 (d, *J* = 2.1 Hz, 1H), 7.49 (d, *J* = 8.7 Hz, 1H), 7.33 (dd, *J* = 8.7, 2.1 Hz, 1H), 7.22 (s, 1H), 4.51-4.44 (m, 2H), 4.35 (t, *J* = 8.4 Hz, 2H), 4.26-4.18 (m, 1H), 4.02-3.97 (m, 2H), 3.34 (t, *J* = 5.9 Hz, 2H). HRMS (ESI): m/z calcd. for C₁₉H₁₆ClN₄O₃S₂ [M+H]⁺: 447.0347, found: 447.0346.

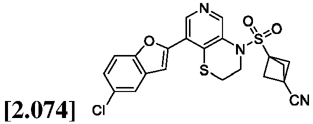

### 3-((8-(5-Chlorobenzofuran-2-yl)-2,3-dihydro-4H-pyrido[4,3-b][1,4]thiazin-4-yl)sulfonyl)b icyclo[1.1.1]pentane-1-carbonitrile (S65)

Following the general method SJ, synthesized with **SI8** (100.0 mg, 0.33 mmol), 3-cyanobicyclo[1.1.1]pentane-1-sulfonyl chloride (126 mg, 0.66 mmol), NaOH (26.4 mg, 0.66 mmol) and TBAHS (6.80 mg, 0.02 mmol) in anhydrous dichloromethane (5 mL) to give **S65** (38.0 mg, 25.2% yield) as a white solid after chromatographic purification (PE/EA = 1/2). ¹H-NMR (400 MHz, Chloroform-*d*) δ 8.33 (s, 1H), 7.96 (s, 1H), 7.53 (d, *J* = 2.1 Hz, 1H), 7.49 (d, *J* = 8.7 Hz, 1H), 7.28 (d, *J* = 2.1 Hz, 1H), 7.15 (s, 1H), 3.45-3.39 (m, 2H), 3.16-3.10 (m, 2H), 2.67-2.59 (m, 4H), 2.22-2.18 (m, 2H). HRMS (ESI): m/z calcd. for C₂₁H₁₇ClN₃O₃S₂ [M+H]⁺: 458.0394, found: 458.0401.

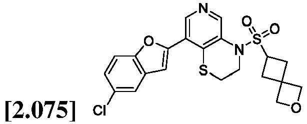

### 4-((2-Oxaspiro[3.3]heptan-6-yl)sulfonyl)-8-(5-chlorobenzofuran-2-yl)-3,4-dihydro-2H-pyr ido[4,3-b][1,4]thiazine (S66)

Following the general method SJ, synthesized with **SI8** (100.0 mg, 0.33 mmol), 2-oxaspiro[3.3]heptane-6-sulfonyl chloride (97.3 mg, 0.50 mmol), NaOH (26.4 mg, 0.66 mmol) and TBAHS (6.80 mg, 0.02 mmol) in anhydrous dichloromethane (5 mL) to give **S66** (18.0 mg, 12.2% yield) as a white solid after chromatographic purification (PE/EA = 1/2). ¹H-NMR (400 MHz, Chloroform-*d*) δ 8.25 (s, 1H), 8.02 (s, 1H), 7.55 (d, *J* = 2.1 Hz, 1H), 7.39 (d, *J* = 8.7 Hz, 1H), 7.28 (d, *J* = 2.1 Hz, 1H), 7.16 (s, 1H), 4.61 (s, 4H), 3.48-3.53 (m, 3H), 2.16-2.21 (m, 2H) 1.93-1.99 (m, 2H). HRMS (ESI): m/z calcd. for C₂₁H₂₀ClN₂O₄S₂ [M+H]⁺: 463.0548, found: 463.0539.

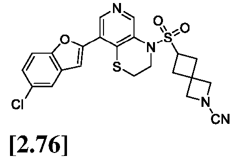

### 6-((8-(5-chlorobenzofuran-2-yl)-2,3-dihydro-4H-pyrido[4,3-b][1,4]thiazin-4-yl)sulfonyl)-2-azas piro[3.3]heptane-2-carbonitrile (S67)

Following the general method SJ, synthesized with **SI8** (100.0 mg, 0.33 mmol), 2-cyano-2-azaspiro[3.3]heptane-6-sulfonyl chloride (145.6 mg, 0.66 mmol), NaOH (26.4 mg, 0.66 mmol) and TBAHS (6.80 mg, 0.02 mmol) in anhydrous dichloromethane (5 mL) to give **S67** (14.5 mg, 9.1% yield) as a white solid after chromatographic purification (PE/EA = 1/2). ¹H-NMR (400 MHz, Chloroform-*d*) δ 8.28 (s, 1H), 8.00 (s, 1H), 7.53 (d, *J* = 2.1 Hz, 1H), 7.42 (d, *J* = 8.7 Hz, 1H), 7.31 (d, *J* = 2.1 Hz, 1H), 7.19 (s, 1H), 3.45-3.52 (m, 7H), 3.16-3.18 (m, 2h), 2.16-2.22 (m, 2H) 1.91-1.95 (m, 2H). HRMS (ESI): m/z calcd. for C₂₂H₂₀ClN₄O₃S₂ [M+H]⁺: 487.0660, found: 487.0671.

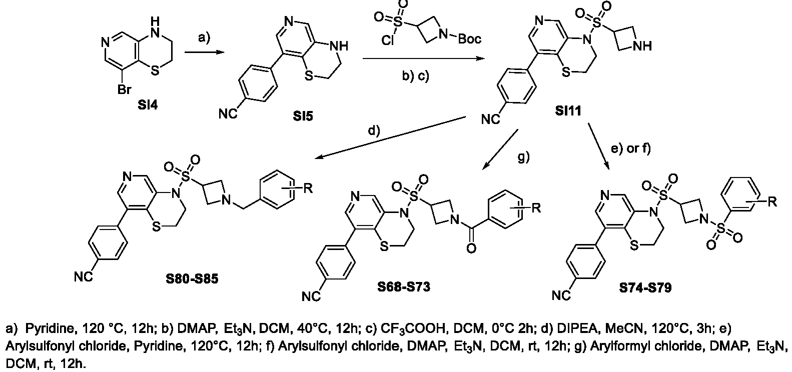

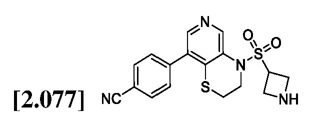

### 4-(4-(azetidin-3-ylsulfonyl)-3,4-dihydro-2H-pyrido[4,3-b][1,4]thiazin-8-yl)benzonitrile (SI11)

Following the general method SE, synthesized with **SI5** (100.0 mg, 0.39 mmol), tert-butyl 3-(chlorosulfonyl)azetidine-1-carboxylate (149.6 mg, 0.59 mmol), DMAP (12.10 mg, 0.10 mmol) and Et₃N (0.16 mL, 1.2 mmol) in anhydrous dichloromethane (5 mL) to give tert-butyl 3-((8-(4-cyanophenyl)-2,3-dihydro-4H-pyrido[4,3-b][1,4]thiazin-4-yl)sulfonyl)azetidine-1-carboxyl ate (146.7 mg, 79.6% yield) as a light yellow oil after chromatographic purification (DCM/MeOH = 50/1). ¹H-NMR (400 MHz, Chloroform-*d*): *δ* 8.71 (s, 1H), 8.27 (s, 1H), 7.76-7.71 (m, 2H), 7.63-7.58 (m, 2H), 4.41-4.37 (m, 2H), 4.31 (dd, *J* = 9.3, 5.7 Hz, 2H), 4.23 (q, *J* = 3.4, 1.8 Hz, 3H), 3.92 (t, *J* = 4.5 Hz, 2H), 1.45 (s, 9H).

To a solution of the resulting intermediate (100.0 mg, 0.21 mmol) in anhydrous dichloromethane (4 mL) was added trifluoroacetic acid (0.16 mL, 2.1 mmol) dropwise at 0 °C before further stirring of 3 h at the same temperature. After the reaction was complete as monitored by TLC, it was quenched with water (5 mL) at 0 °C, adjusted pH to around 10 with saturated Na₂CO₃ aq. and extracted with dichloromethane (3×5 mL). The combined organic layers were washed with brine (5 mL) and dried over anhydrous sodium sulfate. It was then concentrated under reduced pressure to give the crude product, which was subsequently purified by column chromatography with an elution of CH₂Cl₂: MeOH (V/V) = 20:1, yielding the compound **SI11** (64.4 mg, 82.1%yield) as a light yellow solid. ¹H-NMR (400 MHz, DMSO-d6): δ 8.64 (s, 1H), 8.25 (s, 1H), 7.95-7.91 (m, 2H), 7.80-7.76 (m, 2H), 4.79 (p, *J* = 7.5 Hz, 1H), 4.34 (dd, *J* = 5.2, 3.8 Hz, 2H), 3.85 (t, *J* = 4.5 Hz, 3H), 3.80 (s, 2H), 3.68 (d, *J* = 8.5 Hz, 2H).

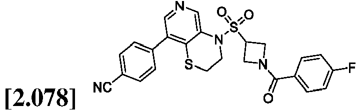

### 4-(4-((1-(4-Fluorobenzoyl)azetidin-3-yl)sulfonyl)-3,4-dihydro-2H-pyrido[4,3-b][1,4]thiazin-8 -yl)benzonitrile (S68)

Following the general method SB, synthesized with **SI11** (50.0 mg, 0.13 mmol), 4-fluorobenzoyl chloride ((30.9 mg, 0.2 mmol), DMAP (14.5 mg, 0.12 mmol), Et₃N (0.05 mL, 0.39 mmol) in anhydrous dichloromethane (6 mL) to give **S68** (56.2 mg, 87.4% yield) as a white solid after chromatographic purification (PE/EA = 5/1). mp: 226.0-227.0 °C. ¹H-NMR (400 MHz, Chloroform-d) δ 8.57 (s, 1H), 8.17 (s, 1H), 7.77 (d, *J* = 8.4 Hz, 2H), 7.63 (dd, *J* = 8.8, 5.3 Hz, 2H), 7.52 (d, *J* = 8.4 Hz, 2H), 7.12 (t, *J* = 8.6 Hz, 2H), 4.55 (d, *J* = 53.6 Hz, 4H), 4.25 (d, *J* = 7.2 Hz, 1H), 4.00 (s, 2H), 3.24 (s, 2H).¹³C-NMR (101 MHz, Chloroform-d) δ 169.68, 166.11 (¹*J*_{CF} = 254.5 Hz), 146.66, 146.40, 140.02, 138.06, 133.66, 132.64 (2C), 130.84, 130.48 (³*J*_{CF} = 9.0 Hz), 130.39, 130.32 (2C), 128.08 (⁴*J*_{CF} = 3.0 Hz), 118.42, 116.13 (²*J*_{CF} = 22.2Hz), 115.91, 112.99, 54.30, 50.79, 44.22 (2C), 28.48. HRMS (ESI): m/z calcd. for C₂₄H₂₀FN₄O₃S₂⁺ [M+H]⁺: 495.0955, found: 495.0950.

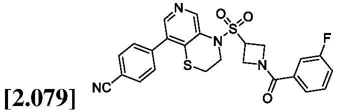

### 4-(4-((1-(3-Fluorobenzoyl)azetidin-3-yl)sulfonyl)-3,4-dihydro-2H-pyrido[4,3-b][1,4]thiazin-8 -yl)benzonitrile (S69)

Following the general method SB, synthesized with **SI11** (50.0 mg, 0.13 mmol), 3-fluorobenzoyl chloride (30.9 mg, 0.2 mmol), DMAP (14.5 mg, 0.12 mmol), Et₃N (0.05 mL, 0.39 mmol) in anhydrous dichloromethane (6 mL) to give **S69** (55.1 mg, 86.7% yield) as a white solid after chromatographic purification (PE/EA = 5/1). mp: 204.0-205.0 °C. ¹H-NMR (400 MHz, Chloroform-*d*) δ 8.58 (s, 1H), 8.18 (s, 1H), 7.77 (d, *J* = 8.3 Hz, 2H), 7.52 (d, *J* = 8.3 Hz, 2H), 7.43 (s, 1H), 7.38 (s, 1H), 7.34-7.29 (m, 1H), 7.24-7.18 (m, 1H), 4.47 (s, 4H), 4.29-4.20 (m, 1H), 3.96 (d, *J* = 31.3 Hz, 2H), 3.25 (s, 2H). ¹³C-NMR (101 MHz, Chloroform-*d*) δ 169.28, 163.98 (¹*J*_{CF} *=* 249.4Hz), 161.51, 146.69, 146.44, 140.02, 134.07, 133.67 (³*J*_{CF} = 8.0Hz), 132.65 (2C), 130.85, 130.58, 130.33 (2C), 123.64 (⁴*J*_{CF} = 3.0Hz), 119.15, 118.94,115.34 (²*J*_{CF} = 23.2Hz), 113.02, 54.24, 50.80, 44.25 (2C), 28.50. HRMS (ESI): m/z calcd. for C₂₄H₂₀FN₄O₃S₂⁺ [M+H]⁺: 495.0955, found: 495.0955.

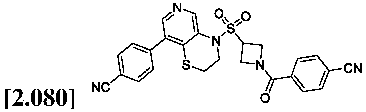

### 4-(4-((1-(4-Cyanobenzoyl)azetidin-3-yl)sulfonyl)-3,4-dihydro-2H-pyrido[4,3-b][1,4]thiazin-8 -yl)benzonitrile (S70)

Following the general method SB, synthesized with **SI11** (50.0 mg, 0.13 mmol), 4-cyanobenzoyl chloride (33.1 mg, 0.2 mmol), DMAP (14.5 mg, 0.12 mmol), Et₃N (0.05 mL, 0.39 mmol) in anhydrous dichloromethane (6 mL) to give **S70** (53.2 mg, 81.4% yield) as a white solid after chromatographic purification (PE/EA = 5/1). mp: 124.0-125.0 °C. ¹H-NMR (400 MHz, Chloroform-d) δ 8.56 (s, 1H), 8.18 (s, 1H), 7.74 (dt, *J* = 16.3, 8.4 Hz, 6H), 7.52 (d, *J* = 8.3 Hz, 2H), 4.55 (d, *J* = 60.0 Hz, 4H), 4.26 (s, 1H), 3.97 (d, *J* = 50.7 Hz, 2H), 3.25 (s, 2H). ¹³C-NMR (101 MHz, Chloroform-*d*) δ 168.65, 146.67, 146.53, 139.96, 138.12, 135.97, 133.71, 132.69 (2C), 132.66 (2C), 130.76, 130.31 (2C), 128.62 (2C), 118.40, 117.90, 115.59, 113.05, 54.17,50.71, 44.29 (2C), 28.50. HRMS (ESI): m/z calcd. for C₂₅H₂₀N₅O₃S₂⁺ [M+H]⁺: 502.1002, found: 502.1003.

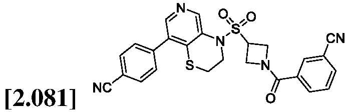

### 3-(3-((8-(4-Cyanophenyl)-2,3-dihydro-4H-pyrido[4,3-b][1,4]thiazin-4-yl)sulfonyl)azetidine-1 -carbonyl)benzonitrile (S71)

Following the general method SB, synthesized with **SI11** (50.0 mg, 0.13 mmol), 3-cyanobenzoyl chloride (33.1 mg, 0.2 mmol), DMAP (14.5 mg, 0.12 mmol), Et₃N (0.05 mL, 0.39 mmol) in anhydrous dichloromethane (6 mL) to give **S71** (54.1 mg, 82.9% yield) as a white solid after chromatographic purification (PE/EA = 5/1). mp: 118.0-119.0 °C. ¹H-NMR (400 MHz, Chloroform-d) δ 8.56 (s, 1H), 8.17 (s, 1H), 7.90-7.82 (m, 2H), 7.82-7.73 (m, 3H), 7.62-7.55 (m, 1H), 7.52 (d, *J* = 8.4 Hz, 2H), 4.57 (d, *J* = 67.1 Hz, 4H), 4.27 (s, 1H), 3.98 (d, *J* = 36.3 Hz, 2H), 3.25 (s, 2H). ¹³C-NMR (101 MHz, Chloroform-*d*) δ 168.19, 146.66, 146.48, 139.96, 138.12, 135.10, 133.67, 133.27, 132.64 (2C), 132.15, 131.54, 130.76, 130.30 (2C), 129.91, 118.41, 117.85, 113.30, 112.97, 54.27,50.69, 44.28 (2C), 28.49. HRMS (ESI): m/z calcd. for C₂₅H₂₀N_{S}O₃S₂⁺ [M+H]⁺: 502.1002, found: 502.1002.

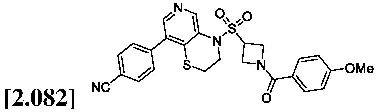

### 4-(4-((1-(4-Methoxybenzoyl)azetidin-3-yl)sulfonyl)-3,4-dihydro-2H-pyrido[4,3-b][1,4]thiazin -8-yl)benzonitrile (S72)

Following the general method SB, synthesized with **SI11** (50.0 mg, 0.13 mmol), 4-methoxybenzoyl chloride (34.1 mg, 0.2 mmol), DMAP (14.5 mg, 0.12 mmol), Et₃N (0.05 mL, 0.39 mmol) in anhydrous dichloromethane (6 mL) to give **S72** (55.8 mg, 84.7% yield) as a white solid after chromatographic purification (PE/EA = 5/1). mp: 144.0-145.0 °C. ¹H-NMR (400 MHz, Chloroform-*d*) *δ* 8.59 (s, 1H), 8.17 (s, 1H), 7.77 (d, *J* = 8.3 Hz, 2H), 7.59 (d, *J* = 8.8 Hz, 2H), 7.52 (d, *J* = 8.4 Hz, 2H), 6.92 (d, *J* = 8.8 Hz, 2H), 4.55 (s, 4H), 4.24 (s, 1H), 3.95 (s, 2H), 3.85 (s, 3H), 3.24 (s, 2H). ¹³C-NMR (101 MHz, Chloroform-*d*) *δ* 170.50, 162.51, 146.69, 146.35, 140.08, 138.01, 132.64 (2C), 130.35 (2C), 130.06 (2C), 124.14, 118.45, 114.07 (2C), 113.00, 55.58, 54.17, 50.95, 44.19 (2C), 28.49. HRMS (ESI): m/z calcd. for C₂₅H₂₃N₄O₄S₂⁺ [M+H]⁺: 507.1155, found: 507.1155.

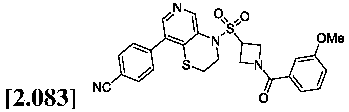

### 4-(4-((1-(3-Methoxybenzoyl)azetidin-3-yl)sulfonyl)-3,4-dihydro-2H-pyrido[4,3-b][1,4]thiazin -8-yl)benzonitrile (S73)

Following the general method SB, synthesized with **SI11** (50.0 mg, 0.13 mmol), 3-methoxybenzoyl chloride (34.1 mg, 0.2 mmol), DMAP (14.5 mg, 0.12 mmol), Et₃N (0.05 mL, 0.39 mmol) in anhydrous dichloromethane (6 mL) to give **S73** (57.1 mg, 86.8% yield) as a white solid after chromatographic purification (PE/EA = 5/1). mp: 136.0-137.0 °C. ¹H-NMR (400 MHz, Chloroform-*d*) *δ* 8.58 (s, 1H), 8.17 (s, 1H), 7.76 (s, 2H), 7.52 (d, *J* = 8.3 Hz, 2H), 7.33 (s, 1H), 7.16 (s, 1H), 7.12 (d, *J* = 7.6 Hz, 1H), 7.04 (d, *J* = 7.3 Hz, 1H), 4.46 (s, 4H), 4.23 (s, 1H), 3.97 (d, *J* = 37.8 Hz, 2H), 3.84 (s, 3H), 3.24 (s, 2H). ¹³C-NMR (101 MHz, Chloroform-*d*) *δ* 170.47, 159.83, 146.59, 146.28, 139.94, 137.88, 133.52, 133.10, 132.52 (2C), 130.77, 130.22 (2C), 129.70, 119.88, 118.31, 117.90, 113.06, 112.88, 55.47, 54.17, 50.74, 44.09 (2C), 28.36. HRMS (ESI): m/z calcd. for C₂₅H₂₃N₄O₄S₂⁺ [M+H]⁺: 507.1155, found: 507.1150.

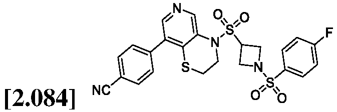

### 4-(4-((1-((4-Fluorophenyl)sulfonyl)azetidin-3-yl)sulfonyl)-3,4-dihydro-2H-pyrido[4,3-b][1,4] thiazin-8-yl)benzonitrile (S74)

Following the general method SE, synthesized with **SI11** (50.0 mg, 0.13 mmol), DMAP (4.7 mg, 0.04 mmol), Et₃N (0.05 mL, 0.39 mmol), 4-fluorobenzenesulfonyl chloride (35.6 mg, 0.2 mmol) in anhydrous dichloromethane (5 mL) to give **S74** (49.9 mg, 71.9% yield) as a white solid after chromatographic purification (PE/EA = 5/1). mp: 206.0-207.0 °C. ¹H-NMR (400 MHz, Chloroform-d) δ 8.43 (s, 1H), 8.17 (s, 1H), 7.84 (d, *J* = 3.9 Hz, 2H), 7.78 (d, *J* = 8.4 Hz, 2H), 7.51 (d, *J* = 8.3 Hz, 2H), 7.30 (t, *J* = 8.5 Hz, 2H), 4.11 (d, *J* = 7.0 Hz, 2H), 4.05 (d, *J* = 6.9 Hz, 1H), 4.00 (d, *J* = 7.2 Hz, 2H), 3.88-3.81 (m, 2H), 3.21-3.13 (m, 2H). ¹³C-NMR (101 MHz, Chloroform-*d*) δ 167.38 (¹*J*_{CF} = 258.5 Hz), 164.82, 146.70, 146.49, 139.90, 138.00, 133.65, 132.67 (2C), 131.31 (³*J*_{CF} = 9.0 Hz), 131.22, 130.17 (2C), 118.40, 117.14 (²*J*_{CF} = 23.2 Hz), 116.91, 113.08, 51.97, 49.32, 44.08 (2C), 28.27. HRMS (ESI): m/z calcd. for C₂₃H₂₀FN₄O₄S₃⁺ [M+H]⁺: 531.0625, found: 531.0623.

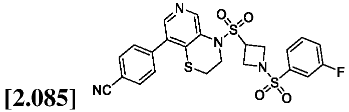

### 4-(4-((1-((3-Fluorophenyl)sulfonyl)azetidin-3-yl)sulfonyl)-3,4-dihydro-2H-pyrido[4,3-b][1,4] thiazin-8-yl)benzonitrile (S75)

Following the general method SE, synthesized with **SI11** (50.0 mg, 0.13 mmol), DMAP (4.7 mg, 0.04 mmol), Et₃N (0.05 mL, 0.39 mmol), 3-fluorobenzenesulfonyl chloride (35.6 mg, 0.2 mmol) in anhydrous dichloromethane (5 mL) to give **S75** (48.2 mg, 70.6% yield) as a white solid after chromatographic purification (PE/EA = 5/1). mp: 199.0-200.0 °C. ¹H-NMR (400 MHz, Chloroform-d) δ 8.43 (s, 1H), 8.22 (s, 1H), 7.77 (d, *J* = 8.3 Hz, 2H), 7.61 (s, 2H), 7.54 (t, *J* = 2.0 Hz, 1H), 7.51 (d, *J =* 8.3 Hz, 2H), 7.41 (s, 1H), 4.14 (s, 2H), 4.09 (s, 1H), 4.05 (s, 2H), 3.85 (s, 2H), 3.18 (s, 2H). ¹³C-NMR (101 MHz, Chloroform-d) δ 164.10 (¹*J*_{CF} = 254.5 Hz),161.58, 146.66, 139.92, 137.99, 136.16, 132.67 (2C), 131.55 (³*J*_{CF} = 7.0 Hz),130.32 (2C),124.22 (⁴*J*_{CF} = 3.0 Hz), 121.47, 121.26, 118.41, 115.88 (²*J*_{CF} = 24.2 Hz), 113.06, 52.11, 49.24 (2C), 44.10, 28.30. HRMS (ESI): m/z calcd. for C₂₃H₂₀FN₄O₄S₃⁺ [M+H]⁺:531.0625, found: 531.0623.

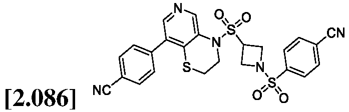

### 4-((3-((8-(4-Cyanophenyl)-2,3-dihydro-4H-pyrido[4,3-b][1,4]thiazin-4-yl)sulfonyl)azetidin-1-yl)sulfonyl)benzonitrile (S76)

Following the general method SE, synthesized with **SI11** (50.0 mg, 0.13 mmol), DMAP (4.7 mg, 0.04 mmol), Et₃N (0.05 mL, 0.39 mmol), 4-cynobenzenesulfonyl chloride (40.3 mg, 0.2 mmol) in anhydrous dichloromethane (5 mL) to give **S76** (52.7 mg, 52.7% yield) as a white solid after chromatographic purification (PE/EA = 5/1). mp: 229.0-230.0 °C. ¹H-NMR (400 MHz, DMSO-*d₆*) δ 8.29 (s, 1H), 8.19 (d, *J* = 8.4 Hz, 2H), 8.15 (s, 1H), 8.04 (d, *J* = 8.3 Hz, 2H), 7.98 (d, *J* = 8.2 Hz, 2H), 7.66 (d, *J =* 8.3 Hz, 2H), 4.72 (s, 1H), 4.20 (s, 2H), 3.96 (s, 2H), 3.78 (s, 2H), 3.19 (s, 2H). ¹³C-NMR (101 MHz, DMSO-*d₆*) δ 145.34, 140.16, 137.64, 137.45, 133.72 (2C), 132.92, 132.54 (2C), 130.46 (2C), 130.40, 129.05 (2C), 118.52, 117.64, 116.21, 111.43, 51.93, 48.16, 43.38 (2C), 27.56. HRMS (ESI): m/z calcd. for C₂₄H₂₀N₅O₄S₃⁺ [M+H]⁺: 538.0672, found: 538.0672.

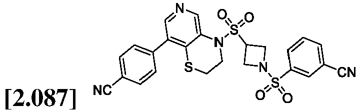

### 3-((3-((8-(4-Cyanophenyl)-2,3-dihydro-4H-pyrido[4,3-b][1,4]thiazin-4-yl)sulfonyl)azetidin-1-yl)sulfonyl)benzonitrile (S77)

Following the general method SE, synthesized with **SI11** (50.0 mg, 0.13 mmol), DMAP (4.7 mg, 0.04 mmol), Et₃N (0.05 mL, 0.39 mmol), 3-cynobenzenesulfonyl chloride (40.3 mg, 0.2 mmol) in anhydrous dichloromethane (5 mL) to give **S77** (54.5 mg, 78.0% yield) as a white solid after chromatographic purification (PE/EA = 5/1). mp: 205.0-206.0 °C. ¹H-NMR (400 MHz, Chloroform-*d*) *δ* 8.41 (s, 1H), 8.19 (s, 1H), 8.11 (s, 1H), 8.02 (s, 1H), 7.96 (s, 1H), 7.80 (d, *J* = 4.6 Hz, 1H), 7.77 (s, 2H), 7.51 (d, *J* = 8.3 Hz, 2H), 4.21 (s, 2H), 4.08 (t, *J* = 7.5 Hz, 1H), 4.04 (d, *J* = 7.9 Hz, 2H), 3.86 (s, 2H), 3.18 (s, 2H).¹³C-NMR (101 MHz, Chloroform-d) δ 146.66, 139.84, 138.13, 137.01, 136.55, 132.69 (2C), 132.13, 131.81, 130.73, 130.31 (2C), 118.39, 117.10, 114.37, 113.12, 52.22, 49.17, 44.19 (2C), 28.37. HRMS (ESI): m/z calcd. for C₂₄H₂₀N₅O₄S₃⁺ [M+H]⁺: 538.0672, found: 538.0674.

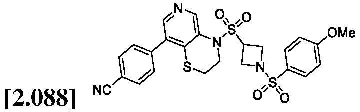

### 4-(4-((1-((4-Methoxyphenyl)sulfonyl)azetidin-3-yl)sulfonyl)-3,4-dihydro-2H-pyrido[4,3-b][1, 4]thiazin-8-yl)benzonitrile (S78)

Following the general method SE, synthesized with **SI11** (50.0 mg, 0.13 mmol), DMAP (4.7 mg, 0.04 mmol), Et₃N (0.05 mL, 0.39 mmol), 4-methoxybenzenesulfonyl chloride (41.3 mg, 0.2 mmol) in anhydrous dichloromethane (5 mL) to give **S78** (55.8 mg, 79.1% yield) as a white solid after chromatographic purification (PE/EA = 5/1). mp: 139.0-140.0 °C. ¹H-NMR (400 MHz, Chloroform-*d*) *δ* 8.43 (s, 1H), 8.16 (s, 1H), 7.78 (d, *J* = 6.9 Hz, 2H), 7.75 (d, *J* = 7.5 Hz, 2H), 7.51 (d, *J* = 8.3 Hz, 2H), 7.07 (d, *J* = 8.9 Hz, 2H), 4.05 (s, 2H), 4.03 (s, 1H), 4.00 (d, *J* = 5.6 Hz, 2H), 3.91 (s, 3H), 3.87-3.81 (m, 2H), 3.20-3.14 (m, 2H). ¹³C-NMR (101 MHz, Chloroform-d) δ 164.12, 146.70, 146.36, 139.97, 137.88, 132.67 (2C), 130.76 (2C), 130.57, 130.34 (2C), 125.23, 118.43, 114.84 (2C), 113.05, 55.91, 51.80, 49.45, 44.01 (2C), 28.24. HRMS (ESI): m/z calcd. for C₂₄H₂₃N₄O₅S₃⁺ [M+H]⁺:543.0825, found: 543.0823.

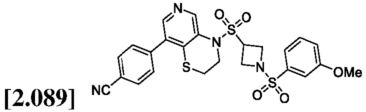

### 4-(4-((1-((3-Methoxyphenyl)sulfonyl)azetidin-3-yl)sulfonyl)-3,4-dihydro-2H-pyrido[4,3-b][1, 4]thiazin-8-yl)benzonitrile (S79)

Following the general method SE, synthesized with **SI11** (50.0 mg, 0.13 mmol), DMAP (4.7 mg, 0.04 mmol), Et₃N (0.05 mL, 0.39 mmol), 3-methoxybenzenesulfonyl chloride (41.3 mg, 0.2 mmol) in anhydrous dichloromethane (5 mL) to give **S79** (53.2 mg, 75.5% yield) as a white solid after chromatographic purification (PE/EA = 5/1). mp: 115.0-116.0 °C. ¹H-NMR (400 MHz, Chloroform-*d*) *δ* 8.42 (s, 1H), 8.16 (s, 1H), 7.77 (d, *J* = 7.8 Hz, 2H), 7.55 (s, 1H), 7.51 (d, *J* = 8.1 Hz, 2H), 7.39 (d, *J* = 8.3 Hz, 1H), 7.31 (s, 1H), 7.22 (d, *J* = 5.7 Hz, 1H), 4.10 (s, 2H), 4.07 (s, 1H), 4.05 (s, 2H), 3.89 (s, 3H), 3.84 (s, 2H), 3.17 (s, 2H). ¹³C-NMR (101 MHz, Chloroform-d) *δ* 160.44, 146.66, 146.37, 139.95, 137.94, 134.94, 132.66 (2C), 130.69, 130.56, 130.33 (2C), 120.61, 120.39, 118.42, 113.19, 113.04, 55.95, 52.03, 49.39, 44.04 (2C), 28.25. HRMS (ESI): m/z calcd. for C₂₄H₂₃N₄O₅S₃⁺ [M+H]⁺:543.0825, found: 543.0824.

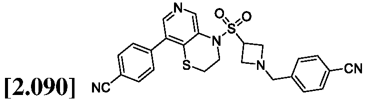

### 4-(4-((1-(4-Cyanobenzyl)azetidin-3-yl)sulfonyl)-3,4-dihydro-2H-pyrido[4,3-b][1,4]thiazin-8-yl)benzonitrile (S80)

Following the general method SA, synthesized with **SI11** (50.0 mg, 0.13 mmol), 4-(bromomethyl)benzonitrile (38.2 mg, 0.2 mmol) and K₂CO₃ (35.9 mg, 0.2 6mmol) in acetonitrile (3.0 mL) to give **S80** (38.5 mg, 60.7% yield) as a white solid after chromatographic purification (PE/EA = 5/1). mp: 159.0-160.0 °C. ¹H-NMR (400 MHz, Chloroform-*d*) δ 8.55 (s, 1H), 8.12 (s, 1H), 7.76 (d, *J* = 8.4 Hz, 2H), 7.61 (d, *J* = 6.6 Hz, 2H), 7.50 (d, *J* = 6.6 Hz, 2H), 7.37 (d, *J* = 8.1 Hz, 2H), 4.11 (p, *J* = 7.6 Hz, 1H), 3.93-3.85 (m, 2H), 3.71 (s, 2H), 3.63 (t, *J* = 8.1 Hz, 2H), 3.53-3.45 (m, 2H), 3.24-3.17 (m, 2H).¹³C-NMR (101 MHz, Chloroform-*d*) δ 146.81, 146.00, 142.61, 140.19, 137.57, 133.42, 132.62 (2C), 132.52 (2C), 130.83, 130.32 (2C), 129.05 (2C), 118.82, 118.45, 112.94, 111.56, 62.54, 55.62, 51.78, 43.67 (2C), 28.11. HRMS (ESI): m/z calcd. for C₂₅H₂₂N₅O₂S₂⁺ [M+H]⁺:488.1209, found: 488.1212.

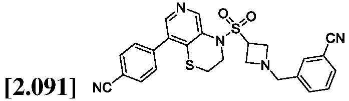

### 3-((3-((8-(4-Cyanophenyl)-2,3-dihydro-4H-pyrido[4,3-b][1,4]thiazin-4-yl)sulfonyl)azetidin-1-yl)methyl)benzonitrile (S81)

Following the general method SA, synthesized with **SI11** (50.0 mg, 0.13 mmol), 3-(bromomethyl)benzonitrile (38.2 mg, 0.2 mmol) and K₂CO₃ (35.9 mg, 0.2 6mmol) in acetonitrile (3.0 mL) to give **S81** (42.6 mg, 67.2% yield) as a white solid after chromatographic purification (PE/EA = 5/1). mp: 137.0-138.0 °C. ¹H-NMR (400 MHz, Chloroform-d) δ 8.56 (s, 1H), 8.13 (s, 1H), 7.76 (d, *J* = 8.3 Hz, 2H), 7.57 (s, 1H), 7.55 (s, 1H), 7.51 (d, *J* = 6.6 Hz, 2H), 7.48 (s, 1H), 7.42 (t, *J* = 7.6 Hz, 1H), 4.11 (s, 1H), 3.89 (s, 2H), 3.68 (s, 2H), 3.64 (s, 2H), 3.49 (s, 2H), 3.21 (s, 2H).¹³C-NMR (101 MHz, Chloroform-*d*) *δ* 146.81, 145.99, 140.19, 138.72, 137.60, 133.42, 132.86, 132.62 (2C), 131.87, 131.36, 130.83, 130.32 (2C), 129.51, 118.79, 118.45, 112.91, 112.86, 62.11, 55.54, 51.70, 43.67 (2C), 28.11. HRMS (ESI): m/z calcd. for C₂₅H₂₂N₅O₂S₂⁺ [M+H]⁺:488.1209, found: 488.1213.

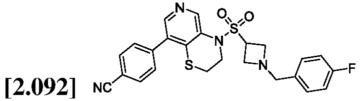

### 4-(4-((1-(4-Fluorobenzyl)azetidin-3-yl)sulfonyl)-3,4-dihydro-2H-pyrido[4,3-b][1,4]thiazin-8-yl)benzonitrile (S82)

Following the general method SA, synthesized with **SI11** (50.0 mg, 0.13 mmol), 1-(bromomethyl)-4-fluorobenzene (28.9 mg, 0.2 mmol) and K₂CO₃ (35.9 mg, 0.2 6mmol) in acetonitrile (3.0 mL) to give **S82** (29.5 mg, 47.2% yield) as a white solid after chromatographic purification (PE/EA = 5/1). mp: 144.0-145.0 °C. ¹H-NMR (400 MHz, Chloroform-*d*) δ 8.57 (s, 1H), 8.12 (s, 1H), 7.76 (d, *J* = 8.4 Hz, 2H), 7.50 (d, *J =* 8.4 Hz, 2H), 7.21 (s, 2H), 6.99 (s, 2H), 4.08 (s, 1H), 3.89 (s, 2H), 3.61 (s, 2H), 3.58 (s, 2H), 3.45 (s, 2H), 3.20 (s, 2H).¹³C-NMR (101 MHz, Chloroform-*d*) *δ* 163.58 (¹*J*_{CF} = 246.4Hz), 161.14, 146.84, 145.92, 140.23, 137.53, 133.38, 132.59 (2C), 130.86, 130.33 (2C), 130.22 (³*J*_{CF} = 8.0 Hz), 130.14, 118.46, 115.66 (²*J*_{CF} = 22.2Hz), 115.44, 112.89, 62.31, 55.33, 51.80, 43.61 (2C), 28.05. HRMS (ESI): m/z calcd. for C₂₄H₂₂FN₄O₂S₂⁺ [M+H]⁺:481.1163, found: 481.1162.

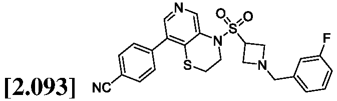

### 4-(4-((1-(3-Fluorobenzyl)azetidin-3-yl)sulfonyl)-3,4-dihydro-2H-pyrido[4,3-b][1,4]thiazin-8-yl)benzonitrile (S83)

Following the general method SA, synthesized with **SI11** (50.0 mg, 0.13 mmol), 1-(bromomethyl)-3-fluorobenzene (28.9 mg, 0.2 mmol) and K₂CO₃ (35.9 mg, 0.2 6mmol) in acetonitrile (3.0 mL) to give **S83** (32.2 mg, 51.5% yield) as a white solid after chromatographic purification (PE/EA = 5/1). mp: 136.0-137.0 °C. ¹H-NMR (400 MHz, Chloroform-*d*) *δ* 8.57 (s, 1H), 8.12 (s, 1H), 7.76 (d, *J =* 8.4 Hz, 2H), 7.50 (d, *J =* 8.3 Hz, 2H), 7.28 (s, 1H), 7.01 (d, *J =* 7.7 Hz, 1H), 6.98 (s, 1H), 6.94 (d, *J =* 8.1 Hz, 1H), 4.10 (s, 1H), 3.89 (s, 2H), 3.64 (s, 2H), 3.62 (s, 2H), 3.47 (s, 2H), 3.20 (s, 2H). ¹³C-NMR (101 MHz, Chloroform-*d*) *δ* 164.31 (¹*J*_{CF} = 246.4 Hz), 146.83, 145.93, 140.23, 139.59, 137.55, 133.38, 132.59 (2C), 130.87, 130.33 (2C), 130.20 (³*J*_{CF} = 8.0 Hz), 124.08 (⁴*J*_{CF} = 3.0 Hz), 118.47, 115.39 (²*J*_{CF} = 21.2 Hz), 114.66, 112.87, 62.50, 55.47, 51.81, 43.62 (2C), 28.07. HRMS (ESI): m/z calcd. for C₂₄H₂₂FN₄O₂S₂⁺ [M+H]⁺:481.1163, found: 481.1153.

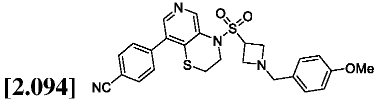

### 4-(4-((1-(4-Methoxybenzyl)azetidin-3-yl)sulfonyl)-3,4-dihydro-2H-pyrido[4,3-b][1,4]thiazin-8-yl)benzonitrile (S84)

Following the general method SA, synthesized with **SI11** (50.0 mg, 0.13 mmol), 1-(chloromethyl)-4-methoxybenzene (31.3 mg, 0.2 mmol) and K₂CO₃ (35.9 mg, 0.2 6mmol) in acetonitrile (3.0 mL) to give **S84** (32.2 mg, 51.5% yield) as a white solid after chromatographic purification (PE/EA = 5/1). mp: 132.0-133.0 °C. ¹H-NMR (400 MHz, Chloroform-d) *δ* 8.57 (s, 1H), 8.11 (s, 1H), 7.75 (d, *J* = 8.3 Hz, 2H), 7.49 (d, *J* = 8.3 Hz, 2H), 7.15 (d, *J* = 8.6 Hz, 2H), 6.84 (d, *J* = 8.6 Hz, 2H), 4.08 (s, 1H), 3.88 (s, 2H), 3.79 (s, 3H), 3.57 (s, 2H), 3.54 (s, 2H), 3.44 (s, 2H), 3.19 (s, 2H). ¹³C-NMR (101 MHz, Chloroform-d) *δ* 159.17, 146.82, 145.85, 140.25, 137.52, 133.35, 132.59 (2C), 130.33 (2C), 129.86 (2C), 128.83, 118.48, 114.06 (2C), 112.84, 62.53, 55.39, 55.22, 51.82, 43.56 (2C), 28.02. HRMS (ESI): m/z calcd. for C₂₅H₂₅N₄O₃S₂⁺ [M+H]⁺:493.1363, found: 493.1342.

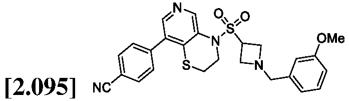

### 4-(4-((1-(3-Methoxybenzyl)azetidin-3-yl)sulfonyl)-3,4-dihydro-2H-pyrido[4,3-b][1,4]thiazin-8-yl)benzonitrile (S85)

Following the general method SA, synthesized with **SI11** (50.0 mg, 0.13 mmol), 1-(chloromethyl)-3-methoxybenzene (31.3 mg, 0.2 mmol) and K₂CO₃ (35.9 mg, 0.2 6mmol) in acetonitrile (3.0 mL) to give **S85** (30.2 mg, 47.2% yield) as a white solid after chromatographic purification (PE/EA = 5/1). mp: 93.0-94.0 °C. ¹H-NMR (400 MHz,Chloroform-*d*) *δ* 8.57 (s, 1H), 8.12 (s, 1H), 7.75 (d, *J* = 8.3 Hz, 2H), 7.50 (d, *J* = 8.3 Hz, 2H), 7.22 (s, 1H), 6.82 (d, *J* = 7.5 Hz, 1H), 6.81 (s, 1H), 6.80-6.77 (m, 1H), 4.10 (s, 1H), 3.89 (s, 2H), 3.79 (s, 3H), 3.62 (s, 2H), 3.61 (d, *J* = 7.8 Hz, 2H), 3.47 (s, 2H), 3.19 (s, 2H).¹³C-NMR (101 MHz, Chloroform-d) *δ* 159.91, 146.80, 145.86, 140.23, 138.36, 137.55, 133.36, 132.58 (2C), 130.87, 130.33 (2C), 129.69, 120.88, 118.48, 114.02, 113.17, 112.83, 63.06, 55.41, 55.34, 51.82, 43.58 (2C), 28.04. HRMS (ESI): m/z calcd. for C₂₅H₂₅N₄O₃S₂⁺ [M+H]⁺:493.1363, found: 493.1366.

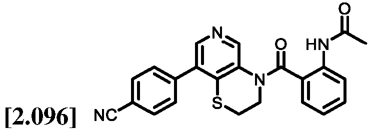

### N-(2-(8-(4-Cyanophenyl)-3,4-dihydro-2H-pyrido[4,3-b][1,4]thiazine-4-carbonyl)phenyl)acet amide (S86)

Following the general method SB, synthesized with **SI5** (41.0 mg, 0.11 mmol), 2-acetamidobenzoyl chloride (13.0 mg, 0.17 mmol), DMAP (3.67 mg, 0.03 mmol), Et₃N (33.4 mg, 0.33 mmol) in anhydrous dichloromethane (5 mL) to give **S86** (11.6 mg, 25.5% yield) as a white solid after chromatographic purification (PE/EA = 1/1). mp: 206.0-207.0 °C, *R*_{f} = 0.19 (PE/EA (V/V) = 1/2). ¹H-NMR (400 MHz, CDCl₃): *δ* 9.09 (s, 1H), 8.12 (d, *J* = 8.3 Hz, 1H), 8.01 (s, 1H), 7.92 (s, 1H), 7.76 (d, *J* = 8.2 Hz, 2H), 7.56 (d, *J* = 8.3 Hz, 2H), 7.35 (t, *J* = 7.6 Hz, 1H), 7.02-6.91 (m, 2H), 4.30-4.08 (m, 2H), 3.28 (t, *J* = 5.6 Hz, 2H), 2.17 (s, 3H); ¹³C-NMR (101 MHz, CDCl₃): δ 169.09, 168.73, 146.33, 145.22, 140.46, 137.72, 137.01, 134.04, 133.25, 132.52 (2C), 132.07, 130.31 (2C), 128.82, 123.55, 123.39, 123.30, 118.49, 112.72, 41.32, 29.29, 24.87; HRMS (ESI): m/z calcd. for C₂₃H₁₈N₄O₂S [M+H]⁺: 415.1150, found: 415.1211.

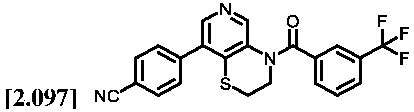

### 4-(4-(3-(Trifluoromethyl)benzoyl)-3,4-dihydro-2H-pyrido[4,3-b][1,4]thiazin-8-yl)benzonitril e (S87)

Following the general method SB, synthesized with **SI5** (100.0 mg, 0.4 mmol), 3-trifluoromethylbenzoyl chloride (123.5 mg, 0.59 mmol), DMAP (14.5 mg, 0.12 mmol), Et₃N (121.4 mg, 1.20 mmol) in anhydrous dichloromethane (6 mL) to give **S87** (154.6 mg, 91.9% yield) as a white solid after chromatographic purification (PE/EA = 2/1). mp: 160.0-161.0 °C, *R*_{f} = 0.47 (PE/EA (V/V) = 1/2). ¹H-NMR (400 MHz, CDCl₃): δ 8.04 (s, 1H), 7.79 (d, *J* = 8.3 Hz, 2H), 7.75 (s, 1H), 7.67 (d, *J* = 7.8 Hz, 1H), 7.59 (d, *J* = 9.2 Hz, 2H), 7.55 (d, *J* = 8.9 Hz, 2H), 7.48 (t, *J* = 7.7 Hz, 1H), 4.32-4.16 (m, 2H), 3.32 (t, *J* = 5.7 Hz, 2H); ¹³C-NMR (101 MHz, CDCl₃): δ 167.73, 146.42, 145.48, 140.37, 137.66, 135.34, 134.29, 133.50, 132.62 (2C), 131.82, 131.40 (²*J*_{CF} = 33.1 Hz), 130.29 (2C), 129.32, 127.80, 125.85 (³*J*_{CF} = 5.0 Hz), 124.87 (¹*J*_{CF} = 273.9 Hz), 118.49, 112.89, 41.18, 29.67; ¹⁹F NMR (376 MHz, CDCl₃): *δ* -62.96; HRMS (ESI): m/z calcd. for C₂₂H₁₄F₃N₃OS [M+H]⁺: 426.0882, found: 426.0858.

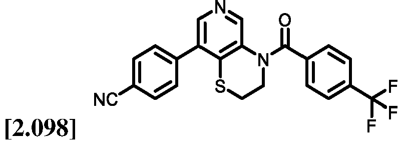

### 4-(4-(4-(Trifluoromethyl)benzoyl)-3,4-dihydro-2H-pyrido[4,3-b][1,4]thiazin-8-yl)benzonitril e (S88)

Following the general method SB, synthesized with **SI5** (100.0 mg, 0.4 mmol), 4-trifluoromethylbenzoyl chloride (123.5 mg, 0.59 mmol), DMAP (14.5 mg, 0.12 mmol), Et₃N (121.4 mg, 1.20 mmol) in anhydrous dichloromethane (6 mL) to give **S88** (154.6 mg, 91.9% yield) as a white solid after chromatographic purification (PE/EA = 2/1). mp: 228.0-229.0 °C, *R*_{f} = 0.47 (PE/EA (V/V) = 1/2). ¹H-NMR (400 MHz, CDCl₃): *δ* 8.05 (s, 1H), 7.83-7.77 (m, 3H), 7.62 (d, *J* = 8.2 Hz, 2H), 7.59 (d, *J* = 8.3 Hz, 2H), 7.49 (d, *J* = 8.3 Hz, 2H), 4.24 (t, *J* = 5.9 Hz, 2H), 3.32 (t, *J* = 5.6 Hz, 2H); ¹³C-NMR (101 MHz, CDCl₃): *δ* 167.87, 146.35, 145.54, 140.37, 138.04, 137.44, 134.20, 133.47, 133.05 (²*J*_{CF} = 33.1 Hz), 132.60 (2C), 130.33 (2C), 129.02 (2C), 125.82 (³*J*_{CF} = 3.7 Hz, 2C), 124.91 (¹*J*_{CF} = 273.9 Hz), 118.48, 112.90, 41.86, 31.04; ¹⁹F NMR (376 MHz, CDCl₃): *δ* -63.09; HRMS (ESI): m/z calcd. for C₂₂H₁₄F₃N₃OS [M+H]⁺: 426.0882, found: 426.0861.

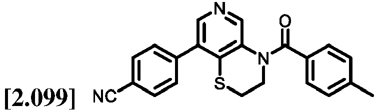

### 4-(4-(4-Methylbenzoyl)-3,4-dihydro-2H-pyrido[4,3-b][1,4]thiazin-8-yl)benzonitrile (S89)

Following the general method SB, synthesized with **SI5** (100.0 mg, 0.4 mmol), 4-methylbenzoyl chloride (91.5 mg, 0.59 mmol), DMAP (14.5 mg, 0.12 mmol), Et₃N (121.4 mg, 1.20 mmol) in anhydrous dichloromethane (6 mL) to give **S89** (154.6 mg, 91.9% yield) as a white solid after chromatographic purification (PE/EA = 2/1). mp: 227.0-228.0 °C, *R*_{f} = 0.40 (PE/EA (V/V) = 1/2). ¹H-NMR (400 MHz, CDCl₃): *δ* 8.00 (s, 1H), 7.80 (s, 1H), 7.78 (d, *J* = 8.6 Hz, 2H), 7.59 (d, *J* = 8.6 Hz, 2H), 7.29-7.26 (m, 2H), 7.13 (d, *J* = 9.2 Hz, 2H), 4.22 (t, *J* = 5.7, 2H), 3.29 (t, *J* = 5.6 Hz, 2H), 2.34 (s, 3H); ¹³C-NMR (101 MHz, CDCl₃): *δ* 169.47, 146.58, 144.75, 141.71, 140.70, 136.75, 135.00, 133.20, 132.52 (2C), 131.51, 130.35 (2C), 129.34 (2C), 128.82 (2C), 118.55, 112.69, 39.61, 29.77, 21.61; HRMS (ESI): m/z calcd. for C₂₂H₁₇N₃OS [M+H]⁺: 372.1092, found: 372.1143.

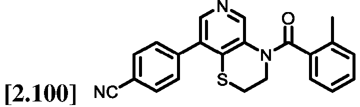

### 4-(4-(2-Methylbenzoyl)-3,4-dihydro-2H-pyrido[4,3-b][1,4]thiazin-8-yl)benzonitrile (S90)

Following the general method SB, synthesized with **SI5** (100.0 mg, 0.4 mmol), 2-methylbenzoyl chloride (91.5 mg, 0.59 mmol), DMAP (14.5 mg, 0.12 mmol), Et₃N (121.4 mg, 1.20 mmol) in anhydrous dichloromethane (6 mL) to give **S90** (154.6 mg, 91.9% yield) as a white solid after chromatographic purification (PE/EA = 2/1). mp: 169.0-170.0 °C, *R*_{f} = 0.52 (PE/EA (V/V) = 1/2). ¹H-NMR (400 MHz, CDCl₃): *δ* 7.99 (s, 1H), 7.77 (d, *J* = 8.2 Hz, 2H), 7.54 (d, *J* = 8.3 Hz, 2H), 7.31-7.25 (m, 2H), 7.19 (s, 1H), 7.16 (d, *J* = 7.2 Hz, 2H), 4.23-4.08 (m, 2H), 3.27 (t, *J* = 5.4 Hz, 2H), 2.32 (s, 3H); ¹³C-NMR (101 MHz, CDCl₃): *δ* 169.96, 146.39, 145.10, 140.62, 137.20, 135.23, 134.87, 133.59, 132.98, 132.52 (2C), 131.02, 130.30 (2C), 130.12, 127.28, 126.11, 118.52, 112.70, 40.57, 29.25, 19.26; HRMS (ESI): m/z calcd. for C₂₂H₁₇N₃OS [M+H]⁺: 372.1092, found: 372.1142.

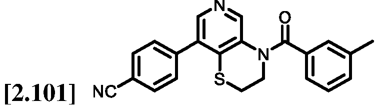

### 4-(4-(3-Methylbenzoyl)-3,4-dihydro-2H-pyrido[4,3-b][1,4]thiazin-8-yl)benzonitrile (S91)

Following the general method SB, synthesized with **SI5** (100.0 mg, 0.4 mmol), 3-methylbenzoyl chloride (91.5 mg, 0.59 mmol), DMAP (14.5 mg, 0.12 mmol), Et₃N (121.4 mg, 1.20 mmol) in anhydrous dichloromethane (6 mL) to give **S91** (154.6 mg, 91.9% yield) as a white solid after chromatographic purification (PE/EA = 2/1). mp: 167.0-168.0 °C, *R*_{f} = 0.50 (PE/EA (V/V) = 1/2). ¹H-NMR (400 MHz, CDCl₃): *δ* 8.00 (s, 1H), 7.85 (s, 1H), 7.78 (d, *J* = 8.2 Hz, 2H), 7.58 (d, *J =* 8.4 Hz, 2H), 7.29 (s, 1H), 7.25-7.14 (m, 2H), 7.05 (d, *J* = 7.3 Hz, 1H), 4.21 (t, *J* = 5.6 Hz, 2H), 3.28 (t, *J* = 5.5 Hz, 2H), 2.33 (s, 3H); ¹³C-NMR (101 MHz, CDCl₃): *δ* 169.73, 146.64, 144.89, 140.71, 138.82, 136.82, 134.74, 134.49, 133.20, 132.55 (2C), 131.97, 130.36 (2C), 129.32, 128.39, 125.49, 118.56, 112.73, 41.03, 29.68, 21.49; HRMS (ESI): m/z calcd. for C₂₂H₁₇N₃OS [M+H]⁺: 372.1092, found: 372.1143.

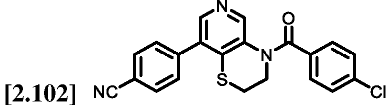

### 4-(4-(4-Chlorobenzoyl)-3,4-dihydro-2H-pyrido[4,3-b][1,4]thiazin-8-yl)benzonitrile (S92)

Following the general method SB, synthesized with **SI5** (100.0 mg, 0.4 mmol), 4-chlorobenzoyl chloride (103.6 mg, 0.59 mmol), DMAP (14.5 mg, 0.12 mmol), Et₃N (121.4 mg, 1.20 mmol) in anhydrous dichloromethane (6 mL) to give **S92** (123.9 mg, 80.0% yield) as a white solid after chromatographic purification (PE/EA = 2/1). mp: 215.0-216.0 °C, *R*_{f} = 0.38 (PE/EA (V/V) = 1/2). ¹H-NMR (400 MHz, CDCl₃): *δ* 8.03 (s, 1H), 7.80 (s, 1H), 7.79-7.76 (m, 2H), 7.58 (d, *J* = 8.6 Hz, 2H), 7.34-7.30 (m, 4H), 4.31-4.13 (m, 2H), 3.30 (t, *J* = 5.6 Hz, 2H); ¹³C-NMR (101 MHz, CDCl₃): *δ* 168.19, 146.40, 145.22, 140.47, 137.48, 137.14, 134.60, 133.38, 132.85, 132.57 (2C), 130.33 (2C), 130.16 (2C), 129.06 (2C), 118.50, 112.82, 40.98, 29.76; HRMS (ESI): m/z calcd. for C₂₁H₁₄ClN₃OS [M+H]⁺: 392.0546, found: 392.0612.

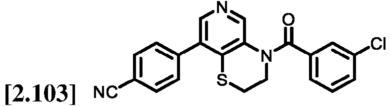

### 4-(4-(3-Chlorobenzoyl)-3,4-dihydro-2H-pyrido[4,3-b][1,4]thiazin-8-yl)benzonitrile (S93)

Following the general method SB, synthesized with **SI5** (100.0 mg, 0.4 mmol), 3-chlorobenzoyl chloride (103.6 mg, 0.59 mmol), DMAP (14.5 mg, 0.12 mmol), Et₃N (121.4 mg, 1.20 mmol) in anhydrous dichloromethane (6 mL) to give **S93** (111.0 mg, 75.7% yield) as a white solid after chromatographic purification (PE/EA = 2/1). mp: 174.0-175.0 °C, *R*_{f} = 0.47 (PE/EA (V/V) = 1/2). ¹H-NMR (400 MHz, CDCl₃): *δ* 8.04 (s, 1H), 7.82 (s, 1H), 7.79 (d, *J* = 8.3 Hz, 2H), 7.58 (d, *J* = 8.4 Hz, 2H), 7.43-7.41 (m, 1H), 7.40-7.37 (m, 1H), 7.27-7.26 (m, 1H), 7.18 (d, *J* = 6.2 Hz, 1H), 4.22 (t, *J* = 5.7 Hz, 2H), 3.29 (t, *J* = 5.6 Hz, 2H); ¹³C-NMR (101 MHz, CDCl₃): δ 167.88, 146.43, 145.36, 140.48, 137.26, 136.29, 134.96, 134.30, 133.40, 132.59 (2C), 131.31, 130.34 (2C), 129.96, 128.87, 126.56, 118.52, 112.84, 41.14, 29.66; HRMS (ESI): m/z calcd. for C₂₁H₁₄ClN₃OS [M+H]⁺: 392.0546, found: 392.0612.

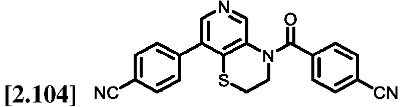

### 4-(4-(4-Cyanobenzoyl)-3,4-dihydro-2H-pyrido[4,3-b][1,4]thiazin-8-yl)benzonitrile (S94)

Following the general method SB, synthesized with **SI5** (100.0 mg, 0.4 mmol), 4-cynobenzoyl chloride (98.0 mg, 0.59 mmol), DMAP (14.5 mg, 0.12 mmol), Et₃N (121.4 mg, 1.20 mmol) in anhydrous dichloromethane (6 mL) to give **S94** (93.6 mg, 62.0% yield) as a white solid after chromatographic purification (PE/EA = 2/1). mp: 245.0-246.0 °C, *R*_{f} = 0.32 (PE/EA (V/V) = 1/2). ¹H-NMR (400 MHz, CDCl₃): *δ* 8.06 (s, 1H), 7.79 (d, *J* = 8.4 Hz, 2H), 7.74 (s, 1H), 7.65 (d, *J* = 8.4 Hz, 2H), 7.58 (d, *J* = 8.4 Hz, 2H), 7.47 (d, *J* = 8.4 Hz, 2H), 4.34-4.10 (m, 2H), 3.31 (t, *J* = 5.6 Hz, 2H); ¹³C-NMR (101 MHz, CDCl₃): *δ* 167.26, 146.20, 145.73, 140.21, 138.81, 137.63, 133.97, 133.55, 132.62 (2C), 132.52 (2C), 130.29 (2C), 129.21 (2C), 118.43, 117.81, 114.81, 112.96, 41.53, 29.66; HRMS (ESI): m/z calcd. for C₂₂H₁₄N₄OS [M+H]⁺: 383.0961, found: 383.0948.

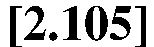

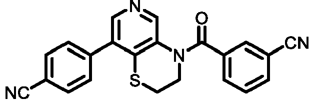

### 3-(8-(4-Cyanophenyl)-3,4-dihydro-2H-pyrido[4,3-b][1,4]thiazine-4-carbonyl)benzonitrile (S95)

Following the general method SB, synthesized with **SI5** (100.0 mg, 0.4 mmol), 3-cynobenzoyl chloride (98.0 mg, 0.59 mmol), DMAP (14.5 mg, 0.12 mmol), Et₃N (121.4 mg, 1.20 mmol) in anhydrous dichloromethane (6 mL) to give **S95** (52.6 mg, 34.8% yield) as a white solid after chromatographic purification (PE/EA = 2/1). mp: 242.0-243.0 °C, *R*_{f} = 0.31 (PE/EA (V/V) = 1/2). ¹H-NMR (400 MHz, CDCl₃): *δ* 8.06 (s, 1H), 7.79 (d, *J* = 8.3 Hz, 2H), 7.70 (d, *J* = 9.3 Hz, 2H), 7.63 (s, 1H), 7.63-7.59 (m, 2H), 7.59 (s, 1H), 7.49 (t, *J* = 7.8 Hz, 1H), 4.35-4.11 (m, 2H), 3.33 (t, *J* = 5.7 Hz, 2H); ¹³C-NMR (101 MHz, CDCl₃): δ 166.87, 146.20, 145.72, 140.21, 137.70, 136.01, 134.43, 134.04, 133.64, 132.78, 132.62 (2C), 132.16, 130.35 (2C), 129.76, 118.46, 117.76, 113.18, 112.96, 41.12, 29.70; HRMS (ESI): m/z calcd. for C₂₂H₁₄N₄OS [M+H]⁺: 383.0961, found: 383.0951.

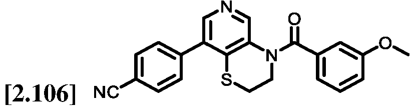

### 4-(4-(3-Methoxybenzoyl)-3,4-dihydro-2H-pyrido[4,3-b][1,4]thiazin-8-yl)benzonitrile (S96)

Following the general method SB, synthesized with **SI5** (150.0 mg, 0.59 mmol), 3-methoxybenzoyl chloride (151.8 mg, 0.89 mmol), DMAP (22.0 mg, 0.18 mmol), Et₃N (179.1 mg, 1.77 mmol) in anhydrous dichloromethane (8 mL) to give **S96** (164.5 mg, 72.0% yield) as a white solid after chromatographic purification (PE/EA = 2/1). mp: 172.0-173.0 °C, *R*_{f} = 0.45 (PE/EA (V/V) = 1/2). ¹H-NMR (400 MHz, CDCl₃): *δ* 8.01 (s, 1H), 7.86 (s, 1H), 7.78 (d, *J* = 8.2 Hz, 2H), 7.57 (d, *J* = 8.3 Hz, 2H), 7.22 (t, *J =* 7.9 Hz, 1H), 6.98 (s, 1H), 6.94 (dd, *J* = 7.6, 2.6 Hz, 1H), 6.86 (d, *J* = 7.6 Hz, 1H), 4.22 (t, *J* = 6.0 Hz, 2H), 3.77 (s, 3H), 3.28 (t, *J* = 5.5 Hz, 2H); ¹³C-NMR (101 MHz, CDCl₃): *δ* 169.19, 159.76, 146.47, 144.97, 140.62, 136.79, 135.75, 134.66, 133.17, 132.51 (2C), 130.31 (2C), 129.69, 120.69, 118.51, 116.95, 114.04, 112.68, 55.46, 41.37, 29.65; HRMS (ESI): m/z calcd. for C₂₂H₁₄N₄OS [M+H]⁺: 383.1114, found: 388.1083.

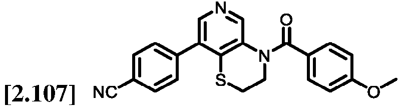

### 4-(4-(4-Methoxybenzoyl)-3,4-dihydro-2H-pyrido[4,3-b][1,4]thiazin-8-yl)benzonitrile (S97)

Following the general method SB, synthesized with **SI5** (150.0 mg, 0.59 mmol), 4-methoxybenzoyl chloride (151.8 mg, 0.89 mmol), DMAP (22.0 mg, 0.18 mmol), Et₃N (179.1 mg, 1.77 mmol) in anhydrous dichloromethane (8 mL) to give **S97** (143.8 mg, 62.9% yield) as a white solid after chromatographic purification (PE/EA = 2/1). mp: 192.0-193.0 °C, *R*_{f} = 0.39 (PE/EA (V/V) = 1/2). ¹H-NMR (400 MHz, CDCl₃): *δ* 8.00 (s, 1H), 7.82-7.74 (m, 3H), 7.62-7.57 (m, 2H), 7.39-7.31 (m, 2H), 6.86-6.78 (m, 2H), 4.23 (t, *J* = 5.7 Hz, 2H), 3.80 (s, 3H), 3.28 (t, *J* = 5.6 Hz, 2H); ¹³C-NMR (101 MHz, CDCl₃): *δ* 169.04, 161.93, 146.53, 144.63, 140.71, 136.64, 135.35, 133.24, 132.52 (2C), 130.96 (2C), 130.42 (2C), 126.36, 118.55, 113.98 (2C), 112.69, 55.49, 40.92, 29.89; HRMS (ESI): m/z calcd. for C₂₂H₁₄N₄OS [M+H]⁺: 383.1114, found: 388.1096.

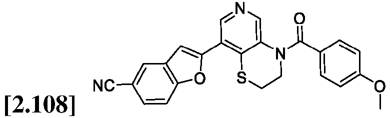

### 2-(4-(4-Methoxybenzoyl)-3,4-dihydro-2H-pyrido[4,3-b][1,4]thiazin-8-yl)benzofuran-5-carbo nitrile (S98)

Following the general method SB, synthesized with **SI4** (100.0 mg, 0.43 mmol), 3-methoxybenzoyl chloride (111.3 mg, 0.65 mmol), DMAP (14.5 mg, 0.12 mmol), Et₃N (121.4 mg, 1.20 mmol) in anhydrous dichloromethane (6 mL) to give (8-bromo-2,3-dihydro-4H-pyrido[4,3-b][1,4]thiazin-4-yl)(3-methoxyphenyl)methanone (111.5 mg, 70.2% yield) as a white solid after chromatographic purification (PE/EA = 8/1). ¹H-NMR (400 MHz, Chloroform-*d*) *δ* 8.24 (s, 1H), 7.66 (s, 1H), 7.28 (s, 2H), 6.80 (s, 2H), 4.20 (s, 2H), 3.79 (s, 3H), 3.37 (s, 2H).

Following the general method SD, synthesized with (8-bromo-2,3-dihydro-4H-pyrido[4,3-b][1,4]thiazin-4-yl)(3-methoxyphenyl)methanone (100.0 mg, 0.27 mmol), (5-cyanobenzofuran-2-yl)boronic acid (56.3 mg, 0.30 mmol), K₂CO₃ (151.5 mg, 1.09 mmol), PdCl₂(PPh₃)₂ (19.2 mg, 0.03 mmol), and Pcy₃ (14.0 mg, 0.05 mmol) in a mixture of dioxane (3 mL) and water (1 mL) to give **S98** (67.9 mg, 57.9% yield) as a light yellow solid after chromatographic purification (PE/EA = 1/1). mp: 245.0-246.0 °C. ¹H-NMR (400 MHz, Chloroform-*d*) *δ* 8.70 (s, 1H), 8.01 (s, 1H), 7.76 (s, 1H), 7.64 (s, 2H), 7.41 (s, 1H), 7.31 (d, *J* = 8.6 Hz, 2H), 6.80 (s, 2H), 4.27 (s, 2H), 3.78 (s, 3H), 3.44 (s, 2H).¹³C-NMR (101 MHz, Chloroform-*d*) *δ* 168.92, 161.94, 156.03, 153.07, 146.67, 144.76, 136.58, 135.68, 131.02 (2C), 129.27, 128.99, 126.56, 126.22, 122.91, 119.32, 113.97 (2C), 112.64, 107.72, 107.46, 55.47, 40.61, 30.17. HRMS (ESI): m/z calcd. for C₂₄H₁₈N₃O₃S⁺ [M+H]⁺: 428.1063, found: 428.1066.

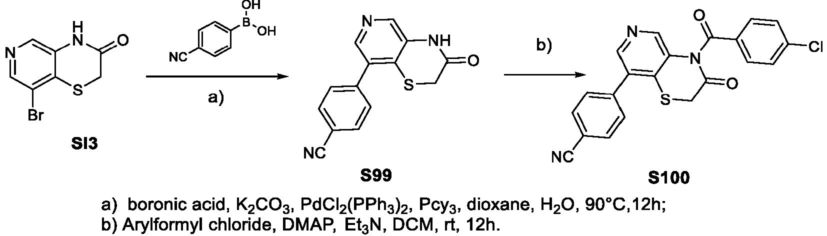

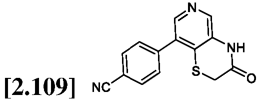

### 4-(3-Oxo-3,4-dihydro-2H-pyrido[4,3-b][1,4]thiazin-8-yl)benzonitrile (S99)

Following the general method SD, synthesized with SI3 (100.0 mg, 0.27 mmol), (4-cyanophenyl)boronic acid (47.6 mg, 0.32 mmol), K₂CO₃ (119.4 mg, 0.86 mmol), PdCl₂(PPh₃)₂ (15.2 mg, 0.02 mmol), and Pcy₃ (12.1 mg, 0.04 mmol) in a mixture of dioxane (3 mL) and water (1 mL) to give **S99** (45.4 mg, 85.0% yield) as a light yellow solid after chromatographic purification (PE/EA = 1/2). ¹H-NMR (400 MHz, CDCl₃) δ 10.32 (s, 1H), 8.89 (s, 1H), 8.29 (s, 1H), 7.75 (d, J = 8.4 Hz, 2H), 7.53 (d, *J =* 8.4 Hz, 2H), 3.99 (s, 2H).

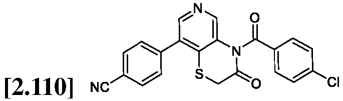

### 4-(4-(4-chlorobenzoyl)-3-oxo-3,4-dihydro-2H-pyrido[4,3-b][1,4]thiazin-8-yl)benzonitrile (S100)

Following the general method SB, synthesized with **S99** (100.0 mg, 0.38 mmol), chlorobenzoyl chloride (103.6 mg, 0.59 mmol), DMAP (14.7 mg, 0.12 mmol), Et₃N (121.4 mg, 1.20 mmol) in anhydrous dichloromethane (8 mL) to give **S100** (54.5 mg, 36.9% yield) as a white solid after chromatographic purification (PE/EA = 2/1). mp: 215.0-216.0 °C, *R*_{f} = 0.38 (PE/EA (V/V) = 1/2). ¹H-NMR (400 MHz, CDCl₃): *δ* 8.15 (s, 1H), 7.92 (s, 1H), 7.83 (d, *J =* 8.4 Hz, 2H), 7.72 (d, *J =* 8.6 Hz, 2H), 7.48-7.55 (m, 4H), 3.86 (s, 2H).

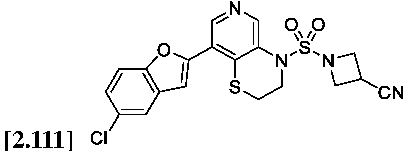

### 1-((8-(5-Chlorobenzofuran-2-yl)-2,3-dihydro-4H-pyrido[4,3-b][1,4]thiazin-4-yl)sulfonyl)azet idine-3-carbonitrile (S101)

A solution of **SI8** (100 mg, 0.33 mmol) and DBU (80 mg, 0.53 mmol) in anhydrous 1,2-dichloroethane (5 mL) was stirred at 0 °C for 5 min under N₂ before adding 3-cyanoazetidine-1-sulfonyl chloride (150 mg, 0.83 mmol) in 1,2-dichloroethane (2 mL) dropwise at 0 °C. The resulting mixture was warmed to room temperature and stirred for 12 h. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA = 2:1) to give **S101** (25.0 mg, 17.2%) as a white solid. ¹H-NMR (400 MHz, Chloroform-*d*) δ 8.83 (s, 1H), 8.16 (s, 1H), 7.68 (d, *J =* 2.1 Hz, 1H), 7.43 (d, *J =* 8.7 Hz, 1H), 7.31 (dd, *J* = 8.7, 2.1 Hz, 1H), 7.25 (s, 1H), 4.35 (t, *J =* 8.4 Hz, 2H), 4.25-4.16 (m, 2H), 3.75-3.67 (m, 2H), 3.45 (t, *J* = 5.9 Hz, 2H), 3.25-3.22 (m, 1H). HRMS (ESI): m/z calcd. for C₁₉H₁₅ClN₄O₃S₂ [M+H]⁺: 447.0347, found: 447.0352.

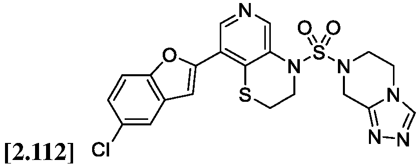

### 8-(5-Chlorobenzofuran-2-yl)-4-((5,6-dihydro-[1,2,4]triazolo[4,3-a]pyrazin-7(8H)-yl)sulfonyl) -3,4-dihydro-2H-pyrido[4,3-b][1,4]thiazine (S102)

A solution of **SI8** (100 mg, 0.33 mmol) and DBU (80 mg, 0.53 mmol) in anhydrous 1,2-dichloroethane (5 mL) was stirred at 0 °C for 5 min under N₂ before adding 5,6-dihydro-[1,2,4]triazolo[4,3-a]pyrazine-7(8H)-sulfonyl chloride (185 mg, 0.83 mmol) in 1,2-dichloroethane (2 mL) dropwise at 0 °C. The resulting mixture was warmed to room temperature and stirred for 12 h. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA = 2:1) to give **S102** (19.0 mg, 9.1%) as a white solid. ¹H-NMR (400 MHz, Chloroform-*d*) δ 8.83 (s, 1H), 8.42 (s, 1H), 8.16 (s, 1H), 7.52 (d, *J* = 2.1 Hz, 1H), 7.40 (d, *J* = 8.7 Hz, 1H), 7.29 (dd, *J* = 8.7, 2.1 Hz, 1H), 7.17 (s, 1H), 4.37 (t, *J* = 8.4 Hz, 2H), 4.12 (t, *J =* 8.6 Hz, 2H), 4.02 (s, 2H), 3.45 (t, *J =* 8.4 Hz, 2H), 3.17 (t, *J =* 8.6 Hz, 2H). HRMS (ESI): m/z calcd. for C₂₀H₁₈C1N₆O₃S₂ [M+H]⁺: 489.0565, found: 489.0512.

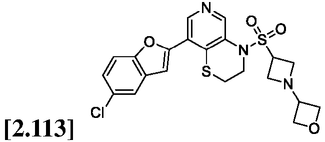

### 8-(5-Chlorobenzofuran-2-yl)-4-((1-(oxetan-3-yl)azetidin-3-yl)sulfonyl)-3,4-dihydro-2H-pyrid o[4,3-b][1,4]thiazine (S103)

To a solution of **S62** (50 mg, 0.11 mmol) and oxetan-3-one (15 mg, 0.20 mmol) in methanol (2 mL) was added sodium cyanoborohydride (NaBH₃CN, 16 mg, 0.25 mmol) slowly in an ice bath. The resulting reaction mixture was allowed to warm to room temperature and continued to stir for 30 min. After the reaction was complete as monitored by TLC, the reaction mixture was diluted with water (4 mL) and extracted with ethyl acetate (3×5 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography (PE/EA) to give **S103** (82.0 mg, 86.0%) as a light yellow solid. ¹H-NMR (400 MHz, Chloroform-*d*) δ 8.74 (s, 1H), 8.49 (s, 1H), 7.54 (d, *J* = 2.1 Hz, 1H), 7.40 (d, *J* = 8.7 Hz, 1H), 7.27 (dd, *J* = 8.7, 2.1 Hz, 1H), 7.16 (s, 1H), 4.62 (t, *J =* 6.8 Hz, 2H), 4.41 (t, *J* = 7.5 Hz, 2H), 4.08-4.01 (m, 1H), 3.89 (t, *J =* 8.4 Hz, 2H), 3.69-3.57 (m, 1H), 3.59 (t, *J =* 6.8 Hz, 2H), 3.50 (t, *J* = 8.4 Hz, 2H), 3.29 (t, *J* = 8.4 Hz, 2H). HRMS (ESI): m/z calcd. for C₂₁H₂₁ClN₃O₄S₂ [M+H]⁺: 478.0657, found: 478.0666.

### Example 3:

Synthetic procedures and structural characterizations of provided compounds of formulae **(X)-(XIV)** and intermediates are described:
**General method CA:** To a solution of the corresponding amine (0.2 mmol), DMAP (7.2 mg, 0.06 mmol), and triethylamine (0.6 mmol) in anhydrous dichloromethane (5 mL) was added dropwise the corresponding acyl chloride (0.3 mmol) at 0°C. The mixture was then warmed to room temperature and stirred for 8 hours. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.
**General method CB:** The corresponding bromide (1 mmol), potassium carbonate (4 mmol), and the corresponding boronic acid (1.5 mmol) were added to a mixture of 1,4-dioxane (3 mL) and water (1 mL). To this mixture were added PdCl₂(PPh₃)₂ (0.1 mmol) and tricyclohexylphosphine (0.2 mmol) under nitrogen atmosphere. The reaction mixture was subsequently heated to 90 °C and stirred for 24 hours. After the reaction was complete as monitored by TLC, it was cooled to room temperature, diluted with water (5 mL), and extracted with ethyl acetate (3×5 mL). The combined organic layers were washed with brine (5 mL) and dried over anhydrous Na₂SO₄. The solvent was removed under reduced pressure to give the crude product, which was subsequently purified by silica gel column chromatography (PE/EA) to yield the target compound.
**General method CC:** Dissolved the corresponding amine (1 mmol) and the corresponding sulfonyl chloride (2 mmol) in pyridine (4 mL). The reaction system was heated to 120 °C under N₂, and stirred for 12 hours. After the reaction was complete as monitored by TLC, the reaction mixture was cooled to room temperature, diluted with water (6 mL), and extracted with ethyl acetate (3×12 mL). The combined organic phase was washed with saturated CuSO₄ solution (5 mL) and brine, and dried over anhydrous Na₂SO₄. The solvent was removed under reduced pressure to give the crude product, which was subsequently purified by silica gel column chromatography (PE/EA) to yield the target compound.
**General method CD:** To a solution of the corresponding amine (1 mmol), carboxylic acid (1.1 mmol), and N,N-diisopropylethylamine (3 mmol) in anhydrous dichloromethane (5 mL), was added HATU (1.05 mmol) at 0 °C. The reaction mixture was warmed to room temperature and stirred overnight. After the reaction was complete as monitored by TLC, the mixture was diluted with water (6 mL) and extracted with dichloromethane (2×6 mL). The combined organic layers were washed with brine and dried over anhydrous Na₂SO₄. The solvent was removed under reduced pressure to give the crude product, which was subsequently purified by silica gel column chromatography (PE/EA) to yield the target compound.

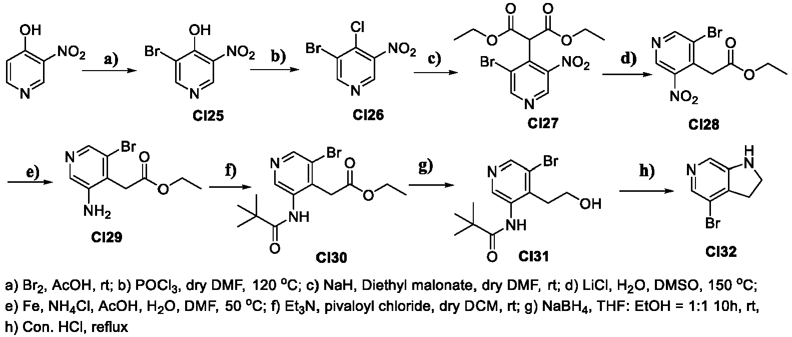

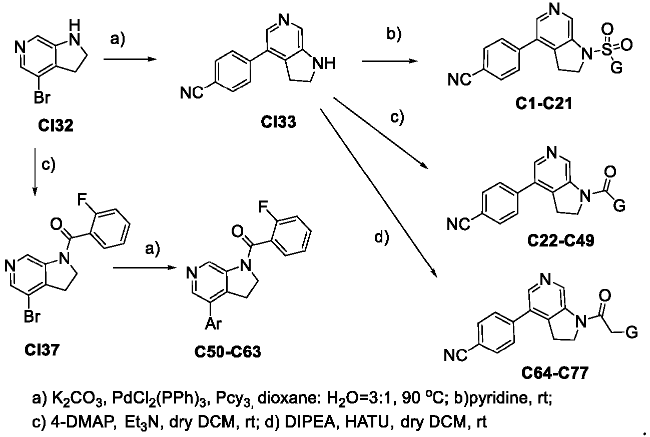

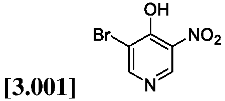

### 3-Bromo-4-chloro-5-nitropyridine (CI25)

To a solution of 3-nitropyridin-4-ol (100 mg, 0.71 mmol) in glacial acetic acid (5 mL) was dropped Br₂ (171 mg, 1.07 mmol). The resulting reaction mixture was warmed to room temperature and stirred for further 4 h. After the reaction was complete as monitored by TLC, participate formed was collected to give intermediate **CI25** as a white solid, which was used directly in the next step without further purification and characterization.

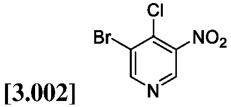

### 3-Bromo-4-chloro-5-nitropyridine (CI26)

To a solution of **CI25** (100 mg, 0.46 mmol) in anhydrous DMF (5 mL) was added phosphorus oxychloride (71 mg, 0.46 mmol) slowly in an ice bath. The resulting mixture was heated to reflux (120 °C) under N₂ for 2 h until the reaction was complete as monitored by TLC. After being cooled to 0 °C, it was quenched with water (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of PE: EA (V/V) = 100:1 to 10:1, yielding the compound **CI26** (95.0 mg, 87.5% yield). It was used directly in the next step without further characterization.

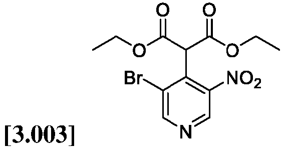

### Diethyl 2-(3-bromo-5-nitropyridin-4-yl)malonate (CI27)

To a suspension of NaH (35 mg, 0.86 mmol) in anhydrous DMF (5 mL) was added diethyl malonate (138 mg, 0.86 mmol) dropwise in an ice bath under N₂. The resulting mixture was stirred at 0 °C for 30 min before **CI26** (100 mg, 0.43 mmol) in anhydrous DMF (2 mL) was dropped in leading to a color change from light yellow to brown. The mixture was subsequently warmed to room temperature and stirred for another hours. After the reaction was complete as monitored by TLC, it was quenched with water (5 mL) at 0 °C, and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of PE: EA (V/V) = 100:1 to 20:1, yielding the compound **CI27** (80.0 mg, 50.3% yield). ¹H-NMR (400 MHz, CDCl₃) δ 9.17 (s, 1H), 9.01 (s, 1H), 5.48 (s, 1H), 4.26 (dd, *J* = 6.7, 5.8 Hz, 4H), 1.26 (t, *J =* 7.1 Hz, 6H).

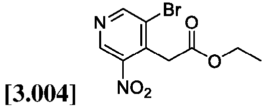

### Ethyl 2-(3-bromo-5-nitropyridin-4-yl)acetate (CI28)

To a solution of **CI27** (100 mg, 0.28 mmol) in a mixture of DMSO and water (2.5 mL, V/V = 4:1) was added LiCl before it was heated to 150 °C and stirred for 3 h under N₂. After the reaction was complete as monitored by TLC, it was cooled to room temperature, quenched with water (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of PE: EA (V/V) = 100:1 to 20:1, yielding the compound **CI28** (60.0 mg, 87.5% yield). ¹H-NMR (400 MHz, CDCl₃) δ 9.13 (s, 1H), 8.98 (s, 1H), 4.25 (s, 2H), 4.23-4.16 (m, 2H), 1.27 (t, *J* = 7.2 Hz, 3H).

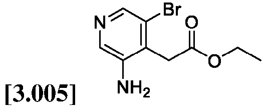

### Ethyl 2-(3-amino-5-bromopyridin-4-yl)acetate (CI29)

To a suspension of NH₄Cl (22 mg, 0.41 mmol) and glacial acetic acid (49 mg, 0.82 mmol) in water (5 mL) was added Fe powder (137 mg, 2.45 mmol). The resulting mixture was heated to 50 °C for 10 min, before **CI28** (100 mg, 0.41 mmol) in DMF (0.5 mL) was added slowly. The reaction mixture was further stirred for 2 h at 50 °C. After the reaction was complete as monitored by TLC, it was cooled to room temperature, quenched with saturated Na₂CO₃ aq. (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of PE: EA (V/V) = 10:1 to 2:1, yielding the compound **CI29** (80.0 mg, 80.5% yield) as a colorless oil. It was used directly in the next step without further characterization.

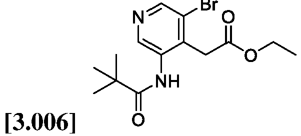

### 5-Bromo-4-(2-ethoxy-2-oxoethyl)pyridin-3-ylpivalate (CI30)

To a solution of **CI29** (100 mg, 0.42 mmol) and Et3N (128 mg, 1.26 mmol) in anhydrous dichloromethane (5 mL) was added pivaloyl chloride (128 mg, 2.5 mmol) slowly at 10 °C. The resulting mixture was warmed to room temperature and further stirred for 2 h. After the reaction was complete as monitored by TLC, it was cooled to room temperature, quenched with saturated Na₂CO₃ aq. (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of PE: EA (V/V) = 20:1 to 5:1, yielding the compound **CI30** (96.0 mg, 66.5% yield) as a light yellow oil. ¹H-NMR (400 MHz, CDCl₃) δ 8.87 (s, 1H), 8.77 (s, 1H), 8.51 (s, 1H), 4.20 (q, *J =* 7.1 Hz, 2H), 3.83 (s, 2H), 1.32 (s, 9H), 1.28 (t, *J =* 7.1 Hz, 3H).

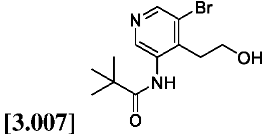

### N-(5-bromo-4-(2-hydroxyethyl)pyridin-3-yl)pivalamide (CI31)

To a solution of **CI30** (50 mg, 0.15 mmol) in a mixture of THF and water (5 mL, V/V = 1:1) was added sodium borohydride (28 mg, 0.75 mmol) slowly in batch at 0 °C. The resulting mixture was warmed to room temperature and further stirred for 4 h. After the reaction was complete as monitored by TLC, it was cooled to room temperature, quenched with water (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with an elution of PE: EA (V/V) = 2:1, yielding the compound **CI31** (28.0 mg, 61.8% yield) as a white solid. ¹H-NMR (400 MHz, CDCl₃) δ 9.41 (s, 1H), 8.71 (s, 1H), 8.27 (s, 1H), 4.02-3.83 (m, 2H), 3.06-2.89 (m, 2H), 1.27 (s, 9H).

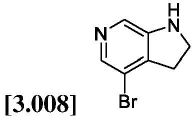

### 4-Bromo-2,3-dihydro-1H-pyrrolo[2,3-c]pyridine (CI32)

The suspension of **CI31** (50 mg, 0.17 mmol) in concentrated HCl (5 mL) was heated to 110 °C and stirred for 24 h. After the reaction was complete as monitored by TLC, it was cooled to room temperature, quenched with saturated Na₂CO₃ aq. (5 mL) adjusting the pH to 8-9, and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography a gradient elution of PE: EA (V/V) = 20:1 to 1:1, yielding the compound **CI32** (31.0 mg, 60.5% yield) as a light yellow solid. ¹H-NMR (400 MHz, CDCl₃) δ 8.00 (s, 1H), 7.84 (s, 1H), 3.66 (t, *J =* 8.7 Hz, 2H), 3.07 (t, *J =* 8.7 Hz, 2H).

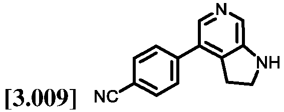

### 4-(2,3-Dihydro-1H-pyrrolo[2,3-c]pyridin-4-yl)benzonitrile (CI33)

Following the general method CB, synthesized with **CI32** (50.0 mg, 0.25 mmol), (4-cyanophenyl)boronic acid (56.1 mg, 0.38 mmol), K₂CO₃ (140 mg, 1.01 mmol), PdCl₂(PPh₃)₂ (8 mg, 0.025 mmol) and Pcy₃ (14 mg, 0.051 mmol) to give **CI33** (36.1 mg, 64.2% yield) as a yellow solid after chromatographic purification (CH₂Cl₂/MeOH = 20/1 to 1/3). ¹H-NMR (400 MHz, CDCl₃) δ 7.96 (s, 2H), 7.69 (d, *J* = 8.4 Hz, 2H), 7.49 (d, *J =* 8.3 Hz, 2H), 3.60 (t, *J =* 8.5 Hz, 2H), 3.08 (t, *J* = 8.5 Hz, 2H).

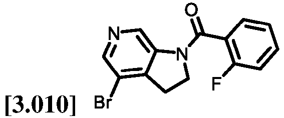

### (4-Bromo-2,3-dihydro-1H-pyrrolo[2,3-c]pyridin-1-yl)(2-fluorophenyl) methanone (CI37)

Following the general method CA, synthesized with **CI32** (100 mg, 0.452 mmol), 2-flourobenzoyl chloride (143 mg, 0.904 mmol), DMAP (17 mg, 0.12 mmol), Et₃N (89 mg, 0.87 mmol) in anhydrous DMF (4 mL) to give **CI37** (165.3 mg, 74.4% yield) as a white solid after chromatographic purification (PE/EA = 1/1). ¹H-NMR (400 MHz, DMSO-*d*₆) δ 9.20 (s, 1H), 8.45 (s, 1H), 7.66-7.55 (m, 2H), 7.46-7.33 (m, 2H), 3.99-3.86 (m, 2H), 3.16 (t, *J =* 8.4 Hz, 2H).

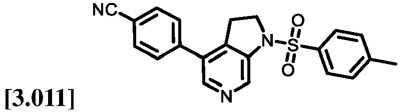

### 4-(1-Tosyl-2,3-dihydro-1H-pyrrolo[2,3-c]pyridin-4-yl)benzonitrile (C1)

Following the general method CC, synthesized with **CI33** (100 mg, 0.452 mmol), 4-methylbenzenesulfonyl chloride (172 mg, 0.904 mmol) in pyridine (4 mL) to give **C1** (98.8 mg, 58.1% yield) as a white solid after chromatographic purification (PE/EA = 10/1 to 1/3). mp: 194.8-195.8 °C. *R*_{f} = 0.25 (PE/EA (V/V) = 2/1). ¹H-NMR (400 MHz, CDCl₃) δ 8.91 (s, 1H), 8.29 (s, 1H), 7.74 (dd, *J* = 12.0, 8.3 Hz, 4H), 7.45 (d, *J =* 8.3 Hz, 2H), 7.30 (d, *J =* 8.1 Hz, 2H), 3.94 (t, *J* = 8.5 Hz, 2H), 3.04 (t, *J =* 8.5 Hz, 2H), 2.40 (s, 3H). ¹³C-NMR (101 MHz, CDCl₃) δ 145.05, 143.81, 140.86, 139.93, 138.62, 135.15, 133.34, 132.77, 2C), 132.67, 130.18 (2C), 129.05 (2C), 127.58 (2C), 118.42, 112.38, 49.68, 27.62, 21.74. HRMS (ESI): m/z calcd. for C₂₁H₁₉N₃O₂S⁺ [M+H]⁺:376.1114, found: 376.1108.

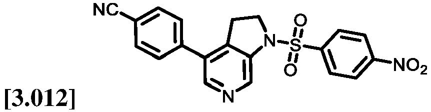

### 4-(1-((4-Nitrophenyl) sulfonyl)-2,3-dihydro-1H-pyrrolo[2,3-c]pyridin-4-yl)benzonitrile (C2)

Following the general method CC, synthesized with **CI33** (100 mg, 0.452 mmol), 4-nitrobenzenesulfonyl chloride (200 mg, 0.904 mmol) in pyridine (4 mL) to give **C2** (93.1 mg, 50.4% yield) as a white solid after chromatographic purification (PE/EA = 10/1 to 1/3). mp: 226.3-227.3 °C. *R*_{f} = 0.25 (PE/EA (V/V) = 2/1). ¹H-NMR (400 MHz, DMSO-*d*₆) δ 8.73 (s, 1H), 8.42 (s, 1H), 8.39 (d, *J =* 6.8 Hz, 2H), 8.22 (d, *J =* 8.9 Hz, 2H), 7.94 (d, *J =* 8.3 Hz, 2H), 7.71 (d, *J* = 8.3 Hz, 2H), 4.02 (t, *J* = 8.4 Hz, 2H), 3.17 (t, *J* = 8.4 Hz, 2H). ¹³C-NMR (101 MHz, DMSO-*d*₆) δ 150.65, 144.43, 140.79, 140.34, 139.66, 138.48, 133.85, 132.70 (2C), 132.33, 129.28 (2C), 128.93 (2C), 124.99 (2C), 118.56, 111.02, 50.00, 26.94. HRMS (ESI): m/z calcd. for C₂₀H₁₅N₄O₄S⁺ [M+H]⁺:407.0814, found: 407.0801.

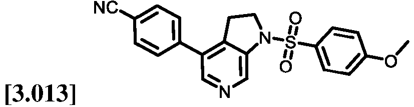

### 4-(1-((4-Methoxyphenyl)sulfonyl)-2,3-dihydro-1H-pyrrolo[2,3-c]pyridin-4-yl)benzonitrile (C3)

Following the general method CC, synthesized with **CI33** (100 mg, 0.452 mmol), 4-methoxybenzenesulfonyl chloride (221 mg, 0.904 mmol) in pyridine (4 mL) to give **C3** (91.1 mg, 51.4% yield) as a white solid after chromatographic purification (PE/EA = 10/1 to 1/3). mp: 166.0-166.9 °C. *R*_{f} = 0.25 (PE/EA (V/V) = 2/1). ¹H-NMR (400 MHz, DMSO-*d*₆) δ 8.88 (s, 1H), 8.49 (s, 1H), 7.94 (d, *J =* 8.2 Hz, 2H), 7.86 (d, *J =* 8.8 Hz, 2H), 7.71 (d, *J =* 8.3 Hz, 2H), 7.13 (d, *J* = 8.8 Hz, 2H), 3.90 (t, *J =* 8.3 Hz, 2H), 3.81 (s, 3H), 3.12 (t, *J =* 8.1 Hz, 2H). ¹³C-NMR (101 MHz, DMSO-*d*₆) δ 163.51, 143.78, 140.58, 139.13, 133.86, 132.68 (2C), 129.70, 129.64 (2C), 129.28 (2C), 126.91, 118.57,114.90 (2C), 110.94, 55.78, 49.77, 26.88. HRMS (ESI): m/z calcd. for C₂₁H₁₈N₃O₃S⁺ [M+H]⁺: 392.1069, found: 392.0929.

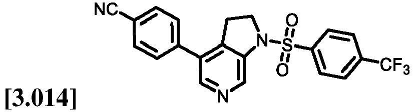

### 4-(1-((4-(Trifluoromethyl)phenyl)sulfonyl)-2,3-dihydro-1H-pyrrolo[2,3-c]pyridin-4-yl)benzo nitrile (C4)

Following the general method CC, synthesized with **CI33** (100 mg, 0.452 mmol), (4-triflouromethyl)benzenesulfonyl chloride (223 mg, 0.904 mmol) in pyridine (4 mL) to give **C4** (97.1 mg, 49.9% yield) as a white solid after chromatographic purification (PE/EA = 10/1 to 1/3). mp: 182.0-182.9 °C. ¹H-NMR (400 MHz, CDCl₃) δ 9.00 (s, 1H), 8.47 (s, 1H), 8.02 (d, *J =* 8.1 Hz, 2H), 7.79 (d, *J =* 8.0 Hz, 2H), 7.74 (d, *J =* 8.0 Hz, 2H), 7.46 (d, *J =* 8.2 Hz, 2H), 3.98 (t, *J =* 8.5 Hz, 2H), 3.06 (t, *J =* 7.6 Hz, 2H). ¹³C-NMR (101 MHz, CDCl₃) δ 140.8, 139.90, 138.38, 138.30, 137.30, 135.61 (²*J*_{CF} = 33 Hz), 134.40, 134.36, 132.83 (2C), 129.10 (2C), 128.03 (2C), 126.78 (³*J*_{CF} = 4.0 Hz, 2C), 123.13 (¹*J*_{CF} = 271 Hz), 118.35, 112.58, 49.78, 27.64. ¹⁹F NMR (377 MHz, CDCl₃) δ -63.22. HRMS (ESI): m/z calcd. for C₂₁H₁₅F₃N₃O₃S⁺ [M+H]⁺: 430.0837, found: 430.0836.

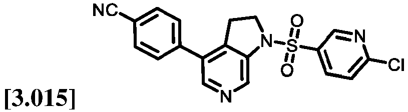

### 4-(1-((6-Chloropyridin-3-yl)sulfonyl)-2,3-dihydro-1H-pyrrolo[2,3-c]pyridin-4-yl)benzonitril e (C5)

Following the general method CC, synthesized with **CI33** (100 mg, 0.452 mmol), 6-chloropyridine-3-sulfonyl chloride (193 mg, 0.904 mmol) in pyridine (4 mL) to give **C5** (94.1 mg, 52.5% yield) as a white solid after chromatographic purification (PE/EA = 10/1 to 1/3). mp: 168.0-168.9 °C. ¹H-NMR (400 MHz, DMSO- *d*₆) δ 8.98 (d, *J* = 2.4 Hz, 1H), 8.74 (s, 1H), 8.39 (dd, *J =* 8.4, 2.5 Hz, 2H), 7.95 (d, *J =* 8.3 Hz, 2H), 7.79 (d, *J =* 8.4 Hz, 1H), 7.72 (d, *J =* 8.3 Hz, 2H), 4.02 (t, *J =* 8.4 Hz, 2H), 3.18 (t, *J =* 8.4 Hz, 2H). ¹³C-NMR (101 MHz, DMSO- *d*₆) δ 155.38, 148.32, 144.41, 140.40, 139.74, 138.65, 134.61, 133.76, 132.64 (2C), 131.72, 131.00, 129.29 (2C), 125.67, 118.57, 111.00, 49.86, 26.97. HRMS (ESI): m/z calcd. for C₁₉H₁₄C1N₄O₂S⁺ [M+H]⁺: 397.0526, found: 397.0515.

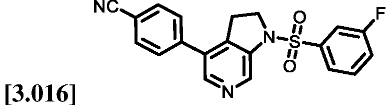

### 4-(1-((3-Fluorophenyl)sulfonyl)-2,3-dihydro-1H-pyrrolo[2,3-c]pyridin-4-yl)benzonitrile (C6)

Following the general method CC, synthesized with **CI33** (100 mg, 0.452 mmol), 3-flourobenzenesulfonyl chloride (170 mg, 0.904 mmol) in pyridine (4 mL) to give **C6** (97.1 mg, 49.9% yield) as a white solid after chromatographic purification (PE/EA = 10/1 to 1/3). mp: 172.3-173.0 °C. ¹H-NMR (400 MHz, DMSO-*d*₆) δ 8.72 (s, 1H), 8.36 (s, 1H), 7.94 (d, *J* = 8.4 Hz, 2H), 7.81 (ddd, *J =* 10.4, 6.3, 4.8 Hz, 2H), 7.74-7.66 (m, 3H), 7.61 (td, *J =* 8.5, 2.0 Hz, 1H), 4.00 (t, *J* = 8.4 Hz, 2H), 3.16 (t, *J =* 8.4 Hz, 2H). ¹³C-NMR (101 MHz, DMSO-*d*₆) δ 161.93 (¹*J*_{CF} = 231 Hz), 144.17, 140.43, 139.49, 138.69, 137.48 (³*J*_{CF} = 6 Hz), 133.85, 132.68 (2C), 132.18 (³*J*_{CF} = 8 Hz), 129.27 (2C), 123.63 (⁴*J*_{CF} = 3 Hz), 121.46 (²*J*_{CF} = 21 Hz), 118.57, 114.49 (²*J*_{CF} = 25 Hz), 110.97, 49.96, 26.91. ¹⁹F NMR (377 MHz, DMSO) δ -109.67. HRMS (ESI): m/z calcd. for C₁₉H₁₄ClN₄O₂S⁺ [M+H]⁺: 397.0526, found: 397.0515.

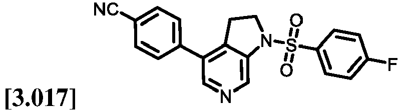

### 4-(1-((4-Fluorophenyl)sulfonyl)-2,3-dihydro-1H-pyrrolo[2,3-c]pyridin-4-yl)benzonitrile (C7)

Following the general method CC, synthesized with **CI33** (100 mg, 0.452 mmol), 4-flourobenzenesulfonyl chloride (170 mg, 0.904 mmol) in pyridine (4 mL) to give **C7** (95.1 mg, 55.2% yield) as a white solid after chromatographic purification (PE/EA = 10/1 to 1/3). mp: 155.0-156.0 °C. ¹H-NMR (400 MHz, DMSO-*d*₆) δ 8.71 (s, 1H), 8.35 (s, 1H), 8.06-7.98 (m, 2H), 7.94 (d, *J =* 8.3 Hz, 2H), 7.71 (d, *J =* 8.4 Hz, 2H), 7.47 (t, *J =* 8.8 Hz, 2H), 3.95 (t, *J =* 8.4 Hz, 2H), 3.15 (t, *J =* 8.4 Hz, 2H). ¹³C-NMR (101 MHz, DMSO-*d*₆) δ 165.25 (¹*J*_{CF} = 252 Hz), 144.11, 140.47, 139.46, 139.04, 134.26, 133.86, 132.70 (2C), 131.91 (⁴*J*_{CF} = 2.9 Hz), 130.55 (³*J*_{CF} = 9, 2C), 129.29 (2C), 118.59, 117.07 (²*J*_{CF} = 22 Hz, 2C), 110.98, 49.87, 26.93. ¹⁹F NMR (377 MHz, DMSO-*d*₆) δ -104.02. HRMS (ESI): m/z calcd. for C₂₀H₁₅FN₃O₂S⁺ [M+H]⁺: 380.0869, found: 380.0863.

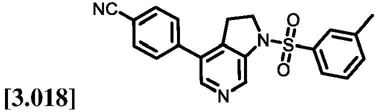

### 4-(1-(m-Tolylsulfonyl)-2,3-dihydro-1H-pyrrolo[2,3-c]pyridin-4-yl)benzonitrile (C8)

Following the general method CC, synthesized with **CI33** (100 mg, 0.452 mmol), 3-methylbenzenesulfonyl chloride (172 mg, 0.904 mmol) in pyridine (4 mL) to give **C8** (92.1 mg, 54.2% yield) as a white solid after chromatographic purification (PE/EA = 10/1 to 1/3). mp: 181.9-182.5 °C. ¹H-NMR (400 MHz, DMSO-*d*₆) δ 8.70 (s, 1H), 8.33 (s, 1H), 7.94 (d, *J =* 8.3 Hz, 2H), 7.72 (dd, *J =* 13.3, 8.6 Hz, 4H), 7.57-7.47 (m, 2H), 3.95 (t, *J* = 8.4 Hz, 2H), 3.14 (t, *J =* 8.4 Hz, 2H), 2.37 (s, 3H). ¹³C-NMR (101 MHz, DMSO-*d*₆) δ 143.90, 140.49, 139.69, 139.32, 139.02, 135.49, 134.85, 133.79, 132.68 (2C), 132.19, 129.58, 129.25 (2C), 127.41, 124.46, 118.58, 110.95, 49.86, 26.87, 20.75. HRMS (ESI): m/z calcd. for C₂₁H₁₈N₃O₂S⁺ [M+H]⁺: 376.1120, found: 376.1111.

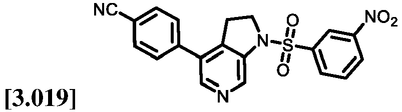

### 4-(1-((3-Nitrophenyl)sulfonyl)-2,3-dihydro-1H-pyrrolo[2,3-c]pyridin-4-yl)benzonitrile (C9)

Following the general method CC, synthesized with **CI33** (100 mg, 0.452 mmol), 3-nitrobenzenesulfonyl chloride (200 mg, 0.904 mmol) in pyridine (4 mL) to give **C9** (90.1 mg, 50.0% yield) as a white solid after chromatographic purification (PE/EA = 10/1 to 1/3). mp: 233.1-234.2 °C. ¹H-NMR (400 MHz, DMSO-*d*₆) δ 8.76 (s, 1H), 8.54 (dd, *J* = 10.5, 1.7 Hz, 2H), 8.37 (d, *J =* 8.1 Hz, 1H), 7.97-7.88 (m, 3H), 7.70 (d, *J =* 8.4 Hz, 2H), 4.03 (t, *J =* 8.4 Hz, 2H), 3.14 (t, *J* = 8.4 Hz, 2H). ¹³C-NMR (101 MHz, DMSO-*d*₆) δ 148.21, 144.46, 140.37, 139.68, 137.01, 134.91, 133.94, 133.91, 133.06, 132.70 (2C), 131.91, 133.06, 132.70 (2C), 131.90, 129.27 (2C), 128.73, 121.95, 118.55, 111.02, 50.03, 27.00. HRMS (ESI): m/z calcd. for C₂₀H₁₅N₄O₄S⁺ [M+H]⁺: 407.0814, found: 407.0814.

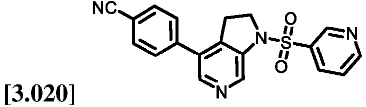

### 4-(1-(Pyridin-3-ylsulfonyl)-2,3-dihydro-1H-pyrrolo[2,3-c]pyridin-4-yl)benzonitrile (C10)

Following the general method CC, synthesized with **CI33** (100 mg, 0.452 mmol), pyridine-3-sulfonyl chloride (151 mg, 0.904 mmol) in pyridine (4 mL) to give **C10** (84.1 mg, 51.5% yield) as a white solid after chromatographic purification (PE/EA = 10/1 to 1/3). mp: 169.1-170.5 °C. ¹H-NMR (400 MHz, DMSO-*d*₆) δ 9.11 (d, *J =* 2.1 Hz, 1H), 8.89 (dd, *J* = 4.8, 1.3 Hz, 1H), 8.74 (s, 1H), 8.37 (ddd, *J* = 6.0, 4.0, 2.2 Hz, 2H), 7.94 (d, *J =* 8.4 Hz, 2H), 7.71 (d, *J =* 8.4 Hz, 2H), 7.68 (dd, *J* = 8.1, 4.9 Hz, 1H), 4.02 (t, *J =* 8.4 Hz, 2H), 3.17 (t, *J =* 8.4 Hz, 2H).¹³C-NMR (101 MHz, DMSO-*d*₆) δ 154.64, 147.41, 144.33, 140.39, 139.61, 138.57, 135.56, 135.48, 133.79, 132.69 (2C), 132.30, 129.28 (2C), 124.81, 118.56, 110.99, 49.87, 26.94. HRMS (ESI): m/z calcd. for C₁₉H₁₅N₄O₂S⁺ [M+H]⁺: 363.0916, found: 363.0898.

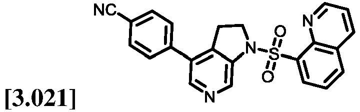

### 4-(1-(Quinolin-8-ylsulfonyl)-2,3-dihydro-1H-pyrrolo[2,3-c]pyridin-4-yl)benzonitrile (C11)

Following the general method CC, synthesized with **CI33** (100 mg, 0.452 mmol), quinoline-8-sulfonyl chloride (199 mg, 0.904 mmol) in pyridine (4 mL) to give **C11** (91.1 mg, 48.4% yield) as a white solid after chromatographic purification (PE/EA = 10/1 to 1/3). mp: 182.1-182.9 °C. ¹H-NMR (400 MHz, DMSO-*d*₆) δ 8.96 (dd, *J =* 4.2, 1.7 Hz, 1H), 8.66 (dd, *J* = 7.4, 1.3 Hz, 1H), 8.52 (dd, *J* = 8.4, 1.7 Hz, 1H), 8.42 (s, 1H), 8.35 (dd, *J* = 8.2, 1.2 Hz, 1H), 8.19 (s, 1H), 7.92 (d, *J* = 8.4 Hz, 2H), 7.83 (t, *J* = 7.8 Hz, 1H), 7.67 (dd, J = 8.3, 3.6 Hz, 3H), 4.57 (t, *J =* 8.5 Hz, 2H), 3.23 (t, *J* = 8.6 Hz, 2H). ¹³C-NMR (101 MHz, DMSO-*d*₆) δ 151.72, 143.08, 143.01, 140.68, 139.14, 139.00, 137.12, 134.96, 134.79, 134.16, 133.35, 132.66 (2C), 132.01, 129.21 (2C), 128.69, 125.86, 122.78, 118.61, 110.84, 50.47, 26.99. HRMS (ESI): m/z calcd. for C₂₃H₁₇N₄O₂S⁺ [M+H]⁺: 413.1072, found: 413.1058.

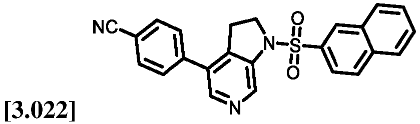

### 4-(1-(Naphthalen-2-ylsulfonyl)-2,3-dihydro-1H-pyrrolo[2,3-c]pyridin-4-yl)benzonitrile (C12)

Following the general method CC, synthesized with **CI33** (100 mg, 0.452 mmol), naphthalene-2-sulfonyl chloride (205 mg, 0.904 mmol) in pyridine (4 mL) to give **C12** (91.1 mg, 48.8% yield) as a white solid after chromatographic purification (PE/EA = 10/1 to 1/3). mp: 181.1-182.0 °C. ¹H-NMR (400 MHz, DMSO-*d*₆) δ 8.78 (d, *J =* 53.9 Hz, 2H), 8.28 (t, *J =* 22.7 Hz, 2H), 8.13 (d, *J =* 8.7 Hz, 1H), 8.04 (d, *J =* 7.9 Hz, 1H), 7.89 (dd, *J* = 10.9, 5.0 Hz, 3H), 7.77-7.60 (m, 4H), 4.02 (t, *J* = 8.4 Hz, 2H), 3.14 (t, *J* = 8.4 Hz, 2H). ¹³C-NMR (101 MHz, DMSO-*d*₆) δ 143.88, 140.48, 139.23, 138.64, 138.15, 134.81, 134.75, 133.75, 132.62 (2C), 131.72, 129.88, 129.56, 129.46, 129.23, 129.10, 127.90, 127.85, 122.14, 118.55, 110.91, 49.95, 26.88. HRMS (ESI): m/z calcd. for C₂₄H₁₈N₃O₂S⁺ [M+H]⁺: 412.1120, found: 412.1114.

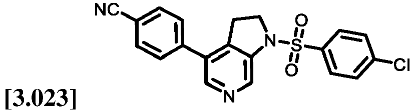

### 4-(1-((4-Chlorophenyl)sulfonyl)-2,3-dihydro-1H-pyrrolo[2,3-c]pyridin-4-yl)benzonitrile (C13)

Following the general method CC, synthesized with **CI33** (100 mg, 0.452 mmol), 4-chlorobenzenesulfonyl chloride (131 mg, 0.904 mmol) in pyridine (4 mL) to give **C13** (97.0 mg, 45.8% yield) as a white solid after chromatographic purification (PE/EA = 10/1 to 1/3). mp: 166.8-167.9 °C. ¹H-NMR (400 MHz, DMSO-*d*₆) δ 8.71 (s, 1H), 8.36 (s, 1H), 7.95 (dd, *J* = 8.5, 2.7 Hz, 4H), 7.71 (dd, *J* = 8.4, 3.4 Hz, 4H), 3.95 (t, *J =* 8.4 Hz, 2H), 3.16 (t, *J =* 8.4 Hz, 2H). ¹³C-NMR (101 MHz, DMSO-*d*₆) δ 144.14, 140.43, 139.48, 139.27, 136.32, 136.24, 134.33, 133.78, 132.69 (2C), 129.94 (2C), 129.28 (2C), 129.23 (2C), 118.57, 110.98, 49.86, 26.91. HRMS (ESI): m/z calcd. for C₂₀H₁₅ClN₃O₂S⁺ [M+H]⁺: 396.0574, found: 396.0570.

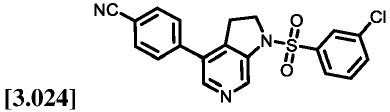

### 4-(1-((3-Chlorophenyl)sulfonyl)-2,3-dihydro-1H-pyrrolo[2,3-c]pyridin-4-yl)benzonitrile (C14)

Following the general method CC, synthesized with **CI33** (100 mg, 0.452 mmol), 3-chlorobenzenesulfonyl chloride (131 mg, 0.904 mmol) in pyridine (4 mL) to give **C14** (97.0 mg, 45.2% yield) as a white solid after chromatographic purification (PE/EA = 10/1 to 1/3). mp: 194.1-195.2 °C. ¹H-NMR (400 MHz, DMSO-*d*₆) δ 8.72 (s, 1H), 8.36 (s, 1H), 7.99-7.89 (m, 4H), 7.82 (ddd, *J* = 8.1, 2.0, 0.9 Hz, 1H), 7.75-7.63 (m, 3H), 3.99 (t, *J =* 8.4 Hz, 2H), 3.16 (t, *J =* 8.4 Hz, 2H). ¹³C-NMR (101 MHz, DMSO-*d*₆) δ 144.69, 140.90, 140.01, 139.16, 137.87, 134.94, 134.72, 134.29, 133.17 (2C), 132.77, 132.30, 129.76 (2C), 127.24, 126.53, 119.05, 111.47, 50.44, 27.40. HRMS (ESI): m/z calcd. for C₂₀H₁₅ClN₃O₂S⁺ [M+H]⁺: 396.0574, found: 396.0558.

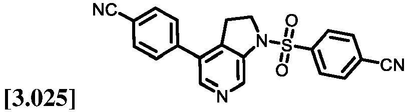

### 4-((4-(4-Cyanophenyl)-2,3-dihydro-1H-pyrrolo[2,3-c]pyridin-1-yl)sulfonyl)benzonitrile (C15)

Following the general method CC, synthesized with **CI33** (100 mg, 0.452 mmol), 4-cynobenzenesulfonyl chloride (132 mg, 0.904 mmol) in pyridine (4 mL) to give **C15** (94.0 mg, 45.0% yield) as a white solid after chromatographic purification (PE/EA = 10/1 to 1/3). mp: 210.1-211.5 °C. ¹H-NMR (400 MHz, DMSO-*d*₆) δ 8.72 (s, 1H), 8.37 (s, 1H), 8.18-8.06 (m, 4H), 7.94 (d, *J =* 8.3 Hz, 2H), 7.71 (d, *J =* 8.3 Hz, 2H), 3.99 (t, *J =* 8.4 Hz, 2H), 3.16 (t, *J =* 8.4 Hz, 2H). ¹³C-NMR (101 MHz, DMSO-*d*₆) δ 144.34, 140.38, 140.13, 139.61, 139.54,134.25, 133.89 (2C), 133.83, 132.70 (2C), 129.29 (2C), 128.03 (2C), 118.57, 117.42, 116.55, 111.01, 49.96, 26.94. HRMS (ESI): m/z calcd. for C₂₁H₁₅N₄O₂S⁺ [M+H]⁺: 387.0916, found: 387.0911.

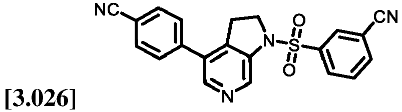

### 3-((4-(4-Cyanophenyl)-2,3-dihydro-1H-pyrrolo[2,3-c]pyridin-1-yl)sulfonyl)benzonitrile (C16)

Following the general method CC, synthesized with **CI33** (100 mg, 0.452 mmol), 3-cynobenzenesulfonyl chloride (132 mg, 0.904 mmol) in pyridine (4 mL) to give **C16** (94.3 mg, 45.1% yield) as a white solid after chromatographic purification (PE/EA = 10/1 to 1/3). mp: 218.5-219.0 °C. ¹H-NMR (400 MHz, DMSO-*d*₆) δ 8.74 (s, 1H), 8.50 (s, 1H), 8.36 (s, 1H), 8.23 (dd, *J =* 19.6, 8.0 Hz, 2H), 7.94 (d, *J =* 8.4 Hz, 2H), 7.83 (t, *J* = 7.9 Hz, 1H), 7.71 (d, *J =* 8.4 Hz, 2H), 4.03 (t, *J =* 8.4 Hz, 2H), 3.16 (t, *J =* 8.4 Hz, 2H). ¹³C-NMR (101 MHz, DMSO-*d*₆) δ 144.26, 140.42, 139.57, 137.75, 136.95, 136.92, 133.86, 133.82, 132.70 (2C), 131.66, 131.14, 131.02, 129.28 (2C), 118.57, 117.31, 113.16, 110.99, 50.01, 26.93. HRMS (ESI): m/z calcd. for C₂₁H₁₅N₄O₂S⁺ [M+H]⁺: 387.0916, found: 387.0907.

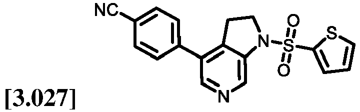

### 4-(1-(Thiophen-2-ylsulfonyl)-2,3-dihydro-1H-pyrrolo[2,3-c]pyridin-4-yl)benzonitrile (C17)

Following the general method CC, synthesized with **CI33** (100 mg, 0.452 mmol), thiophene-2-sulfonyl chloride (156 mg, 0.904 mmol) in pyridine (4 mL) to give **C17** (90.3 mg, 54.0% yield) as a white solid after chromatographic purification (PE/EA = 10/1 to 1/3). mp: 133.0-133.5 °C. ¹H-NMR (400 MHz, DMSO-*d*₆) δ 8.69 (d, *J =* 109.8 Hz, 2H), 8.05 (d, *J* = 4.8 Hz, 1H), 7.96 (d, *J* = 7.7 Hz, 2H), 7.88 (s, 1H), 7.74 (d, *J =* 7.7 Hz, 2H), 7.31-7.21 (m, 1H), 3.95 (t, *J* = 8.1 Hz, 2H), 3.17 (t, *J =* 7.8 Hz, 2H). ¹³C-NMR (101 MHz, DMSO-*d*₆) δ 144.86, 140.98, 139.90, 135.74, 135.68, 134.73, 134.64, 134.61, 134.15, 133.18 (2C), 129.80 (2C), 128.95, 119.05, 111.49, 50.51, 27.41. HRMS (ESI): m/z calcd. for C₁₈H₁₄N₃O₂S₂⁺ [M+H]⁺: 368.0527, found: 368.0513.

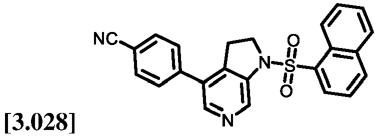

### 4-(1-(Naphthalen-1-ylsulfonyl)-2,3-dihydro-1H-pyrrolo[2,3-c]pyridin-4-yl)benzonitrile (C18)

Following the general method CC, synthesized with **CI33** (100 mg, 0.452 mmol), naphthalene-1-sulfonyl chloride (205 mg, 0.904 mmol) in pyridine (4 mL) to give **C18** (86.1 mg, 46.1% yield) as a white solid after chromatographic purification (PE/EA = 10/1 to 1/3). mp: 172.0-173.5 °C. ¹H-NMR (400 MHz, DMSO-*d*₆) δ 8.67-8.56 (m, 2H), 8.40-8.29 (m, 3H), 8.13 (d, *J* = 7.6 Hz, 1H), 7.93 (d, *J =* 8.4 Hz, 2H), 7.78-7.63 (m, 5H), 4.09 (t, *J =* 8.4 Hz, 2H), 3.13 (t, *J =* 8.4 Hz, 2H). ¹³C-NMR (101 MHz, DMSO-*d*₆) δ 143.80, 140.54, 139.35, 135.37, 134.26, 134.06, 133.66, 132.69 (2C), 132.45, 130.06, 129.25 (2C), 128.72, 127.93, 127.30, 124.86, 123.91, 118.59, 116.40, 110.95, 50.17, 27.02. HRMS (ESI): m/z calcd. for C₂₄H₁₇N₃O₂S⁺ [M+H]⁺: 412.1120, found: 412.1113.

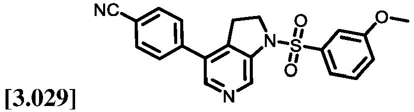

### 4-(1-((3-Methoxyphenyl)sulfonyl)-2,3-dihydro-1H-pyrrolo[2,3-c]pyridin-4-yl)benzonitrile (C19)

Following the general method CC, synthesized with **CI33** (100 mg, 0.452 mmol), 3-methoxybenzenesulfonyl chloride (188 mg, 0.904 mmol) in pyridine (4 mL) to give **C19** (89.3 mg, 50.1% yield) as a white solid after chromatographic purification (PE/EA = 10/1 to 1/3). mp: 163.0-144.0 °C. ¹H-NMR (400 MHz, DMSO-*d*₆) δ 8.71 (s, 1H), 8.34 (s, 1H), 7.94 (d, *J =* 8.4 Hz, 2H), 7.71 (d, *J =* 8.4 Hz, 2H), 7.51 (dt, *J =* 8.0, 4.6 Hz, 2H), 7.36-7.27 (m, 2H), 3.96 (t, *J* = 8.4 Hz, 2H), 3.14 (t, *J =* 8.4 Hz, 2H). ¹³C-NMR (101 MHz, DMSO-*d*₆) δ 159.65, 144.02, 140.46, 139.44, 138.9, 136.61, 133.96, 132.68 (2C), 132.20, 131.03, 129.25 (2C), 120.00, 119.31, 118.57, 112.08, 110.96, 55.72, 49.92, 26.90. HRMS (ESI): m/z calcd. for C₂₁H₁₈N₃O₃S⁺ [M+H]⁺: 392.1069, found: 392.0929.

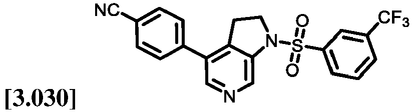

### 4-(1-((3-[Trifluoromethyl]phenyl)sulfonyl)-2,3-dihydro-1H-pyrrolo[2,3-c]pyridin-4-yl)benzo nitrile (C20)

Following the general method CC, synthesized with **CI33** (100 mg, 0.452 mmol), (3-triflouromethyl)benzenesulfonyl chloride (214 mg, 0.904 mmol) in pyridine (4 mL) to give **C20** (98.3 mg, 50.3% yield) as a white solid after chromatographic purification (PE/EA = 10/1 to 1/3). mp: 182.0-182.9 °C. ¹H-NMR (400 MHz, DMSO-*d*₆) δ 8.74 (s, 1H), 8.37 (s, 1H), 8.28 (d, *J =* 8.0 Hz, 1H), 8.14 (d, *J =* 8.3 Hz, 2H), 7.90 (t, *J =* 7.8 Hz, 1H), 7.75-7.66 (m, 2H), 4.00 (t, *J* = 8.4 Hz, 2H), 3.14 (t, *J* = 8.4 Hz, 2H). ¹³C-NMR (101 MHz, DMSO-*d*₆) δ 144.35, 140.37, 139.68, 138.64, 136.81, 133.91, 132.69 (2C), 132.30, 131.58, 11.36, 130.97 (³*J*_{CF} = 4 Hz), 130.25 (²*J*_{CF} = 33 Hz), 129.25 (s, 2C), 123.73 (³*J*_{CF} = 3 Hz), 122.47 (¹*J*_{CF} = 252 Hz), 118.55, 111.00, 49.99, 26.95. ¹⁹F NMR (377 MHz, DMSO-*d*₆) δ -61.34. HRMS (ESI): m/z calcd. for C₂₁H₁₅F₃N₃O₂S⁺ [M+H]⁺: 430.0837, found: 430.0824.

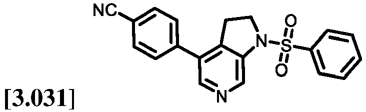

### 4-(1-(Phenylsulfonyl)-2,3-dihydro-1H-pyrrolo[2,3-c]pyridin-4-yl)benzonitrile (C21)

Following the general method CC, synthesized with **CI33** (100 mg, 0.452 mmol), benzenesulfonyl chloride (154 mg, 0.904 mmol) in pyridine (4 mL) to give **C21** (81.3 mg, 49.4% yield) as a white solid after chromatographic purification (PE/EA = 10/1 to 1/3). mp: 115.0-115.9 °C. ¹H-NMR (400 MHz, DMSO-*d*₆) δ 8.71 (s, 1H), 8.33 (s, 1H), 7.96-7.89 (m, 4H), 7.71 (dd, *J* = 17.0, 7.8 Hz, 3H), 7.63 (t, *J =* 7.7 Hz, 2H), 3.95 (t, *J =* 8.4 Hz, 2H), 3.13 (t, *J =* 8.4 Hz, 2H). ¹³C-NMR (101 MHz, DMSO-*d*₆) δ 143.97, 140.45, 139.34, 139.00, 135.53, 134.20, 133.87, 132.67 (2C), 132.22, 129.77 (2C), 129.24 (2C), 127.28 (2C), 118.57, 110.96, 49.85, 26.90. HRMS (ESI): m/z calcd. for C₂₀H₁₅₆N₃O₂S⁺ [M+H]⁺: 362.0963, found: 362.0953.

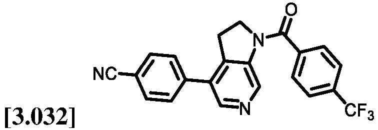

### 4-(1-(4-(Trifluoromethyl)benzoyl)-2,3-dihydro-1H-pyrrolo[2,3-c]pyridin-4-yl)benzonitrile (C22)

Following the general method CA, synthesized with **CI33** (100 mg, 0.452 mmol), (4-triflouromethyl)benzoyl chloride (137 mg, 0.904 mmol), DMAP (17 mg, 0.12 mmol), Et₃N (89 mg, 0.87 mmol) in anhydrous dichloromethane (4 mL) to give **C22** (93.3 mg, 52.6% yield) as a white solid after chromatographic purification (PE/EA = 10/1 to 1/3). mp: 150.1-152.5 °C. ¹H-NMR (400 MHz, DMSO-*d*₆) δ 9.31 (s, 1H), 8.43 (s, 1H), 7.99 (d, *J* = 8.3 Hz, 2H), 7.89 (q, *J* = 8.4 Hz, 4H), 7.79 (d, *J* = 8.4 Hz, 2H), 4.04 (t, *J =* 8.0 Hz, 2H), 3.27 (t, *J* = 8.3 Hz, 2H). ¹³C-NMR (101 MHz, DMSO-*d*₆) δ 167.04, 144.25, 140.82, 140.3, 140.16, 140.10, 137.12, 132.80 (2C), 131.99, 130.46 (²*J*_{CF} = 30 Hz), 129.31 (2C), 127.95 (2C), 123.31 (¹*J*_{CF} = 386 Hz), 125.67 (³*J*_{CF} = 2 Hz), 118.65, 110.96, 50.18, 27.64. ¹⁹F NMR (377 MHz, DMSO-*d*₆) δ -61.36. HRMS (ESI): m/z calcd. for C₂₂H₁₅F₃N₃O⁺ [M+H]⁺: 394.1167, found: 394.1145.

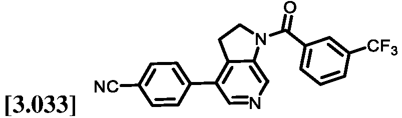

### 4-(1-(3-(Trifluoromethyl)benzoyl)-2,3-dihydro-1H-pyrrolo[2,3-c]pyridin-4-yl)benzonitrile (C23)

Following the general method CA, synthesized with **CI33** (100 mg, 0.452 mmol), (3-triflouromethyl)benzoyl chloride (137 mg, 0.904 mmol), DMAP (17 mg, 0.12 mmol), Et₃N (89 mg, 0.87 mmol) in anhydrous dichloromethane (4 mL) to give **C23** (90.3 mg, 50.9% yield) as a white solid after chromatographic purification (PE/EA = 10/1 to 1/3). mp: 150.3-151.5 °C. ¹H-NMR (400 MHz, DMSO-*d*₆) δ 9.33 (s, 1H), 8.42 (s, 1H), 8.05-7.88 (m, 5H), 7.78 (t, *J =* 8.1 Hz, 3H), 4.06 (t, *J* = 8.2 Hz, 2H), 3.26 (t, *J* = 8.3 Hz, 2H). ¹³C-NMR (101 MHz, DMSO-*d*₆) δ 166.82, 144.19, 144.16, 140.83, 140.04, 137.26, 137.20, 132.79 (2C), 131.10, 129.89, 129.37 (²*J*_{CF} = 31 Hz), 129.29 (2C), 127.10 (³*J*_{CF} = 2 Hz), 123.85 (¹*J*_{CF} = 271 Hz), 123.89 (³*J*_{CF} = 4 Hz), 118.63, 110.91, 50.15, 27.64. ¹⁹F NMR (377 MHz, DMSO-*d*₆) δ -61.17. HRMS (ESI): m/z calcd. for C₂₂H₁₅F₃N₃O⁺ [M+H]⁺: 394.1167, found: 394.1149.

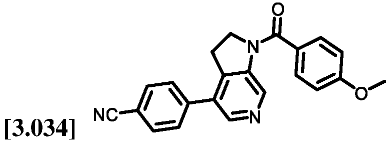

### 4-(1-(4-Methoxybenzoyl)-2,3-dihydro-1H-pyrrolo[2,3-c]pyridin-4-yl)benzonitrile (C24)

Following the general method CA, synthesized with CI33 (100 mg, 0.452 mmol), 4-methoxybenzoyl chloride (113 mg, 0.904 mmol), DMAP (17 mg, 0.12 mmol), Et₃N (89 mg, 0.87 mmol) in anhydrous dichloromethane (4 mL) to give **C24** (94.3 mg, 58.6% yield) as a white solid after chromatographic purification (PE/EA = 10/1 to 1/3). mp: 209.1-210.5 °C. ¹H-NMR (400 MHz, DMSO-*d*₆) δ 8.98 (s, 1H), 8.37 (s, 1H), 7.99 (d, *J* = 8.4 Hz, 2H), 7.78 (d, *J =* 8.4 Hz, 2H), 7.63 (d, *J* = 8.7 Hz, 2H), 7.06 (d, *J =* 8.8 Hz, 2H), 4.11 (t, *J =* 8.3 Hz, 2H), 3.83 (d, *J =* 4.8 Hz, 3H), 3.25 (t, *J* = 8.3 Hz, 2H). ¹³C-NMR (101 MHz, DMSO-*d*₆) δ 168.56, 161.52, 144.08, 141.44, 140.36, 137.43, 137.36, 133.23 (2C), 132.43, 129.89, 129.82 (2C), 129.76 (2C), 128.58, 119.13, 114.27 (2C), 111.31, 55.85, 50.89, 28.14. HRMS (ESI): m/z calcd. for C₂₂H₁₈N₃O₂⁺ [M+H]⁺: 356.1399, found: 356.1385.

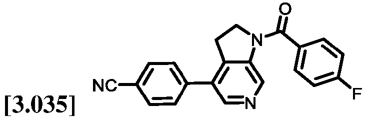

### 4-(1-(4-Fluorobenzoyl)-2,3-dihydro-1H-pyrrolo[2,3-c]pyridin-4-yl)benzonitrile (C25)

Following the general method CA, synthesized with **CI33** (100 mg, 0.452 mmol), 4-flourobenzoyl chloride (103 mg, 0.904 mmol), DMAP (17 mg, 0.12 mmol), Et₃N (89 mg, 0.87 mmol) in anhydrous dichloromethane (4 mL) to give **C25** (94.3 mg, 58.6% yield) as a white solid after chromatographic purification (PE/EA = 10/1 to 1/3). mp: 173.0-173.9 °C. ¹H-NMR (400 MHz, DMSO-*d*₆) δ 9.20 (s, 1H), 8.41 (s, 1H), 7.99 (d, *J =* 8.2 Hz, 2H), 7.84-7.68 (m, 4H), 7.37 (t, *J =* 8.8 Hz, 2H), 4.08 (t, *J* = 8.2 Hz, 2H), 3.26 (t, *J* = 8.2 Hz, 2H). ¹³C-NMR (101 MHz, DMSO-*d*₆) δ 167.40, 164.16 (¹*J*_{CF} = 247 Hz), 143.91, 140.88, 140.41, 139.98, 136.94, 132.87(s, 2C), 132.65 (⁴*J*_{CF} = 3 Hz), 132.00, 129.87 (²*J*_{CF} = 8 Hz, 2C), 129.28 (2C), 118.64, 115.60 (²*J*_{CF} = 22 Hz, 2C), 110.87, 50.27, 27.62. ¹⁹F NMR (376 MHz, DMSO-*d*₆) δ -109.69. HRMS (ESI): m/z calcd. for C₂₁H₁₅FN₃O⁺ [M+H]⁺: 344.1199, found: 344.1194.

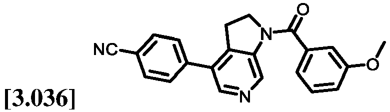

### 4-(1-(3-Methoxybenzoyl)-2,3-dihydro-1H-pyrrolo[2,3-c]pyridin-4-yl)benzonitrile (C26)

Following the general method CA, synthesized with **CI33** (100 mg, 0.452 mmol), 3-methoxybenzoyl chloride (113 mg, 0.904 mmol), DMAP (17 mg, 0.12 mmol), Et₃N (89 mg, 0.87 mmol) in anhydrous dichloromethane (4 mL) to give **C26** (92.3 mg, 57.6% yield) as a white solid after chromatographic purification (PE/EA = 10/1 to 1/3). mp: 198.0-198.9 °C. ¹H-NMR (400 MHz, DMSO-*d*₆) δ = 9.21 (s, 1H), 8.39 (s, 1H), 7.99 (d, *J =* 8.3 Hz, 2H), 7.78 (d, *J =* 8.3 Hz, 2H), 7.44 (t, *J =* 8.0 Hz, 1H), 7.21-7.07 (m, 3H), 4.05 (t, *J* = 8.3 Hz, 2H), 3.81 (s, 3H), 3.25 (t, *J =* 8.3 Hz, 2H). ¹³C-NMR (101 MHz, DMSO-*d*₆) δ 168.51, 159.67, 144.35, 141.37, 140.82, 140.80, 140.46, 138.05, 133.24 (2C), 132.47, 130.37, 129.75 (2C), 119.41, 119.12, 116.63, 112.8, 111.34, 55.81, 50.37, 27.99. HRMS (ESI): m/z calcd. for C₂₂H₁₈N₃O₂⁺ [M+H]⁺: 356.1399, found: 356.1384.

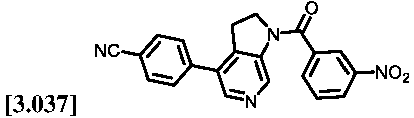

### 4-(1-(3-Nitrobenzoyl)-2,3-dihydro-1H-pyrrolo[2,3-c]pyridin-4-yl)benzonitrile (C27)

Following the general method CA, synthesized with **CI33** (100 mg, 0.452 mmol), 3-nitrobenzoyl chloride (120 mg, 0.904 mmol), DMAP (17 mg, 0.12 mmol), Et₃N (89 mg, 0.87 mmol) in anhydrous dichloromethane (4 mL) to give **C27** (90.3 mg, 53.6% yield) as a white solid after chromatographic purification (PE/EA = 10/1 to 1/3). mp: 192.5-192.9 °C. ¹H-NMR (400 MHz, DMSO-*d*₆) δ 9.29 (s, 1H), 8.43 (ddd, *J =* 9.7, 5.9, 1.6 Hz, 3H), 8.11 (d, *J =* 7.7 Hz, 1H), 7.99 (d, *J* = 8.4 Hz, 2H), 7.89 -7.74 (m, 3H), 4.08 (t, *J* = 8.2 Hz, 2H), 3.28 (t, *J =* 8.2 Hz, 2H). ¹³C-NMR (101 MHz, DMSO-*d*₆) δ 166.57, 148.22, 144.76, 144.72, 141.28, 140.65, 140.56, 140.54, 138.10, 133.97, 133.27 (2C), 132.51, 130.92, 129.77 (2C), 125.61, 122.58, 119.11, 111.27, 50.59, 27.97. HRMS (ESI): m/z calcd. for C₂₂H₁₅N₄O₃⁺ [M+H]⁺: 371.1144, found: 371.1136.

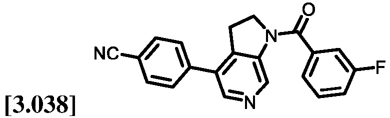

### 4-(1-(3-fluorobenzoyl)-2,3-dihydro-1H-pyrrolo[2,3-c]pyridin-4-yl)benzonitrile (C28)

Following the general method CA, synthesized with **CI33** (100 mg, 0.452 mmol), 3-flourobenzoyl chloride (103 mg, 0.904 mmol), DMAP (17 mg, 0.12 mmol), Et₃N (89 mg, 0.87 mmol) in anhydrous dichloromethane (4 mL) to give **C28** (85.3 mg, 55.6% yield) as a white solid after chromatographic purification (PE/EA = 10/1 to 1/3). mp: 184.5-184.9 °C. ¹H-NMR (400 MHz, DMSO-*d*₆) δ 9.29 (s, 1H), 8.42 (s, 1H), 7.99 (d, *J* = 8.3 Hz, 2H), 7.79 (d, *J =* 8.3 Hz, 2H), 7.65-7.33 (m, 4H), 4.06 (t, *J* = 8.2 Hz, 2H), 3.26 (t, *J* = 8.3 Hz, 2H). ¹³C-NMR (101 MHz, DMSO-*d*₆) δ 166.94, 161.82 (¹*J*_{CF} = 244 Hz), 152.03, 144.10, 140.84, 140.09, 138.39 (³*J*_{CF} = 7 Hz), 136.99, 132.78 (2C), 132.06, 131.97 (³*J*_{CF} = 8 Hz), 129.29 (2C), 123.20 (⁴*J*_{CF} = 3 Hz), 118.64, 117.44 (²*J*_{CF} = 22 Hz), 114.09 (²*J*_{CF} = 22 Hz), 110.90, 50.12, 27.60. ¹⁹F NMR (377 MHz, DMSO) δ -109.67. HRMS (ESI): m/z calcd. for C₂₁H₁₅FN₃O⁺ [M+H]⁺: 344.1199, found: 344.1185.

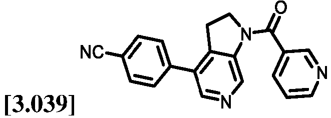

### 4-(1-Nicotinoyl-2,3-dihydro-1H-pyrrolo[2,3-c]pyridin-4-yl)benzonitrile (C29)

Following the general method CA, synthesized with **CI33** (100 mg, 0.452 mmol), nicotinoyl chloride (118 mg, 0.904 mmol), DMAP (17 mg, 0.12 mmol), Et₃N (89 mg, 0.87 mmol) in anhydrous dichloromethane (4 mL) to give **C29** (79.1 mg, 53.3% yield) as a white solid after chromatographic purification (PE/EA = 10/1 to 1/3). mp: 198.0-198.9 °C. ¹H-NMR (400 MHz, DMSO-*d*₆) δ 9.28 (s, 1H), 8.85 (s, 1H), 8.74 (d, *J =* 3.2 Hz, 1H), 8.43 (s, 1H), 8.09 (dt, *J =* 7.8, 1.7 Hz, 1H), 7.99 (d, *J =* 8.5 Hz, 2H), 7.79 (d, *J =* 8.5 Hz, 2H), 7.56 (dd, *J* = 7.9, 4.9 Hz, 1H), 4.09 (t, *J* = 8.2 Hz, 2H), 3.27 (t, *J =* 8.3 Hz, 2H). ¹³C-NMR (101 MHz, DMSO-*d*₆) δ 166.28, 151.26, 147.83, 144.21, 140.82, 140.00, 136.94, 134.88, 132.78 (2C), 132.15, 132.08, 132.02, 129.30 (2C), 123.61, 118.64, 110.90, 50.16, 27.64. HRMS (ESI): m/z calcd. for C₂₀H₁₅N₄O⁺ [M+H]⁺: 327.1246, found: 327.1235.

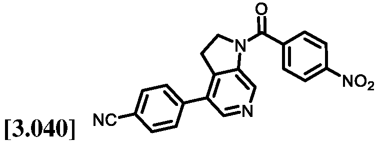

### 4-(1-(4-Nitrobenzoyl)-2,3-dihydro-1H-pyrrolo[2,3-c]pyridin-4-yl)benzonitrile (C30)

Following the general method CA, synthesized with **CI33** (100 mg, 0.452 mmol), 4-nitrobenzoyl chloride (120 mg, 0.904 mmol), DMAP (17 mg, 0.12 mmol), Et₃N (89 mg, 0.87 mmol) in anhydrous dichloromethane (4 mL) to give **C30** (90.2 mg, 54.0% yield) as a white solid after chromatographic purification (PE/EA = 10/1 to 1/3). mp: 215.0-215.9 °C. ¹H-NMR (400 MHz, DMSO-*d*₆) δ 10.64-10.58 (m, 1H), 9.74-9.53 (m, 1H), 9.32 (s, 1H), 8.44 (s, 1H), 8.36 (d, *J =* 8.7 Hz, 2H), 7.99 (d, *J =* 8.3 Hz, 2H), 7.92 (d, *J =* 8.6 Hz, 2H), 7.79 (d, *J =* 8.3 Hz, 2H), 4.02 (t, *J* = 7.2 Hz, 2H), 3.28 (t, *J* = 8.3 Hz, 2H). ¹³C-NMR (101 MHz, DMSO-*d*₆) δ 167.03, 148.83, 144.83, 142.58, 141.25, 140.56, 140.51, 137.50, 133.27 (2C), 132.57, 129.77 (2C), 129.01 (2C), 124.37, 119.11, 111.40, 50.56, 28.00. HRMS (ESI): m/z calcd. for C₂₀H₁₅N₄O₃⁺ [M+H]⁺: 371.1144, found: 371.1137.

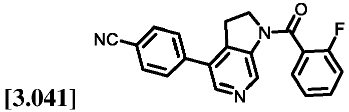

### 4-(1-(2-Fluorobenzoyl)-2,3-dihydro-1H-pyrrolo[2,3-c]pyridin-4-yl)benzonitrile (C31)

Following the general method CA, synthesized with **CI33** (100 mg, 0.452 mmol), 2-flourobenzoyl chloride (103 mg, 0.904 mmol), DMAP (17 mg, 0.12 mmol), Et₃N (89 mg, 0.87 mmol) in anhydrous dichloromethane (4 mL) to give **C31** (92.2 mg, 59.6% yield) as a white solid after chromatographic purification (PE/EA = 10/1 to 1/3). mp: 184.0-184.9 °C. ¹H-NMR (400 MHz, DMSO-*d*₆) δ 9.36 (s, 1H), 8.45 (s, 1H), 7.99 (d, *J =* 8.3 Hz, 2H), 7.79 (d, *J =* 8.3 Hz, 2H), 7.61 (t, *J* = 6.9 Hz, 2H), 7.46-7.32 (m, 2H), 3.91 (t, *J =* 7.8 Hz, 2H), 3.28 (t, *J =* 8.3 Hz, 2H). ¹³C-NMR (101 MHz, DMSO-*d*₆) δ 163.88, 158.83 (¹*J*_{CF} = 244 Hz), 154.66, 144.46, 140.81, 140.08, 136.70, 136.67, 132.76 (2C), 132.30 (³*J*_{CF} = 8 Hz), 129.31 (2C), 128.65, 125.11, 124.57 (²*J*_{CF} = 17 Hz), 118.62, 116.17 (²*J*_{CF} = 17 Hz), 110.92, 48.98, 27.32. ¹⁹F NMR (377 MHz, DMSO-*d*₆) δ -116.26. HRMS (ESI): m/z calcd. for C₂₁H₁₅FN₃O⁺ [M+H]⁺: 344.1199, found: 344.1115.

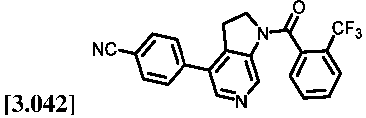

### 4-(1-(2-(Trifluoromethyl)benzoyl)-2,3-dihydro-1H-pyrrolo[2,3-c]pyridin-4-yl)benzonitrile (C32)

Following the general method CA, synthesized with **CI33** (100 mg, 0.452 mmol), (2-triflouromethyl)benzoyl chloride (137 mg, 0.904 mmol), DMAP (17 mg, 0.12 mmol), Et₃N (89 mg, 0.87 mmol) in anhydrous dichloromethane (4 mL) to give **C32** (92.2 mg, 59.2% yield) as a white solid after chromatographic purification (PE/EA = 10/1 to 1/3). mp: 200.0-200.9 °C. ¹H-NMR (400 MHz, DMSO-*d*₆) δ 9.35 (s, 1H), 8.46 (s, 1H), 7.98 (d, *J* = 8.3 Hz, 2H), 7.95-7.71 (m, 6H), 3.75 (s, 2H), 3.30-3.19 (m, 2H). ¹³C-NMR (101 MHz, DMSO-*d*₆) δ 165.77, 144.52, 140.79, 139.91, 136.45, 134.55 (³*J*_{CF} = 2 Hz), 133.97, 133.23, 132.75 (2C), 130.28, 129.34 (2C), 127.48, 126.77 (³*J*_{CF} = 4 Hz,), 125.01 (²*J*_{CF} = 31 Hz), 123.72 (¹*J*_{CF} = 269 Hz), 118.62, 110.93, 49.59, 27.31.¹⁹F NMR (377 MHz, DMSO-*d*₆) δ -58.62. HRMS (ESI): m/z calcd. for C₂₁H₁₅F₃N₃O⁺ [M+H]⁺: 394.1167, found: 394.1069.

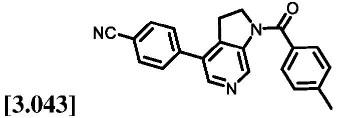

### 4-(1-(4-Methylbenzoyl)-2,3-dihydro-1H-pyrrolo[2,3-c]pyridin-4-yl)benzonitrile (C33)

Following the general method CA, synthesized with **CI33** (100 mg, 0.452 mmol), 4-methylbenzoyl chloride (120 mg, 0.904 mmol), DMAP (17 mg, 0.12 mmol), Et₃N (89 mg, 0.87 mmol) in anhydrous dichloromethane (4 mL) to give **C33** (92.1 mg, 58.5% yield) as a white solid after chromatographic purification (PE/EA = 10/1 to 1/3). mp: 197.0-197.9 °C. ¹H-NMR (400 MHz, DMSO-*d*₆) δ 9.10 (s, 1H), 8.38 (s, 1H), 7.98 (d, *J =* 8.3 Hz, 2H), 7.78 (d, *J =* 8.3 Hz, 2H), 7.54 (d, *J* = 8.0 Hz, 2H), 7.33 (d, *J =* 7.9 Hz, 2H), 4.07 (t, *J =* 8.3 Hz, 2H), 3.25 (t, *J =* 8.3 Hz, 2H), 2.38 (s, 3H). ¹³C-NMR (101 MHz, DMSO-*d*₆) δ 168.40, 143.72, 140.93, 140.48, 139.93, 134.24, 133.31, 132.75 (2C), 131.96, 129.27 (2C), 129.05 (2C), 127.18 (2C), 118.64, 116.39, 110.84, 50.24, 27.58, 21.01. HRMS (ESI): m/z calcd. for C₂₂H₁₈N₃O⁺ [M+H]⁺: 340.1450, found: 340.1438.

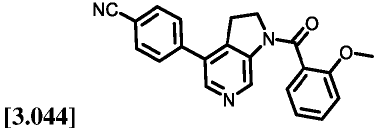

### 4-(1-(2-Methoxybenzoyl)-2,3-dihydro-1H-pyrrolo[2,3-c]pyridin-4-yl)benzonitrile (C34)

Following the general method CA, synthesized with **CI33** (100 mg, 0.452 mmol), 3-methoxybenzoyl chloride (110 mg, 0.904 mmol), DMAP (17 mg, 0.12 mmol), Et₃N (89 mg, 0.87 mmol) in anhydrous dichloromethane (4 mL) to give **C34** (83.1 mg, 51.6% yield) as a white solid after chromatographic purification (PE/EA = 10/1 to 1/3). mp: 56.0-56.9 °C. ¹H-NMR (400 MHz, DMSO-*d*₆) δ 9.35 (s, 1H), 8.41 (s, 1H), 7.98 (d, *J* = 8.3 Hz, 2H), 7.79 (d, *J =* 8.2 Hz, 2H), 7.48 (t, *J* = 7.5 Hz, 1H), 7.35 (dd, *J* = 7.4, 1.3 Hz, 1H), 7.18 (d, *J =* 8.4 Hz, 1H), 7.07 (t, *J =* 7.3 Hz, 1H), 3.83 (s, 3H), 3.82-3.73 (m, 2H), 3.24 (t, *J* = 8.4 Hz, 2H). ¹³C-NMR (101 MHz, DMSO-*d*₆) δ 167.15, 155.28, 144.53, 141.43, 140.68, 140.30, 136.89, 133.21 (2C), 132.61, 131.65, 129.79 (2C), 127.96, 126.59, 121.29, 119.12, 112.26, 111.33, 56.14, 48.85, 27.65. HRMS (ESI): m/z calcd. for C₂₂H₁₈N₃O₂⁺ [M+H]⁺: 356.1399, found: 356.1388.

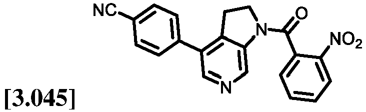

### 4-(1-(2-Nitrobenzoyl)-2,3-dihydro-1H-pyrrolo[2,3-c]pyridin-4-yl)benzonitrile (C35)

Following the general method CA, synthesized with **CI33** (100 mg, 0.452 mmol), 2-nitrobenzoyl chloride (161 mg, 0.904 mmol), DMAP (17 mg, 0.12 mmol), Et₃N (89 mg, 0.87 mmol) in anhydrous dichloromethane (4 mL) to give **C35** (79.1 mg, 47.3% yield) as a white solid after chromatographic purification (PE/EA = 10/1 to 1/3). mp: 231.0-231.9 °C. ¹H-NMR (400 MHz, DMSO-*d*₆) δ 9.33 (s, 1H), 8.46 (s, 1H), 8.30 (dd, *J =* 8.5, 0.9 Hz, 1H), 8.01-7.95 (m, 3H), 7.84-7.79 (m, 4H), 3.84 (t, *J* = 8.3 Hz, 2H), 3.30 (t, *J* = 8.4 Hz, 2H). ¹³C-NMR (101 MHz, DMSO-*d*₆) δ 164.91, 144.59, 144.51, 140.81, 139.92, 139.84, 136.35, 135.46, 132.77 (2C), 132.28, 132.03, 131.07, 129.36 (2C), 128.17, 124.93, 118.64, 110.94, 49.05, 27.32. HRMS (ESI): m/z calcd. for C₂₁H₁₅N₄O₃⁺ [M+H]⁺: 371.1144, found: 371.1141.

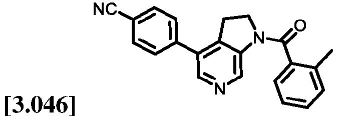

### 4-(1-(2-Methylbenzoyl)-2,3-dihydro-1H-pyrrolo[2,3-c]pyridin-4-yl)benzonitrile (C36)

Following the general method CA, synthesized with **CI33** (100 mg, 0.452 mmol), 2-methylbenzoyl chloride (134.4 mg, 0.904 mmol), DMAP (17 mg, 0.12 mmol), Et₃N (89 mg, 0.87 mmol) in anhydrous dichloromethane (4 mL) to give **C36** (75.2 mg, 46.5% yield) as a white solid after chromatographic purification (PE/EA = 10/1 to 1/3). mp: 116.0-116.8 °C. ¹H-NMR (400 MHz, DMSO-*d*₆) δ 9.39 (s, 1H), 8.42 (s, 1H), 7.98 (d, *J* = 8.3 Hz, 2H), 7.78 (d, *J* = 8.3 Hz, 2H), 7.35 (dd, *J* = 23.7, 7.2 Hz, 4H), 3.82-3.67 (m, 2H), 3.24 (t, *J* = 8.1 Hz, 2H), 2.30 (s, 3H). ¹³C-NMR (101 MHz, DMSO-*d*₆) δ 169.06, 144.57, 141.40, 140.55, 140.46, 137.17, 136.97, 134.03, 133.24 (2C), 130.90, 129.87, 129.77 (2C), 126.51, 125.97, 119.11, 111.35, 100.07, 49.89, 27.76, 19.24. HRMS (ESI): m/z calcd. for C₂₂H₁₈N₃O₂⁺ [M+H]⁺: 340.1450, found: 340.1429.

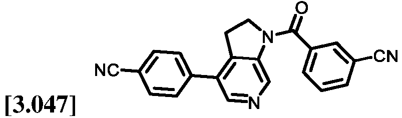

### 3-(4-(4-Cyanophenyl)-2,3-dihydro-1H-pyrrolo[2,3-c]pyridine-1-carbonyl)benzonitrile (C37)

Following the general method CA, synthesized with **CI33** (100 mg, 0.452 mmol), 3-cynobenzoyl chloride (144.4 mg, 0.904 mmol), DMAP (17 mg, 0.12 mmol), Et₃N (89 mg, 0.87 mmol) in anhydrous dichloromethane (4 mL) to give **C37** (97.2 mg, 61.5% yield) as a white solid after chromatographic purification (PE/EA = 10/1 to 1/3). mp: 193.0-193.8 °C. ¹H-NMR (400 MHz, DMSO-*d*₆) δ 9.32 (s, 1H), 8.45 (s, 1H), 8.16 (s, 1H), 8.10-7.92 (m, 4H), 7.86-7.70 (m, 3H), 4.06 (t, *J =* 8.2 Hz, 2H), 3.27 (t, *J* = 8.2 Hz, 2H). ¹³C-NMR (101 MHz, DMSO-*d*₆) δ 166.31, 144.33, 140.89, 140.84, 140.07, 137.38, 137.07, 134.15, 132.82 (2C), 131.86, 130.70, 130.05, 129.34 (2C), 118.67, 118.23, 111.78, 110.94, 99.72, 50.13, 27.67. HRMS (ESI): m/z calcd. for C₂₂H₁₅N₄O⁺ [M+H]⁺: 351.1246, found: 351.1230.

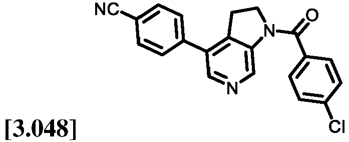

### 4-(1-(4-Chlorobenzoyl)-2,3-dihydro-1H-pyrrolo[2,3-c]pyridin-4-yl)benzonitrile (C38)

Following the general method CA, synthesized with **CI33** (100 mg, 0.452 mmol), 4-chlorobenzoyl chloride (151.2 mg, 0.904 mmol), DMAP (17 mg, 0.12 mmol), Et₃N (89 mg, 0.87 mmol) in anhydrous dichloromethane (4 mL) to give **C38** (94.2 mg, 58.1% yield) as a white solid after chromatographic purification (PE/EA = 10/1 to 1/3). mp: 183.0-183.9 °C. ¹H-NMR (400 MHz, DMSO-*d*₆) δ 9.23 (s, 1H), 8.41 (s, 1H), 7.99 (d, *J =* 8.3 Hz, 2H), 7.78 (d, *J =* 8.3 Hz, 2H), 7.68 (d, *J* = 8.4 Hz, 2H), 7.60 (d, *J =* 8.4 Hz, 2H), 4.06 (t, *J =* 8.2 Hz, 2H), 3.26 (t, *J =* 8.2 Hz, 2H). ¹³C-NMR (101 MHz, DMSO-*d*₆) δ 144.01, 140.8, 140.32, 140.01, 135.26, 134.95, 132.77 (2C), 132.00, 131.52, 131.43, 129.28 (2C), 129.13 (2C), 128.69 (2C), 118.64, 110.88, 50.18, 27.60. HRMS (ESI): m/z calcd. for C₂₁H₁₅ClN₃O⁺ [M+H]⁺: 360.0904, found: 360.0892.

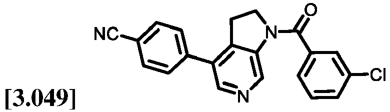

### 4-(1-(3-Chlorobenzoyl)-2,3-dihydro-1H-pyrrolo[2,3-c]pyridin-4-yl)benzonitrile (C39)

Following the general method CA, synthesized with **CI33** (100 mg, 0.452 mmol), 3-chlorobenzoyl chloride (151.2 mg, 0.904 mmol), DMAP (17 mg, 0.12 mmol), Et₃N (89 mg, 0.87 mmol) in anhydrous dichloromethane (4 mL) to give **C39** (89.2 mg, 52.8% yield) as a white solid after chromatographic purification (PE/EA = 10/1 to 1/3). mp: 207.0-207.9 °C. ¹H-NMR (400 MHz, DMSO-*d*₆) δ 9.27 (s, 1H), 8.41 (s, 1H), 7.99 (d, *J =* 7.8 Hz, 2H), 7.78 (d, *J =* 7.7 Hz, 2H), 7.59 (dd, *J* = 18.3, 7.0 Hz, 4H), 4.05 (t, *J* = 7.5 Hz, 2H), 3.26 (t, *J* = 7.9 Hz, 2H). ¹³C-NMR (101 MHz, DMSO-*d*₆) δ 166.59, 140.83, 138.22, 133.31, 132.78 (2C), 132.02, 131.53, 131.43, 130.66, 130.41, 129.29 (2C), 128.81, 128.70, 126.84, 125.68, 118.86, 111.01, 50.12, 27.73. HRMS (ESI): m/z calcd. for C₂₁H₁₅ClN₃O⁺ [M+H]⁺: 360.0904, found: 360.0896.

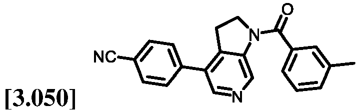

### 4-(1-(3-Methylbenzoyl)-2,3-dihydro-1H-pyrrolo[2,3-c]pyridin-4-yl)benzonitrile (C40)

Following the general method CA, synthesized with **CI33** (100 mg, 0.452 mmol), 3-methylbenzoyl chloride (144.7 mg, 0.904 mmol), DMAP (17 mg, 0.12 mmol), Et₃N (89 mg, 0.87 mmol) in anhydrous dichloromethane (4 mL) to give **C40** (89.5 mg, 54.8% yield) as a white solid after chromatographic purification (PE/EA = 10/1 to 1/3). mp: 186.0-186.9 °C. ¹H-NMR (400 MHz, DMSO-*d*₆) δ 9.24 (s, 1H), 8.39 (s, 1H), 7.99 (d, *J =* 8.3 Hz, 2H), 7.78 (d, *J =* 8.4 Hz, 2H), 7.46-7.34 (m, 4H), 4.05 (t, *J =* 8.3 Hz, 2H), 3.25 (t, *J =* 8.3 Hz, 2H), 2.37 (d, *J =* 6.6 Hz, 3H). ¹³C-NMR (101 MHz, DMSO-*d*₆) δ 168.93, 144.30, 141.39, 140.88, 140.43, 138.52, 136.71, 134.72, 133.24 (2C), 132.47, 131.61, 129.75 (2C), 128.94, 127.88, 124.50, 119.12, 111.33, 50.65, 27.99, 21.39. HRMS (ESI): m/z calcd. for C₂₁H₁₈N₃O⁺ [M+H]⁺: 340.1450, found: 360.0896.

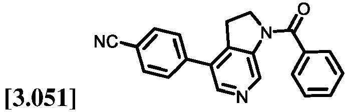

### 4-(1-Benzoyl-2,3-dihydro-1H-pyrrolo[2,3-c]pyridin-4-yl)benzonitrile (C41)

Following the general method CA, synthesized with **CI33** (100 mg, 0.452 mmol), benzoyl chloride (121.4 mg, 0.904 mmol), DMAP (17 mg, 0.12 mmol), Et₃N (89 mg, 0.87 mmol) in anhydrous dichloromethane (4 mL) to give **C41** (75.5 mg, 51.8% yield) as a white solid after chromatographic purification (PE/EA = 10/1 to 1/3). mp: 196.0-196.9 °C. ¹H-NMR (400 MHz, DMSO-*d*₆) δ 9.22 (s, 1H), 8.36 (s, 1H), 7.95 (d, *J =* 8.2 Hz, 2H), 7.75 (d, *J =* 8.3 Hz, 2H), 7.61 (d, *J* = 7.6 Hz, 2H), 7.55-7.46 (m, 3H), 4.02 (t, *J =* 8.2 Hz, 2H), 3.22 (t, *J =* 8.2 Hz, 2H). ¹³C-NMR (101 MHz, DMSO-*d*₆) δ 168.33, 143.85, 140.90, 140.40, 139.96, 136.93, 136.22, 132.76 (2C), 131.98, 130.55, 129.27 (2C), 128.59 (2C), 127.00 (2C), 118.64, 110.86, 50.20, 27.57. HRMS (ESI): m/z calcd. for C₂₁H₁₆N₃O⁺ [M+H]⁺: 326.1293, found: 326.1284.

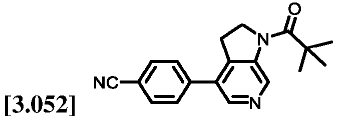

### 4-(1-Pivaloyl-2,3-dihydro-1H-pyrrolo[2,3-c]pyridin-4-yl)benzonitrile (C42)

Following the general method CA, synthesized with **CI33** (100 mg, 0.452 mmol), pivaloyl chloride (109 mg, 0.904 mmol), DMAP (17 mg, 0.12 mmol), Et₃N (89 mg, 0.87 mmol) in anhydrous dichloromethane (4 mL) to give **C42** (102.1 mg, 74.5% yield) as a white solid after chromatographic purification (PE/EA = 10/1 to 1/3). mp: 194.0-194.9 °C. ¹H-NMR (400 MHz, DMSO-*d*₆) δ 9.30 (s, 1H), 8.33 (s, 1H), 7.98 (d, *J* = 8.3 Hz, 2H), 7.77 (d, *J =* 8.3 Hz, 2H), 4.29 (t, *J* = 8.2 Hz, 2H), 3.28 (t, *J =* 8.2 Hz, 2H), 1.31 (s, 9H). ¹³C-NMR (101 MHz, DMSO-*d*₆) δ 176.08, 143.42, 143.37, 142.19, 141.15, 138.68, 137.63, 132.72 (2C), 131.60, 129.30 (2C), 118.66, 110.75, 48.94, 28.40, 27.21 (3C). HRMS (ESI): m/z calcd. for C₁₉H₁₀N₃O⁺ [M+H]⁺: 306.1606, found: 306.1597.

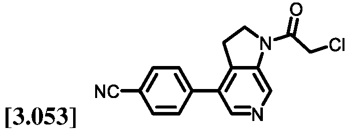

### 4-(1-(2-Chloroacetyl)-2,3-dihydro-1H-pyrrolo[2,3-c]pyridin-4-yl)benzonitrile (C43)

Following the general method CA, synthesized with **CI33** (100 mg, 0.452 mmol), 2-chloroacetyl chloride (102 mg, 0.904 mmol), DMAP (17 mg, 0.12 mmol), Et₃N (89 mg, 0.87 mmol) in anhydrous dichloromethane (4 mL) to give **C43** (100.1 mg, 76.8% yield) as a white solid after chromatographic purification (PE/EA = 10/1 to 1/3). mp: 284.0-284.9 °C. ¹H-NMR (400 MHz, DMSO-*d*₆) δ ¹H-NMR (400 MHz, DMSO): ¹H-NMR (400 MHz, DMSO-*d*₆) δ 9.22 (s, 1H), 8.36 (s, 1H), 7.96 (d, *J =* 7.4 Hz, 2H), 7.78 (d, *J =* 7.4 Hz, 2H), 4.59 (s, 2H), 4.16 (t, *J =* 8.2 Hz, 2H), 3.34 (d, *J* = 8.2 Hz, 2H). ¹³C-NMR (101 MHz, DMSO-*d*₆) δ 164.68, 144.05, 140.87, 140.24, 139.22, 136.15, 132.74 (2C), 132.00, 129.35 (2C), 118.65, 110.88, 46.90, 43.63, 27.35. HRMS (ESI): m/z calcd. for C₁₆H₁₃ClN₃O⁺ [M+H]⁺: 298.0747, found: 298.0730.

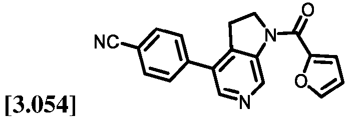

### 4-(1-(Furan-2-carbonyl)-2,3-dihydro-1H-pyrrolo[2,3-c]pyridin-4-yl)benzonitrile (C44)

Following the general method CA, synthesized with **CI33** (100 mg, 0.452 mmol), furan-2-carbonyl chloride (118 mg, 0.904 mmol), DMAP (17 mg, 0.12 mmol), Et₃N (89 mg, 0.87 mmol) in anhydrous dichloromethane (4 mL) to give **C44** (93.1 mg, 65.2% yield) as a white solid after chromatographic purification (PE/EA = 10/1 to 1/3). mp: 244.0-245.0 °C. ¹H-NMR (400 MHz, DMSO-*d*₆) δ 9.30 (s, 1H), 8.39 (s, 1H), 8.00 (dd, *J =* 4.9, 3.5 Hz, 3H), 7.81 (d, *J =* 8.4 Hz, 2H), 7.35 (d, *J =* 3.2 Hz, 1H), 6.75 (dd, *J* = 3.5, 1.7 Hz, 1H), 4.48 (t, *J =* 8.4 Hz, 2H), 3.38 (t, *J =* 8.4 Hz, 2H). ¹³C-NMR (101 MHz, DMSO-*d*₆) δ 156.82, 146.89, 146.31, 143.95, 140.96, 140.91, 139.54, 137.10, 132.76 (2C), 131.93, 129.33 (2C), 118.66, 117.70, 112.09, 110.86, 48.89, 27.90. HRMS (ESI): m/z calcd. for C₁₉H₁₄N₃O₂⁺ [M+H]⁺: 316.1086, found: 316.1074.

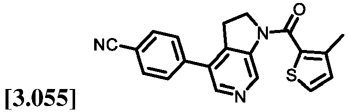

### 4-(1-(3-Methylthiophene-2-carbonyl)-2,3-dihydro-1H-pyrrolo[2,3-c]pyridin-4-yl)benzonitril e (C45)

Following the general method CA, synthesized with **CI33** (100 mg, 0.452 mmol), 3-methylthiophene-2-carbonyl chloride (145 mg, 0.904 mmol), DMAP (17 mg, 0.12 mmol), Et₃N (89 mg, 0.87 mmol) in anhydrous dichloromethane (4 mL) to give **C45** (70.1 mg, 44.9% yield) as a white solid after chromatographic purification (PE/EA = 10/1 to 1/3). mp: 204.0-205.0 °C. ¹H-NMR (400 MHz, DMSO-*d*₆) δ 8.89 (s, 1H), 8.39 (s, 1H), 7.99 (d, *J* = 8.3 Hz, 2H), 7.79 (d, *J* = 8.3 Hz, 2H), 7.72 (d, *J =* 5.0 Hz, 1H), 7.04 (d, *J =* 5.0 Hz, 1H), 4.11 (t, *J =* 8.3 Hz, 2H), 3.29 (t, *J =* 8.4 Hz, 2H), 2.30 (s, 3H). ¹³C-NMR (101 MHz, DMSO-*d*₆) δ 162.68, 143.94, 140.87, 140.17, 140.13, 139.48, 136.57, 132.76 (2C), 132.07, 132.00, 130.41, 129.30 (2C), 127.70, 118.64, 110.88, 49.83, 27.42, 14.77. HRMS (ESI): m/z calcd. for C₂₀H₁₆N₃OS⁺ [M+H]⁺: 346.1014, found: 346.1000.

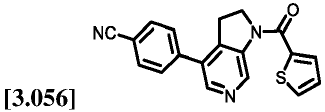

### 4-(1-(Thiophene-2-carbonyl)-2,3-dihydro-1H-pyrrolo[2,3-c]pyridin-4-yl)benzonitrile (C46)

Following the general method CA, synthesized with **CI33** (100 mg, 0.452 mmol), thiophene-2-carbonyl chloride (126 mg, 0.904 mmol), DMAP (17 mg, 0.12 mmol), Et₃N (89 mg, 0.87 mmol) in anhydrous dichloromethane (4 mL) to give **C46** (75.1 mg, 50.2% yield) as a white solid after chromatographic purification (PE/EA = 10/1 to 1/3). mp: 213.0-214.0 °C. ¹H-NMR (400 MHz, DMSO-*d*₆) δ 9.26 (s, 1H), 8.40 (s, 1H), 7.97 (dd, *J* = 24.5, 6.2 Hz, 3H), 7.80 (d, *J* = 8.2 Hz, 3H), 7.25 (d, *J =* 3.9 Hz, 1H), 4.48 (t, *J* = 8.0 Hz, 2H), 3.37 (t, *J =* 8.2 Hz, 2H). ¹³C-NMR (101 MHz, DMSO-*d*₆) δ 160.88, 143.97, 140.93, 140.88, 139.66, 138.59, 137.30, 132.77 (2C), 132.11, 130.85, 129.32 (2C), 128.10, 118.66, 118.61, 110.86, 49.95, 27.99. HRMS (ESI): m/z calcd. for C₁₉H₁₄N₃OS⁺ [M+H]⁺: 332.0858, found: 332.0853.

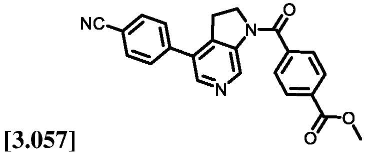

### Methyl 4-(4-(4-cyanophenyl)-2,3-dihydro-1H-pyrrolo[2,3-c]pyridine-1-carbonyl)benzoate (C47)

Following the general method CA, synthesized with **CI33** (100 mg, 0.452 mmol), methyl 4-(chlorocarbonyl)benzoate (91.6 mg, 0.904 mmol), DMAP (17 mg, 0.12 mmol), Et₃N (89 mg, 0.87 mmol) in anhydrous dichloromethane (4 mL) to give **C47** (85.1 mg, 57.2% yield) as a white solid after chromatographic purification (PE/EA = 10/1 to 1/3). mp: 210.0-211.0 °C. ¹H-NMR (400 MHz, DMSO-*d*₆) δ 9.32 (s, 1H), 8.42 (s, 1H), 8.09 (d, *J =* 8.2 Hz, 2H), 7.99 (d, *J =* 8.2 Hz, 2H), 7.79 (d, *J* = 8.2 Hz, 4H), 4.04 (t, *J =* 7.9 Hz, 2H), 3.90 (s, 3H), 3.27 (t, *J =* 8.2 Hz, 2H). ¹³C-NMR (101 MHz, DMSO-*d*₆) δ 167.38, 165.62, 144.21, 140.83, 140.37, 140.20, 140.06, 132.78 (2C), 132.04, 129.43 (2C), 131.08, 129.29 (2C),127.48 (2C), 118.63, 110.89, 52.45, 50.05, 27.57. HRMS (ESI): m/z calcd. for C₂₂H₁₅N₃O₃⁺ [M+H]⁺: 384.1348, found: 384.1332.

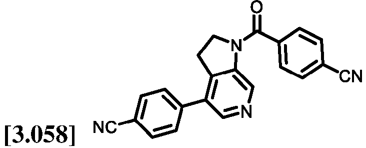

### 4-(1-(4-Cyanobenzoyl)-2,3-dihydro-1H-pyrrolo[2,3-c]pyridin-4-yl)benzonitrile (C48)

Following the general method CA, synthesized with **CI33** (100 mg, 0.452 mmol), methyl 4-cyanobenzoyl chloride (144 mg, 0.904 mmol), DMAP (17 mg, 0.12 mmol), Et₃N (89 mg, 0.87 mmol) in anhydrous dichloromethane (4 mL) to give **C48** (79.1 mg, 50.2% yield) as a white solid after chromatographic purification (PE/EA = 10/1 to 1/3). mp: 185.0-186.0 °C. ¹H-NMR (400 MHz, DMSO-*d*₆) δ 9.31 (s, 1H), 8.43 (s, 1H), 8.00 (dd, *J =* 11.1, 8.3 Hz, 4H), 7.81 (dd, *J* = 20.5, 8.2 Hz, 4H), 4.01 (t, *J =* 7.6 Hz, 2H), 3.26 (t, *J* = 8.2 Hz, 2H). ¹³C-NMR (101 MHz, DMSO-*d*₆) δ 166.98, 140.78, 140.47, 132.78 (2C), 132.73 (2C), 132.05, 132.02, 131.53, 131.43, 129.29 (2C), 128.81, 127.91 (2C), 118.67, 112.97, 110.91, 50.10, 27.65. HRMS (ESI): m/z calcd. for C₂₂H₁₅N₄O⁺ [M+H]⁺: 351.1246, found: 351.1233.

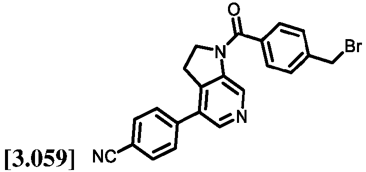

### 4-(1-(4-(Bromomethyl)benzoyl)-2,3-dihydro-1H-pyrrolo[2,3-c]pyridin-4-yl)benzonitrile (C49)

Following the general method CA, synthesized with **CI33** (100 mg, 0.452 mmol), methyl 4-(bromomethyl)benzoyl chloride (158 mg, 0.904 mmol), DMAP (17 mg, 0.12 mmol), Et₃N (89 mg, 0.87 mmol) in anhydrous dichloromethane (4 mL) to give **C49** (85.1 mg, 44.9% yield) as a white solid after chromatographic purification (PE/EA = 10/1 to 1/3). mp: 223.0-224.0 °C. ¹H-NMR (400 MHz, DMSO-*d*₆) δ 9.16 (s, 1H), 8.32 (s, 1H), 7.91 (d, *J* = 8.3 Hz, 2H), 7.71 (d, *J* = 8.3 Hz, 2H), 7.58 (d, *J* = 8.1 Hz, 2H), 7.51 (d, *J =* 8.1 Hz, 2H), 4.77 (s, 2H), 3.99 (t, *J =* 8.2 Hz, 2H), 3.18 (t, *J =* 8.2 Hz, 2H). ¹³C-NMR (101 MHz, DMSO-*d*₆) δ 168.20, 144.04, 140.91, 140.40, 140.07, 136.06, 133.14, 132.81 (2C), 132.06, 129.76, 129.33 (2C), 129.03 (2C), 127.50 (2C), 118.57, 111.00, 50.33, 45.55, 27.48. HRMS (ESI): m/z calcd. for C₂₂H₁₇BrN₃O⁺ [M+H]⁺: 418.0544, found: 418.0555.

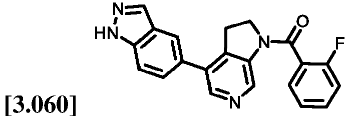

### (4-(1H-Indazol-5-yl)-2,3-dihydro-1H-pyrrolo[2,3-c]pyridin-1-yl)(2-fluorophenyl)methanone (C50)

Following the general method CB, synthesized with **CI37** (50.0 mg, 0.156 mmol), (1H-indazol-5-yl)boronic acid (37 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C50** (41 mg, 72.8% yield) as a white solid after chromatographic purification (PE/EA = 10/1 to 1/3). mp: 99.5-100.5 °C. ¹H-NMR (400 MHz, DMSO-*d*₆) δ 13.22 (s, 1H), 9.30 (s, 1H), 8.44 (s, 1H), 8.15 (s, 1H), 7.95 (s, 1H), 7.67 (d, *J =* 8.7 Hz, 1H), 7.64-7.57 (m, 2H), 7.54 (d, *J =* 8.5 Hz, 1H), 7.39 (dd, *J* = 19.2, 8.6 Hz, 2H), 3.91 (t, *J* = 7.6 Hz, 2H), 3.28 (d, *J* = 8.2 Hz, 2H). ¹³C-NMR (101 MHz, DMSO-*d*₆) δ 163.82, 154.78 (¹*J*_{CF} = 344 Hz), 144.90, 139.76 (³*J*_{CF} = 16 Hz), 139.37, 135.46, 134.02 (2C), 132.19 (³*J*_{CF} = 11 Hz), 128.67, 128.18, 126.54 (2C), 125.12 (⁴*J*_{CF} = 3 Hz), 124.64 (²*J*_{CF} = 18 Hz), 123.22, 120.42, 116.17 (²*J*_{CF} = 22 Hz), 110.59, 49.00, 27.56. ¹⁹F NMR (377 MHz, DMSO-*d*₆) δ -116.32. HRMS (ESI): m/z calcd. for C₂₁H₁₆FN₄O⁺: 359.1308 [M+H]⁺, found: 359.1297.

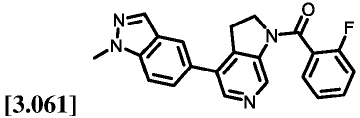

### (2-Fluorophenyl)(4-(1-methyl-1H-indazol-5-yl)-2,3-dihydro-1H-pyrrolo[2,3-c]pyridin-1-yl)m ethanone (C51)

Following the general method CB, synthesized with **CI37** (50.0 mg, 0.156 mmol), (1-methyl-1H-indazol-5-yl)boronic acid (37 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C51** (40 mg, 72.5% yield) as a white solid after chromatographic purification (PE/EA = 10/1 to 1/3). mp: 173.5-174.5 °C. ¹H-NMR (400 MHz, DMSO-*d*₆) δ 9.34 (s, 1H), 8.49 (s, 1H), 8.11 (s, 1H), 7.91-7.81 (m, 2H), 7.62 (dd, *J* = 10.7, 3.7 Hz, 2H), 7.45-7.35 (m, 2H), 7.32 (d, *J* = 8.3 Hz, 1H), 4.09 (s, 3H), 3.92 (t, *J* = 8.0 Hz, 2H), 3.20 (m, 2H). ¹³C-NMR (101 MHz, DMSO-*d*₆) δ 163.88, 159.04 (¹*J*_{CF} = 214 Hz), 145.03, 139.83, 135.92, 133.90, 132.40, 131.28 (³*J*_{CF} = 9 Hz), 131.44 (³*J*_{CF} = 10 Hz), 128.83, 128.70 (⁴*J*_{CF} = 3 Hz), 125.13, 124.69 (²*J*_{CF} = 17 Hz), 122.97, 121.21, 120.91, 116.18 (²*J*_{CF} = 19 Hz), 109.53, 49.01, 35.45, 27.49. ¹⁹F NMR (377 MHz, DMSO-*d*₆) δ -116.32. HRMS (ESI): m/z calcd. for C₂₁H₁₆FN₄O⁺ [M+H]⁺: 359.1308, found: 359.1297.

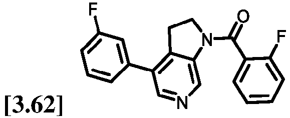

### (2-fluorophenyl)(4-(3-fluorophenyl)-2,3-dihydro-1H-pyrrolo[2,3-c]pyridin-1-yl)methanone (C52)

Following the general method CB, synthesized with **CI37** (50.0 mg, 0.156 mmol), (2-fluorophenyl)boronic acid (28 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C52**(42 mg, 74.5% yield) as a white solid after chromatographic purification (PE/EA = 10/1 to 1/3). mp: 140.5-141.5 °C. ¹H-NMR (400 MHz, DMSO-*d*₆) δ 9.32 (s, 1H), 8.42 (s, 1H), 7.69-7.51 (m, 4H), 7.47-7.40 (m, 3H), 7.29 (td, *J =* 8.5, 2.1 Hz, 1H), 3.90 (t, *J =* 7.9 Hz, 2H), 3.28 (d, *J =* 8.2 Hz, 2H). ¹³C-NMR (101 MHz, DMSO-*d*₆) δ 163.59, 162.35 (¹*J*_{CF} = 238 Hz), 157.61 (¹*J*_{CF} = 244 Hz), 144.55, 139.81 (³*J*_{CF} = 7 Hz), 136.26, 132.49, 132.28 (³*J*_{CF} = 8 Hz), 130.92 (³*J*_{CF} = 8 Hz, 2C), 128.66, 124.94 (²*J*_{CF} = 44 Hz), 124.55 (⁴*J*_{CF} = 3 Hz, 2C), 116.18 (²*J*_{CF} = 22 Hz), 115.16 (²*J*_{CF} = 22 Hz), 115.03 (²*J*_{CF} = 20 Hz), 48.98, 27.36. ¹⁹F NMR (377 MHz, DMSO-*d*₆) δ -112.39, -116.31. HRMS (ESI): m/z calcd. for C₂₀H₁₅F₂N₂O⁺ [M+H]⁺: 337.1067, found: 337.1152.

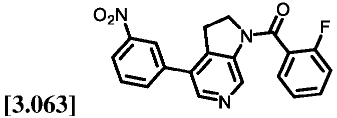

### (2-Fluorophenyl)(4-(3-nitrophenyl)-2,3-dihydro-1H-pyrrolo[2,3-c]pyridin-1-yl)methanone (C53)

Following the general method CB, synthesized with **CI37** (50.0 mg, 0.156 mmol), (3-nitrophenyl)boronic acid (32 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C53** (36 mg, 63.6% yield) as a white solid after chromatographic purification (PE/EA = 10/1 to 1/3). mp: 174.5-175.5 °C. ¹H-NMR (400 MHz, DMSO-*d*₆) δ 9.42 (s, 1H), 8.55 (s, 1H), 8.44 (t, *J* = 1.8 Hz, 1H), 8.36 (dd, *J* = 8.2, 1.4 Hz, 1H), 8.12 (d, *J =* 8.0 Hz, 1H), 7.88 (t, *J =* 8.0 Hz, 1H), 7.67 (t, *J* = 7.0 Hz, 2H), 7.52-7.37 (m, 2H), 3.99 (t, *J* = 8.0 Hz, 2H), 3.35 (d, *J =* 8.3 Hz, 2H). ¹³C-NMR (101 MHz, DMSO-*d*₆) δ 163.92, 157.62 (¹*J*_{CF} = 258 Hz), 148.20, 144.57, 140.23, 139.88, 137.62, 136.67, 134.97, 132.34 (⁴*J*_{CF} = 5 Hz), 130.53 (2C), 128.67, 125.13, 124.58 (²*J*_{CF} = 17 Hz), 122.95 (³*J*_{CF} = 11 Hz, 2C), 116.19 (²*J*_{CF} = 22 Hz), 49.00 (s), 27.29. ¹⁹F NMR (377 MHz, DMSO-*d*₆) δ -116.32. HRMS (ESI): m/z calcd. for C₂₀H₁₅FN₃O⁺ [M+H]⁺: 364.1079, found: 364.1014.

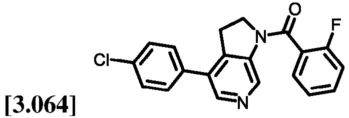

### (4-(4-Chlorophenyl)-2,3-dihydro-1H-pyrrolo[2,3-c]pyridin-1-yl)(2-fluorophenyl)methanone (C54)

Following the general method CB, synthesized with CI37 (50.0 mg, 0.156 mmol), (4-chlorophenyl)boronic acid (33 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C54** (39 mg, 71.0% yield) as a white solid after chromatographic purification (PE/EA = 10/1 to 1/3). mp: 188.5-189.5 °C. ¹H-NMR (400 MHz, DMSO-*d*₆) δ 9.30 (s, 1H), 8.39 (s, 1H), 7.62-7.55 (m, 6H), 7.38 (dd, *J* = 19.2, 8.7 Hz, 2H), 3.90 (t, *J =* 7.8 Hz, 2H), 3.26 (t, *J =* 8.2 Hz, 2H). ¹³C-NMR (101 MHz, DMSO-*d*₆) δ 163.91, 157.66 (¹*J*_{CF} = 244 Hz), 144.48, 139.78 (⁴*J*_{CF} = 4 Hz), 134.88, 133.30, 133.15, 132.56, 132.29 (³*J*_{CF} = 7 Hz), 130.16 (2C), 129.00, 128.91 (2C), 128.69 (³*J*_{CF} = 7 Hz), 125.16, 124.64 (²*J*_{CF} = 18 Hz), 116.14 (²*J*_{CF} = 12 Hz), 48.98, 27.37. ¹⁹F NMR (377 MHz, DMSO-*d*₆) δ -116.30. HRMS (ESI): m/z calcd. for C₂₀H₁₅ClFN₂O⁺ [M+H]⁺: 353.0857, found: 353.0765.

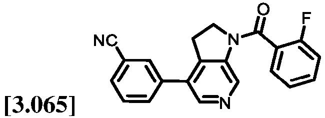

### 3-(1-(2-Fluorobenzoyl)-2,3-dihydro-1H-pyrrolo[2,3-c]pyridin-4-yl)benzonitrile (C55)

Following the general method CB, synthesized with **CI37** (50.0 mg, 0.156 mmol), (2-cynophenyl)boronic acid (33 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C55** (39 mg, 68.4% yield) as a white solid after chromatographic purification (PE/EA = 10/1 to 1/3). mp: 175.5-176.5 °C. ¹H-NMR (400 MHz, DMSO-*d*₆) δ 9.33 (s, 1H), 8.44 (s, 1H), 8.09 (s, 1H), 7.92 (dd, *J* = 7.8, 1.6 Hz, 2H), 7.72 (t, *J* = 7.8 Hz, 1H), 7.61 (t, *J =* 7.1 Hz, 2H), 7.44-7.34 (m, 2H), 3.90 (t, *J* = 8.0 Hz, 2H), 3.28 (d, *J =* 8.2 Hz, 2H). ¹³C-NMR (101 MHz, DMSO-*d*₆) δ 163.93, 158.88 (¹*J*_{CF} = 248 Hz), 144.66, 140.12, 139.82, 139.55, 137.26, 136.50, 133.20, 132.30 (³*J*_{CF} = 6 Hz), 131.90, 130.06, 128.66, 125.13 (³*J*_{CF} = 8 Hz), 124.57 (²*J*_{CF} = 19 Hz), 118.54, 116.19 (²*J*_{CF} = 25 Hz), 112.06, 48.96, 27.20. ¹⁹F NMR (377 MHz, DMSO-*d*₆) δ -116.32 (s). HRMS (ESI): m/z calcd. for C₂₁H₁₅FN₃O⁺ [M+H]⁺: 344.1199, found: 344.1112.

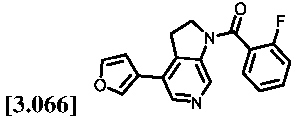

### (2-Fluorophenyl)(4-(furan-3-yl)-2,3-dihydro-1H-pyrrolo[2,3-c]pyridin-1-yl)methanone (C56)

Following the general method CB, synthesized with **CI37** (50.0 mg, 0.156 mmol), furan-3-ylboronic acid (32 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C56** (34 mg, 71.2% yield) as a white solid after chromatographic purification (PE/EA = 10/1 to 1/3). mp: 176.5-177.5 °C. ¹H-NMR (400 MHz, DMSO-*d*₆) δ 9.21 (s, 1H), 8.60 (s, 1H), 8.14 (s, 1H), 7.85 (s, 1H), 7.61 (t, *J =* 7.1 Hz, 2H), 7.39 (dd, *J* = 12.6, 8.3 Hz, 2H), 7.07 (s, 1H), 3.94 (t, *J =* 8.1 Hz, 2H), 3.29 (d, *J =* 8.3 Hz, 2H). ¹³C-NMR (101 MHz, DMSO-*d*₆) δ 163.79, 157.72 (¹*J*_{CF} = 262 Hz), 144.31, 143.36, 141.27, 139.84, 138.29, 135.28, 132.20 (³*J*_{CF} = 7 Hz), 128.64 (⁴*J*_{CF} = 2 Hz), 125.05, 124.71 (²*J*_{CF} = 16 Hz), 121.02, 116.27, 116.15 (²*J*_{CF} = 21 Hz), 109.19, 48.69, 28.09. ¹⁹F NMR (377 MHz, DMSO-*d*₆) δ -116.32. HRMS (ESI): m/z calcd. for C₁₈H₁₄FN₂O₂⁺ [M+H]⁺: 309.1039, found: 309.0954.

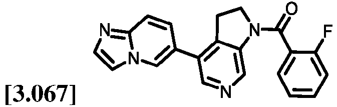

### (2-Fluorophenyl)(4-(imidazo[1,2-a]pyridin-6-yl)-2,3-dihydro-1H-pyrrolo[2,3-c]pyridin-1-yl)m ethanone (C57)

Following the general method CB, synthesized with **CI37** (50.0 mg, 0.156 mmol), imidazo[1,2-a]pyridin-6-ylboronic acid (38 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C57** (30 mg, 54.1% yield) as a white solid after chromatographic purification (PE/EA = 10/1 to 1/3). mp: 215.5-216.5 °C. ¹H-NMR (400 MHz, DMSO-*d*₆) δ 9.33 (s, 1H), 8.83 (s, 1H), 8.47 (s, 1H), 7.99 (s, 1H), 7.68 (d, *J* = 9.3 Hz, 1H), 7.62 (dd, *J* = 11.3, 5.3 Hz, 3H), 7.46 (dd, J = 9.4, 1.8 Hz, 1H), 7.37 (t, *J* = 7.6 Hz, 1H), 3.93 (t, *J* = 8.0 Hz, 2H), 3.31 (s, 2H). ¹³C-NMR (101 MHz, DMSO-*d*₆) δ 163.88, 156.40 (¹*J*_{CF} = 245 Hz), 144.53, 143.67, 140.13, 136.19, 133.90, 132.31 (³*J*_{CF} = 9 Hz), 130.40, 128.67, 126.09, 126.01, 125.11, 125.04, 124.62 (²*J*_{CF} = 18 Hz), 120.76, 116.96, 116.18 (²*J*_{CF} = 22 Hz), 113.75, 48.93, 27.34.¹⁹F NMR (377 MHz, DMSO-*d*₆) δ -116.32. HRMS (ESI): m/z calcd. for C₂₁H₁₅FN₂O₂⁺ [M+H]⁺: 359.1308, found: 359.1316.

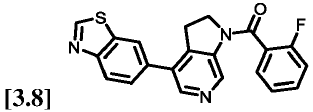

### (4-(benzo[d]thiazol-6-yl)-2,3-dihydro-1H-pyrrolo[2,3-c]pyridin-1-yl)(2-fluorophenyl)methano ne (C58)

Following the general method CB, synthesized with **CI37** (50.0 mg, 0.156 mmol), benzo[d]thiazol-6-ylboronic acid (42 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C58** (39 mg, 66.1% yield) as a white solid after chromatographic purification (PE/EA = 10/1 to 1/3). mp: 173.5-174.5 °C. ¹H-NMR (400 MHz, DMSO-*d*₆) δ 9.47 (s, 1H), 9.33 (s, 1H), 8.45 (d, *J* = 27.2 Hz, 2H), 8.20 (d, *J* = 8.4 Hz, 1H), 7.74 (d, *J* = 8.3 Hz, 1H), 7.56 (d, *J* = 6.3 Hz, 2H), 7.45-7.33 (m, 2H), 3.92 (s, 2H), 2.04 (m, 2H). ¹³C-NMR (101 MHz, DMSO-*d*₆) δ 163.88 (s), 154.98 (¹*J*_{CF} = 258 Hz), 144.84, 139.91, 136.02, 134.35, 133.24 (³*J*_{CF} = 9 Hz), 133.22, 132.06, 131.55, 131.44, 128.82, 128.71, 126.65, 125.11 (⁴*J*_{CF} = 2 Hz), 124.66 (²*J*_{CF} = 19 Hz), 123.21, 122.38, 116.18 (²*J*_{CF} = 21 Hz), 48.98, 27.46. ¹⁹F NMR (377 MHz, DMSO-*d*₆) δ -116.32. HRMS (ESI): m/z calcd. for C₂₁H₁₅FN₃O₂S⁺ [M+H]⁺: 376.0920, found: 376.0914.

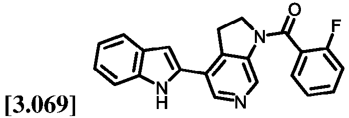

### (4-(1H-indol-2-yl)-2,3-dihydro-1H-pyrrolo[2,3-c]pyridin-1-yl)(2-fluorophenyl)methanone (C59)

Following the general method CB, synthesized with **CI37** (50.0 mg, 0.156 mmol), 1H-indol-2-ylboronic acid (41 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C59** (35 mg, 62.1% yield) as a white solid after chromatographic purification (PE/EA = 10/1 to 1/3). mp: 221.5-222.5 °C. ¹H-NMR (400 MHz, DMSO-*d*₆) δ 11.65 (s, 1H), 9.27 (s, 1H), 8.80 (s, 1H), 7.62 (dd, *J* = 17.5, 7.6 Hz, 3H), 7.49-7.33 (m, 3H), 7.17 (t, *J* = 7.6 Hz, 1H), 7.04 (t, *J* = 7.4 Hz, 1H), 6.80 (s, 1H), 4.03-3.91 (m, 2H), 3.46 (t, *J* = 8.3 Hz, 2H). ¹³C-NMR (101 MHz, DMSO-*d*₆) δ 163.85, 158.83(¹*J*_{CF} = 244 Hz), 142.55, 140.07, 138.37, 136.87, 135.43, 132.72, 132.23 (³*J*_{CF} = 7 Hz), 128.68, 128.31, 125.10 (⁴*J*_{CF} = 3 Hz), 124.72 (²*J*_{CF} = 17 Hz), 122.43, 120.38, 119.62, 116.16 (²*J*_{CF} = 22 Hz), 111.48, 102.87, 48.89, 28.43. ¹⁹F NMR (377 MHz, DMSO-*d*₆) δ -116.32. HRMS (ESI): m/z calcd. for C₂₁H₁₇FN₃O⁺ [M+H]⁺: 358.1356, found: 358.1270.

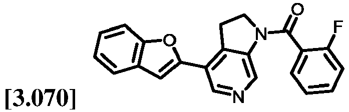

### (4-(Benzofuran-2-yl)-2,3-dihydro-1H-pyrrolo[2,3-c]pyridin-1-yl)(2-fluorophenyl)methanone (C60)

Following the general method CB, synthesized with **CI37** (50.0 mg, 0.156 mmol), benzofuran-2-ylboronic acid (38 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C60** (36 mg, 64.8% yield) as a white solid after chromatographic purification (PE/EA = 10/1 to 1/3). mp: 189.5-190.5 °C. ¹H-NMR (400 MHz, DMSO-*d*₆) δ δ 9.31 (s, 1H), 8.91 (s, 1H), 7.73 (d, *J* = 7.6 Hz, 1H), 7.67 (d, *J* = 8.2 Hz, 1H), 7.63 (t, *J* = 7.1 Hz, 2H), 7.46 (s, 1H), 7.43-7.34 (m, 3H), 7.31 (t, *J* = 7.4 Hz, 1H), 4.01 (t, *J* = 8.3 Hz, 2H), 3.53 (t, *J* = 8.4 Hz, 2H). ¹³C-NMR (101 MHz, DMSO-*d*₆) δ 163.96, 157.65 (¹*J*_{CF} = 240 Hz), 154.15, 151.46, 141.95, 140.33, 138.32, 136.29, 132.38 (³*J*_{CF} = 8 Hz), 128.66, 128.25, 125.41, 125.08, 124.63 (²*J*_{CF} = 19 Hz), 123.53, 121.57, 116.21 (²*J*_{CF} = 23 Hz), 111.30, 105.94, 99.45, 48.86, 28.55. ¹⁹F NMR (377 MHz, DMSO-*d*₆) δ -116.32. HRMS (ESI): m/z calcd. for C₂₂H₁₆FN₂O₂⁺ [M+H]⁺: 359.1196, found: 359.1281.

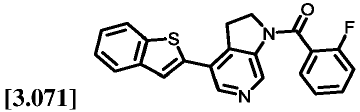

### (4-(Benzo[b]thiophen-2-yl)-2,3-dihydro-1H-pyrrolo[2,3-c]pyridin-1-yl)(2-fluorophenyl)methan one (C61)

Following the general method CB, synthesized with **CI37** (50.0 mg, 0.156 mmol), benzo[b]thiophen-2-yl boronic acid (38 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C61** (31 mg, 62.1% yield) as a white solid after chromatographic purification (PE/EA = 10/1 to 1/3). mp: 168.2-169.2 °C. ¹H-NMR (400 MHz, DMSO) δ 9.32 (s, 1H), 8.71 (s, 1H), 8.10-8.01 (m, 1H), 7.92 (dd, *J* = 16.2, 9.5 Hz, 2H), 7.63 (t, *J* = 7.0 Hz, 2H), 7.48-7.33 (m, 4H), 3.99 (t, *J* = 7.8 Hz, 2H), 3.49 (t, *J* = 8.3 Hz, 2H). ¹³C-NMR (101 MHz, DMSO) δ 163.97, 156.41(¹*J*_{CF} = 272 Hz), 143.63, 139.80, 139.01, 137.58, 136.33, 135.09, 132.29 (³*J*_{CF} = 7 Hz), 131.68, 128.68, 128.28, 127.02, 125.22, 125.09 (⁴*J*_{CF} = 4 Hz), 125.00, 124.15, 123.73, 122.40, 116.19 (²*J*_{CF} = 20 Hz), 48.88, 28.43. ¹⁹F NMR (377 MHz, DMSO-*d*₆) δ -116.32. HRMS (ESI): m/z calcd. for C₂₂H₁₆FN₂OS⁺ [M+H]⁺: 375.0967, found: 375.0959.

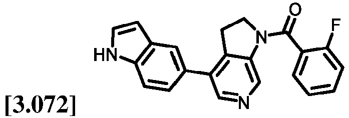

### (4-(1H-indol-5-yl)-2,3-dihydro-1H-pyrrolo[2,3-c]pyridin-1-yl)(2-fluorophenyl)methanone (C62)

Following the general method CB, synthesized with **CI37** (50.0 mg, 0.156 mmol), 1*H*-indol-5-yl boronic acid (38 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C62** (35 mg, 62.3% yield) as a white solid after chromatographic purification (PE/EA = 10/1 to 1/3). mp: 247.2-248.2 °C. ¹H-NMR (400 MHz, DMSO-*d*₆) δ 11.25 (s, 1H), 9.26 (s, 1H), 8.41 (s, 1H), 7.71 (s, 1H), 7.67-7.55 (m, 2H), 7.51 (d, *J* = 8.4 Hz, 1H), 7.41 (d, *J* = 8.4 Hz, 2H), 7.26 (d, *J* = 7.4 Hz, 1H), 6.50 (s, 1H), 3.90 (t, *J* = 7.7 Hz, 2H), 3.28 (d, *J* = 8.3 Hz, 2H). ¹³C-NMR (101 MHz, DMSO-*d*₆) δ 163.75, 157.34 (¹*J*_{CF} = 296 Hz), 144.91, 139.64, 139.24, 135.55, 135.28, 134.99, 132.16 (³*J*_{CF} = 8 Hz), 128.63, 128.00, 126.69, 126.37, 125.06, 124.79 (²*J*_{CF} = 19 Hz), 121.39, 119.95, 116.15 (²*J*_{CF} = 21 Hz), 111.77, 101.50, 49.12, 27.58. ¹⁹F NMR (377 MHz, DMSO-*d*₆) δ -116.32. HRMS (ESI): m/z calcd. for C₂₂H₁₇FN₃O⁺ [M+H]⁺: 358.1356, found: 358.1343.

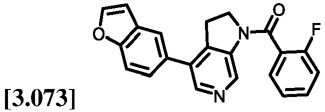

### (4-(Benzofuran-5-yl)-2,3-dihydro-1H-pyrrolo[2,3-c]pyridin-1-yl)(2-fluorophenyl)methanone (C63)

Following the general method CB, synthesized with **CI37** (50.0 mg, 0.156 mmol), benzofuran-5-yl boronic acid (39 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C63** (36 mg, 64.1% yield) as a white solid after chromatographic purification (PE/EA = 10/1 to 1/3). mp: 94.2-95.2 °C. ¹H-NMR (400 MHz, DMSO-*d*₆) δ 9.30 (s, 1H), 8.42 (s, 1H), 8.08 (d, *J* = 1.8 Hz, 1H), 7.84 (s, 1H), 7.73 (d, *J* = 8.5 Hz, 1H), 7.66-7.56 (m, 2H), 7.49 (d, *J* = 8.4 Hz, 1H), 7.44-7.32 (m, 2H), 7.03 (s, 1H), 3.90 (t, *J* = 7.7 Hz, 2H), 3.28 (t, *J* = 8.2 Hz, 2H). ¹³C-NMR (101 MHz, DMSO-*d*₆) δ 163.82, 157.68 (¹*J*_{CF} = 258 Hz), 154.03, 146.96, 144.87, 139.68, 135.63, 134.04, 132.30 (³*J*_{CF} = 8 Hz), 131.04, 128.65, 127.78, 125.10, 125.08, 124.77, 124.62, 121.18, 116.17 (²*J*_{CF} = 21 Hz), 111.64, 106.92, 48.97, 27.50. ¹⁹F NMR (377 MHz, DMSO-*d*₆) δ -116.32. HRMS (ESI): m/z calcd. for C₂₂H₁₆FN₂O₂⁺ [M+H]⁺: 359.1196, found: 359.1184.

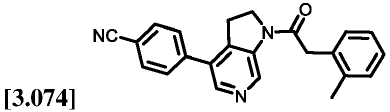

### 4-(1-(2-(o-Tolyl)acetyl)-2,3-dihydro-1H-pyrrolo[2,3-c]pyridin-4-yl)benzonitrile (C64)

Following the general method CD, synthesized with **CI33** (50.0 mg, 0.227 mmol), 2-(o-tolyl)acetic acid (41 mg, 0.272 mmol), HATU (124 mg, 0.341 mmol) and DIPEA (38 mg, 0.681 mmol) in anhydrous dichloromethane (5 mL) to give **C64** (52 mg, 63.5% yield) as a white solid after chromatographic purification (PE/EA = 10/1 to 1/3). mp: 150.2-151.2 °C. ¹H-NMR (400 MHz, DMSO-*d*₆) δ 9.25 (s, 1H), 8.35 (s, 1H), 7.99 (d, *J* = 8.3 Hz, 2H), 7.81 (d, *J* = 8.3 Hz, 2H), 7.22-7.13 (m, 4H), 4.27 (t, *J* = 8.4 Hz, 2H), 3.91 (s, 2H), 3.38 (m, 2H), 2.24 (s, 3H). ¹³C-NMR (101 MHz, DMSO-*d*₆) δ 169.36, 143.44, 141.05, 140.67, 139.16, 137.05, 136.08, 133.80, 132.74 (2C), 131.95, 130.11, 129.85, 129.33 (2C), 126.83, 125.73, 118.68, 110.82, 54.92, 47.48, 27.28, 19.25. HRMS (ESI): m/z calcd. for C₂₃H₂₀N₃O⁺ [M+H]⁺: 353.1606, found: 354.1517.

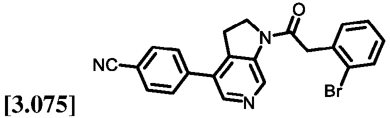

### 4-(1-(2-(2-Bromophenyl)acetyl)-2,3-dihydro-1H-pyrrolo[2,3-c]pyridin-4-yl)benzonitrile (C65)

Following the general method CD, synthesized with **CI33** (50.0 mg, 0.227 mmol), 2-(2-bromophenyl)acetic acid (58.5 mg, 0.272 mmol), HATU (124 mg, 0.341 mmol) and DIPEA (38 mg, 0.681 mmol) in anhydrous dichloromethane (5 mL) to give **C65** (50 mg, 52.6% yield) as a white solid after chromatographic purification (PE/EA = 10/1 to 1/3). mp: 214.2-215.2 °C. ¹H-NMR (400 MHz, DMSO-*d*₆) δ 9.22 (s, 1H), 8.36 (s, 1H), 8.00 (d, *J =* 8.2 Hz, 2H), 7.82 (d, *J =* 8.2 Hz, 2H), 7.64 (d, *J* = 7.9 Hz, 1H), 7.40 (dt, *J* = 14.8, 7.4 Hz, 2H), 7.31-7.20 (m, 1H), 4.31 (t, *J = 8.3* Hz, 2H), 4.04 (s, 2H), 3.39 (t, *J* = 8.4 Hz, 2H). ¹³C-NMR (101 MHz, DMSO-*d*₆) δ 168.22, 143.59, 141.03, 140.52, 139.15, 136.08, 135.27, 132.75 (2C), 132.38, 132.24, 131.96, 129.34 (2C), 128.97, 127.67, 124.88, 118.68, 110.84, 47.55, 42.27, 27.28. HRMS (ESI): m/z calcd. for C₂₂H₁₇BrN₃O⁺ [M+H]⁺: 418.0555, found: 418.0556.

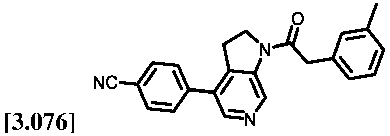

### 4-(1-(2-(m-Tolyl)acetyl)-2,3-dihydro-1H-pyrrolo[2,3-c]pyridin-4-yl)benzonitrile (C66)

Following the general method CD, synthesized with **CI33** (50.0 mg, 0.227 mmol), 2-(*m*-tolyl)acetic acid (58.5 mg, 0.272 mmol), HATU (124 mg, 0.341 mmol) and DIPEA (38 mg, 0.681 mmol) in anhydrous dichloromethane (5 mL) to give **C66** (49 mg, 61.2% yield) as a white solid after chromatographic purification (PE/EA = 10/1 to 1/3). mp: 212.2-213.2 °C. ¹H-NMR (400 MHz, DMSO-*d*₆) δ 9.26 (s, 1H), 8.33 (s, 1H), 7.97 (d, *J* = 8.4 Hz, 2H), 7.78 (d, *J* = 8.4 Hz, 2H), 7.22 (t, *J* = 7.5 Hz, 1H), 7.09 (dd, *J* = 13.9, 6.9 Hz, 3H), 4.20 (t, *J =* 8.4 Hz, 2H), 3.85 (s, 2H), 3.31 (d, *J* = 8.5 Hz, 2H), 2.30 (s, 3H).¹³C-NMR (101 MHz, DMSO-*d*₆) δ 169.64, 143.51, 141.03, 140.61, 139.15, 137.39, 136.16, 134.57, 132.73 (2C), 131.90, 130.13, 129.31 (2C), 128.24, 127.29, 126.62, 118.67, 110.81, 47.58, 41.77, 27.21, 21.00. HRMS (ESI): m/z calcd. for C₂₃H₂₀N₃O⁺ [M+H]⁺: 354.1606, found: 354.1510.

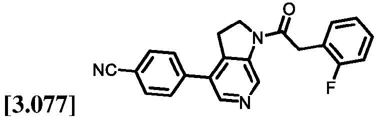

### 4-(1-(2-(2-Fluorophenyl)acetyl)-2,3-dihydro-1H-pyrrolo[2,3-c]pyridin-4-yl)benzonitrile (C67)

Following the general method CD, synthesized with **CI33** (50.0 mg, 0.227 mmol), 2-(2-fluorophenyl) acetic acid (41 mg, 0.272 mmol), HATU (124 mg, 0.341 mmol) and DIPEA (38 mg, 0.681 mmol) in anhydrous dichloromethane (5 mL) to give **C67** (53 mg, 64.5% yield) as a white solid after chromatographic purification (PE/EA = 10/1 to 1/3). mp: 231.2-232.2 °C. ¹H-NMR (400 MHz, DMSO-*d*₆) δ 9.21 (s, 1H), 8.35 (s, 1H), 7.99 (d, *J =* 8.3 Hz, 2H), 7.81 (d, *J =* 8.3 Hz, 2H), 7.50-7.45 (m, 1H), 7.41 (dd, *J* = 5.6, 3.7 Hz, 1H), 7.36-7.30 (m, 2H), 4.30 (t, *J* = 8.4 Hz, 2H), 4.03 (s, 2H), 3.38 (t, *J* = 8.5 Hz, 2H). ¹³C-NMR (101 MHz, DMSO-*d*₆) δ 168.27, 159.05 (¹*J*_{CF} = 283 Hz), 143.58, 141.03, 139.17, 136.07, 133.70, 132.75 (2C), 132.34, 131.95, 131.71 (³*J*_{CF} = 22 Hz), 129.34 (2C), 128.88, 127.10, 1221.95 (²*J*_{CF} = 22 Hz), 118.68, 110.84, 54.92, 47.53, 27.28. ¹⁹F NMR (377 MHz, DMSO-*d*₆) δ -116.31. HRMS (ESI): m/z calcd. for C₂₂H₂₀FN₄O⁺ [M+NH₄]⁺: 375.1621, found: 375.1006.

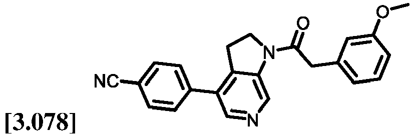

### 4-(1-(2-(3-Bethoxyphenyl)acetyl)-2,3-dihydro-1H-pyrrolo[2,3-c]pyridin-4-yl)benzonitrile (C68)

Following the general method CD, synthesized with **CI33** (50.0 mg, 0.227 mmol), 2-(3-methoxyphenyl)acetic acid (45 mg, 0.272 mmol), HATU (124 mg, 0.341 mmol) and DIPEA (38 mg, 0.681 mmol) in anhydrous dichloromethane (5 mL) to give **C68** (53 mg, 64.3% yield) as a white solid after chromatographic purification (PE/EA = 10/1 to 1/3). mp: 177.2-178.2 °C. ¹H-NMR (400 MHz, DMSO-*d*₆) δ 9.26 (s, 1H), 8.33 (s, 1H), 7.98 (d, *J* = 8.3 Hz, 2H), 7.79 (d, *J* = 8.3 Hz, 2H), 7.25 (t, *J* = 8.1 Hz, 1H), 6.90-6.83 (m, 3H), 4.20 (t, *J* = 8.4 Hz, 2H), 3.87 (s, 2H), 3.75 (s, 3H). ¹³C-NMR (101 MHz, DMSO-*d*₆) δ 169.50, 159.24, 143.54, 141.04, 140.61, 139.17, 136.16, 132.73 (2C), 131.91, 129.35 (2C), 129.32 (2C), 121.78, 118.67, 115.36, 112.01, 110.81, 54.99, 47.59, 41.83, 27.21. HRMS (ESI): m/z calcd. for C₂₃H₂₀N₃O₂⁺ [M+H]⁺:370.1556, found: 370.1474.

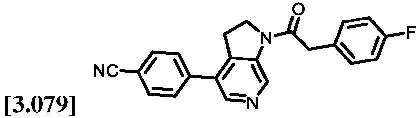

### 4-(1-(2-(4-Fluorophenyl)acetyl)-2,3-dihydro-1H-pyrrolo[2,3-c]pyridin-4-yl)benzonitrile (C69)

Following the general method CD, synthesized with **CI33** (50.0 mg, 0.227 mmol), 2-(4-fluorophenyl)acetic acid (42 mg, 0.272 mmol), HATU (124 mg, 0.341 mmol) and DIPEA (38 mg, 0.681 mmol) in anhydrous dichloromethane (5 mL) to give **C69** (49 mg, 60.3% yield) as a white solid after chromatographic purification (PE/EA = 10/1 to 1/3). mp: 200.5-201.5 °C. ¹H-NMR (400 MHz, DMSO-*d*₆) δ 9.25 (s, 1H), 8.34 (s, 1H), 7.98 (d, *J* = 8.3 Hz, 2H), 7.79 (d, *J* = 8.3 Hz, 2H), 7.34 (dd, *J* = 8.5, 5.7 Hz, 2H), 7.16 (t, *J* = 8.9 Hz, 2H), 4.22 (t, *J* = 8.4 Hz, 2H), 3.91 (s, 2H), 3.35 (d, *J* = 8.5 Hz, 2H). ¹³C-NMR (101 MHz, DMSO-*d*₆) δ 169.55, 162.26 (¹*J*_{CF} = 238 Hz), 143.52, 141.01, 140.59, 139.12, 136.13, 132.73 (2C), 131.89, 131.59 (³*J*_{CF} = 8 Hz, 2C), 130.98 (³*J*_{CF} = 3 Hz), 129.31 (2C), 118.66, 115.05 (²*J*_{CF} = 21 Hz, 2C), 110.81, 47.49, 40.69, 27.21. ¹⁹F NMR (376 MHz, DMSO-*d*₆) δ -104.02. HRMS (ESI): m/z calcd. for C₂₀H₁₇FN₃O₂⁺ [M+H]⁺: 358.1356, found: 358.1268.

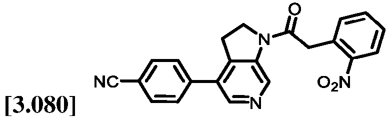

### 4-(1-(2-(2-Nitrophenyl)acetyl)-2,3-dihydro-1H-pyrrolo[2,3-c]pyridin-4-yl)benzonitrile (C70)

Following the general method CD, synthesized with **CI33** (50.0 mg, 0.227 mmol), 2-(2-nitrophenyl)acetic acid (44 mg, 0.272 mmol), HATU (124 mg, 0.341 mmol) and DIPEA (38 mg, 0.681 mmol) in anhydrous dichloromethane (5 mL) to give **C70** (55 mg, 62.6% yield) as a white solid after chromatographic purification (PE/EA = 10/1 to 1/3). mp: 190.5-191.5 °C. ¹H-NMR (400 MHz, DMSO-*d*₆) δ 9.13 (s, 1H), 8.35 (s, 1H), 8.13 (dd, *J* = 8.1, 1.0 Hz, 1H), 8.00 (d, *J* = 8.4 Hz, 2H), 7.82 (d, *J* = 8.4 Hz, 2H), 7.75 (td, *J* = 7.5, 1.2 Hz, 1H), 7.63-7.55 (m, 2H), 4.35 (s, 2H), 4.31 (t, *J* = 8.4 Hz, 2H), 3.42 (t, *J* = 8.4 Hz, 2H). ¹³C-NMR (101 MHz, DMSO-*d*₆) δ 168.09, 148.96, 143.62, 141.01, 140.39, 139.19, 135.97, 133.86, 133.81, 132.74 (2C), 132.00, 130.56, 129.36 (2C), 128.61, 124.49, 118.68, 110.94, 47.49, 27.42, 17.58. HRMS (ESI): m/z calcd. for C₂₂H₁₇N₄O₃⁺ [M+H]⁺: 385.1301, found: 385.1202.

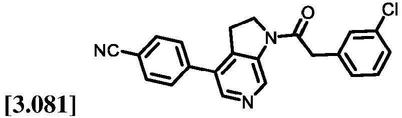

### 4-(1-(2-(3-Chlorophenyl)acetyl)-2,3-dihydro-1H-pyrrolo[2,3-c]pyridin-4-yl)benzonitrile (C71)

Following the general method CD, synthesized with **CI33** (50.0 mg, 0.227 mmol), 2-(3-chlorophenyl)acetic acid (46.4 mg, 0.272 mmol), HATU (124 mg, 0.341 mmol) and DIPEA (38 mg, 0.681 mmol) in anhydrous dichloromethane (5 mL) to give **C71** (50 mg, 59.4% yield) as a white solid after chromatographic purification (PE/EA = 10/1 to 1/3). mp: 157.5-158.5 °C. ¹H-NMR (400 MHz, DMSO-*d*₆) δ 9.24 (s, 1H), 8.35 (s, 1H), 7.98 (d, *J* = 8.3 Hz, 2H), 7.80 (d, *J* = 8.3 Hz, 2H), 7.41-7.34 (m, 3H), 7.27 (d, *J* = 7.2 Hz, 1H), 4.23 (t, *J* = 8.4 Hz, 2H), 3.95 (s, 2H), 2.52 (m, 2H). ¹³C-NMR (101 MHz, DMSO-*d*₆) δ 169.17, 143.51, 140.97, 139.32, 137.37, 136.03, 134.62, 132.75 (2C), 131.95, 131.01, 130.03, 129.70, 129.34 (2C), 128.61, 126.61, 118.67, 110.85, 47.54, 41.01, 27.24. HRMS (ESI): m/z calcd. for C₂₂H₁₇ClN₃O⁺ [M+H]⁺: 374.1060, found: 374.0979.

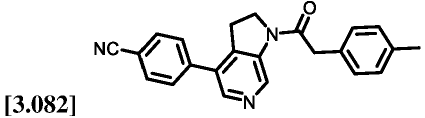

### 4-(1-(2-(p-Tolyl)acetyl)-2,3-dihydro-1H-pyrrolo[2,3-c]pyridin-4-yl)benzonitrile (C72)

Following the general method CD, synthesized with **CI33** (50.0 mg, 0.227 mmol), 2-(p-tolyl)acetic acid (41 mg, 0.272 mmol), HATU (124 mg, 0.341 mmol) and DIPEA (38 mg, 0.681 mmol) in anhydrous dichloromethane (5 mL) to give **C72** (55 mg, 59.3% yield) as a white solid after chromatographic purification (PE/EA = 10/1 to 1/3). mp: 173.5-174.5 °C. ¹H-NMR (400 MHz, DMSO-*d*₆) δ 9.25 (s, 1H), 8.33 (s, 1H), 7.98 (d, *J* = 8.4 Hz, 2H), 7.78 (d, *J* = 8.4 Hz, 2H), 7.17 (q, *J* = 8.0 Hz, 4H), 4.19 (t, *J* = 8.4 Hz, 2H), 3.85 (s, 2H), 3.30 (s, 2H). ¹³C-NMR (101 MHz, DMSO-*d*₆) δ 169.76, 143.47, 141.02, 140.63, 139.16, 136.11, 135.66, 132.72 (2C), 131.90, 131.57, 129.39 (2C), 129.31 (2C), 128.91 (2C), 110.81, 47.54, 41.4, 27.20, 20.67. HRMS (ESI): m/z calcd. for C₂₃H₂₀N₃O⁺ [M+H]⁺: 354.1606, found: 354.1524.

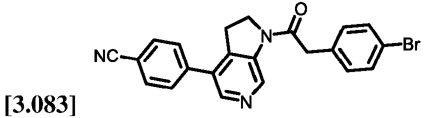

### 4-(1-(2-(4-Bromophenyl)acetyl)-2,3-dihydro-1H-pyrrolo[2,3-c]pyridin-4-yl)benzonitrile (C73)

Following the general method CD, synthesized with **CI33** (50.0 mg, 0.227 mmol), 2-(4-bromophenyl)acetic acid (59 mg, 0.272 mmol), HATU (124 mg, 0.341 mmol) and DIPEA (38 mg, 0.681 mmol) in anhydrous dichloromethane (5 mL) to give **C73** (50 mg, 52.3% yield) as a white solid after chromatographic purification (PE/EA = 10/1 to 1/3). mp: 214.5-215.5 °C. ¹H-NMR (400 MHz, DMSO) δ 9.24 (s, 1H), 8.34 (s, 1H), 7.98 (d, *J* = 8.3 Hz, 2H), 7.80 (d, *J* = 8.3 Hz, 2H), 7.54 (d, *J* = 8.3 Hz, 2H), 7.27 (d, *J* = 8.3 Hz, 2H), 4.22 (t, *J* = 8.4 Hz, 2H), 3.91 (s, 2H), 3.36 (m, 2H). ¹³C-NMR (101 MHz, DMSO) δ 169.23, 143.55, 140.99, 140.55, 139.16, 136.11, 134.30, 132.73 (2C), 132.03 (2C), 131.90, 131.09 (2C), 129.31 (2C), 119.83, 118.66, 110.82, 47.50, 40.9, 27.21. HRMS (ESI): m/z calcd. for C₂₂H₁₇BrN₃O⁺ [M+H]⁺: 418.0555, found: 418.0463;

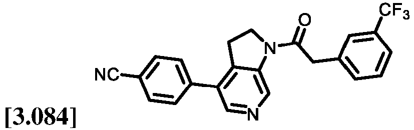

### 4-(1-(2-(3-(Trifluoromethyl)phenyl)acetyl)-2,3-dihydro-1H-pyrrolo[2,3-c]pyridin-4-yl)benzo nitrile (C74)

Following the general method CD, synthesized with **CI33** (50.0 mg, 0.227 mmol), 2-(3-(trifluoromethyl)phenyl)acetic acid (56 mg, 0.272 mmol), HATU (124 mg, 0.341 mmol) and DIPEA (38 mg, 0.681 mmol) in anhydrous dichloromethane (5 mL) to give **C74** (48 mg, 51.4% yield) as a white solid after chromatographic purification (PE/EA = 10/1 to 1/3). mp: 101.5-102.5 °C. ¹H-NMR (400 MHz, DMSO-*d*₆) δ 9.24 (s, 1H), 8.35 (s, 1H), 7.99 (d, *J* = 8.3 Hz, 2H), 7.81 (d, *J* = 8.3 Hz, 2H), 7.70-7.57 (m, 4H), 4.27 (t, *J* = 8.4 Hz, 2H), 4.07 (s, 2H), 3.38 (d, *J* = 8.3 Hz, 2H). ¹³C-NMR (101 MHz, DMSO-*d*₆) δ 169.17, 143.58, 141.00, 140.54, 139.15, 136.42, 136.13, 134.23, 132.74 (2C), 131.91, 129.33 (2C), 129.14, 128.86 (²*J*_{CF} = 32 Hz), 126.52 (³*J*_{CF} = 4 Hz), 124.35 (¹*J*_{CF} = 272 Hz), 123.36 (³*J*_{CF} = 3 Hz), 118.67, 110.83, 47.49, 40.39, 27.23. ¹⁹F NMR (377 MHz, DMSO-*d*₆) δ -60.94. HRMS (ESI): m/z calcd. for C₂₃H₁₇F₃N₃O⁺ [M+H]⁺: 408.1324, found: 418.1228.

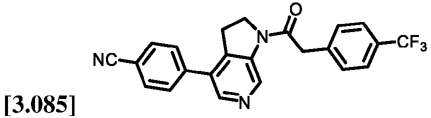

### 4-(1-(2-(4-(Trifluoromethyl)phenyl)acetyl)-2,3-dihydro-1H-pyrrolo[2,3-c]pyridin-4-yl)benzo nitrile (C75)

Following the general method CD, synthesized with **CI33** (50.0 mg, 0.227 mmol), 2-(4-(trifluoromethyl)phenyl)acetic acid (56 mg, 0.272 mmol), HATU (124 mg, 0.341 mmol) and DIPEA (38 mg, 0.681 mmol) in anhydrous dichloromethane (5 mL) to give **C75** (49.6 mg, 50.8% yield) as a white solid after chromatographic purification (PE/EA = 10/1 to 1/3). mp: 145.5-146.5 °C. ¹H-NMR (400 MHz, DMSO-*d*₆) δ 9.24 (s, 1H), 8.35 (s, 1H), 7.99 (d, *J* = 8.2 Hz, 2H), 7.80 (d, *J* = 8.2 Hz, 2H), 7.72 (d, *J* = 8.1 Hz, 2H), 7.54 (d, *J* = 8.0 Hz, 2H), 4.25 (t, *J* = 8.4 Hz, 2H), 4.06 (s, 2H), 3.37 (d, *J* = 8.4 Hz, 2H). ¹³C-NMR (101 MHz, DMSO-*d*₆) δ 169.04, 143.61, 140.99, 140.53, 139.83, 139.18, 136.14, 132.74 (2C), 131.92, 130.73 (2C), 129.33 (2C), 127.35 (²*J*_{CF} = 31 Hz), 126.79 (¹*J*_{CF} = 202 Hz), 125.01 (³*J*_{CF} = 4 Hz, 2C), 110.83, 99.52, 47.52, 41.28, 27.23. HRMS (ESI): m/z calcd. for C₂₃H₁₇F₃N₃O⁺ [M+H]⁺: 408.1324, found: 408.1230.

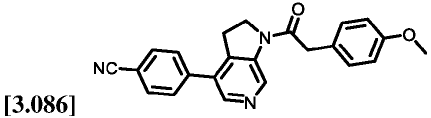

### 4-(1-(2-(4-Methoxyphenyl)acetyl)-2,3-dihydro-1H-pyrrolo[2,3-c]pyridin-4-yl)benzonitrile (C76)

Following the general method CD, synthesized with **CI33** (50.0 mg, 0.227 mmol), 2-(4-methoxyphenyl)acetic acid (45 mg, 0.272 mmol), HATU (124 mg, 0.341 mmol) and DIPEA (38 mg, 0.681 mmol) in anhydrous dichloromethane (5 mL) to give **C76** (50 mg, 59.8% yield) as a white solid after chromatographic purification (PE/EA = 10/1 to 1/3). mp: 135.5-136.5 °C. ¹H-NMR (400 MHz, DMSO-*d*₆) δ 9.25 (s, 1H), 8.33 (s, 1H), 7.98 (d, *J =* 8.3 Hz, 2H), 7.79 (d, *J =* 8.3 Hz, 2H), 7.22 (d, *J* = 8.6 Hz, 2H), 6.90 (d, *J* = 8.6 Hz, 2H), 4.20 (t, *J* = 8.4 Hz, 2H), 3.82 (s, 2H), 3.74 (s, 3H), 3.31 (d, *J* = 9.0 Hz, 2H). ¹³C-NMR (101 MHz, DMSO-*d*₆) δ 169.93, 158.06, 143.44, 141.03, 139.14, 136.06, 134.61, 132.72 (2C), 131.90, 130.56 (2C), 129.31 (2C), 126.50, 118.66, 113.77 (2C), 110.81, 55.04, 47.51, 38.25, 27.21. HRMS (ESI): m/z calcd. for C₂₃H₂₀N₃O₂⁺ [M+H]⁺: 370.1556, found: 370.1457.

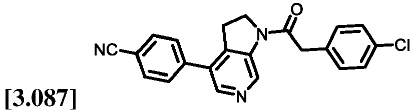

### 4-(1-(2-(4-Chlorophenyl)acetyl)-2,3-dihydro-1H-pyrrolo[2,3-c]pyridin-4-yl)benzonitrile (C77)

Following the general method CD, synthesized with **CI33** (50.0 mg, 0.227 mmol), 2-(4-chlorophenyl)acetic acid (46 mg, 0.272 mmol), HATU (124 mg, 0.341 mmol) and DIPEA (38 mg, 0.681 mmol) in anhydrous dichloromethane (5 mL) to give **C77** (53.3 mg, 62.9%yield) as a white solid after chromatographic purification (PE/EA = 10/1 to 1/3). mp: 175.5-176.5 °C. ¹H-NMR (400 MHz, DMSO-*d*₆) δ 9.24 (s, 1H), 8.34 (s, 1H), 7.98 (d, *J* = 8.1 Hz, 2H), 7.79 (d, *J* = 8.1 Hz, 2H), 7.36 (dd, *J* = 30.1, 8.2 Hz, 4H), 4.22 (t, *J* = 8.2 Hz, 2H), 3.92 (s, 2H), 3.36 (s, 1H), 3.31 (s, 1H). ¹³C-NMR (101 MHz, DMSO-*d*₆) δ 169.30, 143.55, 141.00, 140.58, 139.13, 136.14, 133.87, 132.73 (2C), 131.90, 131.65 (2C), 131.34, 129.31 (2C), 128.16 (2C), 118.66, 110.82, 47.50, 40.86, 27.21. HRMS (ESI): m/z calcd. for C₂₂H₁₈ClN₃O⁺ [M+H]⁺: 374.1060, found: 374.0973.

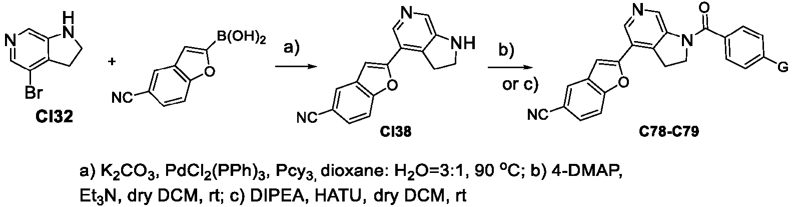

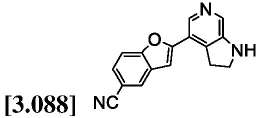

### 2-(2,3-Dihydro-1H-pyrrolo[2,3-c]pyridin-4-yl)benzofuran-5-carbonitrile (CI38)

Following the general method CB, synthesized with CI32 (100 mg, 0.5 mmol), (5-cyanobenzofuran-2-yl)boronic acid (140.2 mg, 0.75 mmol), K₂CO₃ (276.4 mg, 2 mmol), PdCl₂(PPh₃)₂ (35 mg, 0.05 mmol) and Pcy₃ (28 mg, 0.1 mmol) in a mixture of dioxane and water (6 mL, V/V = 3:1) to give **CI38** (88 mg, 67.0% yield) as a white solid after chromatographic purification (PE/EA = 10/1 to 1/3). ¹H-NMR (400MHz, DMSO-d6) δ 8.35 (d, *J* = 1.6 Hz, 1H), 8.22 (t, *J* = 1.8Hz, 1H), 7.89 (m, 2H), 7.77 (dt, *J* = 8.5, 1.6 Hz, 1H), 6.00 (s, 1H), 3.58 (t, *J* = 8.8Hz, 2H).

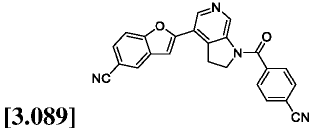

### 4-((4-(5-Chlorobenzofuran-2-yl)-2,3-dihydro-1H-pyrrolo[2,3-c]pyridin-1-yl)sulfonyl)benzoni trile (C78)

Following the general method CA, synthesized with **CI38** (70 mg, 0.27 mmol), 4-cynobenzoyl chloride (88.13 mg, 0.76 mmol), DMAP (9.4 mg, 0.08 mmol) and Et₃N (0.11 mL, 0.77 mol) in anhydrous dichloromethane (4 mL) to give **C78** (73.3 mg, 69.6% yield) as a white solid after chromatographic purification (DCM/MeOH = 75/1). mp: 205.1-206.5 °C. ¹H-NMR (400MHz, DMSO-d6) δ 9.31 (s, 1H), 8.91 (s, 1H), 8.28 (s, 1H), 8.04 (s, 1H), 8.02 (s, 1H), 7.90 (d, *J* = 8.6 Hz, 1H), 7.87 (s, 1H), 7.84 (d, *J* = 7.7 Hz, 1H), 7.81 (s, 1H), 7.58 (s, 1H), 4.12 (t, *J* = 8.3 Hz, 2H), 3.52 (t, *J* = 8.3 Hz, 2H). ¹³C-NMR (101MHz, DMSO-*d6*) δ 168.09, 156.34, 154.26, 152.22, 145.16, 144.38, 140.92, 133.23, 133.22 (2C), 129.45, 128.40 (3C), 127.25, 126.01, 119.65 (2C), 118.76, 113.41, 106.94, 106.04, 52.23, 22.59. HRMS (ESI): m/z calcd. for C₂₄H₁₄N₄O₂⁺ [M+H]⁺: 391.1190, found:391.1191.

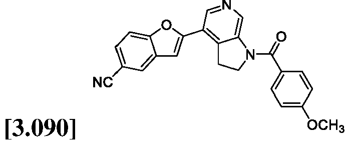

### 2-(1-(4-Methoxybenzoyl)-2,3-dihydro-1H-pyrrolo[2,3-c]pyridin-4-yl)benzofuran-5-carbonitr ile (C79)

Following the general method CA, synthesized with **CI38** (70 mg, 0.27 mmol), 4-methoxybenzoyl chloride (90.72 mg, 0.76 mmol), DMAP (9.4 mg, 0.08 mmol) and Et₃N (0.11 mL, 0.77 mol) in anhydrous dichloromethane (4 mL) to give **C79** (70.3 mg, 69.2% yield) as a white solid after chromatographic purification (DCM/MeOH = 75/1). mp: 115.1-116.5 °C. ¹H-NMR (400MHz, DMSO-*d₆*) δ 8.85 (s, 1H), 8.29 (d, *J* = 1.6Hz, 1H), 7.91 (d, *J* = 8.6Hz, 1H), 7.82 (dd, *J* = 8.6, 1.7 Hz, 1H), 7.66 (d, *J* = 2.1Hz, 1H), 7.64 (d, *J* = 1.9Hz, 1H), 7.56 (s, 1H), 7.07 (d, *J* = 2.1Hz, 1H), 7.06 (d, *J* = 2.0Hz, 1H), 4.22 (t, *J* = 8.3Hz, 2H), 3.84 (s, 3H), 3.52 (t, *J* = 8.3Hz, 2H). ¹³C-NMR (101MHz, DMSO-d6) δ 169.35, 164.09, 159.42, 157.83, 153.00, 142.35, 136.75, 131.78(2C), 129.59, 128.11, 127.22, 125.14, 123.66, 121.37, 118.88, 114.60 (2C), 111.16, 110.06, 108.12, 105.05, 50.96, 24.08. HRMS (ESI): m/z calcd. for C₂₄H₁₇N₃O₃⁺ [M+H]⁺: 396.1343, found: 396.1347.

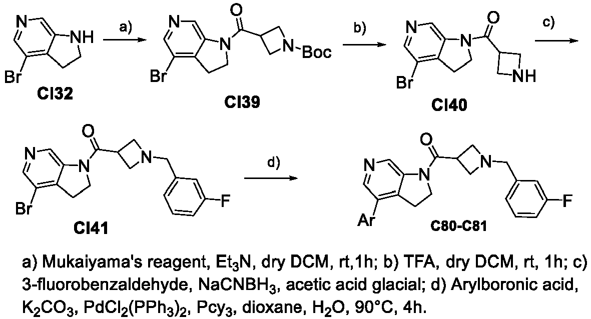

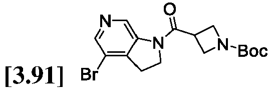

### tert-Butyl 3-(4-bromo-2,3-dihydro-1H-pyrrolo[2,3-c]pyridine-1-carbonyl)azetidine-1-carboxylate (CI39)

To a solution of **CI32** (100 mg, 0.452 mmol), 1-(tert-butoxycarbonyl)azetidine-3-carboxylic acid (136.4 mg, 0.678 mmol) and Et₃N (188 mg, 1.75 mmol) in anhydrous dichloromethane (5 mL) was added *N*-methyl-2-chloropyridinium iodide (Mukaiyama's reagent, NMPI, 346.9 mg, 1.36 mmol). The resulting mixture was stirred at room temperature for 1 h. After the reaction was complete as monitored by TLC, the mixture was diluted with water (5 mL) and extracted with dichloromethane (3×5 mL) for three times. The combined organic layers were washed with brine (10 mL) and dried over anhydrous Na₂SO₄. The solvent was removed under reduced pressure to give the crude product, which was subsequently purified by silica gel column chromatography with a gradient elution of PE:EA (V/V) = 20:1-2:1 to give **CI39** (112.6 mg, 65.2% yield) as a white solid. ¹H-NMR (400 MHz, Chloroform-d) δ 9.22 (s, 1H), 8.29 (s, 1H), 4.18 (s, 2H), 4.07 (t, *J* = 8.5 Hz, 2H), 3.92 (t, *J* = 8.6 Hz, 2H), 3.48 (tt, *J* = 8.6, 6.3 Hz, 1H), 3.15 (t, *J* = 8.6 Hz, 2H), 1.38 (s, 10H).

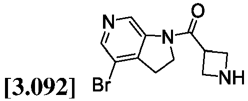

### Azetidin-3-yl(4-bromo-2,3-dihydro-1H-pyrrolo[2,3-c]pyridin-1-yl)methanone (CI40)

To a solution of **CI39** (100 mg, 0.26 mmol) in anhydrous dichloromethane (5 mL) was added trifluoroacetic acid (298.2 mg, 2.62 mmol) in an ice bath. The reaction mixture was subsequently warmed to room temperature and stirred for 1 h. After the reaction was complete as monitored by TLC, it was neutralized to pH of around 7 with saturated Na₂CO₃ aq., and extracted with dichloromethane (3×5 mL). The combined organic layers were washed with brine (5 mL) and dried over anhydrous sodium sulfate. It was then concentrated under reduced pressure to give the crude product, which was subsequently purified by column chromatography with a gradient elution of CH₂Cl₂: MeOH (V/V) = 100:1 to 10:1, yielding the compound **CI40** (70.1 mg, 95.0% yield) as a white solid, which was used directly in the next step without further characterization.

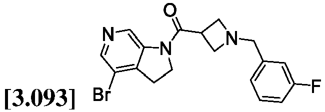

### (4-Bromo-2,3-dihydro-1H-pyrrolo[2,3-c]pyridin-1-yl)(1-(3-fluorobenzyl)azetidin-3-yl)methano ne (CI41)

To a solution of **CI40** (100 mg, 0.35mmol) and 3-fluorobenzaldehyde (57.8 mg, 0.43 mmol) in methanol (2 mL) was added sodium cyanoborohydride (NaBH₃CN, 26.7 mg, 0.43 mmol) slowly in an ice bath. The resulting reaction mixture was allowed to warm to room temperature and continued to stir for 30 min. After the reaction was complete as monitored by TLC, the reaction mixture was diluted with water (4 mL) and extracted with ethyl acetate (3×5 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography with a gradient elution of CH₂Cl₂: MeOH (V/V) = 300:1 to 75:1 to give **CI41** (117.6 mg, 85.1%) as a white solid. ¹H-NMR (400 MHz, Chloroform-*d*) δ 9.52-9.47 (m, 2H), 9.26 (d, *J* = 4.3 Hz, 2H), 8.65 (d, *J* = 4.2 Hz, 1H), 8.29 (t, *J* = 6.1 Hz, 3H), 7.44-7.36 (m, 1H), 7.29 (s, 3H), 7.20 (q, *J* = 8.6, 7.4 Hz, 2H), 7.02-6.83 (m, 7H), 6.57 (t, *J* = 3.7 Hz, 1H), 3.98-3.88 (m, 4H), 3.63 - 3.56 (m, 11H), 3.54 - 3.46 (m, 1H), 3.41-3.33 (m, 4H), 3.22-3.08 (m, 5H).

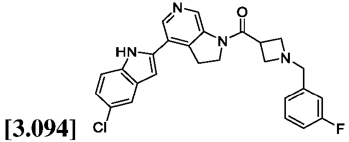

### (4-(5-Chloro-1H-indol-2-yl)-2,3-dihydro-1H-pyrrolo[2,3-c]pyridin-1-yl)(1-(3-fluorobenzyl)azet idin-3-yl)methanone (C80)

Following the general method CB, synthesized with **CI41** (50.0 mg, 0.128 mmol), (5-chloro-1*H*-indol-2-yl)boronic acid (50.1 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C80** (37.2 mg, 63.1% yield) as a white solid after chromatographic purification (DCM/MeOH = 100/1 to 10/1). mp: 200.2-201.2 °C. ¹H-NMR (400 MHz, DMSO-*d₆*) δ 11.80 (d, *J* = 2.3 Hz, 1H), 9.22 (s, 1H), 8.68 (s, 1H), 7.62 (d, *J =* 2.0 Hz, 1H), 7.45 (d, *J* = 8.6 Hz, 1H), 7.35 (td, *J* = 8.0, 6.2 Hz, 1H), 7.18-7.01 (m, 4H), 6.77 (d, *J* = 2.0 Hz, 1H), 4.03 (t, *J* = 8.5 Hz, 2H), 3.68-3.61 (m, 1H), 3.59 (s, 2H), 3.52 (t, *J* = 7.4 Hz, 2H), 3.40 (t, *J* = 8.5 Hz, 2H), 3.31 (t, *J* = 7.1 Hz, 2H).¹⁹F NMR (376 MHz, DMSO-*d₆*) δ -113.66. HRMS (ESI): m/z calcd. for C₂₆H₂₂ClFN₄O⁺ [M+H]⁺ :461.1539, found: 461.1540.

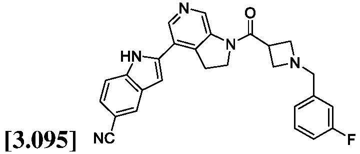

### 2-(1-(1-(3-Fluorobenzyl)azetidine-3-carbonyl)-2,3-dihydro-1H-pyrrolo[2,3-c]pyridin-4-yl)-1 H-indole-5-carbonitrile (C81)

Following the general method CB, synthesized with **CI41** (50.0 mg, 0.128 mmol), (5-cyno-1*H*-indol-2-yl)boronic acid (48.8 mg, 0.23 mmol), K₂CO₃ (87 mg, 0.63 mmol), PdCl₂(PPh₃)₂ (11 mg, 0.01 mmol) and Pcy₃ (9 mg, 0.03 mmol) in a mixture of dioxane and water (4 mL, V/V = 3:1) to give **C80** (37.9 mg, 62.1% yield) as a white solid after chromatographic purification (DCM/MeOH = 100/1 to 10/1). mp: 175.2-176.2 °C. ¹H-NMR (400 MHz, DMSO-*d*₆) δ 12.21 (s, 1H), 9.24 (s, 1H), 8.70 (s, 1H), 8.12 (d, *J* = 1.6 Hz, 1H), 7.59 (d, *J* = 8.5 Hz, 1H), 7.49 (dd, *J* = 8.4, 1.6 Hz, 1H), 7.35 (td, *J* = 8.0, 6.2 Hz, 1H), 7.12 (d, *J* = 7.5 Hz, 1H), 7.11-7.08 (m, 1H), 7.08-7.02 (m, 1H), 6.92 (s, 1H), 4.04 (t, *J* = 8.5 Hz, 2H), 3.68-3.61 (m, 1H), 3.59 (s, 2H), 3.52 (t, *J* = 7.4 Hz, 2H), 3.44 (d, *J* = 8.4 Hz, 2H), 3.31 (t, *J* = 7.1 Hz, 2H).¹³C-NMR (101 MHz, DMSO-*d₆*) δ 170.90, 163.9033 (¹*J*_{CF} *=* 241.0 Hz), 142.32, 141.81, 141.73, 141.32, 138.99, 138.67, 136.10, 136.02, 130.64, 130.56, 128.53, 126.43, 125.39, 124.79, 124.71, 124.6874, 121.04, 115.31, 115.10, 114.20 (²*J*_{CF} = 21.0 Hz), 113.20, 103.69, 102.18, 62.33, 56.10, 47.11, 34.99, 28.81.¹⁹F NMR (376 MHz, DMSO-*d₆*) δ -113.68. HRMS (ESI): m/z calcd. for C₂₇H₂₃FN₅O⁺ [M+H]⁺ :452.1881, found: 452.1884.

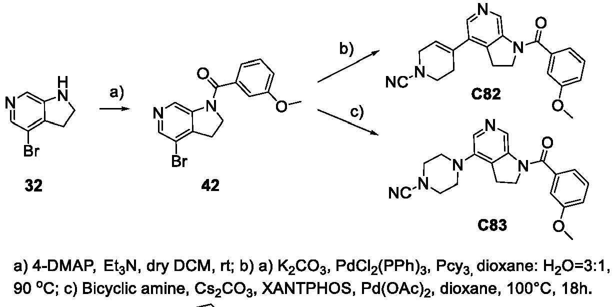

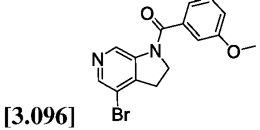

### (4-Bromo-2,3-dihydro-1H-pyrrolo[2,3-c]pyridin-1-yl)(3-methoxyphenyl)methanone (CI42)

To a solution of **CI32** (100 mg, 0.452 mmol), 3-methoxybenzoic acid (103.2 mg, 0.678 mmol) and Et₃N (188 mg, 1.75 mmol) in anhydrous dichloromethane (5 mL) was added N-methyl-2-chloropyridinium iodide (Mukaiyama's reagent, NMPI, 346.9 mg, 1.36 mmol). The resulting mixture was stirred at room temperature for 1 h. After the reaction was complete as monitored by TLC, the mixture was diluted with water (5 mL) and extracted with dichloromethane (3×5 mL) for three times. The combined organic layers were washed with brine (10 mL) and dried over anhydrous Na₂SO₄. The solvent was removed under reduced pressure to give the crude product, which was subsequently purified by silica gel column chromatography with a gradient elution of PE:EA (V/V) = 20:1-2:1 to give **CI42** (110.4 mg, 62.2% yield) as a white solid, which was used directly in the next step without characterization.

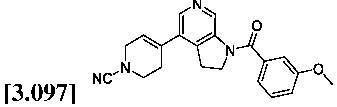

### 4-(1-(3-Methoxybenzoyl)-2,3-dihydro-1H-pyrrolo[2,3-c]pyridin-4-yl)-3,6-dihydropyridine-1(2 H)-carbonitrile (C82)

Following the general method CB, synthesized with **CI42** (100 mg, 0.30 mmol), (1-cyano-1,2,3,6-tetrahydropyridin-4-yl)boronic acid (35.0 mg, 0.23 mmol), (276.4 mg, 2 mmol), PdCl₂(PPh₃)₂ (35 mg, 0.05 mmol) and Pcy₃ (28 mg,0.1 mmol) in a mixture of dioxane and water (6 mL, V/V = 3:1) to give **C80** (71.3 mg, 86.1% yield) as a white solid after chromatographic purification (DCM/MeOH = 100/1 to 10/1). ¹H-NMR (400 MHz, DMSO-*d₆*) δ 8.98 (s, 1H), 8.37 (s, 1H), 7.63 (d, *J* = 8.7 Hz, 2H), 7.06 (d, *J* = 8.8 Hz, 2H), 6.15 (s, 1H), 4.11 (t, *J* = 8.3 Hz, 2H), 3.83 (d, *J* = 4.8 Hz, 3H), 3.28-3.30 (m, 2H), 3.21-3.25 (m, 4H), 2.09-2.10 (m, 2H).

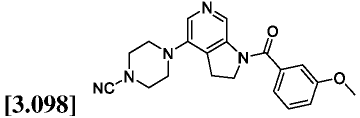

### 4-(1-(3-Methoxybenzoyl)-2,3-dihydro-1H-pyrrolo[2,3-c]pyridin-4-yl)piperazine-1-carbonitrile (C83)

To a suspension of **CI42** (100 mg, 0.30 mmol), Cs₂CO₃ (343.3 mg, 1.05 mmol), XANTPHOS (52.1 mg, 0.09 mmol) and Pd(OAc)₂ (7.2 mg, 0.03 mmol) in dioxane (9 mL) was added piperazine-1-carbonitrile (33.3 mg, 0.30 mmol). The reaction mixture was stirred at 100 °C under N₂ for 18 h. After the reaction was complete as monitored by TLC, it was cooled to room temperature, quenched with water (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of CH₂Cl₂: MeOH (V/V) = 100:1 to 10:1, yielding the compound **c83** (27 mg, 25.1% yield). ¹H-NMR (400 MHz, DMSO-*d*₆) δ 8.98 (s, 1H), 8.37 (s, 1H), 7.63 (d, *J* = 8.7 Hz, 2H), 7.06 (d, *J* = 8.8 Hz, 2H), 4.11 (t, *J* = 8.3 Hz, 2H), 3.83 (d, *J* = 4.8 Hz, 3H), 3.23-3.28 (m, 10H).

### Example 4:

Synthetic procedures and structural characterizations of provided compounds of formulae **(XV)-(XVII)** and intermediates are described:
**General method EA:** The corresponding bromide (1 mmol), Na₂CO₃ (4 mmol), and the corresponding boronic acid (1.5 mmol) were added to a mixture of acetonitrile (3 mL) and water (1 mL). To this mixture was added Pd(PPh₃)₄ (0.05 mmol) under nitrogen atmosphere. The reaction mixture was subsequently heated to 80 °C and stirred for 12 hours. After the reaction was complete as monitored by TLC, it was cooled to room temperature, diluted with water (5 mL), and extracted with ethyl acetate (3×5 mL). The combined organic layers were washed with brine (5 mL) and dried over anhydrous Na₂SO₄. The solvent was removed under reduced pressure to give the crude product, which was subsequently purified by silica gel column chromatography (PE/EA) to yield the target compound.
**General method EB:** To a solution of the corresponding 1*H*-pyrrolo[2,3-c]pyridine (0.2 mmol), DMAP (7.2 mg, 0.06 mmol), and triethylamine (0.6 mmol) in anhydrous dichloromethane (5 mL) was added dropwise the corresponding acyl chloride (0.3 mmol) at 0°C under N₂. The mixture was then warmed to room temperature and stirred for 8 hours. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.
**General method EC:** To a solution of the corresponding 1*H*-pyrrolo[2,3-*c*]pyridine (0.2 mmol), DMAP (7.2 mg, 0.06 mmol), and triethylamine (0.6 mmol) in anhydrous dichloromethane (5 mL) was added dropwise the corresponding sulfonyl chloride (0.3 mmol) at 0°C under N₂. The mixture was then warmed to room temperature and stirred for 8 hours. After the reaction was complete as monitored by TLC, the mixture was diluted with water (5 mL) and extracted with dichloromethane (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.
**General method ED:** To a solution of the corresponding 1*H*-pyrrolo[2,3-c]pyridine (0.2 mmol) in anhydrous DMF (5 mL) was added NaH (0.3 mmol) in batch at 0°C under N₂. The resulting mixture was stirred in an ice bath for 30 min before the corresponding sulfonyl chloride (0.30 mmol) was added dropwise at 0 °C. The mixture was subsequently warmed to room temperature and stirred for further 3 hours. After the reaction was complete as monitored by TLC, the mixture was diluted with water (5 mL) and extracted with ethyl acetate (5 mL) for three times. The combined organic layers were washed with brine (10 mL) and dried over anhydrous Na₂SO₄. The solvent was removed under reduced pressure to give the crude product, which was subsequently purified by silica gel column chromatography (PE/EA) to yield the target compound.

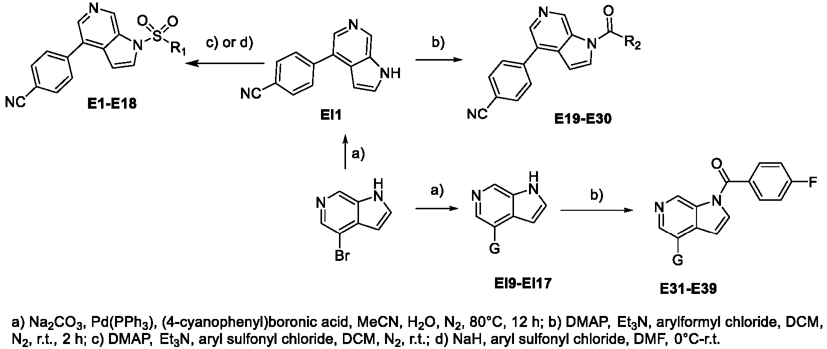

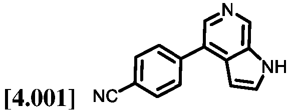

### 4-(1H-Pyrrolo[2,3-c]pyridin-4-yl)benzonitrile (EI1)

Following the general method EA, synthesized with 4-bromo-1*H*-pyrrolo[2,3-c]pyridine (99.0 mg, 0.5 mmol), (4-cyanophenyl)boronic acid (81.1 mg, 0.55 mmol), Na₂CO₃ (87 mg, 0.63 mmol), Pd (PPh₃)₄ (29 mg, 0.025 mmol) in a mixture of acetonitrile and water (4 mL, V/V = 3:1) to give **EI1** (79.4 mg, 72.4% yield) as a brown solid after chromatographic purification (PE/EA = 1/1). ¹H-NMR (400 MHz, DMSO-*d₆*): δ 11.91 (s, 1H), 8.82 (s, 1H), 8.29 (s, 1H), 7.98 (d, *J* = 8.4 Hz, 2H), 7.93 (d, *J* = 8.4 Hz, 2H), 7.75 (s, 1H), 6.68 (s, 1H).

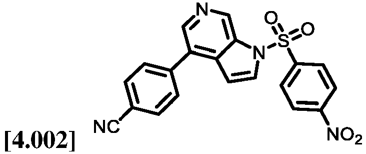

### 4-(1-((4-Nitrophenyl)sulfonyl)-1H-pyrrolo[2,3-c]pyridin-4-yl)benzonitrile (E1)

Following the general method EC, synthesized with **EI1** (0.164 g, 0.75 mmol), 4-nitrobenzenesulfonyl chloride (0.183 g, 0.825 mmol), DMAP (0.028 g, 0.225 mmol) and triethylamine (0.21 mL, 1.5 mmol) in anhydrous dichloromethane (4 mL) to give **E1** (0.207 g, 68.2% yield) as a light yellow solid after chromatographic purification (PE/EA = 15/1), mp: 196.0-197.0 °C. ¹H-NMR (400 MHz, DMSO-*d₆*): δ 9.33 (s, 1H), 8.59 (s, 1H), 8.47-8.43 (m, 2H), 8.42-8.38 (m, 2H), 8.28 (d, *J* = 3.7 Hz, 1H), 8.00 (d, *J* = 8.3 Hz, 2H), 7.85 (d, *J* = 8.2 Hz, 2H), 7.07 (d, *J* = 3.7 Hz, 1H). ¹³C-NMR (100 MHz, DMSO-*d₆*): δ 151.62, 142.48, 141.86, 140.90, 135.19, 134.38, 133.44 (2C), 131.92, 131.58, 130.02 (2C), 129.36 (2C), 128.22, 125.81 (2C), 119.09, 111.42, 108.29. HRMS (ESI): m/z calcd. for C₂₀H₁₃N₄O₄S⁺ [M+H]⁺: 405.0652, found 405.0641.

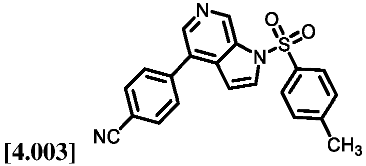

### 4-(1-Tosyl-1H-pyrrolo[2,3-c]pyridin-4-yl)benzonitrile (E2)

Following the general method EC, synthesized with **EI1** (0.164 g, 0.75 mmol), 4-methylbenzenesulfonyl chloride (0.171 g, 0.825 mmol), DMAP (0.028 g, 0.225 mmol) and triethylamine (0.21 mL, 1.5 mmol) in anhydrous dichloromethane (4 mL) to give **E2** (0.186 g, 66.5% yield) as a white solid after chromatographic purification (PE/EA = 15/1), mp: 188.0-189.0 °C. ¹H-NMR (400 MHz, DMSO-*d₆*): δ 9.28 (s, 1H), 8.54 (s, 1H), 8.20 (d, *J* = 3.7 Hz, 1H), 8.03 (d, *J* = 8.4 Hz, 2H), 7.99 (d, *J* = 8.4 Hz, 2H), 7.87-7.83 (m, 2H), 7.44 (d, *J* = 8.2 Hz, 2H), 6.99 (d, *J* = 3.7 Hz, 1H), 2.34 (s, 3H). ¹³C-NMR (100 MHz, DMSO-*d₆*): *δ* 146.76, 141.93, 141.10, 135.23, 134.19, 134.15, 133.42 (2C), 132.03, 131.52, 131.05 (2C), 130.00 (2C), 128.04, 127.60 (2C), 119.13, 111.33, 107.24, 21.55. HRMS (ESI): m/z calcd. for C₂₁H₁₅N₃O₂S⁺ [M+H]⁺: 374.0958, found 374.0942.

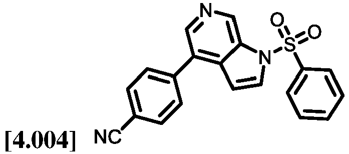

### 4-(1-(Phenylsulfonyl)-1H-pyrrolo[2,3-c]pyridin-4-yl)benzonitrile (E3)

Following the general method EC, synthesized with **EI1** (0.164 g, 0.75 mmol), benzenesulfonyl chloride (0.146 g, 0.825 mmol), DMAP (0.028 g, 0.225 mmol) and triethylamine (0.21 mL, 1.5 mmol) in anhydrous dichloromethane (4 mL) to give **E3** (0.173 g, 64.5% yield) as a white solid after chromatographic purification (PE/EA = 15/1), mp: 178.0-179.0 °C. ¹H-NMR (400 MHz, DMSO-*d*₆): *δ* 9.30 (s, 1H), 8.55 (s, 1H), 8.23 (d, *J* = 3.7 Hz, 1H), 8.19-8.13 (m, 2H), 7.99 (d, *J=* 8.3 Hz, 2H), 7.85 (d, *J* = 8.4 Hz, 2H), 7.76 (dd, *J* = 11.9, 4.4 Hz, 1H), 7.64 (dd, *J* = 12.5, 5.0 Hz, 2H), 7.00 (d, *J* = 3.6 Hz, 1H). ¹³C-NMR (100 MHz, DMSO-*d*₆): *δ* 142.05, 141.08, 137.10, 135.75, 135.16, 134.72, 134.14, 133.41 (2C), 132.00, 130.65 (2C), 130.02 (2C), 127.56 (2C), 119.13, 116.87, 111.34, 107.43. HRMS (ESI): m/z calcd. for C₂₀H₁₄N₃O₂S⁺ [M+H]⁺: 360.0801, found 360.0795.

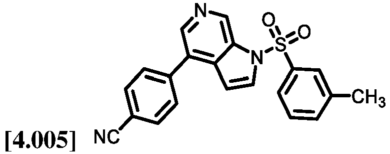

### 4-(1-(m-Tolylsulfonyl)-1H-pyrrolo[2,3-c]pyridin-4-yl)benzonitrile (E4)

Following the general method EC, synthesized with **EI1** (0.164 g, 0.75 mmol), 3-methylbenzenesulfonyl chloride (0.170 g, 0.825 mmol), DMAP (0.028 g, 0.225 mmol) and triethylamine (0.21 mL, 1.5 mmol) in anhydrous dichloromethane (4 mL) to give **E4** (0.188 g, 67.5% yield) as a light yellow solid after chromatographic purification (PE/EA = 15/1), mp: 190.0-191.0 °C. ¹H-NMR (400 MHz, DMSO-*d*₆): *δ* 9.30 (s, 1H), 8.54 (s, 1H), 8.21 (d, *J* = 3.7 Hz, 1H), 8.04-7.90 (m, 4H), 7.88-7.81 (m, 2H), 7.58-7.50 (m, 2H), 7.00 (d, *J* = 3.7 Hz, 1H), 2.37 (s, 3H). ¹³C-NMR (100 MHz, DMSO-*d*₆): δ 141.96, 141.11, 140.75, 137.08, 136.38, 135.26, 134.08, 133.41 (2C), 132.01, 131.54, 130.42, 130.01 (2C), 128.05, 127.54, 124.77, 119.13, 111.34, 107.27, 21.14. HRMS (ESI): m/z calcd. for C₂₁H₁₆N₃O₂S⁺ [M+H]⁺: 374.0957, found 374.0946.

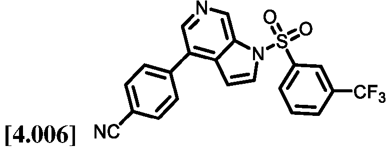

### 4-(1-((3-(Trifluoromethyl)phenyl)sulfonyl)-1H-pyrrolo[2,3-c]pyridin-4-yl)benzonitrile (E5)

Following the general method EC, synthesized with **EI1** (0.164 g, 0.75 mmol), 3-triflouromethylbenzenesulfonyl chloride (0.202 g, 0.825 mmol), DMAP (0.028 g, 0.225 mmol) and triethylamine (0.21 mL, 1.5 mmol) in anhydrous dichloromethane (4 mL) to give **E5** (0.180 g, 56.5% yield) as a light yellow solid after chromatographic purification (PE/EA = 5/1), mp: 187.0-188.0 °C. ¹H-NMR (400 MHz, DMSO-*d*₆): *δ* 9.37 (s, 1H), 8.57 (s, 1H), 8.52 (d, *J* = 7.1 Hz, 2H), 8.35 (dd, *J* = 3.7, 0.5 Hz, 1H), 8.15 (d, *J* = 7.8 Hz, 1H), 7.98 (dd, *J* = 8.5, 1.1 Hz, 2H), 7.90 (d, *J* = 8.4 Hz, 1H), 7.87-7.83 (m, 2H), 7.05 (d, *J* = 3.7 Hz, 1H). ¹³C-NMR (100 MHz, DMSO-*d*₆): *δ* 142.35, 140.98, 138.31, 135.26, 134.29, 133.41 (2C), 132.46, 132.32, 132.07, 131.67, 131.58, 131.12 (²*J*_{CF} = 32.99 Hz), 130.04 (2C), 128.15, 124.37 (³*J*_{CF} = 3.90 Hz), 123.43 (¹*J*_{CF} = 273.28 Hz) 119.12, 111.38, 107.99. HRMS (ESI): m/z calcd. for C₂₁H₁₃F₃N₃O₂S⁺ [M+H]⁺: 428.0675, found 428.0656.

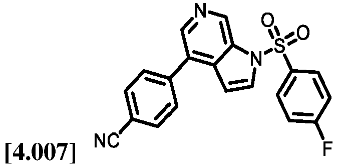

### 4-(1-((4-Fluorophenyl)sulfonyl)-1H-pyrrolo[2,3-c]pyridin-4-yl)benzonitrile (E6)

Following the general method EC, synthesized with **EI1** (0.164 g, 0.75 mmol), 4-flourobenzenesulfonyl chloride (0.161 g, 0.825 mmol), DMAP (0.028 g, 0.225 mmol) and triethylamine (0.21 mL, 1.5 mmol) in anhydrous dichloromethane (4 mL) to give **E6** (0.165 g, 58.5% yield) as a light yellow solid after chromatographic purification (PE/EA = 5/1), mp: 219.0-220.0 °C. ¹H-NMR (400 MHz, DMSO-*d*₆): *δ* 9.31 (s, 1H), 8.56 (s, 1H), 8.30-8.25 (m, 2H), 8.23 (d, *J* = 3.7 Hz, 1H), 7.99 (d, *J* = 8.2 Hz, 2H), 7.86 (d, *J* = 8.2 Hz, 2H), 7.50 (t, *J =* 8.8 Hz, 2H), 7.02 (d, *J* = 3.7 Hz, 1H). ¹³C-NMR (100 MHz, DMSO-*d*₆): *δ* 166.44 (¹*J*_{CF}= 242.49 Hz), 142.13, 141.06, 135.24, 134.18, 133.43 (2C), 131.97, 131.10 (³*J*_{CF}= 10.31 Hz, 2C), 130.03 (2C), 128.10, 126.15 (⁴*J*_{CF}= 4.78 Hz), 119.26, 118.04 (²*J*_{CF}= 23.36 Hz, 2C), 111.36, 107.64, 107.57. HRMS (ESI): m/z calcd. for C₂₀H₁₃FN₃O₂S⁺ [M+H]⁺: 378.0707, found 378.0700.

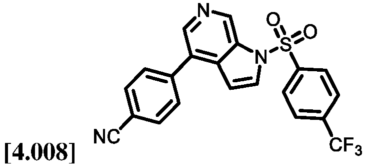

### 4-(1-((4-(Trifluoromethyl)phenyl)sulfonyl)-1H-pyrrolo[2,3-c]pyridin-4-yl)benzonitrile (E7)

Following the general method EC, synthesized with **EI1** (0.164 g, 0.75 mmol), 4-triflouromethylbenzenesulfonyl chloride (0.202 g, 0.825 mmol), DMAP (0.028 g, 0.225 mmol) and triethylamine (0.21 mL, 1.5 mmol) in anhydrous dichloromethane (4 mL) to give **E7** (0.173 g, 54.5% yield) as a light yellow solid after chromatographic purification (PE/EA = 5/1), mp: 193.0-194.0 °C. ¹H-NMR (400 MHz, DMSO-*d*₆): *δ* 9.32 (s, 1H), 8.58 (s, 1H), 8.40 (d, *J =* 8.4 Hz, 2H), 8.27 (d, *J* = 3.7 Hz, 1H), 8.03 (d, *J* = 8.5 Hz, 2H), 7.99 (d, *J* = 8.3 Hz, 2H), 7.85 (d, *J =* 8.3 Hz, 2H), 7.05 (d, *J* = 3.7 Hz, 1H). ¹³C-NMR (100 MHz, DMSO-*d*₆): δ 142.37, 140.95, 140.82, 140.81, 135.19, 135.06, 134.73, 134.32, 133.42 (2C), 131.76 (²*J*_{CF}= 34.39 Hz), 130.03 (2C), 128.72 (2C), 127.87 (³*J*_{CF}= 3.64 Hz, 2C), 123.46 (¹*J*_{CF}= 273.48 Hz), 119.11, 111.40, 108.07. HRMS (ESI): m/z calcd. for C₂₁H₁₃F₃N₃O₂S⁺ [M+H]⁺: 428.0675, found 428.0657.

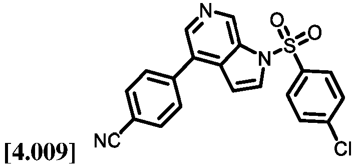

### 4-(1-((4-Chlorophenyl)sulfonyl)-1H-pyrrolo[2,3-c]pyridin-4-yl)benzonitrile (E8)

Following the general method EC, synthesized with **EI1** (0.164 g, 0.75 mmol), 4-chloromethylbenzenesulfonyl chloride (0.174 g, 0.825 mmol), DMAP (0.028 g, 0.225 mmol) and triethylamine (0.21 mL, 1.5 mmol) in anhydrous dichloromethane (4 mL) to give **E8** (0.180 g, 61.2% yield) as a white solid after chromatographic purification (PE/EA = 5/1), mp: 171.0-172.0 °C. ¹H-NMR (400 MHz, DMSO-*d*₆): *δ* 9.30 (s, 1H), 8.57 (s, 1H), 8.23 (d, *J* = 3.7 Hz, 1H), 8.18 (d, *J* = 8.8 Hz, 2H), 8.02-7.97 (m, 2H), 7.87-7.83 (m, 2H), 7.72 (dd, *J* = 8.5, 2.8 Hz, 2H), 7.02 (d, *J* = 3.6 Hz, 1H). ¹³C-NMR (100 MHz, DMSO-*d*₆): *δ* 142.21, 141.02, 140.89, 135.83, 135.23, 134.23, 133.42 (2C), 131.95, 131.54, 130.82 (2C), 130.02 (2C), 129.56 (2C), 128.13, 119.12, 111.37, 107.74. HRMS (ESI): m/z calcd. for C₂₀H₁₃ClN₃O₂S⁺ [M+H]⁺: 394.0412, found 394.0404.

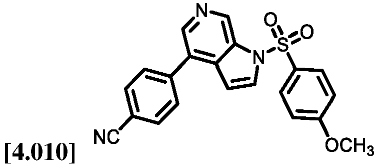

### 4-(1-((4-Methoxyphenyl)sulfonyl)-1H-pyrrolo[2,3-c]pyridin-4-yl)benzonitrile (E9)

Following the general method EC, synthesized with **EI1** (0.164 g, 0.75 mmol), 4-methoxybenzenesulfonyl chloride (0.171 g, 0.825 mmol), DMAP (0.028 g, 0.225 mmol) and triethylamine (0.21 mL, 1.5 mmol) in anhydrous dichloromethane (4 mL) to give **E9** (0.172 g, 59.2% yield) as a light yellow solid after chromatographic purification (PE/EA = 10/1), mp: 143.0-144.0 °C. ¹H-NMR (400 MHz, DMSO-*d*₆): *δ* 9.28 (s, 1H), 8.54 (s, 1H), 8.19 (d, *J* = 3.7 Hz, 1H), 8.09 (d, *J* = 9.0 Hz, 2H), 7.99 (d, *J* = 8.1 Hz, 2H), 7.85 (d, *J* = 8.0 Hz, 2H), 7.13 (d, *J* = 8.5 Hz, 2H), 6.98 (d, *J* = 3.7 Hz, 1H), 3.81 (s, 3H). ¹³C-NMR (100 MHz, DMSO-*d*₆): *δ* 164.71, 141.83, 141.16, 135.25, 134.04, 133.41 (2C), 132.00, 131.48, 130.11 (2C), 129.99 (2C), 128.32, 128.00, 119.14, 115.79 (2C), 111.31, 107.00, 56.45. HRMS (ESI): m/z calcd. for C₂₁H₁₆N₃O₃S⁺ [M+H]⁺: 390.0807, found 390.0886.

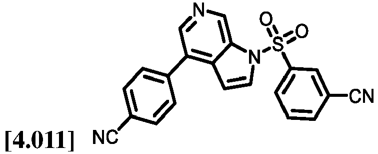

### 3-((4-(4-Cyanophenyl)-1H-pyrrolo[2,3-c]pyridin-1-yl)sulfonyl)benzonitrile (E10)

Following the general method EC, synthesized with **EI1** (0.164 g, 0.75 mmol), 3-cynobenzenesulfonyl chloride (0.167 g, 0.825 mmol), DMAP (0.028 g, 0.225 mmol) and triethylamine (0.21 mL, 1.5 mmol) in anhydrous dichloromethane (4 mL) to give **E10** (0.121 g, 42.2% yield) as a white solid after chromatographic purification (PE/EA = 10/1), mp: 198.0-199.0 °C. ¹H-NMR (400 MHz, DMSO-*d*₆): *δ* 9.38 (s, 1H), 8.84 (d, *J* = 1.6 Hz, 1H), 8.58 (s, 1H), 8.52-8.47 (m, 1H), 8.27 (d, *J* = 3.7 Hz, 1H), 8.22 (d, *J* = 7.9 Hz, 1H), 7.99 (d, *J* = 8.3 Hz, 2H), 7.89-7.80 (m, 3H), 7.05 (d, *J* = 3.7 Hz, 1H). ¹³C-NMR (100 MHz, DMSO-*d*₆): *δ* 142.35, 141.01, 139.22, 138.24, 135.39, 134.26, 133.43 (2C), 131.97 (2C), 131.93, 131.60, 131.52, 130.04 (2C), 131.60, 128.12, 117.44, 113.90, 111.37, 107.95. HRMS (ESI): m/z calcd. for C₂₁H₁₃N₄O₃S⁺ [M+H]⁺: 385.0754, found 385.0747.

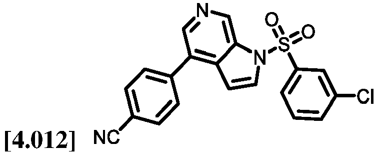

### 4-(1-((3-Chlorophenyl)sulfonyl)-1H-pyrrolo[2,3-c]pyridin-4-yl)benzonitrile (E11)

Following the general method EC, synthesized with **EI1** (0.164 g, 0.75 mmol), 3-chlorobenzenesulfonyl chloride (0.175 g, 0.825 mmol), DMAP (0.028 g, 0.225 mmol) and triethylamine (0.21 mL, 1.5 mmol) in anhydrous dichloromethane (4 mL) to give **E11** (0.201 g, 68.2% yield) as a white solid after chromatographic purification (PE/EA = 10/1), mp: 206.0-207.0 °C. ¹H-NMR (400 MHz, DMSO-*d*₆): *δ* 9.34 (s, 1H), 8.57 (s, 1H), 8.31-8.27 (m, 2H), 8.16 (d, *J* = 8.0 Hz, 1H), 7.99 (d, *J* = 8.2 Hz, 2H), 7.84 (dd, *J* = 12.7, 4.7 Hz, 3H), 7.67 (t, *J* = 8.0 Hz, 1H), 7.04 (d, *J* = 3.7 Hz, 1H). ¹³C-NMR (100 MHz, DMSO-*d*₆): *δ* 142.25, 141.01, 138.79, 135.78, 135.28, 135.19, 134.26, 133.41 (2C), 132.60, 132.07, 131.55, 130.05 (2C), 128.14, 127.14, 126.43, 119.13, 111.37, 107.84. HRMS (ESI): m/z calcd. for C₂₀H₁₃ClN₃O₂S⁺ [M+H]⁺: 394.0412, found 394.0396.

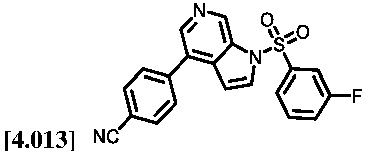

### 4-(1-((3-Fluorophenyl)sulfonyl)-1H-pyrrolo[2,3-c]pyridin-4-yl)benzonitrile (E12)

Following the general method EC, synthesized with **EI1** (0.164 g, 0.75 mmol), 3-flourobenzenesulfonyl chloride (0.161 g, 0.825 mmol), DMAP (0.028 g, 0.225 mmol) and triethylamine (0.21 mL, 1.5 mmol) in anhydrous dichloromethane (4 mL) to give **E12** (0.161 g, 57.2% yield) as a white solid after chromatographic purification (PE/EA = 10/1), mp: 157.0-158.0 °C. ¹H-NMR (400 MHz, DMSO-*d*₆): *δ* 9.34 (s, 1H), 8.57 (s, 1H), 8.25 (d, *J* = 3.7 Hz, 1H), 8.14 (dt, *J* = 8.3, 2.1 Hz, 1H), 8.06-8.02 (m, 1H), 7.99 (d, *J* = 8.5 Hz, 2H), 7.85 (d, *J* = 8.5 Hz, 2H), 7.71 (td, *J* = 8.1, 5.5 Hz, 1H), 7.67-7.60 (m, 1H), 7.08-7.00 (m, 1H). ¹³C-NMR (100 MHz, DMSO-*d*₆): *δ* 162.32 (¹*J*_{CF}= 250.30 Hz), 142.23, 141.02, 135.31, 134.23, 133.41 (2C), 133.11 (³*J*_{CF} = 8.37 Hz), 132.02, 131.55, 130.03 (2C), 128.12, 124.02 (⁴*J*_{CF}= 3.2 Hz), 123.19, 122.98, 119.12, 114.97 (²*J*_{CF}= 25.18 Hz), 111.37, 107.78. HRMS (ESI): m/z calcd. for C₂₀H₁₃FN₃O₂S⁺ [M+H]⁺: 378.0707, found 378.0698.

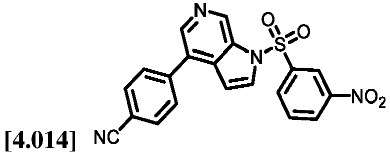

### 4-(1-((3-Nitrophenyl)sulfonyl)-1H-pyrrolo[2,3-c]pyridin-4-yl)benzonitrile (E13)

Following the general method EC, synthesized with **EI1** (0.164 g, 0.75 mmol), 3-nitrobenzenesulfonyl chloride (0.183 g, 0.825 mmol), DMAP (0.028 g, 0.225 mmol) and triethylamine (0.21 mL, 1.5 mmol) in anhydrous dichloromethane (4 mL) to give **E13** (0.155 g, 51.2% yield) as a white solid after chromatographic purification (PE/EA = 10/1), mp: 178.0-179.0 °C. ¹H-NMR (400 MHz, DMSO-*d*₆): *δ* 9.36 (s, 1H), 8.80 (t, *J* = 2.0 Hz, 1H), 8.67-8.62 (m, 1H), 8.59 (s, 1H), 8.55 (dd, *J* = 8.2, 1.4 Hz, 1H), 8.38 (d, *J* = 3.7 Hz, 1H), 8.00 (d, *J* = 8.4 Hz, 2H), 7.94 (t, *J* = 8.1 Hz, 1H), 7.86 (d, *J* = 8.4 Hz, 2H), 7.07 (d, *J* = 3.7 Hz, 1H). ¹³C-NMR (100 MHz, DMSO-*d*₆): *δ* 148.83, 142.43, 140.94, 138.39, 135.23, 134.37, 133.43 (2C), 132.71, 132.09, 131.61, 130.25, 130.03 (2C), 128.18, 122.45, 119.11, 116.87, 111.40, 108.19. HRMS (ESI): m/z calcd. for C₂₀H₁₃N₄O₄S⁺ [M+H]⁺: 405.0652, found 405.0648.

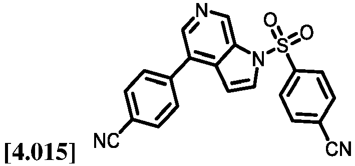

### 4-((4-(4-Cyanophenyl)-1H-pyrrolo[2,3-c]pyridin-1-yl)sulfonyl)benzonitrile (E14)

Following the general method EC, synthesized with **EI1** (0.164 g, 0.75 mmol), 4-cynobenzenesulfonyl chloride (0.166 g, 0.825 mmol), DMAP (0.028 g, 0.225 mmol) and triethylamine (0.21 mL, 1.5 mmol) in anhydrous dichloromethane (4 mL) to give **E14** (0.193 g, 67.2% yield) as a white solid after chromatographic purification (PE/EA = 10/1), mp: 206.0-207.0 °C. ¹H-NMR (400 MHz, DMSO-*d*₆): *δ* 9.33 (s, 1H), 8.59 (s, 1H), 8.38 (d, *J =* 8.6 Hz, 2H), 8.27 (d, *J* = 3.7 Hz, 1H), 8.14 (d, *J* = 8.6 Hz, 2H), 8.00 (d, *J* = 8.2 Hz, 2H), 7.86 (d, *J* = 8.2 Hz, 2H), 7.06 (d, *J =* 3.7 Hz, 1H). ¹³C-NMR (100 MHz, DMSO-*d*₆): *δ* 142.34, 140.90, 140.71, 135.19, 134.69 (2C), 134.39, 133.43 (2C), 132.00, 131.55, 130.04 (2C), 128.29 (2C), 128.22, 119.10, 118.01, 117.58, 111.42, 108.15. HRMS (ESI): m/z calcd. for C₂₁H₁₃N₄O₂S⁺ [M+H]⁺: 385.0754, found 385.0738.

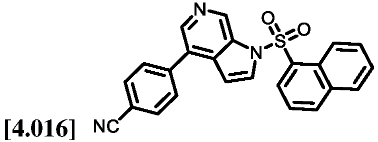

### 4-(1-(Naphthalen-1-ylsulfonyl)-1H-pyrrolo[2,3-c]pyridin-4-yl)benzonitrile (E15)

Following the general method ED, synthesized with **EI1** (0.164 g, 0.75 mmol), naphthalen-1-ylsulfonyl chloride (0.187 g, 0.825mmol), and NaH (0.020 g, 0.825 mmol) in anhydrous DMF (4 mL) to give **E15** (0.163 g, 53.2% yield) as a white solid after chromatographic purification (PE/EA = 20/1), mp: 188.0-189.0 °C. ¹H-NMR (400 MHz, DMSO-*d*₆): *δ* 9.14 (s, 1H), 8.71 (dd, *J* = 17.3, 8.1 Hz, 2H), 8.55 (d, *J* = 3.7 Hz, 1H), 8.49 (s, 1H), 8.39 (d, *J* = 8.2 Hz, 1H), 8.12 (d, *J* = 8.1 Hz, 1H), 7.96 (dd, *J* = 8.2, 1.4 Hz, 2H), 7.84-7.74 (m, 4H), 7.68 (t, *J* = 7.5 Hz, 1H), 6.99-6.94 (m, 1H). ¹³C-NMR (100 MHz, DMSO-*d*₆): *δ* 141.85, 141.03, 137.47, 134.89, 134.32, 133.97, 133.36 (2C), 132.56, 131.95, 131.94, 131.21, 130.20, 130.01 (2C), 128.12, 128.09, 127.40, 125.45, 123.45, 119.12, 116,87, 111.31, 106.32. HRMS (ESI): m/z calcd. for C₂₄H₁₆N₃O₂S⁺ [M+H]⁺: 410.0958, found 410.0949.

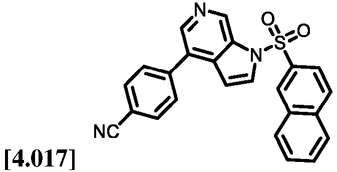

### 4-(1-(Naphthalen-2-ylsulfonyl)-1H-pyrrolo[2,3-c]pyridin-4-yl)benzonitrile (E16)

Following the general method ED, synthesized with **EI1** (0.164 g, 0.75 mmol), naphthalen-2-ylsulfonyl chloride (0.187 g, 0.825mmol), and NaH (0.020 g, 0.825 mmol) in anhydrous DMF (4 mL) to give **E16** (0.157 g, 51.2% yield) as a white solid after chromatographic purification (PE/EA = 20/1), mp: 176.0-177.0 °C. ¹H-NMR (400 MHz, DMSO-*d*₆): *δ* 9.39 (s, 1H), 9.04 (d, *J* = 1.6 Hz, 1H), 8.52 (s, 1H), 8.28 (t, *J =* 5.8 Hz, 2H), 8.14 (d, *J =* 8.9 Hz, 1H), 8.05-7.99 (m, 2H), 7.96 (d, *J =* 8.3 Hz, 2H), 7.82 (d, *J =* 8.4 Hz, 2H), 7.74 (dd, *J* = 12.8, 4.5 Hz, 2H), 7.00 (d, *J* = 3.7 Hz, 1H). ¹³C-NMR (100 MHz, DMSO-*d*₆): *δ* 141.99, 141.08, 135.53, 135.28, 134.13, 133.94, 133.38 (2C), 132.07, 132.02, 131.53, 130.91, 130.70, 130.21, 129.99 (2C), 129.85, 128.76, 128.46, 128.06, 121.73, 119.12, 111.31, 107.33. HRMS (ESI): m/z calcd. for C₂₄H₁₆N₃O₂S⁺ [M+H]⁺: 410.0958, found 410.0940.

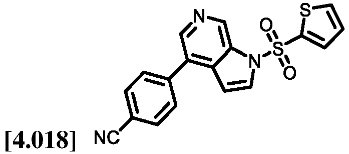

### 4-(1-(Thiophen-2-ylsulfonyl)-1H-pyrrolo[2,3-c]pyridin-4-yl)benzonitrile (E17)

Following the general method ED, synthesized with **EI1** (0.164 g, 0.75 mmol), thiophen-2-ylsulfonyl chloride (0.151 g, 0.825mmol), and NaH (0.020 g, 0.825 mmol) in anhydrous DMF (4 mL) to give **E17** (0.197 g, 72.1% yield) as a white solid after chromatographic purification (PE/EA = 20/1), mp: 161.0-162.0 °C. ¹H-NMR (400 MHz, DMSO-*d*₆): *δ* 9.28 (s, 1H), 8.59 (s, 1H), 8.15 (dt, *J* = 7.0, 3.8 Hz, 3H), 8.00 (d, *J* = 8.4 Hz, 2H), 7.87 (d, *J* = 8.4 Hz, 2H), 7.24 (dd, *J* = 4.8, 4.0 Hz, 1H), 7.03 (d, *J* = 3.7 Hz, 1H). ¹³C-NMR (100 MHz, DMSO-*d*₆): *δ* 142.29, 141.01, 137.99, 136.35, 135.20, 135.09, 134.29, 133.43 (2C), 131.83, 131.46, 130.06 (2C), 129.27, 128.20, 119.13, 111.39, 107.90. HRMS (ESI): m/z calcd. for C₁₈H₁₂N₃O₂S₂⁺ [M+H]⁺: 366.0365, found 366.0348.

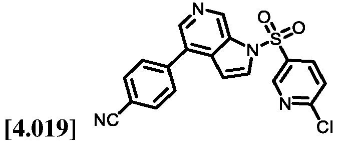

### 4-(1-((6-Chloropyridin-3-yl)sulfonyl)-1H-pyrrolo[2,3-c]pyridin-4-yl)benzonitrile (E18)

Following the general method ED, synthesized with **EI1** (0.164 g, 0.75 mmol), 6-chloropyridine-3-sulfonyl chloride (0.175 g, 0.825mmol), and NaH (0.020 g, 0.825 mmol) in anhydrous DMF (4 mL) to give **E18** (0.195 g, 66.1% yield) as a white solid after chromatographic purification (PE/EA = 20/1), mp: 188.0-189.0 °C. ¹H-NMR (400 MHz, DMSO-*d*₆): *δ* 9.36 (s, 1H), 9.27 (d, *J* = 2.6 Hz, 1H), 8.63 (dd, *J* = 8.6, 2.7 Hz, 1H), 8.59 (s, 1H), 8.25 (d, *J* = 3.7 Hz, 1H), 8.00 (d, *J =* 8.4 Hz, 2H), 7.86 (d, *J =* 8.4 Hz, 2H), 7.81 (d, *J =* 8.5 Hz, 1H), 7.06 (d, *J =* 3.7 Hz, 1H). ¹³C-NMR (100 MHz, DMSO-*d*₆): *δ* 156.88, 149.06, 142.47, 140.98, 138.96, 135.29, 134.38, 133.44 (2C), 133.33, 131.77, 131.55, 130.05 (2C), 128.18, 126.39, 119.12, 111.39, 108.26. HRMS (ESI): m/z calcd. for C₁₉H₁₂ClN₄O₂S⁺ [M+H]⁺: 395.0364, found 395.0357.

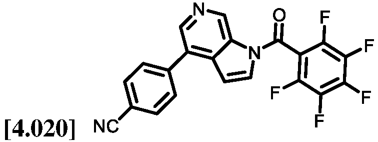

### 4-(1-(Perfluorobenzoyl)-1H-pyrrolo[2,3-c]pyridin-4-yl)benzonitrile (E19)

Following the general method EB, synthesized with **EI1** (0.164 g, 0.75 mmol), 2,3,4,5,6-pentafluorobenzoyl chloride (0.190 g, 0.825 mmol), DMAP (0.028 g, 0.225 mmol) and triethylamine (0.21 mL, 1.5 mmol) in anhydrous dichloromethane (4 mL) to give **E19** (0.201 g, 65.2% yield) as a white solid after chromatographic purification (PE/EA = 10/1), mp: 165.0-166.0 °C. ¹H-NMR (400 MHz, DMSO-*d*₆): *δ* 9.64 (s, 1H), 8.71 (s, 1H), 8.09-8.00 (m, 3H), 7.93 (d, *J* = 8.3 Hz, 2H), 7.07 (d, *J* = 3.8 Hz, 1H). ¹³C-NMR (100 MHz, DMSO-*d*₆): *δ* 183.73, 156.88, 145,27, 144.00, 141.01, 137.37, 137.03, 135.03, 134.71, 134.36, 133.49 (2C), 132.40, 132.28, 130.16 (2C), 128.27, 119.14, 116.87, 111.43, 109.03. HRMS (ESI): m/z calcd. for C₂₁H₉F₅N₃O⁺ [M+H]⁺: 414.0660, found 414.0644.

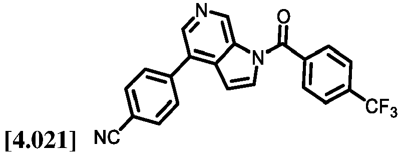

### 4-(1-(4-(Trifluoromethyl)benzoyl)-1H-pyrrolo[2,3-c]pyridin-4-yl)benzonitrile (E20)

Following the general method EB, synthesized with **EI1** (0.164 g, 0.75 mmol), 4-(trifluoromethyl)benzoyl chloride (0.172 g, 0.825 mmol), DMAP (0.028 g, 0.225 mmol) and triethylamine (0.21 mL, 1.5 mmol) in anhydrous dichloromethane (4 mL) to give **E20** (0.194 g, 66.5% yield) as a white solid after chromatographic purification (PE/EA = 10/1), mp: 152.0-153.0 °C. ¹H-NMR (400 MHz, DMSO-*d*₆): *δ* 9.57 (s, 1H), 8.64 (s, 1H), 8.03 (q, *J* = 8.3 Hz, 6H), 7.92 (dd, *J* = 8.3, 1.8 Hz, 2H), 7.79 (d, *J* = 3.7 Hz, 1H), 6.92 (d, *J* = 3.6 Hz, 1H). ¹³C-NMR (100 MHz, DMSO-*d*₆): *δ* 167.43, 142.61, 141.44, 137.81, 137.49, 134.26, 133.51 (2C), 133.35, 133.02, 132.46 (²*J*_{CF}= 31.92 Hz), 130.63, 129.99 (2C), 126.29 (⁴*J*_{CF}= 3.76 Hz, 2C), 126.09 (³*J*_{CF} = 3.93 Hz, 2C), 124.21 (¹*J*_{CF} = 272.94 Hz), 119.18, 111.27, 106.68. HRMS (ESI): m/z calcd. for C₂₂H₁₃F₃N₃O⁺ [M+H]⁺: 392.1005, found 392.0989.

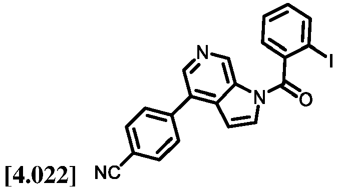

### 4-(1-(2-iodobenzoyl)-1H-pyrrolo[2,3-c]pyridin-4-yl)benzonitrile (E21)

Following the general method EB, synthesized with **EI1** (0.164 g, 0.75 mmol), 2-iodobenzoyl chloride (0.220 g, 0.825 mmol), DMAP (0.028 g, 0.225 mmol) and triethylamine (0.21 mL, 1.5 mmol) in anhydrous dichloromethane (4 mL) to give **E21** (0.192 g, 57.3% yield) as a white solid after chromatographic purification (PE/EA = 10/1), mp: 189.0-190.0 °C. ¹H-NMR (400 MHz, DMSO-*d*₆): *δ* 9.46 (s, 1H), 8.65 (s, 1H), 8.04 (dd, *J =* 10.3, 8.2 Hz, 3H), 7.91 (d, *J =* 8.2 Hz, 2H), 7.74 (dd, J = 7.6, 1.5 Hz, 1H), 7.65 (td, *J =* 7.5, 0.9 Hz, 1H), 7.42 (ddd, *J* = 15.5, 6.5, 2.5 Hz, 2H), 6.92 (d, *J* = 3.8 Hz, 1H). ¹³C-NMR (100 MHz, DMSO-*d*₆): *δ* 168.31, 142.93, 141.30, 140.06, 139.63, 137.39, 134.59, 133.50 (2C), 132.99, 132.46, 132.45, 130.05 (2C), 129.25, 129.15 ), 127.96, 119.16, 111.32, 107.55, 93.93. HRMS (ESI): m/z calcd. for C₂₁H₁₃IN₃O⁺ [M+H]⁺: 450.0098, found 450.0095.

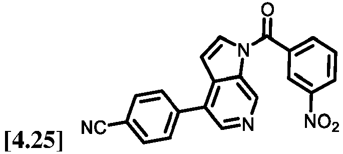

### 4-(1-(3-Nitrobenzoyl)-1H-pyrrolo[2,3-c]pyridin-4-yl)benzonitrile (E22)

Following the general method EB, synthesized with **EI1** (0.164 g, 0.75 mmol), 3-nitrobenzoyl chloride (0.153 g, 0.825 mmol), DMAP (0.028 g, 0.225 mmol) and triethylamine (0.21 mL, 1.5 mmol) in anhydrous dichloromethane (4 mL) to give **E22** (0.188 g, 68.3% yield) as a white solid after chromatographic purification (PE/EA = 10/1), mp: 197.0-198.0 °C. ¹H-NMR (400 MHz, DMSO-*d*₆): *δ* 9.59 (s, 1H), 8.64 (s, 1H), 8.63-8.62 (m, 1H), 8.55 (dd, *J =* 8.3, 1.4 Hz, 1H), 8.27 (d, *J* = 7.8 Hz, 1H), 8.04 (d, *J* = 8.4 Hz, 2H), 7.95-7.90 (m, 3H), 7.86 (d, *J* = 3.8 Hz, 1H), 6.95 (d, *J* = 3.7 Hz, 1H). ¹³C-NMR (100 MHz, DMSO-*d*₆): δ 166.60, 148.14, 141.45, 137.83, 135.98, 135.06, 134.32, 133.52 (2C), 133.43, 133.10, 131.11, 130.02 (2C), 127.80, 127.28, 124.84, 119.18, 111.28, 106.78, 100.00. HRMS (ESI): m/z calcd. for C₂₁H₁₃N₄O₃⁺ [M+H]⁺: 369.0982, found 369.0974.

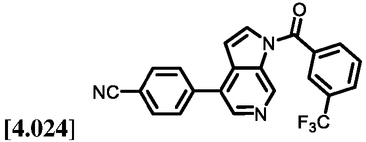

### 4-(1-(3-(Trifluoromethyl)benzoyl)-1H-pyrrolo[2,3-c]pyridin-4-yl)benzonitrile (E23)

Following the general method EB, synthesized with **EI1** (0.164 g, 0.75 mmol), 3-(trifluoromethyl)benzoyl chloride (0.172 g, 0.825 mmol), DMAP (0.028 g, 0.225 mmol) and triethylamine (0.21 mL, 1.5 mmol) in anhydrous dichloromethane (4 mL) to give **E23** (0.167 g, 57.3% yield) as a white solid after chromatographic purification (PE/EA = 10/1), mp: 135.0-136.0 °C. ¹H-NMR (400 MHz, DMSO-*d*₆): *δ* 9.55 (s, 1H), 8.63 (s, 1H), 8.19 (s, 1H), 8.12 (dd, *J* = 13.6, 7.9 Hz, 2H), 8.03 (d, *J* = 8.1 Hz, 2H), 7.92 (d, *J* = 8.2 Hz, 2H), 7.89 (d, *J* = 7.9 Hz, 1H), 7.79 (d, *J* = 3.7 Hz, 1H), 6.93 (d, *J* = 3.8 Hz, 1H). ¹³C-NMR (100 MHz, DMSO-*d*₆): *δ* 167.21, 142.51, 141.46, 137.77, 134.74, 134.24, 133.74, 133.50 (2C), 133.32, 133.07, 130.59, 129.98 (2C), 129.97 (²*J*_{CF}= 32.38 Hz), 129.42 (⁴*J*_{CF}= 3.55 Hz), 127.76, 126.54 (³*J*_{CF}= 4.02 Hz), 124.19 (¹*J*_{CF}= 272.68 Hz), 119.17, 111.26, 106.65. HRMS (ESI): m/z calcd. for C₂₂H₁₃F₃N₃O⁺ [M+H]⁺: 392.1005, found 392.1009.

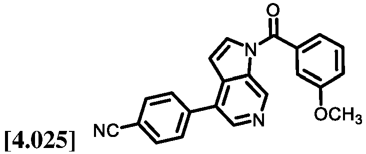

### 4-(1-(3-Methoxybenzoyl)-1H-pyrrolo[2,3-c]pyridin-4-yl)benzonitrile (E24)

Following the general method EB, synthesized with **EI1** (0.164 g, 0.75 mmol), 3-methoxybenzoyl chloride (0.141 g, 0.825 mmol), DMAP (0.028 g, 0.225 mmol) and triethylamine (0.21 mL, 1.5 mmol) in anhydrous dichloromethane (4 mL) to give **E24** (0.178 g, 67.3% yield) as a white solid after chromatographic purification (PE/EA = 10/1), mp: 174.0-175.0 °C. ¹H-NMR (400 MHz, DMSO-*d*₆): *δ* 9.52 (s, 1H), 8.61 (s, 1H), 8.03 (d, *J =* 8.3 Hz, 2H), 7.92 (d, *J =* 8.3 Hz, 2H), 7.81 (d, *J=* 3.7 Hz, 1H), 7.55 (t, *J =* 8.1 Hz, 1H), 7.39-7.36 (m, 2H), 7.30 (dd, *J* = 7.9, 2.2 Hz, 1H), 6.90 (d, *J =* 3.8 Hz, 1H), 3.84 (s, 3H). ¹³C-NMR (100 MHz, DMSO-*d*₆): *δ* 168.18, 159.69, 142.33, 141.53, 138.38, 137.74, 134.79, 133.50 (2C), 133.41, 133.03, 130.60, 129.98 (2C), 127.74, 121.87, 118.99, 114.82, 112.68, 111.24, 106.27, 55.97. HRMS (ESI): m/z calcd. for C₂₂H₁₆N₃O₂⁺ [M+H]⁺: 354.1237, found 354.1241.

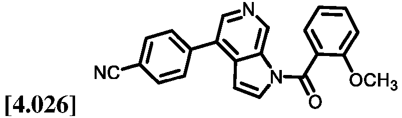

### 4-(1-(2-Methoxybenzoyl)-1H-pyrrolo[2,3-c]pyridin-4-yl)benzonitrile (E25)

Following the general method EB, synthesized with **EI1** (0.164 g, 0.75 mmol), 2-methoxybenzoyl chloride (0.141 g, 0.825 mmol), DMAP (0.028 g, 0.225 mmol) and triethylamine (0.21 mL, 1.5 mmol) in anhydrous dichloromethane (4 mL) to give **E25** (0.176 g, 67.0% yield) as a white solid after chromatographic purification (PE/EA = 10/1), mp: 192.0-193.0 °C. ¹H-NMR (400 MHz, DMSO-*d*₆): *δ* 9.52 (s, 1H), 8.62 (s, 1H), 8.03 (d, *J* = 8.2 Hz, 2H), 7.91 (d, *J* = 8.2 Hz, 2H), 7.65 - 7.59 (m, 2H), 7.51 (d, *J* = 3.7 Hz, 1H), 7.29 (d, *J* = 8.4 Hz, 1H), 7.18 (t, *J* = 7.4 Hz, 1H), 6.85 (d, *J* = 3.7 Hz, 1H), 3.77 (s, 3H). ¹³C-NMR (100 MHz, DMSO-*d*₆): *δ* 167.16, 156.42, 142.55, 141.46, 137.38, 133.47 (2C), 132.96, 132.39, 131.08, 130.01 (2C), 129.65, 127.79, 123.38, 121.44, 120.47, 119.19, 112.79, 111.24, 106.63, 56.32. HRMS (ESI): m/z calcd. for C₂₂H₁₆N₃O₂⁺ [M+H]⁺: 354.1237, found 354.1241.

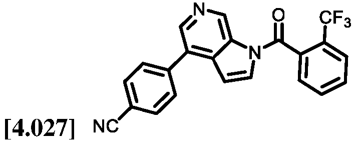

### 4-(1-(2-(Trifluoromethyl)benzoyl)-1H-pyrrolo[2,3-c]pyridin-4-yl)benzonitrile (E26)

Following the general method EB, synthesized with **EI1** (0.164 g, 0.75 mmol), 2-(trifluoromethyl)benzoyl chloride (0.172 g, 0.825 mmol), DMAP (0.028 g, 0.225 mmol) and triethylamine (0.21 mL, 1.5 mmol) in anhydrous dichloromethane (4 mL) to give **E26** (0.153 g, 52.0% yield) as a white solid after chromatographic purification (PE/EA = 10/1), mp: 189.0-190.0 °C. ¹H-NMR (400 MHz, DMSO-*d*₆): *δ* 9.52 (s, 1H), 8.66 (s, 1H), 8.03 (dd, *J=* 5.5, 2.8 Hz, 3H), 7.95-7.89 (m, 5H), 7.52 (s, 1H), 6.89 (d, *J* = 3.7 Hz, 1H). ¹³C-NMR (100 MHz, DMSO-*d*₆): *δ* 166.41, 143.03, 141.26, 137.44, 134.58, 133.66, 133.49 (2C), 132.74, 132.60, 132.29, 132.17, 130.06 (2C), 129.36, 128.07, 127.54 (³*J*_{CF}= 4.58 Hz), 126.69 (²*J*_{CF}= 31.68 Hz), 123.98 (¹*J*_{CF}= 273.76 Hz), 119.15, 111.35, 107.43. HRMS (ESI): m/z calcd. for C₂₂H₁₃F₃N₃O⁺ [M+H]⁺: 392.1005, found 392.0993.

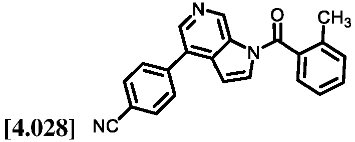

### 4-(1-(2-Methylbenzoyl)-1H-pyrrolo[2,3-c]pyridin-4-yl)benzonitrile (E27)

Following the general method EB, synthesized with **EI1** (0.164 g, 0.75 mmol), 2-methylbenzoyl chloride (0.128g, 0.825 mmol), DMAP (0.028 g, 0.225 mmol) and triethylamine (0.21 mL, 1.5 mmol) in anhydrous dichloromethane (4 mL) to give **E27** (0.159 g, 63.0% yield) as a white solid after chromatographic purification (PE/EA = 10/1), mp: 186.0-187.0 °C. ¹H-NMR (400 MHz, DMSO-*d*₆): *δ* 9.47 (s, 1H), 8.62 (s, 1H), 8.03 (d, *J* = 8.2 Hz, 2H), 7.90 (d, *J* = 8.2 Hz, 2H), 7.57 (t, *J* = 7.9 Hz, 2H), 7.48 (t, *J* = 6.4 Hz, 2H), 7.43 (d, *J* = 7.6 Hz, 1H), 6.88 (d, *J* = 3.7 Hz, 1H), 2.30 (s, 3H). ¹³C-NMR (100 MHz, DMSO-*d*₆): *δ* 168.78, 142.55, 141.44, 137.54, 136.15, 134.44, 133.89, 133.49 (2C), 132.62, 132.56, 131.73, 131.46, 130.01 (2C), 128.34, 127.87, 126.59, 119.17, 111.28, 106.93, 19.34. HRMS (ESI): m/z calcd. for C₂₂H₁₆N₃O⁺ [M+H]⁺: 338.1288, found 338.1286.

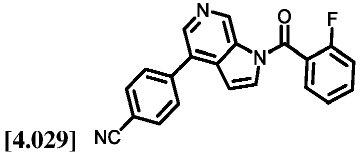

### 4-(1-(2-Fluorobenzoyl)-1H-pyrrolo[2,3-c]pyridin-4-yl)benzonitrile (E28)

Following the general method EB, synthesized with **EI1** (0.164 g, 0.75 mmol), 2-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E28** (0.133 g, 52.0% yield) as a white solid after chromatographic purification (PE/EA = 10/1), mp: 176.0-177.0 °C. ¹H-NMR (400 MHz, DMSO-*d*₆): *δ* 9.56 (s, 1H), 8.64 (s, 1H), 8.03 (d, *J =* 8.3 Hz, 2H), 7.91 (d, *J =* 8.3 Hz, 2H), 7.85-7.80 (m, 1H), 7.79-7.74 (m, 1H), 7.69 (dd, J = 3.6, 2.2 Hz, 1H), 7.53-7.49 (m, 1H), 7.47 (d, *J* = 7.6 Hz, 1H), 6.91 (d, *J =* 3.8 Hz, 1H). ¹³C-NMR (100 MHz, DMSO-*d*₆): *δ* 164.39, 159.08 (¹*J*_{CF}= 249.58 Hz), 142.88, 141.32, 137.48, 134.89 (³*J*_{CF}= 8.44 Hz), 134.49, 133.49 (2C), 132.78, 132.61, 132.36, 131.00, 130.03 (2C), 127.92, 124.92 (⁴*J*_{CF}= 3.66), 119.17, 117.17 (²*J*_{CF}= 20.69 Hz), 111.31, 107.25. HRMS (ESI): m/z calcd. for C₂₁H₁₃FN₃O⁺ [M+H]⁺: 342.1037, found 342.1037.

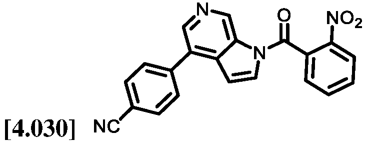

### 4-(1-(2-Nitrobenzoyl)-1H-pyrrolo[2,3-c]pyridin-4-yl)benzonitrile (E29)

Following the general method EB, synthesized with **EI1** (0.164 g, 0.75 mmol), 2-nitrobenzoyl chloride (0.153g, 0.825 mmol), DMAP (0.028 g, 0.225 mmol) and triethylamine (0.21 mL, 1.5 mmol) in anhydrous dichloromethane (4 mL) to give **E29** (0.155 g, 56.0% yield) as a white solid after chromatographic purification (PE/EA = 10/1), mp: 202.0-203.0 °C. ¹H-NMR (400 MHz, DMSO-*d*₆): *δ* 9.59 (s, 1H), 8.66 (s, 1H), 8.43 (d, *J* = 8.3 Hz, 1H), 8.08 - 8.00 (m, 4H), 7.99 (dd, *J* = 8.6, 1.6 Hz, 1H), 7.92 (d, *J* = 8.3 Hz, 2H), 7.64 (s, 1H), 6.89 (d, *J =* 3.7 Hz, 1H). ¹³C-NMR (100 MHz, DMSO-*d*₆): *δ* 165.64, 145.64, 142.98, 141.32, 137.45, 136.08, 134.47, 133.49 (2C), 132.92, 132.69, 132.28, 130.07 (2C), 129.84, 128.03, 125.85, 124.19, 119.17, 111.32, 107.51. HRMS (ESI): m/z calcd. for C₂₁H₁₃N₄O₃⁺ [M+H]⁺: 369.0982, found 369.0964.

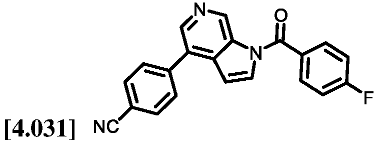

### 4-(1-(4-Fluorobenzoyl)-1H-pyrrolo[2,3-c]pyridin-4-yl)benzonitrile (E30)

Following the general method EB, synthesized with **EI1** (0.164 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E30** (0.131 g, 51.0% yield) as a white solid after chromatographic purification (PE/EA = 10/1), mp: 227.0-228.0 °C. ¹H-NMR (400 MHz, CDCl₃): *δ* 9.70 (s, 1H), 8.60 (s, 1H), 7.89-7.82 (m, 4H), 7.76 (d, *J* = 8.3 Hz, 2H), 7.66 (d, *J* = 3.7 Hz, 1H), 7.29 (t, *J* = 8.5 Hz, 2H), 6.82 (d, *J* = 3.7 Hz, 1H). ¹³C-NMR (100 MHz, CDCl₃): *δ* 166.68, 165.02 (¹*J*_{CF} = 256.18), 148.41, 140.98, 140.54, 136.92, 135.01, 132.95 (2C), 132.41, 132.19 (³*J*_{CF} = 9.37 Hz, 2C), 129.39 (2C), 128.96, 118.46, 116.46 (²*J*_{CF} = 22.15 Hz, 2C), 112.32, 106.25 (2C). HRMS (ESI): m/z calcd. for C₂₁H₁₃FN₃O⁺ [M+H]⁺: 342.1037, found 342.1034.

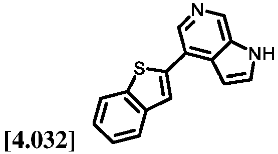

### 4-(Benzo[b]thiophen-2-yl)-1H-pyrrolo[2,3-c]pyridine (EI9)

Following the general method EA, synthesized with 4-bromo-1*H*-pyrrolo[2,3-c]pyridine (99.0 mg, 0.5 mmol), benzo[b]thiophen-2-ylboronic acid (81.1 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI9** (81.4 mg, 65.4% yield) as a yellow solid after chromatographic purification (PE/EA = 1/1). ¹H-NMR (400 MHz, DMSO-*d*₆): *δ* 11.96 (s, 1H), 8.79 (s, 1H), 8.49 (s, 1H), 8.05-8.01 (m, 1H), 8.00 (s, 1H), 7.94 (dd, *J* = 7.1, 1.4 Hz, 1H), 7.83-7.78 (m, 1H), 7.47-7.36 (m, 2H), 7.01 (d, *J* = 2.8 Hz, 1H).

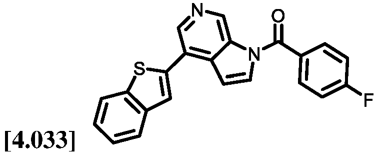

### (4-(Benzo[b]thiophen-2-yl)-1H-pyrrolo[2,3-c]pyridin-1-yl)(4-fluorophenyl)methanone (E31)

Following the general method EB, synthesized with EI9 (0.188 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E31** (0.142 g, 51.0% yield) as a white solid after chromatographic purification (PE/EA = 10/1), mp: 159.0-160.0 °C. ¹H-NMR (400 MHz, DMSO-*d*₆): *δ* 9.57 (s, 1H), 8.85 (d, *J* = 25.0 Hz, 1H), 8.15 (d, *J* = 9.6 Hz, 2H), 8.05 (ddd, *J* = 11.9, 7.0, 3.8 Hz, 3H), 7.95 (d, *J* = 3.8 Hz, 1H), 7.57 (t, *J* = 8.8 Hz, 2H), 7.41 (dd, *J* = 12.3, 5.4 Hz, 2H), 7.34 (d, *J* = 3.8 Hz, 1H). ¹³C-NMR (100 MHz, DMSO-*d*₆): *δ* 165.75, 165.00 (¹*J*_{CF}= 281.893 Hz), 139.44, 138.40, 132.43, 132.23, 131.89 (³*J*_{CF}= 9.27 Hz, 2C), 131.51, 131.42, 124.46, 124.34, 123.53, 122.71, 121.81, 115.40 (²*J*_{CF}= 22.23 Hz, 2C), 115.10 (2C), 114.88 (2C), 105.57. HRMS (ESI): m/z calcd. for C₂₂H₁₄FN₂OS⁺ [M+H]⁺: 373.0805, found 373.0808.

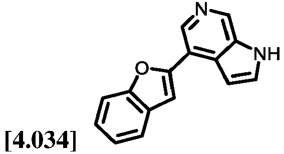

### 4-(Benzofuran-2-yl)-1H-pyrrolo[2,3-c]pyridine (EI10)

Following the general method EA, synthesized with 4-bromo-1H-pyrrolo[2,3-c]pyridine (99.0 mg, 0.5 mmol), benzofuran-2-ylboronic acid (89.1 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give EI10 (74.1 mg, 63.3% yield) as a yellow solid after chromatographic purification (PE/EA = 1/1). ¹H-NMR (400 MHz, DMSO-*d*₆): *δ* 11.94 (s, 1H), 8.79 (s, 1H), 8.73 (s, 1H), 7.81 (t, *J* = 2.7 Hz, 1H), 7.73-7.68 (m, 2H), 7.60 (d, *J* = 0.5 Hz, 1H), 7.38-7.33 (m, 1H), 7.30 (dt, *J* = 7.1, 3.6 Hz, 1H), 7.08 (s, 1H).

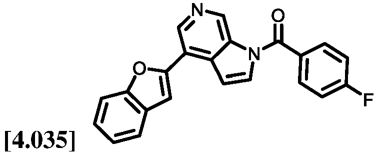

### (4-(Benzofuran-2-yl)-1H-pyrrolo[2,3-c]pyridin-1-yl)(4-fluorophenyl)methanone (E32)

Following the general method EB, synthesized with **EI10** (0.176 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give E32 (0.144 g, 54.0% yield) as a white solid after chromatographic purification (PE/EA = 10/1), mp: 206.0-207.0 °C. ¹H-NMR (400 MHz, DMSO-*d*₆): *δ* 9.48 (s, 1H), 9.04 (s, 1H), 7.95 (dd, *J* = 8.3, 5.6 Hz, 2H), 7.88 (d, *J* = 3.7 Hz, 1H), 7.75 (d, *J* = 9.2 Hz, 3H), 7.50 (t, *J* = 8.7 Hz, 2H), 7.43-7.37 (m, 2H), 7.33 (t, *J* = 7.5 Hz, 1H). ¹³C-NMR (100 MHz, DMSO-*d*₆): *δ* 167.49, 164.98 (¹*J*_{CF}= 251.066 Hz), 154.87, 152.91, 140.41, 137.62, 133.42 (³*J*_{CF} = 9.25 Hz, 2C), 133.05, 132.95, 131.98, 130.07 (⁴*J*_{CF} = 3.19 Hz), 128.94, 125.64, 124.00, 121.90, 118.58, 116.51 (²*J*_{CF}= 22.42 Hz, 2C), 111.85, 107.47, 105.49. HRMS (ESI): m/z calcd. for C₂₂H₁₄FN₂O₂⁺ [M+H]⁺: 357.1034, found 357.1033.

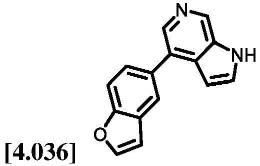

### 4-(Benzofuran-5-yl)-1H-pyrrolo[2,3-c]pyridine (EI11)

Following the general method EA, synthesized with 4-bromo-1H-pyrrolo[2,3-c]pyridine (99.0 mg, 0.5 mmol), benzofuran-5-yl boronic acid (89.2 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI11** (74.0 mg, 63.2% yield) as a yellow solid after chromatographic purification (PE/EA = 1/1). ¹H-NMR (400 MHz, DMSO-*d*₆): *δ* 11.78 (s, 1H), 8.76 (d, *J* = 4.1 Hz, 1H), 8.23 (d, *J* = 3.2 Hz, 1H), 8.06 (d, *J* = 2.1 Hz, 1H), 7.97 (s, 1H), 7.74 (d, *J* = 8.5 Hz, 1H), 7.69 (d, *J* = 2.6 Hz, 1H), 7.65 (dd, J = 8.5, 1.7 Hz, 1H), 7.08-7.03 (m, 1H), 6.65 (d, *J* = 2.7 Hz, 1H).

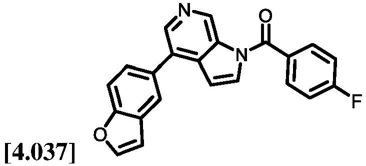

### (4-(Benzofuran-5-yl)-1H-pyrrolo[2,3-c]pyridin-1-yl)(4-fluorophenyl)methanone (E33)

Following the general method EB, synthesized with **EI11** (0.176 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E32** (0.149 g, 56.0% yield) as a white solid after chromatographic purification (PE/EA = 10/1), mp: 156.0-157.0 °C. ¹H-NMR (400 MHz, DMSO-*d*₆): *δ* 9.47 (s, 1H), 8.57 (s, 1H), 8.10 (d, *J* = 2.1 Hz, 1H), 7.97 (d, *J* = 1.5 Hz, 1H), 7.93 (dd, *J* = 8.6, 5.4 Hz, 2H), 7.79 (d, *J* = 8.5 Hz, 1H), 7.75 (d, *J* = 3.8 Hz, 1H), 7.62 (dd, *J* = 8.5, 1.7 Hz, 1H), 7.48 (t, *J* = 8.8 Hz, 2H), 7.12 - 7.05 (m, 1H), 6.90 (d, *J* = 3.7 Hz, 1H). ¹³C-NMR (100 MHz, DMSO-*d*₆): *δ* 167.49, 164.88 (¹*J*_{CF} = 250.99 Hz), 154.62, 147.43, 142.30, 136.53, 134.42, 133.08, 132.89, 132.77 (³*J*_{CF} = 6.85 Hz, 2C), 131.71, 130.22 (⁴*J*_{CF} = 2.85 Hz), 129.80, 128.55, 125.57, 121.85, 116.48 (²*J*_{CF}= 22.17 Hz, 2C), 112.40, 107.54, 106.75. HRMS (ESI): m/z calcd. for C₂₂H₁₄FN₂O₂⁺ [M+H]⁺: 357.1034, found 357.1025.

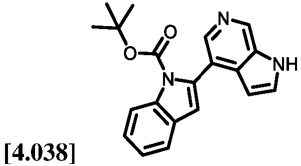

### Tert-butyl-2-(1H-pyrrolo[2,3-c]pyridin-4-yl)-1H-indole-1-carboxylate (EI12)

Following the general method EA, synthesized with 4-bromo-1*H*-pyrrolo[2,3-c]pyridine (99.0 mg, 0.5 mmol), (1-(tert-butoxycarbonyl)-1H-indol-2-yl)boronic acid (143.1 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI12** (97.0 mg, 58.2% yield) as a yellow solid after chromatographic purification (PE/EA = 1/1). ¹H-NMR (400 MHz, DMSO-*d*₆): *δ* 11.75 (s, 1H), 8.78 (s, 1H), 8.16 (t, *J* = 3.8 Hz, 2H), 7.66 (dd, *J* = 5.3, 2.5 Hz, 2H), 7.39-7.34 (m, 1H), 7.29 (td, *J=* 7.6, 1.0 Hz, 1H), 6.83 (s, 1H), 6.31 (d, *J* = 1.9 Hz, 1H), 0.99 (s, 9H).

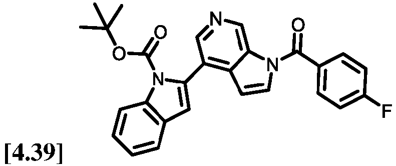

### Tert-butyl-2-(1-(4-fluorobenzoyl)-1H-pyrrolo[2,3-c]pyridin-4-yl)-1H-indole-1-carboxylate (EI12a)

Following the general method EB, synthesized with **EI12** (0.250 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **EI12a**(0.136 g, 40.0% yield) as a white solid after chromatographic purification (PE/EA = 10/1), mp: 125.4-126.0 °C ¹H-NMR (400 MHz, DMSO-*d*₆): *δ* 9.48 (s, 1H), 8.51 (s, 1H), 8.19 (d, *J* = 8.3 Hz, 1H), 7.94-7.88 (m, 2H), 7.75 (d, *J* = 3.7 Hz, 1H), 7.69 (d, *J* = 7.6 Hz, 1H), 7.48 (t, *J* = 8.8 Hz, 2H), 7.40 (t, *J* = 7.2 Hz, 1H), 7.32 (dd, *J* = 12.7, 5.0 Hz, 1H), 6.93 (s, 1H), 6.66 (d, *J* = 3.7 Hz, 1H), 1.13 (s, 9H).

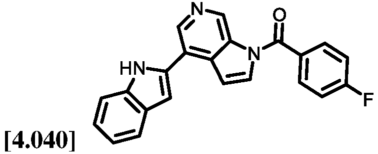

### (4-(1H-Indol-2-yl)-1H-pyrrolo[2,3-c]pyridin-1-yl)(4-fluorophenyl)methanone (E34)

To a solution of **EI12a** (0.228 g, 0.50 mmol) in in anhydrous CH₂Cl₂ (9.0 mL) was added dropwise trifluoroacetic acid (3 mL) at 0°C. The reaction mixture was subsequently warmed to room temperature and stirred for 1 h. After the reaction was complete as monitored by TLC, the reaction mixture was quenched with water, the pH was adjusted to 10, and extracted with CH₂Cl₂. The organic phases were combined and washed with saturated brine, dried over anhydrous sodium sulfate and filtered. The solvent was removed under reduced pressure. The residue was purified by silica gel column chromatography (PE/EA = 3/1) to give **E34** (0.149 g, 84% yield) as a white solid. mp: 188-189°C. ¹H-NMR (400 MHz, DMSO-*d*₆): *δ* 11.85 (s, 1H), 9.54 (s, 1H), 8.93 (s, 1H), 8.12 (t, *J* = 3.3 Hz, 1H), 8.00-7.93 (m, 2H), 7.66 (d, *J* = 7.9 Hz, 1H), 7.56-7.48 (m, 3H), 7.43 (d, *J* = 2.2 Hz, 1H), 7.22 (dd, *J* = 9.4, 4.6 Hz, 2H), 7.09 (t, *J* = 7.4 Hz, 1H). ¹³C-NMR (100 MHz, DMSO-*d*₆): *δ* 167.35, 165.20 (¹*J*_{CF} = 257.65 Hz), 137.83 (2C), 136.26, 134.90, 133.23 (³*J*_{CF} = 9.54 Hz, 2C), 132.46, 129.57, 128.93 (2C), 123.28, 122.58 (⁴*J*_{CF} = 2.34 Hz), 121.44, 121.11, 120.33, 116.61 (²*T*_{CF} = 22.45 Hz, 2C), 112.16, 107.51, 104.02. HRMS (ESI): m/z calcd. for C₂₂H₁₅FN₃O⁺ [M+H]⁺: 356.1194, found 356.1193.

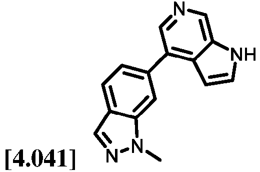

### 1-Methyl-6-(1iH-pyrrolo[2,3-c]pyridin-4-yl)-1H-indazole (EI13)

Following the general method EA, synthesized with 4-bromo-1*H*-pyrrolo[2,3-*c*]pyridine (99.0 mg, 0.5 mmol), (1-methyl-1*H*-indazol-6-yl)boronic acid (97.2 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI13** (81.0 mg, 65.2% yield) as a yellow solid after chromatographic purification (PE/EA = 1/1). ¹H-NMR (400 MHz, DMSO-*d*₆): *δ* 11.81 (s, 1H), 8.78 (s, 1H), 8.32 (s, 1H), 8.10 (s, 1H), 7.93-7.86 (m, 2H), 7.71 (t, *J* = 2.6 Hz, 1H), 7.50 (d, *J* = 8.3 Hz, 1H), 6.73 (s, 1H), 4.11 (s, 3H).

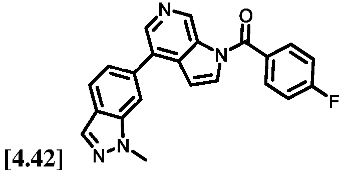

### (4-Fluorophenyl)(4-(1-methyl-1H-indazol-6-yl)-1H-pyrrolo[2,3-c]pyridin-1-yl)methanone (E35)

Following the general method EB, synthesized with **EI13** (0.186 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E35** (0.138 g, 50.0% yield) as a white solid after chromatographic purification (PE/EA = 10/1), mp: 183.0-184.0 °C. ¹H-NMR (400 MHz, DMSO-*d*₆): *δ* 9.50 (s, 1H), 8.65 (s, 1H), 8.14 (s, 1H), 7.97-7.92 (m, 4H), 7.78 (d, *J =* 3.7 Hz, 1H), 7.53-7.44 (m, 3H), 6.99 (d, *J* = 3.7 Hz, 1H), 4.13 (s, 3H). ¹³C-NMR (100 MHz, DMSO-*d*₆): *δ* 167.50, 165.62 (¹*J*_{CF} = 246.09 Hz), 142.52, 140.49, 136.82, 134.61, 132.93, 132.84 (2C), 132.58 (³*J*_{CF} = 9.61 Hz, 2C), 130.17 (⁴*J*_{CF} = 3.22 Hz), 123.55, 121.94, 121.85, 116.61, 116.39, 116.10 (²*J*_{CF} = 22.20 Hz, 2C), 110.08, 106.89, 35.98. HRMS (ESI): m/z calcd. for C₂₂H₁₆FN₄O⁺ [M+H]⁺: 371.1303, found 371.1298.

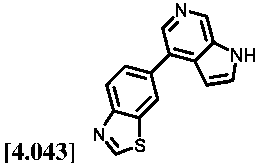

### 6-(1H-Pyrrolo[2,3-c]pyridin-4-yl)benzo[d]thiazole (EI14)

Following the general method EA, synthesized with 4-bromo-1*H*-pyrrolo[2,3-c]pyridine (99.0 mg, 0.5 mmol), benzo[d]thiazol-6-ylboronic acid (98.2 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI14** (82.0 mg, 65.0% yield) as a yellow solid after chromatographic purification (PE/EA = 1/1). ¹H-NMR (400 MHz, DMSO-*d*₆): *δ* 11.84 (s, 1H), 9.43 (d, *J* = 5.0 Hz, 1H), 8.79 (s, 1H), 8.52 (s, 1H), 8.30 (s, 1H), 8.22 (d, *J =* 8.1 Hz, 1H), 7.89 (dd, *J =* 8.5, 1.5 Hz, 1H), 7.72 (t, *J =* 2.6 Hz, 1H), 6.72 (s, 1H).

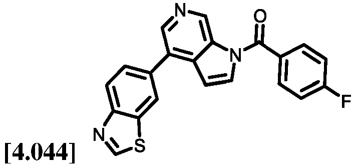

### (4-(Benzo[d]thiazol-6-yl)-1H-pyrrolo[2,3-c]pyridin-1-yl)(4-fluorophenyl)methanone (E36)

Following the general method EB, synthesized with **EI14** (0.188 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E36** (0.146 g, 52.0% yield) as a white solid after chromatographic purification (PE/EA = 10/1), mp: 187.0-188.0 °C. ¹H-NMR (400 MHz, DMSO-*d*₆): *δ* 9.50 (s, 1H), 9.48 (s, 1H), 8.64 (s, 1H), 8.54 (d, *J* = 1.6 Hz, 1H), 8.26 (d, *J =* 8.4 Hz, 1H), 7.96 - 7.91 (m, 3H), 7.87 (dd, *J =* 8.5, 1.8 Hz, 1H), 7.80 (d, *J =* 3.8 Hz, 1H), 7.49 (t, *J* = 8.9 Hz, 3H), 6.98 (d, *J* = 3.8 Hz, 1H). ¹³C-NMR (100 MHz, DMSO-*d*₆): *δ* 167.48, 166.85, 164.92 (¹*J*_{CF} = 251.43 Hz), 157.73, 153.24, 142.38, 136.95, 135.12, 132.94, 132.85, 132.58 (³*J*_{CF} = 9.53 Hz, 2C), 130.15, 127.43, 123.95, 123.00, 116.61, 116.39, 116.10 (²*J*_{CF} = 21.95 Hz, 2C), 106.69. HRMS (ESI): m/z calcd. for C₂₁H₁₃FN₃OS⁺ [M+H]⁺: 374.0758, found 374.0752.

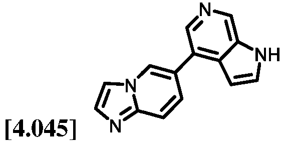

### 6-(1H-Pyrrolo[2,3-c]pyridin-4-yl)imidazo[1,2-a]pyridine (EI15)

Following the general method EA, synthesized with 4-bromo-1*H*-pyrrolo[2,3-c]pyridine (99.0 mg, 0.5 mmol), imidazo[1,2-a]pyridin-6-ylboronic acid (89.2 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI15** (78.0 mg, 67.0% yield) as a yellow solid after chromatographic purification (PE/EA = 1/1). ¹H-NMR (400 MHz, DMSO-*d*₆): *δ* 11.86 (s, 1H), 8.94 (s, 1H), 8.79 (s, 1H), 8.28 (s, 1H), 8.05 (s, 1H), 7.74 (t, *J =* 2.7 Hz, 1H), 7.71 (d, *J =* 9.3 Hz, 1H), 7.65-7.59 (m, 2H), 6.74 (d, *J =* 1.1 Hz, 1H).

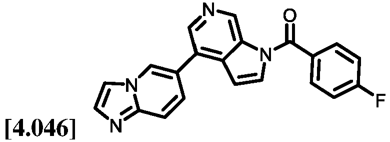

### (4-Fluorophenyl)(4-(imidazo[1,2-a]pyridin-6-yl)-1H-pyrrolo[2,3-c]pyridin-1-yl)methanone (E37)

Following the general method EB, synthesized with **EI15** (0.176 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E37** (0.126 g, 47.0% yield) as a white solid after chromatographic purification (PE/EA = 10/1), mp: 184.0-185.0 °C. ¹H-NMR (400 MHz, DMSO-*d*₆): *δ* 9.50 (s, 1H), 9.00 (s, 1H), 8.63 (s, 1H), 8.09 (s, 1H), 7.93 (dd, *J =* 8.7, 5.4 Hz, 2H), 7.82 (d, *J =* 3.7 Hz, 1H), 7.79 (d, *J* = 9.3 Hz, 1H), 7.72 (s, 1H), 7.65 (d, *J* = 9.3 Hz, 1H), 7.49 (t, *J =* 8.9 Hz, 2H), 7.02 (d, *J =* 3.7 Hz, 1H). ¹³C-NMR (100 MHz, DMSO-*d*₆): *δ* 166.85, 166.24 (¹*J*_{CF} = 248.73 Hz), 142.07, 137.18, 133.31, 133.07, 132.98, 132.88, 132.58 (³*J*_{CF}= 9.53 Hz, 2C), 127.82 (⁴*J*_{CF} = 2.75 Hz), 126.69, 125.84, 117.27, 116.63, 116.41, 116.11 (²*J*_{CF} = 21.93 Hz, 2C), 114.66, 113.58, 106.56. HRMS (ESI): m/z calcd. for C₂₁H₁₄FN₄O⁺ [M+H]⁺: 357.1146, found 357.1134.

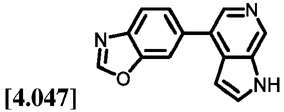

### 6-(1H-Pyrrolo[2,3-c]pyridin-4-yl)benzo[d]oxazole (EI16)

Following the general method EA, synthesized with 4-bromo-1*H*-pyrrolo[2,3-c]pyridine (99.0 mg, 0.5 mmol), benzo[d]oxazol-6-ylboronic acid (135.2 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI16** (75.0 mg, 66.0% yield) as a yellow solid after chromatographic purification (PE/EA = 1/1). ¹H-NMR (400 MHz, DMSO-*d*₆): δ 11.83 (s, 1H), 8.83 (s, 1H), 8.78 (s, 1H), 8.26 (s, 1H), 8.07 (s, 1H), 7.92 (d, *J =* 8.5 Hz, 1H), 7.78 (dd, *J =* 8.4, 1.2 Hz, 1H), 7.71 (s, 1H), 6.65 (d, *J =* 2.6 Hz, 1H).

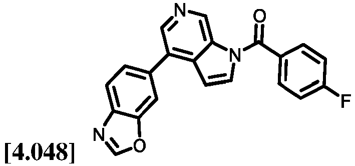

### (4-(Benzo[d]oxazol-6-yl)-1H-pyrrolo[2,3-c]pyridin-1-yl)(4-fluorophenyl)methanone (E38)

Following the general method EB, synthesized with **EI16** (0.176 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E38** (0.125 g, 48.0% yield) as a white solid after chromatographic purification (PE/EA = 10/1), mp: 226.0-227.0 °C. ¹H-NMR (400 MHz, DMSO-*d*₆): *δ* 9.49 (s, 1H), 8.86 (s, 1H), 8.60 (s, 1H), 8.09 (d, *J* = 1.4 Hz, 1H), 7.97 (d, *J =* 8.4 Hz, 1H), 7.93 (dd, *J =* 8.8, 5.4 Hz, 2H), 7.76 (dd, *J =* 10.3, 2.7 Hz, 2H), 7.48 (t, *J =* 8.9 Hz, 2H), 6.90 (d, *J* = 3.7 Hz, 1H). ¹³C-NMR (100 MHz, DMSO-*d*₆): *δ* 167.49, 164.90 (¹*J*_{CF} = 251.01 Hz), 155.59, 149.76, 142.43, 140.98, 136.83, 134.45, 133.64, 133.07, 132.93 (³*J*_{CF} = 4.40 Hz, 2C), 132.82, 130.16 (⁴*J*_{CF} = 2.87 Hz), 129.17, 126.97, 120.52, 116.50 (²*J*_{CF} = 22.22 Hz, 2C), 112.29, 106.57. HRMS (ESI): m/z calcd. for C₂₁H₁₃FN₃O₂⁺ [M+H]⁺: 358.0986, found 358.0991.

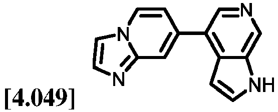

### 7-(1H-Pyrrolo[2,3-c]pyridin-4-yl)imidazo[1,2-a]pyridine (EI17)

Following the general method EA, synthesized with 4-bromo-1*H*-pyrrolo[2,3-c]pyridine (99.0 mg, 0.5 mmol), imidazo[1,2-a]pyridin-7-ylboronic acid (89.2 mg, 0.55 mmol), Na₂CO₃ (0.211 g, 2.0 mmol), Pd (PPh₃)₄ (29 mg, 0.025 mmol) in a mixture of acetonitrile and water (4 mL, V/V = 3:1) to give **EI17** (68.0 mg, 57.0% yield) as a yellow solid after chromatographic purification (PE/EA = 1/1). ¹H-NMR (400 MHz, DMSO-*d*₆): *δ* 11.88 (s, 1H), 8.80 (s, 1H), 8.70-8.64 (m, 1H), 8.34 (s, 1H), 8.02 (s, 1H), 7.85 (s, 1H), 7.75 (t, *J =* 2.5 Hz, 1H), 7.64 (s, 1H), 7.30 (dd, *J* = 7.1, 1.7 Hz, 1H), 6.73 (d, *J* = 2.6 Hz, 1H).

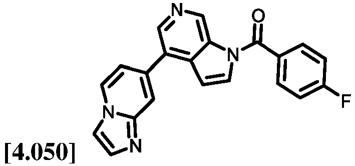

### (4-Fluorophenyl)(4-(imidazo[1,2-a]pyridin-7-yl)-1H-pyrrolo[2,3-c]pyridin-1-yl)methanone (E39)

Following the general method EB, synthesized with **EI17** (0.177 g, 0.75 mmol), 4-flourobenzoyl chloride (0.131g, 0.825 mmol), DMAP (0.028 g, 0.225 mmol) and triethylamine (0.21 mL, 1.5 mmol) in anhydrous dichloromethane (4 mL) to give **E39** (0.115 g, 43.0% yield) as a white solid after chromatographic purification (PE/EA = 10/1), mp: 238.0-239.0 °C. ¹H-NMR (400 MHz, DMSO-*d*₆): *δ* 9.50 (s, 1H), 8.73 (dd, *J* = 7.0, 0.7 Hz, 1H), 8.67 (s, 1H), 8.06 (s, 1H), 7.96-7.91 (m, 2H), 7.86 (s, 1H), 7.80 (d, *J =* 3.8 Hz, 1H), 7.68 (d, *J =* 1.0 Hz, 1H), 7.49 (t, *J =* 8.9 Hz, 2H), 7.26 (dd, *J =* 7.0, 1.7 Hz, 1H), 6.98 (d, *J =* 3.7 Hz, 1H). ¹³C-NMR (100 MHz, DMSO-*d*₆): *δ* 167.47, 164.93 (¹*J*_{CF} = 251.34 Hz), 145.06, 142.09, 137.29, 134.61, 134.07, 133.11, 132.90 (³*J*_{CF} *=* 9.49 Hz, 2C), 132.89, 131.17, 130.11 (⁴*J*_{CF} = 2.9 Hz), 127.84, 127.67, 116.61, 116.31 (²*J*_{CF} = 16.9 Hz, 2C), 113.77, 113.22, 106.51. HRMS (ESI): m/z calcd. for C₂₁H₁₄FN₄O⁺ [M+H]⁺: 357.1146, found 357.1120.

### Example 5:

Synthetic procedures and structural characterizations of provided compounds of formulae (**XVIII**)-(**XIX**) and intermediates are described:

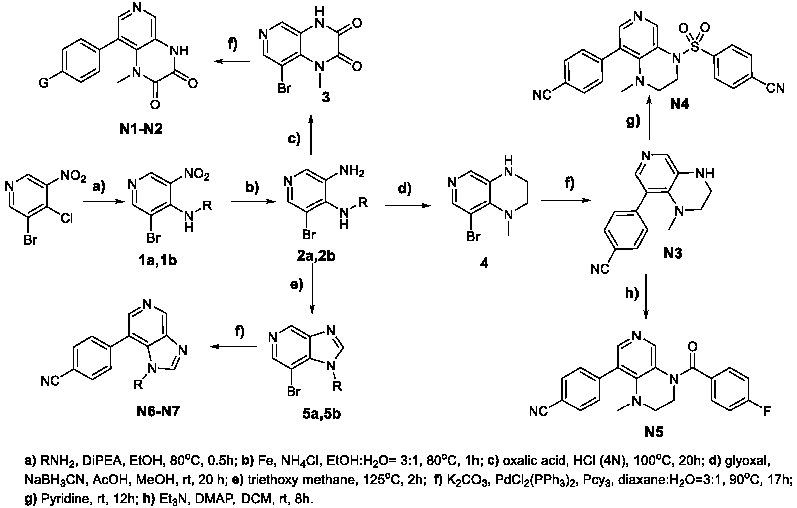

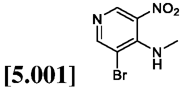

### 3-Bromo-N-methyl-5-nitropyridin-4-amine (NI1a)

To a solution of 3-bromo-4-chloro-5-nitropyridine (1.0 g, 4.21 mmol) in ethanol (10 mL) was added DIPEA (0.73 mL, 4.21 mmol) and 30% methylamine aq. (8.42 mmol) under N₂. The resulting mixture was heated to 80 °C and stirred for 0.5 h. After the reaction was complete as monitored by TLC, it was cooled to room temperature and diluted with water (30 mL) leading to yellow participates. The solid was collected and dried to give intermediate **NI1a** as a yellow solid (1.2 g, 92% yield), which was used directly in the next step without further purification and characterization.

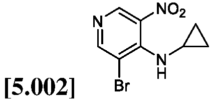

### 3-Bromo-N-cyclopropyl-5-nitropyridin-4-amine (NI1b)

To a solution of 3-bromo-4-chloro-5-nitropyridine (1.0 g, 4.21 mmol) in ethanol (10 mL) was added DIPEA (0.73 mL, 4.21 mmol) and cyclopropylamine. (8.42 mmol) under N₂. The resulting mixture was heated to 80 °C and stirred for 0.5 h. After the reaction was complete as monitored by TLC, it was cooled to room temperature and diluted with water (30 mL) leading to yellow participates. The solid was collected and dried to give intermediate **NI1b** as a yellow solid (1.3 g, 93% yield), which was used directly in the next step without further purification. ¹H-NMR (400 MHz, CDCl₃) δ 8.79 (s, 1H), 8.47 (s, 1H), 6.72 (s, 1H), 3.02 (dq, *J =* 6.7, 3.2 Hz, 1H), 0.85 (q, *J =* 6.8 Hz, 2H), 0.70-0.54 (m, 2H).

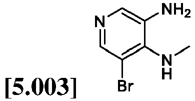

### 5-Bromo-N⁴-methylpyridine-3,4-diamine (NI2a)

To a solution of **NI1a** (90 mg, 0.39 mmol) in a mixture of ethanol (3.0 mL) and water (1.0 mL) was added Fe powder (130 mg, 2.33 mmol) and NH₄Cl (41.6 mg, 0.78 mmol). The resulting mixture was stirred at 80 °C under N₂ for 1 h. After the reaction was complete as monitored by TLC, the mixture was cooled to room temperature and filtered through Celite. The filtrate was diluted with water (5 mL) and extracted with dichloromethane (5 mL) for three times. The combined organic layers were washed with brine (5 mL) and dried over anhydrous Na₂SO₄. The solvent was removed under reduced pressure to give the crude product, which was subsequently purified by silica gel column chromatography (PE/EA) to yield compound **NI2a** as alight yellow solid (46.8 mg, 60.0% yield). The crude product was used directly in the next step without further purification and characterization. ¹H-NMR (400 MHz, CDCl₃) δ 8.04 (s, 1H), 7.85 (s, 1H), 3.64 (s, 2H), 2.89 (s, 3H).

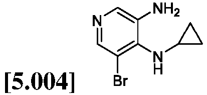

### 5-Bromo-N⁴-cyclopropylpyridine-3,4-diamine (NI2b)

To a solution of **NI1b** (95 mg, 0.39 mmol) in a mixture of ethanol (3.0 mL) and water (1.0 mL) was added Fe powder (130 mg, 2.33 mmol) and NH₄Cl (41.6 mg, 0.78 mmol). The resulting mixture was stirred at 80 °C under N₂ for 1 h. After the reaction was complete as monitored by TLC, the mixture was cooled to room temperature and filtered through Celite. The filtrate was diluted with water (5 mL) and extracted with dichloromethane (5 mL) for three times. The combined organic layers were washed with brine (5 mL) and dried over anhydrous Na₂SO₄. The solvent was removed under reduced pressure to give the crude product, which was subsequently purified by silica gel column chromatography (PE/EA) to yield compound **NI2b** as alight yellow solid (49.5 mg, 63.0% yield). The crude product was used directly in the next step without further purification and characterization. ¹H-NMR (400 MHz, CDCl₃) δ 7.98 (s, 1H), 7.81 (s, 1H), 4.01 (d, *J* = 32.5 Hz, 2H), 2.84 (s, 1H), 0.85 (d, *J* = 5.1 Hz, 2H), 0.73 (d, *J* = 2.7 Hz, 2H).

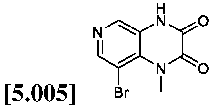

### 8-Bromo-1-methyl-1,4-dihydropyrido[3,4-b]pyrazine-2,3-dione (NI3)

To a suspension of **NI2a** (100.0 mg, 0.50 mmol) in HCl (4N, 0.74 mL) was added oxalic acid (54 mg, 0.6 mmol). The resulting mixture was heated to 100 °C and stirred for 20 h. After the reaction was complete as monitored by TLC, the reaction mixture was cooled to room temperature, neutralized with saturated Na₂CO₃ aq. to pH around 10 and extracted with dichloromethane (3×10 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography (PE/EA = 1/2) to give **I3** as a white solid (77.0 mg, 60% yield). ¹H-NMR (400 MHz, CDCl₃) δ 9.00 (s, 1H), 8.48 (s, 1H), 7.88 (s, 1H), 4.15 (s, 3H).

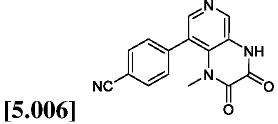

### 4-(1-Methyl-2,3-dioxo-1,2,3,4-tetrahydropyrido[3,4-b]pyrazin-8-yl)benzonitrile (N1)

Following the general method OC, synthesized with **NI3** (100.0 mg, 0.39 mmol), potassium carbonate (237.7 mg, 1.72 mmol), 4-cyanophenylboronic acid (86.0 mg, 0.59 mmol), PdCl₂(PPh₃)₂ (30.2 mg, 0.043 mmol) and tricyclohexylphosphine (24.1 mg, 0.086 mmol) in a mixed solvent of 1,4-dioxane (3 mL) and water (1 mL). The crude product was purified by silica gel column chromatography (DCM/MeOH = 50/1) to give **N1** (86.7 mg, 80% yield) as a white solid. mp: 205.0-206.0 °C. ¹H-NMR (400 MHz, CDCl₃) δ 9.16 (s, 1H), 8.28 (s, 1H), 7.91 (s, 1H), 7.82 (d, *J* = 8.2 Hz, 2H), 7.59 (d, *J* = 8.2 Hz, 2H), 3.49 (s, 3H); ¹³C-NMR (101 MHz, CDCl₃) δ 146.62, 143.62, 142.77, 141.47, 141.41, 140.06, 136.56, 132.28 (2C), 130.96 (2C), 120.77, 118.39, 112.69, 34.24.

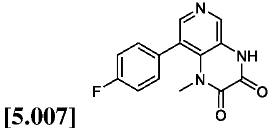

### 8-(4-Fluorophenyl)-1-methyl-1,4-dihydropyrido[3,4-b]pyrazine-2,3-dione (N2)

Following the general method OC, synthesized with **NI3** (100.0 mg, 0.39 mmol), potassium carbonate (237.7 mg, 1.72 mmol), 4-flourophenylboronic acid (82.5 mg, 0.59 mmol), PdCl₂(PPh₃)₂ (30.2 mg, 0.043 mmol) and tricyclohexylphosphine (24.1 mg, 0.086 mmol) in a mixed solvent of 1,4-dioxane (3 mL) and water (1 mL). The crude product was purified by silica gel column chromatography (DCM/MeOH = 50/1) to give **N2** (84.5 mg, 82% yield) as a white solid. mp: 196.0-197.0 °C. ¹H-NMR (400 MHz, CDCl₃) δ 9.11 (s, 1H), 8.27 (s, 1H), 7.86 (s, 1H), 7.41 (dd, *J* = 8.7, 5.3 Hz, 2H), 7.19 (t, *J* = 8.6 Hz, 2H), 3.47 (s, 3H); ¹³C-NMR (101 MHz, CDCl₃) δ 162.84 (¹*J*_{CF} = 248.3), 146.37 (2C), 143.15, 142.84, 141.30, 136.81, 131.86 (³*J*_{CF} = 8.1, 2C), 131.05 (⁴*J*_{CF} = 3.5), 121.56, 115.54 (²*J*_{CF} = 21.7, 2C), 34.05.

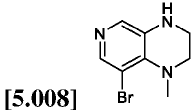

### 8-Bromo-1-methyl-1,2,3,4-tetrahydropyrido[3,4-b]pyrazine (NI4)

To a solution of **NI2a** (100.0 mg, 0.50 mmol), glacial acetic acid (2.8 µL, 0.05mmol) and 40% glyoxal (6.4 µL, 0.50 mmol) in methanol (2 mL) was added sodium cyanoborohydride (NaBH₃CN, 37.7 mg, 0.6 mmol) slowly in an ice bath. The resulting reaction mixture was allowed to warm to room temperature and continued to stir for 20 h. After the reaction was complete as monitored by TLC, the reaction mixture was diluted with water (4 mL) and extracted with ethyl acetate (3×5 mL). The combined organic layers were washed with water (2×10 mL) and brine (2×10 mL) and dried over anhydrous Na₂SO₄. The solvent was removed under reduced pressure to give the crude product, which was subsequently purified by silica gel column chromatography (PE/EA = 1/1) to give **NI4** (43.0 mg, 38.0%) as a light yellow solid. ¹H-NMR (400 MHz, CDCl₃) δ 7.96 (s, 1H), 7.78 (s, 1H), 3.33-3.27 (m, 2H), 3.14-3.07 (m, 2H), 2.93 (s, 3H), 2.07 (s, 1H), 2.04 (s, 1H).

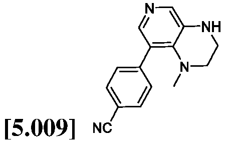

### 4-(1-Methyl-1,2,3,4-tetrahydropyrido[3,4-b]pyrazin-8-yl)benzonitrile (N3)

Following the general method OC, synthesized with **NI4** (100.0 mg, 0.43 mmol), potassium carbonate (237.7 mg, 1.72 mmol), 4-cynophenylboronic acid (94.1 mg, 0.64 mmol), PdCl₂(PPh₃)₂ (30.2 mg, 0.043 mmol) and tricyclohexylphosphine (24.1 mg, 0.086 mmol) in a mixed solvent of 1,4-dioxane (3 mL) and water (1 mL). The crude product was purified by silica gel column chromatography (DCM/MeOH = 20/1) to give **N2** (50.0 mg, 46.5% yield) as a white solid. mp: 233.0-234.0 °C. ¹H-NMR (400 MHz, CDCl₃) δ 7.81 (s, 1H), 7.75 - 7.66 (m, 3H), 7.61 (d, *J =* 8.3 Hz, 2H), 4.02 (s, 1H), 3.30 (dt, *J =* 8.8, 4.1 Hz, 4H), 2.38 (s, 3H).¹³C-NMR (101 MHz, DMSO) δ 144.24, 142.53, 140.31, 135.94, 133.71, 132.44 (2C), 129.44 (2C), 124.09, 119.08, 110.77, 51.25, 44.14, 38.00. HRMS (ESI): m/z calcd. for C₁₅H₁₄N₄ [M+H]⁺: 251.1297, found: 251.1290.

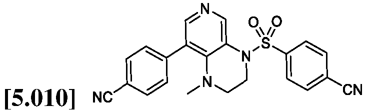

### 4-((8-(4-Cyanophenyl)-1-methyl-2,3-dihydropyrido[3,4-b]pyrazin-4(1H)-yl)sulfonyl)benzonitri le (N4)

Following the general method OB, synthesized with **N3** (80 mg, 0.32 mmol) and 4-cyanobenzenesulfonyl chloride (129 mg, 0.64 mmol) in pyridine (2 mL) to give **N4** (40.0 mg, 30.3% yield) as a light yellow solid after chromatographic purification (PE/EA = 1/1). mp: 186.0-187.0 °C. ¹H-NMR (400 MHz, CDCl₃) δ 8.58 (s, 1H), 8.07 (s, 1H), 7.86 - 7.74 (m, 4H), 7.70 (d, *J* = 8.2 Hz, 2H), 7.43 (d, *J =* 8.2 Hz, 2H), 3.92 (t, *J =* 5.5 Hz, 2H), 3.04 (t, *J =* 5.5 Hz, 2H), 2.13 (s, 3H); ¹³C-NMR (101 MHz, CDCl₃) δ 150.64, 146.10, 145.05, 143.64, 143.26, 133.07 (2C), 132.69 (2C), 129.29 (2C), 128.16 (2C), 122.98, 122.76, 118.55, 117.22, 117.01, 111.58, 50.36, 44.18, 43.08. HRMS (ESI): m/z calcd. for C₂₂H₁₇N₅O₂S [M+H]⁺:416.1181, found: 416.1161.

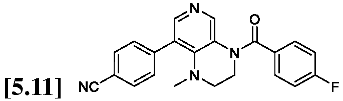

### 4-(4-(4-Fluorobenzoyl)-1-methyl-1,2,3,4-tetrahydropyrido[3,4-b]pyrazin-8-yl)benzonitrile (N5)

Following the general method OA, synthesized with **N3** (70 mg, 0.28 mmol), 4-fluorobenzoyl chloride (49 µL, 0.42 mmol), DMAP (10.3 mg, 0.08 mmol) and triethylamine (0.12 mL, 0.18 mmol) to give **N5** (55 mg, 52.8% yield) as a white solid after chromatographic purification (PE/EA = 2/1), mp: 194.0-195.0 °C. ¹H-NMR (400 MHz, CDCl₃) δ 7.91 (s, 1H), 7.76 (s, 1H), 7.76-7.70 (m, 2H), 7.53 (d, *J =* 8.4 Hz, 2H), 7.48 (dd, *J =* 8.7, 5.3 Hz, 2H), 7.06 (t, *J =* 8.6 Hz, 2H), 4.20-3.99 (m, 2H), 3.61-3.38 (m, 2H), 2.57 (s, 3H); ¹³C-NMR (101 MHz, CDCl₃) δ 167.83, 164.20 (¹*J*_{CF} *=* 252.4), 149.03, 144.88, 143.75, 143.62, 132.58 (2C), 131.17 (³*J*_{CF} =8.7, 2C), 130.72 (⁴*J*_{CF} = 3.4), 129.51 (2C), 125.95, 122.45, 118.71, 115.91 (²*J*_{CF} = 22.0, 2C), 111.38, 53.29, 43.33, 41.85. HRMS (ESI): m/z calcd. for C₂₂H₁₇FN₄O [M+H]⁺: 373.1465, found: 373.1445.

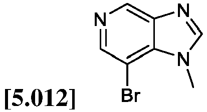

### 7-Bromo-1-methyl-1H-imidazo[4,5-c]pyridine (NI5a)

A solution of **NI2a** (50 mg, 0.25 mmol) in triethoxy methane (1.3 mL)was stirred at 125 °C for 2 h. After the reaction was complete as monitored by TLC, it was cooled to room temperature, diluted with water (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of CH₂Cl₂: MeOH (V/V) = 100:1 to 10:1, yielding the compound **NI5a** (45 mg, 85% yield). ¹H-NMR (400 MHz, CDCl₃) δ 8.98 (s, 1H), 8.47 (s, 1H), 7.87 (s, 1H), 4.14 (s, 3H).

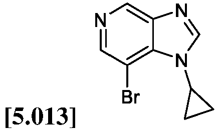

### 7-Bromo-1-cyclopropyl-1H-imidazo[4,5-c]pyridine (5b)

A solution of **NI2a** (55 mg, 0.25 mmol) in triethoxy methane (1.3 mL)was stirred at 125 °C for 2 h. After the reaction was complete as monitored by TLC, it was cooled to room temperature, diluted with water (5 mL), and extracted with ethyl acetate (3×5 mL). The combined organic layers were washed with brine (5 mL) and dried over anhydrous sodium sulfate. It was then concentrated under reduced pressure to give the crude product, which was subsequently purified by column chromatography with a gradient elution of CH₂Cl₂: MeOH (V/V) = 100:1 to 10:1, yielding the compound **NI5B** (56 mg, 95% yield). ¹H-NMR (400 MHz, CDCl₃) δ 8.97 (s, 1H), 8.51 (s, 1H), 7.96 (s, 1H), 3.68 (ddd, *J* = 10.8, 7.2, 3.9 Hz, 1H), 1.38-1.11 (m, 4H).

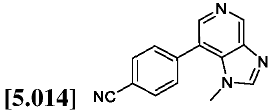

### 4-(1-Methyl-1H-imidazo[4,5-c]pyridin-7-yl)benzonitrile (N6)

Following the general method OC, synthesized with **NI5a** (60 mg, 0.28 mmol), potassium carbonate (154.8 mg, 1.12 mmol), 4-cynophenylboronic acid (62 mg, 0.42 mmol), PdCl₂(PPh₃)₂ (19.7 mg, 0.028 mmol) and tricyclohexylphosphine (15.7 mg, 0.056 mmol) in a mixed solvent of 1,4-dioxane (3 mL) and water (1 mL). The crude product was purified by silica gel column chromatography (DCM/MeOH = 20/1) to give **N6** (58.3 mg, 89.5% yield) as a white solid. mp: 240.0-241.0 °C. ¹H-NMR (400 MHz, CDCl₃) δ 9.15 (s, 1H), 8.28 (s, 1H), 7.91 (s, 1H), 7.81 (d, *J =* 8.0 Hz, 2H), 7.59 (d, *J* = 7.9 Hz, 2H), 3.49 (s, 3H); ¹³C-NMR (101 MHz, CDCl₃) δ 146.61, 143.71, 142.84, 141.48, 140.10, 136.38, 132.30 (2C), 130.97 (2C), 120.77, 118.41, 112.72, 34.25. HRMS (ESI): m/z calcd. for C₁₄H₁₀N₄ [M+H]⁺: 235.0984, found: 235.0982.

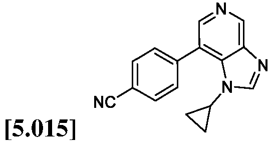

### 4-(1-Cyclopropyl-1H-imidazo[4,5-c]pyridin-7-yl)benzonitrile (N7)

Following the general method OC, synthesized with **NI5b** (63 mg, 0.28 mmol), potassium carbonate (154.8 mg, 1.12 mmol), 4-cynophenylboronic acid (62 mg, 0.42 mmol), PdCl₂(PPh₃)₂ (19.7 mg, 0.028 mmol) and tricyclohexylphosphine (15.7 mg, 0.056 mmol) in a mixed solvent of 1,4-dioxane (3 mL) and water (1 mL). The crude product was purified by silica gel column chromatography (DCM/MeOH = 20/1) to give **N7** (60.3 mg, 91.5% yield) as a white solid. mp: 184.0-185.0 °C. ¹H-NMR (400 MHz, CDCl₃) δ 9.13 (s, 1H), 8.32 (s, 1H), 8.00 (s, 1H), 7.80 (d, *J =* 8.1 Hz, 2H), 7.64 (d, *J =* 8.1 Hz, 2H), 3.25 - 2.90 (m, 1H), 0.69 (s, 2H), 0.55 (d, *J =* 6.4 Hz, 2H); ¹³C-NMR (101 MHz, CDCl₃) δ 146.11, 143.58, 142.98, 141.41, 141.10, 137.31, 132.05 (2C), 130.86 (2C), 121.23, 118.56, 112.14, 28.92, 8.15. HRMS (ESI): m/z calcd. for C₁₆H₁₂N₄ [M+H]⁺: 261.1140, found: 261.1139.

### Example 6:

The methods of determining the bioactivities of disclosed compounds and the corresponding results are provided as following:

### [6.001] Inhibition of hCYP11B1

Genes of human CYP11B1 and rat NADPH-P450 reductase were transfected into hamster V79 cells using a lentivirus vector, and stably-expressing cell lines were selected by taking advantages of the associated puromycin-resistance gene. Cultured cells were diluted and seeded in a 96-well plate with an amount of approximately 10,000 per well. After pre-incubation with suitable concentrations of the test compounds for 2 hours, 11-deoxycortisol of 200 nM was added as the substrate to initiate the catalytic reaction. After further incubation for 30 minutes, it was quenched with ethyl acetate. Subsequently, the substrate and product(s) were quantified using LC-MS/MS or HPLC at 260 nm, and the IC₅₀ values were calculated according to inhibitory percentage.

### [6.002] Inhibition of hCYP11B2

Genes of human CYP11B2 and rat NADPH-P450 reductase were transfected into hamster V79 cells using a lentivirus vector, and stably-expressing cell lines were selected by taking advantages of the associated puromycin-resistance gene. Cultured cells were diluted and seeded in a 96-well plate with an amount of approximately 10,000 per well. After pre-incubation with suitable concentrations of the test compounds for 2 hours, 11-deoxycorticosterone of 200 nM was added as the substrate to initiate the catalytic reaction. After further incubation for 30 minutes, it was quenched with ethyl acetate. Subsequently, the substrate and product(s) were quantified using LC-MS/MS or HPLC at 260 nm, and the IC₅₀ values were calculated according to inhibitory percentage.

### [6.003] Inhibition of hCYP8B1

Genes of human CYP8B1 and rat NADPH-P450 reductase were transfected into hamster V79 cells using a lentivirus vector, and stably-expressing cell lines were selected by taking advantages of the associated puromycin-resistance gene. Cultured cells were diluted and seeded in a 96-well plate with an amount of approximately 10,000 per well. After pre-incubation with suitable concentrations of the test compounds for 2 hours, 7-α-hydroxycholest-4-en-3-one of 200 nM was added as the substrate to initiate the catalytic reaction. After further incubation for 30 minutes, it was quenched with ethyl acetate. Subsequently, the substrate and product(s) were quantified using LC-MS/MS or HPLC at 260 nm, and the IC₅₀ values were calculated according to inhibitory percentage.

### [6.004] Inhibition of hCYP7A1

Genes of human CYP7A1 and rat NADPH-P450 reductase were transfected into hamster V79 cells using a lentivirus vector, and stably-expressing cell lines were selected by taking advantages of the associated puromycin-resistance gene. Cultured cells were diluted and seeded in a 96-well plate with an amount of approximately 10,000 per well. After pre-incubation with suitable concentrations of the test compounds for 2 hours, cholesterol of 200 nM was added as the substrate to initiate the catalytic reaction. After further incubation for 30 minutes, it was quenched with ethyl acetate. Subsequently, the substrate and product(s) were quantified using LC-MS/MS or HPLC at 260 nm, and the IC₅₀ values were calculated according to inhibitory percentage.

### [6.005] Inhibitory potencies

All compounds showed 100% inhibition against *h*CYP7A1 and hCYP8B at 50 µM.

The inhibitory potency of provided compounds against hCYP11B1 and hCYP11B2 are shown in Tables 1 and 2:

**Table 1: Inhibitory potencies provided compounds against hCYP11B1:**

| **Compd** | **CYP11B1 IC₅₀ nM** | **Compd** | **CYP11B1 IC₅₀ nM** | **Compd** | **CYP11B1 IC₅₀ nM** | **Compd** | **CYP11B1 IC₅₀ nM** |
|---|---|---|---|---|---|---|---|
| **Baxdrostat** | 3062 | **Osilodrostat** | **6** | **O1** | 0.22 | **O2** | 0.56 |
| **O3** | 0.10 | **O4** | 0.10 | **O5** | 0.48 | **O6** | 0.66 |
| **O7** | 0.12 | **O8** | 0.02 | **O9** | 1.1 | **O10** | 2.8 |
| **O11** | 2.0 | **O12** | 0.74 | **O13** | 0.31 | **O14** | 0.41 |
| **O15** | 1.46 | **O16** | 0.04 | **O17** | 6.5 | **O18** | 0.91 |
| **O19** | 1347 | **O20** | 1730 | **O21** | 1134 | **O22** | 3400 |
| **O23** | 516 | **O24** | 85.6 | **O25** | 476 | **O26** | 1446 |
| **O27** | 1196 | **O28** | 277 | **O29** | 1710 | **O30** | 4692 |
| **O31** | 187 | **O32** | 785 | **O33** | 1274 | **O34** | 546 |
| **O35** | 101 | **O36** | 262 | **O37** | 86 | **O38** | 559 |
| **O39** | 142 | **O40** | 73 | **O41** | 52 | **O42** | 67 |
| **O43** | 125 | **O44** | 84 | **O45** | 614 | **O46** | 240 |
| **O47** | 52 | **O48** | 117 | **O49** | 54 | **O50** | 42 |
| **O51** | 24 | **O52** | 51 | **O53** | 55 | **O54** | 119 |
| **O55** | 114 | **O56** | 292 | **O57** | 102 | **O58** | 132 |
| **O59** | 276 | **O60** | 2.5 | **O61** | 3.7 | **O62** | 178 |
| **O63** | 181 | **O64** | 63 | **O65** | 521 | **O66** | 495 |
| **O67** | 496 | **O68** | 52 | **O69** | 57 | **O70** | 31.6 |
| **O71** | 2170 | **O72** | 292 | **O73** | 3472 | **O74** | 99 |
| **O75** | 89 | **O76** | 146 | **O77** | 195 | **O78** | 183 |
| **O79** | 137 | **O80** | 185 | **O81** | 72 | **O82** | 69 |
| **O83** | 112 | **O84** | 53 | **O85** | 64 | **O86** | 72 |
| **O87** | 55 | **O88** | 485 | **O89** | 2546 | **O90** | 3255 |
| **O91** | 4156 | **O92** | 4436 | **O93** | 4170 | **O94** | 381 |
| **O95** | 4588 | **O96** | 1336 | **O97** | 219 | **O98** | 677 |
| **O99** | 652 | **O100** | 102 | **O101** | 57 | **O102** | 62 |
| **O103** | 79 | **O04** | 468 | **O105** | 650 | **O106** | 3652 |
| **O107** | 2162 | **O108** | 359 | **O109** | 376 | **O110** | 484 |
| **O111** | 216 | **O112** | 169 | **O113** | 97 | **O114** | 69 |
| **O115** | 104 | **O116** | 54 | **O117** | 45 | **O118** | 39 |
| **O119** | 38 | **O120** | 29 | **O121** | 36 | **O122** | 59 |
| **O123** | 26 | **O124** | 38 | **O125** | 28 | **O126** | 126 |
| **O127** | 197 | **O128** | 256 | **O129** | 243 | **O130** | 97 |
| **O131** | 248 | **O132** | 168 | **O133** | 4216 | **O134** | 3579 |
| **O135** | 4128 | **O136** | 2019 | **O137** | 3962 | | |
| **S1** | >5000 | **S2** | >5000 | **S3** | >5000 | **S4** | 1368 |
| **S5** | 2984 | **S6** | 1742 | **S7** | 4392 | **S8** | 1.5 |
| **S9** | 0.7 | **S10** | 0.8 | **S11** | 2.1 | **S12** | 2.8 |
| **S13** | 1.2 | **S14** | 4.8 | **S15** | 2.2 | **S16** | 12 |
| **S17** | 10.2 | **S18** | 2 | **S19** | 1.2 | **S20** | 3.1 |
| **S21** | 15 | **S22** | 51 | **S23** | 1.9 | **S24** | 1.1 |
| **S25** | 1.82 | **S26** | 28 | **S27** | 2.9 | **S28** | 1.8 |
| **S29** | 7.3 | **S30** | 19 | **S31** | 136 | **S32** | 468 |
| **S33** | 5.4 | **S34** | 13.4 | **S35** | 0.35 | **S36** | 30.4 |
| **S37** | 84 | **S38** | 0.7 | **S39** | 137 | **S40** | 657 |
| **S41** | 99 | **S42** | 24 | **S43** | 621 | **S44** | 782 |
| **S45** | 1455 | **S46** | 322 | **S47** | 472 | **S48** | 14 |
| **S49** | 7 | **S50** | 298 | **S51** | 1968 | **S52** | 697 |
| **S53** | 72 | **S54** | 549 | **S55** | 10.6 | **S56** | 41 |
| **S57** | 66 | **S58** | 27 | **S59** | 48 | **S60** | 54 |
| **S61** | 688 | **S62** | 516 | **S63** | 792 | **S64** | 755 |
| **S65** | 783 | **S66** | 587 | **S67** | 603 | **S68** | 22 |
| **S69** | 73 | **S70** | 49 | **S71** | 349 | **S72** | 23 |
| **S73** | 18 | **S74** | 22 | **S75** | 12 | **S76** | 24 |
| **S77** | 32 | **S78** | 38 | **S79** | 16 | **S80** | 18 |
| **S81** | 19 | **S82** | 9.4 | **S83** | 21 | **S84** | 57 |
| **S85** | 37 | **S86** | 1536 | **S87** | 522 | **S88** | 251 |
| **S89** | 2845 | **S90** | 4436 | **S91** | 3646 | **S92** | 856 |
| **S93** | 1306 | **S94** | 2659 | **S95** | 2056 | **S96** | 643 |
| **S97** | 4207 | **S98** | 1162 | **S99** | 703 | **S100** | 966 |
| **S101** | 2673 | **S102** | 3712 | **S103** | 955 | | |
| **C1** | 0.56 | **C2** | 0.35 | **C3** | 0.20 | **C4** | 0.36 |
| **C5** | 1.1 | **C6** | 1.2 | **C7** | 1.9 | **C8** | 0.25 |
| **C9** | 0.5 | **C10** | 1.7 | **C11** | 0.7 | **C12** | 0.3 |
| **C13** | 0.2 | **C14** | 0.9 | **C15** | 0.16 | **C16** | 0.18 |
| **C17** | 7.8 | **C18** | 0.61 | **C19** | 9.5 | **C20** | 0.4 |
| **C21** | 0.01 | **C22** | 112 | **C23** | 310 | **C24** | 124 |
| **C25** | 988 | **C26** | 356 | **C27** | 117 | **C28** | 310 |
| **C29** | 185 | **C30** | 472 | **C31** | 305 | **C32** | 233 |
| **C33** | 245 | **C34** | 284 | **C35** | 459 | **C36** | 297 |
| **C37** | 766 | **C38** | 606 | **C39** | 298 | **C40** | 352 |
| **C41** | 353 | **C42** | 1570 | **C43** | 897 | **C44** | 139 |
| **C45** | 152 | **C46** | 274 | **C47** | 19 | **C48** | 762 |
| **C49** | 971 | **C50** | 1274 | **C51** | 4253 | **C52** | 891 |
| **C53** | 378 | **C54** | 895 | **C55** | 973 | **C56** | 522 |
| **C57** | 876 | **C58** | 2645 | **C59** | 2737 | **C60** | >10000 |
| **C61** | 1351 | **C62** | 512 | **C63** | 1820 | **C64** | 258 |
| **C65** | 292 | **C66** | 92 | **C67** | 277 | **C68** | 26 |
| **C69** | 134 | **C70** | 108 | **C71** | 28 | **C72** | 51 |
| **C73** | 124 | **C74** | 53 | **C75** | 111 | **C76** | 62 |
| **C77** | 376 | **C78** | 2457 | **C79** | 2952 | **C80** | 476 |
| **C81** | 438 | **C82** | 537 | **C83** | 672 | | |
| **E1** | 0.5 | **E2** | 0.4 | **E3** | 0.3 | **E4** | 0.3 |
| **E5** | 0.6 | **E6** | 0.3 | **E7** | 0.2 | **E8** | 0.6 |
| **E9** | 0.3 | **E10** | 0.3 | **E11** | 0.2 | **E12** | 0.5 |
| **E13** | 0.5 | **E14** | 0.5 | **E15** | 0.7 | **E16** | 0.6 |
| **E17** | 0.7 | **E18** | 3.8 | **E19** | 2.9 | **E20** | 65 |
| **E21** | 5 | **E22** | 21 | **E23** | 79 | **E24** | 82 |
| **E25** | 98 | **E26** | 88 | **E27** | 73 | **E28** | 17 |
| **E29** | 9 | **E30** | 11 | **E31** | 635 | **E32** | 269 |
| **E33** | 673 | **E34** | 375 | **E35** | 857 | **E36** | 1982 |
| **E37** | 455 | **E38** | 49 | **E39** | 678 | | |
| **N1** | 409 | **N2** | 746 | **N3** | 394 | **N4** | 5.2 |
| **N5** | 48 | **N6** | 661 | **N7** | 453 | | |

**Table 2: Inhibitory potencies provided compounds against hCYP11B2:**

| **Compd** | **CYP11B2 IC₅₀ nM** | **Compd** | **CYP11B2 IC₅₀ nM** | **Compd** | **CYP11B2 IC₅₀ nM** | **Compd** | **CYP11B2 IC₅₀ nM** |
|---|---|---|---|---|---|---|---|
| **Baxdrostat** | 127 | **Osilodrostat** | 0.4 | **O1** | 0.16 | **O2** | 0.13 |
| **O3** | 0.14 | **O4** | 0.10 | **O5** | 0.23 | **O6** | 0.41 |
| **O7** | 0.47 | **O8** | 0.02 | **O9** | 0.11 | **O10** | 0.41 |
| **O11** | 0.04 | **O12** | 0.18 | **O13** | 0.48 | **O14** | 0.2 |
| **O15** | 0.25 | **O16** | 0.56 | **O17** | 0.39 | **O18** | 0.20 |
| **O19** | 23.1 | **O20** | 25.2 | **O21** | 26.1 | **O22** | 108.3 |
| **O23** | 29.5 | **O24** | 19.7 | **O25** | 18.1 | **O26** | 22.4 |
| **O27** | 23.6 | **O28** | 20.9 | **O29** | 5.9 | **O30** | 109.8 |
| **O31** | 2.8 | **O32** | 18.0 | **O33** | 18.3 | **O34** | 17.4 |
| **O35** | 0.9 | **O36** | 1.8 | **O37** | 1.4 | **O38** | 16.8 |
| **O39** | 2.6 | **O40** | 0.8 | **O41** | 1.8 | **O42** | 0.6 |
| **O43** | 4.7 | **O44** | 4.8 | **O45** | 15.1 | **O46** | 5.7 |
| **O47** | 3.8 | **O48** | 2.1 | **O49** | 1.6 | **O50** | 5.0 |
| **O51** | 0.7 | **O52** | 1.1 | **O53** | 8.2 | **O54** | 6.9 |
| **O55** | 2.1 | **O56** | 5.1 | **O57** | 1.6 | **O58** | 1.9 |
| **O59** | 2.8 | **O60** | 0.6 | **O61** | 0.51 | **O62** | 3.3 |
| **O63** | 2.0 | **O64** | 0.5 | **O65** | 2.5 | **O66** | 4.5 |
| **O67** | 2.7 | **O68** | 0.7 | **O69** | 2.1 | **O70** | 1.4 |
| **O71** | 39 | **O72** | 3.6 | **O73** | 45 | **O74** | 1.3 |
| **O75** | 1.3 | **O76** | 1.6 | **O77** | 2.0 | **O78** | 2.1 |
| **O79** | 2.9 | **O80** | 1.0 | **O81** | 0.6 | **O82** | 1.4 |
| **O83** | 1.0 | **O84** | 1.6 | **O85** | 4.6 | **O86** | 0.8 |
| **O87** | 2.5 | **O88** | 6 | **O89** | 15.6 | **O90** | 48.8 |
| **O91** | 92 | **O92** | 28.4 | **O93** | 90 | **O94** | 4.7 |
| **O95** | 41 | **O96** | 5.2 | **O97** | 35 | **O98** | 46 |
| **O99** | 39 | **O100** | 0.3 | **O101** | 0.4 | **O102** | 0.3 |
| **O103** | 0.5 | **O104** | 4.1 | **O105** | 29 | **O106** | 48 |
| **O107** | 99 | **O108** | 7.9 | **O109** | 4.8 | **O110** | 19.3 |
| **O111** | 23.2 | **O112** | 27 | **O113** | 3 | **O114** | 7 |
| **O115** | 3.2 | **O116** | 3.1 | **O117** | 2.4 | **O118** | 2.7 |
| **O119** | 4.2 | **O120** | 0.3 | **O121** | 0.4 | **O122** | 0.8 |
| **O123** | 0.3 | **O124** | 0.6 | **O125** | 0.5 | **O126** | 3 |
| **O127** | 2.3 | **O128** | 3.3 | **O129** | 1.9 | **O130** | 5 |
| **O131** | 3.3 | **O132** | 5.4 | **O133** | 55 | **O134** | 52 |
| **O135** | 66 | **O136** | 22 | **O137** | 57 | | |
| **S1** | 451 | **S2** | 473 | **S3** | 243 | **S4** | 34.8 |
| **S5** | 21 | **S6** | 53 | **S7** | 88 | **S8** | 0.1 |
| **S9** | 0.2 | **S10** | 0.1 | **S11** | 0.15 | **S12** | 0.18 |
| **S13** | 0.1 | **S14** | 0.25 | **S15** | 0.05 | **S16** | 1.05 |
| **S17** | 0.4 | **S18** | 0.1 | **S19** | 0.1 | **S20** | 0.1 |
| **S21** | 0.3 | **S22** | 5.2 | **S23** | 0.2 | **S24** | 0.13 |
| **S25** | 0.04 | **S26** | 0.86 | **S27** | 0.7 | **S28** | 0.2 |
| **S29** | 1.5 | **S30** | 1.0 | **S31** | 5.9 | **S32** | 10.3 |
| **S33** | 0.09 | **S34** | 0.17 | **S35** | 0.12 | **S36** | 5.6 |
| **S37** | 6 | **S38** | 2.1 | **S39** | 264 | **S40** | 6.7 |
| **S41** | 0.4 | **S42** | 0.3 | **S43** | 6.5 | **S44** | 46 |
| **S45** | 11.6 | **S46** | 2.8 | **S47** | 2.6 | **S48** | 25 |
| **S49** | 27 | **S50** | 52 | **S51** | 10.3 | **S52** | 32 |
| **S53** | 0.9 | **S54** | 16 | **S55** | 0.2 | **S56** | 0.5 |
| **S57** | 3.9 | **S58** | 2 | **S59** | 0.5 | **S60** | 0.7 |
| **S61** | 21.7 | **S62** | 7.2 | **S63** | 10 | **S64** | 4.5 |
| **S65** | 5 | **S66** | 3.8 | **S67** | 3.2 | **S68** | 0.8 |
| **S69** | 1.3 | **S70** | 1.4 | **S71** | 14 | **S72** | 2.1 |
| **S73** | 0.3 | **S74** | 1.7 | **S75** | 0.7 | **S76** | 0.8 |
| **S77** | 0.8 | **S78** | 1.2 | **S79** | 0.8 | **S80** | 0.6 |
| **S81** | 0.4 | **S82** | 0.4 | **S83** | 0.8 | **S84** | 1.1 |
| **S85** | 0.9 | **S86** | 22 | **S87** | 12 | **S88** | 6.8 |
| **S89** | 43 | **S90** | 64 | **S91** | 44 | **S92** | 13 |
| **S93** | 24 | **S94** | 38 | **S95** | 79 | **S96** | 11 |
| **S97** | 21 | **S98** | 11 | **S99** | 6.9 | **S100** | 10.6 |
| **S101** | 25 | **S102** | 36 | **S103** | 5 | | |
| **C1** | 0.24 | **C2** | 0.12 | **C3** | 0.16 | **C4** | 0.64 |
| **C5** | 0.4 | **C6** | 0.3 | **C7** | 0.15 | **C8** | 1.4 |
| **C9** | 0.3 | **C10** | 1.3 | **C11** | 1 | **C12** | 0.17 |
| **C13** | 0.6 | **C14** | 0.5 | **C15** | 0.28 | **C16** | 1.5 |
| **C17** | 3.5 | **C18** | 0.69 | **C19** | 4.3 | **C20** | 1.8 |
| **C21** | 0.01 | **C22** | 2 | **C23** | 7 | **C24** | 2.3 |
| **C25** | 30 | **C26** | 2.3 | **C27** | 2.3 | **C28** | 7 |
| **C29** | 1.7 | **C30** | 11 | **C31** | 4.6 | **C32** | 18 |
| **C33** | 25 | **C34** | 22 | **C35** | 31 | **C36** | 28 |
| **C37** | 60 | **C38** | 40 | **C39** | 23 | **C40** | 29 |
| **C41** | 40 | **C42** | 45 | **C43** | 99 | **C44** | 27 |
| **C45** | 20 | **C46** | 5.4 | **C47** | 17 | **C48** | 7.3 |
| **C49** | 25 | **C50** | 97 | **C51** | 23 | **C52** | 44 |
| **C53** | 11 | **C54** | 45 | **C55** | 29 | **C56** | 103 |
| **C57** | 27 | **C58** | 75 | **C59** | 131 | **C60** | 152 |
| **C61** | 216 | **C62** | 30 | **C63** | 113 | **C64** | 5.3 |
| **C65** | 7.6 | **C66** | 0.9 | **C67** | 6.5 | **C68** | 0.7 |
| **C69** | 2.4 | **C70** | 12 | **C71** | 2 | **C72** | 6.8 |
| **C73** | 11 | **C74** | 9 | **C75** | 35 | **C76** | 9 |
| **C77** | 8 | **C78** | 20 | **C79** | 46 | **C80** | 22 |
| **C81** | 15 | **C82** | 3 | **C83** | 6 | | |
| **E1** | 0.5 | **E2** | 0.5 | **E3** | 0.4 | **E4** | 0.4 |
| **E5** | 0.3 | **E6** | 0.13 | **E7** | 0.1 | **E8** | 0.1 |
| **E9** | 0.1 | **E10** | 0.6 | **E11** | 0.1 | **E12** | 0.3 |
| **E13** | 0.3 | **E14** | 0.08 | **E15** | 0.4 | **E16** | 0.1 |
| **E17** | 0.1 | **E18** | 1.2 | **E19** | 2.5 | **E20** | 0.8 |
| **E21** | 4 | **E22** | 0.6 | **E23** | 0.8 | **E24** | 1.9 |
| **E25** | 2.1 | **E26** | 3.9 | **E27** | 1.5 | **E28** | 2.1 |
| **E29** | 2.5 | **E30** | 0.1 | **E31** | 10 | **E32** | 13 |
| **E33** | 32 | **E34** | 5 | **E35** | 17 | **E36** | 29 |
| **E37** | 0.6 | **E38** | 8 | **E39** | 12 | | |
| **N1** | 372 | **N2** | 593 | **N3** | 311 | **N4** | 0.6 |
| **N5** | 7.8 | **N6** | 655 | **N7** | 679 | | |

As shown in Table 1 and Table 2, the annulated pyridines described in this invention inhibit the catalytic activity of various human steroidogenesis enzymes (CYP11B1, CYP11B2, CYP8B1, and CYP7A1) to different degrees. It can be expected that these annulated-pyridyl steroidogenesis inhibitors disclosed in this invention can inhibit the production of one or more steroids selected from the group comprising of aldosterone, cortisol, or bile acids to different degrees in animals or humans, thereby treating related diseases.

Compared to Baxdrostat, a CYP11B2 inhibitor currently undergoing clinical trials for hypertension and hyperaldosteronism, the annulated pyridines disclosed in this invention not only possess distinct and novel chemical structures, but also exhibit significantly higher inhibitory potency against *h*CYP11B2 than Baxdrostat.

Compared to Osilodrostat, a CYP11B1 inhibitor already on the market for the treatment of Cushing syndrome, the annulated pyridines disclosed in this invention not only possess distinct and novel chemical structures, but also exhibit significantly higher inhibitory potency against hCYP11B1 than Osilodrostat.

The technical solutions of this invention are not limited to the specific embodiments described above. Any technical modifications, variations and adaptations made based on the technical solutions of this invention fall within the scope of protection of this invention.

## Claims

1. A compound of formula (I): 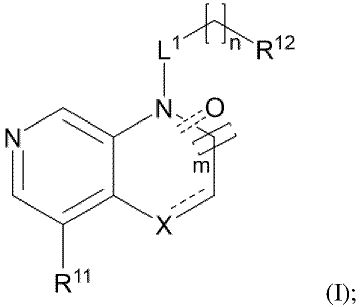 wherein:
n is 0, 1, or 2;
m is 0 or 1;
X is C, N, 0, or S;
L¹ is selected from the group consisting of hydrogen, -SO₂-, -CO-, and -CH₂-;
- - - is a single bond or a double bond;
::::::O means that one or more carbon atoms are replaced by carbonyl groups or not;
R¹¹ is selected from the group consisting of aryl, heteroaryl, spirocycloalkyl, azaspirocycloalkyl, oxaspirocycloalkyl, bicycloalkyl, azacycloalkyl, and azacyclic ketone; said aryl, heteroaryl, spirocycloalkyl, bicycloalkyl, azacycloalkyl, and azacyclic ketone are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens; alternatively, R¹¹ is of the following structure: 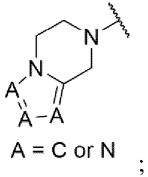
R¹² is selected from the group consisting of hydrogen, alkyl, azacycloalkyl, bicyclic hydrocarbyl, spirocycloalkyl, azaspirocycloalkyl, oxaspirocycloalkyl, aryl, heteroaryl, and absent; said alkyl, aryl, and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens; said azacycloalkyl, bicyclic hydrocarbyl, and spirocycloalkyl are optionally substituted with cyano, fluorophenyl, fluorophenylalkyl, or fluorophenylamino; alternatively, R¹² is selected from the following structures: 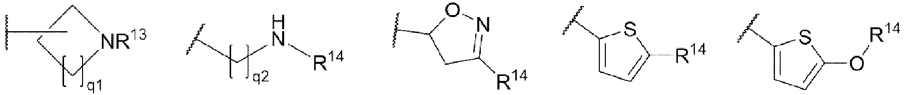 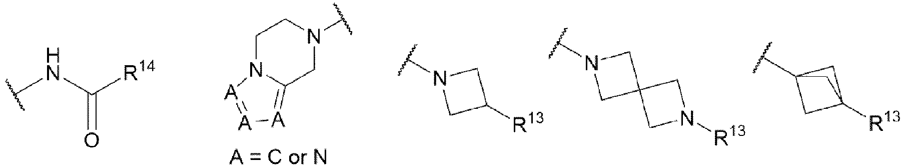
wherein q¹ and q² are each independently selected from 1, 2, and 3; R¹³ is selected from the group consisting of hydrogen, cyano, cyclic ether, and -L²-R¹⁴, where L² is selected from -SO₂-, -CO-, hydrocarbyl, or absent; R¹⁴ is selected from aryl and heteroaryl, said aryl and heteroaryl are optionally substituted with one to three substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, and boronate ester.

2. The compound according to claim 1, wherein the aryl is phenyl or naphthyl; the heteroaryl is selected from the group consisting of quinolinyl, thiophenyl, thiazolyl, indolinyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, benzoxazolyl, indolinonyl, quinolinonyl, triazolopiperazinyl, imidazopiperazinyl, and benzooxazinonyl.

3. The compound according to claims 1 to 2, wherein R¹¹ is selected from the group consisting of aryl and heteroaryl; said aryl and heteroaryl are optionally substituted with one or more substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, boronate ester, cyclic ether, cycloalkyl and alkyl optionally substituted with one to three halogens; preferably, R¹¹ is selected from cyanophenyl, halogen-substituted benzofuranyl, and cyanobenzofuranyl; more preferably, R¹¹ is selected from 4-cyanophenyl, 5-chlorobenzofuran-2-yl, and 5-cyanobenzofuran-2-yl.

4. The compound according to claims 1 to 3, wherein R¹² is selected from the group consisting of phenyl, naphthyl, quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, triazolopiperazine, imidazopiperazine, azetidinyl, piperidinyl, azabicyclo[3.3]heptanyl, oxabicyclo[3.3]heptanyl, and bicyclo[1.1.1]pentanyl; these groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens.

5. The compound according to claims 1 to 4, wherein the alkyl group is selected from methyl, ethyl, propyl, isopropyl, n-butyl, isobutyl and tert-butyl.

6. The compound according to any one of claims 1 to 5, wherein:
n is 0, 1, or 2;
m is 1;
X is O;
L¹ is selected from the group consisting of -SO₂- and -CO-;
- - - is a single bond;
**R¹¹** is selected from the group consisting of aryl, heteroaryl, spirocycloalkyl, azaspirocycloalkyl, oxaspirocycloalkyl, bicyclic hydrocarbyl, azacycloalkyl, or azacyclic ketone; these groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, R¹¹ is selected from cyanophenyl, cyano-substituted bicyclic hydrocarbyl, cyano-substituted azaspirocycloalkyl, or cyano-substituted azacycloalkyl; more preferably, R¹¹ is selected from 4-cyanophenyl, 1-cyano-bicyclo[1.1.1]pentyl, 1-cyanopiperazinyl, and 1-cyano-dihydropyridyl;
R¹² is selected from the group consisting of alkyl, azacycloalkyl, bicyclic hydrocarbyl, spirocycloalkyl, azaspirocycloalkyl, oxaspirocycloalkyl, aryl, and heteroaryl; said alkyl, azacycloalkyl, bicyclic hydrocarbyl, spirocycloalkyl, azaspirocycloalkyl, aryl, and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, cyclic ether, sulfuryl fluoride, boronate ester, and alkyl optionally substituted with one to three halogens.

7. The compound according to any one of claims 1 to 6, wherein the compound is of formula (**II**): 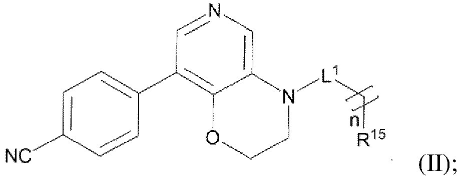 wherein:
n is 0, 1, or 2;
L¹ is selected from the group consisting of -SO₂- and -CO-;
R¹⁵ is selected from the group consisting of alkyl, azacycloalkyl, bicyclic hydrocarbyl, spirocycloalkyl, azaspirocycloalkyl, oxaspirocycloalkyl, aryl, and heteroaryl; said alkyl, azacycloalkyl, bicyclic hydrocarbyl, spirocycloalkyl, azaspirocycloalkyl, oxaspirocycloalkyl, aryl, and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, the alkyl is selected from methyl and ethyl; the aryl is selected from phenyl and naphthyl; the heteroaryl is selected from quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, and thienopyridinyl; the azacycloalkyl is selected from azetidinyl; the bicyclic hydrocarbyl is selected from bicyclo[1.1.1]pentyl and bicyclo[2.2.2]octyl; and the azaspirocycloalkyl is selected from 2,6-diazabicyclo[3.3]heptyl.

8. The compound according to any one of claims 1 to 7, wherein the compound is of formula (**III**): 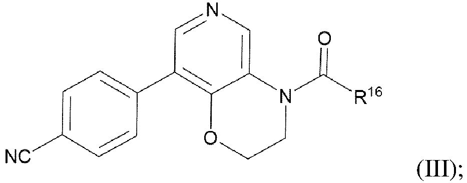 wherein:
R¹⁶ is selected from the following structures: 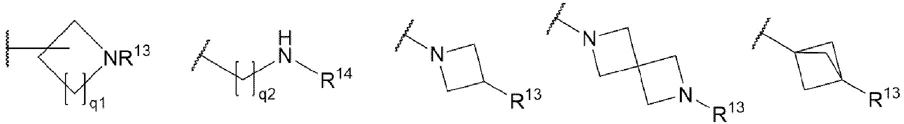
wherein q¹, q², R¹³ and R¹⁴ are defined as in claim 1.

9. The compound according to any one of claims 1 to 8, wherein:
n is 0;
m is 1;
X is S;
L¹ is selected from the group consisting of -SO₂-, -CO-, and -CH₂-;
- - - is a single bond;
R¹¹ is selected from the group consisting of aryl, heteroaryl, spirocycloalkyl, azaspirocycloalkyl, oxaspirocycloalkyl, bicyclic hydrocarbyl, azacycloalkyl, and azacyclic ketone; said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, cyclic ether, and alkyl optionally substituted with one to three halogens; preferably, R¹¹ is selected from cyanophenyl, halogen-substituted benzofuranyl, and cyano-substituted benzofuranyl; more preferably, R¹¹ is selected from 5-chlorobenzofuran-2-yl, 4-cyanophenyl, and 5-cyanobenzofuran-2-yl;
R¹² is selected from the group consisting of aryl, heteroaryl, azacycloalkyl, bicyclic hydrocarbyl, spirocycloalkyl, azaspirocycloalkyl, and oxaspirocycloalkyl; said aryl, heteroaryl, azacycloalkyl, bicyclic hydrocarbyl, spirocycloalkyl, azaspirocycloalkyl, and oxaspirocycloalkyl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

10. The compound according to any one of claims 1 to 9, wherein the compound is of formula (**IV**): 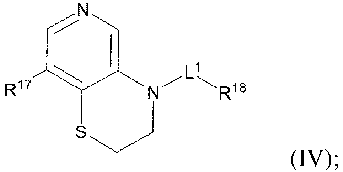 wherein:
L¹ is selected from the group consisting of -SO₂-, -CO- and -CH₂-;
R¹⁷ is selected from the group consisting of aryl and heteroaryl; said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, the aryl is phenyl; the heteroaryl is selected from the group consisting of quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, indolinonyl, and quinolinonyl; more preferably, R¹⁷ is selected from 4-cyanophenyl, 5-chlorobenzofuran-2-yl, and 5-cyanobenzofuran-2-yl;
R¹⁸ is selected from the group consisting of aryl, heteroaryl, azacycloalkyl, bicyclic hydrocarbyl, spirocycloalkyl, azaspirocycloalkyl, and oxaspirocycloalkyl; said aryl, heteroaryl, azacycloalkyl, bicyclic hydrocarbyl, spirocycloalkyl, azaspirocycloalkyl, and oxaspirocycloalkyl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, the aryl group is selected from phenyl and naphthyl; the heteroaryl group is selected from quinolinyl, thiophenyl, pyrazolyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, thienopyridinyl, triazolopiperazinyl, and imidazopiperazinyl; the azacycloalkyl is selected from azetidinyl, the bicyclic hydrocarbyl is selected from bicyclo[1.1.1]pentyl and bicyclo[2.2.2]octyl, and the azaspirocycloalkyl is selected from 2-azabicyclo[3.3]heptyl.

11. The compound according to any one of claims 1 to 10, wherein the compound is of formula (**XI**): 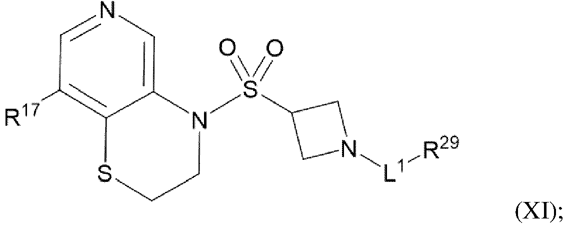 wherein:
L¹ is selected from the group consisting of -SO₂-, -CO-, -CH₂- or absent;
R¹⁷ is as defined in claim 10, and R²⁹ is selected from the group consisting of cyano, cyclic ether, or phenyl optionally substituted with one to three substituents selected from halogen, cyano, and alkoxy.

12. The compound according to any one of claims 1 to 11, wherein the compound is of formula (**XII**): 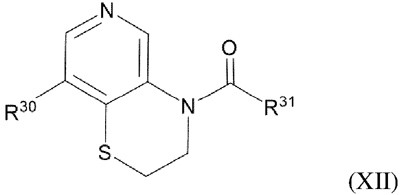 wherein:
R³⁰ is selected from aryl and heteroaryl; said aryl and heteroaryl are optionally substituted with one or two substituents selected from halogen, cyano, and alkyl; preferably, the aryl group is phenyl; and the heteroaryl group is selected from pyridyl, indazolyl, benzothiazolyl, indolyl, benzofuranyl, benzothienyl, benzimidazolyl, benzopyrazolyl, indolinonyl, imidazopyridyl, pyrazolopyridyl, quinolinonyl, and pyrrolopyridyl;
R³¹ is selected from phenyl and pyridyl; the phenyl group is optionally substituted with one to three substituents selected from halogen, cyano, alkoxy, nitro, amino, hydroxyl, acetylamino, cycloalkyl and alkyl optionally substituted with one to three halogens.

13. The compound according to any one of claims 1 to 12, wherein the compound is of formula **(XIII):** 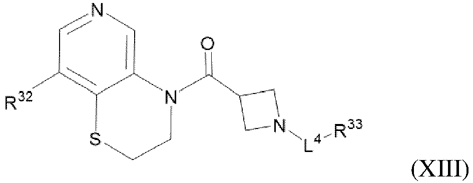
wherein R³² is selected from phenyl and quinolinone; said phenyl and quinolinone are optionally substituted with one or two substituents selected from halogen, cyano, and alkyl;
L⁴ is selected from -SO₂- and -CH₂-;
R³³ is selected from aryl and heteroaryl; these groups are optionally substituted with one or more substituents selected from cyano, nitro, and hydroxyl; preferably, the heteroaryl group is selected from thienyl and pyridyl.

14. The compound according to any one of claims 1 to 12, wherein the compound is of formula (**XIV**): 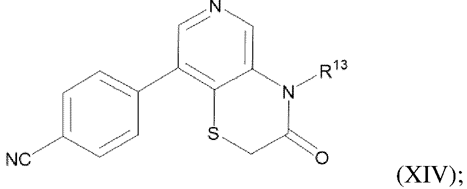 wherein R¹³ is defined as in claim 1.

15. The compound according to any one of claims 1 to 14, wherein:
n is 0, 1, or 2;
m is 0;
X is C;
L¹ is selected from the group consisting of -SO₂-, and -CO-;
- - - is a single bond;
R¹¹ is selected from the group consisting of aryl, heteroaryl, bicyclic hydrocarbyl, azacycloalkyl, azaspirocycloalkyl, and azacyclic ketone; said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, R¹¹ is selected from cyanophenyl, 1-cyanopiperidinyl, and 1(2H)-cyano-3,6-dihydropyridinyl; more preferably, R¹¹ is 4-cyanophenyl;
R¹² is selected from the group consisting of alkyl, aryl, and heteroaryl; these groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

16. The compound according to any one of claims 1 to 15, wherein the compound is of formula (V): 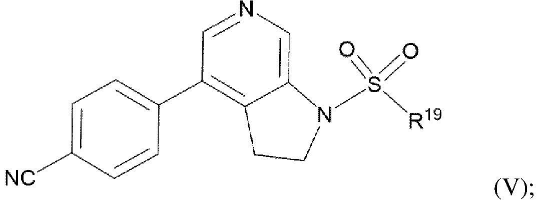 wherein:
R¹⁹ is selected from aryl and heteroaryl; these groups are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, the aryl is selected from phenyl and naphthyl, and the heteroaryl is selected from quinolinyl, thiophenyl, pyridinyl, furanyl, indazolyl, imidazopyridinyl, benzothiazolyl, indolyl, benzofuranyl, benzothiophenyl, pyrrolopyridinyl, pyrrolopyrimidinyl, pyrrolopiperidinyl, imidazopyrimidinyl, imidazopiperidinyl, furanopyridinyl, and thienopyridinyl.

17. The compound according to any one of claims 1 to 16, wherein the compound is of formula (**VI**): 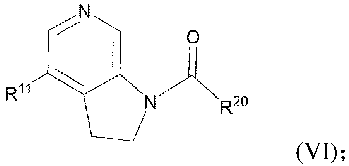 wherein R¹¹ is as defined in claim 15; R²⁰ is selected from alkyl, aryl, and heteroaryl; these groups are optionally substituted with one or more substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, boronate ester, cycloalkyl and alkyl optionally substituted with one to three halogens; preferably, the aryl group is phenyl, and the heteroaryl group is selected from quinolinyl, thienyl, pyridyl, furanyl, indazolyl, imidazopyridyl, benzothiazolyl, indolyl, benzofuranyl, benzothienyl, pyrrolopyridinyl, pyrrolopyrimidinyl, pyrrolidinopyridinyl, imidazopyrimidinyl, imidazopiperidinyl, furopyridinyl, and thienopyridinyl.

18. The compound according to any one of claims 1 to 17, wherein the compound is of formula (**VII**): 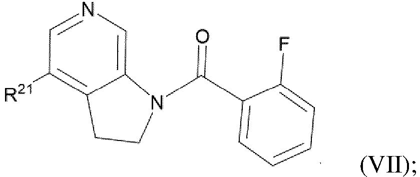 wherein R²¹ is selected from aryl and heteroaryl; said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

19. The compound according to any one of claims 1 to 18, wherein the compound is of formula (**VIII**): 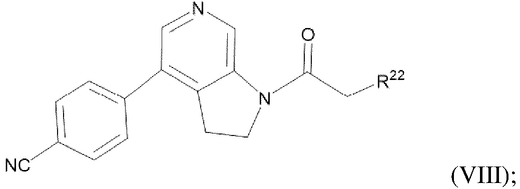 wherein R²² is aryl; said aryl is optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, the aryl is phenyl.

20. The compound according to any one of claims 1 to 19, wherein:
n is 0;
m is 0;
X is C;
L¹ is selected from the group consisting of -SO₂- and -CO-;
- - - is a double bond;
R¹¹ is selected from the group consisting of aryl and heteroaryl; said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens; preferably, R¹¹ is selected from cyanophenyl; more preferably, R¹¹ is 4-cyanophenyl;
R¹² is selected from the group consisting of aryl and heteroaryl; said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

21. The compound according to any one of claims 1 to 20, wherein the compound is of formula (**IX**)**:** 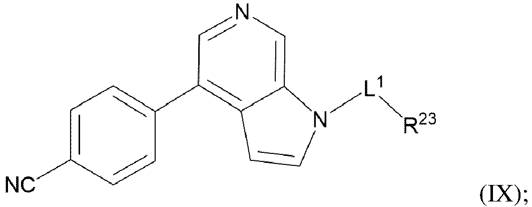 wherein:
L¹ is selected from the group consisting of -SO₂-, and -CO-;
R²³ is selected from aryl and heteroaryl; these groups are optionally substituted with one or more substituents selected from halogen, cyano, acyl, nitro, alkoxy, alkoxycarbonyl, amino, sulfonyl, carboxyl, sulfoximino, trifluoromethyl, hydroxyl, amido, ureido, thioether, sulfonyl fluoride, boronate ester, cycloalkyl and alkyl optionally substituted with one to three halogens.

22. The compound according to any one of claims 1 to 21, wherein the compound is of formula (**X**): 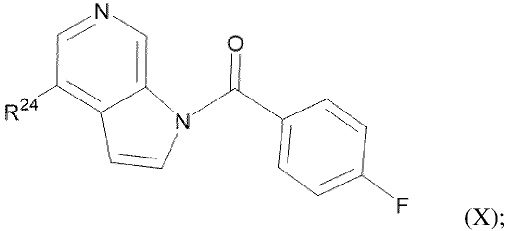 wherein R²⁴ is selected from aryl and heteroaryl; said aryl and heteroaryl are optionally substituted with one or more substituents selected from halogen, cyano, acyl, nitro, alkoxy, alkoxycarbonyl, amino, sulfonyl, carboxy, sulfoximine, trifluoromethyl, hydroxyl, amide, urea, thioether, sulfuryl fluoride, boronate ester, and alkyl optionally substituted with one to three halogens.

23. The compound according to any one of claims 1 to 22, wherein the compound is of formula (**XV**): 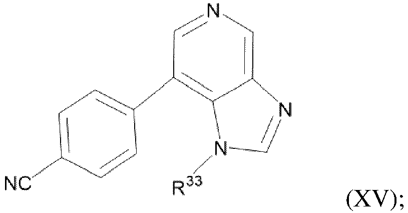 wherein R³³ is selected from hydrocarbyl and cyclohydrocarbyl.

24. The compound according to any one of claims 1 to 23, wherein the compound is of formula (**XVI**): 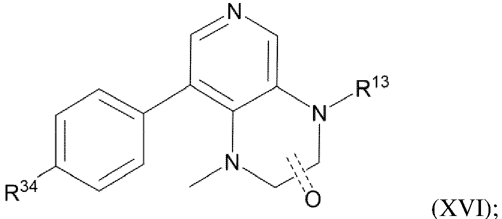
wherein R³⁴ is selected from cyano and halogen;
R¹³ is defined as in claim 1;
:::::: O means that one or more carbon atoms are replaced by carbonyl groups or not.

25. The compound according to any one of claims 1 to 24, selected from any of the following compounds:
4-(4-(phenylsulfonyl)-3,4-dihydro-2*H*-pyrido[4,3-b][1,4]oxazin-8-yl)benzonitrile (O1);
4-(4-((4-fluorophenyl)sulfonyl)-3,4-dihydro-2*H*-pyrido[4,3-b][1,4]oxazin-8-yl)benzonitrile (O2);
4-(4-((3-fluorophenyl)sulfonyl)-3,4-dihydro-2*H*-pyrido[4,3-b][1,4]oxazin-8-yl)benzonitrile (O3);
4-(4-tosyl-3,4-dihydro-2*H*-pyrido[4,3-b][1,4]oxazin-8-yl)benzonitrile (O4);
4-(4-(m-tolylsulfonyl)-3,4-dihydro-2*H*-pyrido[4,3-b][1,4]oxazin-8-yl)benzonitrile (O5);
4-(4-((4-chlorophenyl)sulfonyl)-3,4-dihydro-2*H*-pyrido[4,3-b][1,4]oxazin-8-yl)benzonitrile (O6);
4-(4-((3-chlorophenyl)sulfonyl)-3,4-dihydro-2*H*-pyrido[4,3-b][1,4]oxazin-8-yl)benzonitrile (O7);
4-(4-((4-methoxyphenyl)sulfonyl)-3,4-dihydro-2*H*-pyrido[4,3-b][1,4]oxazin-8-yl)benzonitrile (O8);
4-(4-((4-nitrophenyl)sulfonyl)-3,4-dihydro-2*H*-pyrido[4,3-b][1,4]oxazin-8-yl)benzonitrile (O9);
4-(4-((3-nitrophenyl)sulfonyl)-3,4-dihydro-2*H*-pyrido[4,3-b][1,4]oxazin-8-yl)benzonitrile (O10);
4-(4-((4-(trifluoromethyl)phenyl)sulfonyl)-3,4-dihydro-2*H*-pyrido[4,3-b][1,4]oxazin-8-yl)-benzonitrile (O11);
4-(4-((3-(trifluoromethyl)phenyl)sulfonyl)-3,4-dihydro-2*H*-pyrido[4,3-b][1,4]oxazin-8-yl)-benzonitrile (012);
4-(4-(pyridin-3-ylsulfonyl)-3,4-dihydro-2*H*-pyrido[4,3-b][1,4]oxazin-8-yl)benzonitrile (013);
4-(4-(methylsulfonyl)-3,4-dihydro-2*H*-pyrido[4,3-b][1,4]oxazin-8-yl)benzonitrile (014);
4-(4-(quinolin-8-ylsulfonyl)-3,4-dihydro-2*H*-pyrido[4,3-b][1,4]oxazin-8-yl)benzonitrile (O15);
4-(4-(thiophen-2-ylsulfonyl)-3,4-dihydro-2*H*-pyrido[4,3-b][1,4]oxazin-8-yl)benzonitrile(O16);
4-(4-(naphthalen-1-ylsulfonyl)-3,4-dihydro-2*H*-pyrido[4,3-b][1,4]oxazin-8-yl)benzonitrile (017);
4-(4-(naphthalen-2-ylsulfonyl)-3,4-dihydro-2*H*-pyrido[4,3-b][1,4]oxazin-8-yl)benzonitrile (018);
4-(4-(4-fluorobenzoyl)-3,4-dihydro-2*H*-pyrido[4,3-b][1,4]oxazin-8-yl)benzonitrile (019);
4-(4-(3-fluorobenzoyl)-3,4-dihydro-2*H*-pyrido[4,3-b][1,4]oxazin-8-yl)benzonitrile (O20);
4-(4-(2-fluorobenzoyl)-3,4-dihydro-2*H*-pyrido[4,3-b][1,4]oxazin-8-yl)benzonitrile (O21);
4-(4-(4-methoxybenzoyl)-3,4-dihydro-2*H*-pyrido[4,3-b][1,4]oxazin-8-yl)benzonitrile (O22);
4-(4-(3-methoxybenzoyl)-3,4-dihydro-2*H*-pyrido[4,3-b][1,4]oxazin-8-yl)benzonitrile (O23);
4-(4-(4-(trifluoromethyl)benzoyl)-3,4-dihydro-2*H*-pyrido[4,3-b][1,4]oxazin-8-yl)benzonitrile (O24);
4-(4-(3-(trifluoromethyl)benzoyl)-3,4-dihydro-2*H*-pyrido[4,3-b][1,4]oxazin-8-yl)benzonitrile (O25);
4-(4-(4-nitrobenzoyl)-3,4-dihydro-2*H*-pyrido[4,3-b][1,4]oxazin-8-yl)benzonitrile (O26);
4-(4-(3-nitrobenzoyl)-3,4-dihydro-2*H*-pyrido[4,3-b][1,4]oxazin-8-yl)benzonitrile (O27);
4-(4-(4-chlorobenzoyl)-3,4-dihydro-2*H*-pyrido[4,3-b][1,4]oxazin-8-yl)benzonitrile (O28);
4-(4-(3-chlorobenzoyl)-3,4-dihydro-2*H*-pyrido[4,3-b][1,4]oxazin-8-yl)benzonitrile (O29);
4-(4-nicotinoyl -3,4-dihydro-2*H*-pyrido[4,3-b][1,4]oxazin-8-yl)benzonitrile (O30);
4-(4-(furan-2-carbonyl)-3,4-dihydro-2*H*-pyrido[4,3-b][1,4]oxazin-8-yl)benzonitrile (O31);
4-(4-(2-cyanoacetyl)-3,4-dihydro-2*H*-pyrido[4,3-b][1,4]oxazin-8-yl)benzonitrile (O32);
4-(4-(2-chloroacetyl)-3,4-dihydro-2*H*-pyrido[4,3-b][1,4]oxazin-8-yl)benzonitrile (O33);
4-(4-(2-bromoacetyl)-3,4-dihydro-2*H*-pyrido[4,3-b][1,4]oxazin-8-yl)benzonitrile (O34);
4-(4-(2,2,2-trifluoroacetyl)-3,4-dihydro-2*H*-pyrido[4,3-b][1,4]oxazin-8-yl)benzonitrile (O35);
4-(4-(2-(4-fluorophenyl)acetyl)-3,4-dihydro-2*H*-pyrido[4,3-b][1,4]oxazin-8-yl)benzonitrile (O36);
4-(4-(2-(3-fluorophenyl)acetyl)-3,4-dihydro-2*H*-pyrido[4,3-b][1,4]oxazin-8-yl)benzonitrile (O37);
4-(4-(2-(4-chlorophenyl)acetyl)-3,4-dihydro-2*H*-pyrido[4,3-b][1,4]oxazin-8-yl)benzonitrile (O38);
4-(4-(2-(3-chlorophenyl)acetyl)-3,4-dihydro-2*H*-pyrido[4,3-b][1,4]oxazin-8-yl)benzonitrile (O39);
4-(4-(2-(4-methoxyphenyl)acetyl)-3,4-dihydro-2*H*-pyrido[4,3-b][1,4]oxazin-8-yl)benzonitrile (O40);
4-(4-(2-(3-methoxyphenyl)acetyl)-3,4-dihydro-2*H*-pyrido[4,3-b][1,4]oxazin-8-yl)benzonitrile (O41);
4-(4-(2-(p-tolyl)acetyl)-3,4-dihydro-2*H*-pyrido[4,3-b][1,4]oxazin-8-yl)benzonitrile (O42);
4-(4-(2-(m-tolyl)acetyl)-3,4-dihydro-2*H*-pyrido[4,3-b][1,4]oxazin-8-yl)benzonitrile (O43);
4-(4-(2-(o-tolyl)acetyl)-3,4-dihydro-2*H*-pyrido[4,3-b][1,4]oxazin-8-yl)benzonitrile (O44);
4-(4-(2-(4-(trifluoromethyl)phenyl)acetyl)-3,4-dihydro-2*H*-pyrido[4,3-b][1,4]oxazin-8-yl)-benzonitrile (O45);
4-(4-(2-(3-(trifluoromethyl)phenyl)acetyl)-3,4-dihydro-2H-pyrido[4,3-b][1,4]oxazin-8-yl)-benzonitrile (O46);
4-(4-(3-(4-fluorophenyl)propanoyl)-3,4-dihydro-2*H*-pyrido[4,3-b][1,4]oxazin-8-yl)benzonitrile (O47);
4-(4-(3-(3-fluorophenyl)propanoyl)-3,4-dihydro-2*H*-pyrido[4,3-b][1,4]oxazin-8-yl)benzonitrile (O48);
4-(4-(3-(4-chlorophenyl)propanoyl)-3,4-dihydro-2*H*-pyrido[4,3-b] [1,4]oxazin-8-yl)benzonitrile (O49);
4-(4-(3-(3-chlorophenyl)propanoyl)-3,4-dihydro-2*H*-pyrido[4,3-b] [1,4]oxazin-8-yl)benzonitrile (O50);
4-(4-(3-(4-methoxyphenyl)propanoyl)-3,4-dihydro-2*H-*pyrido[4,3-b][1,4]oxazin-8-yl)benzonitrile (O51);
4-(4-(3-(3-methoxyphenyl)propanoyl)-3,4-dihydro-2*H*-pyrido[4,3-b][1,4]oxazin-8-yl)benzonitrile (O52);
4-(4-(3-(4-(trifluoromethyl)phenyl)propanoyl)-3,4-dihydro-2*H*-pyrido[4,3-b][1,4]oxazin-8-yl)-benzonitrile (O53);
4-(4-(3-(3-(trifluoromethyl)phenyl)-3,4-dihydro-2*H*-pyrido[4,3-b][1,4]oxazin-8-yl)benzonitrile (O54);
4-(4-(1-((3-fluorophenyl)sulfonyl)piperidine-4-carbonyl)-3,4-dihydro-2*H*-pyrido[4,3-b][1,4]oxazin-8-yl)benzonitrile (O55);
4-(4-(azetidine-3-carbonyl)-3,4-dihydro-2*H*-pyrido[4,3-b][1,4]oxazin-8-yl)benzonitrile (O56);
4-(4-(1-(4-fluorobenzoyl)azetidine-3-carbonyl)-3,4-dihydro-2*H*-pyrido[4,3-b][1,4]oxazin-8-yl)-benzonitrile (O57);
4-(4-(1-(3-fluorobenzoyl)azetidine-3-carbonyl)-3,4-dihydro-2*H*-pyrido[4,3-b][1,4]oxazin-8-yl)-benzonitrile(O58);
N-(2-(8-(4-cyanophenyl)-2,3-dihydro-4*H*-pyrido[4,3-b][1,4]oxazin-4-yl)-2-oxoethyl)-4-fluoro-benzamide (O59);
4-(4-(1-((4-fluorophenyl)sulfonyl)azetidine-3-carbonyl)-3,4-dihydro-2*H*-pyrido[4,3-b][1,4]oxazin-8 -yl)benzonitrile (O60);
4-(4-(1-((3-fluorophenyl)sulfonyl)azetidine-3-carbonyl)-3,4-dihydro-2*H*-pyrido[4,3-b][1,4]oxazin-8 -yl)benzonitrile (061);
4-(4-(1-((4-nitrophenyl)sulfonyl)azetidine-3-carbonyl)-3,4-dihydro-2*H*-pyrido[4,3-b][1,4]oxazin-8-yl)benzonitrile (O62);
4-(4-(1-((3-nitrophenyl)sulfonyl)azetidine-3-carbonyl)-3,4-dihydro-2*H*-pyrido[4,3-b][1,4]oxazin-8-yl)benzonitrile (O63);
4-(4-(1-(thiophen-2-ylsulfonyl)azetidine-3-carbonyl)-3,4-dihydro-2*H*-pyrido[4,3-b][1,4]oxazin-8-yl )benzonitrile (O64);
4-(4-(1-tosylazetidine-3-carbonyl)-3,4-dihydro-2*H*-pyrido[4,3-b][1,4]oxazin-8-yl)benzonitrile (O65);
4-(4-(1-((3-methoxyphenyl)sulfonyl)azetidine-3-carbonyl)-3,4-dihydro-2*H*-pyrido[4,3-b][1,4]-oxazin-8-yl)benzonitrile (O66);
4-(4-(1-((4-methoxyphenyl)sulfonyl)azetidine-3-carbonyl)-3,4-dihydro-2*H*-pyrido[4,3-b][1,4]-oxazin-8-yl)benzonitrile (O67);
3-((3-(8-(4-cyanophenyl)-3,4-dihydro-2*H*-pyrido[4,3-b][1,4]oxazine-4-carbonyl)azetidin-1-yl)-sulfonyl)benzonitrile (O68);
4-((3-(8-(4-cyanophenyl)-3,4-dihydro-2*H*-pyrido[4,3-b][1,4]oxazine-4-carbonyl)azetidin-1-yl)-sulfonyl)benzonitrile (O69);
4-(4-(1-(m-tolylsulfonyl)azetidine-3-carbonyl)-3,4-dihydro-2*H*-pyrido[4,3-b][1,4]oxazin-8-yl)-benzonitrile (O70);
4-(4-(3-((3-fluorophenyl)amino)bicyclo[1.1.1]pentane-1-carbonyl)-3,4-dihydro-2H-pyrido[4,3-b] [1, 4]oxazin-8-yl)benzonitrile (O71);
4-(4-(5-(4-fluorophenoxy)thiophene-2-carbonyl)-3,4-dihydro-2*H*-pyrido[4,3-b][1,4]oxazin-8-yl)-benzonitrile (O72);
4-(4-(6-(3-fluorobenzyl)-2,6-diazaspiro[3.3]heptane-2-carbonyl)-3,4-dihydro-2H-pyrido[4,3-b] [1,4] oxazin-8-yl)benzonitrile (O73);
3-((3-(8-(4-cyanophenyl)-3,4-dihydro-2*H*-pyrido[4,3-b][1,4]oxazine-4-carbonyl)azetidin-1-yl)-sulfonyl)benzonitrile (O74);
4-((3-(8-(4-cyanophenyl)-3,4-dihydro-2*H*-pyrido[4,3-b][1,4]oxazine-4-carbonyl)azetidin-1-yl)-sulfonyl)benzonitrile (O75);
4-(4-(1-(3-methoxybenzoyl)azetidine-3-carbonyl)-3,4-dihydro-2*H*-pyrido[4,3-b][1,4]oxazin-8-yl)-benzonitrile (O76);
4-(4-(1-(4-methoxybenzoyl)azetidine-3-carbonyl)-3,4-dihydro-2*H*-pyrido[4,3-b][1,4]oxazin-8-yl)-benzonitrile (O77);
4-(4-(1-(3-fluorobenzyl)azetidine-3-carbonyl)-3,4-dihydro-2*H*-pyrido[4,3-b][1,4]oxazin-8-yl)-benzonitrile (O78);
4-(4-(1-(4-fluorobenzyl)azetidine-3-carbonyl)-3,4-dihydro-2*H*-pyrido[4,3-b][1,4]oxazin-8-yl)-benzonitrile (O79);
3-((3-(8-(4-cyanophenyl)-3,4-dihydro-2*H*-pyrido[4,3-b][1,4]oxazine-4-carbonyl)azetidin-1-yl)-methyl)benzonitrile (O80);
4-(4-(1-(4-cyanobenzyl)azetidine-3-carbonyl)-3,4-dihydro-2*H*-pyrido[4,3-b][1,4]oxazin-8-yl)-benzonitrile (O81);
4-(4-(1-(3-methoxybenzyl)azetidine-3-carbonyl)-3,4-dihydro-2*H*-pyrido[4,3-b][1,4]oxazin-8-yl)-benzonitrile (O82);
4-(4-(1-(4-methoxybenzyl)azetidine-3-carbonyl)-3,4-dihydro-2*H*-pyrido[4,3-b][1,4]oxazin-8-yl)-benzonitrile (O83);
4-(4-(1-(3-fluorophenyl)azetidine-3-carbonyl)-3,4-dihydro-2*H*-pyrido[4,3-b][1,4]oxazin-8-yl)-benzonitrile (O84);
4-(4-(1-(4-fluorophenyl)azetidine-3-carbonyl)-3,4-dihydro-2*H*-pyrido[4,3-b][1,4]oxazin-8-yl)-benzonitrile (O85);
3-(3-(8-(4-cyanophenyl)-3,4-dihydro-2*H*-pyrido[4,3-b][1,4]oxazine-4-carbonyl)azetidin-1-yl)-benzonitrile (O86);
4-(3-(8-(4-cyanophenyl)-3,4-dihydro-2*H*-pyrido[4,3-b][1,4]oxazine-4-carbonyl)azetidin-1-yl)-benzonitrile (O87);
(8-(benzo[d]thiazol-6-yl)-2,3-dihydro-4*H*-pyrido[4,3-b][1,4]oxazin-4-yl)(1-(3-fluorobenzyl)-azetidi n-3-yl)methanone (O88);
5-(4-(1-(3-fluorobenzyl)azetidine-3-carbonyl)-3,4-dihydro-2*H*-pyrido[4,3-b][1,4]oxazin-8-yl)-indolin-2-one (O89);
(1-(3-fluorobenzyl)azetidin-3-yl)(8-(imidazo[1,2-a]pyridin-6-yl)-2,3-dihydro-4*H*-pyrido[4,3-b][1,4] oxazin-4-yl)methanone (O90);
(8-(6-chloro-1*H-*indol-2-yl)-2,3-dihydro-4H-pyrido[4,3-b][1,4]oxazin-4-yl)(1-(3-fluorobenzyl)-azetidin-3-yl)methanone (O91);
2-(4-(1-(3-fluorobenzyl)azetidine-3-carbonyl)-3,4-dihydro-2H-pyrido[4,3-b][1,4]oxazin-8-yl)-1*H-*indole-6-carbonitrile (O92);
(8-(6-fluoro-1*H*-indol-2-yl)-2,3-dihydro-4*H*-pyrido[4,3-b][1,4]oxazin-4-yl)(1-(3-fluorobenzyl)-azetidin-3-yl)methanone (O93);
2-(4-(1-(3-fluorobenzyl)azetidine-3-carbonyl)-3,4-dihydro-2*H*-pyrido[4,3-b][1,4]oxazin-8-yl)-1*H-*indole-5-carbonitrile (O94);
(8-(5-chlorobenzofuran-2-yl)-2,3-dihydro-4*H*-pyrido[4,3-b][1,4]oxazin-4-yl)(1-(3-fluorobenzyl)-azetidin-3-yl)methanone (O95);
2-(4-(1-(3-fluorobenzyl)azetidine-3-carbonyl)-3,4-dihydro-2*H*-pyrido[4,3-b][1,4]oxazin-8-yl)-benzofuran-5-carbonitrile (O96);
4-(4-(1-(3-fluorobenzyl)azetidine-3-carbonyl)-3,4-dihydro-2H-pyrido[4,3-b][1,4]oxazin-8-yl)-3,6-dihydropyridine-1(2H)-carbonitrile (O97);
4-(4-(1-(3-fluorobenzyl)azetidine-3-carbonyl)-3,4-dihydro-2H-pyrido[4,3-b][1,4]oxazin-8-yl)-piperazine-1-carbonitrile (O98);
4-(4-(1-(3-fluorobenzyl)azetidine-3-carbonyl)-3,4-dihydro-2H-pyrido[4,3-b][1,4]oxazin-8-yl)-3-oxopiperazine-1-carbonitrile (O99);
4-(4-((3-(3-methoxyphenyl)-4,5-dihydroisoxazol-5-yl)sulfonyl)-3,4-dihydro-2H-pyrido[4,3-b] [1,4] oxazin-8-yl)benzonitrile (O100);
3-(5-((8-(4-cyanophenyl)-2,3-dihydro-4*H*-pyrido[4,3-b][1,4]oxazin-4-yl)sulfonyl)-4,5-dihydro-isoxazol-3-yl)benzonitrile (O101);
4-(4-((3-phenyl-4,5-dihydroisoxazol-5-yl)sulfonyl)-3,4-dihydro-2*H*-pyrido[4,3-b][1,4]oxazin-8-yl)-benzonitrile (0102);
4-(4-((3-benzyl-4,5-dihydroisoxazol-5-yl)sulfonyl)-3,4-dihydro-2*H*-pyrido[4,3-b][1,4]oxazin-8-yl)-benzonitrile (0103);
(S)-4-(4-(1-(3-fluorobenzyl)pyrrolidine-3-carbonyl)-3,4-dihydro-2*H*-pyrido[4,3-b][1,4]oxazin-8-yl) benzonitrile (0104);
(R)-4-(4-(1-(3-fluorobenzyl)pyrrolidine-3-carbonyl)-3,4-dihydro-2*H*-pyrido[4,3-b][1,4]oxazin-8-yl) benzonitrile (0105);
2-(4-((S)-1-(3-fluorobenzyl)pyrrolidine-3-carbonyl)-3,4-dihydro-2*H*-pyrido[4,3-b][1,4]oxazin-8-yl) -2,3-dihydrobenzofuran-5-carbonitrile (0106);
2-(4-((R)-1-(3-fluorobenzyl)pyrrolidine-3-carbonyl)-3,4-dihydro-2*H*-pyrido[4,3-b][1,4]oxazin-8-yl) -2,3-dihydrobenzofuran-5-carbonitrile (0107);
8-(5-chlorobenzofuran-2-yl)-3,4-dihydro-2*H*-pyrido[4,3-b][1,4]oxazine (0108);
2-(3,4-dihydro-2H-pyrido[4,3-b][1,4]oxazin-8-yl)benzofuran-5-carbonitrile (0109);
4-(4-((1-(3-cyanobenzoyl)azetidin-3-yl)sulfonyl)-3,4-dihydro-2*H*-pyrido[4,3-b][1,4]oxazin-8-yl)-benzonitrile (O110);
4-(4-((1-(4-cyanobenzoyl)azetidin-3-yl)sulfonyl)-3,4-dihydro-2H-pyrido[4,3-b] [1,4]oxazin-8-yl)-b enzonitrile (O111);
4-(4-((1-(3-methoxybenzoyl)azetidin-3-yl)sulfonyl)-3,4-dihydro-2*H*-pyrido[4,3-b][1,4]oxazin-8-yl) benzonitrile (0112);
4-(4-((1-(4-methoxybenzoyl)azetidin-3-yl)sulfonyl)-3,4-dihydro-2*H*-pyrido[4,3-b][1,4]oxazin-8-yl) benzonitrile (0113);
4-((3-((8-(4-cyanophenyl)-2,3-dihydro-4*H*-pyrido[4,3-b] [1,4]oxazin-4-yl)sulfonyl)azetidin-1-yl)-sulfonyl)benzonitrile (0114);
3-((3-((8-(4-cyanophenyl)-2,3-dihydro-4*H*-pyrido[4,3-b][1,4]oxazin-4-yl)sulfonyl)azetidin-1-yl)-sulfonyl)benzonitrile (0115);
4-(4-((1-((4-fluorophenyl)sulfonyl)azetidin-3-yl)sulfonyl)-3,4-dihydro-2*H*-pyrido[4,3-b][1,4]-oxazin-8-yl)benzonitrile (0116);
4-(4-((1-((3-fluorophenyl)sulfonyl)azetidin-3-yl)sulfonyl)-3,4-dihydro-2*H*-pyrido[4,3-b][1,4]-oxazin-8-yl)benzonitrile (0117);
4-(4-((1-((4-methoxyphenyl)sulfonyl)azetidin-3-yl)sulfonyl)-3,4-dihydro-2*H*-pyrido[4,3-b][1,4]-oxazin-8-yl)benzonitrile (0118);
4-(4-((1-((3-methoxyphenyl)sulfonyl)azetidin-3-yl)sulfonyl)-3,4-dihydro-2*H*-pyrido[4,3-b][1,4]-oxazin-8-yl)benzonitrile (0119);
3-((3-((8-(4-cyanophenyl)-2,3-dihydro-4*H*-pyrido[4,3-b][1,4]oxazin-4-yl)sulfonyl)azetidin-1-yl)-methyl)benzonitrile (0120);
4-(4-((1-(4-cyanobenzyl)azetidin-3-yl)sulfonyl)-3,4-dihydro-2*H*-pyrido[4,3-b][1,4]oxazin-8-yl)-benzonitrile (0121);
4-(4-((1-(3-fluorobenzyl)azetidin-3-yl)sulfonyl)-3,4-dihydro-2*H*-pyrido[4,3-b][1,4]oxazin-8-yl)-benzonitrile (0122);
4-(4-((1-(4-fluorobenzyl)azetidin-3-yl)sulfonyl)-3,4-dihydro-2*H*-pyrido[4,3-b][1,4]oxazin-8-yl)-benzonitrile (0123);
4-(4-((1-(3-methoxybenzyl)azetidin-3-yl)sulfonyl)-3,4-dihydro-2*H*-pyrido[4,3-b][1,4]oxazin-8-yl)-benzonitrile (0124);
4-(4-((1-(4-methoxybenzyl)azetidin-3-yl)sulfonyl)-3,4-dihydro-2*H*-pyrido[4,3-b][1,4]oxazin-8-yl)-benzonitrile (0125);
4-(4-((1-(3-fluorophenyl)azetidin-3-yl)sulfonyl)-3,4-dihydro-2*H*-pyrido[4,3-b] [1,4]oxazin-8-yl)-benzonitrile (0126);
4-(4-((1-(4-fluorophenyl)azetidin-3-yl)sulfonyl)-3,4-dihydro-2*H*-pyrido[4,3-b] [1,4]oxazin-8-yl)-benzonitrile (0127);
4-((8-(5-chlorobenzofuran-2-yl)-2,3-dihydro-4*H*-pyrido[4,3-b][1,4]oxazin-4-yl)sulfonyl)-benzonitrile (0128);
2-(4-((4-cyanophenyl)sulfonyl)-3,4-dihydro-2*H*-pyrido[4,3-b] [1,4]oxazin-8-yl)benzofuran-5-carbonitril (0129);
4-((8-(4-cyanophenyl)-2,3-dihydro-4*H*-pyrido[4,3-b][1,4]oxazin-4-yl)sulfonyl)benzonitrile (0130);
4-((8-(benzo[d]oxazol-6-yl)-2,3-dihydro-4*H*-pyrido[4,3-b][1,4]oxazin-4-yl)sulfonyl)benzonitrile (0131);
4-((8-(2-oxoindolin-6-yl)-2,3-dihydro-4*H*-pyrido[4,3-b][1,4]oxazin-4-yl)sulfonyl)benzonitrile (0132);
3-(4-(1-(3-Fluorobenzyl)azetidine-3-carbonyl)-3,4-dihydro-2H-pyrido[4,3-b][1,4]oxazin-8-yl)-bicyclo[1.1.1]pentane-1-carbonitrile (0133);
6-(4-(1-(3-Fluorobenzyl)azetidine-3-carbonyl)-3,4-dihydro-2H-pyrido[4,3-b][1,4]oxazin-8-yl)-2,6-diazaspiro[3.3]heptane-2-carbonitrile (0134);
(8-(5,6-Dihydro-[1,2,4]triazolo[4,3-a]pyrazin-7(8H)-yl)-2,3-dihydro-4H-pyrido[4,3-b][1,4]oxazin-4 -yl)(1-(3-fluorobenzyl)azetidin-3-yl)methanone (0135);
4-(4-(3-(3-fluorobenzyl)azetidine-1-carbonyl)-3,4-dihydro-2H-pyrido[4,3-b] [1,4]oxazin-8-yl)-benzonitrile (0136);
(8-(5,6-dihydroimidazo[1,2-a]pyrazin-7(8H)-yl)-2,3-dihydro-4H-pyrido[4,3-b][1,4]oxazin-4-yl)(1-( 3-fluorobenzyl)azetidin-3-yl)methanone (0137);
4-(4-(3-fluorobenzyl)-3,4-dihydro-2*H*-pyrido[4,3-b][1,4]thiazin-8-yl)benzonitrile (S1);
4-(4-(4-fluorobenzyl)-3,4-dihydro-2*H*-pyrido[4,3-b][1,4]thiazin-8-yl)benzonitrile (S2);
4-(4-(3-nitrobenzyl)-3,4-dihydro-2*H*-pyrido[4,3-b][1,4]thiazin-8-yl)benzonitrile (S3);
4-(4-(3-fluorobenzoyl)-3,4-dihydro-2*H*-pyrido[4,3-b][1,4]thiazin-8-yl)benzonitrile (S4);
4-(4-(4-fluorobenzoyl)-3,4-dihydro-2*H*-pyrido[4,3-b][1,4]thiazin-8-yl)benzonitrile (S5);
4-(4-(4-methoxybenzoyl)-3,4-dihydro-2*H*-pyrido[4,3-b][1,4]thiazin-8-yl)benzonitrile (S6);
4-(4-nicotinoyl-3,4-dihydro-2*H*-pyrido[4,3-b][1,4]thiazin-8-yl)benzonitrile (S7);
4-(4-(phenylsulfonyl)-3,4-dihydro-2*H*-pyrido[4,3-b][1,4]thiazin-8-yl)benzonitrile (S8);
4-(4-((3-fluorophenyl)sulfonyl)-3,4-dihydro-2*H*-pyrido[4,3-b][1,4]thiazin-8-yl)benzonitrile (S9);
4-(4-((4-fluorophenyl)sulfonyl)-3,4-dihydro-2*H*-pyrido[4,3-b][1,4]thiazin-8-yl)benzonitrile (S10);
4-(4-(m-tolylsulfonyl)-3,4-dihydro-2*H*-pyrido[4,3-b][1,4]thiazin-8-yl)benzonitrile (S11);
4-(4-tosyl-3,4-dihydro-2*H*-pyrido[4,3-b][1,4]thiazin-8-yl)benzonitrile (S12);
4-(4-((4-methoxyphenyl)sulfonyl)-3,4-dihydro-2*H*-pyrido[4,3-b][1,4]thiazin-8-yl)benzonitrile (S13);
4-(4-((3-chlorophenyl)sulfonyl)-3,4-dihydro-2*H*-pyrido[4,3-b][1,4]thiazin-8-yl)benzonitrile (S14);
4-(4-((4-chlorophenyl)sulfonyl)-3,4-dihydro-2*H*-pyrido[4,3-b][1,4]thiazin-8-yl)benzonitrile (S15);
4-(4-((3-(trifluoromethyl)phenyl)sulfonyl)-3,4-dihydro-2*H*-pyrido[4,3-b][1,4]thiazin-8-yl) benzonitrile (S16);
4-(4-((4-(trifluoromethyl)phenyl)sulfonyl)-3,4-dihydro-2*H*-pyrido[4,3-b] [1,4]thiazin-8-yl)-benzonitrile (S17);
4-(4-((3-nitrophenyl)sulfonyl)-3,4-dihydro-2*H*-pyrido[4,3-b][1,4]thiazin-8-yl)benzonitrile (S18);
4-(4-((4-nitrophenyl)sulfonyl)-3,4-dihydro-2*H*-pyrido[4,3-b][1,4]thiazin-8-yl)benzonitrile (S19);
3-((8-(4-cyanophenyl)-2,3-dihydro-4*H*-pyrido[4,3-b][1,4]thiazin-4-yl)sulfonyl)benzonitrile (S20);
4-((8-(4-cyanophenyl)-2,3-dihydro-4*H*-pyrido[4,3-b][1,4]thiazin-4-yl)sulfonyl)benzonitrile (S21);
4-(4-(naphthalen-1-ylsulfonyl)-3,4-dihydro-2*H*-pyrido[4,3-b][1,4]thiazin-8-yl)benzonitrile (S22);
4-(4-(naphthalen-2-ylsulfonyl)-3,4-dihydro-2*H*-pyrido[4,3-b][1,4]thiazin-8-yl)benzonitrile (S23);
4-(4-(thiophen-2-ylsulfonyl)-3,4-dihydro-2*H*-pyrido[4,3-b][1,4]thiazin-8-yl)benzonitrile (S24);
4-(4-(pyridin-3-ylsulfonyl)-3,4-dihydro-2*H*-pyrido[4,3-b][1,4]thiazin-8-yl)benzonitrile (S25);
4-(4-(quinolin-8-ylsulfonyl)-3,4-dihydro-2*H*-pyrido[4,3-b][1,4]thiazin-8-yl)benzonitrile (S26);
3-(4-((4-methoxyphenyl)sulfonyl)-3,4-dihydro-2*H*-pyrido[4,3-b][1,4]thiazin-8-yl)benzonitrile (S27);
8-(4-chlorophenyl)-4-((4-methoxyphenyl)sulfonyl)-3,4-dihydro-2*H*-pyrido[4,3-b][1,4]thiazine (S28);
8-(3-chlorophenyl)-4-((4-methoxyphenyl)sulfonyl)-3,4-dihydro-2*H*-pyrido[4,3-b][1,4]thiazine (S29);
8-(4-fluorophenyl)-4-((4-methoxyphenyl)sulfonyl)-3,4-dihydro-2*H*-pyrido[4,3-b][1,4]thiazine (S30)
8-(3-fluorophenyl)-4-((4-m;ethoxyphenyl)sulfonyl)-3,4-dihydro-2*H*-pyrido[4,3-b][1,4]thiazine (S31);
4-((4-methoxyphenyl) sulfonyl)-8-(4-(trifluoromethyl)phenyl)-3,4-dihydro-2*H*-pyrido[4,3-b][1,4]thiazine (S32);
8-(4-fluoro-3-methylphenyl)-4-((4-methoxyphenyl)sulfonyl)-3,4-dihydro-2*H*-pyrido[4,3-b][1,4]-thiazine (S33);
8-(3-chloro-4-fluorophenyl)-4-((4-methoxyphenyl)sulfonyl)-3,4-dihydro-2*H*-pyrido[4,3-b] [1,4]-thiazine (S34);
2-fluoro-4-(4-((4-methoxyphenyl)sulfonyl)-3,4-dihydro-2*H*-pyrido[4,3-b][1,4]thiazin-8-yl)-benzonitrile (S35);
4-((4-methoxyphenyl) sulfonyl)-8-(thiophen-3-yl)-3,4-dihydro-2*H*-pyrido[4,3-b][1,4]thiazine (S36);
4-((4-methoxyphenyl) sulfonyl)-8-(thiophen-2-yl)-3,4-dihydro-2*H*-pyrido[4,3-b][1,4]thiazine (S37);
8-(furan-2-yl)-4-((4-methoxyphenyl)sulfonyl)-3,4-dihydro-2*H*-pyrido[4,3-b][1,4]thiazine (S38);
4-((4-methoxyphenyl)sulfonyl)-8-(1*H*-pyrazol-4-yl)-3,4-dihydro-2*H*-pyrido[4,3-b][1,4]thiazine (S39);
4-((8-(benzofuran-2-yl)-2,3-dihydro-4*H*-pyrido[4,3-b][1,4]thiazin-4-yl)sulfonyl)benzonitrile (S40);
4-((8-(5-chlorobenzofuran-2-yl)-2,3-dihydro-4*H*-pyrido[4,3-b][1,4]thiazin-4-yl)sulfonyl)-benzonitrile (S41);
2-(4-((4-cyanophenyl)sulfonyl)-3,4-dihydro-2*H*-pyrido[4,3-b][1,4]thiazin-8-yl)benzofuran-5-carbonitrile (S42);
4-((8-(benzo[*b*]thiophen-2-yl)-2,3-dihydro-4*H*-pyrido[4,3-b][1,4]thiazin-4-yl)sulfonyl)benzonitrile (S43);
4-((8-(benzofuran-5-yl)-2,3-dihydro-4*H*-pyrido[4,3-b][1,4]thiazin-4-yl)sulfonyl)benzonitrile(S44);
4-((8-(1-methyl-1*H*-indazol-6-yl)-2,3-dihydro-4*H*-pyrido[4,3-b][1,4]thiazin-4-yl)sulfonyl)-benzonitrile (S45);
4-((8-(benzo[*d*]thiazol-6-yl)-2,3-dihydro-4*H*-pyrido[4,3-b][1,4]thiazin-4-yl)sulfonyl)benzonitrile (S46);
4-((8-(benzo[*d*]oxazol-6-yl)-2,3-dihydro-4*H*-pyrido[4,3-b][1,4]thiazin-4-yl)sulfonyl)benzonitrile (47);
4-((8-(1*H*-indazol-5-yl)-2,3-dihydro-4*H*-pyrido[4,3-b][1,4]thiazin-4-yl)sulfonyl)benzonitrile (S48);
4-((8-(1*H*-benzo[*d*]imidazol-6-yl)-2,3-dihydro-4*H*-pyrido[4,3-b][1,4]thiazin-4-yl)sulfonyl)-benzonitrile (S49);
4-((8-(imidazo[1,2-a]pyridin-7-yl)-2,3-dihydro-4*H*-pyrido[4,3-b][1,4]thiazin-4-yl)sulfonyl)-benzonitrile (S50);
4-((8-(2-oxoindolin-5-yl)-2,3-dihydro-4*H*-pyrido[4,3-b][1,4]thiazin-4-yl)sulfonyl)benzonitrile (S51);
4-((8-(8-fluoro-1-methyl-2-oxo-1,2,3,4-tetrahydroquinolin-6-yl)-2,3-dihydro-4*H*-pyrido[4,3-b] [1,4] thiazin-4-yl)sulfonyl)benzonitrile (S52);
8-(5-Chlorobenzofuran-2-yl)-4-((6-methoxypyridin-3-yl)sulfonyl)-3,4-dihydro-2*H*-pyrido[4,3-b][1, 4]thiazine (S53);
5-((8-(5-chlorobenzofuran-2-yl)-2,3-dihydro-4H-pyrido[4,3-b][1,4]thiazin-4-yl)sulfonyl)pyridin-2-ol (S54);
8-(5-Chlorobenzofuran-2-yl)-4-((1-methyl-1H-pyrazol-4-yl)sulfonyl)-3,4-dihydro-2H-pyrido[4,3-b] [1,4]thiazine (S55);
8-(5-chlorobenzofuran-2-yl)-4-((1-methyl-1H-pyrazol-3-yl)sulfonyl)-3,4-dihydro-2H-pyrido[4,3-b][ 1,4]thiazine (S56);
N-(5-((8-(5-Chlorobenzofuran-2-yl)-2,3-dihydro-4H-pyrido[4,3-b][1,4]thiazin-4-yl)sulfonyl)-4-methylthiazol-2-yl)acetamide (S57);
1-(5-((8-(5-Chlorobenzofuran-2-yl)-2,3-dihydro-4H-pyrido[4,3-b][1,4]thiazin-4-yl)sulfonyl)indolin -1-yl)ethan-1-one (S58);
6-((8-(5-Chlorobenzofuran-2-yl)-2,3-dihydro-4H-pyrido[4,3-b][1,4]thiazin-4-yl)sulfonyl)-2H-benzo[b][1,4]oxazin-3(4H)-one (S59);
8-(5-Chlorobenzofuran-2-yl)-4-((5-chlorothiophen-2-yl)sulfonyl)-3,4-dihydro-2H-pyrido[4,3-b][1,4 ]thiazine (S60);
8-(5-Chlorobenzofuran-2-yl)-4-(piperidin-4-ylsulfonyl)-3,4-dihydro-2H-pyrido[4,3-b][1,4]thiazine (S61);
4-(Azetidin-3-ylsulfonyl)-8-(5-chlorobenzofuran-2-yl)-3,4-dihydro-2H-pyrido[4,3-b][1,4]thiazine (S62);
4-((8-(5-Chlorobenzofuran-2-yl)-2,3-dihydro-4H-pyrido[4,3-b][1,4]thiazin-4-yl)sulfonyl)piperidine -1-carbonitrile (S63);
3-((8-(5-Chlorobenzofuran-2-yl)-2,3-dihydro-4H-pyrido[4,3-b][1,4]thiazin-4-yl)sulfonyl)azetidine-1-carbonitrile (S64);
3-((8-(5-chlorobenzofuran-2-yl)-2,3-dihydro-4H-pyrido[4,3-b][1,4]thiazin-4-yl)sulfonyl)bicyclo[1.1 .1]pentane-1-carbonitrile (S65);
4-((2-oxaspiro[3.3]heptan-6-yl)sulfonyl)-8-(5-chlorobenzofuran-2-yl)-3,4-dihydro-2H-pyrido[4,3-b][1,4]thiazine (S66);
6-((8-(5-chlorobenzofuran-2-yl)-2,3-dihydro-4H-pyrido[4,3-b][1,4]thiazin-4-yl)sulfonyl)-2-azaspiro [3.3]heptane-2-carbonitrile (S67);
4-(4-((1-(4-fluorobenzoyl)azetidin-3-yl)sulfonyl)-3,4-dihydro-2*H*-pyrido[4,3-b][1,4]thiazin-8-yl)-benzonitrile (S68);
4-(4-((1-(3-fluorobenzoyl)azetidin-3-yl)sulfonyl)-3,4-dihydro-2*H*-pyrido[4,3-b][1,4]thiazin-8-yl)-benzonitrile (S69);
4-(4-((1-(4-cyanobenzoyl)azetidin-3-yl)sulfonyl)-3,4-dihydro-2*H*-pyrido[4,3-b] [1,4]thiazin-8-yl)-benzonitrile(S70);
3-(3-((8-(4-cyanophenyl)-2,3-dihydro-4H-pyrido[4,3-b] [1,4]thiazin-4-yl)sulfonyl)azetidine-1-carbonyl)benzonitrile (S71);
4-(4-((1-(4-methoxybenzoyl)azetidin-3-yl)sulfonyl)-3,4-dihydro-2*H*-pyrido[4,3-b][1,4]thiazin-8-yl) benzonitrile (S72);
4-(4-((1-(3-methoxybenzoyl)azetidin-3-yl)sulfonyl)-3,4-dihydro-2*H*-pyrido[4,3-b][1,4]thiazin-8-yl) benzonitrile (S73);
4-(4-((1-((4-fluorophenyl)sulfonyl)azetidin-3-yl)sulfonyl)-3,4-dihydro-2*H*-pyrido[4,3-b][1,4]thiazin -8-yl)benzonitrile (S74);
4-(4-((1-((3-fluorophenyl)sulfonyl)azetidin-3-yl)sulfonyl)-3,4-dihydro-2*H*-pyrido[4,3-b][1,4]thiazin -8-yl)benzonitrile (S75);
4-((3-((8-(4-cyanophenyl)-2,3-dihydro-4*H*-pyrido[4,3-b][1,4]thiazin-4-yl)sulfonyl)azetidin-1-yl)-sulfonyl)benzonitrile (S76);
3-((3-((8-(4-cyanophenyl)-2,3-dihydro-4*H*-pyrido[4,3-b][1,4]thiazin-4-yl)sulfonyl)azetidin-1-yl)-sulfonyl)benzonitrile (S77);
4-(4-((1-((4-methoxyphenyl)sulfonyl)azetidin-3-yl)sulfonyl)-3,4-dihydro-2*H*-pyrido[4,3-b][1,4]-thiazin-8-yl)benzonitrile (S78);
4-(4-((1-((3-methoxyphenyl)sulfonyl)azetidin-3-yl)sulfonyl)-3,4-dihydro-2*H*-pyrido[4,3-b] [1,4]-thiazin-8-yl)benzonitrile (S79);
4-(4-((1-(4-cyanobenzyl)azetidin-3-yl)sulfonyl)-3,4-dihydro-2*H*-pyrido[4,3-b][1,4] thiazin-8-yl)-benzonitrile (S80);
3-((3-((8-(4-cyanophenyl)-2,3-dihydro-4*H*-pyrido[4,3-b][1,4]thiazin-4-yl)sulfonyl)azetidin-1-yl)-methyl)benzonitrile (S81);
4-(4-((1-(4-fluorobenzyl)azetidin-3-yl)sulfonyl)-3,4-dihydro-2*H*-pyrido[4,3-b][1,4]thiazin-8-yl)-benzonitrile (S82);
4-(4-((1-(3-fluorobenzyl)azetidin-3-yl)sulfonyl)-3,4-dihydro-2*H*-pyrido[4,3-b][1,4]thiazin-8-yl)-benzonitrile (S83);
4-(4-((1-(4-methoxybenzyl)azetidin-3-yl)sulfonyl)-3,4-dihydro-2*H*-pyrido[4,3-b][1,4]thiazin-8-yl)-benzonitrile (S84);
4-(4-((1-(3-methoxybenzyl)azetidin-3-yl)sulfonyl)-3,4-dihydro-2*H*-pyrido[4,3-b][1,4]thiazin-8-yl)-benzonitrile (S85);
*N*-(2-(8-(4-cyanophenyl)-3,4-dihydro-2*H*-pyrido[4,3-b][1,4]thiazine-4-carbonyl)phenyl)acetamide (S86);
4-(4-(3-(trifluoromethyl)benzoyl)-3,4-dihydro-2*H*-pyrido[4,3-b][1,4]thiazin-8-yl)benzonitrile (S87);
4-(4-(4-(trifluoromethyl)benzoyl)-3,4-dihydro-2*H*-pyrido[4,3-b][1,4]thiazin-8-yl)benzonitrile (S88);
4-(4-(4-methylbenzoyl)-3,4-dihydro-2*H*-pyrido[4,3-b][1,4]thiazin-8-yl)benzonitrile (S89);
4-(4-(2-methylbenzoyl)-3,4-dihydro-2*H*-pyrido[4,3-b][1,4]thiazin-8-yl)benzonitrile (S90);
4-(4-(3-methylbenzoyl)-3,4-dihydro-2*H*-pyrido[4,3-b][1,4]thiazin-8-yl)benzonitrile (S91);
4-(4-(4-chlorobenzoyl)-3,4-dihydro-2*H*-pyrido[4,3-b][1,4]thiazin-8-yl)benzonitrile (S92);
4-(4-(3-chlorobenzoyl)-3,4-dihydro-2*H*-pyrido[4,3-b][1,4]thiazin-8-yl)benzonitrile (S93);
4-(4-(4-cyanobenzoyl)-3,4-dihydro-2*H*-pyrido[4,3-b][1,4]thiazin-8-yl)benzonitrile (S94);
3-(8-(4-cyanophenyl)-3,4-dihydro-2*H*-pyrido[4,3-b][1,4]thiazine-4-carbonyl)benzonitrile (S95);
4-(4-(3-methoxybenzoyl)-3,4-dihydro-2*H*-pyrido[4,3-b][1,4]thiazin-8-yl)benzonitrile (S96);
4-(4-(4-methoxybenzoyl)-3,4-dihydro-2*H*-pyrido[4,3-b][1,4]thiazin-8-yl)benzonitrile (S97);
2-(4-(4-methoxybenzoyl)-3,4-dihydro-2*H*-pyrido[4,3-b][1,4]thiazin-8-yl)benzofuran-5-carbonitrile (S98);
4-(3-oxo-3,4-dihydro-2H-pyrido[4,3-b][1,4]thiazin-8-yl)benzonitrile (S99);
4-(4-(4-chlorobenzoyl)-3-oxo-3,4-dihydro-2H-pyrido[4,3-b][1,4]thiazin-8-yl)benzonitrile (S100);
1-((8-(5-Chlorobenzofuran-2-yl)-2,3-dihydro-4H-pyrido[4,3-b][1,4]thiazin-4-yl)sulfonyl)azetidine-3-carbonitrile (S101);
8-(5-Chlorobenzofuran-2-yl)-4-((5,6-dihydro-[1,2,4]triazolo[4,3-a]pyrazin-7(8H)-yl)sulfonyl)-3,4-dihydro-2H-pyrido[4,3-b][1,4]thiazine (S102);
8-(5-Chlorobenzofuran-2-yl)-4-((1-(oxetan-3-yl)azetidin-3-yl)sulfonyl)-3,4-dihydro-2H-pyrido[4,3-b][1,4]thiazine (S103);
4-(1-tosyl-2,3-dihydro-1*H*-pyrrolo[2,3-c]pyridin-4-yl)benzonitrile (C1);
4-(1-((4-nitrophenyl) sulfonyl)-2,3-dihydro-1*H*-pyrrolo[2,3-*c*]pyridin-4-yl)benzonitrile (C2);
4-(1-((4-methoxyphenyl)sulfonyl)-2,3-dihydro-1*H*-pyrrolo[2,3-*c*]pyridin-4-yl)benzonitrile (C3);
4-(1-((4-(trifluoromethyl)phenyl)sulfonyl)-2,3-dihydro-1*H*-pyrrolo[2,3-*c*]pyridin-4-yl)benzonitrile (C4);
4-(1-((6-chloropyridin-3-yl)sulfonyl)-2,3-dihydro-1*H*-pyrrolo[2,3-*c*]pyridin-4-yl)benzonitrile (C5);
4-(1-((3-fluorophenyl)sulfonyl)-2,3-dihydro-1*H*-pyrrolo[2,3-*c*]pyridin-4-yl)benzonitrile (C6);
4-(1-((4-fluorophenyl)sulfonyl)-2,3-dihydro-1*H*-pyrrolo[2,3-*c*]pyridin-4-yl)benzonitrile (C7);
4-(1-(m-tolylsulfonyl)-2,3-dihydro-1*H*-pyrrolo[2,3-*c*]pyridin-4-yl)benzonitrile (C8);
4-(1-((3-nitrophenyl)sulfonyl)-2,3-dihydro-1*H*-pyrrolo[2,3-*c*]pyridin-4-yl)benzonitrile (C9);
4-(1-(pyridin-3-ylsulfonyl)-2,3-dihydro-1*H*-pyrrolo[2,3-*c*]pyridin-4-yl)benzonitrile (C10);
4-(1-(quinolin-8-ylsulfonyl)-2,3-dihydro-1*H*-pyrrolo[2,3-*c*]pyridin-4-yl)benzonitrile (C11);
4-(1-(naphthalen-2-ylsulfonyl)-2,3-dihydro-1*H*-pyrrolo[2,3-*c*]pyridin-4-yl)benzonitrile (C12);
4-(1-((4-chlorophenyl)sulfonyl)-2,3-dihydro-1*H*-pyrrolo[2,3-*c*]pyridin-4-yl)benzonitrile (C13);
4-(1-((3-chlorophenyl)sulfonyl)-2,3-dihydro-1*H*-pyrrolo[2,3-*c*]pyridin-4-yl)benzonitrile (C14);
4-((4-(4-cyanophenyl)-2,3-dihydro-1*H*-pyrrolo[2,3-*c*]pyridin-1-yl)sulfonyl)benzonitrile (C15);
3-((4-(4-cyanophenyl)-2,3-dihydro-1*H*-pyrrolo[2,3-*c*]pyridin-1-yl)sulfonyl)benzonitrile (C16);
4-(1-(thiophen-2-ylsulfonyl)-2,3-dihydro-1*H*-pyrrolo[2,3-*c*]pyridin-4-yl)benzonitrile (C17);
4-(1-(naphthalen-1-ylsulfonyl)-2,3-dihydro-1*H*-pyrrolo[2,3-*c*]pyridin-4-yl)benzonitrile (C18);
4-(1-((3-methoxyphenyl)sulfonyl)-2,3-dihydro-1*H*-pyrrolo[2,3-*c*]pyridin-4-yl)benzonitrile (C19);
4-(1-((3-[trifluoromethyl]phenyl)sulfonyl)-2,3-dihydro-1*H*-pyrrolo[2,3-*c*]pyridin-4-yl)benzonitrile (C20);
4-(1-(phenylsulfonyl)-2,3-dihydro-1*H*-pyrrolo[2,3-*c*]pyridin-4-yl)benzonitrile (C21);
4-(1-(4-(trifluoromethyl)benzoyl)-2,3-dihydro-1*H*-pyrrolo[2,3-*c*]pyridin-4-yl)benzonitrile (C22);
4-(1-(3-(trifluoromethyl)benzoyl)-2,3-dihydro-1*H*-pyrrolo[2,3-*c*]pyridin-4-yl)benzonitrile (C23);
4-(1-(4-methoxybenzoyl)-2,3-dihydro-1*H*-pyrrolo[2,3-*c*]pyridin-4-yl)benzonitrile (C24);
4-(1-(4-fluorobenzoyl)-2,3-dihydro-1*H*-pyrrolo[2,3-*c*]pyridin-4-yl)benzonitrile (C25);
4-(1-(3-methoxybenzoyl)-2,3-dihydro-1*H*-pyrrolo[2,3-*c*]pyridin-4-yl)benzonitrile (C26);
4-(1-(3-nitrobenzoyl)-2,3-dihydro-1*H*-pyrrolo[2,3-*c*]pyridin-4-yl)benzonitrile (C27);
4-(1-(3-fluorobenzoyl)-2,3-dihydro-1*H*-pyrrolo[2,3-*c*]pyridin-4-yl)benzonitrile (C28);
4-(1-nicotinoyl-2,3-dihydro-1*H*-pyrrolo[2,3-*c*]pyridin-4-yl)benzonitrile (C29);
4-(1-(4-nitrobenzoyl)-2,3-dihydro-1*H*-pyrrolo[2,3-*c*]pyridin-4-yl)benzonitrile (C30);
4-(1-(2-fluorobenzoyl)-2,3-dihydro-1*H*-pyrrolo[2,3-*c*]pyridin-4-yl)benzonitrile (C31);
4-(1-(2-(trifluoromethyl)benzoyl)-2,3-dihydro-1*H*-pyrrolo[2,3-*c*]pyridin-4-yl)benzonitrile (C32);
4-(1-(4-methylbenzoyl)-2,3-dihydro-1*H*-pyrrolo[2,3-*c*]pyridin-4-yl)benzonitrile (C33);
4-(1-(2-methoxybenzoyl)-2,3-dihydro-1*H*-pyrrolo[2,3-*c*]pyridin-4-yl)benzonitrile (C34);
4-(1-(2-nitrobenzoyl)-2,3-dihydro-1*H*-pyrrolo[2,3-*c*]pyridin-4-yl)benzonitrile (C35);
4-(1-(2-methylbenzoyl)-2,3-dihydro-1*H*-pyrrolo[2,3-*c*]pyridin-4-yl)benzonitrile (C36);
3-(4-(4-cyanophenyl)-2,3-dihydro-1*H*-pyrrolo[2,3-*c*]pyridine-1-carbonyl)benzonitrile (C37);
4-(1-(4-chlorobenzoyl)-2,3-dihydro-1*H*-pyrrolo[2,3-*c*]pyridin-4-yl)benzonitrile (C38);
4-(1-(3-chlorobenzoyl)-2,3-dihydro-1*H*-pyrrolo[2,3-*c*]pyridin-4-yl)benzonitrile (C39);
4-(1-(3-methylbenzoyl)-2,3-dihydro-1*H*-pyrrolo[2,3-*c*]pyridin-4-yl)benzonitrile (C40);
4-(1-benzoyl-2,3-dihydro-1*H*-pyrrolo[2,3-*c*]pyridin-4-yl)benzonitrile (C41);
4-(1-pivaloyl-2,3-dihydro-1*H*-pyrrolo[2,3-*c*]pyridin-4-yl)benzonitrile (C42);
4-(1-(2-chloroacetyl)-2,3-dihydro-1*H*-pyrrolo[2,3-*c*]pyridin-4-yl)benzonitrile (C43);
4-(1-(furan-2-carbonyl)-2,3-dihydro-1*H*-pyrrolo[2,3-*c*]pyridin-4-yl)benzonitrile (C44);
4-(1-(3-methylthiophene-2-carbonyl)-2,3-dihydro-1*H*-pyrrolo[2,3-*c*]pyridin-4-yl)benzonitrile (C45);
4-(1-(thiophene-2-carbonyl)-2,3-dihydro-1*H*-pyrrolo[2,3-*c*]pyridin-4-yl)benzonitrile (C46);
Methyl 4-(4-(4-cyanophenyl)-2,3-dihydro-1*H*-pyrrolo[2,3-*c*]pyridine-1-carbonyl)benzoate (C47);
4-(1-(4-cyanobenzoyl)-2,3-dihydro-1*H*-pyrrolo[2,3-*c*]pyridin-4-yl)benzonitrile (C48);
4-(1-(4-(bromomethyl)benzoyl)-2,3-dihydro-1*H*-pyrrolo[2,3-*c*]pyridin-4-yl)benzonitrile (C49);
(4-(1H-indazol-5-yl)-2,3-dihydro-1*H*-pyrrolo[2,3-*c*]pyridin-1-yl)(2-fluorophenyl)methanone (C50);
(2-fluorophenyl)(4-(1-methyl-1H-indazol-5-yl)-2,3-dihydro-1*H*-pyrrolo[2,3-*c*]pyridin-1-yl)-methanone (C51);
(2-fluorophenyl)(4-(3-fluorophenyl)-2,3-dihydro-1*H*-pyrrolo[2,3-*c*]pyridin-1-yl)methanone (C52);
(2-fluorophenyl)(4-(3-nitrophenyl)-2,3-dihydro-1*H*-pyrrolo[2,3-*c*]pyridin-1-yl)methanone (C53);
(4-(4-chlorophenyl)-2,3-dihydro-1*H*-pyrrolo[2,3-*c*]pyridin-1-yl)(2-fluorophenyl)methanone (C54);
3-(1-(2-fluorobenzoyl)-2,3-dihydro-1*H*-pyrrolo[2,3-*c*]pyridin-4-yl)benzonitrile (C55);
(2-fluorophenyl)(4-(furan-3-yl)-2,3-dihydro-1*H*-pyrrolo[2,3-*c*]pyridin-1-yl)methanone (C56);
(2-fluorophenyl)(4-(imidazo[1,2-*a*]pyridin-6-yl)-2,3-dihydro-1*H*-pyrrolo[2,3-c]pyridin-1-yl)-methanone (C57);
(4-(benzo[*d*]thiazol-6-yl)-2,3-dihydro-1*H*-pyrrolo[2,3-*c*]pyridin-1-yl)(2-fluorophenyl)methanone (C58);
(4-(1H-indol-2-yl)-2,3-dihydro-1*H*-pyrrolo[2,3-*c*]pyridin-1-yl)(2-fluorophenyl)methanone (C59);
(4-(benzofuran-2-yl)-2,3-dihydro-1*H*-pyrrolo[2,3-*c*]pyridin-1-yl)(2-fluorophenyl)methanone (C60);
(4-(benzo[*b*]thiophen-2-yl)-2,3-dihydro-1*H*-pyrrolo[2,3-*c*]pyridin-1-yl)(2-fluorophenyl)methanone (C61);
(4-(1H-indol-5-yl)-2,3-dihydro-1*H*-pyrrolo[2,3-*c*]pyridin-1-yl)(2-fluorophenyl)methanone (C62);
(4-(benzofuran-5-yl)-2,3-dihydro-1*H*-pyrrolo[2,3-*c*]pyridin-1-yl)(2-fluorophenyl)methanone (C63);
4-(1-(2-(o-tolyl)acetyl)-2,3-dihydro-1*H*-pyrrolo[2,3-*c*]pyridin-4-yl)benzonitrile (C64);
4-(1-(2-(2-bromophenyl)acetyl)-2,3-dihydro-1*H*-pyrrolo[2,3-*c*]pyridin-4-yl)benzonitrile (C65);
4-(1-(2-(m-tolyl)acetyl)-2,3-dihydro-1*H*-pyrrolo[2,3-*c*]pyridin-4-yl)benzonitrile (C66);
4-(1-(2-(2-fluorophenyl)acetyl)-2,3-dihydro-1*H*-pyrrolo[2,3-*c*]pyridin-4-yl)benzonitrile (C67);
4-(1-(2-(3-methoxyphenyl)acetyl)-2,3-dihydro-1*H*-pyrrolo[2,3-*c*]pyridin-4-yl)benzonitrile (C68);
4-(1-(2-(4-fluorophenyl)acetyl)-2,3-dihydro-1*H*-pyrrolo[2,3-*c*]pyridin-4-yl)benzonitrile (C69);
4-(1-(2-(2-nitrophenyl)acetyl)-2,3-dihydro-1*H*-pyrrolo[2,3-*c*]pyridin-4-yl)benzonitrile (C70);
4-(1-(2-(3-chlorophenyl)acetyl)-2,3-dihydro-1*H*-pyrrolo[2,3-*c*]pyridin-4-yl)benzonitrile (C71);
4-(1-(2-(p-tolyl)acetyl)-2,3-dihydro-1*H*-pyrrolo[2,3-*c*]pyridin-4-yl)benzonitrile (C72);
4-(1-(2-(4-bromophenyl)acetyl)-2,3-dihydro-1*H*-pyrrolo[2,3-*c*]pyridin-4-yl)benzonitrile (C73);
4-(1-(2-(3-(trifluoromethyl)phenyl)acetyl)-2,3-dihydro-1*H*-pyrrolo[2,3-*c*]pyridin-4-yl)benzonitrile (C74);
4-(1-(2-(4-(trifluoromethyl)phenyl)acetyl)-2,3-dihydro-1*H*-pyrrolo[2,3-*c*]pyridin-4-yl)benzonitrile (C75);
4-(1-(2-(4-methoxyphenyl)acetyl)-2,3-dihydro-1*H*-pyrrolo[2,3-*c*]pyridin-4-yl)benzonitrile (C76);
4-(1-(2-(4-chlorophenyl)acetyl)-2,3-dihydro-1*H*-pyrrolo[2,3-*c*]pyridin-4-yl)benzonitrile (C77);
4-((4-(5-chlorobenzofuran-2-yl)-2,3-dihydro-1*H*-pyrrolo[2,3-c]pyridin-1-yl)sulfonyl)benzonitrile (C78);
2-(1-(4-methoxybenzoyl)-2,3-dihydro-1*H*-pyrrolo[2,3-c]pyridin-4-yl)benzofuran-5-carbonitrile (C79);
(4-(5-chloro-1H-indol-2-yl)-2,3-dihydro-1*H*-pyrrolo[2,3-*c*]pyridin-1-yl)(1-(3-fluorobenzyl)azetidin-3-yl)methanone (C80);
2-(1-(1-(3-fluorobenzyl)azetidine-3-carbonyl)-2,3-dihydro-1*H*-pyrrolo[2,3-*c*]pyridin-4-yl)-1*H-*indole-5-carbonitrile (C81);
4-(1-(3-methoxybenzoyl)-2,3-dihydro-1H-pyrrolo[2,3-c]pyridin-4-yl)-3,6-dihydropyridine-1(2H)-carbonitrile (C82);
4-(1-(3-methoxybenzoyl)-2,3-dihydro-1H-pyrrolo[2,3-c]pyridin-4-yl)piperazine-1-carbonitrile (C83);
4-(1-((4-nitrophenyl)sulfonyl)-1*H*-pyrrolo[2,3-*c*]pyridin-4-yl)benzonitrile (E1);
4-(1-tosyl-1*H*-pyrrolo[2,3-*c*]pyridin-4-yl)benzonitrile (E2);
4-(1-(phenylsulfonyl)-1*H*-pyrrolo[2,3-*c*]pyridin-4-yl)benzonitrile (E3);
4-(1-(m-tolylsulfonyl)-1*H*-pyrrolo[2,3-*c*]pyridin-4-yl)benzonitrile (E4);
4-(1-((3-(trifluoromethyl)phenyl)sulfonyl)-1*H*-pyrrolo[2,3-*c*]pyridin-4-yl)benzonitrile (E5);
4-(1-((4-fluorophenyl)sulfonyl)-1*H*-pyrrolo[2,3-*c*]pyridin-4-yl)benzonitrile (E6);
4-(1-((4-(trifluoromethyl)phenyl)sulfonyl)-1*H*-pyrrolo[2,3-*c*]pyridin-4-yl)benzonitrile (E7);
4-(1-((4-chlorophenyl)sulfonyl)-1*H*-pyrrolo[2,3-*c*]pyridin-4-yl)benzonitrile (E8);
4-(1-((4-methoxyphenyl)sulfonyl)-1*H*-pyrrolo[2,3-*c*]pyridin-4-yl)benzonitrile (E9);
3-((4-(4-cyanophenyl)-1*H*-pyrrolo[2,3-*c*]pyridin-1-yl)sulfonyl)benzonitrile (E10);
4-(1-((3-chlorophenyl)sulfonyl)-1*H*-pyrrolo[2,3-*c*]pyridin-4-yl)benzonitrile (E11);
4-(1-((3-fluorophenyl)sulfonyl)-1*H*-pyrrolo[2,3-*c*]pyridin-4-yl)benzonitrile (E12);
4-(1-((3-nitrophenyl)sulfonyl)-1*H*-pyrrolo[2,3-*c*]pyridin-4-yl)benzonitrile (E13);
4-((4-(4-cyanophenyl)-1*H*-pyrrolo[2,3-*c*]pyridin-1-yl)sulfonyl)benzonitrile (E14);
4-(1-(naphthalen-1-ylsulfonyl)-1*H*-pyrrolo[2,3-*c*]pyridin-4-yl)benzonitrile (E15);
4-(1-(naphthalen-2-ylsulfonyl)-1*H*-pyrrolo[2,3-*c*]pyridin-4-yl)benzonitrile (E16);
4-(1-(thiophen-2-ylsulfonyl)-1*H*-pyrrolo[2,3-*c*]pyridin-4-yl)benzonitrile (E17);
4-(1-((6-chloropyridin-3-yl)sulfonyl)-1*H*-pyrrolo[2,3-*c*]pyridin-4-yl)benzonitrile (E18);
4-(1-(perfluorobenzoyl)-1*H*-pyrrolo[2,3-*c*]pyridin-4-yl)benzonitrile (E19);
4-(1-(4-(trifluoromethyl)benzoyl)-1*H*-pyrrolo[2,3-*c*]pyridin-4-yl)benzonitrile (E20);
4-(1-(2-iodobenzoyl)-1*H*-pyrrolo[2,3-*c*]pyridin-4-yl)benzonitrile (E21);
4-(1-(3-nitrobenzoyl)-1*H*-pyrrolo[2,3-*c*]pyridin-4-yl)benzonitrile (E22);
4-(1-(3-(trifluoromethyl)benzoyl)-1*H*-pyrrolo[2,3-*c*]pyridin-4-yl)benzonitrile (E23);
4-(1-(3-methoxybenzoyl)-1*H*-pyrrolo[2,3-*c*]pyridin-4-yl)benzonitrile (E24);
4-(1-(2-methoxybenzoyl)-1*H*-pyrrolo[2,3-*c*]pyridin-4-yl)benzonitrile (E25);
4-(1-(2-(trifluoromethyl)benzoyl)-1*H*-pyrrolo[2,3-*c*]pyridin-4-yl)benzonitrile (E26);
4-(1-(2-methylbenzoyl)-1*H*-pyrrolo[2,3-*c*]pyridin-4-yl)benzonitrile (E27);
4-(1-(2-fluorobenzoyl)-1*H*-pyrrolo[2,3-*c*]pyridin-4-yl)benzonitrile (E28);
4-(1-(2-nitrobenzoyl)-1*H*-pyrrolo[2,3-*c*]pyridin-4-yl)benzonitrile (E29);
4-(1-(4-fluorobenzoyl)-1*H*-pyrrolo[2,3-*c*]pyridin-4-yl)benzonitrile (E30);
(4-(benzo[*b*]thiophen-2-yl)-1*H*-pyrrolo[2,3-c]pyridin-1-yl)(4-fluorophenyl)methanone (E31);
(4-(benzofuran-2-yl)-1*H*-pyrrolo[2,3-*c*]pyridin-1-yl)(4-fluorophenyl)methanone (E32);
(4-(benzofuran-5-yl)-1*H*-pyrrolo[2,3-*c*]pyridin-1-yl)(4-fluorophenyl)methanone (E33);
(4-(1*H*-indol-2-yl)-1*H*-pyrrolo[2,3-*c*]pyridin-1-yl)(4-fluorophenyl)methanone (E34);
(4-fluorophenyl)(4-(1-methyl-1*H*-indazol-6-yl)-1*H*-pyrrolo[2,3-*c*]pyridin-1-yl)methanone (E35);
(4-(benzo[*d*]thiazol-6-yl)-1*H*-pyrrolo[2,3-*c*]pyridin-1-yl)(4-fluorophenyl)methanone (E36);
(4-fluorophenyl)(4-(imidazo[1,2-*a*]pyridin-6-yl)-1*H*-pyrrolo[2,3-*c*]pyridin-1-yl)methanone (E37);
(4-(benzo[*d*]oxazol-6-yl)-1*H*-pyrrolo[2,3-*c*]pyridin-1-yl)(4-fluorophenyl)methanone (E38);
(4-fluorophenyl)(4-(imidazo[1,2-*a*]pyridin-7-yl)-1*H*-pyrrolo[2,3-*c*]pyridin-1-yl)methanone (E39);
4-(1-Methyl-2,3-dioxo-1,2,3,4-tetrahydropyrido[3,4-b]pyrazin-8-yl)benzonitrile (N1);
8-(4-Fluorophenyl)-1-methyl-1,4-dihydropyrido[3,4-b]pyrazine-2,3-dione (N2);
4-(1-Methyl-1,2,3,4-tetrahydropyrido[3,4-b]pyrazin-8-yl)benzonitrile (N3);
4-((8-(4-Cyanophenyl)-1-methyl-2,3-dihydropyrido[3,4-b]pyrazin-4(1H)-yl)sulfonyl)benzonitrile (N4);
4-(4-(4-Fluorobenzoyl)-1-methyl-1,2,3,4-tetrahydropyrido[3,4-b]pyrazin-8-yl)benzonitrile (N5);
4-(1-Methyl-1H-imidazo[4,5-c]pyridin-7-yl)benzonitrile (N6);
4-(1-Cyclopropyl-1H-imidazo[4,5-c]pyridin-7-yl)benzonitrile (N7).

26. A pharmaceutical composition comprising the compound according to any one of claims 1-25 and a pharmaceutically acceptable carrier.

27. The use of the compound according to any one of claims 1 to 25 in the preparation of a medicament for the treatment of diseases related to one or several endogenous steroids.

28. A method for treating diseases related to one or several endogenous steroids in a subject, that is administering to the subject a therapeutically effective amount of the compound according to any one of claims 1-25 or the pharmaceutical composition according to claim 26.

29. The use according to claim 27 or the method according to claim 28, wherein the steroid compound is selected from aldosterone, cortisol, and/or bile acids.

30. The method or the use according to claim 29, wherein the disease associated with aldosterone is selected from the group consisting of primary aldosteronism, secondary aldosteronism, heart failure, renal failure, hypertension, obesity, renal fibrosis, coronary artery disease, cardiac hypertrophy, cardiac fibrosis, arrhythmia, edema, hypokalemia and the resulting muscle weakness, atrial fibrillation, weakened myocardial contraction, congestive heart failure, chronic kidney disease, diabetic nephropathy, cardiorenal syndrome, metabolic syndrome, non-alcoholic fatty liver disease, non-alcoholic steatohepatitis, liver fibrosis, and cirrhosis.

31. The method or the use according to claim 29, wherein the disease associated with cortisol is selected from the group consisting of Cushing's syndrome, metabolic syndrome, insulin resistance, obesity, type 2 diabetes, breast cancer, prostate cancer, tumor metastasis, non-alcoholic fatty liver disease, non-alcoholic steatohepatitis, liver fibrosis, cirrhosis, substance addiction, behavioral addiction, substance use disorders, mood disorders, anxiety disorders, bipolar disorder, sleep disorders, insomnia, post-traumatic stress disorder (PTSD), borderline personality disorder, disruptive behavior disorders, attention deficit hyperactivity disorder (ADHD), major depressive disorder, burnout, chronic fatigue syndrome, fibromyalgia, irritable bowel syndrome, eating disorders, depression, premenstrual syndrome (PMS), obsessive-compulsive disorder (OCD), social anxiety disorder, and generalized anxiety disorder.

32. The method or the use according to claim 29, wherein the disease associated with bile acids is selected from the group consisting of non-alcoholic fatty liver disease (NAFLD), non-alcoholic steatohepatitis (NASH), fatty liver, cirrhosis, liver fibrosis, hepatocellular carcinoma, hyperglycemia, hyperlipidemia, myocardial infarction, heart failure, chronic kidney disease, renal failure, chronic colitis, and ulcerative colitis.

33. A compound selected from the following:

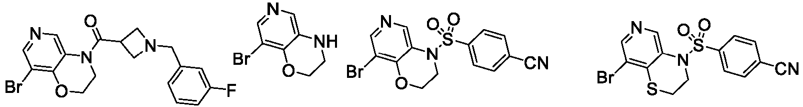

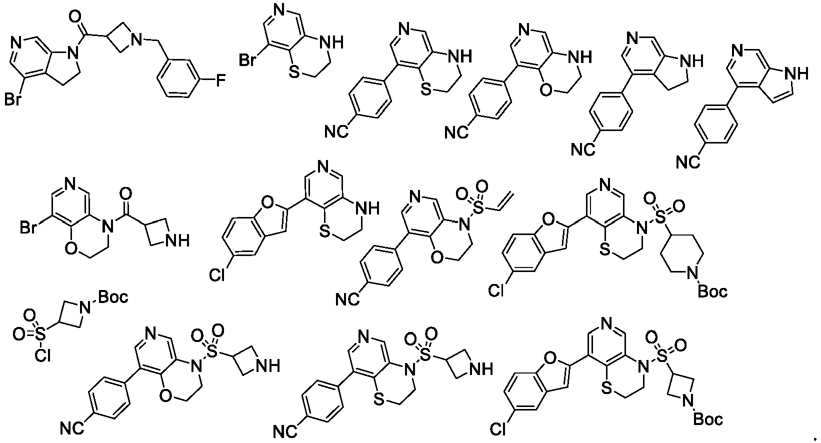

34. A method for preparing any one of the compounds according to any one of claims 1-25.
